(19) Europäisches Patentamt
European Patent Office
Office européen des brevets

(11) **EP 3 431 098 A1**

(12) **EUROPEAN PATENT APPLICATION**

(43) Date of publication:
**23.01.2019 Bulletin 2019/04**

(51) Int Cl.:
***A61K 38/26*** (2006.01)   ***C07K 14/605*** (2006.01)

(21) Application number: **18176085.1**

(22) Date of filing: **12.09.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.09.2011 US 201161573748 P**

(62) Document number(s) of the earlier application(s) in accordance with Art. 76 EPC:
**12831010.9 / 2 755 675**

(71) Applicant: **Amunix Operating Inc.**
**Mountain View, CA 94043 (US)**

(72) Inventors:
• **Schellenberger, Volker**
**Palo Alto, CA 94303 (US)**
• **Silverman, Joshua**
**Sunnyvale, CA 94087 (US)**
• **Stemmer, Willem P.**
**Los Gatos, CA 95051 (US)**
• **Wang, Chia-Wei**
**Milpitas, CA 95035 (US)**
• **Geething, Nathan**
**Acton, MA 01720 (US)**
• **Spink, Benjamin**
**San Carlos, CA 94070 (US)**

(74) Representative: **Mewburn Ellis LLP**
**City Tower**
**40 Basinghall Street**
**London EC2V 5DE (GB)**

Remarks:
•This application was filed on 05-06-2018 as a divisional application to the application mentioned under INID code 62.
•Claims filed after the date of filing of the application/after the date of receipt of the divisional application (Rule 68(4) EPC).

(54) **GLUCAGON-LIKE PEPTIDE-2 COMPOSITIONS AND METHODS OF MAKING AND USING SAME**

(57) The present invention relates to compositions comprising GLP-2 protein or variants thereof linked to extended recombinant polypeptide (XTEN), isolated nucleic acids encoding the compositions and vectors and host cells containing the same, and methods of making and using such compositions in the treatment of GLP-2-related conditions.

```
HGDGSFSDEMNTILDNLAARDFINWLIQTKITDGGSPAGSPTSTEEGTSES
ATPESGPGTSTEPSEGSAPGSPAGSPTSTEEGTSTEPSEGSAPGTSTEPSE
GSAPGTSESATPESGPGSEPATSGSETPGSEPATSGSETPGSPAGSPTSTE
EGTSESATPESGPGTSTEPSEGSAPGTSTEPSEGSAPGSPAGSPTSTEEGT
STEPSEGSAPGTSTEPSEGSAPGTSESATPESGPGTSTEPSEGSAPGTSES
ATPESGPGSEPATSGSETPGTSTEPSEGSAPGTSTEPSEGSAPGTSESATP
ESGPGTSESATPESGPGSPAGSPTSTEEGTSESATPESGPGSEPATSGSET
PGTSESATPESGPGTSTEPSEGSAPGTSTEPSEGSAPGTSTEPSEGSAPGT
STEPSEGSAPGTSTEPSEGSAPGTSTEPSEGSAPGSPAGSPTSTEEGTSTE
PSEGSAPGTSESATPESGPGSEPATSGSETPGTSESATPESGPGSEPATSG
SETPGTSESATPESGPGTSTEPSEGSAPGTSESATPESGPGSPAGSPTSTE
EGSPAGSPTSTEEGSPAGSPTSTEEGTSESATPESGPGTSTEPSEGSAPGT
SESATPESGPGSEPATSGSETPGTSESATPESGPGSEPATSGSETPGTSES
ATPESGPGTSTEPSEGSAPGSPAGSPTSTEEGTSESATPESGPGSEPATSG
SETPGTSESATPESGPGSPAGSPTSTEEGSPAGSPTSTEEGTSTEPSEGSA
PGTSESATPESGPGTSESATPESGPGTSESATPESGPGSEPATSGSETPGS
EPATSGSETPGSPAGSPTSTEEGTSTEPSEGSAPGTSTEPSEGSAPGSEPA
TSGSETPGTSESATPESGPGTSTEPSEGSAPG
```

**FIG. 28**

## Description

### CROSS-REFERENCE TO RELATED APPLICATION

This application claims priority benefit to U.S. Provisional Application Serial No. 61/573,748 filed September 12, 2011, and which application is incorporated herein by reference in its entirety.

### BACKGROUND OF THE INVENTION

Glucagon-like peptide-2 (GLP-2) is an endocrine peptide that, in humans, is generated as a 33 amino acid peptide by post-translational proteolytic cleavage of proglucagon; a process that also liberates the related glucagon-like peptide-1 (GLP-1). GLP-2 is produced and secreted in a nutrient-dependent fashion by the intestinal endocrine L cells. GLP-2 is trophic to the intestinal mucosal epithelium via stimulation of crypt cell proliferation and reduction of enterocyte apoptosis. GLP-2 exerts its effects through specific GLP-2 receptors but the responses in the intestine are mediated by indirect pathways in that the receptor is not expressed on the epithelium but on enteric neurons (Redstone, HA, et al. The Effect of Glucagon-Like Peptide-2 Receptor Agonists on Colonic Anastomotic Wound Healing. Gastroenterol Res Pract. (2010); 2010: Art. ID: 672453).

The effects of GLP-2 are multiple, including intestinaltrophic effects resulting in an increase in intestinal absorption and nutrient assimilation (Lovshin, J. and D.J. Drucker, Synthesis, secretion and biological actions of the glucagon-like peptides. Ped. Diabetes (2000) 1(1):49-57); anti-inflammatory activities; mucosal healing and repair; decreasing intestinal permeability; and an increase in mesenteric blood flow (Bremholm, L. et al. Glucagon-like peptide-2 increases mesenteric blood flow in humans. Scan. J. Gastro. (2009) 44(3):314-319). Exogenously administered GLP-2 produces a number of effects in humans and rodents, including slowing gastric emptying, increasing intestinal blood flow and intestinal growth/mucosal surface area, enhancement of intestinal function, reduction in bone breakdown and neuroprotection. GLP-2 may act in an endocrine fashion to link intestinal growth and metabolism with nutrient intake. In inflamed mucosa, however, GLP-2 action is antiproliferative, decreasing the expression of proinflammatory cytokines while increasing the expression of IGF-1, promoting healing of inflamed mucosa.

Many patients require surgical removal of the small or large bowel for a wide range of conditions, including colorectal cancer, inflammatory bowel disease, irritable bowel syndrome, and trauma. Short bowel syndrome (SBS) patients with end jejunostomy and no colon have reduced release of GLP-2 in response to a meal due to the removal of secreting L cells. Patients with active Crohn's Disease or ulcerative colitis have endogenous serum GLP-2 concentrations that are increased, suggesting the possibility of a normal adaptive response to mucosal injury (Buchman, A. L., et al. Teduglutide, a novel mucosally active analog of glucagon-like peptide-2 (GLP-2) for the treatment of moderate to severe Crohn's disease. Inflammatory Bowel Diseases, (2010) 16:962-973).

Exogenously administered GLP-2 and GLP-2 analogues have been demonstrated in animal models to promote the growth and repair of the intestinal epithelium, including enhanced nutrient absorption following small bowel resection and alleviation of total parenteral nutrition-induced hypoplasia in rodents, as well as demonstration of decreased mortality and improvement of disease-related histopathology in animal models such as indomethacin-induced enteritis, dextran sulfate-induced colitis and chemotherapy-induced mucositis. Accordingly, GLP-2 and related analogs may be treatments for short bowel syndrome, irritable bowel syndrome, Crohn's disease, and other diseases of the intestines (Moor, BA, et al. GLP-2 receptor agonism ameliorates inflammation and gastrointestinal stasis in murine post-operative ileus. J Pharmacol Exp Ther. (2010) 333(2):574-583). However, native GLP-2 has a half-life of approximately seven minutes due to cleavage by dipeptidyl peptidase IV (DPP-IV) (Jeppesen PB, et al., Teduglutide (ALX-0600), a dipeptidyl peptidase IV resistant glucagon-like peptide 2 analogue, improves intestinal function in short bowel syndrome patients. Gut. (2005) 54(9):1224-1231; Hartmann B, et al. (2000) Dipeptidyl peptidase IV inhibition enhances the intestinotrophic effect of glucagon-like peptide-2 in rats and mice. Endocrinology 141:4013-4020). It has been determined that modification of the GLP-2 sequence by replacement of alanine with glycine in position 2 blocks degradation by DPP-IV, extending the half life of the analog called teduglutide to 0.9-2.3 hours (Marier JF, Population pharmacokinetics of teduglutide following repeated subcutaneous administrations in healthy participants and in patients with short bowel syndrome and Crohn's disease. J Clin Pharmacol. (2010) 50(1):36-49). However, recent clinical trials utilizing teduglutide in patients with short bowel syndrome required daily administration of the GLP-2 analog to achieve a clinical benefit (Jeppesen PB, Randomized placebo-controlled trial of teduglutide in reducing parenteral nutrition and/or intravenous fluid requirements in patients with short bowel syndrome. Gut (2011) 60(7):902-914).

Chemical modifications to a therapeutic protein can modify its in vivo clearance rate and subsequent half-life. One example of a common modification is the addition of a polyethylene glycol (PEG) moiety, typically coupled to the protein via an aldehyde or N-hydroxysuccinimide (NHS) group on the PEG reacting with an amine group (e.g. lysine side chain or the N-terminus). However, the conjugation step can result in the formation of heterogeneous product mixtures that need to be separated, leading to significant product loss and complexity of manufacturing and does not result in a completely chemically-uniform product. Also, the pharmacologic function of pharmacologically-active proteins may be hampered if amino acid side chains in the vicinity of its binding site become modified by the PEGylation process. Other approaches include the genetic fusion of an Fc domain to the therapeutic protein, which increases the size of the therapeutic protein, hence reducing the rate of clearance through the kidney. Additionally, the Fc domain confers the ability to bind to, and be recycled from lysosomes by, the FcRn receptor, which results in increased pharmacokinetic half-life. A form of GLP-2 fused to Fc has been evaluated in a murine model of gastrointestinal inflammation associated with postoperative ileus (Moor, BA, et al. GLP-2 receptor agonism ameliorates inflammation and gastrointestinal stasis in murine post-operative ileus. J Pharmacol Exp Ther. (2010) 333(2):574-583). Unfortunately, the Fc domain does not fold efficiently during recombinant expression, and tends to form insoluble precipitates known as inclusion bodies. These inclusion bodies must be solubilized and functional protein must be renatured from the misfolded aggregate, a time-consuming, inefficient, and expensive process.

## SUMMARY OF THE INVENTION

Accordingly, there remains a considerable need for GL-2 compositions and formulations with increased half-life and retention of activity and bioavailability when administered as part of a preventive and/or therapeutic regimen for GLP-2 associated conditions and diseases that can be administered less frequently, and are safer and less complicated and costly to produce. The present invention addresses this need and provides related advantages as well. The present invention relates to novel GLP-2 compositions and uses thereof. Specifically, the compositions provided herein are particularly used for the treatment or improvement of a gastrointestinal a condition. In one aspect, the present invention provides compositions of fusion proteins comprising a recombinant glucagon-like protein-2 ("GLP-2") and one or more extended recombinant polypeptides ("XTEN"). A subject XTEN is typically a polypeptide with a non-repetitive sequence and unstructured conformation that is useful as a fusion partner to GLP-2 peptides in that it confers enhanced properties to the rsulting fusion protein. In one embodiment, one or more XTEN is linked to a GLP-2 or sequence variants thereof, resulting in a GLP-2-XTEN fusion protein ("GLP2-XTEN"). The present disclosure also provides pharmaceutical compositions comprising the fusion proteins and the uses thereof for treating GLP-2-related conditions. In one aspect, the GLP2-XTEN compositions have enhanced pharmacokinetic and/or physicochemical properties compared to recombinant GLP-2 not linked to the XTEN, which permit more convenient dosing and result in improvement in one or more parameters associated with the gastrointestinal condition. The GLP2-XTEN fusion proteins of the embodiments disclosed herein exhibit one or more or any combination of the improved properties and/or the embodiments as detailed herein. In some embodiments, the GLP2-XTEN compositions of the invention do not have a component selected the group consisting of: polyethylene glycol (PEG), albumin, antibody, and an antibody fragment.

In one embodiment, the invention provides a recombinant GLP-2 fusion protein comprising an XTEN, wherein the XTEN is characterized in that a) the XTEN comprises at least 36, or at least 72, or at least 96, or at least 120, or at least 144, or at least 288, or at least 576, or at least 864, or at least 1000, or at least 2000, or at least 3000 amino acid residues; b) the sum of glycine (G), alanine (A), serine (S), threonine (T), glutamate (E) and proline (P) residues constitutes at least about 80%, or at least about 90%, or at least about 95%, or at least about 96%, or at least about 97%, or at least about 98%, or at least about 99%, of the total amino acid residues of the XTEN; c) the XTEN is substantially non-repetitive such that (i) the XTEN contains no three contiguous amino acids that are identical unless the amino acids are serine; (ii) at least about 80%, or at least about 90%, or at least about 91%, or at least about 92%, or at least about 93%, or at least about 94%, or at least about 95%, or at least about 96%, or at least about 97%, or at least about 98%, or at least about 99%, of the XTEN sequence consists of non-overlapping sequence motifs, each of the sequence motifs comprising about 9 to about 14, or about 12 amino acid residues consisting of three, four, five or six types of amino acids selected from glycine (G), alanine (A), serine (S), threonine (T), glutamate (E) and proline (P), wherein any two contiguous amino acid residues do not occur more than twice in each of the non-overlapping sequence motifs; or (iii) the XTEN sequence has a subsequence score of less than 10; d) the XTEN has greater than 90%, or greater than 95%, or greater than 99%, random coil formation as determined by GOR algorithm; e) the XTEN has less than 2% alpha helices and 2% beta-sheets as determined by Chou-Fasman algorithm; f) the XTEN lacks a predicted T-cell epitope when analyzed by TEPITOPE algorithm, wherein the TEPITOPE threshold score for said prediction by said algorithm has a threshold of -9; wherein said fusion protein exhibits an apparent molecular weight factor of at least about 4, or at least about 5, or at least about 6, or at least about 7, or at least about 8, or at least about 9, or at least about 10, or at least about 11, or at least about 12, or at least about 15, or at least about 20 when measured by size exclusion chromatography or comparable method and exhibits an intestinotrophic effect when administered to a subject using a therapeutically effective amount. In the foregoing embodiment, the XTEN can have any one of elements (a)-(d) or any combination of (a)-(d). In another embodiment of the foregoing, the fusion protein exhibits an apparent molecular weight of at least about 200 kDa, or at least about 400 kDa, or at least about 500 kDa, or at least about 700 kDa, or at least about 1000 kDa, or at least about 1400 kDa, or at least about 1600 kDa, or at least about 1800kDa, or at least about 2000 kDa, or at least about 3000 kDa. In another embodiment of the foregoing, the fusion protein exhibits a terminal half-life that is longer than about 24, or about 30, or about 48, or about 72, or about 96, or about 120, or about 144 hours when administered to a subject, wherein the subject is selected from mouse, rat, monkey and man. In one embodiment, the XTEN of the fusion protein is characterized in that at least about 80%, or at least about 90%, or at least about 91%, or at least about 92%, or at least about 93%, or at least about 94%, or at least about 95%, or at least about 96%, or at least about 97%, or at least about 98%, or at least about 99% of the XTEN sequence consists of non-overlapping sequence motifs wherein the motifs are selected from Table 3. In some embodiments, the XTEN of the fusion proteins are further characterized in that the sum of asparagine and glutamine residues is less than 10%, or less than 5%, or less than 2% of the total amino acid sequence of the XTEN. In other embodiments, the XTEN of the fusion proteins are further characterized in that the sum of methionine and tryptophan residues is less than 2% of the total amino acid sequence of the XTEN. In still other embodiments, the XTEN of the fusion proteins are further characterized in that the XTEN has less than 5% amino acid residues with a positive charge. In one embodiment, the intestinotrophic effect of the administered fusion protein is at least about 30%, or at least about 40%, or at least about 50%, or at least about 60%, or at least about 70%, or at least about 80%, or at least about 90%, or at least about 100% or at least about 120% or at least about 150% or at least about 200% of the intestinotrophic effect compared to the corresponding GLP-2 not linked to XTEN and administered to a subject using a comparable dose. In one embodiment, the intestinotrophic effect is manifest in a subject selected from the group consisting of mouse, rat, monkey, and human. In the foregoing embodiments, said administration is subcutaneous, intramuscular, or intravenous. In another embodiment, the intestinotrophic effect is determined after administration of 1 dose, or 3 doses, or 6 doses, or 10 doses, or 12 or more doses of the fusion protein. In another embodiment, the intestinotrophic effect is selected from the group consisting of intestinal growth, increased hyperplasia of the villus epithelium, increased crypt cell proliferation, increased height of the crypt and villus axis, increased healing after intestinal anastomosis, increased small bowel weight, increased small bowel length, decreased small bowel epithelium apoptosis, reduced ulceration, reduced intestinal adhesions, and enhancement of intestinal function.

In one embodiment, the administration of the GLP2-XTEN fusion protein results in an increase in small intestine weight of at least about 10%, or at least about 20%, or at least about 30%. In another embodiment, the administration results in an increase in small intestine length of at least about 5%, or at least about 6%, or at least about 7%, or at least about 8%, or at least about 9%, or at least about 10%, or at least about 20%, or at least about 30%.

In one embodiment, the GLP-2 sequence of the fusion protein has at least 90%, or at least about 91%, or at least about 92%, or at least about 93%, or at least about 94%, or at least about 95%, or at least about 96%, or at least about 97%, or at least about 98%, or at least about 99%, or about 100% sequence identity to a sequence selected from the group consisting of the sequences in Table 1, when optimally aligned. In another embodiment, the GLP-2 of the fusion protein comprises human GLP-2. In another embodiment, the GLP-2 of the fusion protein comprises a GLP-2 of a species origin other than human, such as bovine GLP-2, pig GLP-2, sheep GLP-2, chicken GLP-2, and canine GLP-2. In some embodiments, the GLP-2 of the fusion proteins has an amino acid substitution in place of Ala[2], wherein the substitution is glycine. In yet another embodiment, the GLP-2 of the fusion protein has the sequence HGDGSFSDEMNTILDNLAARDFINWLIQTKITD.

In one embodiment of the GLP2-XTEN fusion protein, the XTEN is linked to the C-terminus of the GLP-2. In another embodiment of the GLP2-XTEN fusion protein wherein the XTEN is linked to the C-terminus of the GLP-2, the fusion protein further comprises a spacer sequence of 1 to about 50 amino acid residues linking the GLP-2 and XTEN components. In one embodiment, the spacer sequence is a single glycine residue.

In one embodiment of the GLP2-XTEN fusion protein, the XTEN is characterized in that: (a) the total XTEN amino acid residues is at least 36 to about 3000, or about 144 to about 2000, or about 288 to about 1000 amino acid residues; and (b) the sum of glycine (G), alanine (A), serine (S), threonine (T), glutamate (E) and proline (P) residues constitutes at least about 90%, or at least about 95%, or at least about 96%, or at least about 97%, or at least about 98%, or at least about 99%, of the total amino acid residues of the XTEN.

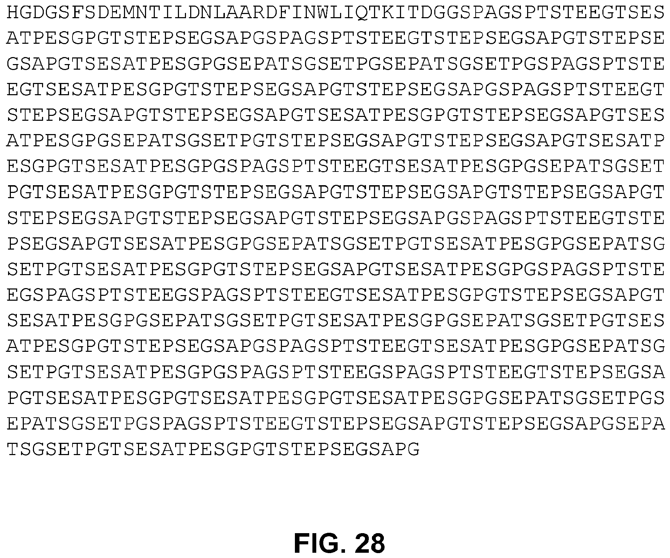

In one embodiment of the GLP2-XTEN fusion protein, the fusion protein comprises one or more XTEN having at least 80%, or at least about 90%, or at least about 95%, or at least about 96%, or at least about 97%, or at least about 98%, or at least about 99% or sequence identity compared to a sequence of comparable length selected from any one of Table 4, Table 8, Table 9, Table 10, Table 11, and Table 12, when optimally aligned. In another embodiment, the fusion protein comprises an XTEN wherein the sequence is AE864 of Table 4. In another embodiment, the fusion protein sequence has a sequence with at least 90%, or at least about 91%, or at least about 92%, or at least about 93%, or at least about 94%, or at least about 95%, or at least about 96%, or at least about 97%, or at least about 98%, or at least about 99%, or 100% sequence identity to the sequence set forth in FIG. 28.

In one embodiment, the fusion protein comprising a GLP-2 and XTEN binds to a GLP-2 receptor with an $EC_{50}$ of less than about 30 nM, or about 100 nM, or about 200 nM, or about 300 nM, or about 370 nM, or about 400 nM, or about 500 nM, or about 600 nM, or about 700 nM, or about 800 nM, or about 1000 nM, or about 1200 nM, or about 1400 nM when assayed using an in vitro GLP2R cell assay. In another embodiment, the fusion protein retains at least about 1%, or about 2%, or about 3%, or about 4%, or about 5%, or about 10%, or about 20%, or about 30% of the potency of the corresponding GLP-2 not linked to XTEN when assayed using an in vitro GLP2R cell assay. In the foregoing embodiments of the paragraph, the GLP2R cell can be a human recombinant GLP-2 glucagon family receptor calcium-optimized cell or another cell comprising GLP2R known in the art.

Non-limiting examples of fusion proteins with a single GLP-2 linked to one or two XTEN are presented in Tables 13 and 32. In one embodiment, the invention provides a fusion protein composition has at least about 80% sequence identity compared to a sequence from Table 13 or Table 33, alternatively at least about 81%, 82%, 83%, 84%, 85%, 86%, 87%, 88%, 89%, 90%, 91%, 92%, 93%, 94%, 95%, 96%, 97%, 98%, 99%, or about 100% sequence identity as compared to a sequence from Table 13 or Table 33. However, the invention also provides substitution of any of the GLP-2 sequences of Table 1 for a GLP-2 in a sequence of Table 33, and substitution of any XTEN sequence of Table 4 for an XTEN in a sequence of Table 33. In some embodiments, the GLP-2 and the XTEN further comprise a spacer sequence of 1 to about 50 amino acid residues linking the GLP-2 and XTEN components, wherein the spacer sequence optionally comprises a cleavage sequence that is cleavable by a protease, including endogenous mammalian proteases. Examples of such protease include, but are not limited to, FXIa, FXIIa, kallikrein, FVIIIa, FVIIIa, FXa, thrombin, elastase-2, granzyme B, MMP-12, MMP-13, MMP-17 or MMP-20, TEV, enterokinase, rhinovirus 3C protease, and sortase A, or a sequence selected from Table 6. In one embodiment, a fusion protein composition with a cleavage sequence has a sequence having at least about 80% sequence identity compared to a sequence from Table 34, alternatively at least about 81%, 82%, 83%, 84%, 85%, 86%, 87%, 88%, 89%, 90%, 91%, 92%, 93%, 94%, 95%, 96%, 97%, 98%, 99%, or about 100% sequence identity as compared to a sequence from Table 34. However, the invention also provides substitution of any of the GLP-2 sequences of Table 1 for a GLP-2 in a sequence of Table 34, and substitution of any XTEN sequence of Table 4 for an XTEN in a sequence of Table 34, and substitution of any cleavage sequence of Table 6 for a cleavage sequence in a sequence of Table 34. In embodiments having the subject cleavage sequences linked to the XTEN, cleavage of the cleavage sequence by the protease releases the XTEN from the fusion protein. In some embodiments of the fusion proteins comprising cleavage sequences that link XTEN to GLP-2, the GLP-2 component becomes biologically active or has an increase in the capacity to bind to GLP-2 receptor upon its release from the XTEN by cleavage of the cleavage sequence, wherein the resulting activity of the cleaved protein is at least about 30%, or at least about 40%, or at least about 50%, or at least about 60%, or at least about 70%, or at least about 80%, or at least about 90% compared to the corresponding GLP-2 not linked to XTEN. In one embodiment of the foregoing, the cleavage sequence is cleavable by a protease of Table 6. In another embodiment, the fusion protein comprises XTEN linked to the GLP-2 by two heterologous cleavage sequences that are cleavable by different proteases, which can be sequences of Table 6. In one embodiment of the foregoing, the cleaved GLP2-XTEN has increased capacity to bind the GLP-2 receptor.

The invention provides that the fusion proteins compositions of the embodiments comprising GLP-2 and XTEN characterized as described above, can be in different N- to C-terminus configurations. In one embodiment of the GLP2-XTEN composition, the invention provides a fusion protein of formula I:

$$(\text{GLP-2})-(\text{XTEN}) \qquad \text{I}$$

wherein independently for each occurrence, GLP-2 is a GLP-2 protein or analog as defined herein, including sequences of Table 1, and XTEN is an extended recombinant polypeptide as defined herein, including sequences exhibiting at least about 80%, or at least about 90%, or at least about 95%, or at least about 99% sequence identity to a sequence of comparable length from any one of of Table 4, Table 8, Table 9, Table 10, Table 11, and Table 12, when optimally aligned. In one embodiment, the XTEN is AE864.

In another embodiment of the GLP2-XTEN composition, the invention provides a fusion protein of formula II:

$$(\text{XTEN})-(\text{GLP-2}) \qquad \text{II}$$

wherein independently for each occurrence, GLP-2 is a GLP-2 protein or analog as defined herein, including sequences of Table 1, and XTEN is an extended recombinant polypeptide as defined herein, including sequences exhibiting at least about 80%, or at least about 90%, or at least about 95%, or at least about 99% sequence identity to a sequence of comparable length from any one of of Table 4, Table 8, Table 9, Table 10, Table 11, and Table 12, when optimally aligned. In one embodiment, the XTEN is AE864.

In another embodiment of the GLP2-XTEN composition, the invention provides an isolated fusion protein, wherein the fusion protein is of formula III:

$$(\text{XTEN})-(\text{GLP-2})-(\text{XTEN}) \qquad \text{III}$$

wherein independently for each occurrence, GLP-2 is a GLP-2 protein or analog as defined herein (e.g., including sequences of Table 1), and XTEN is an extended recombinant polypeptide as defined herein, including sequences exhibiting at least about 80%, or at least about 90%, or at least about 95%, or at least about 99% sequence identity to a sequence of comparable length from any one of of Table 4, Table 8, Table 9, Table 10, Table 11, and Table 12, when optimally aligned. In one embodiment, the XTEN is AE864.

In another embodiment of the GLP2-XTEN composition, the invention provides an isolated fusion protein, wherein the fusion protein is of formula IV:

$$(GLP\text{-}2)\text{-}(XTEN)\text{-}(GLP\text{-}2) \qquad IV$$

wherein independently for each occurrence, GLP-2 is a GLP-2 protein or analog as defined herein (e.g., including sequences of Table 1), and XTEN is an extended recombinant polypeptide as defined herein e.g., including sequences exhibiting at least about 80%, or at least about 90%, or at least about 95%, or at least about 99% sequence identity to a sequence of comparable length from any one of of Table 4, Table 8, Table 9, Table 10, Table 11, and Table 12, when optimally aligned. In one embodiment, the XTEN is AE864.

In another embodiment of the GLP2-XTEN composition, the invention provides an isolated fusion protein, wherein the fusion protein is of formula V:

$$(GLP\text{-}2)\text{-}(S)_x\text{-}(XTEN)_y \ \ V$$

wherein independently for each occurrence, GLP-2 is a GLP-2 protein or analog as defined herein, including sequences of Table 1; S is a spacer sequence having between 1 to about 50 amino acid residues that can optionally include a cleavage sequence or amino acids compatible with restrictions sites; x is either 0 or 1; and XTEN is an extended recombinant polypeptide as defined herein, including sequences exhibiting at least about 80%, or at least about 90%, or at least about 95%, or at least about 99% sequence identity to a sequence of comparable length from any one of of Table 4, Table 8, Table 9, Table 10, Table 11, and Table 12, when optimally aligned. In one embodiment, the XTEN is AE864. In the embodiments of formula V, the spacer sequence comprising a cleavage sequence is a sequence that is cleavable by a mammalian protease selected from the group consisting of factor XIa, factor XIIa, kallikrein, factor VIIa, factor IXa, factor Xa, factor IIa (thrombin), elastase-2, MMP-12, MMP13, MMP-17 and MMP-20. In one embodiment of the fusion protein of formula V, the GLP-2 comprises human GLP-2. In another embodiment of the fusion protein of formula V, the GLP-2 comprises a GLP-2 of a species origin other than human, e.g., bovine GLP-2, pig GLP-2, sheep GLP-2, chicken GLP-2, and canine GLP-2. In another embodiment of the fusion protein of formula V, the GLP-2 has an amino acid substitution in place of $Ala_2$, and wherein the substitution is glycine. In another embodiment, of the fusion protein of formula V, the GLP-2 has the sequence HGDGSFSDEMNTILDNLAARDFINWLIQTKITD. In another embodiment of the fusion protein of formula V, the fusion protein comprises a spacer sequence wherein the spacer sequence is a glycine residue.

In another embodiment of the GLP2-XTEN composition, the invention provides an isolated fusion protein, wherein the fusion protein is of formula VI:

$$(XTEN)_x\text{-}(S)_x\text{-}(GLP\text{-}2)\text{-}(S)_y\text{-}(XTEN)_y \qquad VI$$

 wherein independently for each occurrence, GLP-2 is a GLP-2 protein or analog as defined herein (e.g., including sequences of Table 1); S is a spacer sequence having between 1 to about 50 amino acid residues that can optionally include a cleavage sequence or amino acids compatible with restrictions sites; x is either 0 or 1 and y is either 0 or 1 wherein $x+y \geq 1$; and XTEN is an extended recombinant polypeptide as defined herein, e.g., including exhibiting at least about 80%, or at least about 90%, or at least about 95%, or at least about 99% sequence identity to a sequence of comparable length from any one of of Table 4, Table 8, Table 9, Table 10, Table 11, and Table 12, when optimally aligned. In one embodiment, the XTEN is AE864. In the embodiments of formula VI, the spacer sequence comprising a cleavage sequence is a sequence that is cleavable by a mammalian protease , includingbut not limited to factor XIa, factor XIIa, kallikrein, factor VIIa, factor IXa, factor Xa, factor IIa (thrombin), elastase-2, MMP-12, MMP13, MMP-17 and MMP-20.

In some embodiments, administration of a therapeutically effective dose of a fusion protein of one of formulae I-VI to a subject in need thereof can result in a gain in time of at least two-fold, or at least three-fold, or at least four-fold, or at least five-fold, or at least 10-fold or more spent within a therapeutic window for the fusion protein compared to the corresponding GLP-2 not linked to the XTEN and administered at a comparable dose to a subject. In other cases, administration of a therapeutically effective dose of a fusion protein of an embodiment of formulae I-VI to a subject in need thereof can result in a gain in time between consecutive doses necessary to maintain a therapeutically effective dose regimen of at least 48 h, or at least 72 h, or at least about 96 h, or at least about 120 h, or at least about 7 days, or at least about 14 days, or at least about 21 days between consecutive doses compared to administration of a corresponding GLP-2 not linked to XTEN at a comparable dose.

The fusion protein compositions of the embodiments described herein can be evaluated for retention of activity (including after cleavage of any incorporated XTEN-releasing cleavage sites) using any appropriate *in vitro* assay disclosed herein (e.g., the assays of Table 32 or the assays described in the Examples), to determine the suitability of the configuration for use as a therapeutic agent in the treatment of a GLP-2-factor related condition. In one embodiment, the fusion protein exhibits at least about 2%, or at least about 5%, or at least about 10%, or at least about 20%, or at least about 30%, or at least about 40%, or at least about 50%, or at least about 60%, or at least about 70%, or at least about 80%, or at least about 90% of the activity compared to the corresponding GLP-2 not linked to XTEN. In another embodiment, the GLP-2 component released from the fusion protein by enzymatic cleavage of the incorporated cleavage sequence linking the GLP-2 and XTEN components exhibits at least about 50%, or at least about 60%, or at least about 70%, or at least about 80%, or at least about 90% of the biological activity compared to the corresponding GLP-2 not linked to XTEN.

In some embodiments, fusion proteins comprising GLP-2 and one or more XTEN, wherein the fusion proteins exhibit enhanced pharmacokinetic properties when administered to a subject compared to a GLP-2 not linked to the XTEN, wherein the enhanced properties include but are not limited to longer terminal half-life, larger area under the curve, increased time in which the blood concentration remains within the therapeutic window, increased time between consecutive doses resulting in blood concentrations within the therapeutic window, increased time between $C_{max}$ and $C_{min}$ blood concentrations when consecutive doses are administered, and decreased cumulative dose over time required to be administered compared to a GLP-2 not linked to the XTEN, yet still result in a blood concentration within the therapeutic window. A subject to which a GLP-2-XTEN composition is administered can include but is not limited to mouse, rat, monkey and human. In some embodiments, the terminal half-life of the fusion protein administered to a subject is increased at least about three-fold, or at least about four-fold, or at least about five-fold, or at least about six-fold, or at least about eight-fold, or at least about ten-fold, or at least about 20-fold, or at least about 40-fold, or at least about 60-fold, or at least about 100-fold, or even longer as compared to the corresponding recombinant GLP-2 not linked to the XTEN when the corresponding GLP-2 is administered to a subject at a comparable dose. In other embodiments, the terminal half-life of the fusion protein administered to a subject is at least about 12 h, or at least about 24 h, or at least about 48 h, or at least about 72 h, or at least about 96 h, or at least about 120 h, or at least about 144 h, or at least about 21 days or greater. In other embodiments, the enhanced pharmacokinetic property is reflected by the fact that the blood concentrations remain within the therapeutic window for the fusion protein for a period that is at least about two-fold, or at least about three-fold, or at least about four-fold, or at least about five-fold, or at least about six-fold, or at least about eight-fold, or at least about ten-fold longer, or at least about 20-fold, or at least about 40-fold, or at least about 60-fold, or at least about 100-fold greater compared to the corresponding GLP-2 not linked to the XTEN when thee corresponding GLP-2 is administered to a subject at a comparable dose. The increase in half-life and time spent within the therapeutic window permits less frequent dosing and decreased amounts of the fusion protein (in nmoles/kg equivalent) that are administered to a subject, compared to the corresponding GLP-2 not linked to the XTEN. In one embodiment, administration of three or more doses of a GLP2-XTEN fusion protein to a subject in need thereof using a therapeutically-effective dose regimen results in a gain in time of at least two-fold, or at least three-fold, or at least four-fold, or at least five-fold, or at least six-fold, or at least eight-fold, or at least 10-fold, or at least about 20-fold, or at least about 40-fold, or at least about 60-fold, or at least about 100-fold or higher between at least two consecutive $C_{max}$ peaks and/or $C_{min}$ troughs for blood levels of the fusion protein compared to the corresponding GLP-2 not linked to the XTEN and administered using a comparable dose regimen to a subject. In one embodiment, the GLP2-XTEN administered using a therapeutically effective amount to a subject in need thereof results in blood concentrations of the GLP2-XTEN fusion protein that remain above at least about 500 ng/ml, at least about 1000 ng/ml, or at least about 2000 ng/ml, or at least about 3000 ng/ml, or at least about 4000 ng/ml, or at least about 5000 ng/ml, or at least about 10000 ng/ml, or at least about 15000 ng/ml, or at least about 20000 ng/ml, or at least about 30000 ng/ml, or at least about 40000 ng/ml for at least about 24 hours, or at least about 48 hours, or at least about 72 hours, or at least about 96 hours, or at least about 120 hours, or at least about 144 hours. In another embodiment, the GLP2-XTEN administered at an appropriate dose to a subject results in area under the curve concentrations of the GLP2-XTEN fusion protein of at least 100000 hr*ng/mL, or at least about 200000 hr*ng/mL, or at least about 400000 hr*ng/mL, or at least about 600000 hr*ng/mL, or at least about 800000 hr*ng/mL, or at least about 1000000 hr*ng/mL, or at least about 2000000 hr*ng/mL after a single dose. In one embodiment, the GLP2-XTEN fusion protein has a terminal half-life that results in a gain in time between consecutive doses necessary to maintain a therapeutically effective dose regimen of at least 48 h, or at least 72 h, or at least about 96 h, or at least about 120 h, or at least about 7 days, or at least about 14 days, or at least about 21 days between consecutive doses compared to the regimen of a GLP-2 not linked to XTEN and administered at a comparable dose.

In one embodiment, the GLP2-XTEN fusion protein is characterized in that when an equivalent amount, in nmoles/kg of the fusion protein and the corresponding GLP-2 that lacks the XTEN are each administered to comparable subjects, the fusion protein achieves a terminal half-life in the subject that is at least about 3-fold, or at least 4-fold, or at least 5-fold, or at least 10-fold, or at least 15-fold, or at least 20-fold longer compared to the corresponding GLP-2 that lacks the XTEN. In another embodiment, the GLP2-XTEN fusion protein is characterized in that when a 2-fold, or 3-fold, or 4-fold, or 5-fold, or 6-fold smaller amount, in nmoles/kg, of the fusion protein than the corresponding GLP-2 that lacks the XTEN are each administered to comparable subjects with a gastrointestinal condition, the fusion protein achieves a comparable therapeutic effect in the subject as the corresponding GLP-2 that lacks the XTEN. In another embodiment, the GLP2-XTEN fusion protein is characterized in that when the fusion protein is administered to a subject in consecutive doses to a subject using a dose interval that is at least about 2-fold, or at least 3-fold, or at least 4-fold, or at least 5-fold, or at least 10-fold, or at least 15-fold, or at least 20-fold longer as compared to a dose interval for the corresponding GLP-2 that lacks the XTEN and is administered to a comparable subject using an otherwise equivalent nmoles/kg amount, the fusion protein achieves a similar blood concentration in the subject as compared to the corresponding GLP-2 that lacks the XTEN. In another embodiment, the GLP2-XTEN fusion protein is characterized in that when the fusion protein is administered to a subject in consecutive doses to a subject using a dose interval that is at least about 3-fold, or at least 4-fold, or at least 5-fold, or at least 10-fold, or at least 15-fold, or at least 20-fold longer as compared to a dose interval for the corresponding GLP-2 that lacks the XTEN and is administered to a comparable subject using an otherwise equivalent nmoles/kg amount, the fusion protein achieves a comparable therapeutic effect in the subject as the corresponding GLP-2 that lacks the XTEN. In another embodiment, the GLP2-XTEN fusion protein exhibits any combination of, or all of the foregoing characterisitics of this paragraph. In the embodiments of this paragraph, the subject to which the subject composition is administered can include but is not, limited to mouse, rat, monkey, and human. In one embodiment, the subject is rat. In another embodiment, the subject is human.

In one embodiment, the administration of a GLP2-XTEN fusion protein to a subject results in a greater therapeutic effect compared to the effect seen with the corresponding GLP-2 not linked to XTEN. In another embodiment, the administration of an effective amount the fusion protein results in a greater therapeutic effect in a subject with enteritis compared to the corresponding GLP-2 not linked to XTEN and administered to a comparable subject using a comparable nmoles/kg amount. In the foregoing, the subject is selected from the group consisting of mouse, rat, monkey, and human. In one embodiment of the foregoing, the subject is human and the enteritis is Crohn's disease. In another embodiment of the foregoing, the subject is rat subject and the enteritis is induced with indomethacin. In the foregoing embodiments of this paragraph, the greater therapeutic effect is selected from the group consisting of body weight gain, small intestine length, reduction in TNF$\alpha$ content of the small intestine tissue, reduced mucosal atrophy, reduced incidence of perforated ulcers, and height of villi. In one embodiment, the administration of a GLP2-XTEN fusion protein to a subject results in an increase in small intestine weight of at least about 10%, or at least about 20%, or at least about 30%, or at least about 40% greater compared to that of the corresponding GLP-2 not linked to XTEN. In another embodiment of the administration of a GLP2-XTEN fusion protein to a subject, the administration results in an increase in small intestine length of at least about 5%, or at least about 6%, or at least about 7%, or at least about 8%, or at least about 9%, or at least about 10%, or at least about 20%, or at least about 30%, or at least about 40% greater compared to that of the corresponding GLP-2 not linked to XTEN. In another embodiment of the administration of a GLP2-XTEN fusion protein to a subject, the administration results in an increase in body weight is at least about 5%, or at least about 6%, or at least about 7%, or at least about 8%, or at least about 9%, or at least about 10%, or at least about 20%, or at least about 30%, or at least about 40% greater compared to that of the corresponding GLP-2 not linked to XTEN. In another embodiment of the administration of a GLP2-XTEN fusion protein to a subject, the administration results a reduction in TNF$\alpha$ content of at least about 0.5 ng/g, or at least about 0.6 ng/g, or at least about 0.7 ng/g, or at least about 0.8 ng/g, or at least about 0.9 ng/g, or at least about 1.0 ng/g, or at least about 1.1 ng/g, or at least about 1.2 ng/g, or at least about 1.3 ng/g, or at least about 1.4 ng/g of small intestine tissue or greater compared to that of the corresponding GLP-2 not linked to XTEN. In another embodiment of the administration of a GLP2-XTEN fusion protein to a subject, the administration results in an increase in villi height of at least about 5%, or at least about 6%, or at least about 7%, or at least about 8%, or at least about 9%, or at least about 10%, or at least about 11%, or at least about 12% greater compared to that of the corresponding GLP-2 not linked to XTEN. In the foregoing embodiments of this paragraph, the fusion protein is administered as 1, or 2, or 3, or 4, or 5, or 6, or 10, or 12 or more consecutive doses, wherein the dose amount is at least about 5, or least about 10, or least about 25, or least about 100, or least about 200 nmoles/kg.

In one embodiment, the GLP2-XTEN recombinant fusion protein comprises a GLP-2 linked to the XTEN via a cleavage sequence that is cleavable by a mammalian protease including but not limited to factor XIa, factor XIIa, kallikrein, factor VIIa, factor IXa, factor Xa, factor IIa (thrombin), Elastase-2, MMP-12, MMP13, MMP-17 and MMP-20, wherein cleavage at the cleavage sequence by the mammalian protease releases the GLP-2 sequence from the XTEN sequence, and wherein the released GLP-2 sequence exhibits an increase in receptor binding activity of at least about 30% compared to the uncleaved fusion protein.

The present invention provides methods of producing the GLP2-XTEN fusion proteins. In some embodiments, the method of producing a fusion protein comprising GLP-2 fused to one or more extended recombinant polypeptides (XTEN), comprises providing a host cell comprising a recombinant nucleic acid encoding the fusion protein of any of the embodiments described herein; culturing the host cell under conditions permitting the expression of the fusion protein; and recovering the fusion protein. In one embodiment of the method, the the host cell is a prokaryotic cell. In another embodiment of the method, the host cell is *E. coli.* In another embodiment of the method, the fusion protein is recovered from the host cell cytoplasm in substantially soluble form. In another embodiment of the method, the recombinant nucleic molecule has a sequence with at least 90%, or at least about 91%, or at least about 92%, or at least about 93%, or at least about 94%, or at least about 95%, or at least about 96%, or at least about 97%, or at least about 98%, or at least about 99%, or about 100% sequence identity to a sequence selected from the group consisting of the DNA sequences set forth in Table 13, when optimally aligned, or the complement thereof.

The present invention provides isolated nucleic acids encoding the GLP2-XTEN fusion proteins, vectors, and host cells comprising the vectors and nucleic acids. In one embodiment, the invention provides an isolated nucleic acid comprising a nucleic acid sequence that has at least 70%, or at least about 80%, or at least about 90%,or at least about 91%, or at least about 92%, or at least about 93%, or at least about 94%, or at least about 95%, or at least about 96%, or at least about 97%, or at least about 98%, or at least about 99%, or 100% sequence identity to a DNA sequence selected from Table 13, or the complement thereof. In another embodiment, the invention provides a nucleotide sequence encoding the fusion protein of any of fusion protein embodiments described herein, or the complement thereof. In another embodiment, the invention provides an expression vector or isolated host cell comprising the nucleic acid of the foregoing embodiments of this paragraph. In another embodiment, the invention provides a host cell comprising the foregoing expression vector.

Additionally, the present invention provides pharmaceutical compositions comprising the fusion protein of any of the foregoing embodiments described herein and a pharmaceutically acceptable carrier. In addition, the present invention provides pharmaceutical compositions comprising the fusion protein of any of the foregoing embodiments described herein for use in treating a gastrointestinal condition in a subject. In one embodiment, administration of a therapeutically effective amount of the pharmaceutical composition to a subject with a gastrointestinal condition results in maintaining blood concentrations of the fusion protein within a therapeutic window for the fusion protein at least three-fold longer compared to the corresponding GLP-2 not linked to the XTEN and administered at a comparable amount to the subject. In another embodiment, administration of three or more doses of the pharmaceutical composition to a subject with a gastrointestinal condition using a therapeutically-effective dose regimen results in a gain in time of at least four-fold between at least two consecutive $C_{max}$ peaks and/or $C_{min}$ troughs for blood levels of the fusion protein compared to the corresponding GLP-2 not linked to the XTEN and administered using a comparable dose regimen to a subject. In another embodiment, the intravenous, subcutaneous, or intramuscular administration of the pharmaceutical composition comprising at least about 5, or least about 10, or least about 25, or least about 100, or least about 200 nmoles/kg of the fusion protein to a subject results in fusion protein blood levels maintained above 1000 ng/ml for at least 72 hours. In the foregoing embodiments of the paragraph, the gastrointestinal condition is selected from the group consisting of gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy-associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, and gastrointestinal ischemia. In the foregoing embodiments of the paragraph, the subject is selected from mouse, rat, monkey and human.

In another embodiment, the present invention provides a GLP2-XTEN fusion protein according to any of the embodiments described herein for use in the preparation of a medicament for the treatment of a gastrointestinal condition described herein.

The present invention provides GLP2-XTEN fusion proteins according to any of the embodiments described herein for use in a method of treating a gastrointestinal condition in a subject, comprising administering to the subject a therapeutically effective amount of the fusion protein. In one embodiment, the gastrointestinal condition is selected from the group consisting of gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy-associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, and gastrointestinal ischemia. In another embodiment of the fusion protein for use in a method of treating a gastrointestinal condition in a subject, administration of two or more consecutive doses of the fusion protein administered using a therapeutically effective dose regimen to a subject results in a prolonged period between consecutive $C_{max}$ peaks and/or $C_{min}$ troughs for blood levels of the fusion protein compared to the corresponding GLP-2 that lacks the XTEN and administered using a therapeutically effective dose regimen established for the GLP-2. In another embodiment of the fusion protein for use in a method of treating a gastrointestinal condition in a subject, administration of a smaller amount in nmoles/kg of the fusion protein to a subject in comparison to the corresponding GLP-2 that lacks the XTEN, when administered to a subject under an otherwise equivalent dose regimen, results in the fusion protein achieving a comparable therapeutic effect as the corresponding GLP-2 that lacks the XTEN. In the foregoing, the therapeutic effect is selected from the group consisting of blood concentrations of GLP-2, increased mesenteric blood flow, decreased inflammation, increased weight gain, decreased diarrhea, decreased fecal wet weight, intestinal wound healing, increase in plasma citrulline concentrations, decreased CRP levels, decreased requirement for steroid therapy, enhancing or stimulating mucosal integrity, decreased sodium loss, minimizing, mitigating, or preventing bacterial translocation in the intestines, enhancing, stimulating or accelerating recovery of the intestines after surgery, preventing relapses of inflammatory bowel disease, and maintaining energy homeostasis.

The present invention provides GLP2-XTEN fusion proteins according to any of the embodiments described herein for use in a pharmaceutical regimen for treatment of a gastrointestinal condition in a subject. In one embodiment, the r pharmaceutical egimen comprises a pharmaceutical composition comprising the GLP2-XTEN fusion protein. In another embodiment, the pharmaceutical regimen further comprises the step of determining the amount of pharmaceutical composition needed to achieve a therapeutic effect in the subject, wherein the therapeutic effect is selected from the group consisting of increased mesenteric blood flow, decreased inflammation, increased weight gain, decreased diarrhea, decreased fecal wet weight, intestinal wound healing, increase in plasma citrulline concentrations, decreased CRP levels, decreased requirement for steroid therapy, enhanced mucosal integrity, decreased sodium loss, preventing bacterial translocation in the intestines, accelerated recovery of the intestines after surgery, prevention of relapses of inflammatory bowel disease, and maintaining energy homeostasis. In another embodiment, the pharmaceutical regimen comprises administering the pharmaceutical composition in two or more successive doses to the subject at an effective amount, wherein the administration results in at least a 5%, or 10%, or 20%, or 30%, or 40%, or 50%, or 60%, or 70%, or 80%, or 90% greater improvement of at least one, two, or three parameters associated with the gastrointestinal condition compared to the GLP-2 not linked to XTEN and administered using a comparable nmol/kg amount. In one embodiment of the foregoing, the parameter improved is selected from increased blood concentrations of GLP-2, increased mesenteric blood flow, decreased inflammation, increased weight gain, decreased diarrhea, decreased fecal wet weight, intestinal wound healing, increase in plasma citrulline concentrations, decreased CRP levels, decreased requirement for steroid therapy, enhanced mucosal integrity, decreased sodium loss, preventing bacterial translocation in the intestines, accelerated recovery of the intestines after surgery, prevention of relapses of inflammatory bowel disease, and maintaining energy homeostasis. In another embodiment, the pharmaceutical regimen comprises administering a therapeutically effective amount of the pharmaceutical composition once every 7, or 10, or 14, or 21, or 28 or more days. In an embodiment of the foregoing, the effective amount is at least about 5, or least about 10, or least about 25, or least about 100, or least about 200 nmoles/kg. In the embodiments of the regimen, the administration is subcutaneous, intramuscular, or intravenous.

The present invention provides methods of treating a gastrointestinal condition in a subject. In some embodiments, the method comprises administering to said subject a composition comprising an effective amount of a pharmaceutical composition comprising a GLP2-XTEN fusion protein described herein. In one embodiment of the method, the effective amount is at least about 5, or least about 10, or least about 25, or least about 100, or least about 200 nmoles/kg. In another embodiment of the method, administration of the pharmaeceutical composition is subcutaneous, intramuscular, or intravenous. In another embodiment of the method, administration of the effective amount results in the fusion protein exhibiting a terminal half-life of greater than about 30 hours in the subject, wherein the subject is selected from the group consisting of mouse, rat, monkey, and human. In the foregoing embodiments, the gastrointestinal condition is selected from the group consisting of gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy-associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, and gastrointestinal ischemia. In another embodiment of the method, the method is used to treat a subject with small intestinal damage due to chemotherapeutic agents such as, but not limited to 5-FU, altretamine, bleomycin, busulfan, capecitabine, carboplatin, carmustine, chlorambucil, cisplatin, cladribine, crisantaspase, cyclophosphamide, cytarabine, dacarbazine, dactinomycin, daunorubicin, docetaxel, doxorubicin, epirubicin, etoposide, fludarabine, fluorouracil, gemcitabine, hydroxycarbamide, idarubicin, ifosfamide, irinotecan, liposomal doxorubicin, leucovorin, lomustine, melphalan, mercaptopurine, mesna, methotrexate, mitomycin, mitoxantrone, oxaliplatin, paclitaxel, pemetrexed, pentostatin, procarbazine, raltitrexed, streptozocin, tegafur-uracil, temozolomide, thiotepa, tioguanine, thioguanine, topotecan, treosulfan, vinblastine, vincristine, vindesine, and vinorelbine. In another embodiment of the method, administration of the pharmaeceutical composition results in an intestinotrophic effect in said subject. In yet another embodiment of the method, administration of the pharmaeceutical composition results in an intestinotrophic effect in said subject, wherein the intestinotrophic effect is at least about 30%, or at least about 40%, or at least about 50%, or at least about 60%, or at least about 70%, or at least about 80%, or at least about 90%, or at least about 100% or at least about 120% or at least about 150% or at least about 200% of the intestinotrophic effect compared to the corresponding GLP-2 not linked to XTEN and administered to a subject using a comparable dose. In one embodiment of the foregoing, the intestinotrophic effect is determined after administration of 1 dose, or 3 doses, or 6 doses, or 10 doses, or 12 or more doses of the fusion protein. In another embodiment of the foregoing, the intestinotrophic effect is selected from the group consisting of intestinal growth, increased hyperplasia of the villus epithelium, increased crypt cell proliferation, increased height of the crypt and villus axis, increased healing after intestinal anastomosis, increased small bowel weight, increased small bowel length, decreased small bowel epithelium apoptosis, and enhancement of intestinal function.

In another embodiment, the present invention provides kits, comprising packaging material and at least a first container comprising the pharmaceutical composition comprising a GLP2-XTEN fusion protein described herein and a sheet of instructions for the reconstitution and/or administration of the pharmaceutical compositions to a subject.

The following are non-limiting exemplary embodiments of the invention:

Item 1. A recombinant fusion protein comprising a glucagon-like protein-2 (GLP-2) and an extended recombinant polypeptide (XTEN), wherein the XTEN is characterized in that:

(a) the XTEN comprises at least 36 amino acid residues;
(b) the sum of glycine (G), alanine (A), serine (S), threonine (T), glutamate (E) and proline (P) residues constitutes more than about 80% of the total amino acid residues of the XTEN;
(c) the XTEN is substantially non-repetitive such that (i) the XTEN contains no three contiguous amino acids that are identical unless the amino acids are serine; (ii) at least about 80% of the XTEN sequence consists of non-overlapping sequence motifs, each of the sequence motifs comprising about 9 to about 14 amino acid residues consisting of four to six amino acids selected from glycine (G), alanine (A), serine (S), threonine (T), glutamate (E) and proline (P), wherein any two contiguous amino acid residues do not occur more than twice in each of the non-overlapping sequence motifs; or (iii) the XTEN sequence has a subsequence score of less than 10;
(d) the XTEN has greater than 90% random coil formation as determined by GOR algorithm;
(e) the XTEN has less than 2% alpha helices and 2% beta-sheets as determined by Chou-Fasman algorithm; and
(f) the XTEN lacks a predicted T-cell epitope when analyzed by TEPITOPE algorithm, wherein the TEPITOPE threshold score for said prediction by said algorithm has a threshold of -9, wherein said fusion protein exhibits an apparent molecular weight factor of at least about 4 and exhibits an intestinotrophic effect when administered to a subject using a therapeutically effective amount.

Item 2. The recombinant fusion protein of item 1, wherein the intestinotrophic effect is at least about 30%, or at least about 40%, or at least about 50%, or at least about 60%, or at least about 70%, or at least about 80%, or at least about 90%, or at least about 100% or at least about 120% or at least about 150% or at least about 200% of the intestinotrophic effect compared to the corresponding GLP-2 not linked to XTEN when the corresponding GLP-2 is administered to a subject using a comparable dose.

Item 3. The recombinant fusion protein of item 1, wherein the subject is selected from the group consisting of mouse, rat, monkey, and human.

Item 4. The recombinant fusion protein of any one of the preceding items, wherein said administration is subcutaneous, intramuscular, or intravenous.

Item 5. The recombinant fusion protein of any one of the preceding items, wherein the intestinotrophic effect is determined after administration of 1 dose, or 3 doses, or 6 doses, or 10 doses, or 12 or more doses of the fusion protein.

Item 6. The recombinant fusion protein of any one of the preceding items, wherein the intestinotrophic effect is selected from the group consisting of intestinal growth, increased hyperplasia of the villus epithelium, increased crypt cell proliferation, increased height of the crypt and villus axis, increased healing after intestinal anastomosis, increased small bowel weight, increased small bowel length, decreased small bowel epithelium apoptosis, and enhancement of intestinal function.

Item 7. The recombinant fusion protein of Item 6, wherein the administration results in an increase in small intestine weight of at least about 10%, or at least about 20%, or at least about 30%.

Item 8. The recombinant fusion protein of Item 6, wherein the administration results in an increase in small intestine length of at least about 5%, or at least about 6%, or at least about 7%, or at least about 8%, or at least about 9%, or at least about 10%, or at least about 20%, or at least about 30%.

Item 9. The recombinant fusion protein of any one of the preceding items, wherein the GLP-2 sequence has at least 90%, or at least about 91%, or at least about 92%, or at least about 93%, or at least about 94%, or at least about 95%, or at least about 96%, or at least about 97%, or at least about 98%, or at least about 99%, or 100% sequence identity to a sequence selected from the group consisting of the sequences in Table 1, when optimally aligned.

Item 10. The recombinant fusion protein of any one of the preceding items, wherein the GLP-2 comprises human GLP-2.

Item 11. The recombinant fusion protein of any one of Item 9-Item 11, wherein the GLP-2 is selected from the group consisting of bovine GLP-2, pig GLP-2, sheep GLP-2, chicken GLP-2, and canine GLP-2.

Item 12. The recombinant fusion protein of any one of the preceding items, wherein the GLP-2 has an amino acid substitution in place of $Ala^2$, and wherein the substitution is glycine.

Item 13. The recombinant fusion protein of any one of Item 1-Item 9, wherein the GLP-2 has the sequence HGDGSFSDEMNTILDNLAARDFINWLIQTKITD.

Item 14. The recombinant fusion protein any one of the preceding items, wherein the XTEN is linked to the C-terminus of the GLP-2.

Item 15. The recombinant fusion protein of Item 14, further comprising a spacer sequence of 1 to about 50 amino acid residues linking the GLP-2 and XTEN components.

Item 16. The recombinant fusion protein of Item 15, wherein the spacer sequence is a glycine residue.

Item 17. The recombinant fusion protein of any one of the preceding items, wherein the XTEN is characterized in that:

(a) the total XTEN amino acid residues is at least 36 to about 3000 amino acid residues; and
(b) the sum of glycine (G), alanine (A), serine (S), threonine (T), glutamate (E) and proline (P) residues constitutes at least about 90% of the total amino acid residues of the XTEN;

Item 18. The recombinant fusion protein of any one of the preceding items, wherein the XTEN is characterized in that the sum of asparagine and glutamine residues is less than 10% of the total amino acid sequence of the XTEN.

Item 19. The recombinant fusion protein of any one of the preceding items, wherein the XTEN is characterized in that the sum of methionine and tryptophan residues is less than 2% of the total amino acid sequence of the XTEN.

Item 20. The recombinant fusion protein any one of the preceding items, wherein the XTEN has at least 90%, or at least about 91%, or at least about 92%, or at least about 93%, or at least about 94%, or at least about 95%, or at least about 96%, or at least about 97%, or at least about 98%, or at least about 99%, or about 100% sequence identity when compared to a sequence of comparable length selected from any one of Table 4, Table 8, Table 9, Table 10, Table 11, and Table 12, when optimally aligned.

Item 21. The recombinant fusion protein any one of the preceding items, wherein the XTEN has at least 90%, or at least about 91%, or at least about 92%, or at least about 93%, or at least about 94%, or at least about 95%, or at least about 96%, or at least about 97%, or at least about 98%, or at least about 99%, or about 100% sequence identity when compared to an AE864 sequence from Table 4, when optimally aligned.

Item 22. The recombinant fusion protein of any one of Item 1-Item 9 or Item 13, wherein the fusion protein sequence has a sequence with at least 90%, or at least about 91%, or at least about 92%, or at least about 93%, or at least about 94%, or at least about 95%, or at least about 96%, or at least about 97%, or at least about 98%, or at least about 99%, or 100% sequence identity to the sequence set forth in FIG. 28.

Item 23. The recombinant fusion protein of any one of the preceding items, wherein the fusion protein exhibits a terminal half-life that is at least about 30 hours when administered to a subject.

Item 24. The recombinant fusion protein of any one of the preceding items, wherein the fusion protein binds to a GLP-2 receptor with an $EC_{50}$ of less than about 30 nM, or about 100 nM, or about 200 nM, or about 300 nM, or about 370 nM, or about 400 nM, or about 500 nM, or about 600 nM, or about 700 nM, or about 800 nM, or about 1000 nM, or about 1200 nM, or about 1400 nM when assayed using an in vitro GLP2R cell assay wherein the GLP2R cell is a human recombinant GLP-2 glucagon family receptor calcium-optimized cell.

Item 25. The recombinant fusion protein of any one of the preceding items, wherein the fusion protein retains at least about 1%, or about 2%, or about 3%, or about 4%, or about 5%, or about 10%, or about 20%, or about 30% of the potency of the corresponding GLP-2 not linked to XTEN when assayed using an in vitro GLP2R cell assay wherein the GLP2R cell is a human recombinant GLP-2 glucagon family receptor calcium-optimized cell.

Item 26. The recombinant fusion protein of any one of the preceding items, characterized in that

(a) when an equivalent amount, in nmoles/kg, of the fusion protein and the corresponding GLP-2 that lacks the XTEN are each administered to comparable subjects, the fusion protein achieves a terminal half-life in the subject that is at least about 3-fold, or at least 4-fold, or at least 5-fold, or at least 10-fold, or at least 15-fold, or at least 20-fold longer compared to the corresponding GLP-2 that lacks the XTEN;

(b) when a 2-fold, or 3-fold, or 4-fold, or 5-fold, or 6-fold smaller amount, in nmoles/kg, of the fusion protein than the corresponding GLP-2 that lacks the XTEN are each administered to comparable subjects with a gastrointestinal condition, the fusion protein achieves a comparable therapeutic effect in the subject as the corresponding GLP-2 that lacks the XTEN;

(c) when the fusion protein is administered to a subject in consecutive doses to a subject using a dose interval that is at least about 2-fold, or at least 3-fold, or at least 4-fold, or at least 5-fold, or at least 10-fold, or at least 15-fold, or at least 20-fold longer as compared to a dose interval for the corresponding GLP-2 that lacks the XTEN and is administered to a comparable subject using an otherwise equivalent nmoles/kg amount, the fusion protein achieves a similar blood concentration in the subject as compared to the corresponding GLP-2 that lacks the XTEN; or

(d) when the fusion protein is administered to a subject in consecutive doses to a subject using a dose interval that is at least about 3-fold, or at least 4-fold, or at least 5-fold, or at least 10-fold, or at least 15-fold, or at least 20-fold longer as compared to a dose interval for the corresponding GLP-2 that lacks the XTEN and is administered to a comparable subject using an otherwise equivalent nmoles/kg amount, the fusion protein achieves a comparable therapeutic effect in the subject as the corresponding GLP-2 that lacks the XTEN.

Item 27. The recombinant fusion protein of Item 26, wherein the subject is selected from the group consisting of mouse, rat, monkey, and human.

Item 28. The recombinant fusion protein of Item 27, wherein the subject is rat.

Item 29. The recombinant fusion protein of any one of Item 26-Item 28, wherein the administration results in a greater therapeutic effect compared to the effect seen with the corresponding GLP-2 not linked to XTEN.

Item 30. The recombinant fusion protein of any one of Item 26-Item 29, wherein administration of an effective amount the fusion protein results in a greater therapeutic effect in a subject with enteritis compared to the corresponding GLP-2 not linked to XTEN when the corresponding GLP-2 is administered to a comparable subject using a comparable nmoles/kg amount.

Item 31. The recombinant fusion protein of any one of Item 26-Item 30, wherein the subject is selected from the group consisting of mouse, rat, monkey, and human.

Item 32. The recombinant fusion protein of Item 31, wherein the subject is human and the enteritis is Crohn's disease.

Item 33. The recombinant fusion protein of Item 31, wherein the subject is rat subject and the enteritis is induced with indomethacin.

Item 34. The recombinant fusion protein of any one of Item 29-Item 33, wherein the greater therapeutic effect is selected from the group consisting of body weight gain, small intestine length, reduction in TNF$\alpha$ content of the small intestine tissue, reduced mucosal atrophy, reduced incidence of perforated ulcers, and height of villi.

Item 35. The recombinant fusion protein of Item 34, wherein the administration results in an increase in small intestine weight of at least about 10%, or at least about 20%, or at least about 30%, or at least about 40% greater compared to that of the corresponding GLP-2 not linked to XTEN.

Item 36. The recombinant fusion protein of Item 34, wherein the administration results in an increase in small intestine length of at least about 5%, or at least about 6%, or at least about 7%, or at least about 8%, or at least about 9%, or at least about 10%, or at least about 20%, or at least about 30%, or at least about 40% greater compared to that of the corresponding GLP-2 not linked to XTEN.

Item 37. The recombinant fusion protein of Item 34, wherein the administration results in an increase in body weight is at least about 5%, or at least about 6%, or at least about 7%, or at least about 8%, or at least about 9%, or at least about 10%, or at least about 20%, or at least about 30%, or at least about 40% greater compared to that of the corresponding GLP-2 not linked to XTEN.

Item 38. The recombinant fusion protein of Item 34, wherein the reduction in TNF$\alpha$ content is at least about 0.5 ng/g, or at least about 0.6 ng/g, or at least about 0.7 ng/g, or at least about 0.8 ng/g, or at least about 0.9 ng/g, or at least about 1.0 ng/g, or at least about 1.1 ng/g, or at least about 1.2 ng/g, or at least about 1.3 ng/g, or at least about 1.4 ng/g of small intestine tissue or greater compared to that of the corresponding GLP-2 not linked to XTEN.

Item 39. The recombinant fusion protein of Item 34, wherein the villi height is at least about 5%, or at least about 6%, or at least about 7%, or at least about 8%, or at least about 9%, or at least about 10%, or at least about 11%, or at least about 12% greater compared to that of the corresponding GLP-2 not linked to XTEN.

Item 40. The recombinant fusion protein of any one of Item 29-Item 39, wherein the fusion protein is administered as 1, or 2, or 3, or 4, or 5, or 6, or 10, or 12 or more consecutive doses.

Item 41. The recombinant fusion protein of any one of Item 30-Item 40, wherein the effective amount is at least about 5, or least about 10, or least about 25, or least about 100, or least about 200 nmoles/kg.

Item 42. The recombinant fusion protein of any one of the preceding items, wherein the GLP-2 is linked to the XTEN via a cleavage sequence that is cleavable by a mammalian protease selected from the group consisting of factor XIa, factor XIIa, kallikrein, factor VIIa, factor IXa, factor Xa, factor IIa (thrombin), Elastase-2, MMP-12, MMP13, MMP-17 and MMP-20, wherein cleavage at the cleavage sequence by the mammalian protease releases the GLP-2 sequence from the XTEN sequence, and wherein the released GLP-2 sequence exhibits an increase in receptor binding activity of at least about 30% compared to the uncleaved fusion protein.

Item 43. A method of producing a fusion protein comprising GLP-2 fused to one or more extended recombinant polypeptides (XTEN), comprising:

> (a) providing a host cell comprising a recombinant nucleic acid encoding the fusion protein of any one of items 1 to Item 41;
> (b) culturing the host cell under conditions permitting the expression of the fusion protein; and
> (c) recovering the fusion protein.

Item 44. The method of Item 43, wherein:

> (a) the host cell is a prokaryotic cell; or
> (b) the fusion protein is recovered from the host cell cytoplasm in substantially soluble form.

Item 45. The method of Item 43, wherein the recombinant nucleic acid molecule has a sequence with at least 90%, or at least about 91%, or at least about 92%, or at least about 93%, or at least about 94%, or at least about 95%, or at least about 96%, or at least about 97%, or at least about 98%, or at least about 99%, or about 100% sequence identity to a sequence selected from the group consisting of the DNA sequences set forth in Table 13, when optimally aligned, or the complement thereof.

Item 46. An isolated nucleic acid comprising:

> (a) a nucleic acid sequence that has at least 70%, or at least about 80%, or at least about 90%,or at least about 91%, or at least about 92%, or at least about 93%, or at least about 94%, or at least about 95%, or at least about 96%, or at least about 97%, or at least about 98%, or at least about 99%, or about 100% sequence identity to a DNA sequence selected from Table 13, or the complement thereof; or
> (b) a nucleotide sequence encoding the fusion protein of any of items 1-Item 41, or the complement thereof.

Item 47. An expression vector or isolated host cell comprising the nucleic acid of any one of Item 43-Item 46.

Item 48. A host cell comprising the expression vector of Item 47.

Item 49. A pharmaceutical composition comprising the fusion protein of 1-Item 41, and a pharmaceutically acceptable carrier.

Item 50. The recombinant fusion protein of item 1 configured according to formula V:

> (a)

$$(GLP\text{-}2)\text{-}(S)_x\text{-}(XTEN) \qquad (V)$$

wherein independently for each occurrence,

> (b) GLP-2 is a sequence having at least 90%, or at least about 91%, or at least about 92%, or at least about 93%, or at least about 94%, or at least about 95%, or at least about 96%, or at least about 97%, or at least about 98%, or at least about 99%, or about 100% sequence identity to a sequence selected from the group consisting of the sequences in Table 1, when optimally aligned;
> (c) S is a spacer sequence having between 1 to about 50 amino acid residues that can optionally include a cleavage sequence from Table 6 or amino acids compatible with restrictions sites; and

(d) x is either 0 or 1;

Item 51. The recombinant fusion protein of Item 50, wherein the GLP-2 comprises human GLP-2.

Item 52. The recombinant fusion protein of Item 50, wherein the GLP-2 is selected from the group consisting of bovine GLP-2, pig GLP-2, sheep GLP-2, chicken GLP-2, and canine GLP-2.

Item 53. The recombinant fusion protein of Item 51 or item Item 52, wherein the GLP-2 has an amino acid substitution in place of $Ala^2$, and wherein the substitution is glycine.

Item 54. The recombinant fusion protein of Item 50, wherein the GLP-2 has the sequence HGDGSFSDEMNTILDNLAARDFINWLIQTKITD.

Item 55. The recombinant fusion protein of any one of Item 50-Item 54, comprising a spacer sequence wherein the spacer sequence is a glycine residue.

Item 56. The recombinant fusion protein any one of Item 50-Item 55, wherein the XTEN has at least 90%, or at least about 91%, or at least about 92%, or at least about 93%, or at least about 94%, or at least about 95%, or at least about 96%, or at least about 97%, or at least about 98%, or at least about 99%, or 100% sequence identity when compared to a sequence of comparable length selected from any one of Table 4, Table 8, Table 9, Table 10, Table 11, and Table 12, when optimally aligned.

Item 57. The recombinant fusion protein any one of Item 50-Item 55, wherein the XTEN has at least 90%, or at least about 91%, or at least about 92%, or at least about 93%, or at least about 94%, or at least about 95%, or at least about 96%, or at least about 97%, or at least about 98%, or at least about 99%, or 100% sequence identity when compared to a AE864 sequence from Table 4, when optimally aligned.

Item 58. The pharmaceutical composition of Item 49, wherein administration of a therapeutically effective amount of the pharmaceutical composition to a subject with a gastrointestinal condition results in maintaining blood concentrations of the fusion protein within a therapeutic window for the fusion protein at least three-fold longer compared to the corresponding GLP-2 not linked to the XTEN and administered at a comparable amount to the subject.

Item 59. The pharmaceutical composition of Item 49, wherein administration of three or more doses of the pharmaceutical composition to a subject with a gastrointestinal condition using a therapeutically-effective dose regimen results in a gain in time of at least four-fold between at least two consecutive $C_{max}$ peaks and/or $C_{min}$ troughs for blood levels of the fusion protein compared to the corresponding GLP-2 not linked to the XTEN and administered using a comparable dose regimen to a subject.

Item 60. The pharmaceutical composition of Item 59 or Item 60, wherein the gastrointestinal condition is selected from the group consisting of gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy- associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, and gastrointestinal ischemia.

Item 61. The pharmaceutical composition of Item 49, wherein after intravenous, subcutaneous, or intramuscular administration of the pharmaceutical composition comprising at least about 5, or least about 10, or least about 25, or least about 100, or least about 200 nmoles/kg of the fusion protein to a subject, the fusion protein blood levels are maintained above 1000 ng/ml for at least 72 hours.

Item 62. The pharmaceutical composition of Item 61, wherein the subject is selected from mouse, rat, monkey and human.

Item 63. A recombinant fusion protein according to any one of 1-Item 41 for use in the manufacture of a medicament for the treatment of a gastrointestinal condition.

Item 64. The recombinant fusion protein of Item 63 wherein the gastrointestinal condition is selected from the group consisting of gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy- associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, ischemia, and stroke.

Item 65. A recombinant fusion protein according to any one of 1-Item 41 for use in a method of treating a gastrointestinal condition in a subject, comprising administering to the subject a therapeutically effective amount of the fusion protein.

Item 66. The recombinant fusion protein for use according to item Item 65, wherein the gastrointestinal condition is selected from the group consisting of gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy-associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, ischemia, and stroke.

Item 67. The recombinant fusion protein for use according to item Item 65, wherein administration of two or more consecutive doses of the fusion protein administered using a therapeutically effective dose regimen to a subject results in a prolonged period between consecutive $C_{max}$ peaks and/or $C_{min}$ troughs for blood levels of the fusion protein compared to the corresponding GLP-2 that lacks the XTEN and administered using a therapeutically effective dose regimen established for the GLP-2.

Item 68. The recombinant fusion protein for use according to item Item 65, wherein a smaller amount in nmoles/kg of the fusion protein is administered to a subject in comparison to the corresponding GLP-2 that lacks the XTEN administered to a subject under an otherwise equivalent dose regimen, and the fusion protein achieves a comparable therapeutic effect as the corresponding GLP-2 that lacks the XTEN.

Item 69. The recombinant fusion protein for use according to item Item 68, wherein the therapeutic effect is selected from the group consisting of blood concentrations of GLP-2, increased mesenteric blood flow, decreased inflammation, increased weight gain, decreased diarrhea, decreased fecal wet weight, intestinal wound healing, increase in plasma citrulline concentrations, decreased CRP levels, decreased requirement for steroid therapy, enhancing or stimulating mucosal integrity, decreased sodium loss, minimizing, mitigating, or preventing bacterial translocation in the intestines, enhancing, stimulating or accelerating recovery of the intestines after surgery, preventing relapses of inflammatory bowel disease, and maintaining energy homeostasis.

Item 70. A recombinant fusion protein for use in a pharmaceutical regimen for treatment of a gastrointestinal condition in a subject, said regimen comprising a pharmaceutical composition comprising the fusion protein of any one of 1- Item 41.

Item 71. The recombinant fusion protein of Item 70, wherein the pharmaceutical regimen further comprises the step of determining the amount of pharmaceutical composition needed to achieve a therapeutic effect in the subject, wherein the therapeutic effect is selected from the group consisting of increased mesenteric blood flow, decreased inflammation, increased weight gain, decreased diarrhea, decreased fecal wet weight, intestinal wound healing, increase in plasma citrulline concentrations, decreased CRP levels, decreased requirement for steroid therapy, enhanced mucosal integrity, decreased sodium loss, preventing bacterial translocation in the intestines, accelerated recovery of the intestines after surgery, prevention of relapses of inflammatory bowel disease, and maintaining energy homeostasis.

Item 72. The recombinant fusion protein of Item 70, wherein the gastrointestinal condition is selected from the group consisting of gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy- associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, ischemia, and stroke.

Item 73. The recombinant fusion protein of Item 70, wherein the pharmaceutical regimen for treating a subject with a gastrointestinal condition comprises administering the pharmaceutical composition in two or more successive doses to the subject at an effective amount, wherein the administration results in at least a 5%, or 10%, or 20%, or 30%, or 40%, or 50%, or 60%, or 70%, or 80%, or 90% greater improvement of at least one, two, or three parameters associated with the gastrointestinal condition compared to the GLP-2 not linked to XTEN and administered using a comparable nmol/kg amount.

Item 74. The recombinant fusion protein of Item 73, wherein the parameter improved is selected from increased blood concentrations of GLP-2, increased mesenteric blood flow, decreased inflammation, increased weight gain, decreased diarrhea, decreased fecal wet weight, intestinal wound healing, increase in plasma citrulline concentrations, decreased CRP levels, decreased requirement for steroid therapy, enhanced mucosal integrity, decreased sodium loss, preventing bacterial translocation in the intestines, accelerated recovery of the intestines after surgery, prevention of relapses of inflammatory bowel disease, and maintaining energy homeostasis.

Item 75. The recombinant fusion protein of Item 70, wherein the regimen comprises administering a therapeutically effective amount of the pharmaceutical composition of Item 49 once every 7, or 10, or 14, or 21, or 28 or more days.

Item 76. The recombinant fusion protein of Item 75, wherein the effective amount is at least about 5, or least about 10, or least about 25, or least about 100, or least about 200 nmoles/kg.

Item 77. The recombinant fusion protein of any one of Item 73-Item 76, wherein said administration is subcutaneous, intramuscular, or intravenous.

Item 78. A method of treating a gastrointestinal condition in a subject, comprising administering to said subject a composition comprising an effective amount of the pharmaceutical composition of Item 49.

Item 79. The method of Item 78, wherein the effective amount is at least about 5, or least about 10, or least about 25, or least about 100, or least about 200 nmoles/kg.

Item 80. The method of Item 79, wherein the fusion protein exhibits a terminal half-life of greater than about 30 hours in said subject.

Item 81. The method of any one of Item 78-Item 80, wherein the gastrointestinal condition is selected from the group consisting of gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy- associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, ischemia, and stroke.

Item 82. The method of Item 81, wherein the gastrointestinal condition is Crohn's disease.

Item 83. The method of any one of Item 78-Item 82, wherein the subject is selected from the group consisting of mouse, rat, monkey, and human.

Item 84. The method of any one of Item 78-Item 83, wherein said administration is subcutaneous, intramuscular, or intravenous.

Item 85. The method of any one of Item 78-Item 84, wherein said administration results in an intestinotrophic effect in said subject.

Item 86. The method of Item 85, wherein the intestinotrophic effect is at least about 30%, or at least about 40%, or at least about 50%, or at least about 60%, or at least about 70%, or at least about 80%, or at least about 90%, or at least about 100% or at least about 120% or at least about 150% or at least about 200% of the intestinotrophic effect compared to the corresponding GLP-2 not linked to XTEN and administered to a subject using a comparable dose.

Item 87. The method of Item 85 or Item 86, wherein the intestinotrophic effect is determined after administration of 1 dose, or 3 doses, or 6 doses, or 10 doses, or 12 or more doses of the fusion protein.

Item 88. The method of any one of Item 85-Item 87, wherein the intestinotrophic effect is selected from the group consisting of intestinal growth, increased hyperplasia of the villus epithelium, increased crypt cell proliferation, increased height of the crypt and villus axis, increased healing after intestinal anastomosis, increased small bowel weight, increased small bowel length, decreased small bowel epithelium apoptosis, and enhancement of intestinal function.

It is specifically contemplated that the recombinant GLP2-XTEN fusion proteins can exhibit one or more or any combination of the properties disclosed herein.

## INCORPORATION BY REFERENCE

All publications, patents, and patent applications mentioned in this specification are herein incorporated by reference to the same extent as if each individual publication, patent, or patent application was specifically and individually indicated to be incorporated by reference.

## BRIEF DESCRIPTION OF THE DRAWINGS

The features and advantages of the invention may be further explained by reference to the following detailed description and accompanying drawings that sets forth illustrative embodiments.

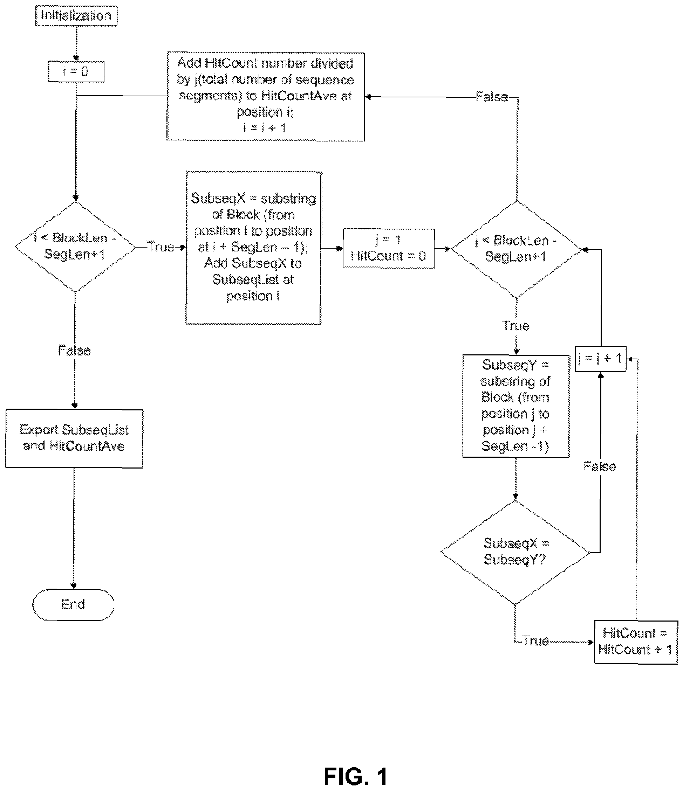

FIG. 1 is a schematic of the logic flow chart of the algorithm SegScore. In the figure the following legend applies: i, j - counters used in the control loops that run through the entire sequence; HitCount- this variable is a counter that keeps track of how many times a subsequence encounters an identical subsequence in a block; SubSeqX - this variable holds the subsequence that is being checked for redundancy; SubSeqY - this variable holds the subsequence that the SubSeqX is checked against; BlockLen - this variable holds the user determined length of the block; SegLen - this variable holds the length of a segment. The program is hardcoded to generate scores for subsequences of lengths 3, 4, 5, 6, 7, 8, 9, and 10; Block - this variable holds a string of length BlockLen. The string is composed of letters from an input XTEN sequence and is determined by the position of the i counter; SubSeqList - this is a list that holds all of the generated subsequence scores.

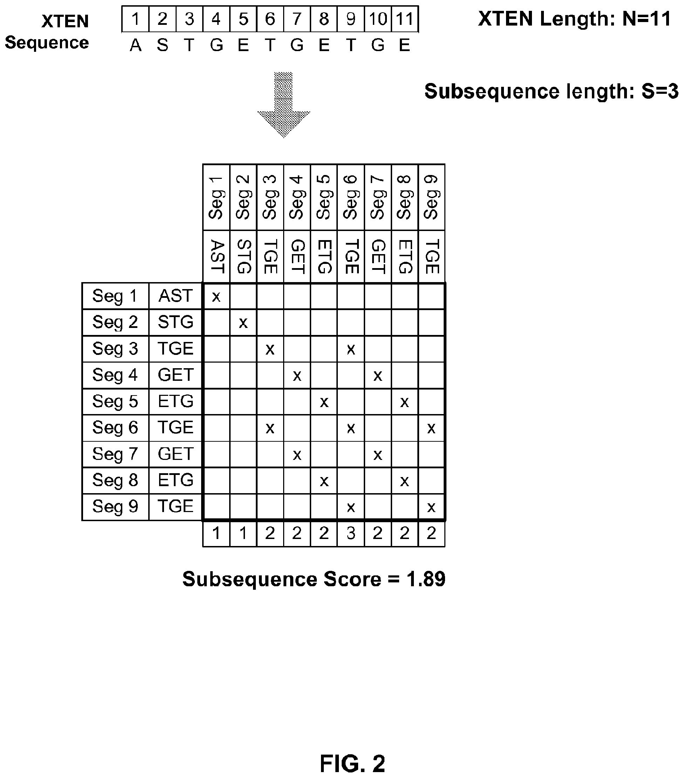

FIG. 2 depicts the application of the algorithm SegScore to a hypothetical XTEN of 11 amino acids in order to determine the repetitiveness. An XTEN sequence consisting of N amino acids is divided into N-S+1 subsequences of length S (S=3 in this case). A pair-wise comparison of all subsequences is performed and the average number of identical subsequences is calculated to result, in this case, in a subsequence score of 1.89.

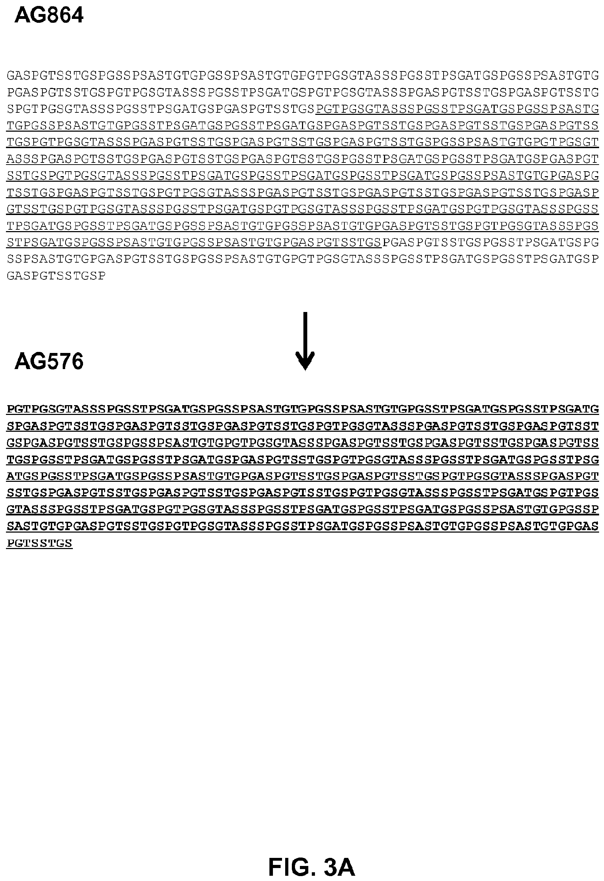

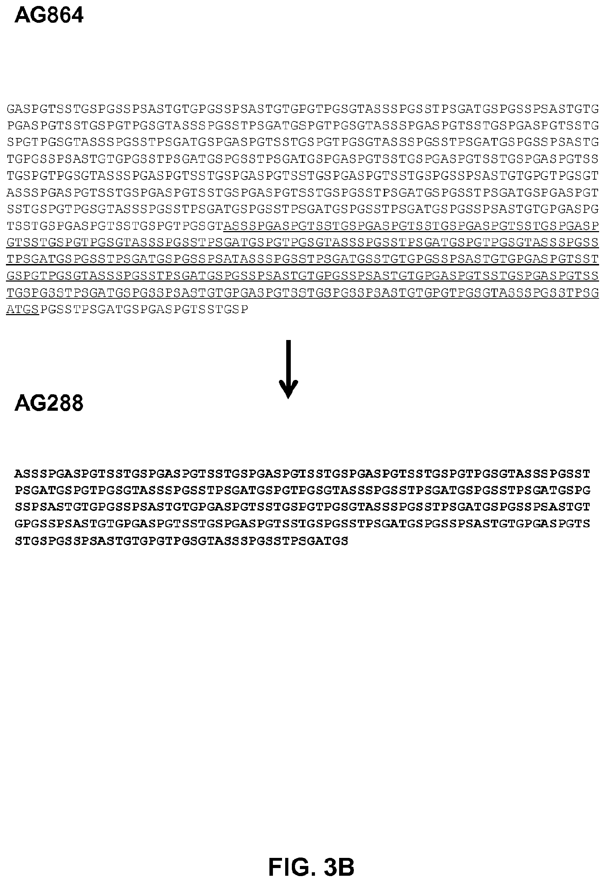

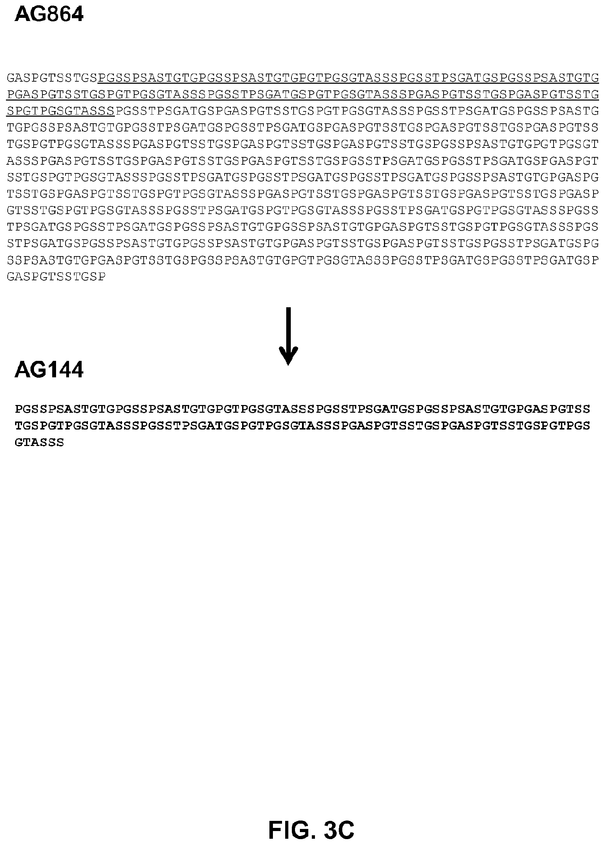

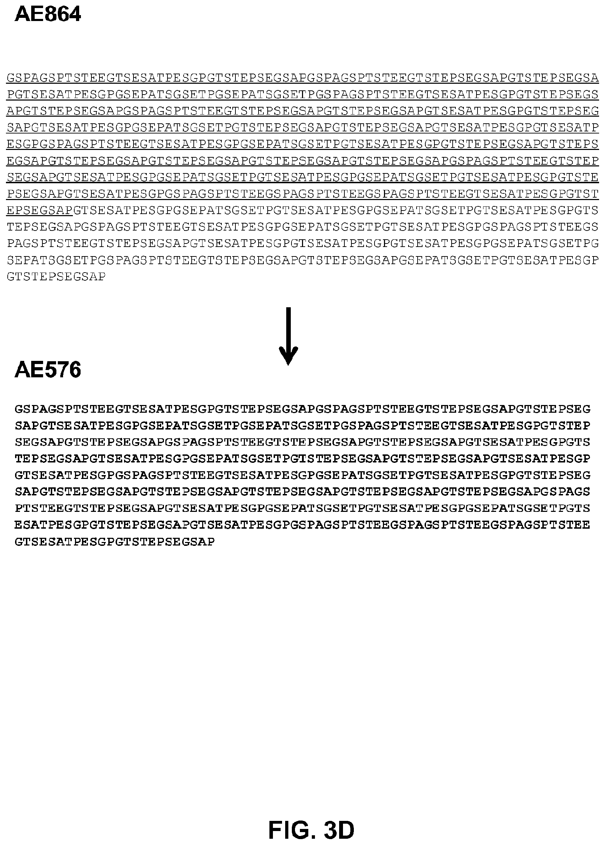

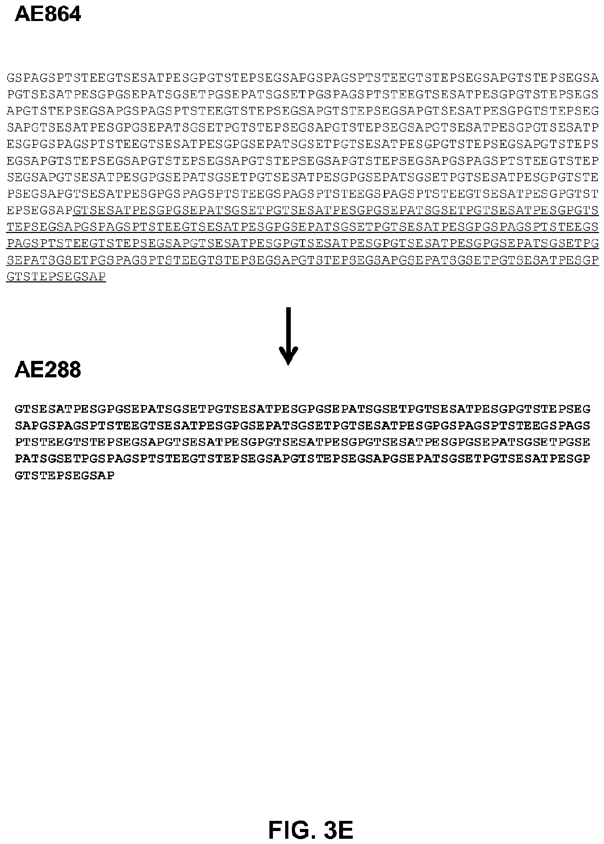

FIG. 3 illustrates the use of donor XTEN sequences to produce truncated XTEN sequences. FIG. 3A provides the sequence of AG864, with the underlined sequence used to generate an AG576 sequence. FIG. 3B provides the sequence of AG864, with the underlined sequence used to generate an AG288 sequence. FIG. 3C provides the sequence of AG864, with the underlined sequence used to generate an AG144 sequence. FIG. 3D provides the sequence of AE864, with the underlined sequence used to generate an AE576 sequence. FIG. 3E provides the sequence of AE864, with the underlined sequence used to generate an AE288 sequence.

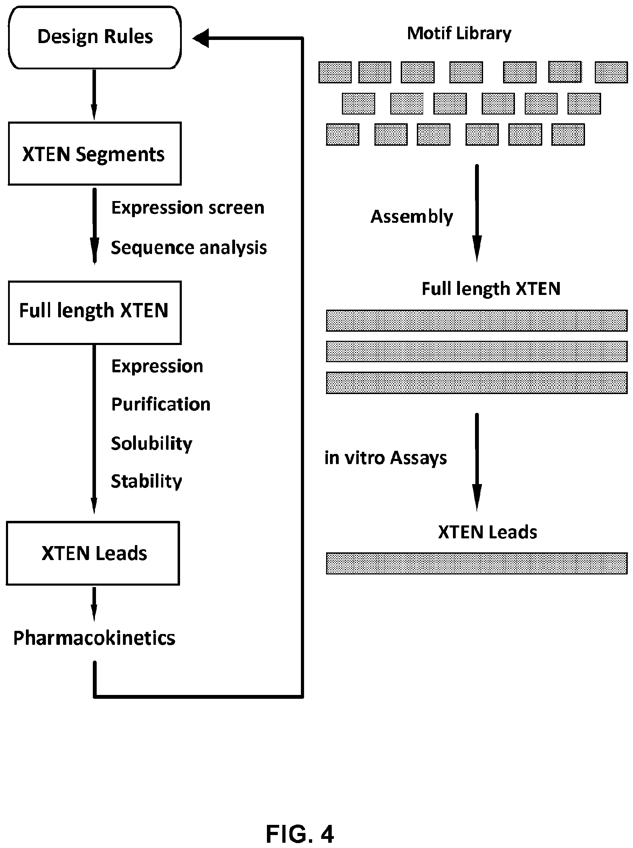

FIG. 4 is a schematic flowchart of representative steps in the assembly, production and the evaluation of an XTEN.

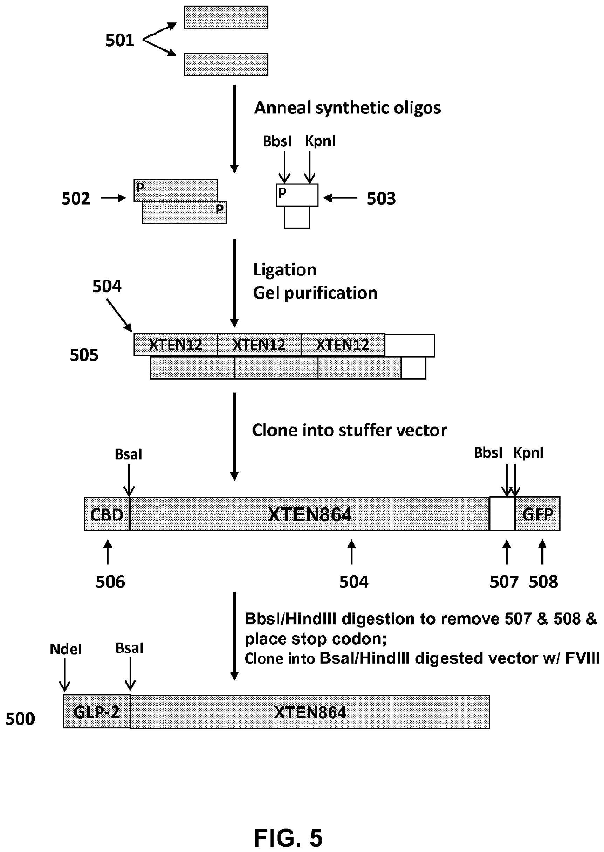

FIG. 5 is a schematic flowchart of representative steps in the assembly of a GLP2-XTEN polynucleotide construct encoding a fusion protein. Individual oligonucleotides 501 are annealed into sequence motifs 502 such as a 12 amino acid motif ("12-mer"), which is ligated to additional sequence motifs from a library to create a pool that encompasses the desired length of the XTEN 504, as well as ligated to a smaller concentration of an oligo containing BbsI, and KpnI restriction sites 503. The resulting pool of ligation products is gel-purified and the band with the desired length of XTEN is cut, resulting in an isolated XTEN gene with a stopper sequence 505. The XTEN gene is cloned into a stuffer vector. In this case, the vector encodes an optional CBD sequence 506 and a GFP gene 508. Digestion is then performed with BbsI/HindIII to remove 507 and 508 and place the stop codon. The resulting product is then cloned into a BsaI/HindIII digested vector containing a gene encoding the GLP-2, resulting in gene 500 encoding a GLP2-XTEN fusion protein.

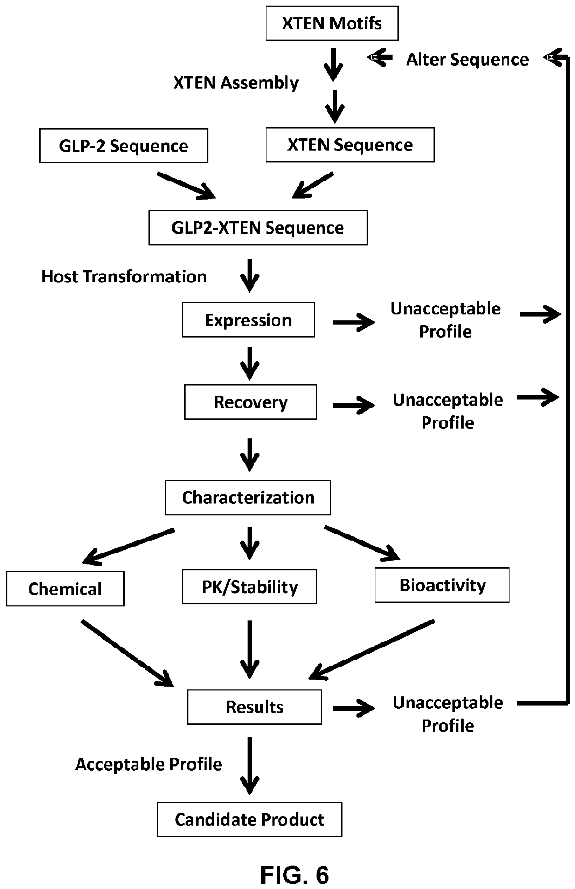

FIG. 6 is a schematic flowchart of representative steps in the assembly of a gene encoding fusion protein comprising a GLP-2 and XTEN, its expression and recovery as a fusion protein, and its evaluation as a candidate GLP2-XTEN product.

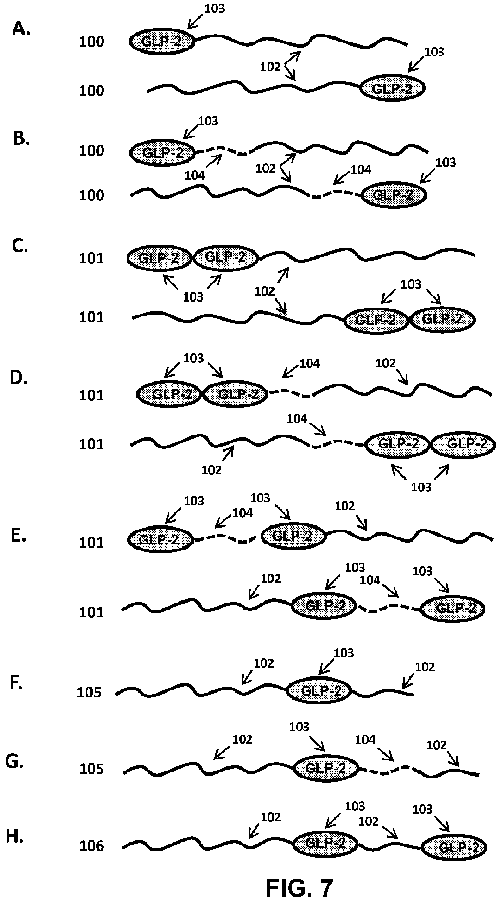

FIG. 7 shows schematic representations of exemplary GLP2-XTEN fusion proteins (FIGS. 7A-H), all depicted in an N- to C-terminus orientation. FIG. 7A shows two different configurations of GLP2-XTEN fusion proteins (100), each comprising a single GLP-2 and an XTEN, the first of which has an XTEN molecule (102) attached to the C-terminus of a GLP-2 (103), and the second of which has an XTEN molecule attached to the N-terminus of a GLP-2 (103).

FIG. 7B shows two different configurations of GLP2-XTEN fusion proteins (100), each comprising a single GLP-2, a spacer sequence and an XTEN, the first of which has an XTEN molecule (102) attached to the C-terminus of a spacer sequence (104) and the spacer sequence attached to the C-terminus of a GLP-2 (103) and the second of which has an XTEN molecule attached to the N-terminus of a spacer sequence (104) and the spacer sequence attached to the N-terminus of a GLP-2 (103). FIG. 7C shows two different configurations of GLP2-XTEN fusion proteins (101), each comprising two molecules of a single GLP-2 and one molecule of an XTEN, the first of which has an XTEN linked to the C-terminus of a first GLP-2 and that GLP-2 is linked to the C-terminus of a second GLP-2, and the second of which is in the opposite orientation in which the XTEN is linked to the N-terminus of a first GLP-2 and that GLP-2 is linked to the N-terminus of a second GLP-2. FIG. 7D shows two different configurations of GLP2-XTEN fusion proteins (101), each comprising two molecules of a single GLP-2, a spacer sequence and one molecule of an XTEN, the first of which has an XTEN linked to the C-terminus of a spacer sequence and the spacer sequence linked to the C-terminus of a first GLP-2 which is linked to the C-terminus of a second GLP-2, and the second of which is in the opposite orientation in which the XTEN is linked to the N-terminus of a spacer sequence and the spacer sequence is linked to the N-terminus of a first GLP-2 that that GLP-2 is linked to the N-terminus of a second GLP-2. FIG. 7E shows two different configurations of GLP2-XTEN fusion proteins (101), each comprising two molecules of a single GLP-2, a spacer sequence and one molecule of an XTEN, the first of which has an XTEN linked to the C-terminus of a first GLP-2 and the first GLP-2 linked to the C-terminus of a spacer sequence which is linked to the C-terminus of a second GLP-2 molecule, and the second of which is in the opposite configuration of XTEN linked to the N-terminus of a first GLP-2 which is linked to the N-terminus of a spacer sequence which in turn is linked to the N-terminus of a second molecule of GLP-2. FIG. 7F shows a configuration of GLP2-XTEN fusion protein (105), each comprising one molecule of GLP-2 and two molecules of an XTEN linked to the N-terminus and the C-terminus of the GLP-2. FIG. 7G shows a configuration (106) of a single GLP-2 linked to two XTEN, with the second XTEN separated from the GLP-2 by a spacer sequence. FIG. 7H shows a configuration (106) of a two GLP-2 linked to two XTEN, with the second XTEN linked to the C-terminus of the first GLP-2 and the N-terminus of the second GLP-2, which is at the C-terminus of the GLP2-XTEN.

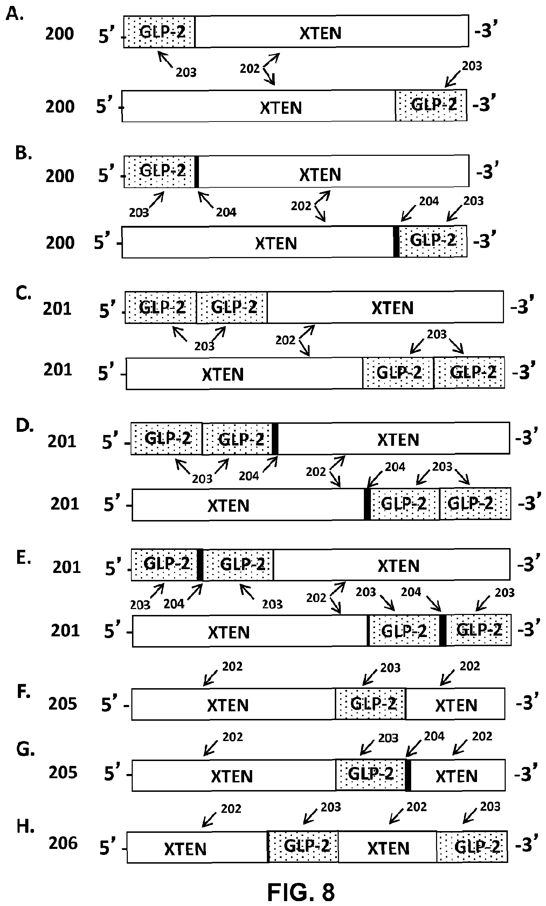

FIG. 8 is a schematic illustration of exemplary polynucleotide constructs (FIGS. 8A-H) of GLP2-XTEN genes that encode the corresponding GLP2-XTEN polypeptides of FIG. 7; all depicted in a 5' to 3' orientation. In these illustrative examples the genes encode GLP2-XTEN fusion proteins with one GLP-2 and XTEN (200); or one GLP-2, one spacer sequence and one XTEN (200); two GLP-2 and one XTEN (201); or two GLP-2, a spacer sequence and one XTEN (201); one GLP-2 and two XTEN (205); or two GLP-2 and two XTEN (206). In these depictions, the polynucleotides encode the following components: XTEN (202), GLP-2 (203), and spacer amino acids that can include a cleavage sequence (204), with all sequences linked in frame.

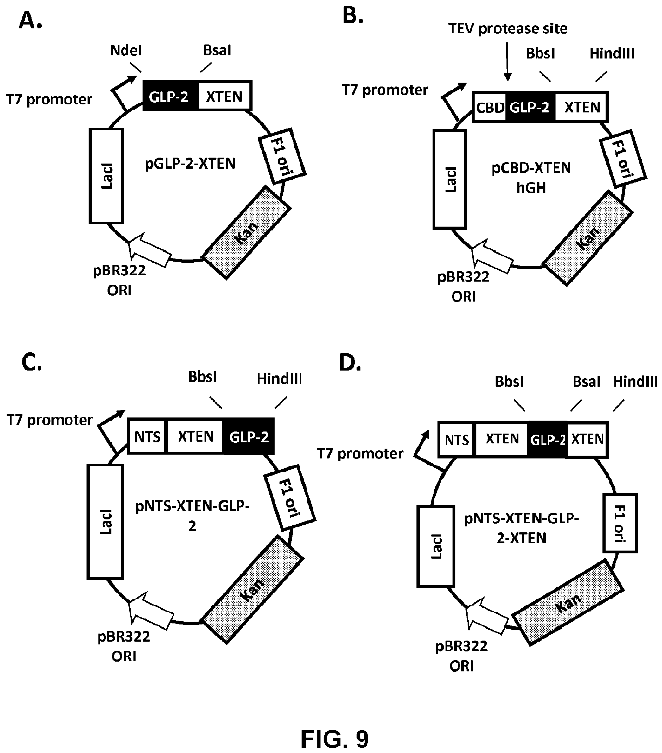

FIG. 9 is a schematic representation of the design of GLP2-XTEN expression vectors with different processing strategies. FIG. 9A shows an exemplary expression vector encoding XTEN fused to the 3' end of the sequence encoding GLP-2. Note that no additional leader sequences are required in this vector. FIG. 9B depicts an expression vector encoding XTEN fused to the 3' end of the sequence encoding GLP-2 with a CBD leader sequence and a TEV protease site. FIG. 9C depicts an expression vector where the CBD and TEV processing site have been replaced with an optimized N-terminal leader sequence (NTS). FIG. 9D depicts an expression vector encoding an NTS sequence, an XTEN, a sequence encoding GLP-2, and then a second sequence encoding an XTEN.

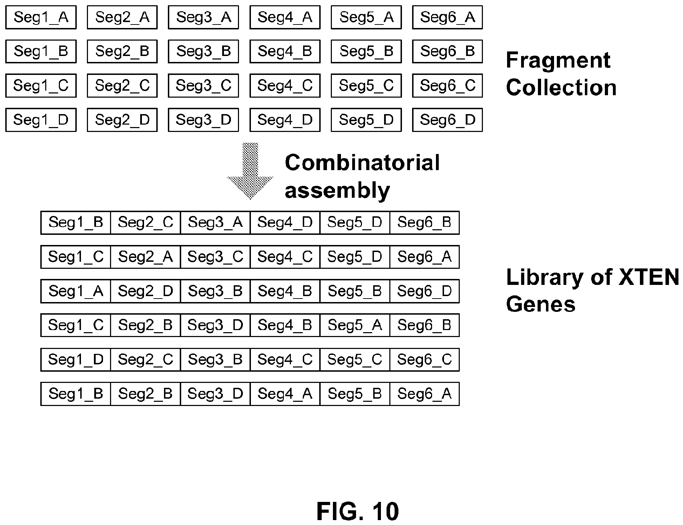

FIG. 10 illustrates the process of combinatorial gene assembly of genes encoding XTEN. In this case, the genes are assembled from 6 base fragments and each fragment is available in 4 different codon versions (A, B, C and D). This allows for a theoretical diversity of 4096 in the assembly of a 12 amino acid motif.

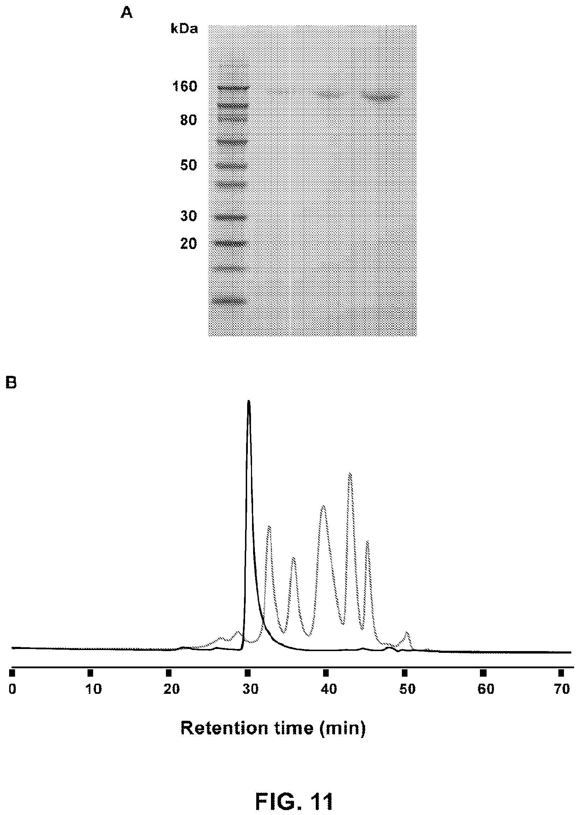

FIG. 11 shows characteriation data of the fusion protein GLP2-2G_AE864. FIG. 11A is an SDS-PAGE gel of GLP2-2G-XTEN_AE864 lot AP690, as described in Example 16. The gels show lanes of molecular weight standards and 2 or 10 μg of reference standard, as indicated. FIG. 11B shows results of a size exclusion chromatography analysis of GLP2-2G-XTEN_AE864 lot AP690, as described in Example 16, compared to molecular weight standards of 667, 167, 44, 17, and 3.5 kDa.

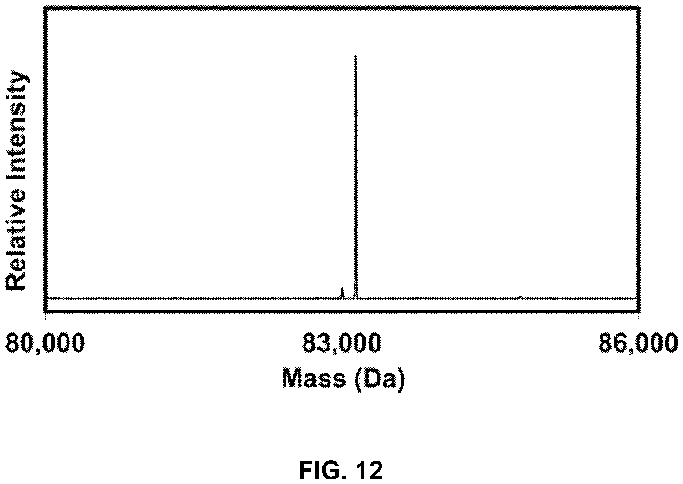

FIG. 12 shows the ESI-MS analysis of GLP2-2G-XTEN_AE864 lot AP690, as described in Example 16, with a major peak at 83,142 Da, indicating full length intact GLP2-2G-XTEN, with an additional minor peak of 83,003 Da detected, representing the des-His GLP2-2G-XTEN at <5% of total protein.

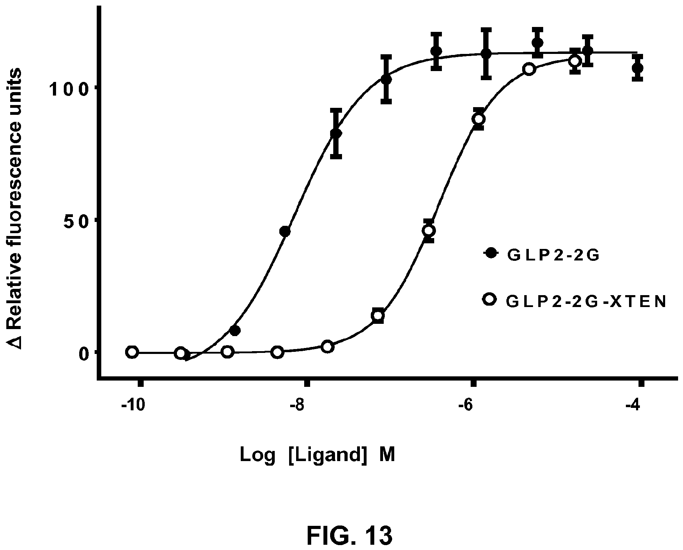

FIG. 13 shows results of the GLP-2 receptor binding assay, as described in Example 17.

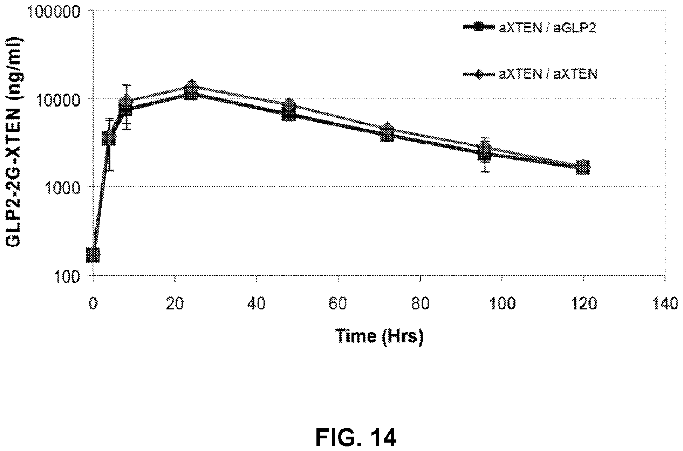

FIG. 14 shows the results of the pharmacokinetics of GLP2-2G-XTEN_AE864 in C57B1/6 mice following subcutaneous (SC) administration. The samples were analyzed for fusion protein concentration, performed by both anti-XTEN/anti- XTEN sandwich ELISA and anti-GLP2/anti-XTEN sandwich ELISA, as described in Example 18, with results for both assays plotted.

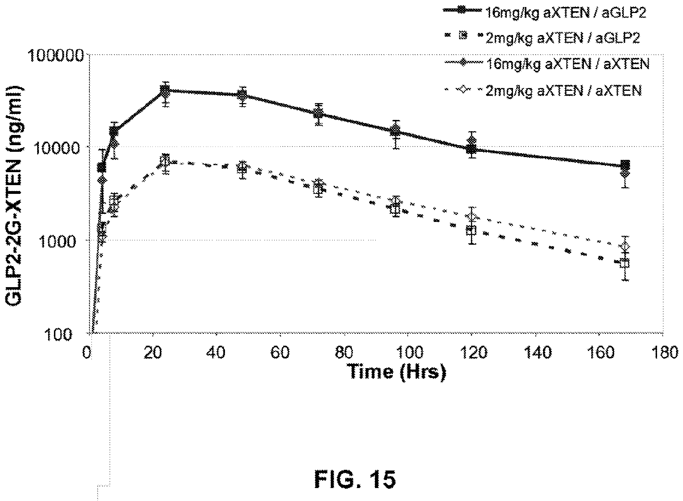

FIG. 15 shows the results of the pharmacokinetics of GLP2-2G-XTEN_AE864 in Wistar rats following SC administration of two different dosage levels, performed by both anti-XTEN/anti- XTEN sandwich ELISA and anti-GLP2/anti-XTEN sandwich ELISA, as described in Example 19, with results for both assays plotted.

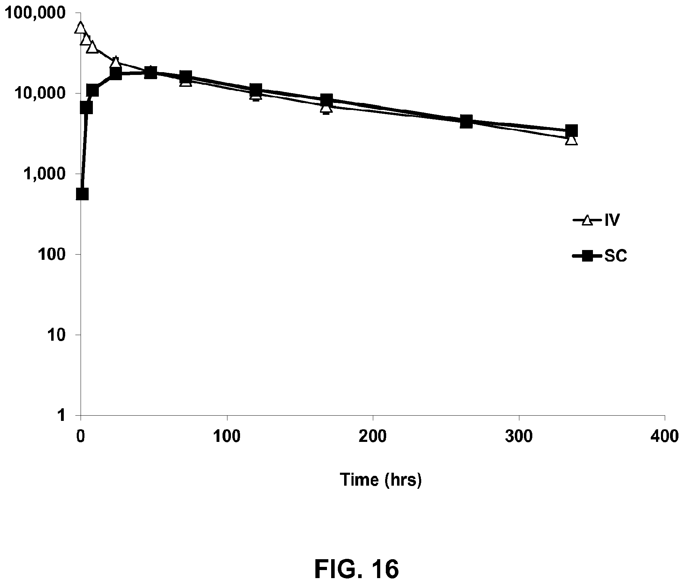

FIG. 16 shows the results of the pharmacokinetics of GLP2-2G-XTEN_AE864 in male cynomolgus monkeys following either subcutaneous (squares) or intravenous (triangles) administration of the fusion protein at a single dosage level (2 mg/kg). The samples were analyzed for fusion protein concentration, performed by anti-GLP2/anti-XTEN ELISA, as described in Example 20.

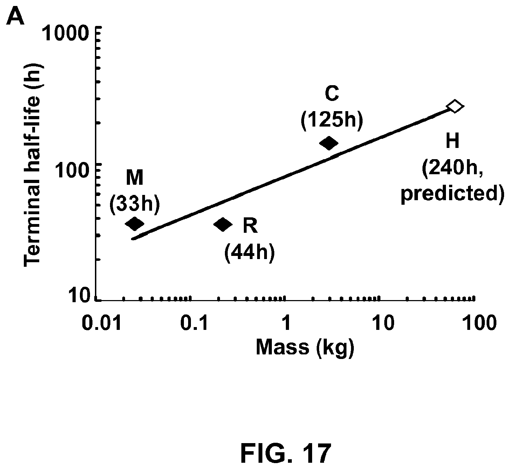

FIG. 17 shows the linear regression of the allometric scaling of GLP2-2G-XTEN half-life from three species used to predict a projected half-life of 240 hours in humans, as described in Example 20.

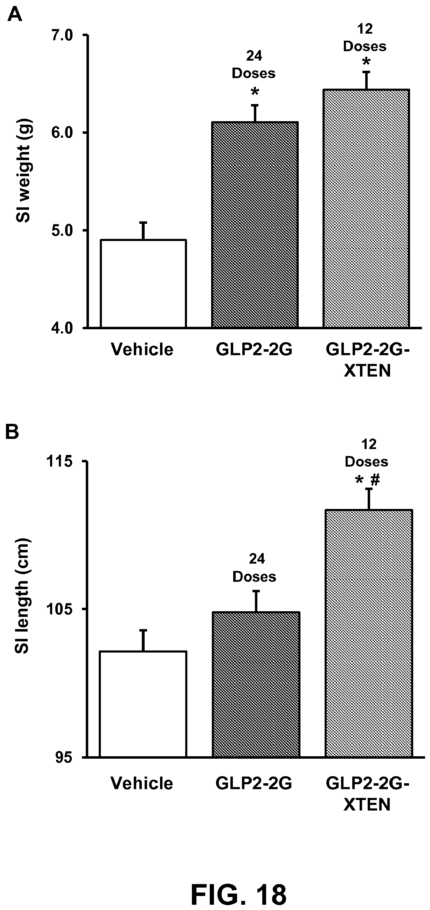

FIG. 18 shows the results in rat small intestine weight and length from vehicle and treatment groups, as described in Example 21.

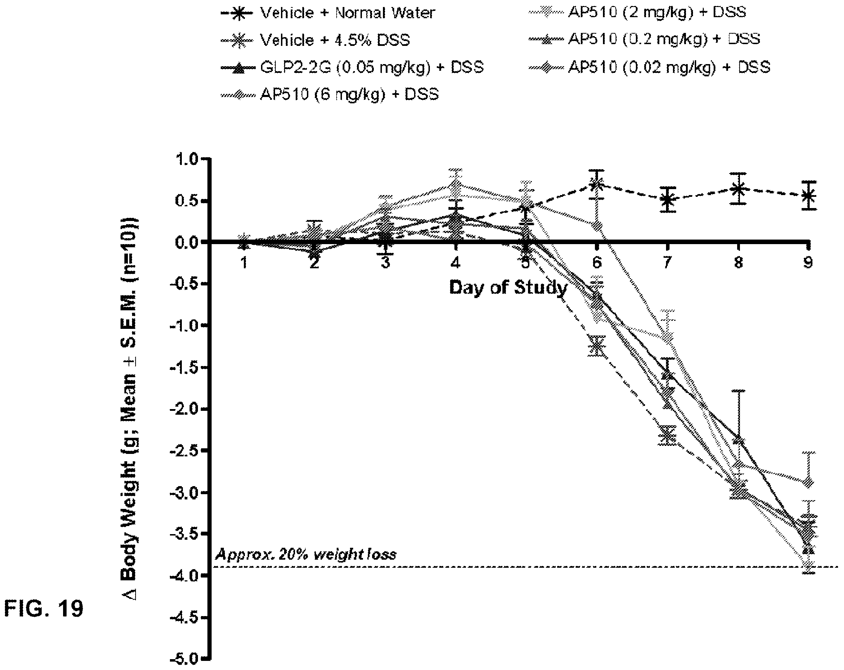

FIG. 19 shows the results of changes in body weight in a murine dextran sodium sulfate (DSS) model, with groups treated with vehicle, GLP2-2G peptide (no XTEN) or GLP2-2G-XTEN, as described in Example 21.

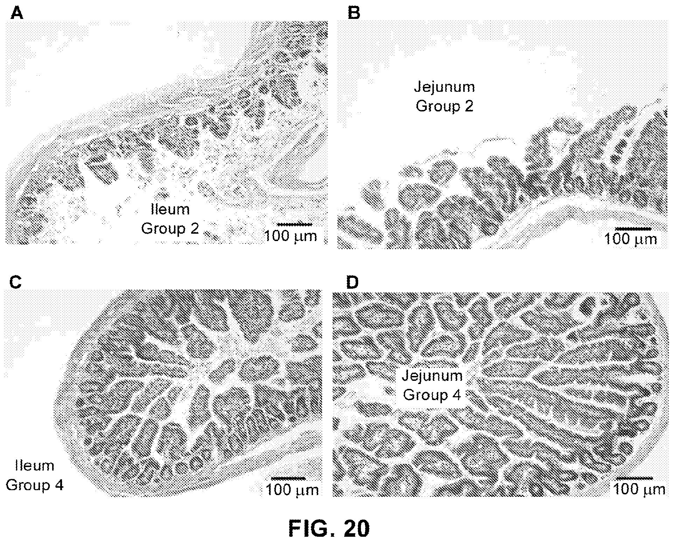

FIG. 20 shows representative histopathology sections of the DSS model mice from vehicle ileum (FIG. 20A) and jejunum (FIG. 20B) and GLP2-2G-XTEN ileum (FIG. 20C) and jejunum (FIG. 20D), as described in Example 21.

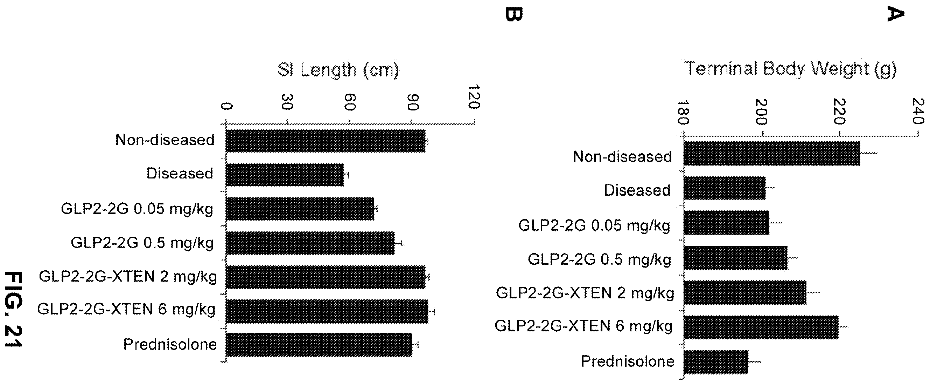

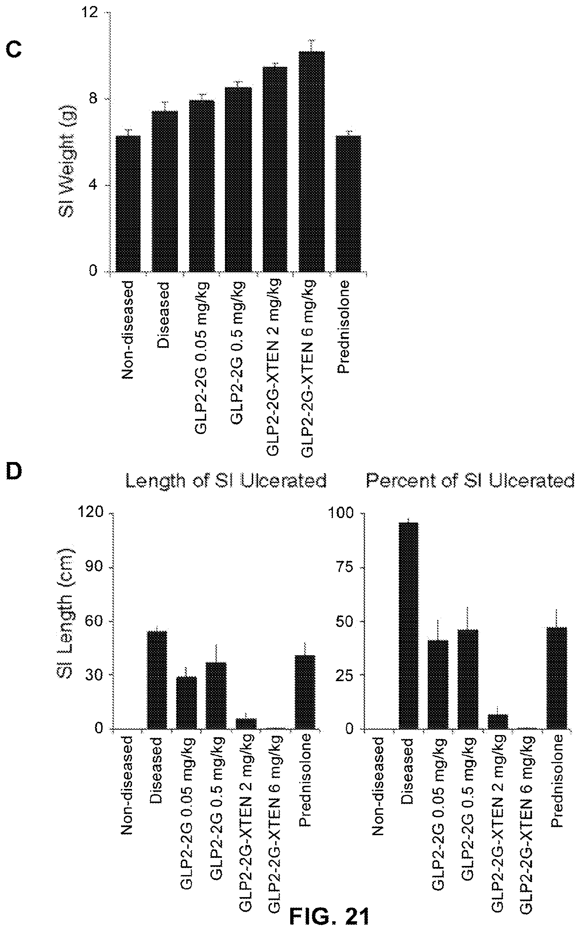

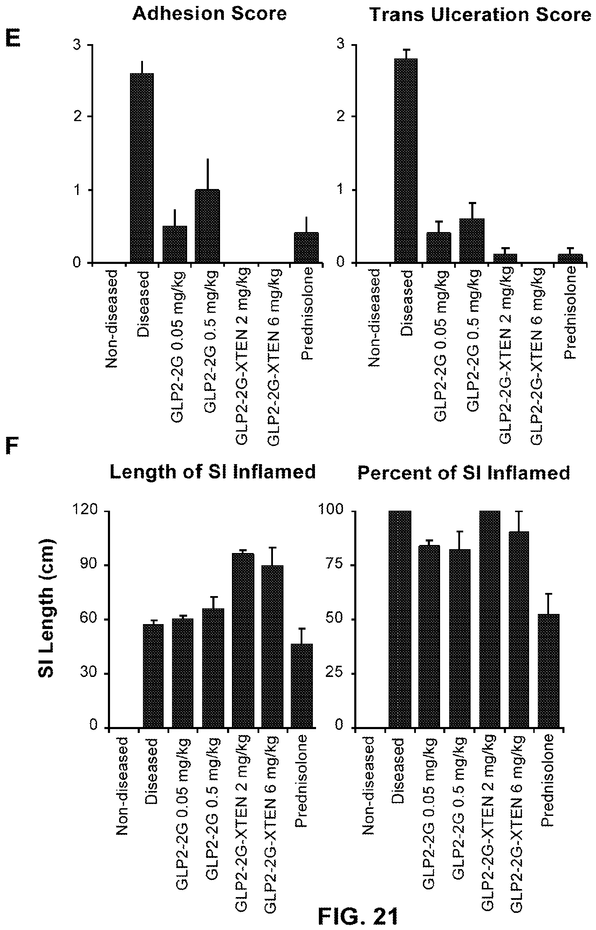

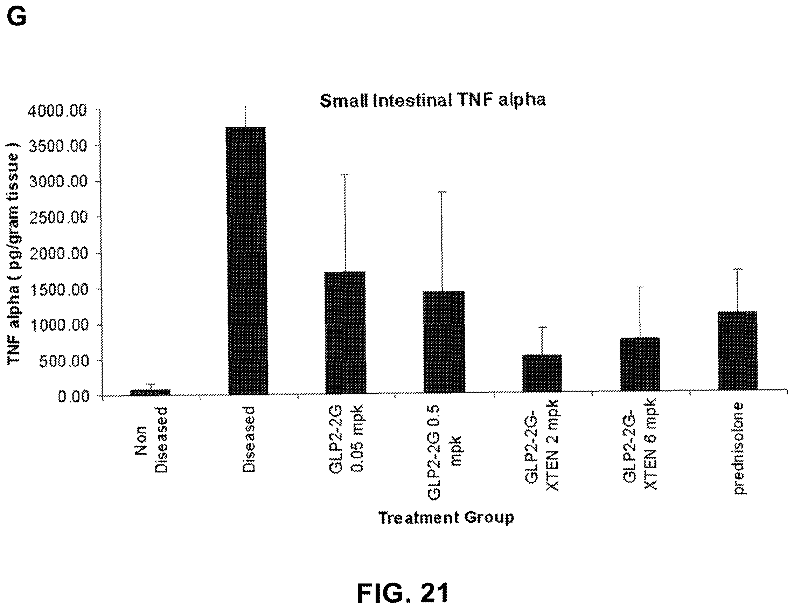

FIG. 21 shows results from Study 1 of a rat model of Crohn's Disease of indomethacin-induced intestinal inflammation, with groups treated with vehicle, GLP2-2G peptide (no XTEN) or GLP2-2G-XTEN and assayed, as described in Example 21. FIG. 21A shows results of the body weight at the termination of the experiment. FIG. 21B shows results of the length of the small intestines from each group. FIG. 21C shows results of the weight of the small intestines from each group. FIG. 21D shows results of the length of ulcerations and the percentage of ulceration in the small intestines from each group. FIG. 21E shows results of the scores of adhesions and transulceration in the small intestines from each group. FIG. 21F shows results of the length and percentage of inflammation of the small intestines from each group. FIG. 21G shows results of the TNF$\alpha$ assay of the small intestines from each group.

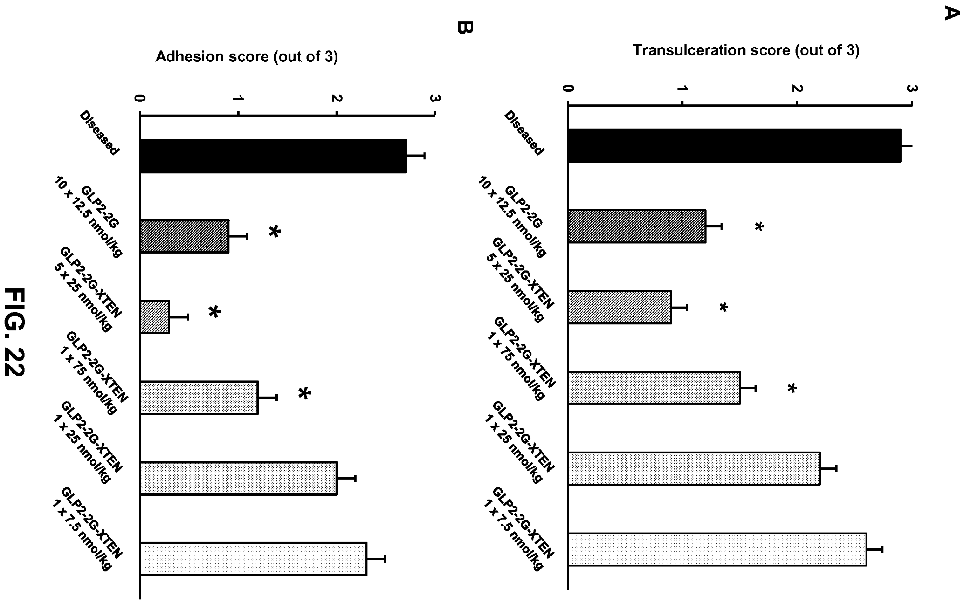

FIG. 22 shows results from Study 2 of a rat model of Crohn's Disease of indomethacin-induced intestinal inflammation, with groups treated with vehicle, GLP2-2G peptide (no XTEN) or GLP2-2G-XTEN and assayed, as described in Example 21. FIG. 22A shows the Trans-Ulceration Score of the small intestines from each group. FIG. 22B shows the Adhesion Score of the small intestines from each group.

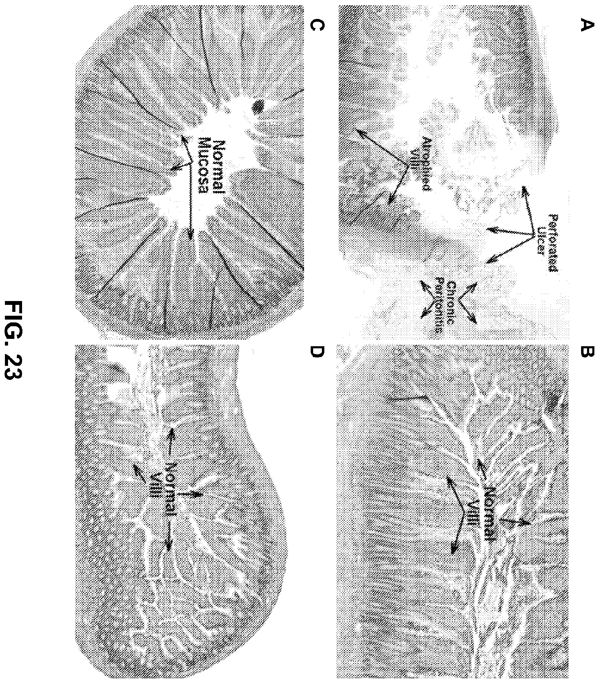

FIG. 23 shows representative histopathology sections from Study 2 of the rat model of Crohn's Disease of indomethacin-induced intestinal inflammation from vehicle-no indomethicin (FIG. 23A), vehicle-indomethicin (FIG. 23B) and GLP2-2G-XTEN treatment groups (FIGS. 22C, D), as described in Example 21.

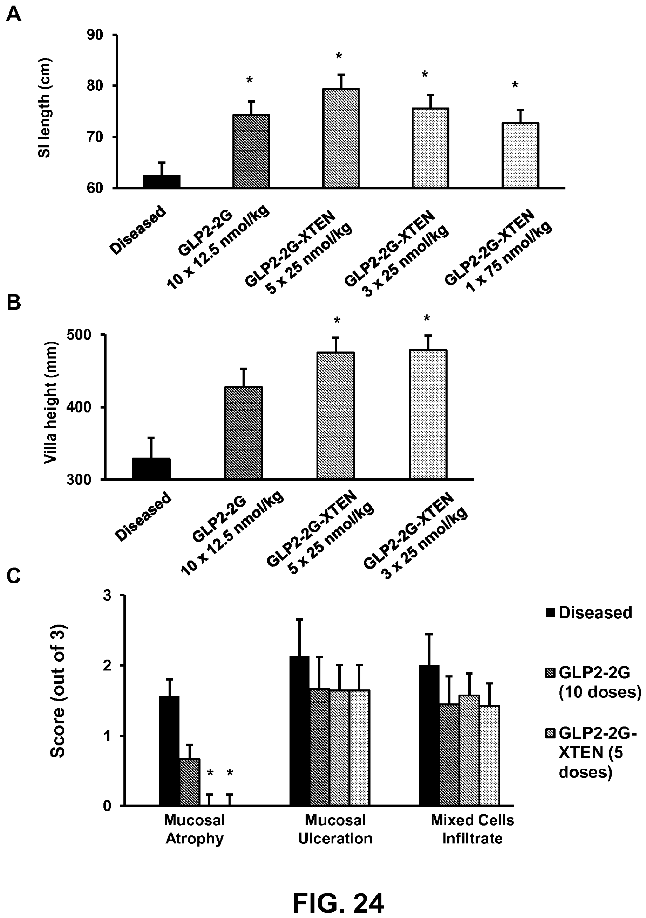

FIG. 24 shows the results of small intestine length (FIG. 24A), villi height (FIG. 24B) and histopathology scoring (FIG. 24C) of mucosal atrophy, ulceration, infiltration measurements from diseased, vehicle-treated, GLP2-2G peptide-treated, and GLP2-2G-XTEN-treated rats, as described in Example 21. Asterisks indicate groups with statistically significant differences from vehicle (diseased) control group.

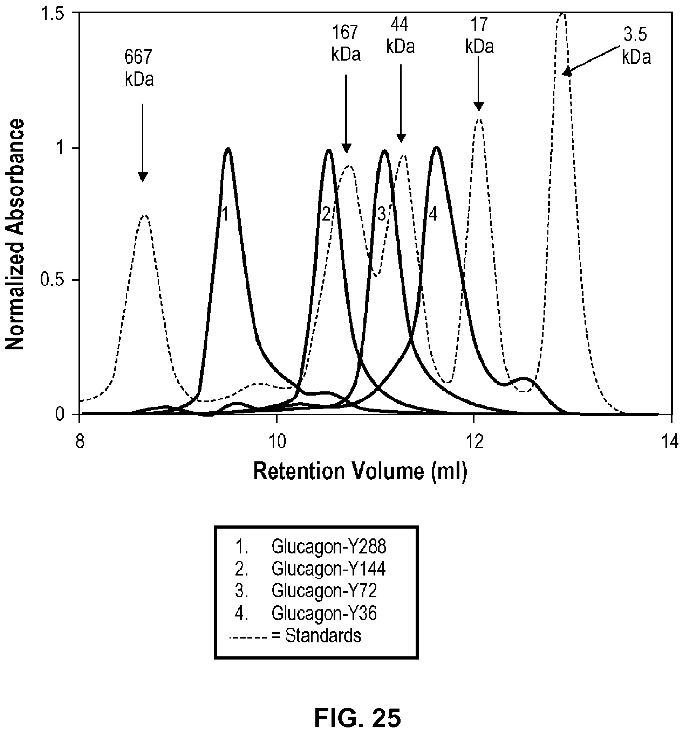

FIG. 25 shows results of a size exclusion chromatography analysis of glucagon-XTEN construct samples measured against protein standards of known molecular weight (as indicated), with the graph output as absorbance versus retention volume, as described in Example 25. The glucagon-XTEN constructs are 1) glucagon-Y288; 2) glucagonY-144; 3) glucagon-Y72; and 4) glucagon-Y36. The results indicate an increase in apparent molecular weight with increasing length of XTEN moiety.

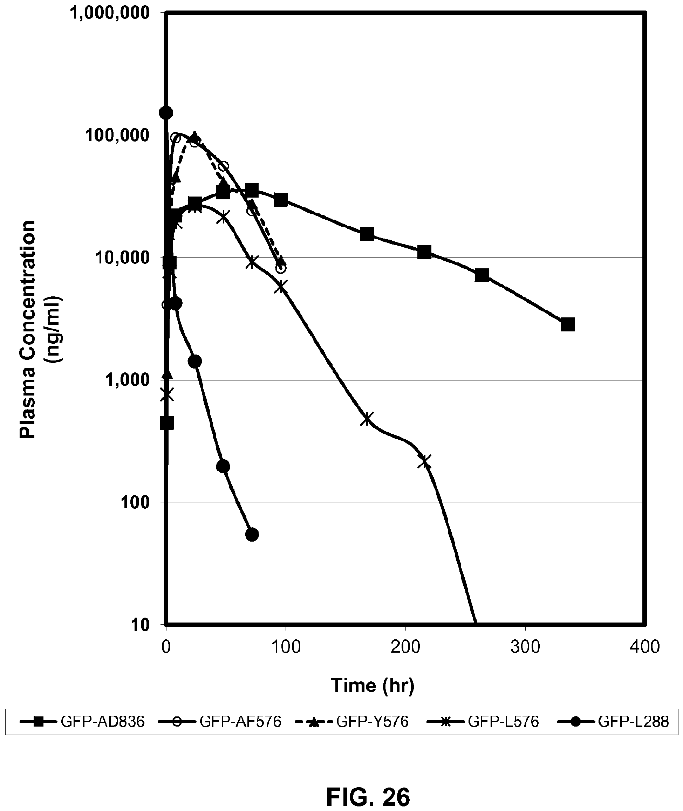

FIG. 26 shows the pharmacokinetic profile (plasma concentrations) in cynomolgus monkeys after single doses of different compositions of GFP linked to unstructured polypeptides of varying length, administered either subcutaneously or intravenously, as described in Example 26. The compositions were GFP-L288, GFP-L576, GFP-XTEN AF576, GFP-Y576 and XTEN_AD836-GFP. Blood samples were analyzed at various times after injection and the concentration of GFP in plasma was measured by ELISA using a polyclonal antibody against GFP for capture and a biotinylated preparation of the same polyclonal antibody for detection. Results are presented as the plasma concentration versus time (h) after dosing and show, in particular, a considerable increase in half-life for the XTEN_AD836-GFP, the composition with the longest sequence length of XTEN. The construct with the shortest sequence length, the GFP-L288 had the shortest half-life.

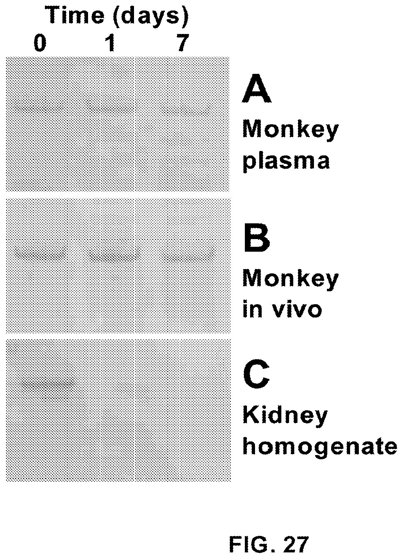

FIG. 27 shows an SDS-PAGE gel of samples from a stability study of the fusion protein of XTEN_AE864 fused to the N-terminus of GFP (see Example 27). The GFP-XTEN was incubated in cynomolgus plasma and rat kidney lysate for up to 7 days at 37°C. In addition, GFP-XTEN administered to cynomolgus monkeys was also assessed. Samples were withdrawn at 0, 1 and 7 days and analyzed by SDS PAGE followed by detection using Western analysis with antibodies against GFP.

FIG. 28 shows the amino acid sequence of GLP2-2G_AE864.

## DETAILED DESCRIPTION OF THE INVENTION

Before the embodiments of the invention are described, it is to be understood that such embodiments are provided by way of example only, and that various alternatives to the embodiments of the invention described herein may be employed in practicing the invention. Numerous variations, changes, and substitutions will now occur to those skilled in the art without departing from the invention.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting. Numerous variations, changes, and substitutions will now occur to those skilled in the art without departing from the invention.

**DEFINITIONS**

In the context of the present application, the following terms have the meanings ascribed to them unless specified otherwise:

As used in the specification and claims, the singular forms "a", "an" and "the" include plural references unless the context clearly dictates otherwise. For example, the term "a cell" includes a plurality of cells, including mixtures thereof.

The terms "polypeptide", "peptide", and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified, for example, by disulfide bond formation, glycosylation, lipidation, acetylation, phosphorylation, or any other manipulation, such as conjugation with a labeling component.

As used herein, the term "amino acid" refers to either natural and/or unnatural or synthetic amino acids, including but not limited to both the D or L optical isomers, and amino acid analogs and peptidomimetics. Standard single or three letter codes are used to designate amino acids.

The term "natural L-amino acid" means the L optical isomer forms of glycine (G), proline (P), alanine (A), valine (V), leucine (L), isoleucine (I), methionine (M), cysteine (C), phenylalanine (F), tyrosine (Y), tryptophan (W), histidine (H), lysine (K), arginine (R), glutamine (Q), asparagine (N), glutamic acid (E), aspartic acid (D), serine (S), and threonine (T).

The term "non-naturally occurring," as applied to sequences and as used herein, means polypeptide or polynucleotide sequences that do not have a counterpart to, are not complementary to, or do not have a high degree of homology with a wild-type or naturally-occurring sequence found in a mammal. For example, a non-naturally occurring polypeptide or fragment may share no more than 99%, 98%, 95%, 90%, 80%, 70%, 60%, 50% or even less amino acid sequence identity as compared to a natural sequence when suitably aligned.

The terms "hydrophilic" and "hydrophobic" refer to the degree of affinity that a substance has with water. A hydrophilic substance has a strong affinity for water, tending to dissolve in, mix with, or be wetted by water, while a hydrophobic substance substantially lacks affinity for water, tending to repel and not absorb water and tending not to dissolve in or mix with or be wetted by water. Amino acids can be characterized based on their hydrophobicity. A number of scales have been developed. An example is a scale developed by Levitt, M, et al., J Mol Biol (1976) 104:59, which is listed in Hopp, TP, et al., Proc Natl Acad Sci U S A (1981) 78:3824. Examples of "hydrophilic amino acids" are arginine, lysine, threonine, alanine, asparagine, and glutamine. Of particular interest are the hydrophilic amino acids aspartate, glutamate, and serine, and glycine. Examples of "hydrophobic amino acids" are tryptophan, tyrosine, phenylalanine, methionine, leucine, isoleucine, and valine.

A "fragment" when applied to a protein, is a truncated form of a native biologically active protein that retains at least a portion of the therapeutic and/or biological activity. A "variant" when applied to a protein, is a protein with sequence homology to the native biologically active protein that retains at least a portion of the therapeutic and/or biological activity of the biologically active protein. For example, a variant protein may share at least 70%, 75%, 80%, 85%, 90%, 95%, 96%, 97%, 98% or 99% amino acid sequence identity compared with the reference biologically active protein. As used herein, the term "biologically active protein moiety" includes proteins modified deliberately, as for example, by site directed mutagenesis, synthesis of the encoding gene, insertions, or accidentally through mutations.

The term "sequence variant" means polypeptides that have been modified compared to their native or original sequence by one or more amino acid insertions, deletions, or substitutions. Insertions may be located at either or both termini of the protein, and/or may be positioned within internal regions of the amino acid sequence. A non-limiting example would be insertion of an XTEN sequence within the sequence of the biologically-active payload protein. In deletion variants, one or more amino acid residues in a polypeptide as described herein are removed. Deletion variants, therefore, include all fragments of a payload polypeptide sequence. In substitution variants, one or more amino acid residues of a polypeptide are removed and replaced with alternative residues. In one aspect, the substitutions are conservative in nature and conservative substitutions of this type are well known in the art.

As used herein, "internal XTEN" refers to XTEN sequences that have been inserted into the sequence of the GLP-2. Internal XTENs can be constructed by insertion of an XTEN sequence into the sequence of GLP-2 by insertion between two adjacent amino acids or wherein XTEN replaces a partial, internal sequence of the GLP-2.

As used herein, "terminal XTEN" refers to XTEN sequences that have been fused to or in the Nor C-terminus of the GLP-2 or to a proteolytic cleavage sequence at the N- or C-terminus of the GLP-2. Terminal XTENs can be fused to the native termini of the GLP-2. Alternatively, terminal XTENs can replace a terminal sequence of the GLP-2.

The term "XTEN release site" refers to a cleavage sequence in GLP2-XTEN fusion proteins that can be recognized and cleaved by a mammalian protease, effecting release of an XTEN or a portion of an XTEN from the GLP2-XTEN fusion protein. As used herein, "mammalian protease" means a protease that normally exists in the body fluids, cells or tissues of a mammal. XTEN release sites can be engineered to be cleaved by various mammalian proteases (a.k.a. "XTEN release proteases") such as FXIa, FXIIa, kallikrein, FVIIIa, FVIIIa, FXa, FIIa (thrombin), Elastase-2, MMP-12, MMP13, MMP-17, MMP-20, or any protease that is present in the subject in proximity to the fusion protein. Other equivalent proteases (endogenous or exogenous) that are capable of recognizing a defined cleavage site can be utilized. The cleavage sites can be adjusted and tailored to the protease utilized.

The term "within", when referring to a first polypeptide being linked to a second polypeptide, encompasses linking that connects the N-terminus of the first or second polypeptide to the C-terminus of the second or first polypeptide, respectively, as well as insertion of the first polypeptide into the sequence of the second polypeptide. For example, when an XTEN is linked "within" a GLP-2 polypeptide, the XTEN may be linked to the N-terminus, the C-terminus, or may be inserted between any two amino acids of the GLP-2 polypeptide.

"Activity" for the purposes herein refers to an action or effect of a component of a fusion protein consistent with that of the corresponding native biologically active protein component of the fusion protein, wherein "biological activity" refers to an in vitro or in vivo biological function or effect, including but not limited to receptor binding, antagonist activity, agonist activity, a cellular or physiologic response, or an effect generally known in the art for the payload GLP-2.

As used herein, the term "ELISA" refers to an enzyme-linked immunosorbent assay as described herein or as otherwise known in the art.

A "host cell" includes an individual cell or cell culture which can be or has been a recipient for the subject vectors. Host cells include progeny of a single host cell. The progeny may not necessarily be completely identical (in morphology or in genomic of total DNA complement) to the original parent cell due to natural, accidental, or deliberate mutation. A host cell includes cells transfected in vivo with a vector of this invention.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. As is apparent to those of skill in the art, a non-naturally occurring polynucleotide, peptide, polypeptide, protein, antibody, or fragments thereof, does not require "isolation" to distinguish it from its naturally occurring counterpart. In addition, a "concentrated", "separated" or "diluted" polynucleotide, peptide, polypeptide, protein, antibody, or fragments thereof, is distinguishable from its naturally occurring counterpart in that the concentration or number of molecules per volume is generally greater than that of its naturally occurring counterpart. In general, a polypeptide made by recombinant means and expressed in a host cell is considered to be "isolated."

An "isolated" nucleic acid is a nucleic acid molecule that is identified and separated from at least one contaminant nucleic acid molecule with which it is ordinarily associated in the natural source of the nucleic acid. For example, an isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal or extra-chromosomal location different from that of natural cells.

A "chimeric" protein contains at least one fusion polypeptide comprising at least one region in a different position in the sequence than that which occurs in nature. The regions may normally exist in separate proteins and are brought together in the fusion polypeptide, or they may normally exist in the same protein but are placed in a new arrangement in the fusion polypeptide. A chimeric protein may be created, for example, by chemical synthesis, or by creating and translating a polynucleotide in which the peptide regions are encoded in the desired relationship.

"Conjugated", "linked," "fused," and "fusion" are used interchangeably herein. These terms refer to the joining together of two or more chemical elements, sequences or components, by whatever means including chemical conjugation or recombinant means. For example, a promoter or enhancer is operably linked to a coding sequence if it affects the transcription of the sequence. Generally, "operably linked" means that the DNA sequences being linked are contiguous, and in reading phase or in-frame. An "in-frame fusion" refers to the joining of two or more open reading frames (ORFs) to form a continuous longer ORF, in a manner that maintains the correct reading frame of the original ORFs. Thus, the resulting recombinant fusion protein is a single protein containing two or more segments that correspond to polypeptides encoded by the original ORFs (which segments are not normally so joined in nature).

In the context of polypeptides, a "linear sequence" or a "sequence" is an order of amino acids in a polypeptide in an amino to carboxyl terminus direction in which residues that neighbor each other in the sequence are contiguous in the primary structure of the polypeptide. A "partial sequence" is a linear sequence of part of a polypeptide that is known to comprise additional residues in one or both directions.

"Heterologous" means derived from a genotypically distinct entity from the rest of the entity to which it is being compared. For example, a glycine rich sequence removed from its native coding sequence and operatively linked to a coding sequence other than the native sequence is a heterologous glycine rich sequence. The term "heterologous" as applied to a polynucleotide, a polypeptide, means that the polynucleotide or polypeptide is derived from a genotypically distinct entity from that of the rest of the entity to which it is being compared.

The terms "polynucleotides", "nucleic acids", "nucleotides" and "oligonucleotides" are used interchangeably. They refer to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

The term "complement of a polynucleotide" denotes a polynucleotide molecule having a complementary base sequence and reverse orientation as compared to a reference sequence, such that it could hybridize with a reference sequence with complete fidelity.

"Recombinant" as applied to a polynucleotide means that the polynucleotide is the product of various combinations of recombination steps which may include cloning, restriction and/or ligation steps, and other procedures that result in an expression of a recombinant protein in a host cell.

The terms "gene" and "gene fragment" are used interchangeably herein. They refer to a polynucleotide containing at least one open reading frame that is capable of encoding a particular protein after being transcribed and translated. A gene or gene fragment may be genomic or cDNA, as long as the polynucleotide contains at least one open reading frame, which may cover the entire coding region or a segment thereof. A "fusion gene" is a gene composed of at least two heterologous polynucleotides that are linked together.

"Homology" or "homologous" or "sequence identity" refers to sequence similarity or interchangeability between two or more polynucleotide sequences or between two or more polypeptide sequences. When using a program such as BestFit to determine sequence identity, similarity or homology between two different amino acid sequences, the default settings may be used, or an appropriate scoring matrix, such as blosum45 or blosum80, may be selected to optimize identity, similarity or homology scores. Preferably, polynucleotides that are homologous are those which hybridize under stringent conditions as defined herein and have at least 70%, preferably at least 80%, more preferably at least 90%, more preferably 95%, more preferably 97%, more preferably 98%, and even more preferably 99% sequence identity compared to those sequences. Polypeptides that are homologous preferably have sequence identities that are at least 70%, preferably at least 80%, even more preferably at least 90%, even more preferably at least 95-99%, and most preferably 100% identical.

"Ligation" refers to the process of forming phosphodiester bonds between two nucleic acid fragments or genes, linking them together. To ligate the DNA fragments or genes together, the ends of the DNA must be compatible with each other. In some cases, the ends will be directly compatible after endonuclease digestion. However, it may be necessary to first convert the staggered ends commonly produced after endonuclease digestion to blunt ends to make them compatible for ligation.

The terms "stringent conditions" or "stringent hybridization conditions" includes reference to conditions under which a polynucleotide will hybridize to its target sequence, to a detectably greater degree than other sequences (e.g., at least 2-fold over background). Generally, stringency of hybridization is expressed, in part, with reference to the temperature and salt concentration under which the wash step is carried out. Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short polynucleotides (e.g., 10 to 50 nucleotides) and at least about 60°C for long polynucleotides (e.g., greater than 50 nucleotides)-for example, "stringent conditions" can include hybridization in 50% formamide, 1 M NaCl, 1% SDS at 37°C, and three washes for 15 min each in 0.1×SSC/1% SDS at 60°C to 65°C. Alternatively, temperatures of about 65°C, 60°C, 55°C, or 42°C may be used. SSC concentration may be varied from about 0.1 to 2×SSC, with SDS being present at about 0.1%. Such wash temperatures are typically selected to be about 5°C to 20°C lower than the thermal melting point for the specific sequence at a defined ionic strength and pH. The Tm is the temperature (under defined ionic strength and pH) at which 50% of the target sequence hybridizes to a perfectly matched probe. An equation for calculating Tm and conditions for nucleic acid hybridization are well known and can be found in Sambrook, J. et al., "Molecular Cloning: A Laboratory Manual," 3rd edition, Cold Spring Harbor Laboratory Press, 2001. Typically, blocking reagents are used to block non-specific hybridization. Such blocking reagents include, for instance, sheared and denatured salmon sperm DNA at about 100-200 µg/ml. Organic solvent, such as formamide at a concentration of about 35-50% v/v, may also be used under particular circumstances, such as for RNA:DNA hybridizations. Useful variations on these wash conditions will be readily apparent to those of ordinary skill in the art.

The terms "percent identity, "percentage of sequence identity," and "% identity," as applied to polynucleotide sequences, refer to the percentage of residue matches between at least two polynucleotide sequences aligned using a standardized algorithm. Such an algorithm may insert, in a standardized and reproducible way, gaps in the sequences being compared in order to optimize alignment between two sequences, and therefore achieve a more meaningful comparison of the two sequences. Percent identity may be measured over the length of an entire defined polynucleotide sequence, or may be measured over a shorter length, for example, over the length of a fragment taken from a larger, defined polynucleotide sequence, for instance, a fragment of at least 45, at least 60, at least 90, at least 120, at least 150, at least 210 or at least 450 contiguous residues. Such lengths are exemplary only, and it is understood that any fragment length supported by the sequences shown herein, in the tables, figures or Sequence Listing, may be used to describe a length over which percentage identity may be measured. The percentage of sequence identity is calculated by comparing two optimally aligned sequences over the window of comparison, determining the number of matched positions (at which identical residues occur in both polypeptide sequences), dividing the number of matched positions by the total number of positions in the window of comparison (i.e., the window size), and multiplying the result by 100 to yield the percentage of sequence identity. When sequences of different length are to be compared, the shortest sequence defines the length of the window of comparison. Conservative substitutions are not considered when calculating sequence identity.

"Percent (%) sequence identity," with respect to the polypeptide sequences identified herein, is defined as the percentage of amino acid residues in a query sequence that are identical with the amino acid residues of a second, reference polypeptide sequence or a portion thereof, after aligning the sequences and introducing gaps, if necessary, to achieve the maximum percent sequence identity, and not considering any conservative substitutions as part of the sequence identity, thereby resulting in optimal alignment. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve optimal alignment over the full length of the sequences being compared. Percent identity may be measured over the length of an entire defined polypeptide sequence, or may be measured over a shorter length, for example, over the length of a fragment taken from a larger, defined polypeptide sequence, for instance, a fragment of at least 15, at least 20, at least 30, at least 40, at least 50, at least 70 or at least 150 contiguous residues. Such lengths are exemplary only, and it is understood that any fragment length supported by the sequences shown herein, in the tables, figures or Sequence Listing, may be used to describe a length over which percentage identity may be measured.

"Repetitiveness" used in the context of polynucleotide sequences refers to the degree of internal homology in the sequence such as, for example, the frequency of identical nucleotide sequences of a given length. Repetitiveness can, for example, be measured by analyzing the frequency of identical sequences.

A "vector" is a nucleic acid molecule, preferably self-replicating in an appropriate host, which transfers an inserted nucleic acid molecule into and/or between host cells. The term includes vectors that function primarily for insertion of DNA or RNA into a cell, replication of vectors that function primarily for the replication of DNA or RNA, and expression vectors that function for transcription and/or translation of the DNA or RNA. Also included are vectors that provide more than one of the above functions. An "expression vector" is a polynucleotide which, when introduced into an appropriate host cell, can be transcribed and translated into a polypeptide(s). An "expression system" usually connotes a suitable host cell comprised of an expression vector that can function to yield a desired expression product.

"Serum degradation resistance," as applied to a polypeptide, refers to the ability of the polypeptides to withstand degradation in blood or components thereof, which typically involves proteases in the serum or plasma. The serum degradation resistance can be measured by combining the protein with human (or mouse, rat, monkey, as appropriate) serum or plasma, typically for a range of days (e.g. 0.25, 0.5, 1, 2, 4, 8, 16 days), typically at about 37°C. The samples for these time points can be run on a Western blot assay and the protein is detected with an antibody. The antibody can be to a tag in the protein. If the protein shows a single band on the western, where the protein's size is identical to that of the injected protein, then no degradation has occurred. In this exemplary method, the time point where 50% of the protein is degraded, as judged by Western blots or equivalent techniques, is the serum degradation half-life or "serum half-life" of the protein.

The terms "$t_{1/2}$", "terminal half-life", "elimination half-life" and "circulating half-life" are used interchangeably herein and, as used herein mean the terminal half-life calculated as $\ln(2)/K_{el}$. $K_{el}$ is the terminal elimination rate constant calculated by linear regression of the terminal linear portion of the log concentration vs. time curve. Half-life typically refers to the time required for half the quantity of an administered substance deposited in a living organism to be metabolized or eliminated by normal biological processes.

"Active clearance" means the mechanisms by which a protein is removed from the circulation other than by filtration, and which includes removal from the circulation mediated by cells, receptors, metabolism, or degradation of the protein.

"Apparent molecular weight factor" and "apparent molecular weight" are related terms referring to a measure of the relative increase or decrease in apparent molecular weight exhibited by a particular amino acid or polypeptide sequence. The apparent molecular weight is determined using size exclusion chromatography (SEC) or similar methods by comparing to globular protein standards and is measured in "apparent kDa" units. The apparent molecular weight factor is the ratio between the apparent molecular weight and the actual molecular weight; the latter predicted by adding, based on amino acid composition, the calculated molecular weight of each type of amino acid in the composition or by estimation from comparison to molecular weight standards in an SDS electrophoresis gel. Determination of both the apparent molecular weight and apparent molecular weight factor for representative proteins is described in the Examples.

The terms "hydrodynamic radius" or "Stokes radius" is the effective radius ($R_h$ in nm) of a molecule in a solution measured by assuming that it is a body moving through the solution and resisted by the solution's viscosity. In the embodiments of the invention, the hydrodynamic radius measurements of the XTEN fusion proteins correlate with the 'apparent molecular weight factor', which is a more intuitive measure. The "hydrodynamic radius" of a protein affects its rate of diffusion in aqueous solution as well as its ability to migrate in gels of macromolecules. The hydrodynamic radius of a protein is determined by its molecular weight as well as by its structure, including shape and compactness. Methods for determining the hydrodynamic radius are well known in the art, such as by the use of size exclusion chromatography (SEC), as described in U.S. Patent Nos. 6,406,632 and 7,294,513. Most proteins have globular structure, which is the most compact three-dimensional structure a protein can have with the smallest hydrodynamic radius. Some proteins adopt a random and open, unstructured, or 'linear' conformation and as a result have a much larger hydrodynamic radius compared to typical globular proteins of similar molecular weight.

"Physiological conditions" refers to a set of conditions in a living host as well as *in vitro* conditions, including temperature, salt concentration, pH, that mimic those conditions of a living subject. A host of physiologically relevant conditions for use in *in vitro* assays have been established. Generally, a physiological buffer contains a physiological concentration of salt and is adjusted to a neutral pH ranging from about 6.5 to about 7.8, and preferably from about 7.0 to about 7.5. A variety of physiological buffers are listed in Sambrook et al. (2001). Physiologically relevant temperature ranges from about 25°C to about 38°C, and preferably from about 35°C to about 37°C.

A "reactive group" is a chemical structure that can be coupled to a second reactive group. Examples for reactive groups are amino groups, carboxyl groups, sulfhydryl groups, hydroxyl groups, aldehyde groups, azide groups. Some reactive groups can be activated to facilitate coupling with a second reactive group. Non-limiting examples for activation are the reaction of a carboxyl group with carbodiimide, the conversion of a carboxyl group into an activated ester, or the conversion of a carboxyl group into an azide function.

"Controlled release agent", "slow release agent", "depot formulation" and "sustained release agent" are used interchangeably to refer to an agent capable of extending the duration of release of a polypeptide of the invention relative to the duration of release when the polypeptide is administered in the absence of agent. Different embodiments of the present invention may have different release rates, resulting in different therapeutic amounts.

The terms "antigen", "target antigen" and "immunogen" are used interchangeably herein to refer to the structure or binding determinant that an antibody fragment or an antibody fragment-based therapeutic binds to or has specificity against.

The term "payload" as used herein refers to a protein or peptide sequence that has biological or therapeutic activity; the counterpart to the pharmacophore of small molecules. Examples of payloads include, but are not limited to, cytokines, enzymes, hormones, blood coagulation factors, and growth factors. Payloads can further comprise genetically fused or chemically conjugated moieties such as chemotherapeutic agents, antiviral compounds, toxins, or contrast agents. These conjugated moieties can be joined to the rest of the polypeptide via a linker that may be cleavable or non-cleavable.

The term "antagonist", as used herein, includes any molecule that partially or fully blocks, inhibits, or neutralizes a biological activity of a native polypeptide disclosed herein. Methods for identifying antagonists of a polypeptide may comprise contacting a native polypeptide with a candidate antagonist molecule and measuring a detectable change in one or more biological activities normally associated with the native polypeptide. In the context of the present invention, antagonists may include proteins, nucleic acids, carbohydrates, antibodies or any other molecules that decrease the effect of a biologically active protein.

The term "agonist" is used in the broadest sense and includes any molecule that mimics a biological activity of a native polypeptide disclosed herein. Suitable agonist molecules specifically include agonist antibodies or antibody fragments, fragments or amino acid sequence variants of native polypeptides, peptides, small organic molecules, etc. Methods for identifying agonists of a native polypeptide may comprise contacting a native polypeptide with a candidate agonist molecule and measuring a detectable change in one or more biological activities normally associated with the native polypeptide.

"Inhibition constant", or "$K_i$", are used interchangeably and mean the dissociation constant of the enzyme-inhibitor complex, or the reciprocal of the binding affinity of the inhibitor to the enzyme.

As used herein, "treat" or "treating," or "palliating" or "ameliorating" are used interchangeably and mean administering a drug or a biologic to achieve a therapeutic benefit, to cure or reduce the severity of an existing condition, or to achieve a prophylactic benefit, prevent or reduce the likelihood of onset or severity the occurrence of a condition. By therapeutic benefit is meant eradication or amelioration of the underlying condition being treated or one or more of the physiological symptoms associated with the underlying condition such that an improvement is observed in the subject, notwithstanding that the subject may still be afflicted with the underlying condition.

A "therapeutic effect" or "therapeutic benefit," as used herein, refers to a physiologic effect, including but not limited to the mitigation, amelioration, or prevention of disease in humans or other animals, or to otherwise enhance physical or mental wellbeing of humans or animals, resulting from administration of a fusion protein of the invention other than the ability to induce the production of an antibody against an antigenic epitope possessed by the biologically active protein. For prophylactic benefit, the compositions may be administered to a subject at risk of developing a particular condition, or to a subject reporting one or more of the physiological symptoms of a condition, even though a diagnosis (e.g., Crohn's Disease) may not have been made.

The terms "therapeutically effective amount" and "therapeutically effective dose", as used herein, refer to an amount of a drug or a biologically active protein, either alone or as a part of a fusion protein composition, that is capable of having any detectable, beneficial effect on any symptom, aspect, measured parameter or characteristics of a disease state or condition when administered in one or repeated doses to a subject. Such effect need not be absolute to be beneficial. Determination of a therapeutically effective amount is well within the capability of those skilled in the art, especially in light of the detailed disclosure provided herein.

The term "therapeutically effective dose regimen", as used herein, refers to a schedule for consecutively administered multiple doses (i.e., at least two or more) of a biologically active protein, either alone or as a part of a fusion protein composition, wherein the doses are given in therapeutically effective amounts to result in sustained beneficial effect on any symptom, aspect, measured parameter or characteristics of a disease state or condition.

## I). GENERAL TECHNIQUES

The practice of the present invention employs, unless otherwise indicated, conventional techniques of immunology, biochemistry, chemistry, molecular biology, microbiology, cell biology, genomics and recombinant DNA, which are within the skill of the art. *See* Sambrook, J. et al., "Molecular Cloning: A Laboratory Manual," 3rd edition, Cold Spring Harbor Laboratory Press, 2001; "Current protocols in molecular biology", F. M. Ausubel, et al. eds.,1987; the series "Methods in Enzymology," Academic Press, San Diego, CA.; "PCR 2: a practical approach", M.J. MacPherson, B.D. Hames and G.R. Taylor eds., Oxford University Press, 1995; "Antibodies, a laboratory manual" Harlow, E. and Lane, D. eds., Cold Spring Harbor Laboratory,1988; "Goodman & Gilman's The Pharmacological Basis of Therapeutics," 11th Edition, McGraw-Hill, 2005; and Freshney, R.I., "Culture of Animal Cells: A Manual of Basic Technique," 4th edition, John Wiley & Sons, Somerset, NJ, 2000, the contents of which are incorporated in their entirety herein by reference.

## II). GLUCAGON-LIKE-2 PROTEIN

The present invention relates, in part, to fusion protein compositions comprising GLP-2 and one or more extended recombinant polypeptide (XTEN), resulting in GLP2-XTEN fusion protein compositions.

"Glucagon-like protein-2" or "GLP-2" means, collectively herein, human glucagon like peptide-2, species homologs of human GLP-2, and non-natural sequence variants having at least a portion of the biological activity of mature GLP-2 including variants such as, but not limited to, a variant with glycine substituted for alanine at position 2 of the mature sequence ("2G") as well as Val, Glu, Lys, Arg, Leu or Ile substituted for alanine at position 2. GLP-2 or sequence variants have been isolated, synthesized, characterized, or cloned, as described in U.S. Patent or Application Nos. 5,789,379; 5,834,428; 5,990,077; 5,994,500; 6,184,201; 7,186,683; 7,563,770; 20020025933; and 20030162703.

Human GLP-2 is a 33 amino acid peptide, co-secreted along with GLP-1 from intestinal endocrine cells in the epithelium of the small and large intestine. The 180 amino-acid product of the proglucagon gene is post-translationally processed in a tissue-specific manner in pancreatic A cells and intestinal L cells into the 33 amino acid GLP-2 (Orskov et al., FEBS Lett. (1989) 247: 193-196; Hartmann et al., Peptides (2000) 21: 73-80). In pancreatic A cells, the major bioactive hormone is glucagon cleaved by PCSK2/PC2. In the intestinal L cells PCSK1/PC1 liberates GLP-1, GLP-2, glicentin and oxyntomodulin. GLP-2 functions as a pleiotropic intestinotrophic hormone with wide-ranging effects that include the promotion of mucosal growth and nutrient absorption, intestinal homeostasis, regulation of gastric motility, gastric acid secretion and intestinal hexose transport, reduction of intestinal permeability and increase in mesenteric blood flow (Estall JL, Drucker DJ (2006) Glucagon-like peptide-2. Annual Rev Nutr26:391-411), (Guan X, et al. (2006) GLP-2 receptor localizes to enteric neurons and endocrine cells expressing vasoactive peptides and mediates increased blood flow. Gastroenterology 130:150-164; Stephens J, et al. (2006) Glucagon-like peptide-2 acutely increases proximal small intestinal blood flow in TPN-fed neonatal piglets. Am J Physiol Regul Integr Comp Physiol 290:R283-R289; Nelson DW, et al. (2007) Localization and activation of GLP-2 receptors on vagal afferents in the rat. Endocrinology 148:1954-1962). The effects mediated by GLP-2 are triggered by the binding and activation of the GLP-2 receptor, a member of the glucagon/secretin G protein-coupled receptor superfamily that is located on enteric (Bjerknes M, Cheng H (2001) Modulation of specific intestinal epithelial progenitors by enteric neurons. Proc Natl Acad Sci USA 98:12497-12502) and vagal (Nelson et al., 2007) nerves, subepithelial myofibroblasts (Orskov C, et al. (2005) GLP-2 stimulates colonic growth via KGF, released by subepithelial myofibroblasts with GLP-2 receptors. Regul Pept 124:105-11), and a subset of intestinal epithelial cells (Thulesen J, et al. (2000) Potential targets for glucagon-like peptide 2 (GLP-2) in the rat: distribution and binding of i.v. injected (125)1-GLP-2. Peptides 21:1511-1517). In addition, GLP-2 has an important role in intestinal adaptation, repair and protection during inflammatory events, including amelioration of the effects of proinflammatory cytokines (Sigalet DL, et al. (2007) Enteric neural pathways mediate the anti-inflammatory actions of glucagon-like peptide 2. Am J Physiol Gastrointest Liver Physiol 293:G211-G221). GLP-2 also enhances nutrient absorption and gut adaptation in rodents or humans with short bowel syndrome (SBS) (Jeppesen et al., (2001) Gastroenterology 120: 806-815).

In one aspect, the invention contemplates inclusion of GLP-2 sequences in the GLP2-XTEN fusion protein compositions that are identical to human GLP-2, sequences that have homology to GLP-2 sequences, sequences that are natural, such as from humans, non-human primates, mammals (including domestic animals) that retain at least a portion of the biologic activity or biological function of native human GLP-2. In one embodiment, the GLP-2 is a non-natural GLP-2 sequence variant, fragment, or a mimetic of a natural sequence that retains at least a portion of the biological activity of the corresponding native GLP-2, such as but not limited to the substitution of the alanine at position 2 of the mature GLP-2 peptide sequence with glycine ("GLP-2-2G"). In another embodiment, the GLP-2 of the fusion protein has the sequence HGDGSFSDEMNTILDNLAARDFINWLIQTKITD. Sequences with homology to GLP-2 may be found by standard homology searching techniques, such as NCBI BLAST, or in public databases such as Chemical Abstracts Services Databases (e.g., the CAS Registry), GenBank, The Universal Protein Resource (UniProt) and subscription provided databases such as GenSeq (e.g., Derwent).

Table 1 provides a non-limiting list of amino acid sequences of GLP-2 that are encompassed by the GLP2-XTEN fusion proteins of the invention. Any of the GLP-2 sequences or homologous derivatives to be incorporated into the fusion protein compositions can be constructed by shuffling individual mutations into and between the amino acids of the sequences of Table 1 or by replacing the amino acids of the sequences of Table 1. The resulting GLP-2 sequences can be evaluated for activity and those that retain at least a portion of the biological activity of the native GLP-2 may be useful for inclusion in the fusion protein compositions of this invention. In some embodiments, GLP-2 that can be incorporated into a GLP2-XTEN include proteins that have at least about 80% sequence identity, or alternatively 81%, 82%, 83%, 84%, 85%, 86%, 87%, 88%, 89%, 90%, 91%, 92%, 93%, 94%, 95%, 96%, 97%, 98%, 99%, or 100% sequence identity compared to an amino acid sequence selected from Table 1.

**Table 1: GLP-2 amino acid sequences**

| Name (source) | Amino Acid Sequence |
|---|---|
| GLP-2 (human) | HADGSFSDEMNTILDNLAARDFINWLIQTKITD |
| GLP-2 variant 1 SEQ ID NO: 3 US Pat No. 7,186,683 | HADGSFSDEMNTILDNLATRDFINWLIQTKITD |
| GLP-2 variant 2 SEQ ID NO:5 US Pat No. 5,789,379 | HGDGSFSDEMNTILDNLAARDFINWLIQTKITD |
| GLP-2 variant 3 | HVDGSFSDEMNTILDNLAARDFINWLIQTKITD |
| GLP-2 variant 4 | HEDGSFSDEMNTILDNLAARDFINWLIQTKITD |
| GLP-2 variant 5 | HKDGSFSDEMNTILDNLAARDFINWLIQTKITD |
| GLP-2 variant 6 | HRDGSFSDEMNTILDNLAARDFINWLIQTKITD |
| GLP-2 variant 7 | HLDGSFSDEMNTILDNLAARDFINWLIQTKITD |
| GLP-2 variant 8 | HIDGSFSDEMNTILDNLAARDFINWLIQTKITD |
| GLP-2 (mouse) | HADGSFSDEMSTILDNLATRDFINWLIQTKITD |
| GLP-2 (rat) | HADGSFSDEMNTILDNLATRDFINWLIQTKITD |
| GLP-2 (bovine) | HADGSFSDEMNTVLDSLATRDFINWLLQTKITD |
| GLP-2 (bovine variant) | HGDGSFSDEMNTVLDSLATRDFINWLLQTKITD |
| GLP-2 (pig) | HADGSFSDEMNTVLDNLATRDFINWLLHTKITDSL |
| GLP-2 (pig variant) | HGDGSFSDEMNTVLDNLATRDFINWLLHTKITDSL |

(continued)

| Name (source) | Amino Acid Sequence |
|---|---|
| GLP-2 (sheep) | HADGSFSDEMNTVLDSLATRDFINWLLQTKI |
| GLP-2 (sheep variant) | HGDGSFSDEMNTVLDSLATRDFINWLLQTKI |
| GLP-2 (canine) | HADGSFSDEMNTVLDTLATRDFINWLLQTKITD |
| GLP-2 (canine variant) | HGDGSFSDEMNTVLDTLATRDFINWLLQTKITD |
| GLP-2 (chicken) | HADGTFTSDINKILDDMAAKEFLKWLINTKVTQ |
| GLP-2 (chicken variant) | HGDGTFTSDINKILDDMAAKEFLKWLINTKVTQ |
| GLP-2 (turkey) | HADGTFTSDINKILDDMAAKEFLKWLINTKVTQ |
| GLP-2 (turkey variant) | HGDGTFTSDINKILDDMAAKEFLKWLINTKVTQ |
| GLP-2 (Xenopus laevis) | HADGSFTNDINKVLDIIAAQEFLDWVINTQETE |

The GLP-2 of the subject compositions are not limited to native, full-length GLP-2 polypeptides, but also include recombinant versions as well as biologically and/or pharmacologically active forms with sequence variants, or fragments thereof. For example, it will be appreciated that various amino acid deletions, insertions and substitutions can be made in the GLP-2 to create variants that exhibit one or more biological activity or pharmacologic properties of the wild-type GLP-2. Examples of conservative substitutions for amino acids in polypeptide sequences are shown in Table 2. In embodiments of the GLP2-XTEN in which the sequence identity of the GLP-2 is less than 100% compared to a specific sequence disclosed herein, the invention contemplates substitution of any of the other 19 natural L-amino acids for a given amino acid residue of a given GLP-2, which may be at any position within the sequence of the GLP-2, including adjacent amino acid residues. In some embodiments, the GLP-2 variant incorporated into the GLP2-XTEN has glycine (G), valine (V), glutamate (E), lysine (K), arginine (R), leucine (K) or isoleucine (I) substituted for alanine (A) at position 2 of the mature peptide. Such substitution may confer resistance to dipeptidyl peptidase-4 (DPP-4). In one embodiment, glycine is substituted for alanine at position 2 of the GLP-2 sequence. If any one substitution results in an undesirable change in biological activity, then one of the alternative amino acids can be employed and the construct protein evaluated by the methods described herein (e.g., the assays of Table 32), or using any of the techniques and guidelines for conservative and non-conservative mutations set forth, for instance, in U.S. Pat. No. 5,364,934 (the content of which is incorporated by reference in its entirety), or using methods generally known in the art. In addition, variants can include, for instance, polypeptides wherein one or more amino acid residues are added or deleted at the N- or C-terminus of the full-length native amino acid sequence of a GLP-2 that retains some if not all of the biological activity of the native peptide; e.g., the ability to bind GLP-2 receptor and/or the ability to activate GLP-2 receptor.

### Table 2: Exemplary conservative amino acid substitutions

| Original Residue | Exemplary Substitutions |
|---|---|
| Ala (A) | val; leu; ile |
| Arg (R) | lys; gln; asn |
| Asn (N) | gln; his; lys; arg |
| Asp (D) | Glu |
| Cys (C) | Ser |
| Gln (Q) | Asn |
| Glu (E) | Asp |
| Gly (G) | Pro |
| His (H) | asn: gln: lys: arg |
| Ile (I) | leu; val; met; ala; phe: norleucine |
| Leu (L) | norleucine: ile: val; met; ala: phe |
| Lys (K) | arg: gln: asn |

(continued)

| Original Residue | Exemplary Substitutions |
|---|---|
| Met (M) | leu; phe; ile |
| Phe (F) | leu: val: ile; ala |
| Pro (P) | Gly |
| Ser (S) | Thr |
| Thr (T) | Ser |
| Trp (W) | Tyr |
| Tyr(Y) | Trp: phe: thr: ser |
| Val (V) | Ile; leu; met; phe; ala; norleucine |

Sequence variants of GLP-2, whether exhibiting substantially the same or better biological activity than a corresponding wild-type GLP-2, or, alternatively, exhibiting substantially modified or reduced biological activity relative to wild-type GLP-2, include, without limitation, polypeptides having an amino acid sequence that differs from the sequence of wild-type GLP-2 by insertion, deletion, or substitution of one or more amino acids. Such GLP-2 variants are known in the art, including those described in US Patent No. 7,186,683 or US Pat. No. 5,789,379, 5,994,500, all of which are incorporated herein by reference.

### III). EXTENDED RECOMBINANT POLYPEPTIDES

In one aspect, the invention provides XTEN polypeptide compositions that are useful as fusion protein partner(s) to link to and/or incorporate within a GLP-2 sequence, resulting in a GLP2-XTEN fusion protein. XTEN are generally polypeptides with non-naturally occurring, substantially non-repetitive sequences having a low degree of or no secondary or tertiary structure under physiologic conditions. XTEN typically have from about 36 to about 3000 amino acids of which the majority or the entirety are small hydrophilic amino acids. As used herein, "XTEN" specifically excludes whole antibodies or antibody fragments (e.g. single-chain antibodies and Fc fragments). XTENs have utility as a fusion protein partners in that they serve in various roles, conferring certain desirable pharmacokinetic, physicochemical and pharmaceutical properties when linked to a GLP-2 protein to a create a GLP2-XTEN fusion protein. Such GLP2-XTEN fusion protein compositions have enhanced properties compared to the corresponding GLP-2 not linked to XTEN, making them useful in the treatment of certain gastrointestinal conditions, as more fully described below.

The selection criteria for the XTEN to be fused to the biologically active proteins generally relate to attributes of physicochemical properties and conformational structure of the XTEN that is, in turn, used to confer the enhanced properties to the fusion proteins compositions. The unstructured characteristic and physical/chemical properties of the XTEN result, in part, from the overall amino acid composition disproportionately limited to 4-6 hydrophilic amino acids, the linking of the amino acids in a quantifiable non-repetitive design, and the length of the XTEN polypeptide. In an advantageous feature common to XTEN but uncommon to polypeptides, the properties of XTEN disclosed herein are not tied to absolute primary amino acid sequences, as evidenced by the diversity of the exemplary sequences of Table 4 that, within varying ranges of length, possess similar properties, many of which are documented in the Examples. The XTEN of the present invention exhibits one or more of the following advantageous properties: conformational flexibility, reduced or lack of secondary structure, high degree of aqueous solubility, high degree of protease resistance, low immunogenicity, low binding to mammalian receptors, a defined degree of charge, and increased hydrodynamic (or Stokes) radii; properties that make them particularly useful as fusion protein partners. In turn, non-limiting examples of the enhanced properties of the fusion proteins comprising GLP-2 fused to the XTEN include increases in the overall solubility and/or metabolic stability, reduced susceptibility to proteolysis, reduced immunogenicity, reduced rate of absorption when administered subcutaneously or intramuscularly, reduced clearance by the kidney, enhanced interactions with substrate, and enhanced pharmacokinetic properties. Enhanced pharmacokinetic properties of the inventive GLP2-XTEN compositions include longer terminal half-life (e.g., two-fold, three-fold, four-fold or more), increased area under the curve (AUC) (e.g., 25%, 50%, 100% or more), lower volume of distribution, slower absorption after subcutaneous or intramuscular injection (compared to GLP-2 not linked to the XTEN and administered by a similar route) such that the $C_{max}$ is lower, which, in turn, results in reductions in adverse effects of the GLP-2 that, collectively, results in an increased period of time that a fusion protein of a GLP2-XTEN composition administered to a subject provides therapeutic activity. In some embodiments, the GLP2-XTEN compositions comprise cleavage sequences (described more fully, below) that permits sustained release of biologically active GLP-2.A GLP2-XTEN having such cleavage sequence can act as a depot when subcutaneously or intramuscularly administered. It is specifically contemplated that the subject GLP2-XTEN fusion proteins of the disclosure can exhibit one or more or any combination of the improved properties disclosed herein. In some embodiments, GLP2-XTEN compositions permit less frequent dosing compared to GLP-2 not linked to the XTEN and administered in a comparable fashion. Such GLP2-XTEN fusion protein compositions have utility to treat certain GLP-2-related diseases, disorders or conditions, as described herein.

A variety of methods and assays are known in the art for determining the physicochemical properties of proteins such as the compositions comprising the inventive XTEN. Such properties include but are not limited to secondary or tertiary structure, solubility, protein aggregation, melting properties, contamination and water content. Such methods include analytical centrifugation, EPR, HPLC-ion exchange, HPLC-size exclusion chromatography (SEC), HPLC-reverse phase, light scattering, capillary electrophoresis, circular dichroism, differential scanning calorimetry, fluorescence, HPLC-ion exchange, IR, NMR, Raman spectroscopy, refractometry, and UV/Visible spectroscopy. Additional methods are disclosed in Arnau, et al., Prot Expr and Purif (2006) 48, 1-13.

The XTEN component(s) of the GLP2-XTEN are designed to behave like denatured peptide sequences under physiological conditions, despite the extended length of the polymer. "Denatured" describes the state of a peptide in solution that is characterized by a large conformational freedom of the peptide backbone. Most peptides and proteins adopt a denatured conformation in the presence of high concentrations of denaturants or at elevated temperature. Peptides in denatured conformation have, for example, characteristic circular dichroism (CD) spectra and are characterized by a lack of long-range interactions as determined by NMR. "Denatured conformation" and "unstructured conformation" are used synonymously herein. In some embodiments, the invention provides XTEN sequences that, under physiologic conditions, resemble denatured sequences that are largely devoid in secondary structure. In other cases, the XTEN sequences are substantially devoid of secondary structure under physiologic conditions. "Largely devoid," as used in this context, means that less than 50% of the XTEN amino acid residues of the XTEN sequence contribute to secondary structure as measured or determined by the means described herein. "Substantially devoid," as used in this context, means that at least about 60%, or about 70%, or about 80%, or about 90%, or about 95%, or at least about 99% of the XTEN amino acid residues of the XTEN sequence do not contribute to secondary structure, as measured or determined by the methods described herein.

A variety of methods have been established in the art to discern the presence or absence of secondary and tertiary structures in a given polypeptide. In particular, secondary structure can be measured spectrophotometrically, e.g., by circular dichroism spectroscopy in the "far-UV" spectral region (190-250 nm). Secondary structure elements, such as alpha-helix and beta-sheet, each give rise to a characteristic shape and magnitude of CD spectra. Secondary structure can also be predicted for a polypeptide sequence via certain computer programs or algorithms, such as the well-known Chou-Fasman algorithm (Chou, P. Y., et al. (1974) Biochemistry, 13: 222-45) and the Garnier-Osguthorpe-Robson algorithm ("Gor algorithm") (Gamier J, Gibrat JF, Robson B. (1996), GOR method for predicting protein secondary structure from amino acid sequence. Methods Enzymol 266:540-553), as described in US Patent Application Publication No. 20030228309A1. For a given sequence, the algorithms can predict whether there exists some or no secondary structure at all, expressed as the total and/or percentage of residues of the sequence that form, for example, alpha-helices or beta-sheets or the percentage of residues of the sequence predicted to result in random coil formation (which lacks secondary structure). Polypeptide sequences can be analyzed using the Chou-Fasman algorithm using sites on the world wide web at, for example, fasta.bioch.virginia.edu/fasta_www2/fasta_www.cgi?rm=misc1 and the Gor algorithm at npsa-pbil.ibcp.fr/cgi-bin/npsa_automat.pl?page=npsa_gor4.html (both accessed on September 5, 2012).

In one embodiment, the XTEN sequences used in the subject fusion protein compositions have an alpha-helix percentage ranging from 0% to less than about 5% as determined by the Chou-Fasman algorithm. In another embodiment, the XTEN sequences of the fusion protein compositions have a beta-sheet percentage ranging from 0% to less than about 5% as determined by the Chou-Fasman algorithm. In some embodiments, the XTEN sequences of the fusion protein compositions have an alpha-helix percentage ranging from 0% to less than about 5% and a beta-sheet percentage ranging from 0% to less than about 5% as determined by the Chou-Fasman algorithm. In one embodiment, the XTEN sequences of the fusion protein compositions have an alpha-helix percentage less than about 2% and a beta-sheet percentage less than about 2%. The XTEN sequences of the fusion protein compositions have a high degree of random coil percentage, as determined by the GOR algorithm. In some embodiments, an XTEN sequence have at least about 80%, more preferably at least about 90%, more preferably at least about 91%, more preferably at least about 92%, more preferably at least about 93%, more preferably at least about 94%, more preferably at least about 95%, more preferably at least about 96%, more preferably at least about 97%, more preferably at least about 98%, and most preferably at least about 99% random coil, as determined by the GOR algorithm. In one embodiment, the XTEN sequences of the fusion protein compositions have an alpha-helix percentage ranging from 0% to less than about 5% and a beta-sheet percentage ranging from 0% to less than about 5% as determined by the Chou-Fasman algorithm and at least about 90% random coil, as determined by the GOR algorithm. In another embodiment, the XTEN sequences of the fusion protein compositions have an alpha-helix percentage less than about 2% and a beta-sheet percentage less than about 2% at least about 90% random coil, as determined by the GOR algorithm.

1. <u>Non-repetitive Sequences</u>

It is contemplated that the XTEN sequences of the GLP2-XTEN embodiments are substantially non-repetitive. In general, repetitive amino acid sequences have a tendency to aggregate or form higher order structures, as exemplified by natural repetitive sequences such as collagens and leucine zippers. These repetitive amino acids may also tend to form contacts resulting in crystalline or pseudocrystaline structures. In contrast, the low tendency of non-repetitive sequences to aggregate enables the design of long-sequence XTENs with a relatively low frequency of charged amino acids that would otherwise be likely to aggregate if the sequences were repetitive. The non-repetitiveness of a subject XTEN can be observed by assessing one or more of the following features. In one embodiment, a "substantially non-repetitive" XTEN sequence has no three contiguous amino acids in the sequence that are of identical amino acid types unless the amino acid is serine, in which case no more than three contiguous amino acids are serine residues. In another embodiment, as described more fully below, a "substantially non-repetitive" XTEN sequence comprises motifs of 9 to 14 amino acid residues wherein the motifs consist of 3, 4, 5, or 6 types of amino acids selected from glycine (G), alanine (A), serine (S), threonine (T), glutamate (E) and proline (P), and wherein the sequence of any two contiguous amino acid residues in any one motif is not repeated more than twice in the sequence motif.

The degree of repetitiveness of a polypeptide or a gene can be measured by computer programs or algorithms or by other means known in the art. According to the current invention, algorithms to be used in calculating the degree of repetitiveness of a particular polypeptide, such as an XTEN, are disclosed herein, and examples of sequences analyzed by algorithms are provided (*see* Examples, below). In one embodiment, the repetitiveness of a polypeptide of a predetermined length can be calculated (hereinafter "subsequence score") according to the formula given by Equation 1:

$$\text{Subsequence score} = \frac{\sum_{i=1}^{m} Count_i}{m} \qquad \text{I}$$

wherein: m = (amino acid length of polypeptide) - (amino acid length of subsequence) + 1; and
$Count_i$ = cumulative number of occurrences of each unique subsequence within sequence$_i$

An algorithm termed "SegScore" was developed to apply the foregoing equation to quantitate repetitiveness of polypeptides, such as an XTEN, providing the subsequence score wherein sequences of a predetermined amino acid length are analyzed for repetitiveness by determining the number of times (a "count") a unique subsequence of length "s" appears in the set length, divided by the absolute number of subsequences within the predetermined length of the sequence. FIG. 1 depicts a logic flowchart of the SegScore algorithm, while FIG. 2 portrays a schematic of how a subsequence score is derived for a fictitious XTEN with 11 amino acids and a subsequence length of 3 amino acid residues. For example, a predetermined polypeptide length of 200 amino acid residues has 192 overlapping 9-amino acid subsequences and 198 3-mer subsequences, but the subsequence score of any given polypeptide will depend on the absolute number of unique subsequences and how frequently each unique subsequence (meaning a different amino acid sequence) appears in the predetermined length of the sequence.

In the context of the present invention, "subsequence score" means the sum of occurrences of each unique 3-mer frame across 200 consecutive amino acids of the cumulative XTEN polypeptide divided by the absolute number of unique 3-mer subsequences within the 200 amino acid sequence. Examples of such subsequence scores derived from 200 consecutive amino acids of repetitive and non-repetitive polypeptides are presented in Example 30. In one embodiment, the invention provides a GLP2-XTEN comprising one XTEN in which the XTEN has a subsequence score less than 12, more preferably less than 10, more preferably less than 9, more preferably less than 8, more preferably less than 7, more preferably less than 6, and most preferably less than 5. In another embodiment, the invention provides GLP2-XTEN comprising two more XTENs in which at least one XTEN has a subsequence score of less than 10, or less than 9, or less than 8, or less than 7, or less than 6, or less than 5, or less. In yet another embodiment, the invention provides GLP2-XTEN comprising at least two XTENs in which each individual XTEN of 36 or more amino acids has a subsequence score of less than 10, or less than 9, or less than 8, or less than 7, or less than 6, or less than 5, or less. In the embodiments of this paragraph, the XTEN is characterized as substantially non-repetitive.

In one aspect, the non-repetitive characteristic of XTEN of the present invention together with the particular types of amino acids that predominate in the XTEN, rather than the absolute primary sequence, confers one or more of the enhanced physicochemical and biological properties of the GLP2-XTEN fusion proteins. These enhanced properties include a higher degree of expression of the fusion protein in the host cell, greater genetic stability of the gene encoding XTEN, a greater degree of solubility, less tendency to aggregate, and enhanced pharmacokinetics of the resulting GLP2-XTEN compared to fusion proteins comprising polypeptides having repetitive sequences. These enhanced properties permit more efficient manufacturing, lower cost of goods, and/or facilitate the formulation of XTEN-comprising pharmaceutical preparations containing extremely high protein concentrations, in some cases exceeding 100 mg/ml. In some embodiments, the XTEN polypeptide sequences of the embodiments are designed to have a low degree of internal repetitiveness in order to reduce or substantially eliminate immunogenicity when administered to a mammal. Polypeptide sequences composed of short, repeated motifs largely limited to only three amino acids, such as glycine, serine and glutamate, may result in relatively high antibody titers when administered to a mammal despite the absence of predicted T-cell epitopes in these sequences. This may be caused by the repetitive nature of polypeptides, as it has been shown that immunogens with repeated epitopes, including protein aggregates, cross-linked immunogens, and repetitive carbohydrates are highly immunogenic and can, for example, result in the cross-linking of B-cell receptors causing B-cell activation. (Johansson, J., et al. (2007) Vaccine, 25 :1676-82 ; Yankai, Z., et al. (2006) Biochem Biophys Res Commun, 345 :1365-71 ; Hsu, C. T., et al. (2000) Cancer Res, 60:3701-5); Bachmann MF, et al. Eur J Immunol. (1995) 25(12):3445-3451).

2. Exemplary Sequence Motifs

The present invention encompasses XTEN used as fusion partners that comprise multiple units of shorter sequences, or motifs, in which the amino acid sequences of the motifs are substantially non-repetitive. The non-repetitive property can be met even using a "building block" approach using a library of sequence motifs that are multimerized to create the XTEN sequences. While an XTEN sequence may consist of multiple units of as few as four different types of sequence motifs, because the motifs themselves generally consist of non-repetitive amino acid sequences, the overall XTEN sequence is designed to render the sequence substantially non-repetitive.

In one embodiment, an XTEN has a substantially non-repetitive sequence of greater than about 36 to about 3000, or about 100 to about 2000, or about 144 to about 1000 amino acid residues, or even longer wherein at least about 80%, or at least about 85%, or at least about 90%, or at least about 95%, or at least about 97%, or about 100% of the XTEN sequence consists of non-overlapping sequence motifs, and wherein each of the motifs has about 9 to 36 amino acid residues. As used herein, "non-overlapping" means that the individual motifs do not share amino acid residues but, rather, are linked to other motifs or amino acid residues in a linear fashion. In other embodiments, at least about 80%, or at least about 85%, or at least about 90%, or at least about 95%, or at least about 97%, or about 100% of the XTEN sequence consists of non-overlapping sequence motifs wherein each of the motifs has 9 to 14 amino acid residues. In still other embodiments, at least about 80%, or at least about 85%, or at least about 90%, or at least about 95%, or at least about 97%, or about 100% of the XTEN sequence consists of non-overlapping sequence motifs wherein each of the motifs has 12 amino acid residues. In these embodiments, it is preferred that the sequence motifs are composed of substantially (e.g., 90% or more) or exclusively small hydrophilic amino acids, such that the overall sequence has an unstructured, flexible characteristic. Examples of amino acids that are included in XTEN are, e.g., arginine, lysine, threonine, alanine, asparagine, glutamine, aspartate, glutamate, serine, and glycine. In one embodiment, XTEN sequences have predominately four to six types of amino acids selected from glycine (G), alanine (A), serine (S), threonine (T), glutamate (E) or proline (P) that are arranged in a substantially non-repetitive sequence that is greater than about 36 to about 3000, or about 100 to about 2000, or about 144 to about 1000 amino acid residues in length. In some embodiments, an XTEN sequence is made of 4, 5, or 6 types of amino acids selected from the group consisting of glycine (G), alanine (A), serine (S), threonine (T), glutamate (E) or proline (P). In some embodiments, XTEN have sequences of greater than about 36 to about 1000, or about 100 to about 2000, or about 400 to about 3000 amino acid residues wherein at least about 80% of the sequence consists of non-overlapping sequence motifs wherein each of the motifs has 9 to 36 amino acid residues and wherein at least 90%, or at least 91%, or at least 92%, or at least 93%, or at least 94%, or at least 95%, or at least 96%, or at least 97%, or 100% of each of the motifs consists of 4 to 6 types of amino acids selected from glycine (G), alanine (A), serine (S), threonine (T), glutamate (E) and proline (P), and wherein the content of any one amino acid type in the full-length XTEN does not exceed 30%. In other embodiments, at least about 90% of the XTEN sequence consists of non-overlapping sequence motifs wherein each of the motifs has 9 to 36 amino acid residues wherein the motifs consist of 4 to 6 types of amino acids selected from glycine (G), alanine (A), serine (S), threonine (T), glutamate (E) and proline (P), and wherein the content of any one amino acid type in the full-length XTEN does not exceed 40%, or about 30%, or about 25%. In other embodiments, at least about 90% of the XTEN sequence consists of non-overlapping sequence motifs wherein each of the motifs has 12 amino acid residues consisting of 4 to 6 types of amino acids selected from glycine (G), alanine (A), serine (S), threonine (T), glutamate (E) and proline (P), and wherein the content of any one amino acid type in the full-length XTEN does not exceed 40%, or 30%, or about 25%. In yet other embodiments, at least about 90%, or about 91%, or about 92%, or about 93%, or about 94%, or about 95%, or about 96%, or about 97%, or about 98%, or about 99%, to about 100% of the XTEN sequence consists of non-overlapping sequence motifs wherein each of the motifs has 12 amino acid residues consisting of glycine (G), alanine (A), serine (S), threonine (T), glutamate (E) and proline (P).

In still other embodiments, XTENs comprise substantially non-repetitive sequences of greater than about 36 to about 3000 amino acid residues wherein at least about 80%, or at least about 90%, or about 91%, or about 92%, or about 93%, or about 94%, or about 95%, or about 96%, or about 97%, or about 98%, or about 99% of the sequence consists of non-overlapping sequence motifs of 9 to 14 amino acid residues wherein the motifs consist of 4 to 6 types of amino acids selected from glycine (G), alanine (A), serine (S), threonine (T), glutamate (E) and proline (P), and wherein the sequence of any two contiguous amino acid residues in any one motif is not repeated more than twice in the sequence motif. In other embodiments, at least about 90%, or about 91%, or about 92%, or about 93%, or about 94%, or about 95%, or about 96%, or about 97%, or about 98%, or about 99% of an XTEN sequence consists of non-overlapping sequence motifs of 12 amino acid residues wherein the motifs consist of four to six types of amino acids selected from glycine (G), alanine (A), serine (S), threonine (T), glutamate (E) and proline (P), and wherein the sequence of any two contiguous amino acid residues in any one sequence motif is not repeated more than twice in the sequence motif. In other embodiments, at least about 90%, or about 91%, or about 92%, or about 93%, or about 94%, or about 95%, or about 96%, or about 97%, or about 98%, or about 99% of an XTEN sequence consists of non-overlapping sequence motifs of 12 amino acid residues wherein the motifs consist of glycine (G), alanine (A), serine (S), threonine (T), glutamate (E) and proline (P), and wherein the sequence of any two contiguous amino acid residues in any one sequence motif is not repeated more than twice in the sequence motif. In yet other embodiments, XTENs consist of 12 amino acid sequence motifs wherein the amino acids are selected from glycine (G), alanine (A), serine (S), threonine (T), glutamate (E) and proline (P), and wherein the sequence of any two contiguous amino acid residues in any one sequence motif is not repeated more than twice in the sequence motif, and wherein the content of any one amino acid type in the full-length XTEN does not exceed 30%. The foregoing embodiments are examples of substantially non-repetitive XTEN sequences. Additional examples are detailed below.

In some embodiments, the invention provides GLP2-XTEN compositions comprising one, or two, or three, or four, five, six or more non-repetitive XTEN sequence(s) of about 36 to about 1000 amino acid residues, or cumulatively about 100 to about 3000 amino acid residues wherein at least about 80%, or at least about 90%, or about 91%, or about 92%, or about 93%, or about 94%, or about 95%, or about 96%, or about 97%, or about 98%, or about 99% to about 100% of the sequence consists of multiple units of four or more non-overlapping sequence motifs selected from the amino acid sequences of Table 3, wherein the overall sequence remains substantially non-repetitive. In some embodiments, the XTEN comprises non-overlapping sequence motifs in which about 80%, or at least about 85%, or at least about 90%, or about 91%, or about 92%, or about 93%, or about 94%, or about 95%, or about 96%, or about 97%, or about 98%, or about 99% or about 100% of the sequence consists of multiple units of non-overlapping sequences selected from a single motif family selected from Table 3, resulting in a family sequence. Family as applied to motifs means that the XTEN has motifs selected from a motif category of Table 3; i.e., AD, AE, AF, AG, AM, AQ, BC, or BD, and that any other amino acids in the XTEN not from a motif family are selected to achieve a needed property, such as to permit incorporation of a restriction site by the encoding nucleotides, incorporation of a cleavage sequence, or to achieve a better linkage to a GLP-2 component of the GLP2-XTEN. In some embodiments of XTEN families, an XTEN sequence comprises multiple units of non-overlapping sequence motifs of the AD motif family, or of the AE motif family, or of the AF motif family, or of the AG motif family, or of the AM motif family, or of the AQ motif family, or of the BC family, or of the BD family, with the resulting XTEN exhibiting the range of homology described above. In other embodiments, of XTEN families, each XTEN of a given family has at least four different motifs of the same family from Table 3; e.g., four motifs of AD or AE or AF or AG or AM, etc. In other embodiments, the XTEN comprises multiple units of motif sequences from two or more of the motif families of Table 3, selected to achieve desired physicochemical characteristics, including such properties as net charge, lack of secondary structure, or lack of repetitiveness that may be conferred by the amino acid composition of the motifs, described more fully below. In the embodiments hereinabove described in this paragraph, the motifs or portions of the motifs incorporated into the XTEN can be selected and assembled using the methods described herein to achieve an XTEN of about 36, about 42, about 72, about 144, about 288, about 576, about 864, about 1000, about 2000 to about 3000 amino acid residues, or any intermediate length. Non-limiting examples of XTEN family sequences useful for incorporation into the subject GLP2-XTEN are presented in Table 4. It is intended that a specified sequence mentioned relative to Table 4 has that sequence set forth in Table 4, while a generalized reference to an AE144 sequence, for example, is intended to encompass any AE sequence having 144 amino acid residues; e.g., AE144_1A, AE144_2A, etc., or a generalized reference to an AG144 sequence, for example, is intended to encompass any AG sequence having 144 amino acid residues, e.g., AG144_1, AG144_2, AG144A, AG144_B, AG144_C, etc.

**Table 3: XTEN Sequence Motifs of 12 Amino Acids and Motif Families**

| Motif Family* | MOTIF SEQUENCE |
|---|---|
| AD | GESPGGSSGSES |
| AD | GSEGSSGPGESS |
| AD | GSSESGSSEGGP |

(continued)

| Motif Family* | MOTIF SEQUENCE |
|---|---|
| AD | GSGGEPSESGSS |
| AE, AM | GSPAGSPTSTEE |
| AE, AM, AQ | GSEPATSGSETP |
| AE, AM, AQ | GTSESATPESGP |
| AE, AM, AQ | GTSTEPSEGSAP |
| AF, AM | GSTSESPSGTAP |
| AF, AM | GTSTPESGSASP |
| AF, AM | GTSPSGESSTAP |
| AF, AM | GSTSSTAESPGP |
| AG, AM | GTPGSGTASSSP |
| AG, AM | GSSTPSGATGSP |
| AG, AM | GSSPSASTGTGP |
| AG, AM | GASPGTSSTGSP |
| AQ | GEPAGSPTSTSE |
| AQ | GTGEPSSTPASE |
| AQ | GSGPSTESAPTE |
| AQ | GSETPSGPSETA |
| AQ | GPSETSTSEPGA |
| AQ | GSPSEPTEGTSA |
| BC | GSGASEPTSTEP |
| BC | GSEPATSGTEPS |
| BC | GTSEPSTSEPGA |
| BC | GTSTEPSEPGSA |
| BD | GSTAGSETSTEA |
| BD | GSETATSGSETA |
| BD | GTSESATSESGA |
| BD | GTSTEASEGSAS |
| * Denotes individual motif sequences that, when used together in various permutations, results in a "family sequence" | |

**Table 4: XTEN Polypeptides**

| XTEN Name | Amino Acid Sequence |
|---|---|
| AE42 | GAPGSPAGSPTSTEEGTSESATPESGPGSEPATSGSETPASS |
| AE42_1 | TEPSEGSAPGSPAGSPTSTEEGTSESATPESGPGSEPATSGS |
| AE42_2 | PAGSPTSTEEGTSTEPSEGSAPGTSESATPESGPGSEPATSG |
| AE42_3 | SEPATSGSETPGTSESATPESGPGTSTEPSEGSAPGSPAGSP |
| AG42_1 | GAPSPSASTGTGPGTPGSGTASSSPGSSTPSGATGSPGPSGP |
| AG42_2 | GPGTPGSGTASSSPGSSTPSGATGSPGSSPSASTGTGPGASP |
| AG42_3 | SPSASTGTGPGASPGTSSTGSPGTPGSGTASSSPGSSTPSGA |
| AG42_4 | SASTGTGPGASPGTSSTGSPGTPGSGTASSSPGSSTPSGATG |
| AE48 | MAEPAGSPTSTEEGTPGSGTASSSPGSSTPSGATGSPGASPGTSSTGS |
| AM48 | MAEPAGSPTSTEEGASPGTSSTGSPGSSTPSGATGSPGSSTPSGATGS |
| AE144 | GSEPATSGSETPGTSESATPESGPGSEPATSGSETPGSPAGSPTSTEEGTSTEPSEGSAPGSEPA TSGSETPGSEPATSGSETPGSEPATSGSETPGTSTEPSEGSAPGTSESATPESGPGSEPATSGSET PGTSTEPSEGSAP |
| AE144_1A | SPAGSPTSTEEGTSESATPESGPGTSTEPSEGSAPGSPAGSPTSTEEGTSTEPSEGSAPGTSTEPS EGSAPGTSESATPESGPGSEPATSGSETPGSEPATSGSETPGSPAGSPTSTEEGTSESATPESGP GTSTEPSEGSAPG |
| AE144_2A | TSTEPSEGSAPGSPAGSPTSTEEGTSTEPSEGSAPGTSTEPSEGSAPGTSESATPESGPGTSTEPS EGSAPGTSESATPESGPGSEPATSGSETPGTSTEPSEGSAPGTSTEPSEGSAPGTSESATPESGP GTSESATPESGPG |
| AE144_2B | TSTEPSEGSAPGSPAGSPTSTEEGTSTEPSEGSAPGTSTEPSEGSAPGTSESATPESGPGTSTEPS EGSAPGTSESATPESGPGSEPATSGSETPGTSTEPSEGSAPGTSTEPSEGSAPGTSESATPESGP GTSESATPESGPG |
| AE144_3A | SPAGSPTSTEEGTSESATPESGPGSEPATSGSETPGTSESATPESGPGTSTEPSEGSAPGTSTEPS EGSAPGTSTEPSEGSAPGTSTEPSEGSAPGTSTEPSEGSAPGTSTEPSEGSAPGSPAGSPTSTEE GTSTEPSEGSAPG |
| AE144_3B | SPAGSPTSTEEGTSESATPESGPGSEPATSGSETPGTSESATPESGPGTSTEPSEGSAPGTSTEPS EGSAPGTSTEPSEGSAPGTSTEPSEGSAPGTSTEPSEGSAPGTSTEPSEGSAPGSPAGSPTSTEE GTSTEPSEGSAPG |

| XTEN Name | Amino Acid Sequence |
|---|---|
| AE144_4A | TSESATPESGPGSEPATSGSETPGTSESATPESGPGSEPATSGSETPGTSESATPESGPGTSTEPS EGSAPGTSESATPESGPGSPAGSPTSTEEGSPAGSPTSTEEGSPAGSPTSTEEGTSESATPESGP GTSTEPSEGSAPG |
| AE144_4B | TSESATPESGPGSEPATSGSETPGTSESATPESGPGSEPATSGSETPGTSESATPESGPGTSTEPS EGSAPGTSESATPESGPGSPAGSPTSTEEGSPAGSPTSTEEGSPAGSPTSTEEGTSESATPESGP GTSTEPSEGSAPG |
| AE144_5A | TSESATPESGPGSEPATSGSETPGTSESATPESGPGSEPATSGSETPGTSESATPESGPGTSTEPS EGSAPGSPAGSPTSTEEGTSESATPESGPGSEPATSGSETPGTSESATPESGPGSPAGSPTSTEE GSPAGSPTSTEEG |
| AE144_6B | TSTEPSEGSAPGTSESATPESGPGTSESATPESGPGTSESATPESGPGSEPATSGSETPGSEPATS GSETPGSPAGSPTSTEEGTSTEPSEGSAPGTSTEPSEGSAPGSEPATSGSETPGTSESATPESGP GTSTEPSEGSAPG |
| AF144 | GTSTPESGSASPGTSPSGESSTAPGTSPSGESSTAPGSTSSTAESPGPGSTSESPSGTAPGSTSST AESPGPGTSPSGESSTAPGTSTPESGSASPGSTSSTAESPGPGTSPSGESSTAPGTSPSGESSTAP GTSPSGESSTAP |
| AG144_1 | SGTASSSPGSSTPSGATGSPGTPGSGTASSSPGSSTPSGATGSPGSSTPSGATGSPGSSPSASTG TGPGSSPSASTGTGPGASPGTSSTGSPGTPGSGTASSSPGSSTPSGATGSPGSSPSASTGTGPGS SPSASTGTGPGASP |
| AG144_2 | PGSSPSASTGTGPGSSPSASTGTGPGTPGSGTASSSPGSSTPSGATGSPGSSPSASTGTGPGASP GTSSTGSPGTPGSGTASSSPGSSTPSGATGSPGTPGSGTASSSPGASPGTSSTGSPGASPGTSST GSPGTPGSGTASSS |
| AG144_A | GASPGTSSTGSPGSSPSASTGTGPGSSPSASTGTGPGTPGSGTASSSPGSSTPSGATGSPGSSPS ASTGTGPGASPGTSSTGSPGTPGSGTASSSPGSSTPSGATGSPGTPGSGTASSSPGASPGTSSTG SPGASPGTSSTGSP |
| AG144_B | GTPGSGTASSSPGSSTPSGATGSPGASPGTSSTGSPGTPGSGTASSSPGSSTPSGATGSPGSSPS ASTGTGPGSSPSASTGTGPGSSTPSGATGSPGSSTPSGATGSPGASPGTSSTGSPGASPGTSSTG SPGASPGTSSTGSP |
| AG144_C | GTPGSGTASSSPGASPGTSSTGSPGASPGTSSTGSPGASPGTSSTGSPGSSPSASTGTGPGTPGS GTASSSPGASPGTSSTGSPGASPGTSSTGSPGASPGTSSTGSPGSSTPSGATGSPGSSTPSGATG SPGASPGTSSTGSP |

(continued)

| XTEN Name | Amino Acid Sequence |
|---|---|
| AG144_F | GSSPSASTGTGPGSSPSASTGTGPGASPGTSSTGSPGASPGTSSTGSPGSSTPSGATGSPGSSPS ASTGTGPGASPGTSSTGSPGSSPSASTGTGPGTPGSGTASSSPGSSTPSGATGSPGSSTPSGATG SPGASPGTSSTGSP |
| AG144_3 | GTPGSGTASSSPGSSTPSGATGSPGSSTPSGATGSPGSSPSASTGTGPGSSPSASTGTGPGASPG TSSTGSPGTPGSGTASSSPGSSTPSGATGSPGSSPSASTGTGPGSSPSASTGTGPGASPGTSSTG SPGASPGTSSTGSP |
| AG144_4 | GTPGSGTASSSPGSSTPSGATGSPGSSPSASTGTGPGSSPSASTGTGPGASPGTSSTGSPGASPG |
|  | TSSTGSPGSSTPSGATGSPGSSPSASTGTGPGASPGTSSTGSPGSSPSASTGTGPGTPGSGTASS SPGSSTPSGATGSP |
| AE288_1 | GTSESATPESGPGSEPATSGSETPGTSESATPESGPGSEPATSGSETPGTSESATPESGPGTSTEP SEGSAPGSPAGSPTSTEEGTSESATPESGPGSEPATSGSETPGTSESATPESGPGSPAGSPTSTEE GSPAGSPTSTEEGTSTEPSEGSAPGTSESATPESGPGTSESATPESGPGTSESATPESGPGSEPA TSGSETPGSEPATSGSETPGSPAGSPTSTEEGTSTEPSEGSAPGTSTEPSEGSAPGSEPATSGSET PGTSESATPESGPGTSTEPSEGSAP |
| AE288_2 | GSPAGSPTSTEEGTSESATPESGPGSEPATSGSETPGTSESATPESGPGTSTEPSEGSAPGTSTEP SEGSAPGTSTEPSEGSAPGTSTEPSEGSAPGTSTEPSEGSAPGTSTEPSEGSAPGSPAGSPTSTEE GTSTEPSEGSAPGTSESATPESGPGSEPATSGSETPGTSESATPESGPGSEPATSGSETPGTSES ATPESGPGTSTEPSEGSAPGTSESATPESGPGSPAGSPTSTEEGSPAGSPTSTEEGSPAGSPTSTE EGTSESATPESGPGTSTEPSEGSAP |
| AG288_1 | PGASPGTSSTGSPGASPGTSSTGSPGTPGSGTASSSPGSSTPSGATGSPGTPGSGTASSSPGSST PSGATGSPGTPGSGTASSSPGSSTPSGATGSPGSSTPSGATGSPGSSPSASTGTGPGSSPSASTG TGPGASPGTSSTGSPGTPGSGTASSSPGSSTPSGATGSPGSSPSASTGTGPGSSPSASTGTGPGA SPGTSSTGSPGASPGTSSTGSPGSSTPSGATGSPGSSPSASTGTGPGASPGTSSTGSPGSSPSAST GTGPGTPGSGTASSSPGSSTPSGATGS |
| AG288_2 | GSSPSASTGTGPGSSPSASTGTGPGTPGSGTASSSPGSSTPSGATGSPGSSPSASTGTGPGASPG TSSTGSPGTPGSGTASSSPGSSTPSGATGSPGTPGSGTASSSPGASPGTSSTGSPGASPGTSSTG SPGTPGSGTASSSPGSSTPSGATGSPGASPGTSSTGSPGTPGSGTASSSPGSSTPSGATGSPGSS PSASTGTGPGSSPSASTGTGPGSSTPSGATGSPGSSTPSGATGSPGASPGTSSTGSPGASPGTSS TGSPGASPGTSSTGSPGTPGSGTASSSP |

(continued)

| XTEN Name | Amino Acid Sequence |
|---|---|
| AF504 | GASPGTSSTGSPGSSPSASTGTGPGSSPSASTGTGPGTPGSGTASSSPGSSTPSGATGSPGSXPS ASTGTGPGASPGTSSTGSPGTPGSGTASSSPGSSTPSGATGSPGTPGSGTASSSPGASPGTSSTG SPGASPGTSSTGSPGTPGSGTASSSPGSSTPSGATGSPGASPGTSSTGSPGTPGSGTASSSPGSS TPSGATGSPGSXPSASTGTGPGSSPSASTGTGPGSSTPSGATGSPGSSTPSGATGSPGASPGTSS TGSPGASPGTSSTGSPGASPGTSSTGSPGTPGSGTASSSPGASPGTSSTGSPGASPGTSSTGSPG ASPGTSSTGSPGSSPSASTGTGPGTPGSGTASSSPGASPGTSSTGSPGASPGTSSTGSPGASPGT SSTGSPGSSTPSGATGSPGSSTPSGATGSPGASPGTSSTGSPGTPGSGTASSSPGSSTPSGATGS PGSSTPSGATGSPGSSTPSGATGSPGSSPSASTGTGPGASPGTSSTGSP |
| AF540 | GSTSSTAESPGPGSTSSTAESPGPGSTSESPSGTAPGSTSSTAESPGPGSTSSTAESPGPGTSTPE SGSASPGSTSESPSGTAPGTSPSGESSTAPGSTSESPSGTAPGSTSESPSGTAPGTSPSGESSTAP GSTSESPSGTAPGSTSESPSGTAPGTSPSGESSTAPGSTSESPSGTAPGSTSESPSGTAPGSTSES PSGTAPGTSTPESGSASPGSTSESPSGTAPGTSTPESGSASPGSTSSTAESPGPGSTSSTAESPGP GTSTPESGSASPGTSTPESGSASPGSTSESPSGTAPGTSTPESGSASPGTSTPESGSASPGSTSES PSGTAPGSTSESPSGTAPGSTSESPSGTAPGSTSSTAESPGPGTSTPESGSASPGTSTPESGSASP GSTSESPSGTAPGSTSESPSGTAPGTSTPESGSASPGSTSESPSGTAPGSTSESPSGTAPGTSTPE SGSASPGTSPSGESSTAPGSTSSTAESPGPGTSPSGESSTAPGSTSSTAESPGPGTSTPESGSASP GSTSESPSGTAP |
| AD576 | GSSESGSSEGGPGSGGEPSESGSSGSSESGSSEGGPGSSESGSSEGGPGSSESGSSEGGPGSSES GSSEGGPGSSESGSSEGGPGESPGGSSGSESGSEGSSGPGESSGSSESGSSEGGPGSSESGSSEG GPGSSESGSSEGGPGSGGEPSESGSSGESPGGSSGSESGESPGGSSGSESGSGGEPSESGSSGSS ESGSSEGGPGSGGEPSESGSSGSGGEPSESGSSGSEGSSGPGESSGESPGGSSGSESGSGGEPSE SGSSGSGGEPSESGSSGSSESGSSEGGPGESPGGSSGSESGESPGGSSGSESG ESPGGSSGSESGESPGGSSGSESGESPGGSSGSESGSSESGSSEGGPGSGGEPSESGSSGSEGSS GPGESSGSSESGSSEGGPGSGGEPSESGSSGSSESGSSEGGPGSGGEPSESGSSGESPGGSSGSE SGESPGGSSGSESGSSESGSSEGGPGSGGEPSESGSSGSSESGSSEGGPGSGGEPSESGSSGSGG EPSESGSSGESPGGSSGSESGSEGSSGPGESSGSSESGSSEGGPGSEGSSGPGESS |
| AE576 | GSPAGSPTSTEEGTSESATPESGPGTSTEPSEGSAPGSPAGSPTSTEEGTSTEPSEGSAPGTSTEP SEGSAPGTSESATPESGPGSEPATSGSETPGSEPATSGSETPGSPAGSPTSTEEGTSESATPESGP GTSTEPSEGSAPGTSTEPSEGSAPGSPAGSPTSTEEGTSTEPSEGSAPGTSTEPSEGSAPGTSES ATPESGPGTSTEPSEGSAPGTSESATPESGPGSEPATSGSETPGTSTEPSEGSAPGTSTEPSEGS APGTSESATPESGPGTSESATPESGPGSPAGSPTSTEEGTSESATPESGPGSEPATSGSETPGTS ESATPESGPGTSTEPSEGSAPGTSTEPSEGSAPGTSTEPSEGSAPGTSTEPSEGSAPGTSTEPSEG SAPGTSTEPSEGSAPGSPAGSPTSTEEGTSTEPSEGSAPGTSESATPESGPGSEPATSGSETPGTS ESATPESGPGSEPATSGSETPGTSESATPESGPGTSTEPSEGSAPGTSESATPESGPGSPAGSPTS TEEGSPAGSPTSTEEGSPAGSPTSTEEGTSESATPESGPGTSTEPSEGSAP |

| XTEN Name | Amino Acid Sequence |
|---|---|
| AF576 | GSTSSTAESPGPGSTSSTAESPGPGSTSESPSGTAPGSTSSTAESPGPGSTSSTAESPGPGTSTPE SGSASPGSTSESPSGTAPGTSPSGESSTAPGSTSESPSGTAPGSTSESPSGTAPGTSPSGESSTAP |
|  | GSTSESPSGTAPGSTSESPSGTAPGTSPSGESSTAPGSTSESPSGTAPGSTSESPSGTAPGSTSES PSGTAPGTSTPESGSASPGSTSESPSGTAPGTSTPESGSASPGSTSSTAESPGPGSTSSTAESPGP GTSTPESGSASPGTSTPESGSASPGSTSESPSGTAPGTSTPESGSASPGTSTPESGSASPGSTSES PSGTAPGSTSESPSGTAPGSTSESPSGTAPGSTSSTAESPGPGTSTPESGSASPGTSTPESGSASP GSTSESPSGTAPGSTSESPSGTAPGTSTPESGSASPGSTSESPSGTAPGSTSESPSGTAPGTSTPE SGSASPGTSPSGESSTAPGSTSSTAESPGPGTSPSGESSTAPGSTSSTAESPGPGTSTPESGSASP GSTSESPSGTAPGSTSSTAESPGPGTSTPESGSASPGTSTPESGSASP |
| AG576 | PGTPGSGTASSSPGSSTPSGATGSPGSSPSASTGTGPGSSPSASTGTGPGSSTPSGATGSPGSST PSGATGSPGASPGTSSTGSPGASPGTSSTGSPGASPGTSSTGSPGTPGSGTASSSPGASPGTSS TGSPGASPGTSSTGSPGASPGTSSTGSPGSSPSASTGTGPGTPGSGTASSSPGASPGTSSTGSP GASPGTSSTGSPGASPGTSSTGSPGSSTPSGATGSPGSSTPSGATGSPGASPGTSSTGSPGTPG SGTASSSPGSSTPSGATGSPGSSTPSGATGSPGSSTPSGATGSPGSSPSASTGTGPGASPGTSST GSPGASPGTSSTGSPGTPGSGTASSSPGASPGTSSTGSPGASPGTSSTGSPGASPGTSSTGSPG ASPGTSSTGSPGTPGSGTASSSPGSSTPSGATGSPGTPGSGTASSSPGSSTPSGATGSPGTPGS GTASSSPGSSTPSGATGSPGSSTPSGATGSPGSSPSASTGTGPGSSPSASTGTGPGASPGTSST GSPGTPGSGTASSSPGSSTPSGATGSPGSSPSASTGTGPGSSPSASTGTGPGASPGTSSTGS |
| AE624 | MAEPAGSPTSTEEGTPGSGTASSSPGSSTPSGATGSPGASPGTSSTGSPGSPAGSPTSTEEGTS ESATPESGPGTSTEPSEGSAPGSPAGSPTSTEEGTSTEPSEGSAPGTSTEPSEGSAPGTSESATP ESGPGSEPATSGSETPGSEPATSGSETPGSPAGSPTSTEEGTSESATPESGPGTSTEPSEGSAPG TSTEPSEGSAPGSPAGSPTSTEEGTSTEPSEGSAPGTSTEPSEGSAPGTSESATPESGPGTSTEP SEGSAPGTSESATPESGPGSEPATSGSETPGTSTEPSEGSAPGTSTEPSEGSAPGTSESATPESG PGTSESATPESGPGSPAGSPTSTEEGTSESATPESGPGSEPATSGSETPGTSESATPESGPGTST EPSEGSAPGTSTEPSEGSAPGTSTEPSEGSAPGTSTEPSEGSAPGTSTEPSEGSAPGTSTEPSEG SAPGSPAGSPTSTEEGTSTEPSEGSAPGTSESATPESGPGSEPATSGSETPGTSESATPESGPGS EPATSGSETPGTSESATPESGPGTSTEPSEGSAPGTSESATPESGPGSPAGSPTSTEEGSPAGSP TSTEEGSPAGSPTSTEEGTSESATPESGPGTSTEPSEGSAP |

EP 3 431 098 A1

| XTEN Name | Amino Acid Sequence |
|---|---|
| AD836 | GSSESGSSEGGPGSSESGSSEGGPGESPGGSSGSESGSGGEPSESGSSGESPGGSSGSESGESP GGSSGSESGSSESGSSEGGPGSSESGSSEGGPGSSESGSSEGGPGESPGGSSGSESGESPGGSS GSESGESPGGSSGSESGSSESGSSEGGPGSSESGSSEGGPGSSESGSSEGGPGSSESGSSEGGP GSSESGSSEGGPGSSESGSSEGGPGSGGEPSESGSSGESPGGSSGSESGESPGGSSGSESGSGG EPSESGSSGSEGSSGPGESSGSSESGSSEGGPGSGGEPSESGSSGSEGSSGPGESSGSSESGSSE GGPGSGGEPSESGSSGESPGGSSGSESGSGGEPSESGSSGSGGEPSESGSSGSSESGSSEGGPG SGGEPSESGSSGSGGEPSESGSSGSEGSSGPGESSGESPGGSSGSESGSEGSSGPGESSGSEGSS GPGESSGSGGEPSESGSSGSSESGSSEGGPGSSESGSSEGGPGESPGGSSGSESGSGGEPSESG SSGSEGSSGPGESSGESPGGSSGSESGSEGSSGPGSSESGSSEGGPGSGGEPSESGSSGSEGSSG PGESSGSEGSSGPGESSGSEGSSGPGESSGSGGEPSESGSSGSGGEPSESGSSGESPGGSSGSES GESPGGSSGSESGSGGEPSESGSSGSEGSSGPGESSGESPGGSSGSESGSSESGSSEGGPGSSES GSSEGGPGSSESGSSEGGPGSGGEPSESGSSGSSESGSSEGGPGESPGGSSGSESGSGGEPSES GSSGSSESGSSEGGPGESPGGSSGSESGSGGEPSESGSSGESPGGSSGSESGSGGEPSESGSS |
| AE864 | GSPAGSPTSTEEGTSESATPESGPGTSTEPSEGSAPGSPAGSPTSTEEGTSTEPSEGSAPGTSTE PSEGSAPGTSESATPESGPGSEPATSGSETPGSEPATSGSETPGSPAGSPTSTEEGTSESATPES GPGTSTEPSEGSAPGTSTEPSEGSAPGSPAGSPTSTEEGTSTEPSEGSAPGTSTEPSEGSAPGTS ESATPESGPGTSTEPSEGSAPGTSESATPESGPGSEPATSGSETPGTSTEPSEGSAPGTSTEPSE GSAPGTSESATPESGPGTSESATPESGPGSPAGSPTSTEEGTSESATPESGPGSEPATSGSETPG TSESATPESGPGTSTEPSEGSAPGTSTEPSEGSAPGTSTEPSEGSAPGTSTEPSEGSAPGTSTEP SEGSAPGTSTEPSEGSAPGSPAGSPTSTEEGTSTEPSEGSAPGTSESATPESGPGSEPATSGSET PGTSESATPESGPGSEPATSGSETPGTSESATPESGPGTSTEPSEGSAPGTSESATPESGPGSPA GSPTSTEEGSPAGSPTSTEEGSPAGSPTSTEEGTSESATPESGPGTSTEPSEGSAPGTSESATPE SGPGSEPATSGSETPGTSESATPESGPGSEPATSGSETPGTSESATPESGPGTSTEPSEGSAPGS PAGSPTSTEEGTSESATPESGPGSEPATSGSETPGTSESATPESGPGSPAGSPTSTEEGSPAGSP TSTEEGTSTEPSEGSAPGTSESATPESGPGTSESATPESGPGTSESATPESGPGSEPATSGSETP GSEPATSGSETPGSPAGSPTSTEEGTSTEPSEGSAPGTSTEPSEGSAPGSEPATSGSETPGTSES ATPESGPGTSTEPSEGSAP |
| AF864 | GSTSESPSGTAPGTSPSGESSTAPGSTSESPSGTAPGSTSESPSGTAPGTSTPESGSASPGTSTPE SGSASPGSTSESPSGTAPGSTSESPSGTAPGTSPSGESSTAPGSTSESPSGTAPGTSPSGESSTAP GTSPSGESSTAPGSTSSTAESPGPGTSPSGESSTAPGTSPSGESSTAPGSTSSTAESPGPGTSTPE SGSASPGTSTPESGSASPGSTSESPSGTAPGSTSESPSGTAPGTSTPESGSASPGSTSSTAESPGP GTSTPESGSASPGSTSESPSGTAPGTSPSGESSTAPGSTSSTAESPGPGTSPSGESSTAPGTSTPE SGSASPGSTSSTAESPGPGSTSSTAESPGPGSTSSTAESPGPGSTSSTAESPGPGTSPSGESSTAP |

EP 3 431 098 A1

| XTEN Name | Amino Acid Sequence |
|---|---|
|  | GSTSESPSGTAPGSTSESPSGTAPGTSTPESGPXXXGASASGAPSTXXXXSESPSGTAPGSTSE SPSGTAPGSTSESPSGTAPGSTSESPSGTAPGSTSESPSGTAPGSTSESPSGTAPGTSTPESGSA SPGTSPSGESSTAPGTSPSGESSTAPGSTSSTAESPGPGTSPSGESSTAPGTSTPESGSASPGSTS ESPSGTAPGSTSESPSGTAPGTSPSGESSTAPGSTSESPSGTAPGTSTPESGSASPGTSTPESGS ASPGTSESPSGTAPGTSTPESGSASPGSTSSTAESPGPGTSESPSGTAPGSTSESPSGTAPGT SPSGESSTAPGSTSSTAESPGPGTSPSGESSTAPGTSTPESGSASPGTSPSGESSTAPGTSPSGES STAPGTSPSGESSTAPGSTSSTAESPGPGTSSTAESPGPGTSPSGESSTAPGSSPSASTGTGPG SSTPSGATGSPGSSTPSGATGSP |
| AG864_2 | GASPGTSSTGSPGSSPSASTGTGPGSSPSASTGTGPGTPGSGTASSSPGSSTPSGATGSPGSSPS ASTGTGPGASPGTSSTGSPGTPGSGTASSSPGSSTPSGATGSPGTPGSGTASSSPGASPGTSST GSPGASPGTSSTGSPGTPGSGTASSSPGSSTPSGATGSPGASPGTSSTGSPGTPGSGTASSSPGS STPSGATGSPGSSPSASTGTGPGSSPSASTGTGPGSSTPSGATGSPGSSTPSGATGSPGASPGT SSTGSPGASPGTSSTGSPGASPGTSSTGSPGTPGSGTASSSPGASPGTSSTGSPGASPGTSSTGS PGASPGTSSTGSPGSSPSASTGTGPGTPGSGTASSSPGASPGTSSTGSPGASPGTSSTGSPGASP GTSSTGSPGSSTPSGATGSPGSSTPSGATGSPGASPGTSSTGSPGTPGSGTASSSPGSSTPSGAT GSPGSSTPSGATGSPGSSTPSGATGSPGSSPSASTGTGPGASPGTSSTGSPGASPGTSSTGSPG TPGSGTASSSPGASPGTSSTGSPGASPGTSSTGSPGASPGTSSTGSPGASPGTSSTGSPGTPGS GTASSSPGSSTPSGATGSPGTPGSGTASSSPGSSTPSGATGSPGTPGSGTASSSPGSSTPSGAT GSPGSSTPSGATGSPGSSPSASTGTGPGSSPSASTGTGPGASPGTSSTGSPGTPGSGTASSSPGS STPSGATGSPGSSPSASTGTGPGSSPSASTGTGPGASPGTSSTGSPGASPGTSSTGSPGSSTPSG ATGSPGSSPSASTGTGPGASPGTSSTGSPGSSPSASTGTGPGTPGSGTASSSPGSSTPSGATGS PGSSTPSGATGSPGASPGTSSTGSP |
| AM875 | GTSTEPSEGSAPGSEPATSGSETPGSPAGSPTSTEEGSTSSTAESPGPGTSTPESGSASPGSTSE SPSGTAPGSTSESPSGTAPGTSTPESGSASPGTSTPESGSASPGSEPATSGSETPGTSESATPES GPGSPAGSPTSTEEGTSTEPSEGSAPGTSESATPESGPGTSTEPSEGSAPGTSTEPSEGSAPGSP AGSPTSTEEGTSTEPSEGSAPGTSTEPSEGSAPGTSESATPESGPGTSESATPESGPGTSTEPSE GSAPGTSTEPSEGSAPGTSESATPESGPGTSTEPSEGSAPGSEPATSGSETPGSPAGSPTSTEEG SSTPSGATGSPGTPGSGTASSSPGSSTPSGATGSPGTSTEPSEGSAPGTSTEPSEGSAPGSEPAT SGSETPGSPAGSPTSTEEGSPAGSPTSTEEGTSTEPSEGSAPGASASGAPSTGGTSESATPESGP GSPAGSPTSTEEGSPAGSPTSTEEGTSSTAESPGPGTSESPSGTAPGTSPSGESSTAPGTPGS GTASSSPGSSTPSGATGSPGSSPSASTGTGPGSEPATSGSETPGTSESATPESGPGSEPATSGSE TPGSTSSTAESPGPGTSSTAESPGPGTSPSGESSTAPGSEPATSGSETPGSEPATSGSETPGTS TEPSEGSAPGSTSSTAESPGPGTSTPESGSASPGSTSESPSGTAPGTSTEPSEGSAPGTSTEPSE GSAPGTSTEPSEGSAPGSSTPSGATGSPGSSPSASTGTGPGASPGTSSTGSPGSEPATSGSETP GTSESATPESGPGSPAGSPTSTEEGSSTPSGATGSPGSSPSASTGTGPGASPGTSSTGSPGTSES ATPESGPGTSTEPSEGSAPGTSTEPSEGSAP |

EP 3 431 098 A1

(continued)

| XTEN Name | Amino Acid Sequence |
|---|---|
| AE912 | MAEPAGSPTSTEEGTPGSGTASSSPGSSTPSGATGSPGASPGTSSTGSPGSPAGSPTSTEEGTS ESATPESGPGTSTEPSEGSAPGSPAGSPTSTEEGTSTEPSEGSAPGTSTEPSEGSAPGTSESATP ESGPGSEPATSGSETPGSEPATSGSETPGSPAGSPTSTEEGTSESATPESGPGTSTEPSEGSAPG TSTEPSEGSAPGSPAGSPTSTEEGTSTEPSEGSAPGTSTEPSEGSAPGTSESATPESGPGTSTEP SEGSAPGTSESATPESGPGSEPATSGSETPGTSTEPSEGSAPGTSTEPSEGSAPGTSESATPESG PGTSESATPESGPGSPAGSPTSTEEGTSESATPESGPGSEPATSGSETPGTSESATPESGPGTST EPSEGSAPGTSTEPSEGSAPGTSTEPSEGSAPGTSTEPSEGSAPGTSTEPSEGSAPGTSTEPSEG SAPGSPAGSPTSTEEGTSTEPSEGSAPGTSESATPESGPGSEPATSGSETPGTSESATPESGPGS EPATSGSETPGTSESATPESGPGTSTEPSEGSAPGTSESATPESGPGSPAGSPTSTEEGSPAGSP TSTEEGSPAGSPTSTEEGTSESATPESGPGTSTEPSEGSAPGTSESATPESGPGSEPATSGSETP GTSESATPESGPGSEPATSGSETPGTSESATPESGPGTSTEPSEGSAPGSPAGSPTSTEEGTSES ATPESGPGSEPATSGSETPGTSESATPESGPGSPAGSPTSTEEGSPAGSPTSTEEGTSTEPSEGS APGTSESATPESGPGTSESATPESGPGTSESATPESGPGSEPATSGSETPGSEPATSGSETPGSP AGSPTSTEEGTSTEPSEGSAPGTSTEPSEGSAPGSEPATSGSETPGTSESATPESGPGTSTEPSE GSAP |
| AM923 | MAEPAGSPTSTEEGASPGTSSTGSPGSSTPSGATGSPGSSTPSGATGSPGTSTEPSEGSAPGSE PATSGSETPGSPAGSPTSTEEGTSSTAESPGPGTSTPESGSASPGSTSESPSGTAPGSTSESPSG TAPGTSTPESGSASPGTSTPESGSASPGSEPATSGSETPGTSESATPESGPGSPAGSPTSTEEGT STEPSEGSAPGTSESATPESGPGTSTEPSEGSAPGTSTEPSEGSAPGSPAGSPTSTEEGTSTEPS EGSAPGTSTEPSEGSAPGTSESATPESGPGTSESATPESGPGTSTEPSEGSAPGTSTEPSEGSAP GTSESATPESGPGTSTEPSEGSAPGSEPATSGSETPGSPAGSPTSTEEGSSTPSGATGSPGTPGS GTASSSPGSSTPSGATGSPGTSTEPSEGSAPGTSTEPSEGSAPGSEPATSGSETPGSPAGSPTST EEGSPAGSPTSTEEGTSTEPSEGSAPGASASGAPSTGGTSESATPESGPGSPAGSPTSTEEGSP |
|  | AGSPTSTEEGTSSTAESPGPGSTSESPSGTAPGTSPSGESSTAPGTPGSTASSSPGSSTPSGA TGSPGSSPSASTGTGPGSEPATSGSETPGTSESATPESGPGSEPATSGSETPGSTSSTAESPGPG STSSTAESPGPGTSPSGESSTAPGSEPATSGSETPGSEPATSGSETPGTSTEPSEGSAPGSTSST AESPGPGTSTPESGSASPGSTSESPSGTAPGTSTEPSEGSAPGTSTEPSEGSAPGTSTEPSEGSA PGSSTPSGATGSPGSSPSASTGTGPGASPGTSSTGSPGSEPATSGSETPGTSESATPESGPGSPA GSPTSTEEGSSTPSGATGSPGSSPSASTGTGPGASPGTSSTGSPGTSESATPESGPGTSTEPSEG SAPGTSTEPSEGSAP |

| XTEN Name | Amino Acid Sequence |
|---|---|
| AM1318 | GTSTEPSEGSAPGSEPATSGSETPGSPAGSPTSTEEGSTSSTAESPGPGTSTPESGSASPGSTSESPSGTAPGSTSESPSGTAPGTSTPESGSASPGTSTPESGSASPGSEPATSGSETPGTSESATPESGPGSPAGSPTSTEEGTSTEPSEGSAPGTSESATPESGPGTSTEPSEGSAPGTSTEPSEGSAPGSPAGSPTSTEEGTSTEPSEGSAPGTSTEPSEGSAPGTSESATPESGPGTSESATPESGPGTSTEPSEGSAPGTSTEPSEGSAPGTSESATPESGPGTSTEPSEGSAPGSEPATSGSETPGSPAGSPTSTEEGSSTPSGATGSPGTPGSGTASSSPGSSTPSGATGSPGTSTEPSEGSAPGTSTEPSEGSAPGSEPATSGSETPGSPAGSPTSTEEGSPAGSPTSTEEGTSTEPSEGSAPGPEPTGPAPSGGSEPATSGSETPGTSESATPESGPGSPAGSPTSTEEGTSESATPESGPGSPAGSPTSTEEGSPAGSPTSTEEGTSESATPESGPGSPAGSPTSTEEGSPAGSPTSTEEGSTSSTAESPGPGTSESPSGTAPGTSPSGESSTAPGSTSESPSGTAPGTSESPSGTAPGTSPSGESSTAPGTSTEPSEGSAPGTSESATPESGPGTSESATPESGPGSEPATSGSETPGTSESATPESGPGTSESATPESGPGTSTEPSEGSAPGTSESATPESGPGTSTEPSEGSAPGTSPSGESSTAPGTSPSGESSTAPGTSPSGESSTAPGTSTEPSEGSAPGSPAGSPTSTEEGTSTEPSEGSAPGSSPSASTGTGPGSSTPSGATGSPGSSTPSGATGSPGSSTPSGATGSPGSSTPSGATGSPGASPGTSSTGSPGASASGAPSTGGTSPSGESSTAPGSTSSTAESPGPGTSPSGESSTAPGTSESATPESGPGTSTEPSEGSAPGTSTEPSEGSAPGSSPSASTGTGPGSSTPSGATGSPGASPGTSSTGSPGTSTPESGSASPGTSPSGESSTAPGTSPSGESSTAPGTSESATPESGPGSEPATSGSETPGTSTEPSEGSAPGSTSESPSGTAPGSTSESPSGTAPGTSTPESGSASPGSPAGSPTSTEEGTSESATPESGPGTSTEPSEGSAPGSPAGSPTSTEEGTSESATPESGPGSEPATSGSETPGSSTPSGATGSPGASPGTSSTGSPGSSTPSGATGSPGTSESPSGTAPGTSPSGESSTAPGSTSSTAESPGPGSSTPSGATGSPGASPGTSSTGSPGTPGSGTASSSPGSPAGSPTSTEEGSPAGSPTSTEEGTSTEPSEGSAP |
| BC 864 | GTSTEPSEPGSAGTSTEPSEPGSAGSEPATSGTEPSGSGASEPTSTEPGSEPATSGTEPSGSEPATSGTEPSGSEPATSGTEPSGSGASEPTSTEPGTSTEPSEPGSAGSEPATSGTEPSGTSTEPSEPGSAGSEPATSGTEPSGSEPATSGTEPSGTSTEPSEPGSAGTSTEPSEPGSAGSEPATSGTEPSGSEPATSGTEPSGTSEPSTSEPGAGSGASEPTSTEPGTSEPSTSEPGAGSEPATSGTEPSGSEPATSGTEPSGTSTEPSEPGSAGTSTEPSEPGSAGSGASEPTSTEPGSEPATSGTEPSGSEPATSGTEPSGSEPATSGTEPSGSEPATSGTEPSGTSTEPSEPGSAGSEPATSGTEPSGSGASEPTSTEPGTSTEPSEPGSAGSEPATSGTEPSGSGASEPTSTEPGTSTEPSEPGSAGSGASEPTSTEPGSEPATSGTEPSGSGASEPTSTEPGTSTEPSEPGSAGSEPATSGTEPSGSGASEPTSTEPGTSTEPSEPGSAGSEPATSGTEPSGTSTEPSEPGSAGSEPATSGTEPSGTSTEPSEPGSAGTSTEPSEPGSAGTSTEPSEPGSAGTSTEPSEPGSAGTSTEPSEPGSAGTSTEPSEPGSAGTSEPSTSEPGAGSGASEPTSTEPGTSTEPSEPGSAGTSTEPSEPGSAGTSTEPSEPGSAGSEPATSGTEPSGSGASEPTSTEPGSEPATSGTEPSGSEPATSGTEPSGSEPATSGTEPSGSEPATSGTEPSGTSEPSTSEPGAGSEPATSGTEPSGSGASEPTSTEPGTSTEPSEPGSAGSEPATSGTEPSGSGASEPTSTEPGTSTEPSEPGSA |

(continued)

| XTEN Name | Amino Acid Sequence |
|---|---|
| BD864 | GSETATSGSETAGTSESATSESGAGSTAGSETSTEAGTSESATSESGAGSETATSGSETAGSE TATSGSETAGTSTEASEGSASGTSTEASEGSASGTSESATSESGAGSETATSGSETAGTSTEA SEGSASGSTAGSETSTEAGTSESATSESGAGTSESATSESGAGSETATSGSETAGTSESATSES GAGTSTEASEGSASGSETATSGSETAGSETATSGSETAGTSTEASEGSASGSTAGSETSTEAG TSESATSESGAGTSTEASEGSASGSETATSGSETAGSTAGSETSTEAGTAGSETSTEAGSET ATSGSETAGTSESATSESGAGTSESATSESGAGSETATSGSETAGTSESATSESGAGTSESATS ESGAGSETATSGSETAGSETATSGSETAGTSTEASEGSASGSTAGSETSTEAGSETATSGSET AGTSESATSESGAGSTAGSETSTEAGSTAGSETSTEAGSTAGSETSTEAGTSTEASEGSASGS TAGSETSTEAGSTAGSETSTEAGTSTEASEGSASGSTAGSETSTEAGSETATSGSETAGTSTE ASEGSASGTSESATSESGAGSETATSGSETAGTSESATSESGAGTSESATSESGAGSETATSG SETAGTSESATSESGAGSETATSGSETAGTSTEASEGSASGTSTEASEGSASGSTAGSETSTE AGSTAGSETSTEAGSETATSGSETAGTSESATSESGAGTSESATSESGAGSETATSGSETAGS ETATSGSETAGSETATSGSETAGTSTEASEGSASGTSESATSESGAGSETATSGSETAGSETA TSGSETAGTSESATSESGAGTSESATSESGAGSETATSGSETA |
| AE948 | GTSTEPSEGSAPGTSESATPESGPGSEPATSGSETPGTSESATPESGPGTSTEPSEGSAPGSPAG SPTSTEEGTSTEPSEGSAPGSEPATSGSETPGTSESATPESGPGTSESATPESGPGSEPATSGSE TPGTSTEPSEGSAPGTSESATPESGPGTSTEPSEGSAPGTSTEPSEGSAPGSEPATSGSETPGTS |
|  | TEPSEGSAPGSEPATSGSETPGSEPATSGSETPGTSTEPSEGSAPGSEPATSGSETPGSEPATSG SETPGTSTEPSEGSAPGSEPATSGSETPGSPAGSPTSTEEGTSESATPESGPGTSTEPSEGSAPG SEPATSGSETPGTSESATPESGPGSEPATSGSETPGSEPATSGSETPGTSESATPESGPGSPAGS PTSTEEGTSESATPESGPGSPAGSPTSTEEGTSTEPSEGSAPGTSESATPESGPGTSTEPSEGSA PGTSTEPSEGSAPGSPAGSPTSTEEGTSTEPSEGSAPGTSESATPESGPGSPAGSPTSTEEGSPA GSPTSTEEGTSESATPESGPGTSESATPESGPGTSTEPSEGSAPGSPAGSPTSTEEGTSESATPE SGPGTSTEPSEGSAPGSEPATSGSETPGSPAGSPTSTEEGTSESATPESGPGTSTEPSEGSAPGS EPATSGSETPGSEPATSGSETPGSEPATSGSETPGSPAGSPTSTEEGTSESATPESGPGSEPATS GSETPGSEPATSGSETPGSPAGSPTSTEEGSPAGSPTSTEEGSPAGSPTSTEEGTSESATPESGP GSEPATSGSETPGTSTEPSEGSAPGTSTEPSEGSAPGSPAGSPTSTEEGSPAGSPTSTEEGSPAG SPTSTEEGTSTEPSEGSAPGTSESATPESGPGTSESATPESGPGTSTEPSEGSAPGTSTEPSEGS APGTSTEPSEGSAPGTSESATPESGPGTSESATPESGP |

| XTEN Name | Amino Acid Sequence |
|---|---|
| AE1044 | GSEPATSGSETPGSEPATSGSETPGTSTEPSEGSAPGTSESATPESGPGSPAGSPTSTEEGTSTEPSEGSAPGTSESATPESGPGTSESATPESGPGSEPATSGSETPGSEPATSGSETPGTSESATPESGPGTSESATPESGPGSPAGSPTSTEEGTSTEPSEGSAPGSEPATSGSETPGTSTEPSEGSAPGSPAGSPTSTEEGSPAGSPTSTEEGSPAGSPTSTEEGTSTEPSEGSAPGTSESATPESGPGSEPATSGSETPGTSESATPESGPGTSESATPESGPGSPAGSPTSTEEGTSTEPSEGSAPGSEPATSGSETPGTSTEPSEGSAPGTSESATPESGPGSPAGSPTSTEEGTSTEPSEGSAPGTSTEPSEGSAPGSPAGSPTSTEEGTSTEPSEGSAPGTSESATPESGPGSPAGSPTSTEEGTSESATPESGPGSEPATSGSETPGTSTEPSEGSAPGSEPATSGSETPGTSESATPESGPGTSTEPSEGSAPGTSESATPESGPGTSTEPSEGSAPGTSTEPSEGSAPGSEPATSGSETPGSPAGSPTSTEEGTSESATPESGPGTSTEPSEGSAPGSPAGSPTSTEEGTSTEPSEGSAPGTSESATPESGPGTSTEPSEGSAPGSEPATSGSETPGSEPATSGSETPGTSESATPESGPGTSESATPESGPGTSESATPESGPGTSTEPSEGSAPGTSTEPSEGSAPGSPAGSPTSTEEGSPAGSPTSTEEGTSESATPESGPGSPAGSPTSTEEGSPAGSPTSTEEGTSESATPESGPGSEPATSGSETPGTSTEPSEGSAPGSEPATSGSETPGSPAGSPTSTEEGTSESATPESGPGTSESATPESGPGSEPATSGSETPGTSTEPSEGSAPGTSTEPSEGSAPGSPAGSPTSTEEGTSTEPSEGSAPGTSTEPSEGSAPGSEPATSGSETPGSPAGSPTSTEEGTSESATPESGPGTSTEPSEGSAPGSPAGSPTSTEEGTSESATPESGPGTSESATPESGPGTST |
| AE1140 | GSEPATSGSETPGSEPATSGSETPGTSTEPSEGSAPGSEPATSGSETPGTSESATPESGPGSEPATSGSETPGSPAGSPTSTEEGTSESATPESGPGSEPATSGSETPGTSTEPSEGSAPGTSESATPESGPGTSESATPESGPGTSTEPSEGSAPGTSTEPSEGSAPGSEPATSGSETPGSPAGSPTSTEEGTSTEPSEGSAPGSEPATSGSETPGSPAGSPTSTEEGTSTEPSEGSAPGTSESATPESGPGSPAGSPTSTEEGTSTEPSEGSAPGTSESATPESGPGSEPATSGSETPGTSESATPESGPGSEPATSGSETPGTSTEPSEGSAPGTSTEPSEGSAPGTSTEPSEGSAPGSPAGSPTSTEEGSPAGSPTSTEEGTSESATPESGPGTSTEPSEGSAPGTSTEPSEGSAPGSPAGSPTSTEEGSPAGSPTSTEEGSPAGSPTSTEEGTSESATPESGPGSEPATSGSETPGTSESATPESGPGSPAGSPTSTEEGTSTEPSEGSAPGTSESATPESGPGSEPATSGSETPGSPAGSPTSTEEGTSTEPSEGSAPGTSESATPESGPGTSESATPESGPGSPAGSPTSTEEGTSTEPSEGSAPGSPAGSPTSTEEGSPAGSPTSTEEGTSESATPESGPGSEPATSGSETPGTSTEPSEGSAPGSEPATSGSETPGTSESATPESGPGTSTEPSEGSAPGTSESATPESGPGTSESATPESGPGTSTEPSEGSAPGTSTEPSEGSAPGSEPATSGSETPGTSESATPESGPGTSTEPSEGSAPGSEPATSGSETPGSPAGSPTSTEEGTSESATPESGPGSPAGSPTSTEEGTSTEPSEGSAPGSEPATSGSETPGSEPATSGSETPGSEPATSGSETPGTSESATPESGPGTSESATPESGPGTSTEPSEGSAPGTSTEPSEGSAPGSEPATSGSETPGTSTEPSEGSAPGTSESATPESGPGSPAGSPTSTEEGSPAGSPTSTEEGTSTEPSEGSAPGSPAGSPTSTEEGSPA |

EP 3 431 098 A1

| XTEN Name | Amino Acid Sequence |
|---|---|
| AE1236 | GSPAGSPTSTEEGTSTEPSEGSAPGSPAGSPTSTEEGTSESATPESGPGSEPATSGSETPGTSTE PSEGSAPGTSTEPSEGSAPGTSESATPESGPGTSTEPSEGSAPGTSTEPSEGSAPGSEPATSGSE TPGSPAGSPTSTEEGTSESATPESGPGSPAGSPTSTEEGTSTEPSEGSAPGSPAGSPTSTEEGTS TEPSEGSAPGSPAGSPTSTEEGTSTEPSEGSAPGSPAGSPTSTEEGSPAGSPTSTEEGTSTEPSE GSAPGSEPATSGSETPGSPAGSPTSTEEGTSESATPESGPGSEPATSGSETPGSEPATSGSETPG TSESATPESGPGSPAGSPTSTEEGTSESATPESGPGSEPATSGSETPGSEPATSGSETPGTSESA TPESGPGTSESATPESGPGTSTEPSEGSAPGTSTEPSEGSAPGTSESATPESGPGSPAGSPTSTE EGTSTEPSEGSAPGSEPATSGSETPGSPAGSPTSTEEGTSTEPSEGSAPGTSTEPSEGSAPGSPA GSPTSTEEGSPAGSPTSTEEGSPAGSPTSTEEGTSESATPESGPGSEPATSGSETPGTSESATPE SGPGTSESATPESGPGTSTEPSEGSAPGTSESATPESGPGSPAGSPTSTEEGTSESATPESGPGT SESATPESGPGTSTEPSEGSAPGSEPATSGSETPGTSTEPSEGSAPGSPAGSPTSTEEGTSESAT PESGPGTSTEPSEGSAPGTSTEPSEGSAPGSEPATSGSETPGSEPATSGSETPGTSESATPESGP GSEPATSGSETPGSPAGSPTSTEEGTSESATPESGPGSEPATSGSETPGTSTEPSEGSAPGTSES ATPESGPGTSESATPESGPGTSTEPSEGSAPGSEPATSGSETPGTSTEPSEGSAPGSPAGSPTST EEGSPAGSPTSTEEGTSESATPESGPGSEPATSGSETPGSPAGSPTSTEEGSPAGSPTSTEEGTS |
| | ESATPESGPGSEPATSGSETPGTSTEPSEGSAPGTSTEPSEGSAPGSEP |
| AE1332 | GSPAGSPTSTEEGTSTEPSEGSAPGTSESATPESGPGTSESATPESGPGSEPATSGSETPGTSTE PSEGSAPGTSESATPESGPGSPAGSPTSTEEGTSTEPSEGSAPGTSESATPESGPGSPAGSPTST EEGTSTEPSEGSAPGTSESATPESGPGTSTEPSEGSAPGSEPATSGSETPGSEPATSGSETPGSE PATSGSETPGTSESATPESGPGSPAGSPTSTEEGTSESATPESGPGSEPATSGSETPGTSESATP ESGPGTSESATPESGPGSPAGSPTSTEEGTSTEPSEGSAPGTSESATPESGPGTSTEPSEGSAPG TSTEPSEGSAPGTSTEPSEGSAPGSEPATSGSETPGTSTEPSEGSAPGTSESATPESGPGTSESA TPESGPGTSTEPSEGSAPGTSESATPESGPGTSESATPESGPGSEPATSGSETPGTSTEPSEGSA PGTSESATPESGPGSPAGSPTSTEEGSPAGSPTSTEEGTSTEPSEGSAPGSEPATSGSETPGSEP ATSGSETPGTSTEPSEGSAPGSEPATSGSETPGSPAGSPTSTEEGTSESATPESGPGSEPATSGS ETPGSEPATSGSETPGTSESATPESGPGTSESATPESGPGTSTEPSEGSAPGSPAGSPTSTEEGT SESATPESGPGTSTEPSEGSAPGSEPATSGSETPGSPAGSPTSTEEGTSTEPSEGSAPGTSTEPS EGSAPGSEPATSGSETPGTSESATPESGPGTSTEPSEGSAPGSPAGSPTSTEEGTSESATPESGP GTSESATPESGPGTSTEPSEGSAPGSEPATSGSETPGSEPATSGSETPGTSTEPSEGSAPGTSES ATPESGPGSPAGSPTSTEEGTSTEPSEGSAPGTSTEPSEGSAPGSPAGSPTSTEEGSPAGSPTST EEGTSTEPSEGSAPGSEPATSGSETPGSPAGSPTSTEEGTSESATPESGPGSEPATSGSETPGSE PATSGSETPGTSESATPESGPGTSESATPESGPGTSTEPSEGSAPGTST |

EP 3 431 098 A1

(continued)

| XTEN Name | Amino Acid Sequence |
|---|---|
| AE1428 | GSEPATSGSETPGSPAGSPTSTEEGTSTEPSEGSAPGSEPATSGSETPGTSESATPESGPGTSTE PSEGSAPGTSESATPESGPGSPAGSPTSTEEGTSTEPSEGSAPGTSTEPSEGSAPGSPAGSPTST EEGTSTEPSEGSAPGTSESATPESGPGSEPATSGSETPGTSESATPESGPGSEPATSGSETPGTS TEPSEGSAPGTSTEPSEGSAPGSEPATSGSETPGTSTEPSEGSAPGTSTEPSEGSAPGSPAGSPT STEEGTSTEPSEGSAPGTSESATPESGPGTSESATPESGPGTSTEPSEGSAPGTSTEPSEGSAPG SPAGSPTSTEEGSPAGSPTSTEEGTSTEPSEGSAPGSEPATSGSETPGSPAGSPTSTEEGTSTEP SEGSAPGSPAGSPTSTEEGSPAGSPTSTEEGTSESATPESGPGSEPATSGSETPGSEPATSGSET PGTSTEPSEGSAPGSPAGSPTSTEEGSPAGSPTSTEEGTSESATPESGPGSEPATSGSETPGSEP ATSGSETPGTSESATPESGPGSEPATSGSETPGSEPATSGSETPGTSESATPESGPGTSESATPE SGPGTSESATPESGPGTSESATPESGPGSPAGSPTSTEEGTSTEPSEGSAPGTSESATPESGPGT STEPSEGSAPGTSTEPSEGSAPGTSESATPESGPGTSTEPSEGSAPGTSESATPESGPGSPAGSP TSTEEGTSTEPSEGSAPGSEPATSGSETPGTSTEPSEGSAPGSEPATSGSETPGTSTEPSEGSAP GTSTEPSEGSAPGSPAGSPTSTEEGTSESATPESGPGTSTEPSEGSAPGSEPATSGSETPGTSES ATPESGPGTSESATPESGPGTSESATPESGPGTSTEPSEGSAPGSEPATSGSETPGSPAGSPTST EEGTSESATPESGPGTSTEPSEGSAPGTSTEPSEGSAPGSPAGSPTSTEEGTSESATPESGPGTS TEPSEGSAPGTSTEPSEGSAPGSPAGSPTSTEEGTSESATPESGPGSPA |
| AE1524 | GTSTEPSEGSAPGTSTEPSEGSAPGTSTEPSEGSAPGTSTEPSEGSAPGSPAGSPTSTEEGSPAG SPTSTEEGTSESATPESGPGSEPATSGSETPGTSTEPSEGSAPGTSESATPESGPGTSTEPSEGS APGTSESATPESGPGTSESATPESGPGSPAGSPTSTEEGTSTEPSEGSAPGSEPATSGSETPGTS TEPSEGSAPGSPAGSPTSTEEGTSTEPSEGSAPGSPAGSPTSTEEGSPAGSPTSTEEGSPAGSPT STEEGTSESATPESGPGSEPATSGSETPGTSESATPESGPGTSESATPESGPGSPAGSPTSTEEG TSTEPSEGSAPGSEPATSGSETPGTSTEPSEGSAPGSPAGSPTSTEEGSPAGSPTSTEEGTSTEP SEGSAPGSEPATSGSETPGSPAGSPTSTEEGTSESATPESGPGSEPATSGSETPGSPAGSPTSTE EGTSESATPESGPGTSESATPESGPGTSTEPSEGSAPGSEPATSGSETPGSEPATSGSETPGTSE SATPESGPGSEPATSGSETPGTSTEPSEGSAPGSPAGSPTSTEEGTSTEPSEGSAPGSEPATSGS ETPGSEPATSGSETPGSPAGSPTSTEEGTSESATPESGPGTSTEPSEGSAPGSPAGSPTSTEEGS PAGSPTSTEEGTSESATPESGPGSPAGSPTSTEEGTSTEPSEGSAPGSEPATSGSETPGSEPATS GSETPGSEPATSGSETPGTSESATPESGPGTSESATPESGPGTSTEPSEGSAPGTSTEPSEGSAP GSEPATSGSETPGTSTEPSEGSAPGTSESATPESGPGSPAGSPTSTEEGSPAGSPTSTEEGTSTE PSEGSAPGTSESATPESGPGSPAGSPTSTEEGTSTEPSEGSAPGSEPATSGSETPGTSESATPES GPGSEPATSGSETPGTSESATPESGPGSEPATSGSETPGSEPATSGSETPGTSTEPSEGSAPGTS ESATPESGPGTSTEPSEGSAPGTSTEPSEGSAPGTSESATPESGPGSPA |

| XTEN Name | Amino Acid Sequence |
|---|---|
| AE1620 | GSEPATSGSETPGTSTEPSEGSAPGSEPATSGSETPGTSTEPSEGSAPGTSESATPESGPGTSES ATPESGPGSEPATSGSETPGTSTEPSEGSAPGTSTEPSEGSAPGTSESATPESGPGTSTEPSEGS APGTSTEPSEGSAPGSEPATSGSETPGSPAGSPTSTEEGTSESATPESGPGTSESATPESGPGTS TEPSEGSAPGTSESATPESGPGSEPATSGSETPGTSESATPESGPGTSESATPESGPGTSTEPSE GSAPGTSTEPSEGSAPGSEPATSGSETPGSPAGSPTSTEEGTSESATPESGPGSEPATSGSETPG SEPATSGSETPGTSESATPESGPGTSESATPESGPGSPAGSPTSTEEGTSTEPSEGSAPGTSESA TPESGPGTSTEPSEGSAPGTSTEPSEGSAPGTSESATPESGPGTSTEPSEGSAPGSPAGSPTSTE EGSPAGSPTSTEEGTSESATPESGPGSEPATSGSETPGSEPATSGSETPGTSTEPSEGSAPGTSE SATPESGPGSPAGSPTSTEEGSPAGSPTSTEEGTSTEPSEGSAPGSPAGSPTSTEEGSPAGSPTS TEEGTSESATPESGPGSEPATSGSETPGTSTEPSEGSAPGTSTEPSEGSAPGSEPATSGSETPGS |
|  | PAGSPTSTEEGTSESATPESGPGSPAGSPTSTEEGTSTEPSEGSAPGTSESATPESGPGSEPATS GSETPGTSESATPESGPGTSESATPESGPGSPAGSPTSTEEGTSTEPSEGSAPGSEPATSGSETP GSPAGSPTSTEEGTSTEPSEGSAPGSEPATSGSETPGSEPATSGSETPGTSESATPESGPGTSES ATPESGPGTSTEPSEGSAPGTSESATPESGPGSEPATSGSETPGTSESATPESGPGTSESATPES GPGTSTEPSEGSAPGTSESATPESGPGSEPATSGSETPGTSTEPSEGSAPGSPAGSPTSTEEGTS ESATPESGPGTSTEPSEGSAPGTSTEPSEGSAPGSPAGSPTSTEEGTST |
| AE1716 | GTSESATPESGPGTSTEPSEGSAPGTSESATPESGPGTSTEPSEGSAPGSEPATSGSETPGSPAG SPTSTEEGTSESATPESGPGSEPATSGSETPGSEPATSGSETPGTSESATPESGPGTSESATPES GPGTSTEPSEGSAPGSEPATSGSETPGSEPATSGSETPGTSESATPESGPGTSESATPESGPGSE PATSGSETPGTSESATPESGPGSPAGSPTSTEEGSPAGSPTSTEEGTSTEPSEGSAPGSEPATSG SETPGSPAGSPTSTEEGTSESATPESGPGSPAGSPTSTEEGTSTEPSEGSAPGSEPATSGSETPG SPAGSPTSTEEGTSESATPESGPGSPAGSPTSTEEGSPAGSPTSTEEGTSTEPSEGSAPGSEPAT SGSETPGSPAGSPTSTEEGTSESATPESGPGTSTEPSEGSAPGSPAGSPTSTEEGTSESATPESG PGSEPATSGSETPGTSTEPSEGSAPGSPAGSPTSTEEGTSTEPSEGSAPGTSESATPESGPGTSE SATPESGPGTSTEPSEGSAPGTSTEPSEGSAPGSEPATSGSETPGTSESATPESGPGTSESATPE SGPGTSTEPSEGSAPGTSTEPSEGSAPGSPAGSPTSTEEGSPAGSPTSTEEGTSTEPSEGSAPGS EPATSGSETPGSEPATSGSETPGTSESATPESGPGSPAGSPTSTEEGTSESATPESGPGTSTEPS EGSAPGTSTEPSEGSAPGSPAGSPTSTEEGSPAGSPTSTEEGTSESATPESGPGSPAGSPTSTEE GTSTEPSEGSAPGSEPATSGSETPGSPAGSPTSTEEGSPAGSPTSTEEGTSTEPSEGSAPGTSES ATPESGPGTSESATPESGPGSPAGSPTSTEEGTSTEPSEGSAPGSPAGSPTSTEEGTSTEPSEGS APGSPAGSPTSTEEGTSTEPSEGSAPGTSTEPSEGSAPGSEPATSGSETPGSPAGSPTSTEEGTS ESATPESGPGSEPATSGSETPGSPAGSPTSTEEGTSESATPESGPGTSE |

EP 3 431 098 A1

| XTEN Name | Amino Acid Sequence |
|---|---|
| AE1812 | GTSESATPESGPGSPAGSPTSTEEGSPAGSPTSTEEGTSESATPESGPGSEPATSGSETPGTSTEPSEGSAPGTSTEPSEGSAPGTSTEPSEGSAPGSPAGSPTSTEEGTSTEPSEGSAPGTSESATPESGPGTSESATPESGPGSPAGSPTSTEEGSPAGSPTSTEEGSPAGSPTSTEEGTSESATPESGPGSEPATSGSETPGTSESATPESGPGTSTEPSEGSAPGTSESATPESGPGTSTEPSEGSAPGTSTEPSEGSAPGTSESATPESGPGSPAGSPTSTEEGTSESATPESGPGTSTEPSEGSAPGSPAGSPTSTEEGTSESATPESGPGSEPATSGSETPGTSTEPSEGSAPGSEPATSGSETPGSEPATSGSETPGTSESATPESGPGTSESATPESGPGTSTEPSEGSAPGTSESATPESGPGSPAGSPTSTEEGTSTEPSEGSAPGSEPATSGSETPGTSESATPESGPGSEPATSGSETPGTSTEPSEGSAPGTSESATPESGPGSPAGSPTSTEEGSPAGSPTSTEEGSPAGSPTSTEEGTSESATPESGPGSEPATSGSETPGTSESATPESGPGTSESATPESGPGSEPATSGSETPGTSTEPSEGSAPGTSTEPSEGSAPGTSTEPSEGSAPGTSESATPESGPGSEPATSGSETPGTSESATPESGPGSEPATSGSETPGTSTEPSEGSAPGSEPATSGSETPGSPAGSPTSTEEGTSESATPESGPGTSESATPESGPGTSESATPESGPGSPAGSPTSTEEGTSTEPSEGSAPGTSESATPESGPGTSESATPESGPGTSTEPSEGSAPGTSTEPSEGSAPGSPAGSPTSTEEGTSESATPESGPGSEPATSGSETPGTSESATPESGPGTSESATPESGPGTSTEPSEGSAPGSEPATSGSETPGTSESATPESGPGSPAGSPTSTEEGTSTEPSEGSAPGSEPATSGSETPGSPAGSPTSTEEGTSESATPESGPGSPAGSPTSTEEGTSTEPSEGSAPGSEP |
| AE1908 | GSEPATSGSETPGTSTEPSEGSAPGTSESATPESGPGSEPATSGSETPGTSTEPSEGSAPGSPAGSPTSTEEGTSESATPESGPGSPAGSPTSTEEGTSTEPSEGSAPGSEPATSGSETPGTSTEPSEGSAPGTSESATPESGPGSEPATSGSETPGSEPATSGSETPGTSESATPESGPGTSTEPSEGSAPGTSESATPESGPGTSESATPESGPGSPAGSPTSTEEGSPAGSPTSTEEGTSESATPESGPGTSESATPESGPGTSTEPSEGSAPGTSTEPSEGSAPGSPAGSPTSTEEGTSTEPSEGSAPGSEPATSGSETPGSEPATSGSETPGSEPATSGSETPGTSESATPESGPGTSESATPESGPGTSESATPESGPGTSTEPSEGSAPGTSESATPESGPGTSESATPESGPGTSTEPSEGSAPGSPAGSPTSTEEGTSTEPSEGSAPGSEPATSGSETPGSPAGSPTSTEEGTSESATPESGPGTSTEPSEGSAPGSEPATSGSETPGTSESATPESGPGTSESATPESGPGTSTEPSEGSAPGSEPATSGSETPGSPAGSPTSTEEGTSESATPESGPGTSESATPESGPGTSTEPSEGSAPGSEPATSGSETPGSPAGSPTSTEEGTSESATPESGPGSEPATSGSETPGTSTEPSEGSAPGSEPATSGSETPGSPAGSPTSTEEGTSESATPESGPGTSTEPSEGSAPGSEPATSGSETPGTSESATPESGPGTSESATPESGPGSPAGSPTSTEEGTSTEPSEGSAPGSPAGSPTSTEEGSPAGSPTSTEEGSPAGSPTSTEEGTSESATPESGPGSEPATSGSETPGTSTEPSEGSAPGTSTEPSEGSAPGTSESATPESGPGTSTEPSEGSAPGTSESATPESGPGSEPATSGSETPGTSTEPSEGSAPGTSESATPESGPGSPAGSPTSTEEGSPAGSPTSTEEGSPAGSPTSTEEGTSESATPESGPGSPAGSPTSTEEGTSESATPESGPGSEP |

(continued)

| XTEN Name | Amino Acid Sequence |
|---|---|
| AE2004A | GTSTEPSEGSAPGSEPATSGSETPGTSTEPSEGSAPGTSTEPSEGSAPGSEPATSGSETPGSPAG SPTSTEEGSPAGSPTSTEEGTSESATPESGPGSEPATSGSETPGTSTEPSEGSAPGTSTEPSEGS APGSPAGSPTSTEEGTSESATPESGPGTSESATPESGPGSPAGSPTSTEEGTSTEPSEGSAPGTS ESATPESGPGTSTEPSEGSAPGSEPATSGSETPGTSESATPESGPGSEPATSGSETPGTSTEPSE GSAPGTSTEPSEGSAPGSPAGSPTSTEEGSPAGSPTSTEEGTSESATPESGPGSPAGSPTSTEEG |
|  | TSTEPSEGSAPGSEPATSGSETPGSEPATSGSETPGSPAGSPTSTEEGTSESATPESGPGTSTEP SEGSAPGSEPATSGSETPGTSESATPESGPGSEPATSGSETPGSPAGSPTSTEEGTSESATPESG PGTSESATPESGPGSEPATSGSETPGTSESATPESGPGTSESATPESGPGTSESATPESGPGTST EPSEGSAPGTSESATPESGPGSEPATSGSETPGTSTEPSEGSAPGSPAGSPTSTEEGTSTEPSEG SAPGSEPATSGSETPGSPAGSPTSTEEGTSESATPESGPGTSESATPESGPGTSTEPSEGSAPGT SESATPESGPGTSTEPSEGSAPGTSESATPESGPGSEPATSGSETPGTSTEPSEGSAPGTSTEPS EGSAPGSPAGSPTSTEEGSPAGSPTSTEEGTSESATPESGPGTSTEPSEGSAPGTSESATPESGP GSPAGSPTSTEEGSPAGSPTSTEEGSPAGSPTSTEEGTSTEPSEGSAPGSPAGSPTSTEEGSPAG SPTSTEEGTSESATPESGPGSEPATSGSETPGSEPATSGSETPGTSESATPESGPGSEPATSGSE TPGTSESATPESGPGTSTEPSEGSAPGTSTEPSEGSAPGSPAGSPTSTEEGSPAGSPTSTEEGTS ESATPESGPGSPAGSPTSTEEGTSTEPSEGSAPGTSESATPESGPGTSE |
| AG948 | GSSTPSGATGSPGTPGSGTASSSPGASPGTSSTGSPGSSPSASTGTGPGTPGSGTASSSPGTPG SGTASSSPGTPGSGTASSSPGSSPSASTGTGPGTPGSGTASSSPGSSPSASTGTGPGSSTPSGAT GSPGSSTPSGATGSPGSSPSASTGTGPGSSTPSGATGSPGASPGTSSTGSPGASPGTSSTGSPGS SPSASTGTGPGSSPSASTGTGPGASPGTSSTGSPGSSPSASTGTGPGASPGTSSTGSPGSSPSAS TGTGPGASPGTSSTGSPGTPGSGTASSSPGTPGSGTASSSPGSSTPSGATGSPGSSTPSGATGS PGSSTPSGATGSPGASPGTSSTGSPGSSPSASTGTGPGASPGTSSTGSPGASPGTSSTGSPGSST PSGATGSPGSSPSASTGTGPGSSPSASTGTGPGTPGSGTASSSPGASPGTSSTGSPGTPGSGTA SSSPGSSTPSGATGSPGSSTPSGATGSPGSSPSASTGTGPGSSPSASTGTGPGSSTPSGATGSPG ASPGTSSTGSPGTPGSGTASSSPGSSPSASTGTGPGSSTPSGATGSPGASPGTSSTGSPGSSTPS GATGSPGTPGSGTASSSPGSSPSASTGTGPGSSTPSGATGSPGASPGTSSTGSPGASPGTSSTG SPGTPGSGTASSSPGTPGSGTASSSPGSSPSASTGTGPGASPGTSSTGSPGASPGTSSTGSPGTP GSGTASSSPGTPGSGTASSSPGSSTPSGATGSPGSSPSASTGTGPGSSTPSGATGSPGSSPSAST GTGPGSSPSASTGTGPGTPGSGTASSSPGSSTPSGATGSPGASPGTSSTGSPGTPGSGTASSSP GTPGSGTASSSPGSSTPSGATGSPGTPGSGTASSSPGASPGTSSTGSPGSSTPSGATGSPGSSTP SGATGSPGTPGSGTASSSPGSSTPSGATGSPGSSTPSGATGSP |

(continued)

| XTEN Name | Amino Acid Sequence |
|---|---|
| AG1044 | GTPGSGTASSSPGTPGSGTASSSPGSSPSASTGTGPGTPGSGTASSSPGASPGTSSTGSPGTPG SGTASSSPGSSPSASTGTGPGSSTPSGATGSPGASPGTSSTGSPGASPGTSSTGSPGSSPSASTG TGPGSSTPSGATGSPGSSPSASTGTGPGSSPSASTGTGPGTPGSGTASSSPGTPGSGTASSSPG ASPGTSSTGSPGSSTPSGATGSPGSSPSASTGTGPGTPGSGTASSSPGASPGTSSTGSPGTPGS GTASSSPGSSPSASTGTGPGSSPSASTGTGPGASPGTSSTGSPGASPGTSSTGSPGSSTPSGAT GSPGSSTPSGATGSPGTPGSGTASSSPGSSPSASTGTGPGSSTPSGATGSPGASPGTSSTGSPGS STPSGATGSPGSSTPSGATGSPGSSPSASTGTGPGASPGTSSTGSPGTPGSGTASSSPGASPGT SSTGSPGSSPSASTGTGPGTPGSGTASSSPGASPGTSSTGSPGASPGTSSTGSPGSSTPSGATGS PGTPGSGTASSSPGSSTPSGATGSPGASPGTSSTGSPGTPGSGTASSSPGTPGSGTASSSPGSST PSGATGSPGSSTPSGATGSPGTPGSGTASSSPGSSTPSGATGSPGSSTPSGATGSPGASPGTSS TGSPGSSPSASTGTGPGSSPSASTGTGPGASPGTSSTGSPGASPGTSSTGSPGTPGSGTASSSP GSSPSASTGTGPGTPGSGTASSSPGSSTPSGATGSPGSSTPSGATGSPGASPGTSSTGSPGSSPS ASTGTGPGASPGTSSTGSPGSSPSASTGTGPGSSPSASTGTGPGASPGTSSTGSPGASPGTSST GSPGSSTPSGATGSPGASPGTSSTGSPGASPGTSSTGSPGTPGSGTASSSPGTPGSGTASSSPG TPGSGTASSSPGSSTPSGATGSPGSSTPSGATGSPGSSPSASTGTGPGSSPSASTGTGPGTPGS GTASSSPGSSPSASTGTGPGASPGTSSTGSPGSSTPSGATGSPGTPGSGTASSSPGSST |
| AG1140 | GASPGTSSTGSPGSSPSASTGTGPGSSTPSGATGSPGASPGTSSTGSPGASPGTSSTGSPGSSTP SGATGSPGTPGSGTASSSPGASPGTSSTGSPGTPGSGTASSSPGTPGSGTASSSPGSSTPSGAT GSPGSSTPSGATGSPGTPGSGTASSSPGSSTPSGATGSPGSSTPSGATGSPGSSPSASTGTGPGS SPSASTGTGPGASPGTSSTGSPGTPGSGTASSSPGASPGTSSTGSPGSSTPSGATGSPGTPGSG TASSSPGSSPSASTGTGPGSSTPSGATGSPGSSPSASTGTGPGSSTPSGATGSPGASPGTSSTGS PGSSPSASTGTGPGTPGSGTASSSPGASPGTSSTGSPGSSTPSGATGSPGASPGTSSTGSPGSSP SASTGTGPGTPGSGTASSSPGTPGSGTASSSPGASPGTSSTGSPGTPGSGTASSSPGTPGSGTA SSSPGSSPSASTGTGPGASPGTSSTGSPGSSTPSGATGSPGASPGTSSTGSPGSSPSASTGTGPG TPGSGTASSSPGSSPSASTGTGPGSSPSASTGTGPGASPGTSSTGSPGASPGTSSTGSPGTPGS GTASSSPGASPGTSSTGSPGASPGTSSTGSPGTPGSGTASSSPGTPGSGTASSSPGSSTPSGAT GSPGSSTPSGATGSPGTPGSGTASSSPGSSPSASTGTGPGTPGSGTASSSPGSSTPSGATGSPGS STPSGATGSPGASPGTSSTGSPGTPGSGTASSSPGASPGTSSTGSPGSSTPSGATGSPGSSTPSG ATGSPGTPGSGTASSSPGSSTPSGATGSPGSSTPSGATGSPGASPGTSSTGSPGSSPSASTGTG PGSSTPSGATGSPGSSTPSGATGSPGSSPSASTGTGPGSSPSASTGTGPGSSTPSGATGSPGASP GTSSTGSPGSSPSASTGTGPGTPGSGTASSSPGASPGTSSTGSPGSSPSASTGTGPGASPGTSST GSPGSSTPSGATGSPGSSPSASTGTGPGSSPSASTGTGPGASPGTSSTGSPGSST |
| AG1236 | GSSPSASTGTGPGTPGSGTASSSPGSSPSASTGTGPGSSPSASTGTGPGTPGSGTASSSPGASP |

(continued)

| XTEN Name | Amino Acid Sequence |
|---|---|
| | GTSSTGSPGSSTPSGATGSPGTPGSGTASSSPGASPGTSSTGSPGTPGSGTASSSPGTPGSGTA SSSPGSSPSASTGTGPGSSPSASTGTGPGSSTPSGATGSPGASPGTSSTGSPGSSPSASTGTGPG TPGSGTASSSPGTPGSGTASSSPGSSTPSGATGSPGASPGTSSTGSPGSSPSASTGTGPGTPGS GTASSSPGTPGSGTASSSPGASPGTSSTGSPGSSTPSGATGSPGSSTPSGATGSPGSSPSASTGT GPGSSPSASTGTGPGASPGTSSTGSPGSSPSASTGTGPGSSTPSGATGSPGASPGTSSTGSPGA SPGTSSTGSPGSSTPSGATGSPGSSPSASTGTGPGASPGTSSTGSPGASPGTSSTGSPGTPGSGT ASSSPGSSTPSGATGSPGASPGTSSTGSPGSSTPSGATGSPGTPGSGTASSSPGSSPSASTGTGP GSSTPSGATGSPGTPGSGTASSSPGSSTPSGATGSPGSSTPSGATGSPGASPGTSSTGSPGSSPS ASTGTGPGSSPSASTGTGPGASPGTSSTGSPGSSPSASTGTGPGTPGSGTASSSPGTPGSGTAS SSPGASPGTSSTGSPGTPGSGTASSSPGASPGTSSTGSPGTPGSGTASSSPGASPGTSSTGSPGS STPSGATGSPGASPGTSSTGSPGSSPSASTGTGPGTPGSGTASSSPGTPGSGTASSSPGSSPSAS TGTGPGTPGSGTASSSPGASPGTSSTGSPGSSTPSGATGSPGTPGSGTASSSPGASPGTSSTGS PGTPGSGTASSSPGTPGSGTASSSPGSSTPSGATGSPGTPGSGTASSSPGSSPSASTGTGPGSST PSGATGSPGTPGSGTASSSPGSSTPSGATGSPGSSTPSGATGSPGSSPSASTGTGPGASPGTSS TGSPGTPGSGTASSSPGSSPSASTGTGPGSSPSASTGTGPGASPGTSSTGSPGASP |
| AG1332 | GSSTPSGATGSPGSSPSASTGTGPGTPGSGTASSSPGSSPSASTGTGPGASPGTSSTGSPGSSPS ASTGTGPGTPGSGTASSSPGASPGTSSTGSPGSSTPSGATGSPGSSPSASTGTGPGSSTPSGAT GSPGASPGTSSTGSPGTPGSGTASSSPGASPGTSSTGSPGSSTPSGATGSPGSSTPSGATGSPGS SPSASTGTGPGTPGSGTASSSPGSSTPSGATGSPGSSTPSGATGSPGSSPSASTGTGPGTPGSG TASSSPGASPGTSSTGSPGTPGSGTASSSPGASPGTSSTGSPGASPGTSSTGSPGTPGSGTASSS PGTPGSGTASSSPGSSTPSGATGSPGSSTPSGATGSPGSSTPSGATGSPGASPGTSSTGSPGSSP SASTGTGPGSSTPSGATGSPGSSPSASTGTGPGSSTPSGATGSPGTPGSGTASSSPGSSPSAST GTGPGSSTPSGATGSPGASPGTSSTGSPGASPGTSSTGSPGSSPSASTGTGPGASPGTSSTGSP GSSTPSGATGSPGASPGTSSTGSPGTPGSGTASSSPGTPGSGTASSSPGSSPSASTGTGPGTPG SGTASSSPGSSTPSGATGSPGSSPSASTGTGPGSSTPSGATGSPGTPGSGTASSSPGTPGSGTA SSSPGSSPSASTGTGPGSSTPSGATGSPGSSTPSGATGSPGSSPSASTGTGPGSSPSASTGTGPG ASPGTSSTGSPGASPGTSSTGSPGSSTPSGATGSPGASPGTSSTGSPGSSPSASTGTGPGTPGS GTASSSPGASPGTSSTGSPGASPGTSSTGSPGTPGSGTASSSPGTPGSGTASSSPGSSPSASTGT GPGTPGSGTASSSPGASPGTSSTGSPGSSTPSGATGSPGTPGSGTASSSPGTPGSGTASSSPGSS TPSGATGSPGSSTPSGATGSPGTPGSGTASSSPGSSPSASTGTGPGSSPSASTGTGPGASPGTS STGSPGSSPSASTGTGPGSSPSASTGTGPGASPGTSSTGSPGASPGTSSTGSPGTPG |

(continued)

| XTEN Name | Amino Acid Sequence |
|---|---|
| AG1428 | GTPGSGTASSSPGSSTPSGATGSPGASPGTSSTGSPGSSTPSGATGSPGTPGSGTASSSPGTPG SGTASSSPGSSTPSGATGSPGSSTPSGATGSPGASPGTSSTGSPGSSPSASTGTGPGSSTPSGAT GSPGASPGTSSTGSPGSSPSASTGTGPGTPGSGTASSSPGASPGTSSTGSPGASPGTSSTGSPG TPGSGTASSSPGTPGSGTASSSPGASPGTSSTGSPGASPGTSSTGSPGTPGSGTASSSPGTPGS GTASSSPGSSPSASTGTGPGSSPSASTGTGPGASPGTSSTGSPGSSPSASTGTGPGSSPSASTGT GPGASPGTSSTGSPGASPGTSSTGSPGTPGSGTASSSPGTPGSGTASSSPGSSPSASTGTGPGA SPGTSSTGSPGSSTPSGATGSPGASPGTSSTGSPGSSPSASTGTGPGSSTPSGATGSPGSSPSAS TGTGPGSSPSASTGTGPGASPGTSSTGSPGSSPSASTGTGPGSSPSASTGTGPGASPGTSSTGS PGASPGTSSTGSPGSSPSASTGTGPGTPGSGTASSSPGASPGTSSTGSPGSSTPSGATGSPGTP GSGTASSSPGTPGSGTASSSPGSSPSASTGTGPGSSTPSGATGSPGASPGTSSTGSPGASPGTSS TGSPGSSTPSGATGSPGSSPSASTGTGPGASPGTSSTGSPGSSTPSGATGSPGTPGSGTASSSP GASPGTSSTGSPGTPGSGTASSSPGASPGTSSTGSPGSSTPSGATGSPGSSTPSGATGSPGASP GTSSTGSPGSSPSASTGTGPGTPGSGTASSSPGTPGSGTASSSPGSSTPSGATGSPGTPGSGTA SSSPGASPGTSSTGSPGSSTPSGATGSPGTPGSGTASSSPGSSPSASTGTGPGSSTPSGATGSPG SSTPSGATGSPGSSPSASTGTGPGSSTPSGATGSPGTPGSGTASSSPGSSPSASTGTGPGTPGS GTASSSPGSSTPSGATGSPGSSPSASTGTGPGSSPSASTGTGPGTPGSGTASSSPGASP |
| AG1524 | GSSTPSGATGSPGTPGSGTASSSPGTPGSGTASSSPGASPGTSSTGSPGSSTPSGATGSPGTPG SGTASSSPGSSTPSGATGSPGSSPSASTGTGPGSSTPSGATGSPGTPGSGTASSSPGTPGSGTA SSSPGSSPSASTGTGPGSSTPSGATGSPGSSPSASTGTGPGTPGSGTASSSPGASPGTSSTGSPG SSTPSGATGSPGASPGTSSTGSPGTPGSGTASSSPGSSTPSGATGSPGTPGSGTASSSPGSSPSA STGTGPGASPGTSSTGSPGASPGTSSTGSPGTPGSGTASSSPGSSTPSGATGSPGSSTPSGATG SPGSSPSASTGTGPGSSPSASTGTGPGASPGTSSTGSPGTPGSGTASSSPGTPGSGTASSSPGSS PSASTGTGPGASPGTSSTGSPGASPGTSSTGSPGTPGSGTASSSPGSSPSASTGTGPGASPGTS STGSPGASPGTSSTGSPGSSTPSGATGSPGSSPSASTGTGPGSSPSASTGTGPGTPGSGTASSSP GTPGSGTASSSPGASPGTSSTGSPGSSTPSGATGSPGTPGSGTASSSPGASPGTSSTGSPGTPG SGTASSSPGTPGSGTASSSPGSSTPSGATGSPGSSTPSGATGSPGTPGSGTASSSPGSSPSASTG TGPGSSTPSGATGSPGSSTPSGATGSPGSSPSASTGTGPGTPGSGTASSSPGSSPSASTGTGPG ASPGTSSTGSPGSSPSASTGTGPGTPGSGTASSSPGASPGTSSTGSPGASPGTSSTGSPGTPGS |
| | GTASSSPGTPGSGTASSSPGSSTPSGATGSPGTPGSGTASSSPGASPGTSSTGSPGSSTPSGAT GSPGSSTPSGATGSPGSSTPSGATGSPGTPGSGTASSSPGSSPSASTGTGPGSSPSASTGTGPGS SPSASTGTGPGASPGTSSTGSPGASPGTSSTGSPGSSPSASTGTGPGTPGSGTASSSPGASPGT SSTGSPGSSTPSGATGSPGASPGTSSTGSPGASPGTSSTGSPGSSTPSGATGSPGTPG |

| XTEN Name | Amino Acid Sequence |
|---|---|
| AG1620 | GSSTPSGATGSPGSSTPSGATGSPGTPGSGTASSSPGSSPSASTGTGPGTPGSGTASSSPGASP GTSSTGSPGSSTPSGATGSPGASPGTSSTGSPGTPGSGTASSSPGASPGTSSTGSPGSSPSASTG TGPGTPGSGTASSSPGASPGTSSTGSPGSSTPSGATGSPGSSPSASTGTGPGSSTPSGATGSPG ASPGTSSTGSPGASPGTSSTGSPGASPGTSSTGSPGSSTPSGATGSPGASPGTSSTGSPGTPGS GTASSSPGSSTPSGATGSPGSSTPSGATGSPGSSPSASTGTGPGSSPSASTGTGPGSSTPSGAT GSPGSSPSASTGTGPGSSPSASTGTGPGASPGTSSTGSPGASPGTSSTGSPGTPGSGTASSSPGS SPSASTGTGPGSSPSASTGTGPGASPGTSSTGSPGSSPSASTGTGPGTPGSGTASSSPGSSPSAS TGTGPGSSTPSGATGSPGASPGTSSTGSPGSSTPSGATGSPGTPGSGTASSSPGSSPSASTGTG PGASPGTSSTGSPGSSTPSGATGSPGASPGTSSTGSPGASPGTSSTGSPGTPGSGTASSSPGASP GTSSTGSPGASPGTSSTGSPGSSTPSGATGSPGTPGSGTASSSPGSSPSASTGTGPGSSTPSGAT GSPGTPGSGTASSSPGSSTPSGATGSPGSSTPSGATGSPGSSPSASTGTGPGSSPSASTGTGPGS STPSGATGSPGASPGTSSTGSPGSSPSASTGTGPGTPGSGTASSSPGASPGTSSTGSPGSSPSAS TGTGPGSSTPSGATGSPGSSPSASTGTGPGSSTPSGATGSPGSSPSASTGTGPGTPGSGTASSS PGTPGSGTASSSPGSSTPSGATGSPGSSTPSGATGSPGTPGSGTASSSPGSSPSASTGTGPGSST PSGATGSPGSSPSASTGTGPGSSPSASTGTGPGSSTPSGATGSPGASPGTSSTGSPGASPGTSS TGSPGTPGSGTASSSPGTPGSGTASSSPGTPGSGTASSSPGSSTPSGATGSPGSST |
| AG1716 | GASPGTSSTGSPGSSPSASTGTGPGSSTPSGATGSPGSSPSASTGTGPGTPGSGTASSSPGSSTP SGATGSPGSSTPSGATGSPGSSPSASTGTGPGSSTPSGATGSPGTPGSGTASSSPGSSPSASTG TGPGSSTPSGATGSPGASPGTSSTGSPGSSPSASTGTGPGSSPSASTGTGPGTPGSGTASSSPG ASPGTSSTGSPGSSTPSGATGSPGSSPSASTGTGPGASPGTSSTGSPGSSTPSGATGSPGTPGS GTASSSPGSSPSASTGTGPGSSPSASTGTGPGSSPSASTGTGPGTPGSGTASSSPGTPGSGTAS SSPGSSTPSGATGSPGTPGSGTASSSPGSSPSASTGTGPGASPGTSSTGSPGASPGTSSTGSPGT PGSGTASSSPGSSPSASTGTGPGASPGTSSTGSPGTPGSGTASSSPGSSTPSGATGSPGSSTPSG ATGSPGASPGTSSTGSPGTPGSGTASSSPGSSPSASTGTGPGTPGSGTASSSPGASPGTSSTGS PGSSTPSGATGSPGASPGTSSTGSPGASPGTSSTGSPGTPGSGTASSSPGTPGSGTASSSPGSSP SASTGTGPGSSPSASTGTGPGSSPSASTGTGPGTPGSGTASSSPGASPGTSSTGSPGSSTPSGA TGSPGASPGTSSTGSPGSSPSASTGTGPGASPGTSSTGSPGSSPSASTGTGPGASPGTSSTGSP GSSPSASTGTGPGSSPSASTGTGPGTPGSGTASSSPGASPGTSSTGSPGTPGSGTASSSPGTPG SGTASSSPGASPGTSSTGSPGSSTPSGATGSPGSSTPSGATGSPGSSPSASTGTGPGSSTPSGAT GSPGTPGSGTASSSPGSSPSASTGTGPGSSTPSGATGSPGSSTPSGATGSPGSSPSASTGTGPGS SPSASTGTGPGASPGTSSTGSPGTPGSGTASSSPGSSPSASTGTGPGSSTPSGATGSPGASPGT SSTGSPGASPGTSSTGSPGSSPSASTGTGPGASPGTSSTGSPGASPGTSSTGSPGTPG |

(continued)

| XTEN Name | Amino Acid Sequence |
|---|---|
| AG1812 | GSSTPSGATGSPGSSPSASTGTGPGASPGTSSTGSPGASPGTSSTGSPGTPGSGTASSSPGSSPS ASTGTGPGASPGTSSTGSPGSSTPSGATGSPGSSTPSGATGSPGASPGTSSTGSPGSSPSASTG TGPGTPGSGTASSSPGTPGSGTASSSPGASPGTSSTGSPGSSTPSGATGSPGSSTPSGATGSPG SSPSASTGTGPGTPGSGTASSSPGASPGTSSTGSPGSSTPSGATGSPGTPGSGTASSSPGSSPSA STGTGPGSSTPSGATGSPGTPGSGTASSSPGSSPSASTGTGPGTPGSGTASSSPGASPGTSSTG SPGSSTPSGATGSPGSSTPSGATGSPGTPGSGTASSSPGSSTPSGATGSPGSSTPSGATGSPGAS PGTSSTGSPGASPGTSSTGSPGASPGTSSTGSPGSSTPSGATGSPGTPGSGTASSSPGSSPSAST GTGPGTPGSGTASSSPGASPGTSSTGSPGTPGSGTASSSPGASPGTSSTGSPGSSTPSGATGSP GSSPSASTGTGPGSSPSASTGTGPGTPGSGTASSSPGASPGTSSTGSPGASPGTSSTGSPGTPG SGTASSSPGASPGTSSTGSPGSSTPSGATGSPGSSTPSGATGSPGSSPSASTGTGPGASPGTSST GSPGASPGTSSTGSPGTPGSGTASSSPGTPGSGTASSSPGSSTPSGATGSPGSSPSASTGTGPGS SPSASTGTGPGASPGTSSTGSPGASPGTSSTGSPGSSTPSGATGSPGSSPSASTGTGPGASPGT SSTGSPGSSTPSGATGSPGTPGSGTASSSPGSSPSASTGTGPGASPGTSSTGSPGSSTPSGATGS PGTPGSGTASSSPGSSTPSGATGSPGSSTPSGATGSPGSSTPSGATGSPGSSPSASTGTGPGSSP SASTGTGPGSSTPSGATGSPGSSPSASTGTGPGSSPSASTGTGPGSSTPSGATGSPGASPGTSS TGSPGASPGTSSTGSPGTPGSGTASSSPGASPGTSSTGSPGSSTPSGATGSPGASP |
| AG1908 | GSSPSASTGTGPGSSPSASTGTGPGSSPSASTGTGPGTPGSGTASSSPGSSPSASTGTGPGSSPS ASTGTGPGASPGTSSTGSPGSSPSASTGTGPGTPGSGTASSSPGTPGSGTASSSPGASPGTSST GSPGTPGSGTASSSPGTPGSGTASSSPGSSPSASTGTGPGSSTPSGATGSPGSSPSASTGTGPG ASPGTSSTGSPGSSPSASTGTGPGASPGTSSTGSPGASPGTSSTGSPGTPGSGTASSSPGTPGS GTASSSPGASPGTSSTGSPGTPGSGTASSSPGTPGSGTASSSPGSSPSASTGTGPGSSTPSGAT GSPGASPGTSSTGSPGSSTPSGATGSPGSSPSASTGTGPGSSPSASTGTGPGSSTPSGATGSPG ASPGTSSTGSPGSSPSASTGTGPGTPGSGTASSSPGASPGTSSTGSPGASPGTSSTGSPGTPGS |
|  | GTASSSPGTPGSGTASSSPGSSTPSGATGSPGTPGSGTASSSPGASPGTSSTGSPGSSTPSGAT GSPGTPGSGTASSSPGSSPSASTGTGPGSSPSASTGTGPGSSTPSGATGSPGASPGTSSTGSPG ASPGTSSTGSPGTPGSGTASSSPGTPGSGTASSSPGSSTPSGATGSPGSSPSASTGTGPGSSPSA STGTGPGASPGTSSTGSPGASPGTSSTGSPGSSTPSGATGSPGASPGTSSTGSPGASPGTSSTG SPGTPGSGTASSSPGTPGSGTASSSPGTPGSGTASSSPGSSTPSGATGSPGSSTPSGATGSPGSS PSASTGTGPGSSPSASTGTGPGSSTPSGATGSPGTPGSGTASSSPGSSPSASTGTGPGASPGTS STGSPGSSTPSGATGSPGSSPSASTGTGPGSSTPSGATGSPGASPGTSSTGSPGSSPSASTGTGP GTPGSGTASSSPGSSPSASTGTGPGSSTPSGATGSPGASPGTSSTGSPGSSPSASTGTGPGTPG SGTASSSPGSSTPSGATGSPGSSTPSGATGSPGASPGTSSTGSPGSSPSASTGTGPGSSP |

| XTEN Name | Amino Acid Sequence |
|---|---|
| AG2004A | GSSPSASTGTGPGTPGSGTASSSPGSSTPSGATGSPGTPGSGTASSSPGSSTPSGATGSPGSSTP SGATGSPGSSPSASTGTGPGSSPSASTGTGPGSSPSASTGTGPGTPGSGTASSSPGASPGTSST GSPGSSTPSGATGSPGTPGSGTASSSPGTPGSGTASSSPGSSTPSGATGSPGSSTPSGATGSPGS SPSASTGTGPGASPGTSSTGSPGASPGTSSTGSPGASPGTSSTGSPGASPGTSSTGSPGTPGSG TASSSPGTPGSGTASSSPGSSPSASTGTGPGSSTPSGATGSPGASPGTSSTGSPGSSTPSGATGS PGSSTPSGATGSPGSSPSASTGTGPGASPGTSSTGSPGTPGSGTASSSPGSSPSASTGTGPGSSP SASTGTGPGASPGTSSTGSPGASPGTSSTGSPGTPGSGTASSSPGASPGTSSTGSPGTPGSGTA SSSPGTPGSGTASSSPGSSTPSGATGSPGTPGSGTASSSPGASPGTSSTGSPGSSTPSGATGSPG SSTPSGATGSPGSSPSASTGTGPGSSTPSGATGSPGSSTPSGATGSPGASPGTSSTGSPGSSPSA STGTGPGTPGSGTASSSPGASPGTSSTGSPGSSPSASTGTGPGTPGSGTASSSPGSSPSASTGT GPGTPGSGTASSSPGASPGTSSTGSPGASPGTSSTGSPGTPGSGTASSSPGTPGSGTASSSPGSS TPSGATGSPGSSTPSGATGSPGTPGSGTASSSPGSSTPSGATGSPGSSTPSGATGSPGASPGTS STGSPGSSPSASTGTGPGSSTPSGATGSPGSSPSASTGTGPGSSPSASTGTGPGASPGTSSTGSP GSSTPSGATGSPGASPGTSSTGSPGTPGSGTASSSPGTPGSGTASSSPGSSPSASTGTGPGASP GTSSTGSPGSSTPSGATGSPGSSPSASTGTGPGSSPSASTGTGPGASPGTSSTGSPGTPGSGTA SSSPGSSTPSGATGSPGTPGSGTASSSPGSSPSASTGTGPGSSPSASTGTGPGASP |
| AE72B | SPAGSPTSTEEGSPAGSPTSTEEGTSESATPESGPGTSTEPSEGSAPGTSESATPESGPGSEPAT SGSETPG |
| AE72C | TSESATPESGPGTSESATPESGPGSEPATSGSETPGSEPATSGSETPGSPAGSPTSTEEGTSTEP SEGSAPG |
| AE108A | TEEGTSESATPESGPGTSTEPSEGSAPGSPAGSPTSTEEGTSTEPSEGSAPGTSTEPSEGSAPGT SESATPESGPGSEPATSGSETPGSEPATSGSETPGSPAGSPTS |
| AE108B | GSPAGSPTSTEEGTSTEPSEGSAPGTSESATPESGPGTSESATPESGPGTSESATPESGPGSEPA TSGSETPGSEPATSGSETPGSPAGSPTSTEEGTSTEPSEGSAP |
| AE144A | STEPSEGSAPGSPAGSPTSTEEGTSTEPSEGSAPGTSESATPESGPGSEPATSGSETPGTSESAT PESGPGSEPATSGSETPGTSESATPESGPGTSTEPSEGSAPGTSESATPESGPGSPAGSPTSTEE GSPAGSPTSTEEGS |
| AE144B | SEPATSGSETPGTSESATPESGPGSEPATSGSETPGTSESATPESGPGTSTEPSEGSAPGSPAGS PTSTEEGTSESATPESGPGSEPATSGSETPGTSESATPESGPGSPAGSPTSTEEGSPAGSPTSTE EGTSTEPSEGSAPG |

(continued)

| XTEN Name | Amino Acid Sequence |
|---|---|
| AE180A | TSTEEGTSESATPESGPGSEPATSGSETPGTSESATPESGPGSPAGSPTSTEEGSPAGSPTSTEEGTSTEPSEGSAPGTSESATPESGPGTSESATPESGPGTSESATPESGPGSEPATSGSETPGSEPATSGSETPGSPAGSPTSTEEGTSTEPSEGSAPGTSTEPSEGSAPGSEPATS |
| AE216A | PESGPGTSTEPSEGSAPGSPAGSPTSTEEGTSESATPESGPGSEPATSGSETPGTSESATPESGPGSPAGSPTSTEEGSPAGSPTSTEEGTSTEPSEGSAPGTSESATPESGPGTSESATPESGPGTSESATPESGPGSEPATSGSETPGSEPATSGSETPGSPAGSPTSTEEGTSTEPSEGSAPGTSTEPSEGSAPGSEPATSGSETPGTSESAT |
| AE252A | ESGPGSEPATSGSETPGTSESATPESGPGTSTEPSEGSAPGSPAGSPTSTEEGTSESATPESGPGSEPATSGSETPGTSESATPESGPGSPAGSPTSTEEGSPAGSPTSTEEGTSTEPSEGSAPGTSESATPESGPGTSESATPESGPGTSESATPESGPGSEPATSGSETPGSEPATSGSETPGSPAGSPTSTEEGTSTEPSEGSAPGTSTEPSEGSAPGSEPATSGSETPGTSESATPESGPGTSTEPSE |
| AE288A | TPESGPGTSTEPSEGSAPGTSESATPESGPGSEPATSGSETPGTSESATPESGPGSEPATSGSETPGTSESATPESGPGTSTEPSEGSAPGSPAGSPTSTEEGTSESATPESGPGSEPATSGSETPGTSESATPESGPGSPAGSPTSTEEGSPAGSPTSTEEGTSTEPSEGSAPGTSESATPESGPGTSESATPESGPGTSESATPESGPGSEPATSGSETPGSEPATSGSETPGSPAGSPTSTEEGTSTEPSEGSAPGTSTEPSEGSAPGSEPATSGSETPGTSESA |
| AE324A | PESGPGSPAGSPTSTEEGSPAGSPTSTEEGSPAGSPTSTEEGTSESATPESGPGTSTEPSEGSAPGTSESATPESGPGSEPATSGSETPGTSESATPESGPGSEPATSGSETPGTSESATPESGPGTSTEPSEGSAPGSPAGSPTSTEEGTSESATPESGPGSEPATSGSETPGTSESATPESGPGSPAGSPTSTEEGSPAGSPTSTEEGTSTEPSEGSAPGTSESATPESGPGTSESATPESGPGTSESATPESGPGSE |
|  | PATSGSETPGSEPATSGSETPGSPAGSPTSTEEGTSTEPSEGSAPGTSTEPSEGSAPGSEPATS |
| AE360A | PESGPGTSTEPSEGSAPGTSESATPESGPGSPAGSPTSTEEGSPAGSPTSTEEGSPAGSPTSTEEGTSESATPESGPGTSTEPSEGSAPGTSESATPESGPGSEPATSGSETPGTSESATPESGPGSEPATSGSETPGTSESATPESGPGTSTEPSEGSAPGSPAGSPTSTEEGTSESATPESGPGSEPATSGSETPGTSESATPESGPGSPAGSPTSTEEGSPAGSPTSTEEGTSTEPSEGSAPGTSESATPESGPGTSESATPESGPGTSESATPESGPGSEPATSGSETPGSEPATSGSETPGSPAGSPTSTEEGTSTEPSEGSAPGTSTEPSEGSAPGSEPATSGSETPGTSESAT |
| AE396A | PESGPGSEPATSGSETPGTSESATPESGPGTSTEPSEGSAPGTSESATPESGPGSPAGSPTSTEEGSPAGSPTSTEEGSPAGSPTSTEEGTSESATPESGPGTSTEPSEGSAPGTSESATPESGPGSEPATSGSETPGTSESATPESGPGSEPATSGSETPGTSESATPESGPGTSTEPSEGSAPGSPAGSPTSTEEGTSESATPESGPGSEPATSGSETPGTSESATPESGPGSPAGSPTSTEEGSPAGSPTSTEEGTSTEPSEGSAPGTSESATPESGPGTSESATPESGPGTSESATPESGPGSEPATSGSETPGSEPATSGSETPGSPAGSPTSTEEGTSTEPSEGSAPGTSTEPS |

| XTEN Name | Amino Acid Sequence |
|---|---|
| AE432A | EGSAPGSPAGSPTSTEEGTSTEPSEGSAPGTSESATPESGPGSEPATSGSETPGTSESATPESGP GSEPATSGSETPGTSESATPESGPGTSTEPSEGSAPGTSESATPESGPGSPAGSPTSTEEGSPAG SPTSTEEGSPAGSPTSTEEGTSESATPESGPGTSTEPSEGSAPGTSESATPESGPGSEPATSGSE TPGTSESATPESGPGSEPATSGSETPGTSESATPESGPGTSTEPSEGSAPGSPAGSPTSTEEGTS ESATPESGPGSEPATSGSETPGTSESATPESGPGSPAGSPTSTEEGSPAGSPTSTEEGTSTEPSE GSAPGTSESATPESGPGTSESATPESGPGTSESATPESGPGSEPATSGSETPGSEPATSGSETPG SPAGSPTSTEEGTSTEPSEGSAPGTSTEPSEGSAPGSEPATS |
| AE468A | EGSAPGTSTEPSEGSAPGTSTEPSEGSAPGSPAGSPTSTEEGTSTEPSEGSAPGTSESATPESGP GSEPATSGSETPGTSESATPESGPGSEPATSGSETPGTSESATPESGPGTSTEPSEGSAPGTSES ATPESGPGSPAGSPTSTEEGSPAGSPTSTEEGSPAGSPTSTEEGTSESATPESGPGTSTEPSEGS APGTSESATPESGPGSEPATSGSETPGTSESATPESGPGSEPATSGSETPGTSESATPESGPGTS TEPSEGSAPGSPAGSPTSTEEGTSESATPESGPGSEPATSGSETPGTSESATPESGPGSPAGSPTS TEEGSPAGSPTSTEEGTSTEPSEGSAPGTSESATPESGPGTSESATPESGPGTSESATPESGPGSE PATSGSETPGSEPATSGSETPGSPAGSPTSTEEGTSTEPSEGSAPGTSTEPSEGSAPGSEPATSGS ETPGTSESAT |
| AE504A | EGSAPGTSTEPSEGSAPGTSTEPSEGSAPGTSTEPSEGSAPGTSTEPSEGSAPGSPAGSPTSTEE GTSTEPSEGSAPGTSESATPESGPGSEPATSGSETPGTSESATPESGPGSEPATSGSETPGTSES ATPESGPGTSTEPSEGSAPGTSESATPESGPGSPAGSPTSTEEGSPAGSPTSTEEGSPAGSPTSTE EGTSESATPESGPGTSTEPSEGSAPGTSESATPESGPGSEPATSGSETPGTSESATPESGPGSEP ATSGSETPGTSESATPESGPGTSTEPSEGSAPGSPAGSPTSTEEGTSESATPESGPGSEPATSGS ETPGTSESATPESGPGSPAGSPTSTEEGSPAGSPTSTEEGTSTEPSEGSAPGTSESATPESGPGTS ESATPESGPGTSESATPESGPGSEPATSGSETPGSEPATSGSETPGSPAGSPTSTEEGTSTEPSEG SAPGTSTEPSEGSAPGSEPATSGSETPGTSESATPESGPGTSTEPS |
| AE540A | TPESGPGSPAGSPTSTEEGTSESATPESGPGSEPATSGSETPGTSESATPESGPGTSTEPSEGSAP GTSTEPSEGSAPGTSTEPSEGSAPGTSTEPSEGSAPGTSTEPSEGSAPGTSTEPSEGSAPGSPAG SPTSTEEGTSTEPSEGSAPGTSESATPESGPGSEPATSGSETPGTSESATPESGPGSEPATSGSET PGTSESATPESGPGTSTEPSEGSAPGTSESATPESGPGSPAGSPTSTEEGSPAGSPTSTEEGSPA GSPTSTEEGTSESATPESGPGTSTEPSEGSAPGTSESATPESGPGSEPATSGSETPGTSESATPES GPGSEPATSGSETPGTSESATPESGPGTSTEPSEGSAPGSPAGSPTSTEEGTSESATPESGPGSEP ATSGSETPGTSESATPESGPGSPAGSPTSTEEGSPAGSPTSTEEGTSTEPSEGSAPGTSESATPES GPGTSESATPESGPGTSESATPESGPGSEPATSGSETPGSEPATSGSETPGSPAGSPTSTEEGTS TEPSEGSAPGTSTEP |

| XTEN Name | Amino Acid Sequence |
|---|---|
| AE576A | TPESGPGTSESATPESGPGSPAGSPTSTEEGTSESATPESGPGSEPATSGSETPGTSESATPESGP GTSTEPSEGSAPGTSTEPSEGSAPGTSTEPSEGSAPGTSTEPSEGSAPGTSTEPSEGSAPGTSTEP SEGSAPGSPAGSPTSTEEGTSTEPSEGSAPGTSESATPESGPGSEPATSGSETPGTSESATPESGP GSEPATSGSETPGTSESATPESGPGTSTEPSEGSAPGTSESATPESGPGSPAGSPTSTEEGSPAG SPTSTEEGSPAGSPTSTEEGTSESATPESGPGTSTEPSEGSAPGTSESATPESGPGSEPATSGSET PGTSESATPESGPGSEPATSGSETPGTSESATPESGPGTSTEPSEGSAPGSPAGSPTSTEEGTSES ATPESGPGSEPATSGSETPGTSESATPESGPGSPAGSPTSTEEGSPAGSPTSTEEGTSTEPSEGS APGTSESATPESGPGTSESATPESGPGTSESATPESGPGSEPATSGSETPGSEPATSGSETPGSP AGSPTSTEEGTSTEPSEGSAPGTSTEPSEGSAPGSEPATSGSETPGTSESA |
| AE612A | GSETPGTSTEPSEGSAPGTSTEPSEGSAPGTSESATPESGPGTSESATPESGPGSPAGSPTSTEE GTSESATPESGPGSEPATSGSETPGTSESATPESGPGTSTEPSEGSAPGTSTEPSEGSAPGTSTEP SEGSAPGTSTEPSEGSAPGTSTEPSEGSAPGTSTEPSEGSAPGSPAGSPTSTEEGTSTEPSEGSAP GTSESATPESGPGSEPATSGSETPGTSESATPESGPGSEPATSGSETPGTSESATPESGPGTSTEP SEGSAPGTSESATPESGPGSPAGSPTSTEEGSPAGSPTSTEEGSPAGSPTSTEEGTSESATPESGP |
| | GTSTEPSEGSAPGTSESATPESGPGSEPATSGSETPGTSESATPESGPGSEPATSGSETPGTSES ATPESGPGTSTEPSEGSAPGSPAGSPTSTEEGTSESATPESGPGSEPATSGSETPGTSESATPES GPGSPAGSPTSTEEGSPAGSPTSTEEGTSTEPSEGSAPGTSESATPESGPGTSESATPESGPGTS ESATPESGPGSEPATSGSETPGSEPATSGSETPGSPAGSPTSTEEGTSTEPSEGSAPGTSTEPSEG SAPGSEPATSGSETPGTSESAT |
| AE648A | PESGPGTSTEPSEGSAPGTSESATPESGPGSEPATSGSETPGTSTEPSEGSAPGTSTEPSEGSAPG TSESATPESGPGTSESATPESGPGSPAGSPTSTEEGTSESATPESGPGSEPATSGSETPGTSESAT PESGPGTSTEPSEGSAPGTSTEPSEGSAPGTSTEPSEGSAPGTSTEPSEGSAPGTSTEPSEGSAPG TSTEPSEGSAPGSPAGSPTSTEEGTSTEPSEGSAPGTSESATPESGPGSEPATSGSETPGTSESAT PESGPGSEPATSGSETPGTSESATPESGPGTSTEPSEGSAPGTSESATPESGPGSPAGSPTSTEEG SPAGSPTSTEEGSPAGSPTSTEEGTSESATPESGPGTSTEPSEGSAPGTSESATPESGPGSEPATS GSETPGTSESATPESGPGSEPATSGSETPGTSESATPESGPGTSTEPSEGSAPGSPAGSPTSTEE GTSESATPESGPGSEPATSGSETPGTSESATPESGPGSPAGSPTSTEEGSPAGSPTSTEEGTSTEP SEGSAPGTSESATPESGPGTSESATPESGPGTSESATPESGPGSEPATSGSETPGSEPATSGSETP GSPAGSPTSTEEGTSTEPSEGSAPGTSTEPSEGSAPGSEPATSGSETPGTSESAT |

| XTEN Name | Amino Acid Sequence |
|---|---|
| AE684A | EGSAPGSPAGSPTSTEEGTSTEPSEGSAPGTSTEPSEGSAPGTSESATPESGPGTSTEPSEGSAP GTSESATPESGPGSEPATSGSETPGTSTEPSEGSAPGTSTEPSEGSAPGTSESATPESGPGTSES ATPESGPGSPAGSPTSTEEGTSESATPESGPGSEPATSGSETPGTSESATPESGPGTSTEPSEGS APGTSTEPSEGSAPGTSTEPSEGSAPGTSTEPSEGSAPGTSTEPSEGSAPGTSTEPSEGSAPGSP AGSPTSTEEGTSTEPSEGSAPGTSESATPESGPGSEPATSGSETPGTSESATPESGPGSEPATSG SETPGTSESATPESGPGTSTEPSEGSAPGTSESATPESGPGSPAGSPTSTEEGSPAGSPTSTEEGS PAGSPTSTEEGTSESATPESGPGTSTEPSEGSAPGTSESATPESGPGSEPATSGSETPGTSESATP ESGPGSEPATSGSETPGTSESATPESGPGTSTEPSEGSAPGSPAGSPTSTEEGTSESATPESGPGS EPATSGSETPGTSESATPESGPGSPAGSPTSTEEGSPAGSPTSTEEGTSTEPSEGSAPGTSESATP ESGPGTSESATPESGPGTSESATPESGPGSEPATSGSETPGSEPATSGSETPGSPAGSPTSTEEG TSTEPSEGSAPGTSTEPSEGSAPGSEPATS |
| AE720A | TSGSETPGSEPATSGSETPGSPAGSPTSTEEGTSESATPESGPGTSTEPSEGSAPGTSTEPSEGSA PGSPAGSPTSTEEGTSTEPSEGSAPGTSTEPSEGSAPGTSESATPESGPGTSTEPSEGSAPGTSES ATPESGPGSEPATSGSETPGTSTEPSEGSAPGTSTEPSEGSAPGTSESATPESGPGTSESATPES GPGSPAGSPTSTEEGTSESATPESGPGSEPATSGSETPGTSESATPESGPGTSTEPSEGSAPGTS TEPSEGSAPGTSTEPSEGSAPGTSTEPSEGSAPGTSTEPSEGSAPGSPAGSPTS TEEGTSTEPSEGSAPGTSESATPESGPGSEPATSGSETPGTSESATPESGPGSEPATSGSETPGTS ESATPESGPGTSTEPSEGSAPGTSESATPESGPGSPAGSPTSTEEGSPAGSPTSTEEGSPAGSPTS TEEGTSESATPESGPGTSTEPSEGSAPGTSESATPESGPGSEPATSGSETPGTSESATPESGPGSE PATSGSETPGTSESATPESGPGTSTEPSEGSAPGSPAGSPTSTEEGTSESATPESGPGSEPATSGS ETPGTSESATPESGPGSPAGSPTSTEEGSPAGSPTSTEEGTSTEPSEGSAPGTSESATPESGPGTS ESATPESGPGTSESATPESGPGSEPATSGSETPGSEPATSGSETPGSPAGSPTSTEEGTSTE |
| AE756A | TSGSETPGSEPATSGSETPGSPAGSPTSTEEGTSESATPESGPGTSTEPSEGSAPGTSTEPSEGSA PGSPAGSPTSTEEGTSTEPSEGSAPGTSTEPSEGSAPGTSESATPESGPGTSTEPSEGSAPGTSES ATPESGPGSEPATSGSETPGTSTEPSEGSAPGTSTEPSEGSAPGTSESATPESGPGTSESATPES GPGSPAGSPTSTEEGTSESATPESGPGSEPATSGSETPGTSESATPESGPGTSTEPSEGSAPGTS TEPSEGSAPGTSTEPSEGSAPGTSTEPSEGSAPGTSTEPSEGSAPGSPAGSPTS TEEGTSTEPSEGSAPGTSESATPESGPGSEPATSGSETPGTSESATPESGPGSEPATSGSETPGTS ESATPESGPGTSTEPSEGSAPGTSESATPESGPGSPAGSPTSTEEGSPAGSPTSTEEGSPAGSPTS TEEGTSESATPESGPGTSTEPSEGSAPGTSESATPESGPGSEPATSGSETPGTSESATPESGPGSE PATSGSETPGTSESATPESGPGTSTEPSEGSAPGSPAGSPTSTEEGTSESATPESGPGSEPATSGS ETPGTSESATPESGPGSPAGSPTSTEEGSPAGSPTSTEEGTSTEPSEGSAPGTSESATPESGPGTS ESATPESGPGTSESATPESGPGSEPATSGSETPGSEPATSGSETPGSPAGSPTSTEEGTSTEPSEG SAPGTSTEPSEGSAPGSEPATSGSETPGTSES |

| XTEN Name | Amino Acid Sequence |
|---|---|
| AE792A | EGSAPGTSESATPESGPGSEPATSGSETPGSEPATSGSETPGSPAGSPTSTEEGTSESATPESGP<br>GTSTEPSEGSAPGTSTEPSEGSAPGSPAGSPTSTEEGTSTEPSEGSAPGTSTEPSEGSAPGTSES<br>ATPESGPGTSTEPSEGSAPGTSESATPESGPGSEPATSGSETPGTSTEPSEGSAPGTSTEPSEGS<br>APGTSESATPESGPGTSESATPESGPGSPAGSPTSTEEGTSESATPESGPGSEPATSGSETPGTS<br>ESATPESGPGTSTEPSEGSAPGTSTEPSEGSAPGTSTEPSEGSAPGTSTEPSEGSAPGTSTEPSEG<br>SAPGTSTEPSEGSAPGSPAGSPTSTEEGTSTEPSEGSAPGTSESATPESGPGSEPATSGSETPGTS<br>ESATPESGPGSEPATSGSETPGTSESATPESGPGTSTEPSEGSAPGTSESATPESGPGSPAGSPTS<br>TEEGSPAGSPTSTEEGSPAGSPTSTEEGTSESATPESGPGTSTEPSEGSAPGTSESATPESGPGSE<br>PATSGSETPGTSESATPESGPGSEPATSGSETPGTSESATPESGPGTSTEPSEGSAPGSPAGSPTS<br>TEEGTSESATPESGPGSEPATSGSETPGTSESATPESGPGSPAGSPTSTEEGSPAGSPTSTEEGTS |
| | TEPSEGSAPGTSESATPESGPGTSESATPESGPGTSESATPESGPGSEPATSGSETPGSEPATSGS<br>ETPGSPAGSPTSTEEGTSTEPSEGSAPGTSTEPSEGSAPGSEPATSGSETPGTSESATPESGPGTS<br>TEPS |
| AE828A | PESGPGTSTEPSEGSAPGSPAGSPTSTEEGTSTEPSEGSAPGTSTEPSEGSAPGTSESATPESGPG<br>SEPATSGSETPGSEPATSGSETPGSPAGSPTSTEEGTSESATPESGPGTSTEPSEGSAPGTSTEPS<br>EGSAPGSPAGSPTSTEEGTSTEPSEGSAPGTSTEPSEGSAPGTSESATPESGPGTSTEPSEGSAP<br>GTSESATPESGPGSEPATSGSETPGTSTEPSEGSAPGTSTEPSEGSAPGTSESATPESGPGTSES<br>ATPESGPGSPAGSPTSTEEGTSESATPESGPGSEPATSGSETPGTSESATPESGPGTSTEPSEGS<br>APGTSTEPSEGSAPGTSTEPSEGSAPGTSTEPSEGSAPGTSTEPSEGSAPGTSTEPSEGSAPGSP<br>AGSPTSTEEGTSTEPSEGSAPGTSESATPESGPGSEPATSGSETPGTSESATPESGPGSEPATSG<br>SETPGTSESATPESGPGTSTEPSEGSAPGTSESATPESGPGSPAGSPTSTEEGSPAGSPTSTEEGS<br>PAGSPTSTEEGTSESATPESGPGTSTEPSEGSAPGTSESATPESGPGSEPATSGSETPGTSESATP<br>ESGPGSEPATSGSETPGTSESATPESGPGTSTEPSEGSAPGSPAGSPTSTEEGTSESATPESGPGS<br>EPATSGSETPGTSESATPESGPGSPAGSPTSTEEGSPAGSPTSTEEGTSTEPSEGSAPGTSESATP<br>ESGPGTSESATPESGPGTSESATPESGPGSEPATSGSETPGSEPATSGSETPGSPAGSPTSTEEG<br>TSTEPSEGSAPGTSTEPSEGSAPGSEPATSGSETPGTSESAT |
| AG72A | GPGSSPSASTGTGPGTPGSGTASSSPGSSTPSGATGSPGSSPSASTGTGPGASPGTSSTGSPGTP<br>GSGTASS |
| AG72B | GSSTPSGATGSPGSSTPSGATGSPGSSPSASTGTGPGSSPSASTGTGPGASPGTSSTGSPGTPGS<br>GTASSSP |
| AG72C | SPSASTGTGPGASPGTSSTGSPGSSPSASTGTGPGTPGSGTASSSPGSSTPSGATGSPGSSTPSG<br>ATGSPGA |

| XTEN Name | Amino Acid Sequence |
|---|---|
| AG108A | SASTGTGPGSSPSASTGTGPGTPGSGTASSSPGSSTPSGATGSPGSSPSASTGTGPGASPGTSST GSPGTPGSGTASSSPGSSTPSGATGSPGTPGSGTASSSPGASP |
| AG108B | PGTPGSGTASSSPGSSTPSGATGSPGTPGSGTASSSPGSSTPSGATGSPGSSTPSGATGSPGSSP SASTGTGPGSSPSASTGTGPGASPGTSSTGSPGTPGSGTASSS |
| AG144A | PGSSPSASTGTGPGSSPSASTGTGPGTPGSGTASSSPGSSTPSGATGSPGSSPSASTGTGPGASP GTSSTGSPGTPGSGTASSSPGSSTPSGATGSPGTPGSGTASSSPGASPGTSSTGSPGASPGTSST GSPGTPGSGTASSS |
| AG144B | PSGATGSPGTPGSGTASSSPGSSTPSGATGSPGSSTPSGATGSPGSSPSASTGTGPGSSPSASTG TGPGASPGTSSTGSPGTPGSGTASSSPGSSTPSGATGSPGSSPSASTGTGPGSSPSASTGTGPGA SPGTSSTGSPGASP |
| AG180A | TSSTGSPGSSPSASTGTGPGSSPSASTGTGPGTPGSGTASSSPGSSTPSGATGSPGSSPSASTGT GPGASPGTSSTGSPGTPGSGTASSSPGSSTPSGATGSPGTPGSGTASSSPGASPGTSSTGSPGAS PGTSSTGSPGTPGSGTASSSPGSSTPSGATGSPGASPGTSSTGSPGTPGS |
| AG216A | TGTGPGSSPSASTGTGPGTPGSGTASSSPGSSTPSGATGSPGSSPSASTGTGPGASPGTSSTGSP GTPGSGTASSSPGSSTPSGATGSPGTPGSGTASSSPGASPGTSSTGSPGASPGTSSTGSPGTPGS GTASSSPGSSTPSGATGSPGASPGTSSTGSPGTPGSGTASSSPGSSTPSGATGSPGSSPSASTGT GPGSSPSASTGTGPGSSTPSG |
| AG252A | TSSTGSPGSSPSASTGTGPGSSPSASTGTGPGTPGSGTASSSPGSSTPSGATGSPGSSPSASTGT GPGASPGTSSTGSPGTPGSGTASSSPGSSTPSGATGSPGTPGSGTASSSPGASPGTSSTGSPGAS PGTSSTGSPGTPGSGTASSSPGSSTPSGATGSPGASPGTSSTGSPGTPGSGTASSSPGSSTPSGA TGSPGSSPSASTGTGPGSSPSASTGTGPGSSTPSGATGSPGSSTPSGATGSPGASPG |
| AG288A | TSSTGSPGSSPSASTGTGPGSSPSASTGTGPGTPGSGTASSSPGSSTPSGATGSPGSSPSASTGT GPGASPGTSSTGSPGTPGSGTASSSPGSSTPSGATGSPGTPGSGTASSSPGASPGTSSTGSPGAS PGTSSTGSPGTPGSGTASSSPGSSTPSGATGSPGASPGTSSTGSPGTPGSGTASSSPGSSTPSGA TGSPGSSPSASTGTGPGSSPSASTGTGPGSSTPSGATGSPGSSTPSGATGSPGASPGTSSTGSPG ASPGTSSTGSPGASPGTSSTGSPGTPGS |
| AG324A | TSSTGSPGTPGSGTASSSPGASPGTSSTGSPGASPGTSSTGSPGASPGTSSTGSPGASPGTSSTG SPGTPGSGTASSSPGSSTPSGATGSPGTPGSGTASSSPGSSTPSGATGSPGTPGSGTASSSPGSS TPSGATGSPGSSTPSGATGSPGSSPSASTGTGPGSSPSASTGTGPGASPGTSSTGSPGTPGSGTA SSSPGSSTPSGATGSPGSSPSASTGTGPGSSPSASTGTGPGASPGTSSTGSPGASPGTSSTGSPG SSTPSGATGSPGSSPSASTGTGPGASPGTSSTGSPGSSPSASTGTGPGTPGSGTASSSPGSSTP |

EP 3 431 098 A1

(continued)

| XTEN Name | Amino Acid Sequence |
|---|---|
| AG360A | TSSTGSPGASPGTSSTGSPGTPGSGTASSSPGASPGTSSTGSPGASPGTSSTGSPGASPGTSSTG SPGASPGTSSTGSPGTPGSGTASSSPGSSTPSGATGSPGTPGSGTASSSPGSSTPSGATGSPGTP GSGTASSSPGSSTPSGATGSPGSSTPSGATGSPGSSPSASTGTGPGSSPSASTGTGPGASPGTSS TGSPGTPGSGTASSSPGSSTPSGATGSPGSSPSASTGTGPGSSPSASTGTGPGASPGTSSTGSPG ASPGTSSTGSPGSSTPSGATGSPGSSPSASTGTGPGASPGTSSTGSPGSSPSASTGTGPGTPGSG TASSSPGSSTPSGATGSPGSSTPSGATGSPGASPG |
| AG396A | GATGSPGSSTPSGATGSPGSSPSASTGTGPGASPGTSSTGSPGASPGTSSTGSPGTPGSGTASSS PGASPGTSSTGSPGASPGTSSTGSPGASPGTSSTGSPGASPGTSSTGSPGTPGSGTASSSPGSST PSGATGSPGTPGSGTASSSPGSSTPSGATGSPGTPGSGTASSSPGSSTPSGATGSPGSSTPSGAT GSPGSSPSASTGTGPGSSPSASTGTGPGASPGTSSTGSPGTPGSGTASSSPGSSTPSGATGSPGS SPSASTGTGPGSSPSASTGTGPGASPGTSSTGSPGASPGTSSTGSPGSSTPSGATGSPGSSPSAS TGTGPGASPGTSSTGSPGSSPSASTGTGPGTPGSGTASSSPGSSTPSGATGSPGSSTPSGATGSP GASPGT |
| AG432A | GATGSPGSSTPSGATGSPGASPGTSSTGSPGTPGSGTASSSPGSSTPSGATGSPGSSTPSGATGS PGSSTPSGATGSPGSSPSASTGTGPGASPGTSSTGSPGASPGTSSTGSPGTPGSGTASSSPGASP GTSSTGSPGASPGTSSTGSPGASPGTSSTGSPGASPGTSSTGSPGTPGSGTASSSPGSSTPSGAT GSPGTPGSGTASSSPGSSTPSGATGSPGTPGSGTASSSPGSSTPSGATGSPGSSTPSGATGSPGS SPSASTGTGPGSSPSASTGTGPGASPGTSSTGSPGTPGSGTASSSPGSSTPSGATGSPGSSPSAS TGTGPGSSPSASTGTGPGASPGTSSTGSPGASPGTSSTGSPGSSTPSGATGSPGSSPSASTGTGP GASPGTSSTGSPGSSPSASTGTGPGTPGSGTASSSPGSSTPS |
| AG468A | TSSTGSPGSSPSASTGTGPGTPGSGTASSSPGASPGTSSTGSPGASPGTSSTGSPGASPGTSSTG SPGSSTPSGATGSPGSSTPSGATGSPGASPGTSSTGSPGTPGSGTASSSPGSSTPSGATGSPGSS TPSGATGSPGSSTPSGATGSPGSSPSASTGTGPGASPGTSSTGSPGASPGTSSTGSPGTPGSGTA SSSPGASPGTSSTGSPGASPGTSSTGSPGASPGTSSTGSPGASPGTSSTGSPGTPGSGTASSSPG SSTPSGATGSPGTPGSGTASSSPGSSTPSGATGSPGTPGSGTASSSPGSSTPSGATGSPGSSTPS GATGSPGSSPSASTGTGPGSSPSASTGTGPGASPGTSSTGSPGTPGSGTASSSPGSSTPSGATGS PGSSPSASTGTGPGSSPSASTGTGPGASPGTSSTGSPGASPGTSSTGSPGSSTPSGATGSPGSSP SASTGTGPGASPG |

EP 3 431 098 A1

(continued)

| XTEN Name | Amino Acid Sequence |
|---|---|
| AG504A | TSSTGSPGSSPSASTGTGPGTPGSGTASSSPGASPGTSSTGSPGASPGTSSTGSPGASPGTSSTG SPGSSTPSGATGSPGSSTPSGATGSPGASPGTSSTGSPGTPGSGTASSSPGSSTPSGATGSPGSS TPSGATGSPGSSTPSGATGSPGSSPSASTGTGPGASPGTSSTGSPGASPGTSSTGSPGTPGSGTA SSSPGASPGTSSTGSPGASPGTSSTGSPGASPGTSSTGSPGASPGTSSTGSPGTPGSGTASSSPG SSTPSGATGSPGTPGSGTASSSPGSSTPSGATGSPGTPGSGTASSSPGSSTPSGATGSPGSSTPS GATGSPGSSPSASTGTGPGSSPSASTGTGPGASPGTSSTGSPGTPGSGTASSSPGSSTPSGATGS PGSSPSASTGTGPGSSPSASTGTGPGASPGTSSTGSPGASPGTSSTGSPGSSTPSGATGSPGSSP SASTGTGPGASPGTSSTGSPGSSPSASTGTGPGTPGSGTASSSPGSSTP |
| AG540A | TSSTGSPGASPGTSSTGSPGSSPSASTGTGPGTPGSGTASSSPGASPGTSSTGSPGASPGTSSTG SPGASPGTSSTGSPGSSTPSGATGSPGSSTPSGATGSPGASPGTSSTGSPGTPGSGTASSSPGSS TPSGATGSPGSSTPSGATGSPGSSTPSGATGSPGSSPSASTGTGPGASPGTSSTGSPGASPGTSS TGSPGTPGSGTASSSPGASPGTSSTGSPGASPGTSSTGSPGASPGTSSTGSPGASPGTSSTGSPG TPGSGTASSSPGSSTPSGATGSPGTPGSGTASSSPGSSTPSGATGSPGTPGSGTASSSPGSSTPS GATGSPGSSTPSGATGSPGSSPSASTGTGPGSSPSASTGTGPGASPGTSSTGSPGTPGSGTASSS PGSSTPSGATGSPGSSPSASTGTGPGSSPSASTGTGPGASPGTSSTGSPGASPGTSSTGSPGSST PSGATGSPGSSPSASTGTGPGASPGTSSTGSPGSSPSASTGTGPGTPGSGTASSSPGSSTPSGAT GSPGSSTPSGATGSPGASPG |
| AG576A | TSSTGSPGTPGSGTASSSPGASPGTSSTGSPGASPGTSSTGSPGASPGTSSTGSPGSSPSASTGT GPGTPGSGTASSSPGASPGTSSTGSPGASPGTSSTGSPGASPGTSSTGSPGSSTPSGATGSPGSS TPSGATGSPGASPGTSSTGSPGTPGSGTASSSPGSSTPSGATGSPGSSTPSGATGSPGSSTPSGA TGSPGSSPSASTGTGPGASPGTSSTGSPGASPGTSSTGSPGTPGSGTASSSPGASPGTSSTGSPG ASPGTSSTGSPGASPGTSSTGSPGASPGTSSTGSPGTPGSGTASSSPGSSTPSGATGSPGTPGSG TASSSPGSSTPSGATGSPGTPGSGTASSSPGSSTPSGATGSPGSSTPSGATGSPGSSPSASTGTG PGSSPSASTGTGPGASPGTSSTGSPGTPGSGTASSSPGSSTPSGATGSPGSSPSASTGTGPGSSP SASTGTGPGASPGTSSTGSPGASPGTSSTGSPGSSTPSGATGSPGSSPSASTGTGPGASPGTSST GSPGSSPSASTGTGPGTPGSGTASSSPGSSTPSGATGSPGSSTPSGATGSPGASPG |
| AG612A | STGSPGTPGSGTASSSPGSSTPSGATGSPGASPGTSSTGSPGTPGSGTASSSPGSSTPSGATGSP GSSPSASTGTGPGSSPSASTGTGPGSSTPSGATGSPGSSTPSGATGSPGASPGTSSTGSPGASPG TSSTGSPGASPGTSSTGSPGTPGSGTASSSPGASPGTSSTGSPGASPGTSSTGSPGASPGTSSTG SPGSSPSASTGTGPGTPGSGTASSSPGASPGTSSTGSPGASPGTSSTGSPGASPGTSSTGSPGSS TPSGATGSPGSSTPSGATGSPGASPGTSSTGSPGTPGSGTASSSPGSSTPSGATGSPGSSTPSGA TGSPGSSTPSGATGSPGSSPSASTGTGPGASPGTSSTGSPGASPGTSSTGSPGTPGSGTASSSPG ASPGTSSTGSPGASPGTSSTGSPGASPGTSSTGSPGASPGTSSTGSPGTPGSGTASSSPGSSTPS GATGSPGTPGSGTASSSPGSSTPSGATGSPGTPGSGTASSSPGSSTPSGATGSPGSSTPSGATGS PGSSPSASTGTGPGSSPSASTGTGPGASPGTSSTGSPGTPGSGTASSSPGSSTPSGATGSPGSSP SASTGTGPGSSPSASTGTGPGASPGTS |

EP 3 431 098 A1

| XTEN Name | Amino Acid Sequence |
|---|---|
| AG648A | GTASSSPGSSTPSGATGSPGSSPSASTGTGPGSSPSASTGTGPGSSTPSGATGSPGSSTPSGATG |
|  | SPGASPGTSSTGSPGASPGTSSTGSPGASPGTSSTGSPGTPGSGTASSSPGASPGTSSTGSPGAS PGTSSTGSPGASPGTSSTGSPGSSPSASTGTGPGTPGSGTASSSPGASPGTSSTGSPGASPGTSS TGSPGASPGTSSTGSPGSSTPSGATGSPGSSTPSGATGSPGASPGTSSTGSPGTPGSGTASSSPG SSTPSGATGSPGSSTPSGATGSPGSSTPSGATGSPGSSPSASTGTGPGASPGTSSTGSPGASPGT SSTGSPGTPGSGTASSSPGASPGTSSTGSPGASPGTSSTGSPGASPGTSSTGSPGASPGTSSTGS PGTPGSGTASSSPGSSTPSGATGSPGTPGSGTASSSPGSSTPSGATGSPGTPGSGTASSSPGSST PSGATGSPGSSTPSGATGSPGSSPSASTGTGPGSSPSASTGTGPGASPGTSSTGSPGTPGSGTAS SSPGSSTPSGATGSPGSSPSASTGTGPGSSPSASTGTGPGASPGTSSTGSPGASPGTSSTGSPGS STPSGATGSPGSSPSASTGTGPGASPGTSSTGSPGSSPSASTGTGPGTPGSGTASSSPGSSTP |
| AG684A | TSSTGSPGTPGSGTASSSPGSSTPSGATGSPGSSPSASTGTGPGSSPSASTGTGPGSSTPSGATG SPGSSTPSGATGSPGASPGTSSTGSPGASPGTSSTGSPGASPGTSSTGSPGTPGSGTASSSPGAS PGTSSTGSPGASPGTSSTGSPGASPGTSSTGSPGSSPSASTGTGPGTPGSGTASSSPGASPGTSS TGSPGASPGTSSTGSPGASPGTSSTGSPGSSTPSGATGSPGSSTPSGATGSPGASPGTSSTGSPG TPGSGTASSSPGSSTPSGATGSPGSSTPSGATGSPGSSTPSGATGSPGSSPSASTGTGPGASPGT SSTGSPGASPGTSSTGSPGTPGSGTASSSPGASPGTSSTGSPGASPGTSSTGSPGASPGTSSTGS PGASPGTSSTGSPGTPGSGTASSSPGSSTPSGATGSPGTPGSGTASSSPGSSTPSGATGSPGTPG SGTASSSPGSSTPSGATGSPGSSTPSGATGSPGSSPSASTGTGPGSSPSASTGTGPGASPGTSST GSPGTPGSGTASSSPGSSTPSGATGSPGSSPSASTGTGPGSSPSASTGTGPGASPGTSSTGSPGA SPGTSSTGSPGSSTPSGATGSPGSSPSASTGTGPGASPGTSSTGSPGSSPSASTGTGPGTPGSGT ASSSPGSSTPSGATGSPGSSTPSGATGSPGASPG |
| AG720A | TSSTGSPGTPGSGTASSSPGSSTPSGATGSPGASPGTSSTGSPGTPGSGTASSSPGSSTPSGATG SPGSSPSASTGTGPGSSPSASTGTGPGSSTPSGATGSPGSSTPSGATGSPGASPGTSSTGSPGAS PGTSSTGSPGASPGTSSTGSPGTPGSGTASSSPGASPGTSSTGSPGASPGTSSTGSPGASPGTSS TGSPGSSPSASTGTGPGTPGSGTASSSPGASPGTSSTGSPGASPGTSSTGSPGASPGTSSTGSPG SSTPSGATGSPGSSTPSGATGSPGASPGTSSTGSPGTPGSGTASSSPGSSTPSGATGSPGSSTPS GATGSPGSSTPSGATGSPGSSPSASTGTGPGASPGTSSTGSPGASPGTSSTGSPGTPGSGTASSS PGASPGTSSTGSPGASPGTSSTGSPGASPGTSSTGSPGASPGTSSTGSPGTPGSGTASSSPGSST PSGATGSPGTPGSGTASSSPGSSTPSGATGSPGTPGSGTASSSPGSSTPSGATGSPGSSTPSGAT GSPGSSPSASTGTGPGSSPSASTGTGPGASPGTSSTGSPGTPGSGTASSSPGSSTPSGATGSPGS SPSASTGTGPGSSPSASTGTGPGASPGTSSTGSPGASPGTSSTGSPGSSTPSGATGSPGSSPSAS TGTGPGASPGTSSTGSPGSSPSASTGTGPGTPGSGTASSSPGSSTPSGATGSPGSSTPSGATGSP GASPG |

| XTEN Name | Amino Acid Sequence |
|---|---|
| AG756A | TSSTGSPGSSPSASTGTGPGSSPSASTGTGPGTPGSGTASSSPGSSTPSGATGSPGSSPSASTGT GPGASPGTSSTGSPGTPGSGTASSSPGSSTPSGATGSPGTPGSGTASSSPGASPGTSSTGSPGAS PGTSSTGSPGTPGSGTASSSPGSSTPSGATGSPGASPGTSSTGSPGTPGSGTASSSPGSSTPSGA TGSPGSSPSASTGTGPGSSPSASTGTGPGSSTPSGATGSPGSSTPSGATGSPGASPGTSSTGSPG ASPGTSSTGSPGASPGTSSTGSPGTPGSGTASSSPGASPGTSSTGSPGASPGTSSTGSPGASPGT SSTGSPGSSPSASTGTGPGTPGSGTASSSPGASPGTSSTGSPGASPGTSSTGSPGASPGTSSTGS PGSSTPSGATGSPGSSTPSGATGSPGASPGTSSTGSPGTPGSGTASSSPGSSTPSGATGSPGSST PSGATGSPGSSTPSGATGSPGSSPSASTGTGPGASPGTSSTGSPGASPGTSSTGSPGTPGSGTAS SSPGASPGTSSTGSPGASPGTSSTGSPGASPGTSSTGSPGASPGTSSTGSPGTPGSGTASSSPGS STPSGATGSPGTPGSGTASSSPGSSTPSGATGSPGTPGSGTASSSPGSSTPSGATGSPGSSTPSG ATGSPGSSPSASTGTGPGSSPSASTGTGPGASPGTSSTGSPGTPGSGTASSSPGSSTPSGATGSP GSSPSASTGTGPGSSPSASTGTGPGASPGTSSTGSPGASPG |
| AG792A | TSSTGSPGSSPSASTGTGPGSSPSASTGTGPGTPGSGTASSSPGSSTPSGATGSPGSSPSASTGT GPGASPGTSSTGSPGTPGSGTASSSPGSSTPSGATGSPGTPGSGTASSSPGASPGTSSTGSPGAS PGTSSTGSPGTPGSGTASSSPGSSTPSGATGSPGASPGTSSTGSPGTPGSGTASSSPGSSTPSGA TGSPGSSPSASTGTGPGSSPSASTGTGPGSSTPSGATGSPGSSTPSGATGSPGASPGTSSTGSPG ASPGTSSTGSPGASPGTSSTGSPGTPGSGTASSSPGASPGTSSTGSPGASPGTSSTGSPGASPGT SSTGSPGSSPSASTGTGPGTPGSGTASSSPGASPGTSSTGSPGASPGTSSTGSPGASPGTSSTGS PGSSTPSGATGSPGSSTPSGATGSPGASPGTSSTGSPGTPGSGTASSSPGSSTPSGATGSPGSST PSGATGSPGSSTPSGATGSPGSSPSASTGTGPGASPGTSSTGSPGASPGTSSTGSPGTPGSGTAS SSPGASPGTSSTGSPGASPGTSSTGSPGASPGTSSTGSPGASPGTSSTGSPGTPGSGTASSSPGS STPSGATGSPGTPGSGTASSSPGSSTPSGATGSPGTPGSGTASSSPGSSTPSGATGSPGSSTPSG ATGSPGSSPSASTGTGPGSSPSASTGTGPGASPGTSSTGSPGTPGSGTASSSPGSSTPSGATGSP GSSPSASTGTGPGSSPSASTGTGPGASPGTSSTGSPGASPGTSSTGSPGSSTPSGATGSPGSSPS ASTGTGPGASPG |
| AG828A | TSSTGSPGSSPSASTGTGPGSSPSASTGTGPGTPGSGTASSSPGSSTPSGATGSPGSSPSASTGT GPGASPGTSSTGSPGTPGSGTASSSPGSSTPSGATGSPGTPGSGTASSSPGASPGTSSTGSPGAS |

| XTEN Name | Amino Acid Sequence |
|---|---|
|  | PGTSSTGSPGTPGSGTASSSPGSSTPSGATGSPGASPGTSSTGSPGTPGSGTASSSPGSSTPSGA TGSPGSSPSASTGTGPGSSPSASTGTGPGSSTPSGATGSPGSSTPSGATGSPGASPGTSSTGSPG ASPGTSSTGSPGASPGTSSTGSPGTPGSGTASSSPGASPGTSSTGSPGASPGTSSTGSPGASPGT SSTGSPGSSPSASTGTGPGTPGSGTASSSPGASPGTSSTGSPGASPGTSSTGSPGASPGTSSTGS PGSSTPSGATGSPGSSTPSGATGSPGASPGTSSTGSPGTPGSGTASSSPGSSTPSGATGSPGSST PSGATGSPGSSTPSGATGSPGSSPSASTGTGPGASPGTSSTGSPGASPGTSSTGSPGTPGSGTAS SSPGASPGTSSTGSPGASPGTSSTGSPGASPGTSSTGSPGASPGTSSTGSPGTPGSGTASSSPGS STPSGATGSPGTPGSGTASSSPGSSTPSGATGSPGTPGSGTASSSPGSSTPSGATGSPGSSTPSG ATGSPGSSPSASTGTGPGSSPSASTGTGPGASPGTSSTGSPGTPGSGTASSSPGSSTPSGATGSP GSSPSASTGTGPGSSPSASTGTGPGASPGTSSTGSPGASPGTSSTGSPGSSTPSGATGSPGSSPS ASTGTGPGASPGTSSTGSPGSSPSASTGTGPGTPGSGTASSSPGSSTP |
| AG288_DE | GTPGSGTASSSPGSSTPSGATGSPGSSTPSGATGSPGSSTPSGATGSPGSSPSASTGTGPGASPG TSSTGSPGASPGTSSTGSPGTPGSGTASSSPGASPGTSSTGSPGASPGTSSTGSPGASPGTSSTG SPGASPGTSSTGSPGTPGSGTASSSPGSSTPSGATGSPGTPGSGTASSSPGSSTPSGATGSPGTP GSGTASSSPGSSTPSGATGSPGSSTPSGATGSPGSSPSASTGTGPGSSPSASTGTGPGASPGTSS TGSPGTPGSGTASSSPGSSTPSGATGSP |

In other embodiments, the GLP2-XTEN composition comprises one or more non-repetitive XTEN sequences of about 36 to about 3000 amino acid residues or about 144 to about 2000 amino acid residues or about 288 or about 1000 amino acid residues, wherein at least about 80%, or at least about 90%, or about 91%, or about 92%, or about 93%, or about 94%, or about 95%, or about 96%, or about 97%, or about 98%, or about 99% to about 100% of the sequence consists of non-overlapping 36 amino acid sequence motifs selected from one or more of the polypeptide sequences of Tables 8-11, either as a family sequence, or where motifs are selected from two or more families of motifs.

In those embodiments wherein the XTEN component of the GLP2-XTEN fusion protein has less than 100% of its amino acids consisting of four to six amino acid selected from glycine (G), alanine (A), serine (S), threonine (T), glutamate (E) and proline (P), or less than 100% of the sequence consisting of the sequence motifs from Table 3 or the sequences of Tables 4, and 8-12 or less than 100% sequence identity compared with an XTEN from Table 4, the other amino acid residues are selected from any other of the 14 natural L-amino acids, but are preferentially selected from hydrophilic amino acids such that the XTEN sequence contains at least about 90%, or at least about 91%, or at least about 92%, or at least about 93%, or at least about 94%, or at least about 95%, or at least about 96%, or at least about 97%, or at least about 98%, or at least about 99% hydrophilic amino acids. The XTEN amino acids that are not glycine (G), alanine (A), serine (S), threonine (T), glutamate (E) and proline (P) are interspersed throughout the XTEN sequence, are located within or between the sequence motifs, or are concentrated in one or more short stretches of the XTEN sequence. In such cases where the XTEN component of the GLP2-XTEN comprises amino acids other than glycine (G), alanine (A), serine (S), threonine (T), glutamate (E) and proline (P), it is desirable that the amino acids not be hydrophobic residues and should not substantially confer secondary structure of the XTEN component. Hydrophobic residues that are less favored in construction of XTEN include tryptophan, phenylalanine, tyrosine, leucine, isoleucine, valine, and methionine. Additionally, one can design the XTEN sequences to contain less than 5% or less than 4% or less than 3% or less than 2% or less than 1% or none of the following amino acids: cysteine (to avoid disulfide formation and oxidation), methionine (to avoid oxidation), asparagine and glutamine (to avoid desamidation). Thus, in some embodiments, the XTEN component of the GLP2-XTEN fusion protein comprising other amino acids in addition to glycine (G), alanine (A), serine (S), threonine (T), glutamate (E) and proline (P) would have a sequence with less than 5% of the residues contributing to alpha-helices and beta-sheets as measured by the Chou-Fasman algorithm and have at least 90%, or at least about 95% or more random coil formation as measured by the GOR algorithm.

3. Length of Sequence

In another aspect, the invention provides XTEN of varying lengths for incorporation into GLP2-XTEN compositions wherein the length of the XTEN sequence(s) are chosen based on the property or function to be achieved in the fusion protein. Depending on the intended property or function, the GLP2-XTEN compositions comprise short or intermediate length XTEN and/or longer XTEN sequences that can serve as carriers. While not intended to be limiting, the XTEN or fragments of XTEN include short segments of about 6 to about 99 amino acid residues, intermediate lengths of about 100 to about 399 amino acid residues, and longer lengths of about 400 to about 3000 amino acid residues. Thus, the subject GLP2-XTEN encompass XTEN or fragments of XTEN with lengths of about 6, or about 12, or about 36, or about 40, or about 100, or about 144, or about 288, or about 401, or about 500, or about 600, or about 700, or about 800, or about 900, or about 1000, or about 1500, or about 2000, or about 2500, or up to about 3000 amino acid residues in length. In other cases, the XTEN sequences can be about 6 to about 50, or about 100 to 150, about 150 to 250, about 250 to 400, about 400 to about 500, about 500 to 900, about 900 to 1500, about 1500 to 2000, or about 2000 to about 3000 amino acid residues in length. The precise length of an XTEN can vary without adversely affecting the biological activity of a GLP2-XTEN composition. In one embodiment, one or more of the XTEN used in the GLP2-XEN disclosed herein has 36 amino acids, 42 amino acids, 144 amino acids, 288 amino acids, 576 amino acids, or 864 amino acids in length and may be selected from one of the XTEN family sequences; i.e., AD, AE, AF, AG, AM, AQ, BC or BD. In another embodiment, one or more of the XTEN used herein is selected from the group consisting of XTEN_AE864, XTEN_AE576, XTEN_AE288, XTEN_AE144, XTEN_AE42, XTEN_AG864, XTEN_AG576, XTEN_AG288, XTEN_AG144, and XTEN_AG42 or other XTEN sequences in Table 4. In the embodiments of the GLP2-XTEN, the one or more XTEN or fragments of XTEN sequences individually exhibit at least about 80% sequence identity, or alternatively 81%, 82%, 83%, 84%, 85%, 86%, 87%, 88%, 89%, 90%, 91%, 92%, 93%, 94%, 95%, 96%, 97%, 98%, 99%, or 100% sequence identity compared to a motif or an XTEN selected from Table 4, or a fragment thereof with comparable length. In some embodiments, the GLP2-XTEN fusion proteins comprise a first and at least a second XTEN sequence, wherein the cumulative length of the residues in the XTEN sequences is greater than about 100 to about 3000 or about 400 to about 1000 amino acid residues and the XTEN can be identical or they can be different in sequence or in length. As used herein, "cumulative length" is intended to encompass the total length, in amino acid residues, when more than one XTEN is incorporated into the GLP2-XTEN fusion protein.

As described more fully below, methods are disclosed in which the GLP2-XTEN is designed by selecting the length of the XTEN to confer a target half-life or other physicochemical property on a fusion protein administered to a subject. When XTEN are used as a carrier, the invention takes advantage of the discovery that increasing the length of the non-repetitive, unstructured polypeptides enhances the unstructured nature of the XTENs and correspondingly enhances the biological and pharmacokinetic properties of fusion proteins comprising the XTEN carrier. In general, XTEN cumulative lengths longer that about 400 residues incorporated into the fusion protein compositions result in longer half-life compared to shorter cumulative lengths, e.g., shorter than about 280 residues. As described more fully in the Examples, proportional increases in the length of the XTEN, even if created by a repeated order of single family sequence motifs (e.g., the four AE motifs of Table 3), result in a sequence with a higher percentage of random coil formation, as determined by GOR algorithm, or reduced content of alpha-helices or beta-sheets, as determined by Chou-Fasman algorithm, compared to shorter XTEN lengths. In addition, increasing the length of the unstructured polypeptide fusion partner, as described in the Examples, results in a fusion protein with a disproportionate increase in terminal half-life compared to fusion proteins with unstructured polypeptide partners with shorter sequence lengths.

In some embodiments, where the XTEN serve primarily as a carrier, the invention encompasses GLP2-XTEN compositions comprising one or more XTEN wherein the cumulative XTEN sequence length of the fusion protein(s) is greater than about 100, 200, 400, 500, 600, 800, 900, or 1000 to about 3000 amino acid residues, wherein the fusion protein exhibits enhanced pharmacokinetic properties when administered to a subject compared to a GLP-2 not linked to the XTEN and administered at a comparable dose. In one embodiment of the foregoing, the one or more XTEN sequences exhibit at least about 80%, 90%, 91%, 92%, 93%, 94%, 95%, 96%, 97%, or 98% or more identity to a sequence selected from Table 4, and the remainder, if any, of the carrier sequence(s) contains at least 90% hydrophilic amino acids and less than about 2% of the overall sequence consists of hydrophobic or aromatic amino acids or cysteine. The enhanced pharmacokinetic properties of the GLP2-XTEN in comparison to GLP-2 not linked to XTEN are described more fully, below.

In another aspect, the invention provides methods to create XTEN of short or intermediate lengths from longer "donor" XTEN sequences, wherein the longer donor sequence is created by truncating at the N-terminus, or the C-terminus, or a fragment is created from the interior of a donor sequence, thereby resulting in a short or intermediate length XTEN. In non-limiting examples, as schematically depicted in FIG. 3A-C, the AG864 sequence of 864 amino acid residues can be truncated to yield an AG144 with 144 residues, an AG288 with 288 residues, an AG576 with 576 residues, or other intermediate lengths, while the AE864 sequence (as depicted in FIG. 3D, E) can be truncated to yield an AE288 or AE576 or other intermediate lengths. It is specifically contemplated that such an approach can be utilized with any of the XTEN embodiments described herein or with any of the sequences listed in Tables 4 or 8-12 to result in XTEN of a desired length.

4. Net charge

In other embodiments, the XTEN polypeptides have an unstructured characteristic imparted by incorporation of amino acid residues with a net charge and containing a low proportion or no hydrophobic amino acids in the XTEN sequence. The overall net charge and net charge density is controlled by modifying the content of charged amino acids in the XTEN sequences, either positive or negative, with the net charge typically represented as the percentage of amino acids in the polypeptide contributing to a charged state beyond those residues that are cancelled by a residue with an opposing charge. In some embodiments, the net charge density of the XTEN of the compositions may be above +0.1 or below -0.1 charges/residue. By "net charge density" of a protein or peptide herein is meant the net charge divided by the total number of amino acids in the protein or propeptide. In other embodiments, the net charge of an XTEN can be about 0%, about 1%, about 2%, about 3%, about 4%, about 5%, about 6%, about 7%, about 8%, about 9%, about 10% about 11%, about 12%, about 13%, about 14%, about 15%, about 16%, about 17%, about 18%, about 19%, or about 20% or more. In some embodiments, the XTEN sequence comprises charged residues separated by other residues such as serine or glycine, which leads to better expression or purification behavior. Based on the net charge, some XTENs have an isoelectric point (pI) of 1.0, 1.5, 2.0, 2.5, 3.0, 3.5, 4.0, 4.5, 5.0, 5.5, 6.0, or even 6.5. In one embodiment, the XTEN will have an isoelectric point between 1.5 and 4.5 and carry a net negative charge under physiologic conditions.

Since most tissues and surfaces in a human or animal have a net negative charge, in some embodiments the XTEN sequences are designed to have a net negative charge to minimize non-specific interactions between the XTEN containing compositions and various surfaces such as blood vessels, healthy tissues, or various receptors. Not to be bound by a particular theory, an XTEN can adopt open conformations due to electrostatic repulsion between individual amino acids of the XTEN polypeptide that individually carry a net negative charge and that are distributed across the sequence of the XTEN polypeptide. In some embodiments, the XTEN sequence is designed with at least 90% or 95% of the charged residues separated by other residues such as serine, alanine, threonine, proline or glycine, which leads to a more uniform distribution of charge, better expression or purification behavior. Such a distribution of net negative charge in the extended sequence lengths of XTEN can lead to an unstructured conformation that, in turn, can result in an effective increase in hydrodynamic radius. In preferred embodiments, the negative charge of the subject XTEN is conferred by incorporation of glutamic acid residues. Generally, the glutamic residues are spaced uniformly across the XTEN sequence. In some cases, the XTEN can contain about 10-80, or about 15-60, or about 20-50 glutamic residues per 20kDa of XTEN that can result in an XTEN with charged residues that would have very similar pKa, which can increase the charge homogeneity of the product and sharpen its isoelectric point, enhance the physicochemical properties of the resulting GLP2-XTEN fusion protein for, and hence, simplifying purification procedures. For example, where an XTEN with a negative charge is desired, the XTEN can be selected solely from an AE family sequence, which has approximately a 17% net charge due to incorporated glutamic acid, or can include varying proportions of glutamic acid-containing motifs of Table 3 to provide the desired degree of net charge. Non-limiting examples of AE XTEN include, but are not limited to the AE36, AE42, AE48, AE144, AE288, AE576, AE624, AE864, and AE912 polypeptide sequences of Tables 4 or 9, or fragments thereof. In one embodiment, an XTEN sequence of Tables 4 or 9 can be modified to include additional glutamic acid residues to achieve the desired net negative charge. Accordingly, in one embodiment the invention provides XTEN in which the XTEN sequences contain about 1%, 2%, 4%, 8%, 10%, 15%, 17%, 20%, 25%, or even about 30% glutamic acid. In some cases, the XTEN can contain about 10-80, or about 15-60, or about 20-50 glutamic residues per 20kDa of XTEN that can result in an XTEN with charged residues that would have very similar pKa, which can increase the charge homogeneity of the product and sharpen its isoelectric point, enhance the physicochemical properties of the resulting GLP2-XTEN fusion protein for, and hence, simplifying purification procedures. In one embodiment, the invention contemplates incorporation of aspartic acid residues into XTEN in addition to glutamic acid in order to achieve a net negative charge.

Not to be bound by a particular theory, the XTEN of the GLP2-XTEN compositions with the higher net negative charge are expected to have less non-specific interactions with various negatively-charged surfaces such as blood vessels, tissues, or various receptors, which would further contribute to reduced active clearance. Conversely, it is believed that the XTEN of the GLP2-XTEN compositions with a low (or no) net charge would have a higher degree of interaction with surfaces that can potentiate the biological activity of the associated GLP-2, given the known contribution of phagocytic cells in the inflammatory process in the intestines.

In other cases, where no net charge is desired, the XTEN can be selected from, for example, AG family XTEN components, such as the AG motifs of Table 3, or those AM motifs of Table 3 that have approximately no net charge. Non-limiting examples of AG XTEN include, but are not limited to AG42, AG144, AG288, AG576, and AG864 polypeptide sequences of Tables 4 and 11, or fragments thereof. In another embodiment, the XTEN can comprise varying proportions of AE and AG motifs (in order to have a net charge that is deemed optimal for a given use or to maintain a given physicochemical property.

The XTEN of the compositions of the present invention generally have no or a low content of positively charged amino acids. In some embodiments, the XTEN may have less than about 10% amino acid residues with a positive charge, or less than about 7%, or less than about 5%, or less than about 2%, or less than about 1% amino acid residues with a positive charge. However, the invention contemplates constructs where a limited number of amino acids with a positive charge, such as lysine, are incorporated into XTEN to permit conjugation between the epsilon amine of the lysine and a reactive group on a GLP-2 peptide, a linker bridge, or a reactive group on a drug or small molecule to be conjugated to the XTEN backbone. In one embodiment of the foregoing, the XTEN has between about 1 to about 100 lysine residues, or about 1 to about 70 lysine residues, or about 1 to about 50 lysine residues, or about 1 to about 30 lysine residues, or about 1 to about 20 lysine residues, or about 1 to about 10 lysine residues, or about 1 to about 5 lysine residues, or alternatively only a single lysine residue. Using the foregoing lysine-containing XTEN, fusion proteins are constructed that comprises XTEN, a GLP-2, plus a chemotherapeutic agent useful in the treatment of GLP-2-related diseases or disorders, wherein the maximum number of molecules of the agent incorporated into the XTEN component is determined by the numbers of lysines or other amino acids with reactive side chains (e.g., cysteine) incorporated into the XTEN. Accordingly, the invention also provides XTEN with 1 to about 10 cysteine residues, or about 1 to about 5 cysteine residues, or alternatively only a single cysteine residue wherein fusion proteins are constructed that comprises XTEN, a GLP-2, plus a chemotherapeutic agent useful in the treatment of GLP-2-related diseases or disorders, wherein the maximum number of molecules of the agent incorporated into the XTEN component is determined by the numbers of cysteines.

As hydrophobic amino acids impart structure to a polypeptide, the invention provides that the content of hydrophobic amino acids in the XTEN will typically be less than 5%, or less than 2%, or less than 1% hydrophobic amino acid content. In one embodiment, the amino acid content of methionine and tryptophan in the XTEN component of a GLP2-XTEN fusion protein is typically less than 5%, or less than 2%, and most preferably less than 1%. In another embodiment, the XTEN will have a sequence that has less than 10% amino acid residues with a positive charge, or less than about 7%, or less that about 5%, or less than about 2% amino acid residues with a positive charge, the sum of methionine and tryptophan residues will be less than 2%, and the sum of asparagine and glutamine residues will be less than 5% of the total XTEN sequence.

5. Low immunogenicity

In another aspect, the invention provides compositions in which the XTEN sequences have a low degree of immunogenicity or are substantially non-immunogenic. Several factors can contribute to the low immunogenicity of XTEN, e.g., the non-repetitive sequence, the unstructured conformation, the high degree of solubility, the low degree or lack of self-aggregation, the low degree or lack of proteolytic sites within the sequence, and the low degree or lack of epitopes in the XTEN sequence.

Conformational epitopes are formed by regions of the protein surface that are composed of multiple discontinuous amino acid sequences of the protein antigen. The precise folding of the protein brings these sequences into a well-defined, stable spatial configurations, or epitopes, that can be recognized as "foreign" by the host humoral immune system, resulting in the production of antibodies to the protein or the activation of a cell-mediated immune response. In the latter case, the immune response to a protein in an individual is heavily influenced by T-cell epitope recognition that is a function of the peptide binding specificity of that individual's HLA-DR allotype. Engagement of a MHC Class II peptide complex by a cognate T-cell receptor on the surface of the T-cell, together with the cross-binding of certain other co-receptors such as the CD4 molecule, can induce an activated state within the T-cell. Activation leads to the release of cytokines further activating other lymphocytes such as B cells to produce antibodies or activating T killer cells as a full cellular immune response.

The ability of a peptide to bind a given MHC Class II molecule for presentation on the surface of an APC (antigen presenting cell) is dependent on a number of factors; most notably its primary sequence. In one embodiment, a lower degree of immunogenicity is achieved by designing XTEN sequences that resist antigen processing in antigen presenting cells, and/or choosing sequences that do not bind MHC receptors well. The invention provides GLP2-XTEN fusion proteins with substantially non-repetitive XTEN polypeptides designed to reduce binding with MHC II receptors, as well as avoiding formation of epitopes for T-cell receptor or antibody binding, resulting in a low degree of immunogenicity. Avoidance of immunogenicity can attribute to, at least in part, a result of the conformational flexibility of XTEN sequences; i.e., the lack of secondary structure due to the selection and order of amino acid residues. For example, of particular interest are sequences having a low tendency to adapt compactly folded conformations in aqueous solution or under physiologic conditions that could result in conformational epitopes. The administration of fusion proteins comprising XTEN, using conventional therapeutic practices and dosing, would generally not result in the formation of neutralizing antibodies to the XTEN sequence, and also reduce the immunogenicity of the GLP-2 fusion partner in the GLP2-XTEN compositions.

In one embodiment, the XTEN sequences utilized in the subject fusion proteins can be substantially free of epitopes recognized by human T cells. The elimination of such epitopes for the purpose of generating less immunogenic proteins has been disclosed previously; see for example WO 98/52976, WO 02/079232, and WO 00/3317 which are incorporated by reference herein. Assays for human T cell epitopes have been described (Stickler, M., et al. (2003) J Immunol Methods, 281: 95-108). Of particular interest are peptide sequences that can be oligomerized without generating T cell epitopes or non-human sequences. This is achieved by testing direct repeats of these sequences for the presence of T-cell epitopes and for the occurrence of 6 to 15-mer and, in particular, 9-mer sequences that are not human, and then altering the design of the XTEN sequence to eliminate or disrupt the epitope sequence. In some embodiments, the XTEN sequences are substantially non-immunogenic by the restriction of the numbers of epitopes of the XTEN predicted to bind MHC receptors. With a reduction in the numbers of epitopes capable of binding to MHC receptors, there is a concomitant reduction in the potential for T cell activation as well as T cell helper function, reduced B cell activation or upregulation and reduced antibody production. The low degree of predicted T-cell epitopes can be determined by epitope prediction algorithms such as, e.g., TEPITOPE (Stumiolo, T., et al. (1999) Nat Biotechnol, 17: 555-61), as shown in Example 31. The TEPITOPE score of a given peptide frame within a protein is the log of the $K_d$ (dissociation constant, affinity, off-rate) of the binding of that peptide frame to multiple of the most common human MHC alleles, as disclosed in Sturniolo, T. et al. (1999) Nature Biotechnology 17:555). The score ranges over at least 20 logs, from about 10 to about -10 (corresponding to binding constraints of $10e^{10} K_d$ to $10e^{-10} K_d$), and can be reduced by avoiding hydrophobic amino acids that serve as anchor residues during peptide display on MHC, such as M, I, L, V, F. In some embodiments, an XTEN component incorporated into a GLP2-XTEN does not have a predicted T-cell epitope at a TEPITOPE threshold score of about -5, or -6, or -7, or -8, or -9, or at a TEPITOPE score of -10. As used herein, a score of "-9" would be a more stringent TEPITOPE threshold than a score of -5.

In another embodiment, the inventive XTEN sequences, including those incorporated into the subject GLP2-XTEN fusion proteins, are rendered substantially non-immunogenic by the restriction of known proteolytic sites from the sequence of the XTEN, reducing the processing of XTEN into small peptides that can bind to MHC II receptors. In another embodiment, the XTEN sequence is rendered substantially non-immunogenic by the use a sequence that is substantially devoid of secondary structure, conferring resistance to many proteases due to the high entropy of the structure. Accordingly, the reduced TEPITOPE score and elimination of known proteolytic sites from the XTEN render the XTEN compositions, including the XTEN of the GLP2-XTEN fusion protein compositions, substantially unable to be bound by mammalian receptors, including those of the immune system. In one embodiment, an XTEN of a GLP2-XTEN fusion protein can have >100 nM $K_d$ binding to a mammalian receptor, or greater than 500 nM $K_d$, or greater than 1 $\mu$M $K_d$ towards a mammalian cell surface or circulating polypeptide receptor.

Additionally, the non-repetitive sequence and corresponding lack of epitopes of XTEN limit the ability of B cells to bind to or be activated by XTEN. A repetitive sequence is recognized and can form multivalent contacts with even a few B cells and, as a consequence of the cross-linking of multiple T-cell independent receptors, can stimulate B cell proliferation and antibody production. In contrast, while a XTEN can make contacts with many different B cells over its extended sequence, each individual B cell may only make one or a small number of contacts with an individual XTEN due to the lack of repetitiveness of the sequence. Not being to be bound by any theory, XTENs typically have a much lower tendency to stimulate proliferation of B cells and thus an immune response. In one embodiment, the GLP2-XTEN have reduced immunogenicity as compared to the corresponding GLP-2 that is not fused to an XTEN. In one embodiment, the administration of up to three parenteral doses of a GLP2-XTEN to a mammal result in detectable anti-GLP2-XTEN IgG at a serum dilution of 1:100 but not at a dilution of 1:1000. In another embodiment, the administration of up to three parenteral doses of a GLP2-XTEN to a mammal result in detectable anti-GLP-2 IgG at a serum dilution of 1:1000 but not at a dilution of 1:10,000. In another embodiment, the administration of up to three parenteral doses of a GLP2-XTEN to a mammal result in detectable anti-XTEN IgG at a serum dilution of 1:10,000 but not at a dilution of 1:1,000,000. In the foregoing embodiments, the mammal can be a mouse, a rat, a rabbit, or a cynomolgus monkey.

An additional feature of XTENs with non-repetitive sequences relative to sequences with a high degree of repetitiveness is non-repetitive XTENs form weaker contacts with antibodies. Antibodies are multivalent molecules. For instance, IgGs have two identical binding sites and IgMs contain 10 identical binding sites. Thus antibodies against repetitive sequences can form multivalent contacts with such repetitive sequences with high avidity, which can affect the potency and/or elimination of such repetitive sequences. In contrast, antibodies against non-repetitive XTENs may yield monovalent interactions, resulting in less likelihood of immune clearance such that the GLP2-XTEN compositions can remain in circulation for an increased period of time.

6. Increased hydrodynamic radius

In another aspect, the present invention provides XTEN in which the XTEN polypeptides have a high hydrodynamic radius that confers a corresponding increased apparent molecular weight to the GLP2-XTEN fusion protein incorporating the XTEN. As detailed in Example 25, the linking of XTEN to therapeutic protein sequences results in GLP2-XTEN compositions that can have increased hydrodynamic radii, increased apparent molecular weight, and increased apparent molecular weight factor compared to a therapeutic protein not linked to an XTEN. For example, in therapeutic applications in which prolonged half-life is desired, compositions in which a XTEN with a high hydrodynamic radius is incorporated into a fusion protein comprising a therapeutic protein can effectively enlarge the hydrodynamic radius of the composition beyond the glomerular pore size of approximately 3-5 nm (corresponding to an apparent molecular weight of about 70 kDA) (Caliceti. 2003. Pharmacokinetic and biodistribution properties of poly(ethylene glycol)-protein conjugates. Adv Drug Deliv Rev 55:1261-1277), resulting in reduced renal clearance of circulating proteins with a corresponding increase in terminal half-life and other enhanced pharmacokinetic properties. The hydrodynamic radius of a protein is determined by its molecular weight as well as by its structure, including shape or compactness. Not to be bound by a particular theory, the XTEN can adopt open conformations due to electrostatic repulsion between individual charges of the peptide or the inherent flexibility imparted by the particular amino acids in the sequence that lack potential to confer secondary structure. The open, extended and unstructured conformation of the XTEN polypeptide can have a greater proportional hydrodynamic radius compared to polypeptides of a comparable sequence length and/or molecular weight that have secondary and/or tertiary structure, such as typical globular proteins. Methods for determining the hydrodynamic radius are well known in the art, such as by the use of size exclusion chromatography (SEC), as described in U.S. Patent Nos. 6,406,632 and 7,294,513. As the results of Example 25 demonstrate, the addition of increasing lengths of XTEN results in proportional increases in the parameters of hydrodynamic radius, apparent molecular weight, and apparent molecular weight factor, permitting the tailoring of GLP2-XTEN to desired characteristic cut-off apparent molecular weights or hydrodynamic radii. Accordingly, in certain embodiments, the GLP2-XTEN fusion protein can be configured with an XTEN such that the fusion protein can have a hydrodynamic radius of at least about 5 nm, or at least about 8 nm, or at least about 10 nm, or 12 nm, or at least about 15 nm. In the foregoing embodiments, the large hydrodynamic radius conferred by the XTEN in a GLP2-XTEN fusion protein can lead to reduced renal clearance of the resulting fusion protein, leading to a corresponding increase in terminal half-life, an increase in mean residence time, and/or a decrease in renal clearance rate.

When the molecular weights of the GLP2-XTEN fusion proteins are derived from size exclusion chromatography analyses, the open conformation of the XTEN due to the low degree of secondary structure results in an increase in the apparent molecular weight of the fusion proteins. In some embodiments the GLP2-XTEN comprising a GLP-2 and at least a first or multiple XTEN exhibits an apparent molecular weight of at least about 200 kDa, or at least about 400 kDa, or at least about 500 kDa, or at least about 700 kDa, or at least about 1000 kDa, or at least about 1400 kDa. Accordingly, the GLP2-XTEN fusion proteins comprising one or more XTEN exhibit an apparent molecular weight that is about 2-fold greater, or about 3-fold greater or about 4-fold greater, or about 8-fold greater, or about 10-fold greater, or about 12-fold greater, or about 15-fold greater, or about 20-fold greater than the actual molecular weight of the fusion protein. In one embodiment, the isolated GLP2-XTEN fusion protein of any of the embodiments disclosed herein exhibit an apparent molecular weight factor under physiologic conditions that is greater than about 2, or about 3, or about 4, or about 5, or about 6, or about 7, or about 8, or about 10, or about 15, or greater than about 20. In another embodiment, the GLP2-XTEN fusion protein has, under physiologic conditions, an apparent molecular weight factor that is about 3 to about 20, or is about 5 to about 15, or is about 8 to about 14, or is about 10 to about 12 relative to the actual molecular weight of the fusion protein.

## IV). GLP2-XTEN COMPOSITIONS

The present invention relates in part to fusion protein compositions comprising GLP-2 linked to one or more XTEN, wherein the fusion protein would act to replace or augment existing GLP-2 when administered to a subject. The invention addresses a long-felt need in increasing the terminal half-life of exogenously administered GLP-2 to a subject in need thereof. One way to increase the circulation half-life of a therapeutic protein is to ensure that renal clearance of the protein is reduced. Another way to increase the circulation half-life is to reduce the active clearance of the therapeutic protein, whether mediated by receptors, active metabolism of the protein, or other endogenous mechanisms. Both may be achieved by conjugating the protein to a polymer, which, in some cases, is capable of conferring an increased molecular size (or hydrodynamic radius) to the protein and, hence, reduced renal clearance, and, in other cases, interferes with binding of the protein to clearance receptors or other proteins that contribute to metabolism or clearance. Thus, certain objects of the present invention include, but are not limited to, providing improved GLP-2 molecules with a longer circulation or terminal half-life, decreasing the number or frequency of necessary administrations of GLP-2 compositions, retaining at least a portion of the biological activity of the native GLP-2, and enhancing the ability to treat GLP-2-related diseases or gastrointestinal conditions with resulting improvement in clinical symptoms and overall well-being more efficiently, more effectively, more economically, and with greater safety compared to presently available GLP-2 preparations.

To meet these needs, in a first aspect, the invention provides isolated fusion protein compositions comprising a biologically active GLP-2 covalently linked to one or more XTEN, resulting in a GLP2-XTEN fusion protein composition. The subject GLP-2-XTEN can mediate one or more biological or therapeutic activities of a wild-type GLP-2. GLP2-XTEN can be produced recombinantly or by chemical conjugation of a GLP-2 to and XTEN. In one embodiment, the GLP-2 is native GLP-2. In another embodiment, the GLP-2 is a sequence variant of a natural sequence that retains at least a portion of the biological activity of the native GLP-2. In one embodiment, the GLP-2 is a sequence having at least 90%, or at least about 91%, or at least about 92%, or at least about 93%, or at least about 94%, or at least about 95%, or at least about 96%, or at least about 97%, or at least about 98%, or at least about 99%, or 100% sequence identity to a sequence selected from the group consisting of the sequences in Table 1, when optimally aligned. In another embodiment, the GLP-2 is a sequence variant with glycine substituted for alanine at residue number 2 of the mature GLP-2 peptide. In one embodiment, the GLP2-XTEN comprises a GLP-2 having the sequence HGDGSFSDEMNTILDNLAARDFINWLIQTKITD. In one embodiment, the invention provides GLP2-XTEN fusion proteins comprising GLP-2 N- and/or C-terminally modified forms comprising one or more XTEN.

The GLP-2 of the subject compositions, particularly those disclosed in Table 1, together with their corresponding nucleic acid and amino acid sequences, are well known in the art and descriptions and sequences are available in public databases such as Chemical Abstracts Services Databases (e.g., the CAS Registry), GenBank, The Universal Protein Resource (UniProt) and subscription provided databases such as GenSeq (e.g., Derwent). Polynucleotide sequences may be a wild type polynucleotide sequence encoding a given GLP-2 (e.g., either full length or mature), or in some instances the sequence may be a variant of the wild type polynucleotide sequence (e.g., a polynucleotide which encodes the wild type biologically active protein, wherein the DNA sequence of the polynucleotide has been optimized, for example, for expression in a particular species; or a polynucleotide encoding a variant of the wild type protein, such as a site directed mutant or an allelic variant. It is well within the ability of the skilled artisan to use a wild-type or consensus cDNA sequence or a codon-optimized sequence variant of a GLP-2 to create GLP2-XTEN constructs contemplated by the invention using methods known in the art and/or in conjunction with the guidance and methods provided herein and described more fully in the Examples.

In some embodiments, the GLP2-XTEN fusion proteins retain at least a portion of the biological activity of native GLP-2. A GLP2-XTEN fusion protein of the invention is capable of binding and activating a GLP-2 receptor. In one embodiment, the GLP2-XTEN fusion protein of the present invention has an $EC_{50}$ value, when assessed using an in vitro GLP-2 receptor binding assay such as described herein or others known in the art, of less than about 30 nM, or about 100 nM, or about 200 nM, or about 300 nM, or about 400 nM, or about 500 nM, or about 600 nM, or about 700 nM, or about 800 nM, or about 1000 nM, or about 1200 nM, or about 1400 nM. In another embodiment, the GLP2-XTEN fusion protein of the present invention retains at least about 1%, or about 2%, or about 3%, or about 4%, or about 5%, or about 10%, or about 20%, or about 30% of the potency of the corresponding GLP-2 not linked to XTEN when assayed using an in vitro GLP2R cell assay such as described in the Examples or others known in the art.

In some embodiments, GLP2-XTEN fusion proteins of the disclosure have intestinotrophic, wound healing and anti-inflammatory activity. In some embodiments, the GLP2-XTEN fusion protein compositions exhibit an improvement in one, two, three or more gastrointestinal-related parameters disclosed herein that are at least about 20%, or 30%, or 40%, or 50%, or 60%, or 70%, or 80%, or 90%, or 100%, or 120%, or 140%, at least about 150% greater compared to the parameter(s) achieved by the corresponding GLP-2 component not linked to the XTEN when administered to a subject. The parameter can be a measured parameter selected from blood concentrations of GLP-2, increased mesenteric blood flow, decreased inflammation, increased weight gain, decreased diarrhea, decreased fecal wet weight, intestinal wound healing, increase in plasma citrulline concentrations, decreased CRP levels, decreased requirement for steroid therapy, enhancing or stimulating mucosal integrity, decreased sodium loss, decreased parenteral nutrition required to maintain body weight, minimizing, mitigating, or preventing bacterial translocation in the intestines, enhancing, stimulating or accelerating recovery of the intestines after surgery, preventing relapses of inflammatory bowel disease, or achieving or maintaining energy homeostasis, among others. In one embodiment, administration of the GLP2-XTEN fusion protein to a subject results in a greater ability to increase small intestine weight and/or length when administered to a subject with a surgically-resected intestine (e.g., short-bowel syndrome) or Crohn's Disease, compared to the corresponding GLP-2 not linked to XTEN and administered at a comparable dose in nmol/kg and dose regimen. In another embodiment, a GLP2-XTEN fusion protein exhibits at least about 10%, or 20%, or 30%, or 40%, or 50%, or 60%, or 70%, or 80%, or at least about 90% greater ability to reduce ulceration when administered to a subject with Crohn's Disease (either naturally acquired or experimentally induced) compared to the corresponding GLP-2 component not linked to the XTEN and administered at a comparable nmol/kg dose and dose regimen. In another embodiment, the fusion protein exhibits the ability to reduce inflammatory cytokines when administered to a subject with Crohn's Disease (either naturally acquired or experimentally induced) by at least about 20%, or 30%, or 40%, or 50%, or 60%, or 70%, or 80%, or at least about 90% compared the corresponding GLP-2 component not linked to the XTEN and administered at a comparable nmol/kg dose and dose regimen. In another embodiment, a GLP2-XTEN fusion protein exhibits at least about 10%, or 20%, or 30%, or 40%, or 50%, or 60%, or 70%, or 80%, or at least about 90% greater ability to reduce mucosal atrophy when administered to a subject with Crohn's Disease (either naturally acquired or experimentally induced; e.g., administration of indomethacin) compared to the corresponding GLP-2 component not linked to the XTEN and administered at a comparable nmol/kg dose and dose regimen. In another embodiment, a GLP2-XTEN fusion protein exhibits at least about 5%, or at least about 6%, or 7%, or 8%, or 9%, or 10%, or 11%, or 12%, or 15%, or at least about 20% greater ability to increase height of intestinal villi when administered to a subject with Crohn's Disease (either naturally acquired or experimentally induced; e.g., administration of indomethacin) compared to the corresponding GLP-2 component not linked to the XTEN and administered at a comparable nmol/kg dose and dose regimen. In another embodiment, a GLP2-XTEN fusion protein exhibits at least about 10%, or 20%, or 30%, or 40%, or 50%, or 60%, or 70%, or 80%, or at least about 90% greater ability to increase body weight when administered to a subject with Crohn's Disease (either naturally acquired or experimentally induced; e.g., administration of indomethacin) compared to the corresponding GLP-2 component not linked to the XTEN and administered at a comparable nmol/kg dose and dose regimen. In the foregoing embodiments of the paragraph, the subject is selected from the group consisting of mouse, rat, monkey and human.

The compositions of the invention include fusion proteins that are useful, when administered to a subject, for mediating or preventing or ameliorating a gastrointestinal condition associated with GLP-2 such as, but not limited to ulcers, gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy-associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis associated with cancer chemotherapy and irritable bowel disease, pouchitis, ischemia, and stroke.

Of particular interest are GLP2-XTEN fusion protein compositions for which an increase in a pharmacokinetic parameter, increased solubility, increased stability, or some other enhanced pharmaceutical property compared to native GLP-2 is obtained, providing compositions with enhanced efficacy, safety, or that result in reduced dosing frequency and/or improve patient management. The GLP2-XTEN fusion proteins of the embodiments disclosed herein exhibit one or more or any combination of the improved properties and/or the embodiments as detailed herein. Thus, the subject GLP2-XTEN fusion protein compositions are designed and prepared with various objectives in mind, including improving the therapeutic efficacy of the bioactive GLP-2 by, for example, increasing the *in vivo* exposure or the length that the GLP2-XTEN remains within the therapeutic window when administered to a subject, compared to a GLP-2 not linked to XTEN.

In one embodiment, a GLP2-XTEN fusion protein comprises a single GLP-2 molecule linked to a single XTEN (e.g., an XTEN as described above). In another embodiment, the GLP2-XTEN comprises a single GLP-2 linked to two XTEN, wherein the XTEN may be identical or they may be different. In another embodiment, the GLP2-XTEN fusion protein comprises a single GLP-2 molecule linked to a first and a second XTEN, in which the GLP-2 is a sequence that has at least about 80% sequence identity, or alternatively 81%, 82%, 83%, 84%, 85%, 86%, 87%, 88%, 89%, 90%, 91%, 92%, 93%, 94%, 95%, 96%, 97%, 98%, or at least about 99%, or 100% sequence identity compared to a protein sequence selected from Table 1, and the first and the second XTEN are each sequences that have at least about 80% sequence identity, or alternatively 81%, 82%, 83%, 84%, 85%, 86%, 87%, 88%, 89%, 90%, 91%, 92%, 93%, 94%, 95%, 96%, 97%, 98%, or at least about 99%, or 100% sequence identity compared to one or more sequences selected from Table 4, or fragments thereof. In another embodiment, the GLP2-XTEN fusion protein comprises a sequence with at least about 80% sequence identity, or alternatively 81%, 82%, 83%, 84%, 85%, 86%, 87%, 88%, 89%, 90%, 91%, 92%, 93%, 94%, 95%, 96%, 97%, 98%, or at least about 99%, or 100% sequence identity to a sequence from Table 33 and 34.

## 1. GLP2-XTEN Fusion Protein Configurations

The invention provides GLP2-XTEN fusion protein compositions with the GLP-2 and XTEN components linked in specific N- to C-terminus configurations.

In one embodiment of the GLP2-XTEN composition, the invention provides a fusion protein of formula I:

$$(GLP\text{-}2)\text{-}(XTEN) \qquad I$$

wherein independently for each occurrence, GLP-2 is a GLP-2 protein or variant as defined herein, including sequences having at least about 80%, or at least about 90%, or at least about 95%, or at least about 96%, or at least about 97%, or at least about 98%, or at least about 99% or 100% sequence identity with sequenced from Table 1, and XTEN is an extended recombinant polypeptide as described herein, including, but not limited to sequences having at least about 80%, or at least about 90%, or at least about 95%, or at least about 96%, or at least about 97%, or at least about 98%, or at least about 99% or 100% sequence identity to sequences set forth in Table 4.

In another embodiment of the GLP2-XTEN composition, the invention provides a fusion protein of formula II:

$$(XTEN)\text{-}(GLP\text{-}2) \qquad II$$

wherein independently for each occurrence, GLP-2 is a GLP-2 protein or variant as defined herein, including sequences having at least about 80%, or at least about 90%, or at least about 95%, or at least about 96%, or at least about 97%, or at least about 98%, or at least about 99% or 100% sequence identity with sequenced from Table 1, and XTEN is an extended recombinant polypeptide as described herein, including, but not limited to sequences having at least about 80%, or at least about 90%, or at least about 95%, or at least about 96%, or at least about 97%, or at least about 98%, or at least about 99% or 100% sequence identity to sequences set forth in Table 4.

In another embodiment of the GLP2-XTEN composition, the invention provides an isolated fusion protein, wherein the fusion protein is of formula III:

$$(XTEN)\text{-}(GLP\text{-}2)\text{-}(XTEN) \qquad III$$

wherein independently for each occurrence, GLP-2 is a GLP-2 protein or variant as defined herein, including sequences having at least about 80%, or at least about 90%, or at least about 95%, or at least about 96%, or at least about 97%, or at least about 98%, or at least about 99% or 100% sequence identity with sequenced from Table 1, and XTEN is an extended recombinant polypeptide as described herein, including, but not limited to sequences having at least about 80%, or at least about 90%, or at least about 95%, or at least about 96%, or at least about 97%, or at least about 98%, or at least about 99% or 100% sequence identity to sequences set forth in Table 4..

In another embodiment of the GLP2-XTEN composition, the invention provides an isolated fusion protein, wherein the fusion protein is of formula IV:

$$(\text{GLP-2})\text{-}(\text{XTEN})\text{-}(\text{GLP-2}) \qquad \text{IV}$$

wherein independently for each occurrence, GLP-2 is a GLP-2 protein or variant as defined herein, including sequences having at least about 80%, or at least about 90%, or at least about 95%, or at least about 96%, or at least about 97%, or at least about 98%, or at least about 99% or 100% sequence identity with sequenced from Table 1, and XTEN is an extended recombinant polypeptide as described herein, including, but not limited to sequences having at least about 80%, or at least about 90%, or at least about 95%, or at least about 96%, or at least about 97%, or at least about 98%, or at least about 99% or 100% sequence identity to sequences set forth in Table 4.

In another embodiment of the GLP2-XTEN composition, the invention provides an isolated fusion protein, wherein the fusion protein is of formula V:

$$(\text{GLP-2})\text{-}(\text{S})_x\text{-}(\text{XTEN}) \qquad \text{V}$$

wherein independently for each occurrence, GLP-2 is a GLP-2 protein or variant as defined herein, including sequences having at least about 80%, or at least about 90%, or at least about 95%, or at least about 96%, or at least about 97%, or at least about 98%, or at least about 99% or 100% sequence identity with sequenced from Table 1; S is a spacer sequence having between 1 to about 50 amino acid residues that can optionally include a cleavage sequence or amino acids compatible with restrictions sites; x is either 0 or 1; and XTEN is an extended recombinant polypeptide as described herein, including, but not limited to sequences having at least about 80%, or at least about 90%, or at least about 95%, or at least about 96%, or at least about 97%, or at least about 98%, or at least about 99% or 100% sequence identity to sequences set forth in Table 4.

In another embodiment of the GLP2-XTEN composition, the invention provides an isolated fusion protein, wherein the fusion protein is of formula VI:

$$(\text{XTEN})_x\text{-}(\text{S})_x\text{-}(\text{GLP-2})\text{-}(\text{S})_y\text{-}(\text{XTEN})_y \qquad \text{VI}$$

wherein independently for each occurrence, GLP-2 is a GLP-2 protein or variant as defined herein, including sequences having at least about 80%, or at least about 90%, or at least about 95%, or at least about 96%, or at least about 97%, or at least about 98%, or at least about 99% or 100% sequence identity with sequenced from Table 1; S is a spacer sequence having between 1 to about 50 amino acid residues that can optionally include a cleavage sequence or amino acids compatible with restrictions sites; x is either 0 or 1 and y is either 0 or 1 wherein $x+y \geq 1$; and XTEN is an extended recombinant polypeptide as described herein, including, but not limited to sequences having at least about 80%, or at least about 90%, or at least about 95%, or at least about 96%, or at least about 97%, or at least about 98%, or at least about 99% or 100% sequence identity to sequences set forth in Table 4.

The embodiments of formulae I-VI encompass GLP2-XTEN configurations wherein one or more XTEN of lengths ranging from about 36 amino acids to 3000 amino acids (e.g., sequences selected from Table 4 or fragments thereof, or sequences exhibiting at least about 90-95% or more sequence identity thereto) are linked to the N- or C-terminus of the GLP-2. The embodiments of formula V further provide configurations wherein the XTEN are linked to GLP-2 via spacer sequences that can optionally comprise amino acids compatible with restrictions sites or can include cleavage sequences (e.g., the sequences of Tables 5 and 6, described more fully below) such that the XTEN encoding sequence can, in the case of a restriction site, be integrated into a GLP2-XTEN construct and, in the case of a cleavage sequence, the XTEN can be released from the fusion protein by the action of a protease appropriate for the cleavage sequence. In one embodiment of formula V, the fusion protein comprises a spacer sequence that is a single glycine residue.

2. GLP2-XTEN Fusion Protein Configurations with Spacer and Cleavage Sequences

In another aspect, the invention provides GLP2-XTEN configured with one or more spacer sequences incorporated into or adjacent to the XTEN that are designed to incorporate or enhance a functionality or property to the composition, or as an aid in the assembly or manufacture of the fusion protein compositions. Such properties include, but are not limited to, inclusion of cleavage sequence(s), such at TEV or other cleavage sequences of Table 6, to permit release of components, inclusion of amino acids compatible with nucleotide restrictions sites to permit linkage of XTEN-encoding nucleotides to GLP-2-encoding nucleotides or that facilitate construction of expression vectors, and linkers designed to reduce steric hindrance in regions of GLP2-XTEN fusion proteins.

In an embodiment, a spacer sequence can be introduced between an XTEN sequence and a GLP-2 component to decrease steric hindrance such that the GLP-2 component may assume its desired tertiary structure and/or interact appropriately with its target receptor. For spacers and methods of identifying desirable spacers, see, for example, George, et al. (2003) Protein Engineering 15:871-879, specifically incorporated by reference herein. In one embodiment, the spacer comprises one or more peptide sequences that are between 1-50 amino acid residues in length, or about 1-25 residues, or about 1-10 residues in length. Spacer sequences, exclusive of cleavage sites, can comprise any of the 20 natural L amino acids, and will preferably have XTEN-like properties in that 1) they will comprise hydrophilic amino acids that are satirically unhindered such as, but not limited to, glycine (G), alanine (A), serine (S), threonine (T), glutamate (E), proline (P) and aspartate (D); and 2) will be substantially non-repetitive. In addition, spacer sequences are designed to avoid the introduction of T-cell epitopes; determination of which are described above and in the Examples. In some cases, the spacer can be polyglycines or polyalanines, or is predominately a mixture of combinations of glycine, serine and alanine residues. In one embodiment, a spacer sequence, exclusive of cleavage site amino acids, has about 1 to 10 amino acids that consist of amino acids selected from glycine (G), alanine (A), serine (S), threonine (T), glutamate (E), and proline (P) and are substantially devoid of secondary structure; e.g., less than about 10%, or less than about 5% as determined by the Chou-Fasman and/or GOR algorithms. In one embodiment, the spacer sequence is GPEGPS. In another embodiment, the spacer sequence is a single glycine residue. In another embodiment, the spacer sequence is GPEGPS linked to a cleavage sequence of Table 6.

In a particular embodiment, the GLP2-XTEN fusion protein comprises one or more spacer sequences linked at the junction(s) between the payload GLP-2 sequence and the one more XTEN incorporated into the fusion protein, wherein the spacer sequences comprise amino acids that are compatible with nucleotides encoding restriction sites. In another embodiment, the GLP2-XTEN fusion protein comprises one or more spacer sequences linked at the junction(s) between the payload GLP-2 sequence and a signal sequence incorporated into the fusion protein, wherein the spacer sequences comprise a cleavage sequence (e.g., TEV) to release the GLP2-XTEN after expression. In another embodiment, the GLP2-XTEN fusion protein comprises one or more spacer sequences linked at the junction(s) between the payload GLP-2 sequence and the one more XTEN incorporated into the fusion protein wherein the spacer sequences comprise amino acids that are compatible with nucleotides encoding restriction sites and the amino acids and the one more spacer sequence amino acids are chosen from glycine (G), alanine (A), serine (S), threonine (T), glutamate (E), and proline (P). In another embodiment, the GLP2-XTEN fusion protein comprises one or more spacer sequences linked at the junction(s) between the payload GLP-2 sequence and the one more XTEN incorporated into the fusion protein wherein the spacer sequences comprise amino acids that are compatible with nucleotides encoding restriction sites and the one more spacer sequences are chosen from the sequences of Table 5. The exact sequence of each spacer sequence is chosen to be compatible with cloning sites in expression vectors that are used for a particular GLP2-XTEN construct. For embodiments in which a single XTEN is attached to the N- or C-terminus, only a single spacer sequence at the junction of the two components would be required. As would be apparent to one of ordinary skill in the art, the spacer sequences comprising amino acids compatible with restriction sites could be omitted from the construct when an entire GLP2-XTEN gene is synthetically generated, rather than ligated using GLP-2 and XTEN encoding genes.

**Table 5: Spacer Sequences Compatible with Restriction Sites**

| Spacer Sequence | Restriction Enzyme |
|---|---|
| GSPG | BsaI |
| ETET | BsaI |
| PGSSS | BbsI |
| GAP | AscI |
| GPA | FseI |
| GPSGP | SfiI |
| AAA | SacII |
| TG | AgeI |
| GT | KpnI |
| GAGSPGAETA | SfiI |
| ASS | XhoI |

In another aspect, the present invention provides GLP2-XTEN configurations with cleavage sequences incorporated into the spacer sequences. In some embodiments, a spacer sequence in a GLP2-XTEN fusion protein composition comprises one or more cleavage sequences, which are identical or different, wherein the cleavage sequence may be acted on by a protease to release the XTEN sequence(s) from the fusion protein. In one embodiment, the incorporation of the cleavage sequence into the GLP2-XTEN is designed to permit release of a GLP-2 that becomes active or more active upon its release from the XTEN component. The cleavage sequences are located sufficiently close to the GLP-2 sequences, generally within 18, or within 12, or within 6, or within 2 amino acids of the GLP-2 sequence, such that any remaining residues attached to the GLP-2s after cleavage do not appreciably interfere with the activity (e.g., such as binding to a GLP-2 receptor) of the GLP-2, yet provide sufficient access to the protease to be able to effect cleavage of the cleavage sequence. In some cases, the GLP2-XTEN comprising the cleavage sequences will also have one or more spacer sequence amino acids between the GLP-2 and the cleavage sequence or the XTEN and the cleavage sequence to facilitate access of the protease to the cleavage sequence; the spacer amino acids comprising any natural amino acid, including glycine, serine and alanine as preferred amino acids. In one embodiment, the cleavage site is a sequence that can be cleaved by a protease endogenous to the mammalian subject such that the GLP2-XTEN can be cleaved after administration to a subject. In such case, the GLP2-XTEN can serve as a prodrug or a circulating depot for the GLP-2. In a particular construct of the foregoing, the GLP2-XTEN would have one or two XTEN linked to the N- and/or the C-terminus such that the XTEN could be released, leaving the active form of GLP-2 free. In one embodiment of the foregoing construct, the GLP-2 that is released from the fusion protein by cleavage of the cleavage sequence exhibits at least about a two-fold, or at least about a three-fold, or at least about a four-fold, or at least about a five-fold, or at least about a six-fold, or at least about a eight-fold, or at least about a ten-fold, or at least about a 20-fold increase in biological activity compared to the intact GLP2-XTEN fusion protein.

Examples of cleavage sites contemplated by the invention include, but are not limited to, a polypeptide sequence cleavable by a mammalian endogenous protease selected from FXIa, FXIIa, kallikrein, FVIIIa, FVIIIa, FXa, FIIa (thrombin), Elastase-2, granzyme B, MMP-12, MMP-13, MMP-17 or MMP-20, or by non-mammalian proteases such as TEV, enterokinase, PreScission™ protease (rhinovirus 3C protease), and sortase A. Sequences known to be cleaved by the foregoing proteases and others are known in the art. Exemplary cleavage sequences contemplated by the invention and the respective cut sites within the sequences are presented in Table 6, as well as sequence variants thereof. Thus, cleavage sequences, particularly those of Table 6 that are susceptible to the endogenous proteases present during inflammation would provide for release of GLP-2 that, in certain embodiments of the GLP2-XTEN, provide a higher degree of activity for the GLP-2 component released from the intact form of the GLP2-XTEN, as well as additional safety margin for high doses of GLP2-XTEN administered to a subject. For example, it has been demonstrated that many of the metaloproteinases are elevated in Crohn's Disease and inflamed intestines (D Schuppan and T Freitag. Fistulising Crohn's disease: MMPs gone awry. Gut (2004) 53(5): 622-624). In one embodiment, the invention provides GLP2-XTEN comprising one or more cleavage sequences operably positioned to release the GLP-2 from the fusion protein upon cleavage, wherein the one or more cleavage sequences has at least about 86%, or at least about 92% or greater sequence identity to a sequence selected from Table 6. In another embodiment, the GLP2-XTEN comprising a cleavage sequence would have at least about 80%, or at least about 85%, or at least about 90%, or at least about 95%, or at least about 96%, or at least about 97%, or at least about 98%, or at least about 99% sequence identity compared to a sequence selected from Table 34.

In some embodiments, only the two or three amino acids flanking both sides of the cut site (four to six amino acids total) are incorporated into the cleavage sequence that, in turn, is incorporated into the GLP2-XTEN of the embodiments. In other embodiments, the incorporated cleavage sequence of Table 6 can have one or more deletions or insertions or one or two or three amino acid substitutions for any one or two or three amino acids in the known sequence, wherein the deletions, insertions or substitutions result in reduced or enhanced susceptibility but not an absence of susceptibility to the protease, resulting in an ability to tailor the rate of release of the GLP-2 from the XTEN. Exemplary substitutions are shown in Table 6.

**Table 6: Protease Cleavage Sequences**

| Protease Acting Upon Sequence | Exemplary Cleavage Sequence | Minimal Cut Site* |
|---|---|---|
| FXIa | KLTR↓AET | KD/FL/T/R↓VA/VE/GT/GV |
| FXIa | DFTR↓VVG | KD/FL/T/R↓VA/VE/GT/GV |
| FXIIa | TMTR↓IVGG | NA |
| Kallikrein | SPFR↓STGG | -/-/FL/RY↓SR/RT/-/- |
| FVIIa | LQVR↓IVGG | NA |
| FIXa | PLGR↓IVGG | -/-/G/R↓-/-/-/- |

(continued)

| Protease Acting Upon Sequence | Exemplary Cleavage Sequence | Minimal Cut Site* |
|---|---|---|
| FXa | IEGR↓TVGG | IA/E/GFP/R↓STI/VFS/-/G |
| FIIa (thrombin) | LTPR↓SLLV | -/-/PLA/R↓SAG/-/-/- |
| Elastase-2 | LGPV↓SGVP | -/-/-/VIAT↓-/-/-/- |
| Granzyme-B | VAGD↓SLEE | V/-/-/D↓-/-/-/- |
| MMP-12 | GPAG↓LGGA | G/PA/-/G↓L/-/G/- |
| MMP-13 | GPAG↓LRGA | G/P/-/G↓L/-/GA/- |
| MMP-17 | APLG↓LRLR | -/PS/-/-↓LQ/-/LT/- |
| MMP-20 | PALP↓LVAQ | NA |
| TEV | ENLYFQ↓G | ENLYFQ↓G/S |
| Enterokinase | DDDK↓VGG | DDDK↓IVGG |
| Protease 3C (PreScission™) | LEVLFQ↓GP | LEVLFQ↓GP |
| Sortase A | LPKT↓GSES | L/P/KEAD/T↓G/-/EKS/S |
| ↓ indicates cleavage site NA: not applicable ||| 
| * the listing of multiple amino acids before, between, or after a slash indicate alternative amino acids that can be substituted at the position; "-" indicates that any amino acid may be substituted for the corresponding amino acid indicated in the middle column ||| 

3. Exemplary GLP2-XTEN Fusion Protein Sequences

Non-limiting examples of sequences of fusion proteins containing a single GLP-2 linked to one or two XTEN, either joined at the N- or C-termini are presented in Tables 13 and 32. In one embodiment, a GLP2-XTEN composition would comprise a fusion protein having at least about 80% sequence identity compared to a GLP2-XTEN selected from Table 13 or Table 33, alternatively at least about 81%, 82%, 83%, 84%, 85%, 86%, 87%, 88%, 89%, 90%, 91%, 92%, 93%, 94%, 95%, 96%, 97%, 98%, 99%, or about 100% sequence identity as compared to a GLP2-XTEN from Table 13 or Table 33. However, the invention also contemplates substitution of any of the GLP-2 sequences of Table 1 for a GLP-2 component of the GLP2-XTEN of Table 13 or Table 33, and/or substitution of any sequence of Table 4 for an XTEN component of the GLP2-XTEN of Table 13 or Table 33. In preferred embodiments, the resulting GLP2-XTEN of the foregoing examples retain at least a portion of the biological activity of the corresponding GLP-2 not linked to the XTEN; e.g., the ability to bind and activate a GLP-2 receptor and/or result in an intestinotrophic, proliferative, or wound-healing effect. In the foregoing fusion proteins hereinabove described in this paragraph, the GLP2-XTEN fusion protein can further comprise one or more cleavage sequences; e.g., a sequence from Table 6, the cleavage sequence being located between the GLP-2 and the XTEN. In some embodiments comprising cleavage sequence(s), the intact GLP2-XTEN composition has less biological activity but a longer half-life in its intact form compared to a corresponding GLP-2 not linked to the XTEN, but is designed such that upon administration to a subject, the GLP-2 component is gradually released from the fusion protein by cleavage at the cleavage sequence(s) by endogenous proteases, whereupon the GLP-2 component exhibits activity, i.e., the ability to effectively bind to the GLP-2 receptor. In non-limiting examples, the GLP2-XTEN with a cleavage sequence has about 80% sequence identity compared to a sequence from Table 34, or about 85%, or about 90%, or about 95%, or about 97%, or about 98%, or about 99% sequence identity compared to a sequence from Table 34. However, the invention also contemplates substitution of any of the GLP-2 sequences of Table 1 for a GLP-2 component of the GLP2-XTEN of Table 34, substitution of any sequence of Table 4 for an XTEN component of the GLP2-XTEN of Table 34, and substitution of any cleavage sequence of Table 6 for a cleavage component of the GLP2-XTEN of Table 34. In some cases, the GLP2-XTEN of the foregoing embodiments in this paragraph serve as prodrugs or a circulating depot, resulting in a longer terminal half-life compared to GLP-2 not linked to the XTEN. In such cases, a higher concentration of GLP2-XTEN can be administered to a subject to maintain therapeutic blood levels for an extended period of time compared to the corresponding GLP-2 not linked to XTEN because a smaller proportion of the circulating composition is active.

The GLP2-XTEN compositions of the embodiments can be evaluated for biological activity using assays or *in vivo* parameters as described herein (e.g., assays of the Examples or assays of Table 32), or a pharmacodynamic effect in a preclinical model of GLP-2 deficiency or in clinical trials in humans, using methods as described in the Examples or other methods known in the art for assessing GLP-2 biological activity to determine the suitability of the configuration or the GLP-2 sequence variant, and those GLP2-XTEN compositions (including after cleavage of any incorporated XTEN-releasing cleavage sites) that retain at least about 40%, or about 50%, or about 55%, or about 60%, or about 70%, or about 80%, or about 90%, or about 95% or more biological activity compared to native GLP-2 sequence are considered suitable for use in the treatment of GLP-2-related conditions.

## V). PROPERTIES OF THE GLP2-XTEN COMPOSITIONS OF THE INVENTION

(a) Pharmacokinetic Properties of GLP2-XTEN

It is an object of the present invention to provide GLP2-XTEN fusion proteins with enhanced pharmacokinetics compared to GLP-2 not linked to the XTEN. The pharmacokinetic properties of a GLP-2 that can be enhanced by linking a given XTEN to the GLP-2 include, but are not limited to, terminal half-life, area under the curve (AUC), $C_{max}$, volume of distribution, maintaining the biologically active GLP2-XTEN within the therapeutic window above the minimum effective dose or blood unit concentration for a longer period of time compared to the GLP-2 not linked to XTEN, and bioavailability; properties that permits less frequent dosing or an enhanced pharmacologic effect, resulting in enhanced utility in the treatment of gastrointestinal conditions.

Native GLP-2 has been reported to have a terminal half-life in humans of approximately seven minutes (Jeppesen PB, et al., Teduglutide (ALX-0600), a dipeptidyl peptidase IV resistant glucagon-like peptide 2 analogue, improves intestinal function in short bowel syndrome patients. Gut. (2005) 54(9):1224-1231; Hartmann B, et al. (2000) Dipeptidyl peptidase IV inhibition enhances the intestinotrophic effect of glucagon-like peptide-2 in rats and mice. Endocrinology 141:4013-4020), while an analog teduglutide exhibited a terminal half-life of approximately 0.9-2.3 hr in humans (Marier JF, Population pharmacokinetics of teduglutide following repeated subcutaneous administrations in healthy participants and in patients with short bowel syndrome and Crohn's disease. J Clin Pharmacol. (2010) 50(1):36-49). It will be understood by the skilled artisan that the pharmacokinetic properties of the GLP2-XTEN embodiments are to be compared to comparable forms of GLP-2 not linked to the XTEN, i.e., recombinant, native sequence or a teduglutide-like analog.

As a result of the enhanced properties conferred by XTEN, the GLP2-XTEN, when used at the dose and dose regimen determined to be appropriate for the composition by the methods described herein, administration of a GLP2-XTEN fusion protein composition can achieve a circulating concentration resulting in a desired pharmacologic or clinical effect for an extended period of time compared to a comparable dose of the corresponding GLP-2 not linked to the XTEN. As used herein, a "comparable dose" means a dose with an equivalent moles/kg for the active GLP-2 pharmacophore (e.g., GLP-2) that is administered to a subject in a comparable fashion. It will be understood in the art that a "comparable dosage" of GLP2-XTEN fusion protein would represent a greater weight of agent but would have essentially the same mole-equivalents of GLP-2 in the dose of the fusion protein administered.

In one embodiment, the invention provides GLP2-XTEN that enhance the pharmacokinetics of the fusion protein by linking one or more XTEN to the GLP-2 component of the fusion protein, wherein the fusion protein has an increase in apparent molecular weight factor of at least about two-fold, or at least about three-fold, or at least about four-fold, or at least about five-fold, or at least about six-fold, or at least about seven-fold, or at least about eight-fold, or at least about ten-fold, or at least about twelve-fold, or at least about fifteen-fold, and wherein the terminal half-life of the GLP2-XTEN when administered to a subject is increased at least about 2-fold, or at least about 3-fold, or at least about 4-fold, or at least about 5-fold, or at least about 6-fold, or at least about 7-fold, or at least about 8-fold, or at least about 10-fold or more compared to the corresponding GLP-2 not linked to the XTEN. In the foregoing embodiment, wherein the fusion protein comprises at least two XTEN molecules incorporated into the GLP2-XTEN, the XTEN can be identical or they can be of a different sequence composition (and net charge) or length. The XTEN can have at least about 80% sequence identity, or at least about 90%, or at least about 95%, or at least about 98%, or at least about 99% sequence identity compared to a sequence selected from Table 4. Not to be bound by a particular theory, the XTEN of the GLP2-XTEN compositions with the higher net charge are expected, as described above, to have less non-specific interactions with various negatively-charged surfaces such as blood vessels, tissues, or various receptors, which would further contribute to reduced active clearance. Conversely, the XTEN of the GLP2-XTEN compositions with a low (or no) net charge are expected to have a higher degree of interaction with surfaces that potentiate the biological activity of the associated GLP-2, given the known association of inflammatory cells in the intestines during an inflammatory response. Thus, the invention provides GLP2-XTEN in which the degree of potency, bioavailability, and half-life of the fusion protein can be tailored by the selection and placement of the type and length of the XTEN in the GLP2-XTEN compositions. Accordingly, the invention contemplates compositions in which a GLP-2 from Table 1 and XTEN from Table 4 are combined and are produced, for example, in a configuration selected from any one of formulae I-VI such that the construct has enhanced pharmacokinetic properties and reduced systemic clearance. The invention further takes advantage of the fact that certain ligands with reduced binding to a clearance receptor, either as a result of a decreased on-rate or an increased off-rate, may be effected by the obstruction of either the N- or C-terminus and using that terminus as the linkage to another polypeptide of the composition, whether another molecule of a GLP-2, an XTEN, or a spacer sequence results in the reduced binding. The choice of the particular configuration of the GLP2-XTEN fusion protein can be tested by methods disclosed herein to confirm those configurations that reduce the degree of binding to a clearance receptor such that a reduced rate of active clearance is achieved.

In one embodiment, the invention provides GLP2-XTEN with enhanced pharmacokinetic properties wherein the GLP2-XTEN is a sequence that has at least about 80% sequence identity, or alternatively 81%, 82%, 83%, 84%, 85%, 86%, 87%, 88%, 89%, 90%, 91%, 92%, 93%, 94%, 95%, 96%, 97%, 98%, 99%, or 100% sequence identity compared to a sequence selected from any one of Tables 13, 32 or 33. In other embodiments, the GLP2-XTEN with enhanced pharmacokinetic properties comprises a GLP-2 sequence that has at least about 80% sequence identity, or alternatively 81%, 82%, 83%, 84%, 85%, 86%, 87%, 88%, 89%, 90%, 91%, 92%, 93%, 94%, 95%, 96%, 97%, 98%, or about 99% sequence identity compared to a sequence from Table 1 linked to one or more XTEN that has at least about 80% sequence identity, or alternatively 81%, 82%, 83%, 84%, 85%, 86%, 87%, 88%, 89%, 90%, 91%, 92%, 93%, 94%, 95%, 96%, 97%, 98%, or about 99% sequence identity compared to a sequence from Table 4. For the subject compositions, GLP2-XTEN with a longer terminal half-life is generally preferred, so as to improve patient convenience, to increase the interval between doses and to reduce the amount of drug required to achieve a sustained effect. In the embodiments hereinabove described in this paragraph the administration of the fusion protein results in an improvement in at least one, two, three or more of the parameters disclosed herein as being useful for assessing the subject conditions; e.g., maintaining a blood concentration, maintaining bowel function, preventing onset of a symptom associated with a gastrointestinal condition such as colitis, short bowel syndrome or Crohn's Disease, using a lower dose of fusion protein compared to the corresponding GLP-2 component not linked to the fusion protein and administered at a comparable dose or dose regimen to a subject. Alternatively, in the embodiments hereinabove described in this paragraph the administration of the fusion protein results in an improvement in at least one of the parameters disclosed herein as being useful for assessing the subject conditions using a comparable dose of fusion protein but administered using a dose regimen that has a 2-fold, or 3-fold, or 4-fold, or 5-fold, or 6-fold, or 7-fold, or 8-fold, or 10-fold, or 20-fold greater interval between dose administrations compared to the corresponding GLP-2 component not linked to the fusion protein and administered to the subject. In the foregoing embodiments, the total dose in millimoles/kg administered to achieve the improvement in the parameter(s) is at least about three-fold lower, or at least about four-fold, or at least about five-fold, or at least about six-fold, or at least about eight-fold, or at least about 10-fold lower compared to the corresponding GLP-2 component not linked to the XTEN.

As described more fully in the Examples pertaining to pharmacokinetic characteristics of fusion proteins comprising XTEN, it was observed that increasing the length of the XTEN sequence confers a disproportionate increase in the terminal half-life of a fusion protein comprising the XTEN. Accordingly, the invention provides GLP2-XTEN fusion proteins comprising XTEN wherein the XTEN is selected to provide a targeted half-life for the GLP2-XTEN composition administered to a subject. In some embodiments, the invention provides monomeric GLP2-XTEN fusion proteins comprising XTEN wherein the XTEN is selected to confer an increase in the terminal half-life for the GLP2-XTEN administered to a subject, compared to the corresponding GLP-2 not linked to the XTEN and administered at a comparable dose, wherein the increase is at least about two-fold longer, or at least about three-fold, or at least about four-fold, or at least about five-fold, or at least about six-fold, or at least about seven-fold, or at least about eight-fold, or at least about nine-fold, or at least about ten-fold, or at least about 15-fold, or at least a 20-fold, or at least a 40-fold or greater an increase in terminal half-life compared to the GLP-2 not linked to the XTEN. In another embodiment, the administration of a therapeutically effective amount of GLP2-XTEN to a subject in need thereof results in a terminal half-life that is at least 12 h greater, or at least about 24 h greater, or at least about 48 h greater, or at least about 72 h greater, or at least about 96 h greater, or at least about 144 h greater, or at least about 7 days greater, or at least about 14 days greater, or at least about 21 days greater compared to a comparable dose of the corresponding GLP-2 not linked to the XTEN. In another embodiment, administration of a therapeutically effective dose of a GLP2-XTEN fusion protein to a subject in need thereof can result in a gain in time between consecutive doses necessary to maintain a therapeutically effective blood level of the fusion protein of at least 48 h, or at least 72 h, or at least about 96 h, or at least about 120 h, or at least about 7 days, or at least about 14 days, or at least about 21 days between consecutive doses compared to the corresponding GLP-2 not linked to the XTEN and administered at a comparable dose. It will be understood in the art that the time between consecutive doses to maintain a "therapeutically effective blood level" will vary greatly depending on the physiologic state of the subject, and it will be appreciated that a patient with Crohn's Disease may require more frequent and longer dosing of a GLP-2 preparation compared to a patient receiving the same preparation for short bowel syndrome. The foregoing notwithstanding, it is believed that the GLP2-XTEN of the present invention permit less frequent dosing, as described above, compared to a GLP-2 not linked to the XTEN. In one embodiment, the GLP2-XTEN administered using a therapeutically-effective amount to a subject results in blood concentrations of the GLP2-XTEN fusion protein that remains above at least 500 ng/ml, or at least about 1000 ng/ml, or at least about 2000 ng/ml, or at least about 3000 ng/ml, or at least about 4000 ng/ml, or at least about 5000 ng/ml, or at least about 10000 ng/ml, or at least about 15000 ng/ml, or at least about 20000 ng/ml, or at least about 30000 ng/ml, or at least about 40000 ng/ml for at least about 24 hours, or at least about 48 hours, or at least about 72 hours, or at least about 96 hours, or at least about 120 hours, or at least about 144 hours.

In one embodiment, the present invention provides GLP2-XTEN fusion proteins that exhibits an increase in AUC of at least about 50%, or at least about 60%, or at least about 70%, or at least about 80%, or at least about 90%, or at least about a 100%, or at least about 150%, or at least about 200%, or at least about 300%, or at least about 500%, or at least about 1000%, or at least about a 2000% compared to the corresponding GLP-2 not linked to the XTEN and administered to a subject at a comparable dose. In another embodiment, the GLP2-XTEN administered at an appropriate dose to a subject results in area under the curve concentrations of the GLP2-XTEN fusion protein of at least 100000 hr*ng/mL, or at least about 200000 hr*ng/mL, or at least about 400000 hr*ng/mL, or at least about 600000 hr*ng/mL, or at least about 800000 hr*ng/mL, or at least about 1000000 hr*ng/mL, or at least about 2000000 hr*ng/mL after a single dose. The pharmacokinetic parameters of a GLP2-XTEN can be determined by standard methods involving dosing, the taking of blood samples at times intervals, and the assaying of the protein using ELISA, HPLC, radioassay, or other methods known in the art or as described herein, followed by standard calculations of the data to derive the half-life and other PK parameters.

The enhanced PK parameters allow for reduced dosing of the GLP2-XTEN compositions, compared to GLP-2 not linked to the XTEN, particularly for those subjects receiving doses for routine prophylaxis or chronic treatment of a gastrointestinal condition. In one embodiment, a smaller moles-equivalent amount of about two-fold less, or about three-fold less, or about four-fold less, or about five-fold less, or about six-fold less, or about eight-fold less, or about 10-fold less or greater of the fusion protein is administered in comparison to the corresponding GLP-2 not linked to the XTEN under a dose regimen needed to maintain a comparable area under the curve as the corresponding amount of the GLP-2 not linked to the XTEN. In another embodiment, a smaller amount of moles of about two-fold less, or about three-fold less, or about four-fold less, or about five-fold less, or about six-fold less, or about eight-fold less, or about 10-fold less or greater of the fusion protein is administered in comparison to the corresponding GLP-2 not linked to the XTEN under a dose regimen needed to maintain a blood concentration above at least about 500 ng/ml, at least about 1000 ng/ml, or at least about 2000 ng/ml, or at least about 3000 ng/ml, or at least about 4000 ng/ml, or at least about 5000 ng/ml, or at least about 10000 ng/ml, or at least about 15000 ng/ml, or at least about 20000 ng/ml, or at least about 30000 ng/ml, or at least about 40000 ng/ml for at least about 24 hours, or at least about 48 h, or at least 72 h, or at least 96 h, or at least 120 h compared to the corresponding amount of the GLP-2 not linked to the XTEN. In another embodiment, the GLP2-XTEN fusion protein requires less frequent administration for treatment of a subject with gastrointestinal condition, wherein the dose is administered about every four days, about every seven days, about every 10 days, about every 14 days, about every 21 days, or about monthly of the fusion protein administered to a subject, and the fusion protein achieves a comparable area under the curve as the corresponding GLP-2 not linked to the XTEN. In yet other embodiments, an accumulatively smaller amount of moles of about 5%, or about 10%, or about 20%, or about 40%, or about 50%, or about 60%, or about 70%, or about 80%, or about 90% less of the fusion protein is administered to a subject in comparison to the corresponding amount of the GLP-2 not linked to the XTEN under a dose regimen needed to achieve the therapeutic outcome or clinical parameter, yet the fusion protein achieves at least a comparable area under the curve as the corresponding GLP-2 not linked to the XTEN. The accumulative smaller amount is measure for a period of at least about one week, or about 14 days, or about 21 days, or about one month.

(b) Pharmacology and Pharmaceutical Properties of GLP2-XTEN

The present invention provides GLP2-XTEN compositions comprising GLP-2 covalently linked to the XTEN that can have enhanced properties compared to GLP-2 not linked to XTEN, as well as methods to enhance the therapeutic and/or biologic activity or effect of the respective two GLP-2 components of the compositions. In addition, GLP2-XTEN fusion proteins provide significant advantages over chemical conjugates, such as pegylated constructs of GLP-2, notably the fact that recombinant GLP2-XTEN fusion proteins can be made in host cell expression systems, which can reduce time and cost at both the research and development and manufacturing stages of a product, as well as result in a more homogeneous, defined product with less toxicity for both the product and metabolites of the GLP2-XTEN compared to pegylated conjugates.

As therapeutic agents, the GLP2-XTEN possesses a number of advantages over therapeutics not comprising XTEN, including one or more of the following non-limiting enhanced properties: increased solubility, increased thermal stability, reduced immunogenicity, increased apparent molecular weight, reduced renal clearance, reduced proteolysis, reduced metabolism, enhanced therapeutic efficiency, a lower effective therapeutic dose, increased bioavailability, increased time between dosages capable of maintaining a subject without increased symptoms of colitis, enteritis, or Crohn's Disease, the ability to administer the GLP2-XTEN composition intravenously, subcutaneously, or intramuscularly, a "tailored" rate of absorption when administered intravenously, subcutaneously, or intramuscularly, enhanced lyophilization stability, enhanced serum/plasma stability, increased terminal half-life, increased solubility in blood stream, decreased binding by neutralizing antibodies, decreased active clearance, reduced side effects, reduced immunogenicity, retention of substrate binding affinity, stability to degradation, stability to freeze-thaw, stability to proteases, stability to ubiquitination, ease of administration, compatibility with other pharmaceutical excipients or carriers, persistence in the subject, increased stability in storage (e.g., increased shelf-life), reduced toxicity in an organism or environment and the like. The GLP2-XTEN fusion proteins of the embodiments disclosed herein exhibit one or more or any combination of the improved properties and/or the embodiments as detailed herein. The net effect of the enhanced properties is that the use of a GLP2-XTEN composition can result in enhanced therapeutic and/or biologic effect compared to a GLP-2 not linked to the XTEN, result in economic benefits associated with less frequent dosing, or result in improved patient compliance when administered to a subject with a GLP-2-related condition.

In one embodiment, XTEN as a fusion partner increases the solubility of the GLP-2 payload. Accordingly, where enhancement of the pharmaceutical or physicochemical properties of the GLP-2 is desirable, such as the degree of aqueous solubility or stability, the length and/or the motif family composition of the XTEN sequences incorporated into the fusion protein may each be selected to confer a different degree of solubility and/or stability on the respective fusion proteins such that the overall pharmaceutical properties of the GLP2-XTEN composition are enhanced. The GLP2-XTEN fusion proteins can be constructed and assayed, using methods described herein, to confirm the physicochemical properties and the XTEN adjusted, as needed, to result in the desired properties. In one embodiment, the GLP2-XTEN has an aqueous solubility that is at least about 25% greater compared to a GLP-2 not linked to the fusion protein, or at least about 30%, or at least about 40%, or at least about 50%, or at least about 75%, or at least about 100%, or at least about 200%, or at least about 300%, or at least about 400%, or at least about 500%, or at least about 1000% greater than the corresponding GLP-2 not linked to the fusion protein.

The invention provides methods to produce and recover expressed GLP2-XTEN from a host cell with enhanced solubility and ease of recovery compared to GLP-2 not linked to the XTEN. In one embodiment, the method includes the steps of transforming a host cell with a polynucleotide encoding a GLP2-XTEN with one or more XTEN components of cumulative sequence length greater than about 100, or greater than about 200, or greater than about 400, or greater than about 800 amino acid residues, expressing the GLP2-XTEN fusion protein in the host cell, and recovering the expressed fusion protein in soluble form. In the foregoing embodiment, the XTEN of the GLP2-XTEN fusion proteins can have at least about 80% sequence identity, or about 90%, or about 91%, or about 92%, or about 93%, or about 94%, or about 95%, or about 96%, or about 97%, or about 98%, or about 99%, to about 100% sequence identity compared to one or more XTEN selected from Table 4, and the GLP-2 can have at least about 80% sequence identity, or about 90%, or about 91%, or about 92%, or about 93%, or about 94%, or about 95%, or about 96%, or about 97%, or about 98%, or about 99%, or 100% sequence identity compared to a GLP-2 selected from Table 1 and the GLP2-XTEN components can be in an N- to C-terminus configuration selected from any one of formulae I-VI.

The invention provides methods to produce the GLP2-XTEN compositions that can maintain the GLP-2 component at therapeutic levels when administered to a subject in need thereof for at least a two-fold, or at least a three-fold, or at least a four-fold, or at least a five-fold greater period of time compared to comparable dosages of the corresponding GLP-2 not linked to the XTEN. It will be understood in the art that a "comparable dosage" of GLP2-XTEN fusion protein would represent a greater weight of agent but would have the same approximate moles of GLP-2 in the dose of the fusion protein and/or would have the same approximate nmol/kg concentration relative to the dose of GLP-2 not linked to the XTEN. The method to produce the compositions that can maintain the GLP-2 component at therapeutic levels includes the steps of selecting the XTEN appropriate for conjugation to a GLP-2 to provide the desired pharmacokinetic properties in view of a given dose and dose regimen, creating an expression construct that encodes the GLP2-XTEN using a configuration described herein, transforming an appropriate host cell with an expression vector comprising the encoding gene, expressing and recovering the GLP2-XTEN, administration of the GLP2-XTEN to a subject followed by assays to verify the pharmacokinetic properties, the activity of the GLP2-XTEN fusion protein (e.g., the ability to bind receptor), and the safety of the administered composition. The subject can be selected from mouse, rat, monkey and human. By the methods, GLP2-XTEN provided herein can result in increased efficacy of the administered composition by maintaining the circulating concentrations of the GLP-2 at therapeutic levels for an enhanced period of time.

In another aspect, the GLP2-XTEN compositions of the invention are capable of resulting in an intestinotrophic effect. As used herein, "intestinotrophic effect" means that a subject, e.g., mouse, rat, monkey or human, exhibits at least one of the following after administration of a GLP-2 containing composition: intestinal growth, increased hyperplasia of the villus epithelium, increased crypt cell proliferation, increased the height of the crypt and villus axis, increased healing after intestinal anastomosis, increased small bowel weight, increased small bowel length, decreased small bowel epithelium apoptosis, or enhancement of intestinal function. The GLP2-XTEN compositions may act in an endocrine fashion to link intestinal growth and metabolism with nutrient intake.. GLP-2 and related analogs may be treatments for short bowel syndrome, Crohn's disease, osteoporosis and as adjuvant therapy during cancer chemotherapy, amongst other gastrointestinal conditions described herein. In one embodiment, a GLP2-XTEN is capable of resulting in at least one, or two, or three or more intestinotrophic effects when administered to a subject using an effective amount.

The characteristics of GLP2-XTEN compositions of the invention, including functional characteristics or biologic and pharmacologic activity and parameters that result, can be determined by any suitable screening assay known in the art for measuring the desired characteristic. The invention provides methods to assay the GLP2-XTEN fusion proteins of differing composition or configuration in order to provide GLP2-XTEN with the desired degree of biologic and/or therapeutic activity, as well as safety profile. Specific *in vitro*, *in vivo* and *ex vivo* biological assays are used to assess the activity of each configured GLP2-XTEN and/or GLP-2 component to be incorporated into GLP2-XTEN, including but not limited to the assays of the Examples, assays of Table 32, determination of inflammatory cytokine levels, GLP-2 blood concentrations, ELISA assays, or bowel function tests, as well as clinical endpoints such as bleeding, inflammation, colitis, diarrhea, fecal wet weight, weight loss, sodium loss, intestinal ulcers, intestinal obstruction, fistulae, and abscesses, survival, among others known in the art. The foregoing assays or endpoints can also be used in preclinical assays to assess GLP-2 sequence variants (assayed as single components or as GLP2-XTEN fusion proteins) and can be compared to the native human GLP-2 to determine whether they have the same degree of biologic activity as the native GLP-2, or some fraction thereof such that they are suitable for inclusion in GLP2-XTEN. In one embodiment, the invention provides GLP2-XTEN fusion proteins that exhibit at least about 30%, or at least about 40%, or at least about 50%, or at least about 60%, or at least about 70%, or at least about 80%, or at least about 90%, or at least about 100% or at least about 120% or at least about 150% or at least about 200% of the intestinotrophic effect compared to the corresponding GLP-2 not linked to XTEN and administered to a subject using a comparable dose.

Dose optimization is important for all drugs. A therapeutically effective dose or amount of the GLP2-XTEN varies according to factors such as the disease state, age, sex, and weight of the individual, and the ability of the administered fusion protein to elicit a desired response in the individual. For example, a standardized single dose of GLP-2 for all patients presenting with diverse pulmonary conditions or abnormal clinical parameters (e.g., neutralizing antibodies) may not always be effective. A consideration of these factors is well within the purview of the ordinarily skilled clinician for the purpose of determining the therapeutically or pharmacologically effective amount of the GLP2-XTEN and the appropriated dosing schedule, versus that amount that would result in insufficient potency such that clinical improvement is not achieved.

The methods of the invention includes administration of consecutive doses of a therapeutically effective amount of the GLP2-XTEN for a period of time sufficient to achieve and/or maintain the desired parameter or clinical effect, and such consecutive doses of a therapeutically effective amount establishes the therapeutically effective dose regimen for the GLP2-XTEN, i.e., the schedule for consecutively administered doses of the fusion protein composition, wherein the doses are given in amounts to result in a sustained beneficial effect on any clinical sign or symptom, aspect, measured parameter or characteristic of a GLP-2-related disease state or condition, including, but not limited to, those described herein. A prophylactically effective amount refers to an amount of GLP2-XTEN required for the period of time necessary to prevent a physiologic or clinical result or event; e.g., reduced mesenteric blood flow, bleeding, inflammation, colitis, diarrhea, fecal wet weight, weight loss, sodium loss, intestinal ulcers, intestinal obstruction, fistulae, and abscesses, changed frequency in bowel movements, uveitis, as well growth failure in children, or maintaining blood concentrations of GLP-2 above a threshold level, e.g., 100 ng/ml of GLP-2 equivalent (or approximately 2200 ng/ml of GLP-2-2G_XTEN_AE864) or 30 pmol/L. In the methods of treatment, the dosage amount of the GLP2-XTEN that is administered to a subject ranges from about 0.2 to 500 mg/kg/dose (2.5 nmol/kg - 6250 nmol/kg), or from about 2 to 300 mg/kg/dose (25 nmol/kg - 3750 nmol/kg), or from about 6 to about 100 mg/kg/dose (75 nmol/kg/dose - 1250 nmol/kg/dose), or from about 10 to about 60 mg/kg/dose (125 nmol/kg/dose - 750 nmol/kg/dose) for a subject. A suitable dosage may also depend on other factors that may influence the response to the drug; e.g., subjects with surgically resected bowel generally requiring higher doses compared to irritable bowel syndrome. In some embodiments, the method comprises administering a therapeutically-effective amount of a pharmaceutical composition comprising a GLP2-XTEN fusion protein composition comprising GLP-2 linked to one or more XTEN sequences and at least one pharmaceutically acceptable carrier to a subject in need thereof that results in a greater improvement in at least one of the disclosed parameters or physiologic conditions, or results in a more favorable clinical outcome compared to the effect on the parameter, condition or clinical outcome mediated by administration of a pharmaceutical composition comprising a GLP-2 not linked to XTEN and administered at a comparable dose. In one embodiment of the foregoing, the improvement is achieved by administration of the GLP2-XTEN pharmaceutical composition at a therapeutically effective dose. In another embodiment of the foregoing, the improvement is achieved by administration of multiple consecutive doses of the GLP2-XTEN pharmaceutical composition using a therapeutically effective dose regimen (as defined herein) for the length of the dosing period.

In many cases, the therapeutic levels for GLP-2 in subjects of different ages or degree of disease have been established and are available in published literature or are stated on the drug label for approved products containing the GLP-2. In other cases, the therapeutic levels can be established for new compositions, including those GLP2-XTEN fusion proteins of the disclosure. The methods for establishing the therapeutic levels and dosing schedules for a given composition are known to those of skill in the art (*see,* e.g., Goodman & Gilman's The Pharmacological Basis of Therapeutics, 11th Edition, McGraw-Hill (2005)). For example, by using dose-escalation studies in subjects with the target disease or condition to determine efficacy or a desirable pharmacologic effect, appearance of adverse events, and determination of circulating blood levels, the therapeutic blood levels for a given subject or population of subjects can be determined for a given drug or biologic. The dose escalation studies can evaluate the activity of a GLP2-XTEN through metabolic studies in a subject or group of subjects that monitor physiological or biochemical parameters, as known in the art or as described herein for one or more parameters associated with the GLP-2-related condition, or clinical parameters associated with a beneficial outcome for the particular indication, together with observations and/or measured parameters to determine the no effect dose, adverse events, minimum effective dose and the like, together with measurement of pharmacokinetic parameters that establish the determined or derived circulating blood levels. The results can then be correlated with the dose administered and the blood concentrations of the therapeutic that are coincident with the foregoing determined parameters or effect levels. By these methods, a range of doses and blood concentrations can be correlated to the minimum effective dose as well as the maximum dose and blood concentration at which a desired effect occurs and the period for which it can be maintained, thereby establishing the therapeutic blood levels and dosing schedule for the composition. Thus, by the foregoing methods, a $C_{min}$ blood level is established, below which the GLP2-XTEN fusion protein would not have the desired pharmacologic effect and a $C_{max}$ blood level, above which side effects may occur.

One of skill in the art can, by the means disclosed herein or by other methods known in the art, confirm that the administered GLP2-XTEN remains at therapeutic blood levels yet retains adequate safety (thereby establishing the "therapeutic window") to maintain biological activity for the desired interval or requires adjustment in dose or length or sequence of XTEN. Further, the determination of the appropriate dose and dose frequency to keep the GLP2-XTEN within the therapeutic window establishes the therapeutically effective dose regimen; the schedule for administration of multiple consecutive doses using a therapeutically effective dose of the fusion protein to a subject in need thereof resulting in consecutive $C_{max}$ peaks and/or $C_{min}$ troughs that remain above therapeutically-effective concentrations and result in an improvement in at least one measured parameter relevant for the target condition. In one embodiment, the GLP2-XTEN administered at an appropriate dose to a subject results in blood concentrations of the GLP2-XTEN fusion protein that remains above the minimum effective concentration to maintain a given activity or effect (as determined by the assays of the Examples or Table 32) for a period at least about two-fold longer compared to the corresponding GLP-2 not linked to XTEN and administered at a comparable dose; alternatively at least about three-fold longer; alternatively at least about four-fold longer; alternatively at least about five-fold longer; alternatively at least about six-fold longer; alternatively at least about seven-fold longer; alternatively at least about eight-fold longer; alternatively at least about nine-fold longer, alternatively at least about ten-fold longer, or at least about twenty-fold longer or greater compared to the corresponding GLP-2 not linked to XTEN and administered at a comparable dose. As used herein, an "appropriate dose" means a dose of a drug or biologic that, when administered to a subject, would result in a desirable therapeutic or pharmacologic effect and/or a blood concentration within the therapeutic window. For example, serum or plasma levels of GLP-2 or XTEN-containing fusion proteins comprising GLP-2 can be measured by nephelometry, ELISA, HPLC, radioimmunoassay or by immunoelectrophoresis (Jeppesen PB. Impaired meal stimulated glucagon-like peptide 2 response in ileal resected short bowel patients with intestinal failure. Gut. (1999) 45(4):559-963; assays of Examples 18-21). Phenotypic identification of GLP-2 or GLP-2 variants can be accomplished by a number of methods including isoelectric focusing (IEF) (Jeppsson et al., Proc. Natl. Acad. Sci. USA, 81:5690-93, 1994), or by DNA analysis (Kidd et al., Nature, 304:230-34, 1983; Braun et al., Eur. J. Clin. Chem. Clin. Biochem., 34:761-64, 1996).

In one embodiment, administration of at least two doses, or at least three doses, or at least four or more doses of a GLP2-XTEN using a therapeutically effective dose regimen results in a gain in time of at least about three-fold longer; alternatively at least about four-fold longer; alternatively at least about five-fold longer; alternatively at least about six-fold longer; alternatively at least about seven-fold longer; alternatively at least about eight-fold longer; alternatively at least about nine-fold longer or at least about ten-fold longer between at least two consecutive $C_{max}$ peaks and/or $C_{min}$ troughs for blood levels of the fusion protein compared to the corresponding biologically active protein of the fusion protein not linked to the XTEN and administered at a comparable dose regimen to a subject. In another embodiment, the GLP2-XTEN administered at a therapeutically effective dose regimen results in a comparable improvement in one, or two, or three or more measured parameters using less frequent dosing or a lower total dosage in moles of the fusion protein of the pharmaceutical composition compared to the corresponding biologically active protein component(s) not linked to the XTEN and administered to a subject using a therapeutically effective dose regimen for the GLP-2. The measured parameters include any of the clinical, biochemical, or physiological parameters disclosed herein, or others known in the art for assessing subjects with GLP-2-related condition. Non-limiting examples of parameters or physiologic effects that can be assayed to assess the activity of the GLP2-XTEN fusion proteins include assays of the Example, Table 32 or tests or assays to detect reduced mesenteric blood flow, bleeding, inflammation, colitis, diarrhea, fecal wet weight, sodium loss, weight loss, intestinal ulcers, intestinal obstruction, fistulae, and abscesses, changed frequency in bowel movements, uveitis, growth failure in children, or maintaining blood concentrations of GLP-2 above a threshold level, e.g., 100 ng/ml of GLP-2 equivalent (or approximately 2200 ng/ml of GLP-2-2G_XTEN_AE864), as well as parameters obtained from experimental animal models of enteritis such as body weight gain, small intestine length, reduction in TNFα content of the small intestine, reduced mucosal atrophy, reduced incidence of perforated ulcers, and height of villi.

In some embodiments, the biological activity of the GLP-2 component is manifested by the intact GLP2-XTEN fusion protein, while in other cases the biological activity of the GLP-2 component is primarily manifested upon cleavage and release of the GLP-2 from the fusion protein by action of a protease that acts on a cleavage sequence incorporated into the GLP2-XTEN fusion protein using configurations and sequences described herein. In the foregoing, the GLP2-XTEN is designed to reduce the binding affinity of the GLP-2 component for the GLP-2 receptor when linked to the XTEN but have restored or increased affinity when released from XTEN through the cleavage of cleavage sequence(s) incorporated into the GLP2-XTEN sequence. In one embodiment of the foregoing, the invention provides an isolated fusion protein comprising a GLP-2 linked to at least a first XTEN by a cleavage sequence, wherein the fusion protein has less than 10% or the biological activity (e.g., receptor binding) prior to cleavage and wherein the GLP-2 released from the fusion protein by proteolytic cleavage at the cleavage sequence has biological activity that is at least about 40%, at least about 50%, at least about 60%, or at least about 70%, or at least about 80%, or at least about 90%, or at least about 95% as active compared to native GLP-2 not linked to the XTEN.

In one aspect, the invention provides GLP2-XTEN compositions designed to reduce active clearance of the fusion protein, thereby increasing the terminal half-life of GLP2-XTEN administered to a subject, while still retaining biological activity. Without being bound by any particular theory, it is believed that the GLP2-XTEN of the present invention have comparatively higher and/or sustained activity achieved by reduced active clearance of the molecule by the addition of unstructured XTEN to the GLP-2. Uptake, elimination, and inactivation of GLP-2 can occur in the circulatory system as well as in the extravascular space.

## VI). USES OF THE GLP2-XTEN COMPOSITIONS

In another aspect, the invention provides GLP2-XTEN fusion proteins for use in methods of treatment, including treatment for achieving a beneficial effect in a gastrointestinal condition mediated or ameliorated by GLP-2. As used herein, "gastrointestinal condition" is intended to include, but is not limited to gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, obesity, steatorrhea, autoimmune diseases, gastrointestinal barrier disorders, sepsis, bacterial peritonitis, burn-induced intestinal damage, decreased gastrointestinal motility, intestinal failure, chemotherapy-associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, and gastrointestinal-induced ischemia.

The present invention provides GLP2-XTEN fusion proteins for use in methods for treating a subject, such as a human, with a GLP-2-related disease, disorder or gastrointestinal condition in order to achieve a beneficial effect, addressing disadvantages and/or limitations of other methods of treatment using GLP-2 preparations that have a relatively short terminal half-life, require repeated administrations, or have unfavorable pharmacoeconomics. The fact that GLP-2 native, recombinant or synthetic proteins have a short half-life necessitates frequent dosing in order to achieve clinical benefit, which results in difficulties in the management of such patients.

In one embodiment, the method of treatment comprises administering a therapeutically-effective amount of a GLP2-XTEN composition to a subject with a gastrointestinal condition. In another embodiment of the method of treatment, the administration of the GLP2-XTEN composition results in the improvement of one, two, three or more biochemical, physiological or clinical parameters associated with the gastrointestinal condition. In the foregoing method, the administered GLP2-XTEN comprises a GLP-2 with at least about 80%, or at least about 90%, or at least about 95%, or at least about 97%, or at least about 99% sequence identity to a GLP-2 of Table 1 linked to at least a first XTEN with at least about 80%, or at least about 90%, or at least about 95%, or at least about 97%, or at least about 99% sequence identity to a XTEN selected from any one of Tables 4, and 8-12. In another embodiment of the foregoing method, the administered GLP2-XTEN has a sequence with at least about 80%, or at least about 90%, or at least about 95%, or at least about 97%, or at least about 99% sequence identity to a sequence from Tables 13, 32, or 33. In one embodiment, the method of treatment comprises administering a therapeutically-effective amount of a GLP2-XTEN composition in one or more doses to a subject with a gastrointestinal condition wherein the administration results in the improvement of one, two, three or more biochemical, physiological or clinical parameters or a therapeutic effect associated with the condition for a period at least two-fold longer, or at least four-fold longer, or at least five-fold longer, or at least six-fold longer compared to a GLP-2 not linked to the XTEN and administered using a comparable amount. In another embodiment, the method of treatment comprises administering a therapeutically-effective amount of a GLP2-XTEN composition to a subject suffering from GLP-2 deficiency wherein the administration results in preventing onset of a clinically relevant parameter or symptom or dropping below a clinically-relevant blood concentration for a duration at least two-fold, or at least three-fold, or at least four-fold longer compared to a GLP-2 not linked to the XTEN. In another embodiment, the method of treatment comprises administering a therapeutically-effective amount of a GLP2-XTEN to a subject with a gastrointestinal condition, wherein the administration results in at least a 5%, or 10%, or 20%, or 30%, or 40%, or 50%, or 60%, or 70%, or 80%, or 90% greater improvement of at least one, two, or three parameters associated with the gastrointestinal condition compared to the GLP-2 not linked to XTEN and administered using a comparable nmol/kg amount. In the foregoing embodiments of the method of treatment, the administration is subcutaneous, intramuscular, or intravenous. In the foregoing embodiments of the method of treatment, the subject is selected from the group consisting of mouse, rat, monkey, and human. In the foregoing embodiments of the method of treatment, the therapeutic effect or parameter includes, but is not limited to, blood concentrations of GLP-2, increased mesenteric blood flow, decreased inflammation, increased weight gain, decreased diarrhea, decreased fecal wet weight, intestinal wound healing, increase in plasma citrulline concentrations, decreased CRP levels, decreased requirement for steroid therapy, enhancing or stimulating mucosal integrity, decreased sodium loss, minimizing, mitigating, or preventing bacterial translocation in the intestines, enhancing, stimulating or accelerating recovery of the intestines after surgery; preventing relapses of inflammatory bowel disease; or achieving or maintaining energy homeostasis, among others.

In one embodiment, the method of treatment is used to treat a subject with small intestinal damage due to chemotherapeutic agents such as, but not limited to 5-FU, altretamine, bleomycin, busulfan, capecitabine, carboplatin, carmustine, chlorambucil, cisplatin, cladribine, crisantaspase, cyclophosphamide, cytarabine, dacarbazine, dactinomycin, daunorubicin, docetaxel, doxorubicin, epirubicin, etoposide, fludarabine, fluorouracil, gemcitabine, hydroxycarbamide, idarubicin, ifosfamide, irinotecan, liposomal doxorubicin, leucovorin, lomustine, melphalan, mercaptopurine, mesna, methotrexate, mitomycin, mitoxantrone, oxaliplatin, paclitaxel, pemetrexed, pentostatin, procarbazine, raltitrexed, streptozocin, tegafur-uracil, temozolomide, thiotepa, tioguanine, thioguanine, topotecan, treosulfan, vinblastine, vincristine, vindesine, and vinorelbine.

Prior to administering treatment by the described methods, a diagnosis of a gastrointestinal condition may be obtained. A gastrointestinal condition can be diagnosed by standard of care means known in the art. Ulcers, for example, may be diagnosed by barium x-ray of the esophagus, stomach, and intestine, by endoscopy, or by blood, breath, and stomach tissue biopsy (e.g., to detect the presence of *Helicobacter pylori*). Malabsorption syndromes can be diagnosed by blood tests or stool tests that monitor nutrient levels in the blood or levels of fat in stool that are diagnostic of a malabsorption syndrome. Celiac sprue can be diagnosed by antibody tests which may include testing for antiendomysial antibody (IgA), antitransglutaminase (IgA), antigliadin (IgA and IgG), and total serum IgA. Endoscopy or small bowel biopsy can be used to detect abnormal intestinal lining where symptoms such as flattening of the villi, which are diagnostic of celiac sprue. Tropical sprue can be diagnosed by detecting malabsorption or infection using small bowel biopsy or response to chemotherapy. Inflammatory bowel disease can be detected by colonoscopy or by an x-ray following a barium enema in combination with clinical symptoms, where inflammation, bleeding, or ulcers on the colon wall are diagnostic of inflammatory bowel diseases such as ulcerative colitis or Crohn's disease.

In some embodiments of the method of treatment, administration of the GLP2-XTEN to a subject results in an improvement in one or more of the biochemical, physiologic, or clinical parameters that is of greater magnitude than that of the corresponding GLP-2 component not linked to the XTEN, determined using the same assay or based on a measured clinical parameter. In one embodiment of the foregoing, the administration of a therapeutically effective amount of a GLP2-XTEN composition to a subject in need thereof results in a greater reduction of parenteral nutrition (PN) dependence in patients with adult short bowel syndrome (SBS) of about 10%, or about 20%, or about 30%, or about 40%, or about 50%, or about 60%, or about 70%, or more in the subject at 2-7 days after administration compared to a comparable amount of the corresponding GLP-2 not linked to the XTEN. In another embodiment, the administration of a GLP2-XTEN to a subject in need thereof using a therapeutically effective dose regimen results in an increase of body weight of 10%, or about 20%, or about 30%, or about 40%, or about 50% or more in the subject at 7, 10, 14, 21 or 30 days after initiation of administration compared to a comparable therapeutically effective dose regimen of the corresponding GLP-2 not linked to the XTEN. In another embodiment, the administration of a therapeutically effective amount of a GLP2-XTEN composition to a subject in need thereof results in a greater reduction in fecal wet weight in patients with adult short bowel syndrome (SBS) of about 10%, or about 20%, or about 30%, or about 40%, or about 50%, or about 60%, or about 70%, or more in the subject at 2-7 days after administration compared to a comparable amount of the corresponding GLP-2 not linked to the XTEN. In another embodiment, the administration of a therapeutically effective amount of a GLP2-XTEN composition to a subject in need thereof results in a greater reduction in sodium loss in patients with adult short bowel syndrome (SBS) of about 10%, or about 20%, or about 30%, or about 40%, or about 50%, or about 60%, or about 70%, or more in the subject at 2-7 days after administration compared to a comparable amount of the corresponding GLP-2 not linked to the XTEN.

In some embodiments of the method of treatment, (i) a smaller amount of moles of about two-fold less, or about three-fold less, or about four-fold less, or about five-fold less, or about six-fold less, or about eight-fold less, or about 10-fold less of the GLP2-XTEN fusion protein is administered to a subject in need thereof in comparison to the corresponding GLP-2 not linked to the XTEN under an otherwise same dose regimen, and the fusion protein achieves a comparable area under the curve and/or a comparable therapeutic effect as the corresponding GLP-2 not linked to the XTEN; (ii) the GLP2-XTEN fusion protein is administered less frequently (e.g., every three days, about every seven days, about every 10 days, about every 14 days, about every 21 days, or about monthly) in comparison to the corresponding GLP-2 not linked to the XTEN under an otherwise same dose amount, and the fusion protein achieves a comparable area under the curve and/or a comparable therapeutic effect as the corresponding GLP-2 not linked to the XTEN; or (iii) an accumulative smaller amount of moles of at least about 20%, or about 30%, or about 40%, or about 50%, or about 60%, or about 70%, or about 80%, or about 90% less of the fusion protein is administered in comparison to the corresponding GLP-2 not linked to the XTEN under an otherwise same dose regimen and the GLP2-XTEN fusion protein achieves a comparable area under the curve and/or a comparable therapeutic effect as the corresponding GLP-2 not linked to the XTEN. The accumulative smaller amount is measured for a period of at least about one week, or about 14 days, or about 21 days, or about one month. In the foregoing embodiments of the method of treatment, the therapeutic effect can be determined by any of the measured parameters described herein, including but not limited to blood concentrations of GLP-2, assays of Table 32, or assays to detect reduced mesenteric blood flow, bleeding, inflammation, colitis, diarrhea, fecal wet weight, weight loss, sodium loss, intestinal ulcers, intestinal obstruction, fistulae, and abscesses, changed frequency in bowel movements, uveitis, growth failure in children, or maintaining blood concentrations of GLP-2 above a threshold level, e.g., 100 ng/ml of GLP-2 equivalent (or approximately 2200 ng/ml of GLP-2-2G_XTEN_AE864), among others known in the art for GLP-2-related conditions.

The invention provides GLP2-XTEN fusion proteins for use in a pharmaceutical regimen for treating a subject with a gastrointestinal condition. In one embodiment, the regimen comprises a pharmaceutical composition comprising a GLP2-XTEN fusion protein described herein. In another embodiment, the pharmaceutical regimen further comprises the step of determining the amount of pharmaceutical composition needed to achieve a therapeutic effect in the subject. In another embodiment, the pharmaceutical regimen for treating a subject with a gastrointestinal condition comprises administering the pharmaceutical composition in two or more successive doses to the subject at an effective amount, wherein the administration results in at least a 5%, or 10%, or 20%, or 30%, or 40%, or 50%, or 60%, or 70%, or 80%, or 90% greater improvement of at least one, two, or three parameters associated with the gastrointestinal condition compared to the GLP-2 not linked to XTEN and administered using a comparable nmol/kg amount. In another embodiment of the pharmaceutical regiment, the effective amount is at least about 5, or least about 10, or least about 25, or least about 100, or least about 200 nmoles/kg, or any amount intermediate to the foregoing. In another embodiment, the pharmaceutical regimen for treating a subject with a gastrointestinal condition comprises administering a therapeutically effective amount of the pharmaceutical composition once about every 3, 6, 7, 10, 14, 21, 28 or more days. In another embodiment, the pharmaceutical regimen for treating a subject with a gastrointestinal condition comprises administering the GLP2-XTEN pharmaceutical composition wherein said administration is subcutaneous, intramuscular, or intravenous. In another embodiment, the pharmaceutical regimen for treating a subject with a gastrointestinal condition comprises administering a therapeutically effective amount of the pharmaceutical composition, wherein the therapeutically effective amount results in maintaining blood concentrations of the fusion protein within a therapeutic window for the fusion protein at least three-fold longer compared to the corresponding GLP-2 not linked to the XTEN administered at a comparable amount to the subject.

The invention further contemplates that the GLP2-XTEN used in accordance with the methods provided herein can be administered in conjunction with other treatment methods and compositions (e.g., anti-inflammatory agents such as steroids or NSAIDS) useful for treating GLP-2-related conditions, or conditions for which GLP-2 is or could be adjunctive therapy.

In another aspect, the invention provides GLP2-XTEN fusion proteins for use in a method of preparing a medicament for treatment of a GLP-2-related condition In one embodiment, the method of preparing a medicament comprises linking a GLP-2 sequence with at least about 80%, or at least about 90%, or at least about 95%, or at least about 97%, or at least about 99% sequence identity to a GLP-2 of Table 1 to at least a first XTEN with at least about 80%, or at least about 90%, or at least about 95%, or at least about 97%, or at least about 99% sequence identity to a XTEN selected from any one of Tables 4, and 8-12, wherein the GLP2-XTEN retains at least a portion of the biological activity of the native GLP-2, and further combining the GLP2-XTEN with at least one pharmaceutically acceptable carrier. In another embodiment, the GLP2-XTEN has a sequence with at least about 80%, or at least about 90%, or at least about 95%, or at least about 97%, or at least about 99% sequence identity compared to a sequence selected from any one of Tables 13, 32 or 33.

In another aspect, the invention provides a method of designing the GLP2-XTEN compositions to achieve desired pharmacokinetic, pharmacologic or pharmaceutical properties. In general, the steps in the design and production of the fusion proteins and the inventive compositions, as illustrated in FIGS. 4-6, include: (1) selecting a GLP-2 (e.g., native proteins, sequences of Table 1, analogs or derivatives with activity) to treat the particular condition; (2) selecting the XTEN that will confer the desired PK and physicochemical characteristics on the resulting GLP2-XTEN (e.g., the administration of the GLP2-XTEN composition to a subject results in the fusion protein being maintained within the therapeutic window for a greater period compared to GLP-2 not linked to the XTEN); (3) establishing a desired N- to C-terminus configuration of the GLP2-XTEN to achieve the desired efficacy or PK parameters; (4) establishing the design of the expression vector encoding the configured GLP2-XTEN; (5) transforming a suitable host with the expression vector; and (6) expressing and recovering of the resultant fusion protein. For those GLP2-XTEN for which an increase in half-life or an increased period of time spent above the minimum effective concentration is desired, the XTEN chosen for incorporation generally has at least about 288, or about 432, or about 576, or about 864, or about 875, or about 912, or about 923 amino acid residues where a single XTEN is to be incorporated into the GLP2-XTEN. In another embodiment, the GLP2-XTEN comprises a first XTEN of the foregoing lengths, and at least a second XTEN of about 36, or about 72, or about 144, or about 288, or about 576, or about 864, or about 875, or about 912, or about 923, or about 1000 or more amino acid residues.

In another aspect, the invention provides methods of making GLP2-XTEN compositions to improve ease of manufacture, result in increased stability, increased water solubility, and/or ease of formulation, as compared to the native GLP-2. In one embodiment, the invention includes a method of increasing the water solubility of a GLP-2 comprising the step of linking the GLP-2 to one or more XTEN such that a higher concentration in soluble form of the resulting GLP2-XTEN can be achieved, under physiologic conditions, compared to the GLP-2 in an un-fused state. In some embodiments, the method results in a GLP2-XTEN fusion protein wherein the water solubility is at least about 20%, or at least about 30% greater, or at least about 50% greater, or at least about 75% greater, or at least about 90% greater, or at least about 100% greater, or at least about 150% greater, or at least about 200% greater, or at least about 400% greater, or at least about 600% greater, or at least about 800% greater, or at least about 1000% greater, or at least about 2000% greater under physiologic conditions, compared to the un-fused GLP-2. Factors that contribute to the property of XTEN to confer increased water solubility of GLP-2 when incorporated into a fusion protein include the high solubility of the XTEN fusion partner and the low degree of self-aggregation between molecules of XTEN in solution. In one embodiment of the foregoing, the GLP2-XTEN comprises a GLP-2 linked to an XTEN having at least about 36, or about 48, or about 96, or about 144, or about 288, or about 576, or about 864 amino acid residues in which the solubility of the fusion protein under physiologic conditions is at least three-fold greater than the corresponding GLP-2 not linked to the XTEN, or alternatively, at least four-fold, or five-fold, or six-fold, or seven-fold, or eight-fold, or nine-fold, or at least 10-fold, or at least 20-fold, or at least 30-fold, or at least 50-fold, or at least 60-fold or greater than GLP-2 not linked to the XTEN. In one embodiment of the foregoing, the GLP-2 has at least about 80%, or at least about 90%, or at least about 95%, or at least about 97%, or at least about 99% sequence identity to a GLP-2 of Table 1 linked to at least an XTEN with at least about 80%, or at least about 90%, or at least about 95%, or at least about 97%, or at least about 99% sequence identity to a XTEN selected from any one of Tables 4, and 8-12.

In another embodiment, the invention includes a method of increasing the shelf-life of a GLP-2 comprising the step of linking the GLP-2 with one or more XTEN selected such that the shelf-life of the resulting GLP2-XTEN is extended compared to the GLP-2 in an un-fused state. As used herein, shelf-life refers to the period of time over which the functional activity of a GLP-2 or GLP2-XTEN that is in solution or in some other storage formulation remains stable without undue loss of activity. As used herein, "functional activity" refers to a pharmacologic effect or biological activity, such as the ability to bind a receptor or ligand, or substrate, or trigger an up-regulated activity, or to display one or more known functional activities associated with a GLP-2, as known in the art. A GLP-2 that degrades or aggregates generally has reduced functional activity or reduced bioavailability compared to one that remains in solution. Factors that contribute to the ability of the method to extend the shelf life of GLP-2s when incorporated into a fusion protein include increased water solubility, reduced self-aggregation in solution, and increased heat stability of the XTEN fusion partner. In particular, the low tendency of XTEN to aggregate facilitates methods of formulating pharmaceutical preparations containing higher drug concentrations of GLP-2s, and the heat-stability of XTEN contributes to the property of GLP2-XTEN fusion proteins to remain soluble and functionally active for extended periods. In one embodiment, the method results in GLP2-XTEN fusion proteins with "prolonged" or "extended" shelf-life that exhibit greater activity relative to a standard that has been subjected to the same storage and handling conditions. The standard may be the un-fused full-length GLP-2. In one embodiment, the method includes the step of formulating the isolated GLP2-XTEN with one or more pharmaceutically acceptable excipients that enhance the ability of the XTEN to retain its unstructured conformation and for the GLP2-XTEN to remain soluble in the formulation for a time that is greater than that of the corresponding un-fused GLP-2. In one embodiment, the method comprises linking a GLP-2 to one or more XTEN selected from Table 4 to create a GLP2-XTEN fusion protein results in a solution that retains greater than about 100% of the functional activity, or greater than about 105%, 110%, 120%, 130%, 150% or 200% of the functional activity of a standard when compared at a given time point and when subjected to the same storage and handling conditions as the standard, thereby increasing its shelf-life.

Shelf-life may also be assessed in terms of functional activity remaining after storage, normalized to functional activity when storage began. GLP2-XTEN fusion proteins of the invention with prolonged or extended shelf-life as exhibited by prolonged or extended functional activity retain about 50% more functional activity, or about 60%, 70%, 80%, or 90% more of the functional activity of the equivalent GLP-2 not linked to the XTEN when subjected to the same conditions for the same period of time. For example, a GLP2-XTEN fusion protein of the invention comprising GLP-2 fused to one or more XTEN sequences selected from Table 4 retains about 80% or more of its original activity in solution for periods of up to 2 weeks, or 4 weeks, or 6 weeks, or 12 weeks or longer under various elevated temperature conditions. In some embodiments, the GLP2-XTEN retains at least about 50%, or about 60%, or at least about 70%, or at least about 80%, and most preferably at least about 90% or more of its original activity in solution when heated at 80°C for 10 min. In other embodiments, the GLP2-XTEN retains at least about 50%, preferably at least about 60%, or at least about 70%, or at least about 80%, or alternatively at least about 90% or more of its original activity in solution when heated or maintained at 37°C for about 7 days. In another embodiment, GLP2-XTEN fusion protein retains at least about 80% or more of its functional activity after exposure to a temperature of about 30°C to about 70°C over a period of time of about one hour to about 18 hours. In the foregoing embodiments hereinabove described in this paragraph, the retained activity of the GLP2-XTEN is at least about two-fold, or at least about three-fold, or at least about four-fold, or at least about five-fold, or at least about six-fold greater at a given time point than that of the corresponding GLP-2 not linked to the XTEN.

## VII). THE NUCLEIC ACIDS SEQUENCES OF THE INVENTION

The present invention provides isolated polynucleic acids encoding GLP2-XTEN chimeric fusion proteins and sequences complementary to polynucleic acid molecules encoding GLP2-XTEN chimeric fusion proteins, including homologous variants thereof. In another aspect, the invention encompasses methods to produce polynucleic acids encoding GLP2-XTEN chimeric fusion proteins and sequences complementary to polynucleic acid molecules encoding GLP2-XTEN chimeric fusion protein, including homologous variants thereof. In general, and as illustrated in FIGS. 4-6, the methods of producing a polynucleotide sequence coding for a GLP2-XTEN fusion protein and expressing the resulting gene product include assembling nucleotides encoding GLP-2 and XTEN, ligating the components in frame, incorporating the encoding gene into an expression vector appropriate for a host cell, transforming the appropriate host cell with the expression vector, and culturing the host cell under conditions causing or permitting the fusion protein to be expressed in the transformed host cell, thereby producing the biologically-active GLP2-XTEN polypeptide, which is recovered as an isolated fusion protein by standard protein purification methods known in the art. Standard recombinant techniques in molecular biology are used to make the polynucleotides and expression vectors of the present invention.

In accordance with the invention, nucleic acid sequences that encode GLP2-XTEN (or its complement) are used to generate recombinant DNA molecules that direct the expression of GLP2-XTEN fusion proteins in appropriate host cells. Several cloning strategies are suitable for performing the present invention, many of which is used to generate a construct that comprises a gene coding for a fusion protein of the GLP2-XTEN composition of the present invention, or its complement. In some embodiments, the cloning strategy is used to create a gene that encodes a monomeric GLP2-XTEN that comprises at least a first GLP-2 and at least a first XTEN polypeptide, or their complement. In one embodiment of the foregoing, the gene comprises a sequence encoding a GLP-2 or sequence variant. In other embodiments, the cloning strategy is used to create a gene that encodes a monomeric GLP2-XTEN that comprises nucleotides encoding at least a first molecule of GLP-2 or its complement and a first and at least a second XTEN or their complement that is used to transform a host cell for expression of the fusion protein of the GLP2-XTEN composition. In the foregoing embodiments hereinabove described in this paragraph, the genes can further comprise nucleotides encoding spacer sequences that also encode cleavage sequence(s).

In designing a desired XTEN sequences, it was discovered that the non-repetitive nature of the XTEN of the inventive compositions is achieved despite use of a "building block" molecular approach in the creation of the XTEN-encoding sequences. This was achieved by the use of a library of polynucleotides encoding peptide sequence motifs, described above, that are then ligated and/or multimerized to create the genes encoding the XTEN sequences (see FIGS. 4, 5, 8, 9 and Examples). Thus, while the XTEN(s) of the expressed fusion protein may consist of multiple units of as few as four different sequence motifs, because the motifs themselves consist of non-repetitive amino acid sequences, the overall XTEN sequence is rendered non-repetitive. Accordingly, in one embodiment, the XTEN-encoding polynucleotides comprise multiple polynucleotides that encode non-repetitive sequences, or motifs, operably linked in frame and in which the resulting expressed XTEN amino acid sequences are non-repetitive.

In one approach, a construct is first prepared containing the DNA sequence corresponding to GLP2-XTEN fusion protein. In those embodiments in which a mammalian native GLP-2 sequence is to be employed in the fusion protein, DNA encoding the GLP-2 of the compositions is obtained from a cDNA library prepared using standard methods from tissue or isolated cells believed to possess GLP-2 mRNA and to express it at a detectable level. Libraries are screened with probes containing, for example, about 20 to 100 bases designed to identify the GLP-2 gene of interest by hybridization using conventional molecular biology techniques. The best candidates for probes are those that represent sequences that are highly homologous for GLP-2, and should be of sufficient length and sufficiently unambiguous that false positives are minimized, but may be degenerate at one or more positions. If necessary, the coding sequence can be obtained using conventional primer extension procedures as described in Sambrook, *et al., supra,* to detect precursors and processing intermediates of mRNA that may not have been reverse-transcribed into cDNA. One can then use polymerase chain reaction (PCR) methodology to amplify the target DNA or RNA coding sequence to obtain sufficient material for the preparation of the GLP2-XTEN constructs containing the GLP-2 gene. Assays can then be conducted to confirm that the hybridizing full-length genes are the desired GLP-2 gene(s). By these conventional methods, DNA can be conveniently obtained from a cDNA library prepared from such sources. In those embodiments in which a GLP-2 analog (with one or more amino acid substitutions, such as sequences of Table 1) for the preparation of the GLP2-XTEN constructs, the GLP-2 encoding gene(s) is created by standard synthetic procedures known in the art (e.g., automated nucleic acid synthesis using, for example one of the methods described in Engels et al. (Agnew. Chem. Int. Ed. Engl., 28:716-734 1989)), using DNA sequences obtained from publicly available databases, patents, or literature references. Such procedures are well known in the art and well described in the scientific and patent literature. For example, sequences can be obtained from Chemical Abstracts Services (CAS) Registry Numbers (published by the American Chemical Society) and/or GenBank Accession Numbers (e.g., Locus ID, NP_XXXXX, and XP_XXXXX) Model Protein identifiers available through the National Center for Biotechnology Information (NCBI) webpage, available on the world wide web at ncbi.nlm.nih.gov that correspond to entries in the CAS Registry or GenBank database that contain an amino acid sequence of the protein of interest or of a fragment or variant of the protein. For such sequence identifiers provided herein, the summary pages associated with each of these CAS and GenBank and GenSeq Accession Numbers as well as the cited journal publications (e.g., PubMed ID number (PMID)) are each incorporated by reference in their entireties, particularly with respect to the amino acid sequences described therein. In one embodiment, the GLP-2 encoding gene encodes a protein from any one of Table 1, or a fragment or variant thereof.

A gene or polynucleotide encoding the GLP-2 portion of the subject GLP2-XTEN protein, in the case of an expressed fusion protein that comprises a single GLP-2 is then be cloned into a construct, which is a plasmid or other vector under the control of appropriate transcription and translation sequences for high level protein expression in a biological system. In a later step, a second gene or polynucleotide coding for the XTEN is genetically fused to the nucleotides encoding the N- and/or C-terminus of the GLP-2 gene by cloning it into the construct adjacent and in frame with the gene(s) coding for the GLP-2. This second step occurs through a ligation or multimerization step. In the foregoing embodiments hereinabove described in this paragraph, it is to be understood that the gene constructs that are created can alternatively be the complement of the respective genes that encode the respective fusion proteins.

The gene encoding for the XTEN can be made in one or more steps, either fully synthetically or by synthesis combined with enzymatic processes, such as restriction enzyme-mediated cloning, PCR and overlap extension, including methods more fully described in the Examples. The methods disclosed herein can be used, for example, to ligate short sequences of polynucleotides encoding XTEN into longer XTEN genes of a desired length and sequence. In one embodiment, the method ligates two or more codon-optimized oligonucleotides encoding XTEN motif or segment sequences of about 9 to 14 amino acids, or about 12 to 20 amino acids, or about 18 to 36 amino acids, or about 48 to about 144 amino acids, or about 144 to about 288 or longer, or any combination of the foregoing ranges of motif or segment lengths.

Alternatively, the disclosed method is used to multimerize XTEN-encoding sequences into longer sequences of a desired length; e.g., a gene encoding 36 amino acids of XTEN can be dimerized into a gene encoding 72 amino acids, then 144, then 288, etc. Even with multimerization, XTEN polypeptides can be constructed such that the XTEN-encoding gene has low or virtually no repetitiveness through design of the codons selected for the motifs of the shortest unit being used, which can reduce recombination and increase stability of the encoding gene in the transformed host.

Genes encoding XTEN with non-repetitive sequences are assembled from oligonucleotides using standard techniques of gene synthesis. The gene design can be performed using algorithms that optimize codon usage and amino acid composition. In one method of the invention, a library of relatively short XTEN-encoding polynucleotide constructs is created and then assembled, as described above. The resulting genes are then assembled with genes encoding GLP-2 or regions of GLP-2, as illustrated in FIGS. 5 and 8, and the resulting genes used to transform a host cell and produce and recover the GLP2-XTEN for evaluation of its properties, as described herein.

In some embodiments, the GLP2-XTEN sequence is designed for optimized expression by inclusion of an N-terminal sequence (NTS) XTEN, rather than using a leader sequence known in the art. In one embodiment, the NTS is created by inclusion of encoding nucleotides in the XTEN gene determined to result in optimized expression when joined to the gene encoding the fusion protein. In one embodiment, the N-terminal XTEN sequence of the expressed GLP2-XTEN is optimized for expression in a eukaryotic cell, such as but not limited to CHO, HEK, yeast, and other cell types know in the art.

Polynucleotide libraries

In another aspect, the invention provides libraries of polynucleotides that encode XTEN sequences that are used to assemble genes that encode XTEN of a desired length and sequence.

In certain embodiments, the XTEN-encoding library constructs comprise polynucleotides that encode polypeptide segments of a fixed length. As an initial step, a library of oligonucleotides that encode motifs of 9-14 amino acid residues can be assembled. In a preferred embodiment, libraries of oligonucleotides that encode motifs of 12 amino acids are assembled.

The XTEN-encoding sequence segments can be dimerized or multimerized into longer encoding sequences. Dimerization or multimerization can be performed by ligation, overlap extension, PCR assembly or similar cloning techniques known in the art. This process of can be repeated multiple times until the resulting XTEN-encoding sequences have reached the organization of sequence and desired length, providing the XTEN-encoding genes. As will be appreciated, a library of polynucleotides that encodes, e.g., 12 amino acid motifs can be dimerized and/or ligated into a library of polynucleotides that encode 36 amino acids. Libraries encoding motifs of different lengths; e.g., 9-14 amino acid motifs leading to libraries encoding 27 to 42 amino acids are contemplated by the invention. In turn, the library of polynucleotides that encode 27 to 42 amino acids, and preferably 36 amino acids (as described in the Examples) can be serially dimerized into a library containing successively longer lengths of polynucleotides that encode XTEN sequences of a desired length for incorporation into the gene encoding the GLP2-XTEN fusion protein, as disclosed herein.

A more efficient way to optimize the DNA sequence encoding XTEN is based on combinatorial libraries. The gene encoding XTEN can be designed and synthesized in segment such that multiple codon versions are obtained for each segment. These segments can be randomly assembled into a library of genes such that each library member encodes the same amino acid sequences but library members comprise a large number of codon versions. Such libraries can be screened for genes that result in high-level expression and/or a low abundance of truncation products. The process of combinatorial gene assembly is illustrated in FIG. 10. The genes in FIG. 10 are assembled from 6 base fragments and each fragment is available in 4 different codon versions. This allows for a theoretical diversity of 4096.

In some embodiments, libraries are assembled of polynucleotides that encode amino acids that are limited to specific sequence XTEN families; e.g., AD, AE, AF, AG, AM, or AQ sequences of Table 3. In other embodiments, libraries comprise sequences that encode two or more of the motif family sequences from Table 3. The names and sequences of representative, non-limiting polynucleotide sequences of libraries that encode 36mers are presented in Tables 8-11, and the methods used to create them are described more fully in the respective Examples. In other embodiments, libraries that encode XTEN are constructed from segments of polynucleotide codons linked in a randomized sequence that encode amino acids wherein at least about 80%, or at least about 90%, or at least about 91%, or at least about 92%, or at least about 93%, or at least about 94%, or at least about 95%, or at least about 97%, or at least about 98%, or at least about 99% of the codons are selected from the group consisting of condons for glycine (G), alanine (A), serine (S), threonine (T), glutamate (E) and proline (P) amino acids. The libraries can be used, in turn, for serial dimerization or ligation to achieve polynucleotide sequence libraries that encode XTEN sequences, for example, of 48, 72, 144, 288, 576, 864, 875, 912, 923, 1318 amino acids, or up to a total length of about 3000 amino acids, as well as intermediate lengths, in which the encoded XTEN can have one or more of the properties disclosed herein, when expressed as a component of a GLP2-XTEN fusion protein. In some cases, the polynucleotide library sequences may also include additional bases used as "sequencing islands," described more fully below.

FIG. 5 is a schematic flowchart of representative, non-limiting steps in the assembly of an XTEN polynucleotide construct and a GLP2-XTEN polynucleotide construct in the embodiments of the invention. Individual oligonucleotides **501** are annealed into sequence motifs **502** such as a 12 amino acid motif ("12-mer"), which is ligated to additional sequence motifs from a library to create a pool that encompasses the desired length of the XTEN **504,** as well as ligated to a smaller concentration of an oligo containing BbsI, and KpnI restriction sites **503**. The resulting pool of ligation products is gel-purified and the band with the desired length of XTEN is cut, resulting in an isolated XTEN gene with a stopper sequence **505**. The XTEN gene is cloned into a stuffer vector. In this case, the vector encodes an optional CBD sequence **506** and a GFP gene **508**. Digestion is than performed with BbsI/HindIII to remove **507** and **508** and place the stop codon. The resulting product is then cloned into a BsaI/HindIII digested vector containing a gene encoding the GLP-2, resulting in the gene **500** encoding a GLP2-XTEN fusion protein. A non-exhaustive list of the polynucleotides encoding XTEN and precursor sequences is provided in Tables 7-12.

**Table 7: DNA sequences of XTEN and precursor sequences**

| XTEN Name | DNA Nucleotide Sequence |
|---|---|
| AE48 | ATGGCTGAACCTGCTGGCTCTCCAACCTCCACTGAGGAAGGTACCCCGGGTAGCGGTACTG CTTCTTCCTCTCCAGGTAGCTCTACCCCTTCTGGTGCAACCGGCTCTCCAGGTGCTTCTCCG GGCACCAGCTCTACCGGTTCT |
| AM48 | ATGGCTGAACCTGCTGGCTCTCCAACCTCCACTGAGGAAGGTGCATCCCCGGGCACCAGCT CTACCGGTTCTCCAGGTAGCTCTACCCCGTCTGGTGCTACCGGCTCTCCAGGTAGCTCTACC CCGTCTGGTGCTACTGGCTCT |

(continued)

| XTEN Name | DNA Nucleotide Sequence |
|---|---|
| AE144 | GGTAGCGAACCGGCAACTTCCGGCTCTGAAACCCCAGGTACTTCTGAAAGCGCTACTCCTGAGTCTGGCCCAGGTAGCGAACCTGCTACCTCTGGCTCTGAAACCCCAGGTAGCCCGGCAGGCTCTCCGACTTCCACCGAGGAAGGTACCTCTACTGAACCTTCTGAGGGTAGCGCTCCAGGTAGCGAACCGGCAACCTCTGGCTCTGAAACCCCAGGTAGCGAACCTGCTACCTCCGGCTCTGAAACTCCAGGTAGCGAACCGGCTACTTCCGGTTCTGAAACTCCAGGTACCTCTACCGAACCTTCCGAAGGCAGCGCACCAGGTACTTCTGAAAGCGCAACCCCTGAATCCGGTCCAGGTAGCGAACCGGCTACTTCTGGCTCTGAGACTCCAGGTACTTCTACCGAACCGTCCGAAGGTAGCGCACCA |
| AF144 | GGTACTTCTACTCCGGAAAGCGGTTCCGCATCTCCAGGTACTTCTCCTAGCGGTGAATCTTCTACTGCTCCAGGTACCTCTCCTAGCGGCGAATCTTCTACTGCTCCAGGTTCTACCAGCTCTACCGCTGAATCTCCTGGCCCAGGTTCTACCAGCGAATCCCCGTCTGGCACCGCACCAGGTTCTACTAGCTCTACCGCAGAATCTCCGGGTCCAGGTACTTCCCCTAGCGGTGAATCTTCTACTGCTCCAGGTACCTCTACTCCGGAAAGCGGCTCCGCATCTCCAGGTTCTACTAGCTCTACTGCTGAATCTCCTGGTCCAGGTACCTCCCCTAGCGGCGAATCTTCTACTGCTCCAGGTACCTCTCCTAGCGGCGAATCTTCTACCGCTCCAGGTACCTCCCCTAGCGGTGAATCTTCTACCGCACCA |
| AE288 | GGTACCTCTGAAAGCGCAACTCCTGAGTCTGGCCCAGGTAGCGAACCTGCTACCTCCGGCTCTGAGACTCCAGGTACCTCTGAAAGCGCAACCCCGGAATCTGGTCCAGGTAGCGAACCTGCAACCTCTGGCTCTGAAACCCCAGGTACCTCTGAAAGCGCTACTCCTGAATCTGGCCCAGGTACTTCTACTGAACCGTCCGAGGGCAGCGCACCAGGTAGCCCTGCTGGCTCTCCAACCTCCACCGAAGAAGGTACCTCTGAAAGCGCAACCCCTGAATCCGGCCCAGGTAGCGAACCGGCAACCTCCGGTTCTGAAACCCCAGGTACTTCTGAAAGCGCTACTCCTGAGTCCGGCCCAGGTAGCCCGGCTGGCTCTCCGACTTCCACCGAGGAAGGTAGCCCGGCTGGCTCTCCAACTTCTACTGAAGAAGGTACTTCTACCGAACCTTCCGAGGGCAGCGCACCAGGTACTTCTGAAAGCGCTACCCCTGAGTCCGGCCCAGGTACTTCTGAAAGCGCTACTCCTGAATCCGGTCCAGGTACTTCTGAAAGCGCTACCCCGGAATCTGGCCCAGGTAGCGAACCGGCTACTTCTGGTTCTGAAACCCCAGGTAGCGAACCGGCTACCTCCGGTTCTGAAACTCCAGGTAGCCCAGCAGGCTCTCCGACTTCCACTGAGGAAGGTACTTCTACTGAACCTTCCGAAGGCAGCGCACCAGGTACCTCTACTGAACCTTCTGAGGGCAGCGCTCCAGGTAGCGAACCTGCAACCTCTGGCTCTGAAACCCCAGGTACCTCTGAAAGCGCTACTCCTGAATCTGGCCCAGGTACTTCTACTGAACCGTCCGAGGGCAGCGCACCA |
| AE576 | GGTAGCCCGGCTGGCTCTCCTACCTCTACTGAGGAAGGTACTTCTGAAAGCGCTACTCCTGAGTCTGGTCCAGGTACCTCTACTGAACCGTCCGAAGGTAGCGCTCCAGGTAGCCCAGCAGGCTCTCCGACTTCCACTGAGGAAGGTACTTCTACTGAACCTTCCGAAGGCAGCGCACCAGGTACCTCTACTGAACCTTCTGAGGGCAGCGCTCCAGGTACTTCTGAAAGCGCTACCCCGGAATCTGGCCCAGGTAGCGAACCGGCTACTTCTGGTTCTGAAACCCCAGGTAGCGAACCGGCTACCTCCGGTTCTGAAACTCCAGGTAGCCCGGCAGGCTCTCCGACCTCTACTGAGGAAGGTACTTCTGAAAGCGCAACCCCGGAGTCCGGCCCAGGTACCTCTACCGAACCGTCTGAGGGCAGCGCACCAGGTACTTCTACCGAACCGTCCGAGGGTAGCGCACCAGGTAGCCCAGCAGGTTCTCCTACCTCCACCGAGGAAGGTACTTCTACCGAACCGTCCGAGGGTAGCGCACCAGGTACCTCTACTGAACCTTCTGAGGGCAGCGCTCCAGGTACTTCTGAAAGCGCTACCCCGGAGTCCGGTCCAGGTACTTCTACTGAACCGTCCGAAGGTAGCGCACCAGGTACTTCTGAAAGCGCAACCCCTGAATCCGGTCCAGGTAGCGAACCGGCTACTTCTGGCTCTGAGACTCCAGGTACTTCTACCGAACCGTCCGAAGGTAGCGCACCAGGTACTTCTACTGAACCGTCTGAAGGTAGCGCACCAGGTACTTCTGAAAGCGCAACCCCGGAATCCGGCCCAGGTACCTCTGAAAGCGCAACCCCGGAGTCCGGCCCAGGTAGCCCTGCTGGCTCTCCAACCTCCACCGAAGAAGGTACCTCTGAAAGCGCAACCCCTGAATCCGGCCCAGGTAGCGAACCGGCAACCTCCGGTTCTGAAAC |

(continued)

| XTEN Name | DNA Nucleotide Sequence |
|---|---|
| | CCCAGGTACCTCTGAAAGCGCTACTCCGGAGTCTGGCCCAGGTACCTCTACTGAACCGTCTGAGGGTAGCGCTCCAGGTACTTCTACTGAACCGTCCGAAGGTAGCGCACCAGGTACTTCTACCGAACCGTCCGAAGGCAGCGCTCCAGGTACCTCTACTGAACCTTCCGAGGGCAGCGCTCCAGGTACCTCTACCGAACCTTCTGAAGGTAGCGCACCAGGTACTTCTACCGAACCGTCCGAGGGTAGCGCACCAGGTAGCCCAGCAGGTTCTCCTACCTCCACCGAGGAAGGTACTTCTACCGAACCGTCCGAGGGTAGCGCACCAGGTACCTCTGAAAGCGCAACTCCTGAGTCTGGCCCAGGTAGCGAACCTGCTACCTCCGGCTCTGAGACTCCAGGTACCTCTGAAAGCGCAACCCCGGAATCTGGTCCAGGTAGCGAACCTGCAACCTCTGGCTCTGAAACCCCAGGTACCTCTGAAAGCGCTACTCCTGAATCTGGCCCAGGTACTTCTACTGAACCGTCCGAGGGCAGCGCACCAGGTACTTCTGAAAGCGCTACTCCTGAGTCCGGCCCAGGTAGCCCGGCTGGCTCTCCGACTTCCACCGAGGAAGGTAGCCCGGCTGGCTCTCCAACTTCTACTGAAGAAGGTAGCCCGGCAGGCTCTCCGACCTCTACTGAGGAAGGTACTTCTGAAAGCGCAACCCCGGAGTCCGGCCCAGGTACCTCTACCGAACCGTCTGAGGGCAGCGCACCA |
| AF576 | GGTTCTACTAGCTCTACCGCTGAATCTCCTGGCCCAGGTTCCACTAGCTCTACCGCAGAATCTCCGGGCCCAGGTTCTACTAGCGAATCCCCTTCTGGTACCGCTCCAGGTTCTACTAGCTCTACCGCTGAATCTCCGGGTCCAGGTTCTACCAGCTCTACTGCAGAATCTCCTGGCCCAGGTACTTCTACTCCGGAAAGCGGTTCCGCTTCTCCAGGTTCTACCAGCGAATCTCCTTCTGGCACCGCTCCAGGTACCTCTCCTAGCGGCGAATCTTCTACCGCTCCAGGTTCTACTAGCGAATCTCCTTCTGGCACTGCACCAGGTTCTACCAGCGAATCTCCTTCTGGCACCGCTCCAGGTACCTCTCCTAGCGGCGAATCTTCTACCGCTCCAGGTTCTACTAGCGAATCTCCTTCTGGCACTGCACCAGGTTCTACCAGCGAATCTCCTTCTGGCACCGCTCCAGGTACCTCTCCTAGCGGCGAATCTTCTACCGCTCCAGGTTCTACTAGCGAATCTCCTTCTGGCACTGCACCAGGTTCTACTAGCGAATCTCCTTCTGGCACTGCACCAGGTTCTACCAGCGAATCTCCGTCTGGCACTGCACCAGGTACCTCTACCCCTGAAAGCGGTTCCGCTTCTCCAGGTTCTACTAGCGAATCTCCTTCTGGTACCGCTCCAGGTACTTCTACCCCTGAAAGCGGCTCCGCTTCTCCAGGTTCCACTAGCTCTACCGCTGAATCTCCGGGTCCAGGTTCTACTAGCTCTACTGCAGAATCTCCTGGCCCAGGTACCTCTACTCCGGAAAGCGGCTCTGCATCTCCAGGTACTTCTACCCCTGAAAGCGGTTCTGCATCTCCAGGTTCTACTAGCGAATCCCGTCTGGTACCGCACCAGGTACTTCTACCCCGGAAAGCGGCTCTGCTTCTCCAGGTACTTCTACCCCGGAAAGCGGCTCCGCATCTCCAGGTTCTACTAGCGAATCTCCTTCTGGTACCGCTCCAGGTTCTACCAGCGAATCCCCGTCTGGTACTGCTCCAGGTTCTACCAGCGAATCTCCTTCTGGTACTGCACCAGGTTCTACTAGCTCTACTGCAGAATCTCCTGGCCCAGGTACCTCTACTCCGGAAAGCGGCTCTGCATCTCCAGGTACTTCTACCCCTGAAAGCGGTTCTGCATCTCCAGGTTCTACTAGCGAATCTCCTTCTGGCACTGCACCAGGTTCTACCAGCGAATCTCCGTCTGGCACTGCACCAGGTACCTCTACCCCTGAAAGCGGTTCCGCTTCTCCAGGTTCTACTAGCGAATCTCCTTCTGGCACTGCACCAGGTTCTACCAGCGAATCTCCGTCTGGCACTGCACCAGGTACCTCTACCCCTGAAAGCGGTTCCGCTTCTCCAGGTACTTCTCCGAGCGGTGAATCTTCTACCGCACCAGGTTCTACTAGCTCTACCGCTGAATCTCCGGGCCCAGGTACTTCTCCGAGCGGTGAATCTTCTACTGCTCCAGGTTCCACTAGCTCTACTGCTGAATCTCCTGGCCCAGGTACTTCTACTCCGGAAAGCGGTTCCGCTTCTCCAGGTTCTACTAGCGAATCTCCGTCTGGCACCGCACCAGGTTCTACTAGCTCTACTGCAGAATCTCCTGGCCCAGGTACCTCTACTCCGGAAAGCGGCTCTGCATCTCCAGGTACTTCTACCCCTGAAAGCGGTTCTGCATCTCCA |

(continued)

| XTEN Name | DNA Nucleotide Sequence |
|---|---|
| AE624 | ATGGCTGAACCTGCTGGCTCTCCAACCTCCACTGAGGAAGGTACCCCGGGTAGCGGTACTGCTTCTTCCTCTCCAGGTAGCTCTACCCCTTCTGGTGCAACCGGCTCTCCAGGTGCTTCTCCGGGCACCAGCTCTACCGGTTCTCCAGGTAGCCCGGCTGGCTCTCCTACCTCTACTGAGGAAGGTACTTCTGAAAGCGCTACTCCTGAGTCTGGTCCAGGTACCTCTACTGAACCGTCCGAAGGTAGCGCTCCAGGTAGCCCAGCAGGCTCTCCGACTTCCACTGAGGAAGGTACTTCTACTGAACCTTCCGAAGGCAGCGCACCAGGTACCTCTACTGAACCTTCTGAGGGCAGCGCTCCAGGTACTTCTGAAAGCGCTACCCCGGAATCTGGCCCAGGTAGCGAACCGGCTACTTCTGGTTCTGAAACCCCAGGTAGCGAACCGGCTACCTCCGGTTCTGAAACTCCAGGTAGCCCGGCAGGCTCTCCGACCTCTACTGAGGAAGGTACTTCTGAAAGCGCAACCCCGGAGTCCGGCCCAGGTACCTCTACCGAACCGTCTGAGGGCAGCGCACCAGGTACTTCTACCGAACCGTCCGAGGGTAGCGCACCAGGTAGCCCAGCAGGTTCTCCTACCTCCACCGAGGAAGGTACTTCTACCGAACCGTCCGAGGGTAGCGCACCAGGTACCTCTACTGAACCTTCTGAGGGCAGCGCTCCAGGTACTTCTGAAAGCGCTACCCCGGAGTCCGGTCCAGGTACTTCTACTGAACCGTCCGAAGGTAGCGCACCAGGTACTTCTGAAAGCGCAACCCCTGAATCCGGTCCAGGTAGCGAACCGGCTACTTCTGGCTCTGAGACTCCAGGTACTTCTACCGAACCGTCCGAAGGTAGCGCACCAGGTACTTCTACTGAACCGTCTGAAGGTAGCGCACCAGGTACTTCTGAAAGCGCAACCCCGGAATCCGGCCCAGGTACCTCTGAAAGCGCAACCCCGGAGTCCGGCCCAGGTAGCCCTGCTGGCTCTCCA |
| | ACCTCCACCGAAGAAGGTACCTCTGAAAGCGCAACCCCTGAATCCGGCCCAGGTAGCGAACCGGCAACCTCCGGTTCTGAAACCCCAGGTACCTCTGAAAGCGCTACTCCGGAGTCTGGCCCAGGTACCTCTACTGAACCGTCTGAGGGTAGCGCTCCAGGTACTTCTACTGAACCGTCCGAAGGTAGCGCACCAGGTACTTCTACCGAACCGTCCGAAGGCAGCGCTCCAGGTACCTCTACTGAACCTTCCGAGGGCAGCGCTCCAGGTACCTCTACCGAACCTTCTGAAGGTAGCGCACCAGGTACTTCTACCGAACCGTCCGAGGGTAGCGCACCAGGTAGCCCAGCAGGTTCTCCTACCTCCACCGAGGAAGGTACTTCTACCGAACCGTCCGAGGGTAGCGCACCAGGTACCTCTGAAAGCGCAACTCCTGAGTCTGGCCCAGGTAGCGAACCTGCTACCTCCGGCTCTGAGACTCCAGGTACCTCTGAAAGCGCAACCCCGGAATCTGGTCCAGGTAGCGAACCTGCAACCTCTGGCTCTGAAACCCCAGGTACCTCTGAAAGCGCTACTCCTGAATCTGGCCCAGGTACTTCTACTGAACCGTCCGAGGGCAGCGCACCAGGTACTTCTGAAAGCGCTACTCCTGAGTCCGGCCCAGGTAGCCCGGCTGGCTCTCCGACTTCCACCGAGGAAGGTAGCCCGGCTGGCTCTCCAACTTCTACTGAAGAAGGTAGCCCGGCAGGCTCTCCGACCTCTACTGAGGAAGGTACTTCTGAAAGCGCAACCCCGGAGTCCGGCCCAGGTACCTCTACCGAACCGTCTGAGGGCAGCGCACCA |

(continued)

| XTEN Name | DNA Nucleotide Sequence |
|---|---|
| AM875 | GGTACTTCTACTGAACCGTCTGAAGGCAGCGCACCAGGTAGCGAACCGGCTACTTCCGGTT CTGAAACCCCAGGTAGCCCAGCAGGTTCTCCAACTTCTACTGAAGAAGGTTCTACCAGCTC TACCGCAGAATCTCCTGGTCCAGGTACCTCTACTCCGGAAAGCGGCTCTGCATCTCCAGGT TCTACTAGCGAATCTCCTTCTGGCACTGCACCAGGTTCTACTAGCGAATCCCCGTCTGGTA CTGCTCCAGGTACTTCTACTCCTGAAAGCGGTTCCGCTTCTCCAGGTACCTCTACTCCGGAA AGCGGTTCTGCATCTCCAGGTAGCGAACCGGCAACCTCCGGCTCTGAAACCCCAGGTACCT CTGAAAGCGCTACTCCTGAATCCGGCCCAGGTAGCCCGGCAGGTTCTCCGACTTCCACTGA GGAAGGTACCTCTACTGAACCTTCTGAGGGCAGCGCTCCAGGTACTTCTGAAAGCGCTACC CCGGAGTCCGGTCCAGGTACTTCTACTGAACCGTCCGAAGGTAGCGCACCAGGTACTTCTA CCGAACCGTCCGAGGGTAGCGCACCAGGTAGCCCAGCAGGTTCTCCTACCTCCACCGAGG AAGGTACTTCTACCGAACCGTCCGAGGGTAGCGCACCAGGTACTTCTACCGAACCTTCCGA GGGCAGCGCACCAGGTACTTCTGAAAGCGCTACCCCTGAGTCCGGCCCAGGTACTTCTGA AAGCGCTACTCCTGAATCCGGTCCAGGTACCTCTACTGAACCTTCCGAAGGCAGCGCTCCA GGTACCTCTACCGAACCGTCCGAGGGCAGCGCACCAGGTACTTCTGAAAGCGCAACCCCT GAATCCGGTCCAGGTACTTCTACTGAACCTTCCGAAGGTAGCGCTCCAGGTAGCGAACCTG CTACTTCTGGTTCTGAAACCCCAGGTAGCCCGGCTGGCTCTCCGACCTCCACCGAGGAAGG TAGCTCTACCCCGTCTGGTGCTACTGGTTCTCCAGGTACTCCGGGCAGCGGTACTGCTTCTT CCTCTCCAGGTAGCTCTACCCCTTCTGGTGCTACTGGCTCTCCAGGTACCTCTACCGAACCG TCCGAGGGTAGCGCACCAGGTACCTCTACTGAACCGTCTGAGGGTAGCGCTCCAGGTAGC GAACCGGCAACCTCCGGTTCTGAAACTCCAGGTAGCCCTGCTGGCTCTCCGACTTCTACTG AGGAAGGTAGCCCGGCTGGTTCTCCGACTTCTACTGAGGAAGGTACTTCTACCGAACCTTC CGAAGGTAGCGCTCCAGGTGCAAGCGCAAGCGGCGCGCCAAGCACGGGAGGTACTTCTGA AAGCGCTACTCCTGAGTCCGGCCCAGGTAGCCCGGCTGGCTCTCCGACTTCCACCGAGGAA GGTAGCCCGGCTGGCTCTCCAACTTCTACTGAAGAAGGTTCTACCAGCTCTACCGCTGAAT CTCCTGGCCCAGGTTCTACTAGCGAATCTCCGTCTGGCACCGCACCAGGTACTTCCCCTAG CGGTGAATCTTCTACTGCACCAGGTACCCCTGGCAGCGGTACCGCTTCTTCCTCTCCAGGT AGCTCTACCCCGTCTGGTGCTACTGGCTCTCCAGGTTCTAGCCCGTCTGCATCTACCGGTAC CGGCCCAGGTAGCGAACCGGCAACCTCCGGCTCTGAAACTCCAGGTACTTCTGAAAGCGC TACTCCGGAATCCGGCCCAGGTAGCGAACCGGCTACTTCCGGCTCTGAAACCCCAGGTTCC ACCAGCTCTACTGCAGAATCTCCGGGCCCAGGTTCTACTAGCTCTACTGCAGAATCTCCGG GTCCAGGTACTTCTCCTAGCGGCGAATCTTCTACCGCTCCAGGTAGCGAACCGGCAACCTC TGGCTCTGAAACTCCAGGTAGCGAACCTGCAACCTCCGGCTCTGAAACCCCAGGTACTTCT ACTGAACCTTCTGAGGGCAGCGCACCAGGTTCTACCAGCTCTACCGCAGAATCTCCTGGTC CAGGTACCTCTACTCCGGAAAGCGGCTCTGCATCTCCAGGTTCTACTAGCGAATCTCCTTC TGGCACTGCACCAGGTACTTCTACCGAACCGTCCGAAGGCAGCGCTCCAGGTACCTCTACT GAACCTTCCGAGGGCAGCGCTCCAGGTACCTCTACCGAACCTTCTGAAGGTAGCGCACCA GGTAGCTCTACTCCGTCTGGTGCAACCGGCTCCCCAGGTTCTAGCCCGTCTGCTTCCACTG GTACTGGCCCAGGTGCTTCCCCGGGCACCAGCTCTACTGGTTCTCCAGGTAGCGAACCTGC TACCTCCGGTTCTGAAACCCCAGGTACCTCTGAAAGCGCAACTCCGGAGTCTGGTCCAGGT AGCCCTGCAGGTTCTCCTACCTCCACTGAGGAAGGTAGCTCTACTCCGTCTGGTGCAACCG GCTCCCCAGGTTCTAGCCCGTCTGCTTCCACTGGTACTGGCCCAGGTGCTTCCCCGGGCAC CAGCTCTACTGGTTCTCCAGGTACCTCTGAAAGCGCTACTCCGGAGTCTGGCCCAGGTACC TCTACTGAACCGTCTGAGGGTAGCGCTCCAGGTACTTCTACTGAACCGTCCGAAGGTAGCG CACCA |

(continued)

| XTEN Name | DNA Nucleotide Sequence |
|---|---|
| AE864 | GGTAGCCCGGCTGGCTCTCCTACCTCTACTGAGGAAGGTACTTCTGAAAGCGCTACTCCTGAGTCTGGTCCAGGTACCTCTACTGAACCGTCCGAAGGTAGCGCTCCAGGTAGCCCAGCAGGCTCTCCGACTTCCACTGAGGAAGGTACTTCTACTGAACCTTCCGAAGGCAGCGCACCAGGTACCTCTACTGAACCTTCTGAGGGCAGCGCTCCAGGTACTTCTGAAAGCGCTACCCCGGAATCTGGCCCAGGTAGCGAACCGGCTACTTCTGGTTCTGAAACCCCAGGTAGCGAACCGGCTACCTCCGGTTCTGAAACTCCAGGTAGCCCGGCAGGCTCTCCGACCTCTACTGAGGAAGGTACTTCTGAAAGCGCAACCCCGGAGTCCGGCCCAGGTACCTCTACCGAACCGTCTGAGGGCAGCGCACCAGGTACTTCTACCGAACCGTCCGAGGGTAGCGCACCAGGTAGCCCAGCAGGTTCTCCTACCTCCACCGAGGAAGGTACTTCTACCGAACCGTCCGAGGGTAGCGCACCAGGTACCTCTACTGAACCTTCTGAGGGCAGCGCTCCAGGTACTTCTGAAAGCGCTACCCCGGAGTCCGGTCCAGGTACTTCTACTGAACCGTCCGAAGGTAGCGCACCAGGTACTTCTGAAAGCGCAACCCCTGAATCCGGTCCAGGTAGCGAACCGGCTACTTCTGGCTCTGAGACTCCAGGTACTTCTACCGAACCGTCCGAAGGTAGCGCACCAGGTACTTCTACTGAACCGTCTGAAGGTAGCGCACCAGGTACTTCTGAAAGCGCAACCCCGGAATCCGGCCCAGGTACCTCTGAAAGCGCAACCCCGGAGTCCGGCCCAGGTAGCCCTGCTGGCTCTCCAACCTCCACCGAAGAAGGTACCTCTGAAAGCGCAACCCCTGAATCCGGCCCAGGTAGCGAACCGGCAACCTCCGGTTCTGAAACCCCAGGTACCTCTGAAAGCGCTACTCCGGAGTCTGGCCCAGGTACCTCTACTGAACCGTCTGAGGGTAGCGCTCCAGGTACTTCTACTGAACCGTCCGAAGGTAGCGCACCAGGTACTTCTACCGAACCGTCCGAAGGCAGCGCTCCAGGTACCTCTACTGAACCTTCCGAGGGCAGCGCTCCAGGTACCTCTACCGAACCTTCTGAAGGTAGCGCACCAGGTACTTCTACCGAACCGTCCGAGGGTAGCGCACCAGGTAGCCCAGCAGGTTCTCCTACCTCCACCGAGGAAGGTACTTCTACCGAACCGTCCGAGGGTAGCGCACCAGGTACCTCTGAAAGCGCAACTCCTGAGTCTGGCCCAGGTAGCGAACCTGCTACCTCCGGCTCTGAGACTCCAGGTACCTCTGAAAGCGCAACCCCGGAATCTGGTCCAGGTAGCGAACCTGCAACCTCTGGCTCTGAAACCCCAGGTACCTCTGAAAGCGCTACTCCTGAATCTGGCCCAGGTACTTCTACTGAACCGTCCGAGGGCAGCGCACCAGGTACTTCTGAAAGCGCTACTCCTGAGTCCGGCCCAGGTAGCCCGGCTGGCTCTCCGACTTCCACCGAGGAAGGTAGCCCGGCTGGCTCTCCAACTTCTACTGAAGAAGGTAGCCCGGCAGGCTCTCCGACCTCTACTGAGGAAGGTACTTCTGAAAGCGCAACCCCGGAGTCCGGCCCAGGTACCTCTACCGAACCGTCTGAGGGCAGCGCACCAGGTACCTCTGAAAGCGCAACTCCTGAGTCTGGCCCAGGTAGCGAACCTGCTACCTCCGGCTCTGAGACTCCAGGTACCTCTGAAAGCGCAACCCCGGAATCTGGTCCAGGTAGCGAACCTGCAACCTCTGGCTCTGAAACCCCAGGTACCTCTGAAAGCGCTACTCCTGAATCTGGCCCAGGTACTTCTACTGAACCGTCCGAGGGCAGCGCACCAGGTAGCCCTGCTGGCTCTCCAACCTCCACCGAAGAAGGTACCTCTGAAAGCGCAACCCCTGAATCCGGCCCAGGTAGCGAACCGGCAACCTCCGGTTCTGAAACCCCAGGTACTTCTGAAAGCGCTACTCCTGAGTCCGGCCCAGGTAGCCCGGCTGGCTCTCCGACTTCCACCGAGGAAGGTAGCCCGGCTGGCTCTCCAACTTCTACTGAAGAAGGTACTTCTACCGAACCTTCCGAGGGCAGCGCACCAGGTACTTCTGAAAGCGCTACCCCTGAGTCCGGCCCAGGTACTTCTGAAAGCGCTACTCCTGAATCCGGTCCAGGTACTTCTGAAAGCGCTACCCCGGAATCTGGCCCAGGTAGCGAACCGGCTACTTCTGGTTCTGAAACCCCAGGTAGCGAACCGGCTACCTCCGGTTCTGAAACTCCAGGTAGCCCAGCAGGCTCTCCGACTTCCACTGAGGAAGGTACTTCTACTGAACCTTCCGAAGGCAGCGCACCAGGTACCTCTACTGAACCTTCTGAGGGCAGCGCTCCAGGTAGCGAACCTGCAACCTCTGGCTCTGAAACCCCAGGTACCTCTGAAAGCGCTACTCCTGAATCTGGCCCAGGTACTTCTACTGAACCGTCCGAGGGCAGCGCACCA |

(continued)

| XTEN Name | DNA Nucleotide Sequence |
|---|---|
| AF864 | GGTTCTACCAGCGAATCTCCTTCTGGCACCGCTCCAGGTACCTCTCCTAGCGGCGAATCTT CTACCGCTCCAGGTTCTACTAGCGAATCTCCTTCTGGCACTGCACCAGGTTCTACTAGCGA ATCCCCGTCTGGTACTGCTCCAGGTACTTCTACTCCTGAAAGCGGTTCCGCTTCTCCAGGTA CCTCTACTCCGGAAAGCGGTTCTGCATCTCCAGGTTCTACCAGCGAATCTCCTTCTGGCAC CGCTCCAGGTTCTACTAGCGAATCCCCGTCTGGTACCGCACCAGGTACTTCTCCTAGCGGC GAATCTTCTACCGCACCAGGTTCTACTAGCGAATCTCCGTCTGGCACTGCTCCAGGTACTT CTCCTAGCGGTGAATCTTCTACCGCTCCAGGTACTTCCCCTAGCGGCGAATCTTCTACCGCT CCAGGTTCTACTAGCTCTACTGCAGAATCTCCGGGCCCAGGTACCTCTCCTAGCGGTGAAT CTTCTACCGCTCCAGGTACTTCTCCGAGCGGTGAATCTTCTACCGCTCCAGGTTCTACTAGC TCTACTGCAGAATCTCCTGGCCCAGGTACCTCTACTCCGGAAAGCGGCTCTGCATCTCCAG GTACTTCTACCCCTGAAAGCGGTTCTGCATCTCCAGGTTCTACTAGCGAATCTCCTTCTGGC ACTGCACCAGGTTCTACCAGCGAATCTCCGTCTGGCACTGCACCAGGTACCTCTACCCCTG AAAGCGGTTCCGCTTCTCCAGGTTCTACCAGCTCTACCGCAGAATCTCCTGGTCCAGGTAC CTCTACTCCGGAAAGCGGCTCTGCATCTCCAGGTTCTACTAGCGAATCTCCTTCTGGCACT GCACCAGGTACTTCTCCGAGCGGTGAATCTTCTACCGCACCAGGTTCTACTAGCTCTACCG CTGAATCTCCGGGCCCAGGTACTTCTCCGAGCGGTGAATCTTCTACTGCTCCAGGTACCTC |
| | TACTCCTGAAAGCGGTTCTGCATCTCCAGGTTCCACTAGCTCTACCGCAGAATCTCCGGGC CCAGGTTCTACTAGCTCTACTGCTGAATCTCCTGGCCCAGGTTCTACTAGCTCTACTGCTGA ATCTCCGGGTCCAGGTTCTACCAGCTCTACTGCTGAATCTCCTGGTCCAGGTACCTCCCCG AGCGGTGAATCTTCTACTGCACCAGGTTCTACTAGCGAATCTCCTTCTGGCACTGCACCAG GTTCTACCAGCGAATCTCCGTCTGGCACTGCACCAGGTACCTCTACCCCTGAAAGCGGTCC XXXXXXXXXXXXXTGCAAGCGCAAGCGGCGCGCCAAGCACGGGAXXXXXXXXXTAGCGAAT CTCCTTCTGGTACCGCTCCAGGTTCTACCAGCGAATCCCCGTCTGGTACTGCTCCAGGTTCT ACCAGCGAATCTCCTTCTGGTACTGCACCAGGTTCTACTAGCGAATCTCCTTCTGGTACCG CTCCAGGTTCTACCAGCGAATCCCCGTCTGGTACTGCTCCAGGTTCTACCAGCGAATCTCC TTCTGGTACTGCACCAGGTACTTCTACTCCGGAAAGCGGTTCCGCATCTCCAGGTACTTCTC CTAGCGGTGAATCTTCTACTGCTCCAGGTACCTCTCCTAGCGGCGAATCTTCTACTGCTCCA GGTTCTACCAGCTCTACTGCTGAATCTCCGGGTCCAGGTACTTCCCCGAGCGGTGAATCTT CTACTGCACCAGGTACTTCTACTCCGGAAAGCGGTTCCGCTTCTCCAGGTTCTACCAGCGA ATCTCCTTCTGGCACCGCTCCAGGTTCTACTAGCGAATCCCCGTCTGGTACCGCACCAGGT ACTTCTCCTAGCGGCGAATCTTCTACCGCACCAGGTTCTACTAGCGAATCCCCGTCTGGTA CCGCACCAGGTACTTCTACCCCGGAAAGCGGCTCTGCTTCTCCAGGTACTTCTACCCCGGA AAGCGGCTCCGCATCTCCAGGTTCTACTAGCGAATCTCCTTCTGGTACCGCTCCAGGTACT TCTACCCCTGAAAGCGGCTCCGCTTCTCCAGGTTCCACTAGCTCTACCGCTGAATCTCCGG GTCCAGGTTCTACCAGCGAATCTCCTTCTGGCACCGCTCCAGGTTCTACTAGCGAATCCCC GTCTGGTACCGCACCAGGTACTTCTCCTAGCGGCGAATCTTCTACCGCACCAGGTTCTACC AGCTCTACTGCTGAATCTCCGGGTCCAGGTACTTCCCCGAGCGGTGAATCTTCTACTGCAC CAGGTACTTCTACTCCGGAAAGCGGTTCCGCTTCTCCAGGTACCTCCCCTAGCGGCGAATC TTCTACTGCTCCAGGTACCTCTCCTAGCGGCGAATCTTCTACCGCTCCAGGTACCTCCCCTA GCGGTGAATCTTCTACCGCACCAGGTTCTACTAGCTCTACTGCTGAATCTCCGGGTCCAGG TTCTACCAGCTCTACTGCTGAATCTCCTGGTCCAGGTACCTCCCCGAGCGGTGAATCTTCTA CTGCACCAGGTTCTAGCCCTTCTGCTTCCACCGGTACCGGCCCAGGTAGCTCTACTCCGTCT GGTGCAACTGGCTCTCCAGGTAGCTCTACTCCGTCTGGTGCAACCGGCTCCCCA |
| | XXXX was inserted in two areas where no sequence information is available. |

(continued)

| XTEN Name | DNA Nucleotide Sequence |
|---|---|
| AG864 | GGTGCTTCCCCGGGCACCAGCTCTACTGGTTCTCCAGGTTCTAGCCCGTCTGCTTCTACTGGTACTGGTCCAGGTTCTAGCCCTTCTGCTTCCACTGGTACTGGTCCAGGTACCCCGGGGTAGCGGTACCGCTTCTTCTTCTCCAGGTAGCTCTACTCCGTCTGGTGCTACCGGCTCTCCAGGTTCTAACCCTTCTGCATCCACCGGTACCGGCCCAGGTGCTTCTCCGGGCACCAGCTCTACTGGTTCTCCAGGTACCCCGGGCAGCGGTACCGCATCTTCTTCTCCAGGTAGCTCTACTCCTTCTGGTGCAACTGGTTCTCCAGGTACTCCTGGCAGCGGTACCGCTTCTTCTTCTCCAGGTGCTTCTCCTGGTACTAGCTCTACTGGTTCTCCAGGTGCTTCTCCGGGCACTAGCTCTACTGGTTCTCCAGGTACCCCGGGTAGCGGTACTGCTTCTTCCTCTCCAGGTAGCTCTACCCCTTCTGGTGCAACCGGCTCTCCAGGTGCTTCTCCGGGCACCAGCTCTACCGGTTCTCCAGGTACCCCGGGTAGCGGTACCGCTTCTTCTTCTCCAGGTAGCTCTACTCCGTCTGGTGCTACCGGCTCTCCAGGTTCTAACCCTTCTGCATCCACCGGTACCGGCCCAGGTTCTAGCCCTTCTGCTTCCACCGGTACTGGCCCAGGTAGCTCTACCCCTTCTGGTGCTACCGGCTCCCCAGGTAGCTCTACTCCTTCTGGTGCAACTGGCTCTCCAGGTGCATCTCCGGGCACTAGCTCTACTGGTTCTCCAGGTGCATCCCCTGGCACTAGCTCTACTGGTTCTCCAGGTGCTTCTCCTGGTACCAGCTCTACTGGTTCTCCAGGTACTCCTGGCAGCGGTACCGCTTCTTCTTCTCCAGGTGCTTCTCCTGGTACTAGCTCTACTGGTTCTCCAGGTGCTTCTCCGGGCACTAGCTCTACTGGTTCTCCAGGTGCTTCCCCGGGCACTAGCTCTACCGGTTCTCCAGGTTCTAGCCCTTCTGCATCTACTGGTACTGGCCCAGGTACTCCGGGCAGCGGTACTGCTTCTTCCTCTCCAGGTGCATCTCCGGGCACTAGCTCTACTGGTTCTCCAGGTGCATCCCCTGGCACTAGCTCTACTGGTTCTCCAGGTGCTTCTCCTGGTACCAGCTCTACTGGTTCTCCAGGTAGCTCTACTCCGTCTGGTGCAACCGGTTCCCCAGGTAGCTCTACTCCTTCTGGTGCTACTGGCTCCCCAGGTGCATCCCCTGGCACCAGCTCTACCGGTTCTCCAGGTACCCCGGGCAGCGGTACCGCATCTTCCTCTCCAGGTAGCTCTACCCCGTCTGGTGCTACCGGTTCCCCAGGTAGCTCTACCCCGTCTGGTGCAACCGGCTCCCCAGGTAGCTCTACTCCGTCTGGTGCAACCGGCTCCCCAGGTTCTAGCCCGTCTGCTTCCACTGGTACTGGCCCAGGTGCTTCCCCGGGCACCAGCTCTACTGGTTCTCCAGGTGCATCCCCGGGTACCAGCTCTACCGGTTCTCCAGGTACTCCTGGCAGCGGTACTGCATCTTCCTCTCCAGGTGCTTCTCCGGGCACCAGCTCTACTGGTTCTCCAGGTGCATCTCCGGGCACTAGCTCTACTGGTTCTCCAGGTGCATCCCCTGGCACTAGCTCTACTGGTTCTCCAGGTGCTTCTCCTGGTACCAGCTCTACTGGTTCTCCAGGTACCCCTGGTAGCGGTACTGCTTCTTCCTCTCCAGGTAGCTCTACTCCGTCTGGTGCTA |
| | CCGGTTCTCCAGGTACCCCGGGTAGCGGTACCGCATCTTCTTCTCCAGGTAGCTCTACCCCGTCTGGTGCTACTGGTTCTCCAGGTACTCCGGGCAGCGGTACTGCTTCTTCCTCTCCAGGTAGCTCTACCCCTTCTGGTGCTACTGGCTCTCCAGGTAGCTCTACCCCGTCTGGTGCTACTGGCTCCCCAGGTTCTAGCCCTTCTGCATCCACCGGTACCGGTCCAGGTTCTAGCCCGTCTGCATCTACTGGTACTGGTCCAGGTGCATCCCCGGGCACTAGCTCTACCGGTTCTCCAGGTACTCCTGGTAGCGGTACTGCTTCTTCTTCTCCAGGTAGCTCTACTCCTTCTGGTGCTACTGGTTCTCCAGGTTCTAGCCCTTCTGCATCCACCGGTACCGGCCCAGGTTCTAGCCCGTCTGCTTCTACCGGTACTGGTCCAGGTGCTTCTCCGGGTACTAGCTCTACTGGTTCTCCAGGTGCATCTCCTGGTACTAGCTCTACTGGTTCTCCAGGTAGCTCTACTCCGTCTGGTGCAACCGGCTCTCCAGGTTCTAGCCCTTCTGCATCTACCGGTACTGGTCCAGGTGCATCCCTGGTACCAGCTCTACCGGTTCTCCAGGTTCTAGCCCTTCTGCTTCTACCGGTACCGGTCCAGGTACCCCTGGCAGCGGTACCGCATCTTCCTCTCCAGGTAGCTCTACTCCGTCTGGTGCAACCGGTTCCCCAGGTAGCTCTACTCCTTCTGGTGCTACTGGCTCCCCAGGTGCATCCCCTGGCACCAGCTCTACCGGTTCTCCA |

(continued)

| XTEN Name | DNA Nucleotide Sequence |
|---|---|
| AM923 | ATGGCTGAACCTGCTGGCTCTCCAACCTCCACTGAGGAAGGTGCATCCCCGGGCACCAGCTCTACCGGTTCTCCAGGTAGCTCTACCCCGTCTGGTGCTACCGGCTCTCCAGGTAGCTCTACCCCGTCTGGTGCTACTGGCTCTCCAGGTACTTCTACTGAACCGTCTGAAGGCAGCGCACCAGGTAGCGAACCGGCTACTTCCGGTTCTGAAACCCCAGGTAGCCCAGCAGGTTCTCCAACTTCTACTGAAGAAGGTTCTACCAGCTCTACCGCAGAATCTCCTGGTCCAGGTACCTCTACTCCGGAAAGCGGCTCTGCATCTCCAGGTTCTACTAGCGAATCTCCTTCTGGCACTGCACCAGGTTCTACTAGCGAATCCCCGTCTGGTACTGCTCCAGGTACTTCTACTCCTGAAAGCGGTTCCGCTTCTCCAGGTACCTCTACTCCGGAAAGCGGTTCTGCATCTCCAGGTAGCGAACCGGCAACCTCCGGCTCTGAAACCCCAGGTACCTCTGAAAGCGCTACTCCTGAATCCGGCCCAGGTAGCCCGGCAGGTTCTCCGACTTCCACTGAGGAAGGTACCTCTACTGAACCTTCTGAGGGCAGCGCTCCAGGTACTTCTGAAAGCGCTACCCCGGAGTCCGGTCCAGGTACTTCTACTGAACCGTCCGAAGGTAGCGCACCAGGTACTTCTACCGAACCGTCCGAGGGTAGCGCACCAGGTAGCCCAGCAGGTTCTCCTACCTCCACCGAGGAAGGTACTTCTACCGAACCGTCCGAGGGTAGCGCACCAGGTACTTCTACCGAACCTTCCGAGGGCAGCGCACCAGGTACTTCTGAAAGCGCTACCCCTGAGTCCGGCCCAGGTACTTCTGAAAGCGCTACTCCTGAATCCGGTCCAGGTACCTCTACTGAACCTTCCGAAGGCAGCGCTCCAGGTACCTCTACCGAACCGTCCGAGGGCAGCGCACCAGGTACTTCTGAAAGCGCAACCCCTGAATCCGGTCCAGGTACTTCTACTGAACCTTCCGAAGGTAGCGCTCCAGGTAGCGAACCTGCTACTTCTGGTTCTGAAACCCCAGGTAGCCCGGCTGGCTCTCCGACCTCCACCGAGGAAGGTAGCTCTACCCCGTCTGGTGCTACTGGTTCTCCAGGTACTCCGGGCAGCGGTACTGCTTCTTCCTCTCCAGGTAGCTCTACCCCTTCTGGTGCTACTGGCTCTCCAGGTACCTCTACCGAACCGTCCGAGGGTAGCGCACCAGGTACCTCTACTGAACCGTCTGAGGGTAGCGCTCCAGGTAGCGAACCGGCAACCTCCGGTTCTGAAACTCCAGGTAGCCCTGCTGGCTCTCCGACTTCTACTGAGGAAGGTAGCCCGGCTGGTTCTCCGACTTCTACTGAGGAAGGTACTTCTACCGAACCTTCCGAAGGTAGCGCTCCAGGTGCAAGCGCAAGCGGCGCGCCAAGCACGGGAGGTACTTCTGAAAGCGCTACTCCTGAGTCCGGCCCAGGTAGCCCGGCTGGCTCTCCGACTTCCACCGAGGAAGGTAGCCCGGCTGGCTCTCCAACTTCTACTGAAGAAGGTTCTACCAGCTCTACCGCTGAATCTCCTGGCCCAGGTTCTACTAGCGAATCTCCGTCTGGCACCGCACCAGGTACTTCCCCTAGCGGTGAATCTTCTACTGCACCAGGTACCCCTGGCAGCGGTACCGCTTCTTCCTCTCCAGGTAGCTCTACCCCGTCTGGTGCTACTGGCTCTCCAGGTTCTAGCCCGTCTGCATCTACCGGTACCGGCCCAGGTAGCGAACCGGCAACCTCCGGCTCTGAAACTCCAGGTACTTCTGAAAGCGCTACTCCGGAATCCGGCCCAGGTAGCGAACCGGCTACTTCCGGCTCTGAAACCCCAGGTTCCACCAGCTCTACTGCAGAATCTCCGGGCCCAGGTTCTACTAGCTCTACTGCAGAATCTCCGGGTCCAGGTACTTCTCCTAGCGGCGAATCTTCTACCGCTCCAGGTAGCGAACCGGCAACCTCTGGCTCTGAAACTCCAGGTAGCGAACCTGCAACCTCCGGCTCTGAAACCCCAGGTACTTCTACTGAACCTTCTGAGGGCAGCGCACCAGGTTCTACCAGCTCTACCGCAGAATCTCCTGGTCCAGGTACCTCTACTCCGGAAAGCGGCTCTGCATCTCCAGGTTCTACTAGCGAATCTCCTTCTGGCACTGCACCAGGTACTTCTACCGAACCGTCCGAAGGCAGCGCTCCAGGTACCTCTACTGAACCTTCCGAGGGCAGCGCTCCAGGTACCTCTACCGAACCTTCTGAAGGTAGCGCACCAGGTAGCTCTACTCCGTCTGGTGCAACCGGCTCCCCAGGTTCTAGCCCGTCTGCTTCCACTGGTACTGGCCCAGGTGCTTCCCCGGGCACCAGCTCTACTGGTTCTCCAGGTAGCGAACCTGCTACCTCCGGTTCTGAAACCCCAGGTACCTCTGAAAGCGCAACTCCGGAGTCTGGTCCAGGTAGCCCTGCAGGTTCTCCTACCTCCACTGAGGAAGGTAGCTCTACTCCGTCTGGTGCAACCGGCTCCCCAGGTTCTAGCCCGTCTGCTTCCACTGGTACTGGCCCAGGTGCTTCCCCGGGCACCAGCTCTACTGGTTCTCCAGGTACCTCTGAAAGCGCTACTCCGGAGTCTGGCCCAGGTACCTCTACTGAACCGTCTGAGGGTAGCGCTCCAGGTACTTCTACTGAACCGTCCGAAGGTAGCGCACCA |

(continued)

| XTEN Name | DNA Nucleotide Sequence |
|---|---|
| AE912 | ATGGCTGAACCTGCTGGCTCTCCAACCTCCACTGAGGAAGGTACCCCGGGTAGCGGTACTG CTTCTTCCTCTCCAGGTAGCTCTACCCCTTCTGGTGCAACCGGCTCTCCAGGTGCTTCTCCG GGCACCAGCTCTACCGGTTCTCCAGGTAGCCCGGCTGGCTCTCCTACCTCTACTGAGGAAG GTACTTCTGAAAGCGCTACTCCTGAGTCTGGTCCAGGTACCTCTACTGAACCGTCCGAAGG TAGCGCTCCAGGTAGCCCAGCAGGCTCTCCGACTTCCACTGAGGAAGGTACTTCTACTGAA CCTTCCGAAGGCAGCGCACCAGGTACCTCTACTGAACCTTCTGAGGGCAGCGCTCCAGGTA CTTCTGAAAGCGCTACCCCGGAATCTGGCCCAGGTAGCGAACCGGCTACTTCTGGTTCTGA AACCCCAGGTAGCGAACCGGCTACCTCCGGTTCTGAAACTCCAGGTAGCCCGGCAGGCTC TCCGACCTCTACTGAGGAAGGTACTTCTGAAAGCGCAACCCCGGAGTCCGGCCCAGGTAC CTCTACCGAACCGTCTGAGGGCAGCGCACCAGGTACTTCTACCGAACCGTCCGAGGGTAG CGCACCAGGTAGCCCAGCAGGTTCTCCTACCTCCACCGAGGAAGGTACTTCTACCGAACCG TCCGAGGGTAGCGCACCAGGTACCTCTACTGAACCTTCTGAGGGCAGCGCTCCAGGTACTT CTGAAAGCGCTACCCCGGAGTCCGGTCCAGGTACTTCTACTGAACCGTCCGAAGGTAGCG CACCAGGTACTTCTGAAAGCGCAACCCCTGAATCCGGTCCAGGTAGCGAACCGGCTACTTC TGGCTCTGAGACTCCAGGTACTTCTACCGAACCGTCCGAAGGTAGCGCACCAGGTACTTCT ACTGAACCGTCTGAAGGTAGCGCACCAGGTACTTCTGAAAGCGCAACCCCGGAATCCGGC CCAGGTACCTCTGAAAGCGCAACCCCGGAGTCCGGCCCAGGTAGCCCTGCTGGCTCTCCA ACCTCCACCGAAGAAGGTACCTCTGAAAGCGCAACCCCTGAATCCGGCCCAGGTAGCGAA CCGGCAACCTCCGGTTCTGAAACCCCAGGTACCTCTGAAAGCGCTACTCCGGAGTCTGGCC CAGGTACCTCTACTGAACCGTCTGAGGGTAGCGCTCCAGGTACTTCTACTGAACCGTCCGA AGGTAGCGCACCAGGTACTTCTACCGAACCGTCCGAAGGCAGCGCTCCAGGTACCTCTACT GAACCTTCCGAGGGCAGCGCTCCAGGTACCTCTACCGAACCTTCTGAAGGTAGCGCACCA GGTACTTCTACCGAACCGTCCGAGGGTAGCGCACCAGGTAGCCCAGCAGGTTCTCCTACCT CCACCGAGGAAGGTACTTCTACCGAACCGTCCGAGGGTAGCGCACCAGGTACCTCTGAAA GCGCAACTCCTGAGTCTGGCCCAGGTAGCGAACCTGCTACCTCCGGCTCTGAGACTCCAGG TACCTCTGAAAGCGCAACCCCGGAATCTGGTCCAGGTAGCGAACCTGCAACCTCTGGCTCT GAAACCCCAGGTACCTCTGAAAGCGCTACTCCTGAATCTGGCCCAGGTACTTCTACTGAAC CGTCCGAGGGCAGCGCACCAGGTACTTCTGAAAGCGCTACTCCTGAGTCCGGCCCAGGTA GCCCGGCTGGCTCTCCGACTTCCACCGAGGAAGGTAGCCCGGCTGGCTCTCCAACTTCTAC TGAAGAAGGTAGCCCGGCAGGCTCTCCGACCTCTACTGAGGAAGGTACTTCTGAAAGCGC AACCCCGGAGTCCGGCCCAGGTACCTCTACCGAACCGTCTGAGGGCAGCGCACCAGGTAC CTCTGAAAGCGCAACTCCTGAGTCTGGCCCAGGTAGCGAACCTGCTACCTCCGGCTCTGAG ACTCCAGGTACCTCTGAAAGCGCAACCCCGGAATCTGGTCCAGGTAGCGAACCTGCAACC TCTGGCTCTGAAACCCCAGGTACCTCTGAAAGCGCTACTCCTGAATCTGGCCCAGGTACTT CTACTGAACCGTCCGAGGGCAGCGCACCAGGTAGCCCTGCTGGCTCTCCAACCTCCACCG AAGAAGGTACCTCTGAAAGCGCAACCCCTGAATCCGGCCCAGGTAGCGAACCGGCAACCT CCGGTTCTGAAACCCCAGGTACTTCTGAAAGCGCTACTCCTGAGTCCGGCCCAGGTAGCCC GGCTGGCTCTCCGACTTCCACCGAGGAAGGTAGCCCGGCTGGCTCTCCAACTTCTACTGAA GAAGGTACTTCTACCGAACCTTCCGAGGGCAGCGCACCAGGTACTTCTGAAAGCGCTACC CCTGAGTCCGGCCCAGGTACTTCTGAAAGCGCTACTCCTGAATCCGGTCCAGGTACTTCTG AAAGCGCTACCCCGGAATCTGGCCCAGGTAGCGAACCGGCTACTTCTGGTTCTGAAACCCC AGGTAGCGAACCGGCTACCTCCGGTTCTGAAACTCCAGGTAGCCCAGCAGGCTCTCCGACT TCCACTGAGGAAGGTACTTCTACTGAACCTTCCGAAGGCAGCGCACCAGGTACCTCTACTG AACCTTCTGAGGGCAGCGCTCCAGGTAGCGAACCTGCAACCTCTGGCTCTGAAACCCCAG GTACCTCTGAAAGCGCTACTCCTGAATCTGGCCCAGGTACTTCTACTGAACCGTCCGAGGG CAGCGCACCA |

(continued)

| XTEN Name | DNA Nucleotide Sequence |
|---|---|
| AM1318 | GGTACTTCTACTGAACCGTCTGAAGGCAGCGCACCAGGTAGCGAACCGGCTACTTCCGGTTCTGAAACCCCAGGTAGCCCAGCAGGTTCTCCAACTTCTACTGAAGAAGGTTCTACCAGCTCTACCGCAGAATCTCCTGGTCCAGGTACCTCTACTCCGGAAAGCGGCTCTGCATCTCCAGGTTCTACTAGCGAATCTCCTTCTGGCACTGCACCAGGTTCTACTAGCGAATCCCCGTCTGGTACTGCTCCAGGTACTTCTACTCCTGAAAGCGGTTCCGCTTCTCCAGGTACCTCTACTCCGGAAAGCGGTTCTGCATCTCCAGGTAGCGAACCGGCAACCTCCGGCTCTGAAACCCCAGGTACCTCTGAAAGCGCTACTCCTGAATCCGGCCCAGGTAGCCCGGCAGGTTCTCCGACTTCCACTGAGGAAGGTACCTCTACTGAACCTTCTGAGGGCAGCGCTCCAGGTACTTCTGAAAGCGCTACCCCGGAGTCCGGTCCAGGTACTTCTACTGAACCGTCCGAAGGTAGCGCACCAGGTACTTCTACCGAACCGTCCGAGGGTAGCGCACCAGGTAGCCCAGCAGGTTCTCCTACCTCCACCGAGGAAGGTACTTCTACCGAACCGTCCGAGGGTAGCGCACCAGGTACTTCTACCGAACCTTCCGAGGGCAGCGCACCAGGTACTTCTGAAAGCGCTACCCCTGAGTCCGGCCCAGGTACTTCTGAAAGCGCTACTCCTGAATCCGGTCCAGGTACCTCTACTGAACCTTCCGAAGGCAGCGCTCCA |

(continued)

| XTEN Name | DNA Nucleotide Sequence |
|---|---|
| | GGTACCTCTACCGAACCGTCCGAGGGCAGCGCACCAGGTACTTCTGAAAGCGCAACCCCT GAATCCGGTCCAGGTACTTCTACTGAACCTTCCGAAGGTAGCGCTCCAGGTAGCGAACCTG CTACTTCTGGTTCTGAAACCCCAGGTAGCCCGGCTGGCTCTCCGACCTCCACCGAGGAAGG TAGCTCTACCCCGTCTGGTGCTACTGGTTCTCCAGGTACTCCGGGCAGCGGTACTGCTTCTT CCTCTCCAGGTAGCTCTACCCCTTCTGGTGCTACTGGCTCTCCAGGTACCTCTACCGAACCG TCCGAGGGTAGCGCACCAGGTACCTCTACTGAACCGTCTGAGGGTAGCGCTCCAGGTAGC GAACCGGCAACCTCCGGTTCTGAAACTCCAGGTAGCCCTGCTGGCTCTCCGACTTCTACTG AGGAAGGTAGCCCGGCTGGTTCTCCGACTTCTACTGAGGAAGGTACTTCTACCGAACCTTC CGAAGGTAGCGCTCCAGGTCCAGAACCAACGGGGCCGGCCCCAAGCGGAGGTAGCGAAC CGGCAACCTCCGGCTCTGAAACCCCAGGTACCTCTGAAAGCGCTACTCCTGAATCCGGCCC AGGTAGCCCGGCAGGTTCTCCGACTTCCACTGAGGAAGGTACTTCTGAAAGCGCTACTCCT GAGTCCGGCCCAGGTAGCCCGGCTGGCTCTCCGACTTCCACCGAGGAAGGTAGCCCGGCT GGCTCTCCAACTTCTACTGAAGAAGGTACTTCTGAAAGCGCTACTCCTGAGTCCGGCCCAG GTAGCCCGGCTGGCTCTCCGACTTCCACCGAGGAAGGTAGCCCGGCTGGCTCTCCAACTTC TACTGAAGAAGGTTCTACCAGCTCTACCGCTGAATCTCCTGGCCCAGGTTCTACTAGCGAA TCTCCGTCTGGCACCGCACCAGGTACTTCCCCTAGCGGTGAATCTTCTACTGCACCAGGTT CTACCAGCGAATCTCCTTCTGGCACCGCTCCAGGTTCTACTAGCGAATCCCGTCTGGTAC CGCACCAGGTACTTCTCCTAGCGGCGAATCTTCTACCGCACCAGGTACTTCTACCGAACCT TCCGAGGGCAGCGCACCAGGTACTTCTGAAAGCGCTACCCCTGAGTCCGGCCCAGGTACTT CTGAAAGCGCTACTCCTGAATCCGGTCCAGGTAGCGAACCGGCAACCTCTGGCTCTGAAA CCCCAGGTACCTCTGAAAGCGCTACTCCGGAATCTGGTCCAGGTACTTCTGAAAGCGCTAC TCCGGAATCCGGTCCAGGTACCTCTACTGAACCTTCTGAGGGCAGCGCTCCAGGTACTTCT GAAAGCGCTACCCCGGAGTCCGGTCCAGGTACTTCTACTGAACCGTCCGAAGGTAGCGCA CCAGGTACCTCCCCTAGCGGCGAATCTTCTACTGCTCCAGGTACCTCTCCTAGCGGCGAAT CTTCTACCGCTCCAGGTACCTCCCCTAGCGGTGAATCTTCTACCGCACCAGGTACTTCTACC GAACCGTCCGAGGGTAGCGCACCAGGTAGCCCAGCAGGTTCTCCTACCTCCACCGAGGAA GGTACTTCTACCGAACCGTCCGAGGGTAGCGCACCAGGTTCTAGCCCTTCTGCTTCCACCG GTACCGGCCCAGGTAGCTCTACTCCGTCTGGTGCAACTGGCTCTCCAGGTAGCTCTACTCC GTCTGGTGCAACCGGCTCCCCAGGTAGCTCTACCCCGTCTGGTGCTACCGGCTCTCCAGGT AGCTCTACCCCGTCTGGTGCAACCGGCTCCCCAGGTGCATCCCCGGGTACTAGCTCTACCG GTTCTCCAGGTGCAAGCGCAAGCGGCGCGCCAAGCACGGGAGGTACTTCTCCGAGCGGTG AATCTTCTACCGCACCAGGTTCTACTAGCTCTACCGCTGAATCTCCGGGCCCAGGTACTTCT CCGAGCGGTGAATCTTCTACTGCTCCAGGTACCTCTGAAAGCGCTACTCCGGAGTCTGGCC CAGGTACCTCTACTGAACCGTCTGAGGGTAGCGCTCCAGGTACTTCTACTGAACCGTCCGA AGGTAGCGCACCAGGTTCTAGCCCTTCTGCATCTACTGGTACTGGCCCAGGTAGCTCTACT CCTTCTGGTGCTACCGGCTCTCCAGGTGCTTCTCCGGGTACTAGCTCTACCGGTTCTCCAGG TACTTCTACTCCGGAAAGCGGTTCCGCATCTCCAGGTACTTCTCCTAGCGGTGAATCTTCTA CTGCTCCAGGTACCTCTCCTAGCGGCGAATCTTCTACTGCTCCAGGTACTTCTGAAAGCGC AACCCCTGAATCCGGTCCAGGTAGCGAACCGGCTACTTCTGGCTCTGAGACTCCAGGTACT TCTACCGAACCGTCCGAAGGTAGCGCACCAGGTTCTACCAGCGAATCCCCTTCTGGTACTG CTCCAGGTTCTACCAGCGAATCCCCTTCTGGCACCGCACCAGGTACTTCTACCCCTGAAAG CGGCTCCGCTTCTCCAGGTAGCCCGGCAGGCTCTCCGACCTCTACTGAGGAAGGTACTTCT GAAAGCGCAACCCCGGAGTCCGGCCCAGGTACCTCTACCGAACCGTCTGAGGGCAGCGCA CCAGGTAGCCCTGCTGGCTCTCCAACCTCCACCGAAGAAGGTACCTCTGAAAGCGCAACC CCTGAATCCGGCCCAGGTAGCGAACCGGCAACCTCCGGTTCTGAAACCCCAGGTAGCTCT ACCCCGTCTGGTGCTACCGGTTCCCCAGGTGCTTCTCCTGGTACTAGCTCTACCGGTTCTCC AGGTAGCTCTACCCCGTCTGGTGCTACTGGCTCTCCAGGTTCTACTAGCGAATCCCCGTCT GGTACTGCTCCAGGTACTTCCCCTAGCGGTGAATCTTCTACTGCTCCAGGTTCTACCAGCTC TACCGCAGAATCTCCGGGTCCAGGTAGCTCTACCCCTTCTGGTGCAACCGGCTCTCCAGGT GCATCCCCGGGTACCAGCTCTACCGGTTCTCCAGGTACTCCGGGTAGCGGTACCGCTTCTT CCTCTCCAGGTAGCCCTGCTGGCTCTCCGACTTCTACTGAGGAAGGTAGCCCGGCTGGTTC TCCGACTTCTACTGAGGAAGGTACTTCTACCGAACCTTCCGAAGGTAGCGCTCCA |

(continued)

| XTEN Name | DNA Nucleotide Sequence |
|---|---|
| BC864 | GGTACTTCCACCGAACCATCCGAACCAGGTAGCGCAGGTACTTCCACCGAACCATCCGAACCTGGCAGCGCAGGTAGCGAACCGGCAACCTCTGGTACTGAACCATCAGGTAGCGGCGCATCCGAGCCTACCTCTACTGAACCAGGTAGCGAACCGGCTACCTCCGGTACTGAGCCATCAGGTAGCGAACCGGCAACTTCCGGTACTGAACCATCAGGTAGCGAACCGGCAACTTCCGGCACTGAACCATCAGGTAGCGGTGCATCTGAGCCGACCTCTACTGAACCAGGTACTTCTACTGAACCATCTGAGCCGGGCAGCGCAGGTAGCGAACCAGCTACTTCTGGCACTGAACCATCAGGTACTTCTACTGAACCATCCGAACCAGGTAGCGCAGGTAGCGAACCTGCTACCTCTGGTACT |
| | GAGCCATCAGGTAGCGAACCGGCTACCTCTGGTACTGAACCATCAGGTACTTCTACCGAACCATCCGAGCCTGGTAGCGCAGGTACTTCTACCGAACCATCCGAGCCAGGCAGCGCAGGTAGCGAACCGGCAACCTCTGGCACTGAGCCATCAGGTAGCGAACCAGCAACTTCTGGTACTGAACCATCAGGTACTAGCGAGCCATCTACTTCCGAACCAGGTGCAGGTAGCGGCGCATCCGAACCTACTTCCACTGAACCAGGTACTAGCGAGCCATCCACCTCTGAACCAGGTGCAGGTAGCGAACCGGCAACTTCCGGCACTGAACCATCAGGTAGCGAACCGGCTACCTCTGGTACTGAACCATCAGGTACTTCTACCGAACCATCCGAGCCTGGTAGCGCAGGTACTTCTACCGAACCATCCGAGCCAGGCAGCGCAGGTAGCGGTGCATCCGAGCCGACCTCTACTGAACCAGGTAGCGAACCAGCAACTTCTGGCACTGAGCCATCAGGTAGCGAACCAGCTACCTCTGGTACTGAACCATCAGGTAGCGAACCGGCTACTTCCGGCACTGAACCATCAGGTAGCGAACCAGCAACCTCCGGTACTGAACCATCAGGTACTTCCACTGAACCATCCGAACCGGGTAGCGCAGGTAGCGAACCGGCAACTTCCGGCACTGAACCATCAGGTAGCGGTGCATCTGAGCCGACCTCTACTGAACCAGGTACTTCTACTGAACCATCTGAGCCGGGCAGCGCAGGTAGCGAACCTGCAACCTCCGGCACTGAGCCATCAGGTAGCGGCGCATCTGAACCAACCTCTACTGAACCAGGTACTTCCACCGAACCATCTGAGCCAGGCAGCGCAGGTAGCGGCGCATCTGAACCAACCTCTACTGAACCAGGTAGCGAACCAGCAACTTCTGGTACTGAACCATCAGGTAGCGGCGCATCTGAGCCTACTTCCACTGAACCAGGTAGCGAACCGGCAACTTCCGGCACTGAACCATCAGGTAGCGGTGCATCTGAGCCGACCTCTACTGAACCAGGTACTTCTACTGAACCATCTGAGCCGGGCAGCGCAGGTAGCGAACCGGCAACTTCCGGCACTGAACCATCAGGTAGCGGTGCATCTGAGCCGACCTCTACTGAACCAGGTACTTCTACTGAACCATCTGAGCCGGGCAGCGCAGGTAGCGAACCAGCTACTTCTGGCACTGAACCATCAGGTACTTCTACTGAACCATCCGAACCAGGTAGCGCAGGTAGCGAACCTGCTACCTCTGGTACTGAGCCATCAGGTACTTCTACTGAACCATCCGAGCCGGGTAGCGCAGGTACTTCCACTGAACCATCTGAACCTGGTAGCGCAGGTACTTCCACTGAACCATCCGAACCAGGTAGCGCAGGTACTTCTACTGAACCATCCGAGCCGGGTAGCGCAGGTACTTCCACTGAACCATCTGAACCTGGTAGCGCAGGTACTTCCACTGAACCATCCGAACCAGGTAGCGCAGGTACTAGCGAACCATCCACCTCCGAACCAGGCGCAGGTAGCGGTGCATCTGAACCGACTTCTACTGAACCAGGTACTTCCACTGAACCATCTGAGCCAGGTAGCGCAGGTACTTCCACCGAACCATCCGAACCAGGTAGCGCAGGTACTTCCACCGAACCATCCGAACCTGGCAGCGCAGGTAGCGAACCGGCAACCTCTGGTACTGAACCATCAGGTAGCGGTGCATCCGAGCCGACCTCTACTGAACCAGGTAGCGAACCAGCAACTTCTGGCACTGAGCCATCAGGTAGCGAACCAGCTACCTCTGGTACTGAACCATCAGGTAGCGAACCGGCAACCTCTGGCACTGAGCCATCAGGTAGCGAACCAGCAACTTCTGGTACTGAACCATCAGGTACTAGCGAGCCATCTACTTCCGAACCAGGTGCAGGTAGCGAACCTGCAACCTCCGGCACTGAGCCATCAGGTAGCGGCGCATCTGAACCAACCTCTACTGAACCAGGTACTTCCACCGAACCATCTGAGCCAGGCAGCGCAGGTAGCGAACCTGCAACCTCCGGCACTGAGCCATCAGGTAGCGGCGCATCTGAACCAACCTCTACTGAACCAGGTACTTCCACCGAACCATCTGAGCCAGGCAGCGCA |

(continued)

| XTEN Name | DNA Nucleotide Sequence |
|---|---|
| BD864 | GGTAGCGAAACTGCTACTTCCGGCTCTGAGACTGCAGGTACTAGTGAATCCGCAACTAGC GAATCTGGCGCAGGTAGCACTGCAGGCTCTGAGACTTCCACTGAAGCAGGTACTAGCGAG TCCGCAACCAGCGAATCCGGCGCAGGTAGCGAAACTGCTACCTCTGGCTCCGAGACTGCA GGTAGCGAAACTGCAACCTCTGGCTCTGAAACTGCAGGTACTTCCACTGAAGCAAGTGAA GGCTCCGCATCAGGTACTTCCACCGAAGCAAGCGAAGGCTCCGCATCAGGTACTAGTGAG TCCGCAACTAGCGAATCCGGTGCAGGTAGCGAAACCGCTACCTCTGGTTCCGAAACTGCA GGTACTTCTACCGAGGCTAGCGAAGGTTCTGCATCAGGTAGCACTGCTGGTTCCGAGACTT CTACTGAAGCAGGTACTAGCGAATCTGCTACTAGCGAATCCGGCGCAGGTACTAGCGAAT CCGCTACCAGCGAATCCGGCGCAGGTAGCGAAACTGCAACCTCTGGTTCCGAGACTGCAG GTACTAGCGAGTCCGCTACTAGCGAATCTGGCGCAGGTACTTCCACTGAAGCTAGTGAAG GTTCTGCATCAGGTAGCGAAACTGCTACTTCTGGTTCCGAAACTGCAGGTAGCGAAACCGC TACCTCTGGTTCCGAAACTGCAGGTACTTCTACCGAGGCTAGCGAAGGTTCTGCATCAGGT AGCACTGCTGGTTCCGAGACTTCTACTGAAGCAGGTACTAGCGAGTCCGCTACTAGCGAAT CTGGCGCAGGTACTTCCACTGAAGCTAGTGAAGGTTCTGCATCAGGTAGCGAAACTGCTAC TTCTGGTTCCGAAACTGCAGGTAGCACTGCTGGCTCCGAGACTTCTACCGAAGCAGGTAGC ACTGCAGGTTCCGAAACTTCCACTGAAGCAGGTAGCGAAACTGCTACCTCTGGCTCTGAGA CTGCAGGTACTAGCGAATCTGCTACTAGCGAATCCGGCGCAGGTACTAGCGAATCCGCTA CCAGCGAATCCGGCGCAGGTAGCGAAACTGCAACCTCTGGTTCCGAGACTGCAGGTACTA GCGAATCTGCTACTAGCGAATCCGGCGCAGGTACTAGCGAATCCGCTACCAGCGAATCCG GCGCAGGTAGCGAAACTGCAACCTCTGGTTCCGAGACTGCAGGTAGCGAAACCGCTACCT CTGGTTCCGAAACTGCAGGTACTTCTACCGAGGCTAGCGAAGGTTCTGCATCAGGTAGCAC TGCTGGTTCCGAGACTTCTACTGAAGCAGGTAGCGAAACTGCTACTTCCGGCTCTGAGACT GCAGGTACTAGTGAATCCGCAACTAGCGAATCTGGCGCAGGTAGCACTGCAGGCTCTGAG |
| | ACTTCCACTGAAGCAGGTAGCACTGCTGGTTCCGAAACCTCTACCGAAGCAGGTAGCACT GCAGGTTCTGAAACCTCCACTGAAGCAGGTACTTCCACTGAGGCTAGTGAAGGCTCTGCAT CAGGTAGCACTGCTGGTTCCGAAACCTCTACCGAAGCAGGTAGCACTGCAGGTTCTGAAA CCTCCACTGAAGCAGGTACTTCCACTGAGGCTAGTGAAGGCTCTGCATCAGGTAGCACTGC AGGTTCTGAGACTTCCACCGAAGCAGGTAGCGAAACTGCTACTTCTGGTTCCGAAACTGCA GGTACTTCCACTGAAGCTAGTGAAGGTTCCGCATCAGGTACTAGTGAGTCCGCAACCAGC GAATCCGGCGCAGGTAGCGAAACCGCAACCTCCGGTTCTGAAACTGCAGGTACTAGCGAA TCCGCAACCAGCGAATCTGGCGCAGGTACTAGTGAGTCCGCAACCAGCGAATCCGGCGCA GGTAGCGAAACCGCAACCTCCGGTTCTGAAACTGCAGGTACTAGCGAATCCGCAACCAGC GAATCTGGCGCAGGTAGCGAAACTGCTACTTCCGGCTCTGAGACTGCAGGTACTTCCACCG AAGCAAGCGAAGGTTCCGCATCAGGTACTTCCACCGAGGCTAGTGAAGGCTCTGCATCAG GTAGCACTGCTGGCTCCGAGACTTCTACCGAAGCAGGTAGCACTGCAGGTTCCGAAACTTC CACTGAAGCAGGTAGCGAAACTGCTACCTCTGGCTCTGAGACTGCAGGTACTAGCGAATC TGCTACTAGCGAATCCGGCGCAGGTACTAGCGAATCCGCTACCAGCGAATCCGGCGCAGG TAGCGAAACTGCAACCTCTGGTTCCGAGACTGCAGGTAGCGAAACTGCTACTTCCGGCTCC GAGACTGCAGGTAGCGAAACTGCTACTTCTGGCTCCGAAACTGCAGGTACTTCTACTGAGG CTAGTGAAGGTTCCGCATCAGGTACTAGCGAGTCCGCAACCAGCGAATCCGGCGCAGGTA GCGAAACTGCTACCTCTGGCTCCGAGACTGCAGGTAGCGAAACTGCAACCTCTGGCTCTGA AACTGCAGGTACTAGCGAATCTGCTACTAGCGAATCCGGCGCAGGTACTAGCGAATCCGC TACCAGCGAATCCGGCGCAGGTAGCGAAACTGCAACCTCTGGTTCCGAGACTGCA |

One may clone the library of XTEN-encoding genes into one or more expression vectors known in the art. To facilitate the identification of well-expressing library members, one can construct the library as fusion to a reporter protein. Non-limiting examples of suitable reporter genes are green fluorescent protein, luciferace, alkaline phosphatase, and beta-galactosidase. By screening, one can identify short XTEN sequences that can be expressed in high concentration in the host organism of choice. Subsequently, one can generate a library of random XTEN dimers and repeat the screen for high level of expression. Subsequently, one can screen the resulting constructs for a number of properties such as level of expression, protease stability, or binding to antiserum.

One aspect of the invention is to provide polynucleotide sequences encoding the components of the fusion protein wherein the creation of the sequence has undergone codon optimization. Of particular interest is codon optimization with the goal of improving expression of the polypeptide compositions and to improve the genetic stability of the encoding gene in the production hosts. For example, codon optimization is of particular importance for XTEN sequences that are rich in glycine or that have very repetitive amino acid sequences. Codon optimization is performed using computer programs (Gustafsson, C., et al. (2004) Trends Biotechnol, 22: 346-53), some of which minimize ribosomal pausing (Coda Genomics Inc.). In one embodiment, one can perform codon optimization by constructing codon libraries where all members of the library encode the same amino acid sequence but where codon usage is varied. Such libraries can be screened for highly expressing and genetically stable members that are particularly suitable for the large-scale production of XTEN-containing products. When designing XTEN sequences one can consider a number of properties. One can minimize the repetitiveness in the encoding DNA sequences. In addition, one can avoid or minimize the use of codons that are rarely used by the production host (e.g. the AGG and AGA arginine codons and one leucine codon in *E. coli*). In the case of *E. coli,* two glycine codons, GGA and GGG, are rarely used in highly expressed proteins. Thus codon optimization of the gene encoding XTEN sequences can be very desirable. DNA sequences that have a high level of glycine tend to have a high GC content that can lead to instability or low expression levels. Thus, when possible, it is preferred to choose codons such that the GC-content of XTEN-encoding sequence is suitable for the production organism that will be used to manufacture the XTEN.

Optionally, the full-length XTEN-encoding gene comprises one or more sequencing islands. In this context, sequencing islands are short-stretch sequences that are distinct from the XTEN library construct sequences and that include a restriction site not present or expected to be present in the full-length XTEN-encoding gene. In one embodiment, a sequencing island is the sequence 5'-AGGTGCAAGCGCAAGCGGCGCGCGCCAAGCACGGGAGGT-3'. In another embodiment, a sequencing island is the sequence 5'-AGGTCCAGAACCAACGGGGCCGGCCCCAAGCGGAGGT-3'.

In one embodiment, polynucleotide libraries are constructed using the disclosed methods wherein all members of the library encode the same amino acid sequence but the codon usage for the respective amino acids in the sequence is varied. Such libraries can be screened for highly expressing and genetically stable members that are particularly suitable for the large-scale production of XTEN-containing products.

Optionally, one can sequence clones in the library to eliminate isolates that contain undesirable sequences. The initial library of short XTEN sequences allows some variation in amino acid sequence. For instance one can randomize some codons such that a number of hydrophilic amino acids can occur in a particular position. During the process of iterative multimerization one can screen the resulting library members for other characteristics like solubility or protease resistance in addition to a screen for high-level expression.

Once the gene that encodes the XTEN of desired length and properties is selected, it is genetically fused at the desired location to the nucleotides encoding the GLP-2 gene(s) by cloning it into the construct adjacent and in frame with the gene coding for GLP-2, or alternatively in frame with nucleotides encoding a spacer/cleavage sequence linked to a terminal XTEN. The invention provides various permutations of the foregoing, depending on the GLP2-XTEN to be encoded. For example, a gene encoding a GLP2-XTEN fusion protein comprising a GLP-2 and two XTEN, such as embodied by formula III, as depicted above, the gene would have polynucleotides encoding GLP-2, and polynucleotides encoding two XTEN, which can be identical or different in composition and sequence length. In one non-limiting embodiment of the foregoing, the GLP-2 polynucleotides would encode native GLP-2 and the polynucleotides encoding the C-terminus XTEN would encode AE864 and the polynucleotides encoding an N-terminal XTEN AE912. The step of cloning the GLP-2 genes into the XTEN construct can occur through a ligation or multimerization step, as shown in FIG. 5 in a schematic flowchart of representative steps in the assembly of a GLP2-XTEN polynucleotide construct. Individual oligonucleotides **501** are annealed into sequence motifs **502** such as a 12 amino acid motif ("12-mer"), which is ligated to additional sequence motifs from a library that can multimerize to create a pool that encompasses the desired length of the XTEN **504,** as well as ligated to a smaller concentration of an oligo containing BbsI, and KpnI restriction sites **503.** The motif libraries can be limited to specific sequence XTEN families; e.g., AD, AE, AF, AG, AM, or AQ sequences of Table 3. As illustrated in FIG. 5, the XTEN polynucleotides encode a length, in this case, of 36 amino acid residues, but longer lengths can be achieved by this process. For example, multimerization can be performed by ligation, overlap extension, PCR assembly or similar cloning techniques known in the art. The resulting pool of ligation products is gel-purified and the band with the desired length of XTEN is cut, resulting in an isolated XTEN gene with a stopper sequence **505.** The XTEN gene can be cloned into a stuffer vector. In this case, the vector encodes an optional CBD sequence **506** and a GFP gene **508.** Digestion is than performed with BbsI/HindIII to remove **507** and **508** and place the stop codon. The resulting product is then cloned into a BsaI/HindIII digested vector containing a gene encoding the GLP-2, resulting in the gene **500** encoding a GLP2-XTEN fusion protein. As would be apparent to one of ordinary skill in the art, the methods can be applied to create constructs in alternative configurations and with varying XTEN lengths.

The constructs encoding GLP2-XTEN fusion proteins can be designed in different configurations of the components XTEN, GLP-2, and spacer sequences, such as shown in FIG. 8. In one embodiment, the construct comprises polynucleotide sequences complementary to, or those that encode a monomeric polypeptide of components in the following order (5' to 3') GLP-2 and XTEN. In another embodiment, the construct comprises polynucleotide sequences complementary to, or those that encode a monomeric polypeptide of components in the following order (5' to 3') XTEN and GLP-2. In another embodiment, the construct comprises polynucleotide sequences complementary to, or those that encode a monomeric polypeptide of components in the following order (5' to 3') XTEN, GLP-2, and a second XTEN. In another embodiment, the construct comprises polynucleotide sequences complementary to, or those that encode a monomeric polypeptide of components in the following order (5' to 3') GLP-2, spacer sequence, and XTEN. In another embodiment, the construct comprises polynucleotide sequences complementary to, or those that encode a monomeric polypeptide of components in the following order (5' to 3') XTEN, spacer sequence, and GLP-2. The spacer polynucleotides can optionally comprise sequences encoding cleavage sequences. As will be apparent to those of skill in the art, other permutations or multimers of the foregoing are possible.

The invention also encompasses polynucleotides comprising XTEN-encoding polynucleotide variants that have a high percentage of sequence identity compared to (a) a polynucleotide sequence from Table 7, or (b) sequences that are complementary to the polynucleotides of (a). A polynucleotide with a high percentage of sequence identity is one that has at least about an 80% nucleic acid sequence identity, alternatively at least about 81%, alternatively at least about 82%, alternatively at least about 83%, alternatively at least about 84%, alternatively at least about 85%, alternatively at least about 86%, alternatively at least about 87%, alternatively at least about 88%, alternatively at least about 89%, alternatively at least about 90%, alternatively at least about 91%, alternatively at least about 92%, alternatively at least about 93%, alternatively at least about 94%, alternatively at least about 95%, alternatively at least about 96%, alternatively at least about 97%, alternatively at least about 98%, and alternatively at least about 99% nucleic acid sequence identity compared to (a) or (b) of the foregoing, or that can hybridize with the target polynucleotide or its complement under stringent conditions.

Homology, sequence similarity or sequence identity of nucleotide or amino acid sequences may also be determined conventionally by using known software or computer programs such as the BestFit or Gap pairwise comparison programs (GCG Wisconsin Package, Genetics Computer Group, 575 Science Drive, Madison, Wis. 53711). BestFit uses the local homology algorithm of Smith and Waterman (Advances in Applied Mathematics. 1981. 2: 482-489), to find the best segment of identity or similarity between two sequences. Gap performs global alignments: all of one sequence with all of another similar sequence using the method of Needleman and Wunsch, (Journal of Molecular Biology. 1970. 48:443-453). When using a sequence alignment program such as BestFit, to determine the degree of sequence homology, similarity or identity, the default setting may be used, or an appropriate scoring matrix may be selected to optimize identity, similarity or homology scores.

Nucleic acid sequences that are "complementary" are those that are capable of base-pairing according to the standard Watson-Crick complementarity rules. As used herein, the term "complementary sequences" means nucleic acid sequences that are substantially complementary, as may be assessed by the same nucleotide comparison set forth above, or as defined as being capable of hybridizing to the polynucleotides that encode the GLP2-XTEN sequences under stringent conditions, such as those described herein.

The resulting polynucleotides encoding the GLP2-XTEN chimeric fusion proteins can then be individually cloned into an expression vector. The nucleic acid sequence is inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence (FIG. 9). Construction of suitable vectors containing one or more of these components employs standard ligation techniques which are known to the skilled artisan. Such techniques are well known in the art and well described in the scientific and patent literature.

Various vectors are publicly available. The vector may, for example, be in the form of a plasmid, cosmid, viral particle, or phage that may conveniently be subjected to recombinant DNA procedures, and the choice of vector will often depend on the host cell into which it is to be introduced. Thus, the vector may be an autonomously replicating vector, i.e., a vector, which exists as an extrachromosomal entity, the replication of which is independent of chromosomal replication, e.g., a plasmid. Alternatively, the vector may be one which, when introduced into a host cell, is integrated into the host cell genome and replicated together with the chromosome(s) into which it has been integrated.

The invention provides for the use of plasmid vectors containing replication and control sequences that are compatible with and recognized by the host cell, and are operably linked to the GLP2-XTEN gene for controlled expression of the GLP2-XTEN fusion proteins. The vector ordinarily carries a replication site, as well as sequences that encode proteins that are capable of providing phenotypic selection in transformed cells. Such vector sequences are well known for a variety of bacteria, yeast, and viruses. Useful expression vectors that can be used include, for example, segments of chromosomal, non-chromosomal and synthetic DNA sequences. "Expression vector" refers to a DNA construct containing a DNA sequence that is operably linked to a suitable control sequence capable of effecting the expression of the DNA encoding the fusion protein in a suitable host. The requirements are that the vectors are replicable and viable in the host cell of choice. Low- or high-copy number vectors may be used as desired.

Suitable vectors include, but are not limited to, derivatives of SV40 and pcDNA and known bacterial plasmids such as col EI, pCRI, pBR322, pMal-C2, pET, pGEX as described by Smith, et al., Gene 57:31-40 (1988), pMB9 and derivatives thereof, plasmids such as RP4, phage DNAs such as the numerous derivatives of phage I such as NM98 9, as well as other phage DNA such as M13 and filamentous single stranded phage DNA; yeast plasmids such as the 2 micron plasmid or derivatives of the 2m plasmid, as well as centomeric and integrative yeast shuttle vectors; vectors useful in eukaryotic cells such as vectors useful in insect or mammalian cells; vectors derived from combinations of plasmids and phage DNAs, such as plasmids that have been modified to employ phage DNA or the expression control sequences; and the like. Yeast expression systems that can also be used in the present invention include, but are not limited to, the non-fusion pYES2 vector (Invitrogen), the fusion pYESHisA, B, C (Invitrogen), pRS vectors and the like.

The control sequences of the vector include a promoter to effect transcription, an optional operator sequence to control such transcription, a sequence encoding suitable mRNA ribosome binding sites, and sequences that control termination of transcription and translation. The promoter may be any DNA sequence, which shows transcriptional activity in the host cell of choice and may be derived from genes encoding proteins either homologous or heterologous to the host cell.

Examples of suitable promoters for directing the transcription of the DNA encoding the GLP2-XTEN in mammalian cells are the SV40 promoter (Subramani et al., Mol. Cell. Biol. 1 (1981), 854-864), the MT-1 (metallothionein gene) promoter (Palmiter et al., Science 222 (1983), 809-814), the CMV promoter (Boshart et al., Cell 41:521-530, 1985) or the adenovirus 2 major late promoter (Kaufman and Sharp, Mol. Cell. Biol, 2:1304-1319, 1982). The vector may also carry sequences such as UCOE (ubiquitous chromatin opening elements).

Examples of suitable promoters for use in filamentous fungus host cells are, for instance, the ADH3 promoter or the tpiA promoter. Examples of other useful promoters are those derived from the gene encoding *A. oryzae* TAKA amylase, *Rhizomucor miehei* aspartic proteinase, *A. niger* neutral α-amylase, *A. niger* acid stable α-amylase, *A. niger* or *A. awamoriglucoamylase* (gluA), *Rhizomucor miehei* lipase, *A. oryzae* alkaline protease, *A. oryzae* triose phosphate isomerase or *A. nidulans* acetamidase. Preferred are the TAKA-amylase and gluA promoters. Yeast expression systems that can also be used in the present invention include, but are not limited to, the non-fusion pYES2 vector (Invitrogen), the fusion pYESHisA, B, C (Invitrogen), pRS vectors and the like.

Promoters suitable for use in expression vectors with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)], all is operably linked to the DNA encoding GLP2-XTEN polypeptides. Promoters for use in bacterial systems can also contain a Shine-Dalgarno (S.D.) sequence, operably linked to the DNA encoding GLP2-XTEN polypeptides.

The invention contemplates use of other expression systems including, for example, a baculovirus expression system with both non-fusion transfer vectors, such as, but not limited to pVL941 Summers, et al., Virology 84:390-402 (1978)), pVL1393 (Invitrogen), pVL1392 (Summers, et al., Virology 84:390-402 (1978) and Invitrogen) and pBlueBacIII (Invitrogen), and fusion transfer vectors such as, but not limited to, pAc7 00 (Summers, et al., Virology 84:390-402 (1978)), pAc701 and pAc70-2 (same as pAc700, with different reading frames), pAc360 Invitrogen) and pBlueBacHisA, B, C (Invitrogen) can be used.

The DNA sequences encoding the GLP2-XTEN may also, if necessary, be operably connected to a suitable terminator, such as the hGH terminator (Palmiter et al., Science 222, 1983, pp. 809-814) or the TPI1 terminators (Alber and Kawasaki, J. Mol. Appl. Gen. 1, 1982, pp. 419-434) or ADH3 (McKnight et al., The EMBO J. 4, 1985, pp. 2093-2099). Expression vectors may also contain a set of RNA splice sites located downstream from the promoter and upstream from the insertion site for the GLP2-XTEN sequence itself, including splice sites obtained from adenovirus. Also contained in the expression vectors is a polyadenylation signal located downstream of the insertion site. Particularly preferred polyadenylation signals include the early or late polyadenylation signal from SV40 (Kaufman and Sharp, ibid.), the polyadenylation signal from the adenovirus 5 Elb region, the hGH terminator (DeNoto et al. Nucl. Acids Res. 9:3719-3730, 1981). The expression vectors may also include a noncoding viral leader sequence, such as the adenovirus 2 tripartite leader, located between the promoter and the RNA splice sites; and enhancer sequences, such as the SV40 enhancer.

In one embodiment, the polynucleotide encoding a GLP2-XTEN fusion protein composition is fused C-terminally to an N-terminal signal sequence appropriate for the expression host system. Signal sequences are typically proteolytically removed from the protein during the translocation and secretion process, generating a defined N-terminus. A wide variety of signal sequences have been described for most expression systems, including bacterial, yeast, insect, and mammalian systems. A non-limiting list of preferred examples for each expression system follows herein. Preferred signal sequences are OmpA, PhoA, and DsbA for *E. coli* expression. Signal peptides preferred for yeast expression are ppL-alpha, DEX4, invertase signal peptide, acid phosphatase signal peptide, CPY, or INU1. For insect cell expression the preferred signal sequences are sexta adipokinetic hormone precursor, CP1, CP2, CP3, CP4, TPA, PAP, or gp67. For mammalian expression the preferred signal sequences are IL2L, SV40, IgG kappa and IgG lambda.

In another embodiment, a leader sequence, potentially comprising a well-expressed, independent protein domain, can be fused to the N-terminus of the GLP2-XTEN sequence, separated by a protease cleavage site. While any leader peptide sequence which does not inhibit cleavage at the designed proteolytic site can be used, sequences in preferred embodiments will comprise stable, well-expressed sequences such that expression and folding of the overall composition is not significantly adversely affected, and preferably expression, solubility, and/or folding efficiency are significantly improved. A wide variety of suitable leader sequences have been described in the literature. A non-limiting list of suitable sequences includes maltose binding protein, cellulose binding domain, glutathione S-transferase, 6xHis tag, FLAG tag, hemaglutinin tag, and green fluorescent protein. The leader sequence can also be further improved by codon optimization, especially in the second codon position following the ATG start codon, by methods well described in the literature and hereinabove.

The procedures used to ligate the DNA sequences coding for the GLP2-XTEN, the promoter and optionally the terminator and/or secretory signal sequence, respectively, and to insert them into suitable vectors containing the information necessary for replication, are well known to persons skilled in the art (cf., for instance, Sambrook, J. et al., "Molecular Cloning: A Laboratory Manual," 3rd edition, Cold Spring Harbor Laboratory Press, 2001).

In other embodiments, the invention provides constructs and methods of making constructs comprising an polynucleotide sequence optimized for expression that encodes at least about 20 to about 60 amino acids with XTEN characteristics that can be included at the N-terminus of an XTEN carrier encoding sequence (in other words, the polynucleotides encoding the 20-60 encoded optimized amino acids are linked in frame to polynucleotides encoding an XTEN component that is N-terminal to GLP-2) to promote the initiation of translation to allow for expression of XTEN fusions at the N-terminus of proteins without the presence of a helper domain. In an advantage of the foregoing, the sequence does not require subsequent cleavage, thereby reducing the number of steps to manufacture XTEN-containing compositions. As described in more detail in the Examples, the optimized N-terminal sequence has attributes of an unstructured protein, but may include nucleotide bases encoding amino acids selected for their ability to promote initiation of translation and enhanced expression. In one embodiment of the foregoing, the optimized polynucleotide encodes an XTEN sequence with at least about 90% sequence identity compared to AE912. In another embodiment of the foregoing, the optimized polynucleotide encodes an XTEN sequence with at least about 90% sequence identity compared to AM923. In another embodiment of the foregoing, the optimized polynucleotide encodes an XTEN sequence with at least about 90% sequence identity compared to AE48. In another embodiment of the foregoing, the optimized polynucleotide encodes an XTEN sequence with at least about 90% sequence identity compared to AM48. In one embodiment, the optimized polynucleotide NTS comprises a sequence that exhibits at least about 80%, at least about 85%, at least about 90%, at least about 91%, at least about 92%, at least about 93%, at least about 94%, at least about 95%, at least about 96%, at least about 97%, at least about 98%, or at least about 99%, sequence identity compared to a sequence or its complement selected from AE 48: 5'-

ATGGCTGAACCTGCTGGCTCTCCAACCTCCACTGAGGAAGGTACCCCGGGTAGCGGTACTGC
TTCTTCCTCTCCAGGTAGCTCTACCCCTTCTGGTGCAACCGGCTCTCCAGGTGCTTCTCCGGG
CACCAGCTCTACCGGTTCTCCA-3'

and
AM 48: 5'-

ATGGCTGAACCTGCTGGCTCTCCAACCTCCACTGAGGAAGGTGCATCCCCGGGCACCAGCTC
TACCGGTTCTCCAGGTAGCTCTACCCCGTCTGGTGCTACCGGCTCTCCAGGTAGCTCTACCCC
GTCTGGTGCTACTGGCTCTCCA-3'.

In this manner, a chimeric DNA molecule coding for a monomeric GLP2-XTEN fusion protein is generated. Optionally, this chimeric DNA molecule may be transferred or cloned into another construct that is a more appropriate expression vector. At this point, a host cell capable of expressing the chimeric DNA molecule can be transformed with the chimeric DNA molecule. The vectors containing the DNA segments of interest can be transferred into the host cell by well-known methods, depending on the type of cellular host. For example, calcium chloride transfection is commonly utilized for prokaryotic cells, whereas calcium phosphate treatment, lipofection, or electroporation may be used for other cellular hosts. Other methods used to transform mammalian cells include the use of polybrene, protoplast fusion, liposomes, electroporation, and microinjection. *See,* generally, Sambrook, *et al., supra.*

The transformation may occur with or without the utilization of a carrier, such as an expression vector. Then, the transformed host cell is cultured under conditions suitable for the expression of the chimeric DNA molecule encoding of GLP2-XTEN.

The present invention also provides a host cell for expressing the monomeric fusion protein compositions disclosed herein. Examples of mammalian cell lines for use in the present invention are the COS-1 (ATCC CRL 1650), COS-7 (ATCC CRL 1651), BHK-21 (ATCC CCL 10)) and BHK-293 (ATCC CRL 1573; Graham et al., J. Gen. Virol. 36:59-72, 1977), BHK-570 cells (ATCC CRL 10314), CHO-K1 (ATCC CCL 61), CHO-S (Invitrogen 11619-012), and 293-F (Invitrogen R790-7). A tk-ts13 BHK cell line is also available from the ATCC under accession number CRL 1632. In addition, a number of other cell lines may be used within the present invention, including Rat Hep I (Rat hepatoma; ATCC CRL 1600), Rat Hep II (Rat hepatoma; ATCC CRL 1548), TCMK (ATCC CCL 139), Human lung (ATCC HB 8065), NCTC 1469 (ATCC CCL 9.1), CHO (ATCC CCL 61) and DUKX cells (Urlaub and Chasin, Proc. Natl. Acad. Sci. USA 77:4216-4220, 1980).

Examples of suitable yeasts host cells include cells of *Saccharomyces* spp. or *Schizosaccharomyces* spp., in particular strains of *Saccharomyces cerevisiae* or *Saccharomyces kluyveri.* Other yeasts include *Schizosaccharomyces pombe* (Beach and Nurse, Nature, 290: 140 [1981]; EP 139,383 published 2 May 1985); *Kluyveromyces* hosts (U.S. Pat. No. 4,943,529; Fleer et al., Bio/Technology, 9:968-975 (1991)) such as, e.g., *K. lactis* (MW98-8C, CBS683, CBS4574; Louvencourt et al., J. Bacteriol., 737 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans*, and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 Oct. 1990). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* Further examples of suitable yeast cells are strains of *Kluyveromyces,* such as *Hansenula* , e.g. *H. polymorpha*, or *Pichia*, e.g. *P. pastoris* (cf. Gleeson et al., J. Gen. Microbiol. 132, 1986, pp. 3459-3465; U.S. Pat. No. 4,882,279). A list of specific species that are exemplary of this class of yeasts may be found in C. Anthony, The Biochemistry of Methylotrophs, 269 (1982). Methods for transforming yeast cells with heterologous DNA and producing heterologous polypeptides there from are described, e.g. in U.S. Pat. No. 4,599,311, U.S. Pat. No. 4,931,373, U.S. Pat. No. 4,870,008, 5,037,743, and U.S. Pat. No. 4,845,075, all of which are hereby incorporated by reference.

Examples of other fungal cells are cells of filamentous fungi, e.g. *Aspergillus* spp., *Neurospora* spp., *Fusarium* spp. or *Trichoderma* spp., in particular strains of *A. oryzae, A. nidulans* or *A. niger.* The use of *Aspergillus* spp. for the expression of proteins is described in, e.g., EP 272 277, EP 238 023, EP 184 438 The transformation of *F. oxysporum* may, for instance, be carried out as described by Malardier et al., 1989 , Gene 78: 147-156. The transformation of *Trichoderma* spp. may be performed for instance as described in EP 244 234.

Other suitable cells that can be used in the present invention include, but are not limited to, prokaryotic host cells strains such as *Escherichia coli, (*e.g., strain DH5-α), *Bacillus subtilis, Salmonella typhimurium,* or strains of the genera of *Pseudomonas, Streptomyces and Staphylococcus.* Non-limiting examples of suitable prokaryotes include those from the genera: *Actinoplanes; Archaeoglobus; Bdellovibrio; Borrelia; Chloroflexus; Enterococcus; Escherichia; Lactobacillus; Listeria; Oceanobacillus; Paracoccus; Pseudomonas; Staphylococcus; Streptococcus; Streptomyces; Thermoplasma; and Vibrio.*

Transformed cells are selected by a phenotype determined by a selectable marker, commonly drug resistance or the ability to grow in the absence of a particular nutrient, e.g. leucine. A preferred vector for use in yeast is the POT1 vector disclosed in U.S. Pat. No. 4,931,373. The DNA sequences encoding the GLP2-XTEN may be preceded by a signal sequence and optionally a leader sequence, e.g. as described above. Methods of transfecting mammalian cells and expressing DNA sequences introduced in the cells are described in e.g., Kaufman and Sharp, J. Mol. Biol. 159 (1982), 601-621; Southern and Berg, J. Mol. Appl. Genet. 1 (1982), 327-341; Loyter et al., Proc. Natl. Acad. Sci. USA 79 (1982), 422-426; Wigler et al., Cell 14 (1978), 725; Corsaro and Pearson, Somatic Cell Genetics 7 (1981), 603, Graham and van der Eb, Virology 52 (1973), 456; and Neumann et al., EMBO J. 1 (1982), 841-845.

Cloned DNA sequences are introduced into cultured mammalian cells by, for example, calcium phosphate-mediated transfection (Wigler et al., Cell 14:725-732, 1978; Corsaro and Pearson, Somatic Cell Genetics 7:603-616, 1981; Graham and Van der Eb, Virology 52d:456-467, 1973), transfection with many commercially available reagents such as FuGENEG Roche Diagnostics, Mannheim, Germany) or lipofectamine (Invitrogen) or by electroporation (Neumann et al., EMBO J. 1:841-845, 1982). To identify and select cells that express the exogenous DNA, a gene that confers a selectable phenotype (a selectable marker) is generally introduced into cells along with the gene or cDNA of interest. Preferred selectable markers include genes that confer resistance to drugs such as neomycin, hygromycin, puromycin, zeocin, and methotrexate. The selectable marker may be an amplifiable selectable marker. A preferred amplifiable selectable marker is a dihydrofolate reductase (DHFR) sequence. Further examples of selectable markers are well known to one of skill in the art and include reporters such as enhanced green fluorescent protein (EGFP), beta-galactosidase (β-gal) or chloramphenicol acetyltransferase (CAT). Selectable markers are reviewed by Thilly (Mammalian Cell Technology, Butterworth Publishers, Stoneham, Mass., incorporated herein by reference). A person skilled in the art will easily be able to choose suitable selectable markers. Any known selectable marker may be employed so long as it is capable of being expressed simultaneously with the nucleic acid encoding a gene product.

Selectable markers may be introduced into the cell on a separate plasmid at the same time as the gene of interest, or they may be introduced on the same plasmid. On the same plasmid, the selectable marker and the gene of interest may be under the control of different promoters or the same promoter, the latter arrangement produces a dicistronic message. Constructs of this type are known in the art (for example, Levinson and Simonsen, U.S. Pat. No. 4,713,339). It may also be advantageous to add additional DNA, known as "carrier DNA," to the mixture that is introduced into the cells.

After the cells have taken up the DNA, they are grown in an appropriate growth medium, typically 1-2 days, to begin expressing the gene of interest. As used herein the term "appropriate growth medium" means a medium containing nutrients and other components required for the growth of cells and the expression of the GLP2-XTEN of interest. Media generally include a carbon source, a nitrogen source, essential amino acids, essential sugars, vitamins, salts, phospholipids, protein and growth factors. For production of gamma-carboxylated proteins, the medium will contain vitamin K, preferably at a concentration of about 0.1 $\mu$g/ml to about 5 $\mu$g/ml. Drug selection is then applied to select for the growth of cells that are expressing the selectable marker in a stable fashion. For cells that have been transfected with an amplifiable selectable marker the drug concentration may be increased to select for an increased copy number of the cloned sequences, thereby increasing expression levels. Clones of stably transfected cells are then screened for expression of the GLP-2 polypeptide variant of interest.

The transformed or transfected host cell is then cultured in a suitable nutrient medium under conditions permitting expression of the GLP2-XTEN fusion protein after which the resulting peptide may be recovered from the culture. The medium used to culture the cells may be any conventional medium suitable for growing the host cells, such as minimal or complex media containing appropriate supplements. Suitable media are available from commercial suppliers or may be prepared according to published recipes (e.g. in catalogues of the American Type Culture Collection). The culture conditions, such as temperature, pH and the like, are those previously used with the host cell selected for expression, and will be apparent to the ordinarily skilled artisan.

Gene expression may be measured in a sample directly, for example, by conventional Northern blotting to quantitate the transcription of mRNA [Thomas, Proc. Natl. Acad. Sci. USA, 77:5201-5205 (1980)], dot blotting (DNA analysis), or in situ hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological of fluorescent methods, such as immunohistochemical staining of cells or tissue sections and assay of cell culture or body fluids or the detection of selectable markers, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence GLP-2 polypeptide or against a synthetic peptide based on the DNA sequences provided herein or against exogenous sequence fused to GLP-2 and encoding a specific antibody epitope. Examples of selectable markers are well known to one of skill in the art and include reporters such as enhanced green fluorescent protein (EGFP), beta-galactosidase ($\beta$-gal) or chloramphenicol acetyltransferase (CAT).

Expressed GLP2-XTEN polypeptide product(s) may be purified via methods known in the art or by methods disclosed herein. Procedures such as gel filtration, affinity purification (e.g., using an anti-GLP-2 antibody column), salt fractionation, ion exchange chromatography, size exclusion chromatography, hydroxyapatite adsorption chromatography, hydrophobic interaction chromatography and gel electrophoresis may be used; each tailored to recover and purify the fusion protein produced by the respective host cells. Additional purification may be achieved by conventional chemical purification means, such as high performance liquid chromatography. Some expressed GLP2-XTEN may require refolding during isolation and purification. Methods of purification are described in Robert K. Scopes, Protein Purification: Principles and Practice, Charles R. Castor (ed.), Springer-Verlag 1994, and Sambrook, *et al., supra.* Multi-step purification separations are also described in Baron, et al., Crit. Rev. Biotechnol. 10:179-90 (1990) and Below, et al., J. Chromatogr. A. 679:67-83 (1994). For therapeutic purposes it is preferred that the GLP2-XTEN fusion proteins of the invention are substantially pure. Thus, in a preferred embodiment of the invention the GLP2-XTEN of the invention is purified to at least about 90 to 95% homogeneity, preferably to at least about 98% homogeneity. Purity may be assessed by, e.g., gel electrophoresis, HPLC, and amino-terminal amino acid sequencing.

## VIII). PHARMACEUTICAL COMPOSITIONS

The present invention provides pharmaceutical compositions comprising GLP2-XTEN. In one embodiment, the pharmaceutical composition comprises a GLP2-XTEN fusion protein disclosed herein and at least one pharmaceutically acceptable carrier. GLP2-XTEN polypeptides of the present invention can be formulated according to known methods to prepare pharmaceutically useful compositions, whereby the polypeptide is combined in admixture with a pharmaceutically acceptable carrier vehicle, such as aqueous solutions, buffers, solvents and/or pharmaceutically acceptable suspensions, emulsions, stabilizers or excipients. Examples of non-aqueous solvents include propylethylene glycol, polyethylene glycol and vegetable oils. Formulations of the pharmaceutical compositions are prepared for storage by mixing the active GLP2-XTEN ingredient having the desired degree of purity with optional physiologically acceptable carriers, excipients (e.g., sodium chloride, a calcium salt, sucrose, or polysorbate) or stabilizers (e.g., sucrose, trehalose, raffinose, arginine, a calcium salt, glycine or histidine), as described in Remington's Pharmaceutical Sciences 16th edition, Osol, A. Ed. (1980), in the form of lyophilized formulations or aqueous solutions.

In one embodiment, the pharmaceutical composition may be supplied as a lyophilized powder to be reconstituted prior to administration. In another embodiment, the pharmaceutical composition may be supplied in a liquid form, which can be administered directly to a patient. In another embodiment, the composition is supplied as a liquid in a pre-filled syringe for administration of the composition. In another embodiment, the composition is supplied as a liquid in a pre-filled vial that can be incorporated into a pump.

The pharmaceutical compositions can be administered by any suitable means or route, including subcutaneously, subcutaneously by infusion pump, intramuscularly, intravenously, or via the pulmonary route. It will be appreciated that the preferred route will vary with the disease and age of the recipient, and the severity of the condition being treated.

In one embodiment, the GLP2-XTEN pharmaceutical composition in liquid form or after reconstitution (when supplied as a lyophilized powder) comprises GLP-2 linked to XTEN, which composition is capable of increasing GLP-2-related activity to at least 10% of the normal GLP-2 plasma level in the blood for at least about 72 hours, or at least about 96 hours, or at least about 120 hours, or at least about 7 days, or at least about 10 days, or at least about 14 days, or at least about 21 days after administration of the GLP-2 pharmaceutical composition to a subject in need. In another embodiment, the GLP2-XTEN pharmaceutical composition in liquid form or after reconstitution (when supplied as a lyophilized powder) and administration to a subject is capable of increasing GLP2-XTEN concentrations to at least 500 ng/ml, or at least 1000 ng/ml, or at least about 2000 ng/ml, or at least about 3000 ng/ml, or at least about 4000 ng/ml, or at least about 5000 ng/ml, or at least about 10000 ng/ml, or at least about 15000 ng/ml, or at least about 20000 ng/ml, or at least about 30000 ng/ml, or at least about 40000 ng/ml for at least about 24 hours, or at least about 48 hours, or at least about 72 hours, or at least about 96 hours, or at least about 120 hours, or at least about 144 hours after administration of the GLP-2 pharmaceutical composition to a subject in need. It is specifically contemplated that the pharmaceutical compositions of the foregoing embodiments in this paragraph can be formulated to include one or more excipients, buffers or other ingredients known in the art to be compatible with administration by the intravenous route or the subcutaneous route or the intramuscular route. Thus, in the embodiments hereinabove described in this paragraph, the pharmaceutical composition is administered subcutaneously, intramuscularly, or intravenously.

The compositions of the invention may be formulated using a variety of excipients. Suitable excipients include microcrystalline cellulose (e.g. Avicel PH102, Avicel PH101), polymethacrylate, poly(ethyl acrylate, methyl methacrylate, trimethylammonioethyl methacrylate chloride) (such as Eudragit RS-30D), hydroxypropyl methylcellulose (Methocel K100M, Premium CR Methocel K100M, Methocel E5, Opadry®), magnesium stearate, talc, triethyl citrate, aqueous ethylcellulose dispersion (Surelease®), and protamine sulfate. The slow release agent may also comprise a carrier, which can comprise, for example, solvents, dispersion media, coatings, antibacterial and antifungal agents, isotonic and absorption delaying agents. Pharmaceutically acceptable salts can also be used in these slow release agents, for example, mineral salts such as hydrochlorides, hydrobromides, phosphates, or sulfates, as well as the salts of organic acids such as acetates, proprionates, malonates, or benzoates. The composition may also contain liquids, such as water, saline, glycerol, and ethanol, as well as substances such as wetting agents, emulsifying agents, or pH buffering agents. Liposomes may also be used as a carrier.

In another embodiment, the compositions of the present invention are encapsulated in liposomes, which have demonstrated utility in delivering beneficial active agents in a controlled manner over prolonged periods of time. Liposomes are closed bilayer membranes containing an entrapped aqueous volume. Liposomes may also be unilamellar vesicles possessing a single membrane bilayer or multilamellar vesicles with multiple membrane bilayers, each separated from the next by an aqueous layer. The structure of the resulting membrane bilayer is such that the hydrophobic (non-polar) tails of the lipid are oriented toward the center of the bilayer while the hydrophilic (polar) heads orient towards the aqueous phase. In one embodiment, the liposome may be coated with a flexible water soluble polymer that avoids uptake by the organs of the mononuclear phagocyte system, primarily the liver and spleen. Suitable hydrophilic polymers for surrounding the liposomes include, without limitation, PEG, polyvinylpyrrolidone, polyvinylmethylether, polymethyloxazoline, polyethyloxazoline, polyhydroxypropyloxazoline, polyhydroxypropylmethacrylamide, polymethacrylamide, polydimethylacrylamide, polyhydroxypropylmethacrylate, polyhydroxethylacrylate, hydroxymethylcellulose hydroxyethylcellulose, polyethyleneglycol, polyaspartamide and hydrophilic peptide sequences as described in U.S. Pat. Nos. 6,316,024; 6,126,966; 6,056,973; 6,043,094, the contents of which are incorporated by reference in their entirety.

Liposomes may be comprised of any lipid or lipid combination known in the art. For example, the vesicle-forming lipids may be naturally-occurring or synthetic lipids, including phospholipids, such as phosphatidylcholine, phosphatidylethanolamine, phosphatidic acid, phosphatidylserine, phasphatidylglycerol, phosphatidylinositol, and sphingomyelin as disclosed in U.S. Pat. Nos. 6,056,973 and 5,874,104. The vesicle-forming lipids may also be glycolipids, cerebrosides, or cationic lipids, such as 1,2-dioleyloxy-3-(trimethylamino) propane (DOTAP); N-[1-(2,3,-ditetradecyloxy)propyl]-N,N-dimethyl-N-hydroxyethylammonium bromide (DMRIE); N-[1[(2,3,-dioleyloxy)propyl]-N,N-dimethyl-N-hydroxy ethylammonium bromide (DORIE); N-[1-(2,3-dioleyloxy)propyl]-N,N,N-trimethylammonium chloride (DOTMA); 3 [N-(N',N'-dimethylaminoethane) carbamoly] cholesterol (DC-Chol); or dimethyldioctadecylammonium (DDAB) also as disclosed in U.S. Pat. No. 6,056,973. Cholesterol may also be present in the proper range to impart stability to the vesicle as disclosed in U.S. Pat. Nos. 5,916,588 and 5,874,104.

Additional liposomal technologies are described in U.S. Pat. Nos. 6,759,057; 6,406,713; 6,352,716; 6,316,024; 6,294,191; 6,126,966; 6,056,973; 6,043,094; 5,965,156; 5,916,588; 5,874,104; 5,215,680; and 4,684,479, the contents of which are incorporated herein by reference. These describe liposomes and lipid-coated microbubbles, and methods for their manufacture. Thus, one skilled in the art, considering both the disclosure of this invention and the disclosures of these other patents could produce a liposome for the extended release of the polypeptides of the present invention.

For liquid formulations, a desired property is that the formulation be supplied in a form that can pass through a 25, 28, 30, 31, 32 gauge needle for intravenous, intramuscular, intraarticular, or subcutaneous administration. In another embodiment, a desired property is that the formulation be supplied in a form that can be nebulized into an aerosal of suitable particle size for inhalation therapy.

Osmotic pumps may be used as slow release agents in the form of tablets, pills, capsules or implantable devices. Osmotic pumps are well known in the art and readily available to one of ordinary skill in the art from companies experienced in providing osmotic pumps for extended release drug delivery. Examples are ALZA's DUROS™; ALZA's OROS™; Osmotica Pharmaceutical's Osmodex™ system; Shire Laboratories' EnSoTrol™ system; and Alzet™. Patents that describe osmotic pump technology are U.S. Pat. Nos. 6,890,918; 6,838,093; 6,814,979; 6,713,086; 6,534,090; 6,514,532; 6,361,796; 6,352,721; 6,294,201; 6,284,276; 6,110,498; 5,573,776; 4,200,0984; and 4,088,864, the contents of which are incorporated herein by reference. One skilled in the art, considering both the disclosure of this invention and the disclosures of these other patents could produce an osmotic pump for the extended release of the polypeptides of the present invention.

Syringe pumps may also be used as slow release agents. Such devices are described in U.S. Pat. Nos. 4,976,696; 4,933,185; 5,017,378; 6,309,370; 6,254,573; 4,435,173; 4,398,908; 6,572,585; 5,298,022; 5,176,502; 5,492,534; 5,318,540; and 4,988,337, the contents of which are incorporated herein by reference. One skilled in the art, considering both the disclosure of this invention and the disclosures of these other patents could produce a syringe pump for the extended release of the compositions of the present invention.

### IX). PHARMACEUTICAL KITS

In another aspect, the invention provides a kit to facilitate the use of the GLP2-XTEN polypeptides. The kit comprises the pharmaceutical composition provided herein, a label identifying the pharmaceutical composition, and an instruction for storage, reconstitution and/or administration of the pharmaceutical compositions to a subject. In some embodiment, the kit comprises, preferably: (a) an amount of a GLP2-XTEN fusion protein composition sufficient to treat a gastrointestinal condition upon administration to a subject in need thereof; (b) an amount of a pharmaceutically acceptable carrier; and (c) together in a formulation ready for injection or for reconstitution with sterile water, buffer, or dextrose; together with a label identifying the GLP2-XTEN drug and storage and handling conditions, and a sheet of the approved indications for the drug, instructions for the reconstitution and/or administration of the GLP2-XTEN drug for the use for the prevention and/or treatment of an approved indication, appropriate dosage and safety information, and information identifying the lot and expiration of the drug. In another embodiment of the foregoing, the kit can comprise a second container that can carry a suitable diluent for the GLP2-XTEN composition, the use of which will provide the user with the appropriate concentration of GLP2-XTEN to be delivered to the subject.

### EXAMPLES

### Example 1: Construction of XTEN_AD36 motif segments

The following example describes the construction of a collection of codon-optimized genes encoding motif sequences of 36 amino acids. As a first step, a stuffer vector pCW0359 was constructed based on a pET vector and that includes a T7 promoter. pCW0359 encodes a cellulose binding domain (CBD) and a TEV protease recognition site followed by a stuffer sequence that is flanked by BsaI, BbsI, and KpnI sites. The BsaI and BbsI sites were inserted such that they generate compatible overhangs after digestion. The stuffer sequence is followed by a truncated version of the GFP gene and a His tag. The stuffer sequence contains stop codons and thus E. coli cells carrying the stuffer plasmid pCW0359 form non-fluorescent colonies. The stuffer vector pCW0359 was digested with BsaI and KpnI to remove the stuffer segment and the resulting vector fragment was isolated by agarose gel purification. The sequences were designated XTEN_AD36, reflecting the AD family of motifs. Its segments have the amino acid sequence $[X]_3$ where X is a 12mer peptide with the sequences: GESPGGSSGSES, GSEGSSGPGESS, GSSESGSSEGGP, or GSGGEPSESGSS. The insert was obtained by annealing the following pairs of phosphorylated synthetic oligonucleotide pairs:

AD1for: AGGTGAATCTCCDGGTGGYTCYAGCGGTTCYGARTC
AD 1 rev: ACCTGAYTCRGAACCGCTRGARCCACCHGGAGATTC
AD2for: AGGTAGCGAAGGTTCTTCYGGTCCDGGYGARTCYTC
AD2rev: ACCTGARGAYTCRCCHGGACCRGAAGAACCTTCGCT
AD3for: AGGTTCYTCYGAAAGCGGTTCTTCYGARGGYGGTCC
AD3rev: ACCTGGACCRCCYTCRGAAGAACCGCTTTCRGARGA
AD4for: AGGTTCYGGTGGYGAACCDTCYGARTCTGGTAGCTC

We also annealed the phosphorylated oligonucleotide 3KpnIstopperFor: AGGTTCGTCTTCACTCGAGGGTAC and the non-phosphorylated oligonucleotide pr_3KpnIstopperRev: CCTCGAGTGAAGACGA. The annealed oligonucleotide pairs were ligated, which resulted in a mixture of products with varying length that represents the varying number of 12mer repeats ligated to one BbsI/KpnI segment. The products corresponding to the length of 36 amino acids were isolated from the mixture by preparative agarose gel electrophoresis and ligated into the BsaI/KpnI digested stuffer vector pCW0359. Most of the clones in the resulting library designated LCW0401 showed green fluorescence after induction, which shows that the sequence of XTEN_AD36 had been ligated in frame with the GFP gene and that most sequences of XTEN_AD36 had good expression levels.

We screened 96 isolates from library LCW0401 for high level of fluorescence by stamping them onto agar plate containing IPTG. The same isolates were evaluated by PCR and 48 isolates were identified that contained segments with 36 amino acids as well as strong fluorescence. These isolates were sequenced and 39 clones were identified that contained correct XTEN_AD36 segments. The file names of the nucleotide and amino acid constructs for these segments are listed in Table 8.

Table 8: DNA and Amino Acid Sequences for 36-mer motifs

| File name | Amino acid sequence | Nucleotide sequence |
|---|---|---|
| LCW0401_001_GFP-N_A01.ab1 | GSGGEPSESGSSGESPGG SSGSESGESPGGSSGSES | GGTTCTGGTGGCGAACCGTCCGAGTCTGGTAGC TCAGGTGAATCTCCGGGTGGCTCTAGCGGTTCC GAGTCAGGTGAATCTCCTGGTGGTTCCAGCGGT TCCGAGTCA |
| LCW0401_002_GFP-N_B01.ab1 | GSEGSSGPGESSGESPGG SSGSESGSSESGSSEGGP | GGTAGCGAAGGTTCTTCTGGTCCTGGCGAGTCT TCAGGTGAATCTCCTGGTGGTTCCAGCGGTTCT GAATCAGGTTCCTCCGAAAGCGGTTCTTCCGAG GGCGGTCCA |
| LCW0401_003_GFP-N_C01.ab1 | GSSESGSSEGGPGSSESG SSEGGPGESPGGSSGSES | GGTTCCTCTGAAAGCGGTTCTTCCGAAGGTGGT CCAGGTTCCTCTGAAAGCGGTTCTTCTGAGGGT GGTCCAGGTGAATCTCCGGGTGGCTCCAGCGGT TCCGAGTCA |
| LCW0401_004_GFP-N_D01.ab1 | GSGGEPSESGSSGSSESG SSEGGPGSGGEPSESGSS | GGTTCCGGTGGCGAACCGTCTGAATCTGGTAGC TCAGGTTCTTCTGAAAGCGGTTCTTCCGAGGGT GGTCCAGGTTCTGGTGGTGAACCTTCCGAGTCT GGTAGCTCA |
| LCW0401_007_GFP-N_F01.ab1 | GSSESGSSEGGPGSEGSS GPGESSGSEGSSGPGESS | GGTTCTTCCGAAAGCGGTTCTTCTGAGGGTGGT CCAGGTAGCGAAGGTTCTTCCGGTCCAGGTGAG TCTTCAGGTAGCGAAGGTTCTTCTGGTCCTGGT GAATCTTCA |
| LCW0401_008_GFP-N_G01.ab1 | GSSESGSSEGGPGESPGG SSGSESGSEGSSGPGESS | GGTTCCTCTGAAAGCGGTTCTTCCGAGGGTGGT CCAGGTGAATCTCCAGGTGGTTCCAGCGGTTCT GAGTCAGGTAGCGAAGGTTCTTCTGGTCCAGGT GAATCCTCA |
| LCW0401_012_GFP-N_H01.ab1 | GSGGEPSESGSSGSGGEP SESGSSGSEGSSGPGESS | GGTTCTGGTGGTGAACCGTCTGAGTCTGGTAGC TCAGGTTCCGGTGGCGAACCATCCGAATCTGGT AGCTCAGGTAGCGAAGGTTCTTCCGGTCCAGGT GAGTCTTCA |
| LCW0401_015_GFP-N_A02.ab1 | GSSESGSSEGGPGSEGSS | GGTTCTTCCGAAAGCGGTTCTTCCGAAGGCGGT |
| | GPGESSGESPGGSSGSES | CCAGGTAGCGAAGGTTCTTCTGGTCCAGGCGAA TCTTCAGGTGAATCTCCTGGTGGCTCCAGCGGT TCTGAGTCA |

| File name | Amino acid sequence | Nucleotide sequence |
|---|---|---|
| LCW0401_016_GFP-N_B02.ab1 | GSSESGSSEGGPGSSESG SSEGGPGSSESGSSEGGP | GGTTCCTCCGAAAGCGGTTCTTCTGAGGGCGGT CCAGGTTCCTCCGAAAGCGGTTCTTCCGAGGGC GGTCCAGGTTCTTCTGAAAGCGGTTCTTCCGAG GGCGGTCCA |
| LCW0401_020_GFP-N_E02.ab1 | GSGGEPSESGSSGSEGSS GPGESSGSSESGSSEGGP | GGTTCCGGTGGCGAACCGTCCGAATCTGGTAGC TCAGGTAGCGAAGGTTCTTCTGGTCCAGGCGAA TCTTCAGGTTCCTCTGAAAGCGGTTCTTCTGAG GGCGGTCCA |
| LCW0401 022_GFP-N_F02.ab1 | GSGGEPSESGSSGSSESG SSEGGPGSGGEPSESGSS | GGTTCTGGTGGTGAACCGTCCGAATCTGGTAGC TCAGGTTCTTCCGAAAGCGGTTCTTCTGAAGGT GGTCCAGGTTCCGGTGGCGAACCTTCTGAATCT GGTAGCTCA |
| LCW0401_024_GFP-N_G02.ab1 | GSGGEPSESGSSGSSESG SSEGGPGESPGGSSGSES | GGTTCTGGTGGCGAACCGTCCGAATCTGGTAGC TCAGGTTCCTCCGAAAGCGGTTCTTCTGAAGGT GGTCCAGGTGAATCTCCAGGTGGTTCTAGCGGT TCTGAATCA |
| LCW0401_026_GFP-N_H02.ab1 | GSGGEPSESGSSGESPGG SSGSESGSEGSSGPGESS | GGTTCTGGTGGCGAACCGTCTGAGTCTGGTAGC TCAGGTGAATCTCCTGGTGGCTCCAGCGGTTCT GAATCAGGTAGCGAAGGTTCTTCTGGTCCTGGT GAATCTTCA |
| LCW0401_027_GFP-N_A03.ab1 | GSGGEPSESGSSGESPGG SSGSESGSGGEPSESGSS | GGTTCCGGTGGCGAACCTTCCGAATCTGGTAGC TCAGGTGAATCTCCGGGTGGTTCTAGCGGTTCT GAGTCAGGTTCTGGTGGTGAACCTTCCGAGTCT GGTAGCTCA |
| LCW0401_028_GFP-N_B03.ab1 | GSSESGSSEGGPGSSESG SSEGGPGSSESGSSEGGP | GGTTCCTCTGAAAGCGGTTCTTCTGAGGGCGGT CCAGGTTCTTCCGAAAGCGGTTCTTCCGAGGGC GGTCCAGGTTCTTCCGAAAGCGGTTCTTCTGAA GGCGGTCCA |
| LCW0401_030_GFP-N_C03.ab1 | GESPGGSSGSESGSEGSS GPGESSGSEGSSGPGESS | GGTGAATCTCCGGGTGGCTCCAGCGGTTCTGAG TCAGGTAGCGAAGGTTCTTCCGGTCCGGGTGAG TCCTCAGGTAGCGAAGGTTCTTCCGGTCCTGGT GAGTCTTCA |

EP 3 431 098 A1

116

(continued)

| File name | Amino acid sequence | Nucleotide sequence |
|---|---|---|
| LCW0401_031_GFP-N_D03.ab1 | GSGGEPSESGSSGSGGEP SESGSSGSSESGSSEGGP | GGTTCTGGTGGCGAACCTTCCGAATCTGGTAGC TCAGGTTCCGGTGGTGAACCTTCTGAATCTGGT AGCTCAGGTTCTTCTGAAAGCGGTTCTTCCGAG GGCGGTCCA |
| LCW0401 _033_GFP-N_E03.ab1 | GSGGEPSESGSSGSGGEP SESGSSGSGGEPSESGSS | GGTTCCGGTGGTGAACCTTCTGAATCTGGTAGC TCAGGTTCCGGTGGCGAACCATCCGAGTCTGGT AGCTCAGGTTCCGGTGGTGAACCATCCGAGTCT GGTAGCTCA |
| LCW0401_037_GFP-N_F03.ab1 | GSGGEPSESGSSGSSESG SSEGGPGSEGSSGPGESS | GGTTCCGGTGGCGAACCTTCTGAATCTGGTAGC TCAGGTTCCTCCGAAAGCGGTTCTTCTGAGGGC GGTCCAGGTAGCGAAGGTTCTTCTGGTCCGGGC GAGTCTTCA |
| LCW0401_038_GFP-N_G03.ab1 | GSGGEPSESGSSGSEGSS GPGESSGSGGEPSESGSS | GGTTCCGGTGGTGAACCGTCCGAGTCTGGTAGC TCAGGTAGCGAAGGTTCTTCTGGTCCGGGTGAG TCTTCAGGTTCTGGTGGCGAACCGTCCGAATCT GGTAGCTCA |
| LCW0401_039_GFP-N_H03.ab1 | GSGGEPSESGSSGESPGG SSGSESGSGGEPSESGSS | GGTTCTGGTGGCGAACCGTCCGAATCTGGTAGC TCAGGTGAATCTCCTGGTGGTTCCAGCGGTTCC GAGTCAGGTTCTGGTGGCGAACCTTCCGAATCT GGTAGCTCA |
| LCW0401_040_GFP-N_A04.ab1 | GSSESGSSEGGPGSGGEP SESGSSGSSESGSSEGGP | GGTTCTTCCGAAAGCGGTTCTTCCGAGGGCGGT CCAGGTTCCGGTGGTGAACCATCTGAATCTGGT AGCTCAGGTTCTTCTGAAAGCGGTTCTTCTGAA GGTGGTCCA |
| LCW0401_042_GFP-N_C04.ab1 | GSEGSSGPGESSGESPGG SSGSESGSEGSSGPGESS | GGTAGCGAAGGTTCTTCCGGTCCTGGTGAGTCT TCAGGTGAATCTCCAGGTGGCTCTAGCGGTTCC GAGTCAGGTAGCGAAGGTTCTTCTGGTCCTGGC GAGTCCTCA |
| LCW0401_046_GFP-N_D04.ab1 | GSSESGSSEGGPGSSESG SSEGGPGSSESGSSEGGP | GGTTCCTCTGAAAGCGGTTCTTCCGAAGGCGGT CCAGGTTCTTCCGAAAGCGGTTCTTCTGAGGGC GGTCCAGGTTCCTCCGAAAGCGGTTCTTCTGAG GGTGGTCCA |

(continued)

| File name | Amino acid sequence | Nucleotide sequence |
|---|---|---|
| LCW0401_047_GFP-N_E04.ab1 | GSGGEPSESGSSGESPGG SSGSESGESPGGSSGSES | GGTTCTGGTGGCGAACCTTCCGAGTCTGGTAGC TCAGGTGAATCTCCGGGTGGTTCTAGCGGTTCC GAGTCAGGTGAATCTCCGGGTGGTTCCAGCGGT TCTGAGTCA |
| LCW0401_051_GFP-N_F04.ab1 | GSGGEPSESGSSGSEGSS GPGESSGESPGGSSGSES | GGTTCTGGTGGCGAACCATCTGAGTCTGGTAGC TCAGGTAGCGAAGGTTCTTCCGGTCCAGGCGAG TCTTCAGGTGAATCTCCTGGTGGCTCCAGCGGT TCTGAGTCA |
| LCW0401_053_GFP-N_H04.ab1 | GESPGGSSGSESGESPGG SSGSESGESPGGSSGSES | GGTGAATCTCCTGGTGGTTCCAGCGGTTCCGAG TCAGGTGAATCTCCAGGTGGCTCTAGCGGTTCC GAGTCAGGTGAATCTCCTGGTGGTTCTAGCGGT TCTGAATCA |
| LCW0401_054_GFP-N_A05.ab1 | GSEGSSGPGESSGSEGSS GPGESSGSGGEPSESGSS | GGTAGCGAAGGTTCTTCCGGTCCAGGTGAATCT TCAGGTAGCGAAGGTTCTTCTGGTCCTGGTGAA TCCTCAGGTTCCGGTGGCGAACCATCTGAATCT GGTAGCTCA |
| LCW0401_059_GFP-N_D05.ab1 | GSGGEPSESGSSGSEGSS GPGESSGESPGGSSGSES | GGTTCTGGTGGCGAACCATCCGAATCTGGTAGC TCAGGTAGCGAAGGTTCTTCTGGTCCTGGCGAA TCTTCAGGTGAATCTCCAGGTGGCTCTAGCGGT TCCGAATCA |
| LCW0401_060_GFP-N_E05.ab1 | GSGGEPSESGSSGSSESG SSEGGPGSGGEPSESGSS | GGTTCCGGTGGTGAACCGTCCGAATCTGGTAGC TCAGGTTCCTCTGAAAGCGGTTCTTCCGAGGGT GGTCCAGGTTCCGGTGGTGAACCTTCTGAGTCT GGTAGCTCA |
| LCW0401_061_GFP-N_F05.ab1 | GSSESGSSEGGPGSGGEP SESGSSGSEGSSGPGESS | GGTTCCTCTGAAAGCGGTTCTTCTGAGGGCGGT CCAGGTTCTGGTGGCGAACCATCTGAATCTGGT AGCTCAGGTAGCGAAGGTTCTTCCGGTCCGGGT GAATCTTCA |
| LCW0401_063_GFP-N_H05.ab1 | GSGGEPSESGSSGSEGSS GPGESSGSEGSSGPGESS | GGTTCTGGTGGTGAACCGTCCGAATCTGGTAGC TCAGGTAGCGAAGGTTCTTCTGGTCCTGGCGAG TCTTCAGGTAGCGAAGGTTCTTCTGGTCCTGGT GAATCTTCA |

(continued)

| File name | Amino acid sequence | Nucleotide sequence |
|---|---|---|
| LCW0401_066_GFP-N_B06.ab1 | GSGGEPSESGSSGSSESG SSEGGPGSGGEPSESGSS | GGTTCTGGTGGCGAACCATCCGAGTCTGGTAGC TCAGGTTCTTCCGAAAGCGGTTCTTCCGAAGGC GGTCCAGGTTCTGGTGGTGAACCGTCCGAATCT GGTAGCTCA |
| LCW0401_067_GFP-N_C06.ab1 | GSGGEPSESGSSGESPGG SSSGSESGSESPGGSSGSES | GGTTCCGGTGGCGAACCTTCCGAATCTGGTAGC TCAGGTGAATCTCCGGGTGGTTCTAGCGGTTCC GAATCAGGTGAATCTCCAGGTGGTTCTAGCGGT TCCGAATCA |
| LCW0401_069_GFP-N_D06.ab1 | GSGGEPSESGSSGSGGEP SESGSSGESPGGSSGSES | GGTTCCGGTGGTGAACCATCTGAGTCTGGTAGC TCAGGTTCCGGTGGCGAACCGTCCGAGTCTGGT AGCTCAGGTGAATCTCCGGGTGGTTCCAGCGGT TCCGAATCA |
| LCW0401_070_GFP-N_E06.ab1 | GSEGSSGPGESSGSSESG SSEGGPGSEGSSGPGESS | GGTAGCGGAAGGTTCTTCTGGTCCGGGCGAATCC TCAGGTTCTTCCGAAAGCGGTTCTTCCGAAGGT GGTCCAGGTAGCGGAAGGTTCTTCCGGTCCTGGT GAATCTTCA |
| LCW0401_078_GFP-N_F06.ab1 | GSSESGSSEGGPGESPGG SSSGSESGSESPGGSSGSES | GGTTCCTCTGAAAGCGGGTTCTTCTGAAGGCGGT CCAGGTGAATCTCCGGGTGGCTCCAGCGGTTCT GAATCAGGTGAATCTCCTGGTGGCTCCAGCGGT TCCGAGTCA |
| LCW0401_079_GFP-N_G06.ab1 | GSEGSSGPGESSGSEGSS GPGESSGSGGGEPSESGSS | GGTAGCGGAAGGTTCTTCTGGTCCAGGCGAGTCT TCAGGTAGCGGAAGGTTCTTCCGGTCCTGGCGAG TCTTCAGGTTCCGGTGGCGAACCGTCCGAATCT GGTAGCTCA |

**Example 2: Construction of XTEN_AE36 segments**

A codon library encoding XTEN sequences of 36 amino acid length was constructed. The XTEN sequence was designated XTEN AE36. Its segments have the amino acid sequence [X]$_3$ where X is a 12mer peptide with the sequence: GSPAGSPTSTEE, GSEPATSGSE TP, GTSESA TPESGP, or GTSTEPSEGSAP. The insert was obtained by annealing the following pairs of phosphorylated synthetic oligonucleotide pairs:

AE1for: AGGTAGCCCDGCWGGYTCTCCDACYTCYACYGARGA

AE1rev: ACCTTCYTCRGTRGARGTHGGAGARCCWGCHGGGCT

AE2for: AGGTAGCGAACCKGCWACYTCYGGYTCTGARACYCC

AE2rev: ACCTGGRGTYTCAGARCCRGARGTWGCMGGTTCGCT

AE3for: AGGTACYTCTGAAAGCGCWACYCCKGARTCYGGYCC

AE3rev: ACCTGGRCCRGAYTCMGGRGTWGCGCTTTCAGARGT

AE4for: AGGTACYTCTACYGAACCKTCYGARGGYAGCGCWCC

AE4rev: ACCTGGWGCGCTRCCYTCRGAMGGTTCRGTAGARGT

We also annealed the phosphorylated oligonucleotide 3KpnIstopperFor: AGGTTCGTCTTCACTCGAGGGTAC and the non-phosphorylated oligonucleotide pr_3KpnIstopperRev: CCTCGAGTGAAGACGA. The annealed oligonucleotide pairs were ligated, which resulted in a mixture of products with varying length that represents the varying number of 12mer repeats ligated to one BbsI/KpnI segment. The products corresponding to the length of 36 amino acids were isolated from the mixture by preparative agarose gel electrophoresis and ligated into the BsaI/KpnI digested stuffer vector pCW0359. Most of the clones in the resulting library designated LCW0402 showed green fluorescence after induction which shows that the sequence of XTEN_AE36 had been ligated in frame with the GFP gene and most sequences of XTEN_AE36 show good expression.

We screened 96 isolates from library LCW0402 for high level of fluorescence by stamping them onto agar plate containing IPTG. The same isolates were evaluated by PCR and 48 isolates were identified that contained segments with 36 amino acids as well as strong fluorescence. These isolates were sequenced and 37 clones were identified that contained correct XTEN_AE36 segments. The file names of the nucleotide and amino acid constructs for these segments are listed in Table 9.

Table 9: DNA and Amino Acid Sequences for 36-mer motifs

| File name | Amino acid sequence | Nucleotide sequence |
|---|---|---|
| LCW0402_002_GFP-N_A07.ab1 | GSPAGSPTSTEEGTSE SATPESGPGTSTEPSE GSAP | GGTAGCCCGGCAGGCTCTCCGACCTCTACTGAGGAA GGTACTTCTGAAAGCGCAACCCCGGAGTCCGGCCCA GGTACCTCTACCGAACCGTCTGAGGGCAGCGCACCA |
| LCW0402_003_GFP-N_B07.ab1 | GTSTEPSEGSAPGTST EPSEGSAPGTSTEPSE GSAP | GGTACTTCTACCGAACCGTCCGAAGGCAGCGCTCCA GGTACCTCTACTGAACCTTCCGAGGGCAGCGCTCCA GGTACCTCTACCGAACCTTCTGAAGGTAGCGCACCA |
| LCW0402_004_GFP-N_C07.ab1 | GTSTEPSEGSAPGTSE SATPESGPGTSESATP ESGP | GGTACCTCTACCGAACCGTCTGAAGGTAGCGCACCA GGTACCTCTGAAAGCGCAACTCCTGAGTCCGGTCCA GGTACTTCTGAAAGCGCAACCCCGGAGTCTGGCCCA |
| LCW0402_005_GFP-N_D07.ab1 | GTSTEPSEGSAPGTSE SATPESGPGTSESATP ESGP | GGTACTTCTACTGAACCGTCTGAAGGTAGCGCACCA GGTACTTCTGAAAGCGCAACCCCGGAATCCGGCCCA GGTACCTCTGAAAGCGCAACCCCGGAGTCCGGCCCA |
| LCW0402_006_GFP-N_E07.ab1 | GSEPATSGSETPGTSE SATPESGPGSPAGSPT STEE | GGTAGCGAACCGGCAACCTCCGGCTCTGAAACCCCA GGTACCTCTGAAAGCGCTACTCCTGAATCCGGCCCA GGTAGCCCGGCAGGTTCTCCGACTTCCACTGAGGAA |
| LCW0402_008_GFP-N_F07.ab1 | GTSESATPESGPGSEP ATSGSETPGTSTEPSE GSAP | GGTACTTCTGAAAGCGCAACCCCTGAATCCGGTCCA GGTAGCGAACCGGCTACTTCTGGCTCTGAGACTCCA GGTACTTCTACCGAACCGTCCGAAGGTAGCGCACCA |
| LCW0402_009_GFP-N G07.ab1 | GSPAGSPTSTEEGSPA GSPTSTEEGSEPATSG SETP | GGTAGCCCGGCTGGCTCTCCAACCTCCACTGAGGAA GGTAGCCCGGCTGGCTCTCCAACCTCCACTGAAGAA GGTAGCGAACCGGCTACCTCCGGCTCTGAAACTCCA |
| LCW0402_011_GFP-N_A08.ab1 | GSPAGSPTSTEEGTSE SATPESGPGTSTEPSE GSAP | GGTAGCCCGGCTGGCTCTCCTACCTCTACTGAGGAA GGTACTTCTGAAAGCGCTACTCCTGAGTCTGGTCCA GGTACCTCTACTGAACCGTCCGAAGGTAGCGCTCCA |
| LCW0402_012_GFP-N_B08.ab1 | GSPAGSPTSTEEGSPA GSPTSTEEGTSTEPSE GSAP | GGTAGCCCTGCTGGCTCTCCGACTTCTACTGAGGAA GGTAGCCCGGCTGGTTCTCCGACTTCTACTGAGGAA GGTACTTCTACCGAACCTTCCGAAGGTAGCGCTCCA |
| LCW0402_013_GFP-N_C08.ab1 | GTSESATPESGPGTST EPSEGSAPGTSTEPSE GSAP | GGTACTTCTGAAAGCGCTACTCCGGAGTCCGGTCCA GGTACCTCTACCGAACCGTCCGAAGGCAGCGCTCCA GGTACTTCTACTGAACCTTCTGAGGGTAGCGCTCCA |

(continued)

| File name | Amino acid sequence | Nucleotide sequence |
|---|---|---|
| LCW0402_014_GFP-N_D08.ab1 | GTSTEPSEGSAPGSPA GSPTSTEEGTSTEPSE GSAP | GGTACCTCTACCGAACCTTCCGAAGGTAGCGCTCCA GGTAGCCCGGCAGGTTCTCCTACTTCCACTGAGGAA GGTACTTCTACCGAACCTTCTGAGGGTAGCGCACCA |
| LCW0402 _015_GFP-N_E08.ab1 | GSEPATSGSETPGSPA GSPTSTEEGTSESATP ESGP | GGTAGCGAACCGGCTACTTCCGGCTCTGAGACTCCA GGTAGCCCTGCTGGCTCTCCGACCTCTACCGAAGAA GGTACCTCTGAAAGCGCTACCCCTGAGTCTGGCCCA |
| LCW0402_016_GFP-N_F08.ab1 | GTSTEPSEGSAPGTSE SATPESGPGTSESATP ESGP | GGTACTTCTACCGAACCTTCCGAGGGCAGCGCACCA GGTACTTCTGAAAGCGCTACCCCTGAGTCCGGCCCA GGTACTTCTGAAAGCGCTACTCCTGAATCCGGTCCA |
| LCW0402_020_GFP-N_G08.ab1 | GTSTEPSEGSAPGSEP ATSGSETPGSPAGSPT STEE | GGTACTTCTACTGAACCGTCTGAAGGCAGCGCACCA GGTAGCGAACCGGCTACTTCCGGTTCTGAAACCCCA GGTAGCCCAGCAGGTTCTCCAACTTCTACTGAAGAA |
| LCW0402_023_GFP-N_A09.ab1 | GSPAGSPTSTEEGTSE SATPESGPGSEPATSG SETP | GGTAGCCCTGCTGGCTCTCCAACCTCCACCGAAGAA GGTACCTCTGAAAGCGCAACCCCTGAATCCGGCCCA GGTAGCGAACCGGCAACCTCCGGTTCTGAAACCCCA |
| LCW0402_024_GFP-N_B09.ab1 | GTSESATPESGPGSPA GSPTSTEEGSPAGSPT STEE | GGTACTTCTGAAAGCGCTACTCCTGAGTCCGGCCCA GGTAGCCCGGCTGGCTCTCCGACTTCCACCGAGGAA GGTAGCCCGGCTGGCTCTCCAACTTCTACTGAAGAA |
| LCW0402_025_GFP-N_C09.ab1 | GTSTEPSEGSAPGTSE SATPESGPGTSTEPSE GSAP | GGTACCTCTACTGAACCTTCTGAGGGCAGCGCTCCA GGTACTTCTGAAAGCGCTACCCCGGAGTCCGGTCCA GGTACTTCTACTGAACCGTCCGAAGGTAGCGCACCA |
| LCW0402_026_GFP-N_D09.ab1 | GSPAGSPTSTEEGTST EPSEGSAPGSEPATSG SETP | GGTAGCCCGGCAGGCTCTCCGACTTCCACCGAGGAA GGTACCTCTACTGAACCTTCTGAGGGTAGCGCTCCA GGTAGCGAACCGGCAACCTCTGGCTCTGAAACCCCA |
| LCW0402_027_GFP-N_E09.ab1 | GSPAGSPTSTEEGTST EPSEGSAPGTSTEPSE GSAP | GGTAGCCCAGCAGGCTCTCCGACTTCCACTGAGGAA GGTACTTCTACTGAACCTTCCGAAGGCAGCGCACCA GGTACCTCTACTGAACCTTCTGAGGGCAGCGCTCCA |
| LCW0402_032_GFP-N_H09.ab1 | GSEPATSGSETPGTSE SATPESGPGSPAGSPT STEE | GGTAGCGAACCTGCTACCTCCGGTTCTGAAACCCCA GGTACCTCTGAAAGCGCAACTCCGGAGTCTGGTCCA GGTAGCCCTGCAGGTTCTCCTACCTCCACTGAGGAA |

EP 3 431 098 A1

| File name | Amino acid sequence | Nucleotide sequence |
|---|---|---|
| LCW0402_034_GFP-N_A10.ab1 | GTSESATPESGPGTST EPSEGSAPGTSTEPSE GSAP | GGTACCTCTGAAAGCGCTACTCCGGAGTCTGGCCCA GGTACCTCTACTGAACCGTCTGAGGGTAGCGCTCCA GGTACTTCTACTGAACCGTCCGAAGGTAGCGCACCA |
| LCW0402_036_GFP-N C10.ab1 | GSPAGSPTSTEEGTST EPSEGSAPGTSTEPSE | GGTAGCCCGGCTGGTTCTCCGACTTCCACCGAGGAA GGTACCTCTACTGAACCTTCTGAGGGTAGCGCTCCA |
| | GSAP | GGTACCTCTACTGAACCTTCCGAAGGCAGCGCTCCA |
| LCW0402_039_GFP-N_E10.ab1 | GTSTEPSEGSAPGTST EPSEGSAPGTSTEPSE GSAP | GGTACTTCTACCGAACCGTCCGAGGGCAGCGCTCCA GGTACTTCTACTGAACCTTCTGAAGGCAGCGCTCCA GGTACTTCTACTGAACCTTCCGAAGGTAGCGCACCA |
| LCW0402_040_GFP-N_F10.ab1 | GSEPATSGSETPGTSE SATPESGPGTSTEPSE GSAP | GGTAGCGAACCTGCAACCTCTGGCTCTGAAACCCCA GGTACCTCTGAAAGCGCTACTCCTGAATCTGGCCCA GGTACTTCTACTGAACCGTCCGAGGGCAGCGCACCA |
| LCW0402_041_GFP-N_G10.ab1 | GTSTEPSEGSAPGSPA GSPTSTEEGTSTEPSE GSAP | GGTACTTCTACCGAACCGTCCGAGGGTAGCGCACCA GGTAGCCCAGCAGGTTCTCCTACCTCCACCGAGGAA GGTACTTCTACCGAACCGTCCGAGGGTAGCGCACCA |
| LCW0402_050_GFP-N_A11.ab1 | GSEPATSGSETPGTSE SATPESGPGSEPATSG SETP | GGTAGCGAACCGGCAACCTCCGGCTCTGAAACTCCA GGTACTTCTGAAAGCGCTACTCCGGAATCCGGCCCA GGTAGCGAACCGGCTACTTCCGGCTCTGAAACCCCA |
| LCW0402_051_GFP-N_B11.ab1 | GSEPATSGSETPGTSE SATPESGPGSEPATSG SETP | GGTAGCGAACCGGCAACTTCCGGCTCTGAAACCCCA GGTACTTCTGAAAGCGCTACTCCTGAGTCTGGCCCA GGTAGCGAACCTGCTACCTCTGGCTCTGAAACCCCA |
| LCW0402_059_GFP-N_E11.ab1 | GSEPATSGSETPGSEP ATSGSETPGTSTEPSE GSAP | GGTAGCGAACCGGCAACCTCTGGCTCTGAAACTCCA GGTAGCGAACCTGCAACCTCCGGCTCTGAAACCCCA GGTACTTCTACTGAACCTTCTGAGGGCAGCGCACCA |
| LCW0402_060_GFP-N_F11.ab1 | GTSESATPESGPGSEP ATSGSETPGSEPATSG SETP | GGTACTTCTGAAAGCGCTACCCCGGAATCTGGCCCA GGTAGCGAACCGGCTACTTCTGGTTCTGAAACCCCA GGTAGCGAACCGGCTACCTCCGGTTCTGAAACTCCA |

EP 3 431 098 A1

123

(continued)

| File name | Amino acid sequence | Nucleotide sequence |
|---|---|---|
| LCW0402_061_GFP-N_G11.ab1 | GTSTEPSEGSAPGTST EPSEGSAPGTSESATP ESGP | GGTACCTCTACTGAACCTTCCGAAGGCAGCGCTCCA GGTACCTCTACCGAACCGTCCGAGGGCAGCGCACCA GGTACTTCTGAAAGCGCAACCCCTGAATCCGGTCCA |
| LCW0402_065_GFP-N_A12.ab1 | GSEPATSGSETPGTSE SATPESGPGTSESATP ESGP | GGTAGCGAACCGGCAACCTCTGGCTCTGAAACCCCA GGTACCTCTGAAAGCGCTACTCCGGAATCTGGTCCA GGTACTTCTGAAAGCGCTACTCCGGAATCCGGTCCA |
| LCW0402_066_ GFP-N_B12.ab1 | GSEPATSGSETPGSEP ATSGSETPGTSTEPSE GSAP | GGTAGCGAACCTGCTACCTCCGGCTCTGAAACTCCA GGTAGCGAACCGGCTACTTCCGGTTCTGAAACTCCA GGTACCTCTACCGAACCTTCCGAAGGCAGCGCACCA |
| LCW0402_067_ GFP-N_C12.ab1 | GSEPATSGSETPGTST EPSEGSAPGSEPATSG SETP | GGTAGCGAACCTGCTACTTCTGGTTCTGAAACTCCA GGTACTTCTACCGAACCGTCCGAGGGTAGCGCTCCA GGTAGCGAACCTGCTACTTCTGGTTCTGAAACTCCA |
| LCW0402_069_ GFP-N_D12.ab1 | GTSTEPSEGSAPGTST EPSEGSAPGSEPATSG SETP | GGTACCTCTACCGAACCGTCCGAGGGTAGCGCACCA GGTACCTCTACTGAACCGTCTGAGGGTAGCGCTCCA GGTAGCGAACCGGCAACCTCCGGTTCTGAAACTCCA |
| LCW0402_073_GFP-N_F12.ab1 | GTSTEPSEGSAPGSEP ATSGSETPGSPAGSPT STEE | GGTACTTCTACTGAACCTTCCGAAGGTAGCGCTCCA GGTAGCGAACCTGCTACTTCTGGTTCTGAAACCCCA GGTAGCCCGGCTGGCTCTCCGACCTCCACCGAGGAA |
| LCW0402_074_GFP-N_G12.ab1 | GSEPATSGSETPGSPA GSPTSTEEGTSESATP ESGP | GGTAGCGAACCGGCTACTTCCGGCTCTGAGACTCCA GGTAGCCCAGCTGGTTCTCCAACCTCTACTGAGGAA GGTACTTCTGAAAGCGCTACCCCTGAATCTGGTCCA |
| LCW0402_075_GFP-N_H12.ab1 | GTSESATPESGPGSEP ATSGSETPGTSESATP ESGP | GGTACCTCTGAAAGCGCAACTCCTGAGTCTGGCCCA GGTAGCGAACCTGCTACCTCCGGCTCTGAGACTCCA GGTACCTCTGAAAGCGCAACCCCGGAATCTGGTCCA |

## Example 3: Construction of XTEN_AF36 segments

A codon library encoding sequences of 36 amino acid length was constructed. The sequences were designated XTEN_AF36. Its segments have the amino acid sequence [X]3 where X is a 12mer peptide with the sequence: GSTSESPSGTAP, GTSTPESGSASP, GTSPSGESSTAP, or GSTSSTAESPGP. The insert was obtained by annealing the following pairs of phosphorylated synthetic oligonucleotide pairs:

AF1for: AGGTTCTACYAGCGAATCYCCKTCTGGYACYGCWCC

AF1rev: ACCTGGWGCRGTRCCAGAMGGRGATTCGCTRGTAGA

AF2for: AGGTACYTCTACYCCKGAAAGCGGYTCYGCWTCTCC

AF2rev: ACCTGGAGAWGCRGARCCGCTTTCMGGRGTAGARGT

AF3for: AGGTACYTCYCCKAGCGGYGAATCTTCTACYGCWCC

AF3rev: ACCTGGWGCRGTAGAAGATTCRCCGCTMGGRGARGT

AF4for: AGGTTCYACYAGCTCTACYGCWGAATCTCCKGGYCC

AF4rev: ACCTGGRCCMGGAGATTCWGCRGTAGAGCTRGTRGA

We also annealed the phosphorylated oligonucleotide 3KpnIstopperFor: AGGTTCGTCTTCACTCGAGGGTAC and the non-phosphorylated oligonucleotide pr_3KpnIstopperRev: CCTCGAGTGAAGACGA. The annealed oligonucleotide pairs were ligated, which resulted in a mixture of products with varying length that represents the varying number of 12mer repeats ligated to one BbsI/KpnI segment The products corresponding to the length of 36 amino acids were isolated from the mixture by preparative agarose gel electrophoresis and ligated into the BsaI/KpnI digested stuffer vector pCW0359. Most of the clones in the resulting library designated LCW0403 showed green fluorescence after induction which shows that the sequence of XTEN_AF36 had been ligated in frame with the GFP gene and most sequences of XTEN_AF36 show good expression.

We screened 96 isolates from library LCW0403 for high level of fluorescence by stamping them onto agar plate containing IPTG. The same isolates were evaluated by PCR and 48 isolates were identified that contained segments with 36 amino acids as well as strong fluorescence. These isolates were sequenced and 44 clones were identified that contained correct XTEN_AF36 segments. The file names of the nucleotide and amino acid constructs for these segments are listed in Table 10.

**Table 10: DNA and Amino Acid Sequences for 36-mer motifs**

| File name | Amino acid sequence | Nucleotide sequence |
|---|---|---|
| LCW0403_004_GFP-N_A01.ab1 | GTSTPESGSASPGTSP SGESSTAPGTSPSGES STAP | GGTACTTCTACTCCGGAAAGCGGTTCCGCATCTCCA GGTACTTCTCCTAGCGGTGAATCTTCTACTGCTCCAG GTACCTCTCCTAGCGGCGAATCTTCTACTGCTCCA |
| LCW0403_005_GFP-N_B01.ab1 | GTSPSGESSTAPGSTS STAESPGPGTSPSGES STAP | GGTACTTCTCCGAGCGGTGAATCTTCTACCGCACCA GGTTCTACTAGCTCTACCGCTGAATCTCCGGGCCCAG GTACTTCTCCGAGCGGTGAATCTTCTACTGCTCCA |
| LCW0403_006_GFP-N_C01.ab1 | GSTSSTAESPGPGTSP SGESSTAPGTSTPESG SASP | GGTTCCACCAGCTCTACTGCTGAATCTCCTGGTCCAG GTACCTCTCCTAGCGGTGAATCTTCTACTGCTCCAGG TACTTCTACTCCTGAAAGCGGCTCTGCTTCTCCA |
| LCW0403_007_GFP-N_D01.ab1 | GSTSSTAESPGPGSTS STAESPGPGTSPSGES STAP | GGTTCTACCAGCTCTACTGCAGAATCTCCTGGCCCAG GTTCCACCAGCTCTACCGCAGAATCTCCGGGTCCAG GTACTTCCCCTAGCGGTGAATCTTCTACCGCACCA |
| LCW0403_008_GFP-N_E01.ab1 | GSTSSTAESPGPGTSP SGESSTAPGTSTPESG SASP | GGTTCTACTAGCTCTACTGCTGAATCTCCTGGCCCAG GTACTTCTCCTAGCGGTGAATCTTCTACCGCTCCAGG TACCTCTACTCCGGAAAGCGGTTCTGCATCTCCA |
| LCW0403_010_GFP-N_F01.ab1 | GSTSSTAESPGPGTST PESGSASPGSTSESPS GTAP | GGTTCTACCAGCTCTACCGCAGAATCTCCTGGTCCAG GTACCTCTACTCCGGAAAGCGGCTCTGCATCTCCAG GTTCTACTAGCGAATCTCCTTCTGGCACTGCACCA |
| LCW0403_011_GFP-N_G01.ab1 | GSTSSTAESPGPGTST PESGSASPGTSTPESG SASP | GGTTCTACTAGCTCTACTGCAGAATCTCCTGGCCCAG GTACCTCTACTCCGGAAAGCGGCTCTGCATCTCCAG GTACTTCTACCCCTGAAAGCGGTTCTGCATCTCCA |
| LCW0403_012_GFP-N H01.ab1 | GSTSESPSGTAPGTSP SGESSTAPGSTSESPS | GGTTCTACCAGCGAATCTCCTTCTGGCACCGCTCCAG GTACCTCTCCTAGCGGCGAATCTTCTACCGCTCCAGG |
| | GTAP | TTCTACTAGCGAATCTCCTTCTGGCACTGCACCA |
| LCW0403_013_GFP-N_A02.ab1 | GSTSSTAESPGPGSTS STAESPGPGTSPSGES STAP | GGTTCCACCAGCTCTACTGCAGAATCTCCGGGCCCA GGTTCTACTAGCTCTACTGCAGAATCTCCGGGTCCAG GTACTTCTCCTAGCGGCGAATCTTCTACCGCTCCA |
| LCW0403_014_GFP-N_B02.ab1 | GSTSSTAESPGPGTST PESGSASPGSTSESPS GTAP | GGTTCCACTAGCTCTACTGCAGAATCTCCTGGCCCAG GTACCTCTACCCCTGAAAGCGGCTCTGCATCTCCAG GTTCTACCAGCGAATCCCCGTCTGGCACCGCACCA |

(continued)

| File name | Amino acid sequence | Nucleotide sequence |
|---|---|---|
| LCW0403_015_GFP-N_C02.ab1 | GSTSSTAESPGPGSTS STAESPGPGTSPSGES STAP | GGTTCTACTAGCTCTACTGCTGAATCTCCGGGTCCAG GTTCTACCAGCTCTACTGCTGAATCTCCTGGTCCAGG TACCTCCCCGAGCGGTGAATCTTCTACTGCACCA |
| LCW0403_017_GFP-N_D02.ab1 | GSTSSTAESPGPGSTS ESPSGTAPGSTSSTAE SPGP | GGTTCTTACCAGCTCTACCGCTGAATCTCCTGGCCCAG GTTCTACCAGCGAATCCCCGTCTGGCACCGCACCAG GTTCTACTAGCTCTACCGCTGAATCTCCGGGTCCA |
| LCW0403_018_GFP-N_E02.ab1 | GSTSSTAESPGPGSTS STAESPGPGTSSTAE SPGP | GGTTCTACCAGCTCTACCGCAGAATCTCCTGGCCCA GGTTCCACTAGCTCTACCGCTGAATCTCCTGGTCCAG GTTCTACTAGCTCTACCGCTGAATCTCCTGGTCCA |
| LCW0403_019_GFP-N_F02.ab1 | GSTSESPSGTAPGSTS STAESPGPGTSSTAE SPGP | GGTTCTACTAGCGAATCCCCTTCTGGTACTGCTCCAG GTTCCACTAGCTCTACCGCTGAATCTCCTGGCCCAGG TTCCACTAGCTCTACTGCAGAATCTCCTGGTCCA |
| LCW0403_023_GFP-N_H02.ab1 | GSTSESPSGTAPGSTS ESPSGTAPGSTSESPS GTAP | GGTTCTACTAGCGAATCTCCTTCTGGTACCGCTCCAG GTTCTACCAGCGAATCCCCGTCTGGTACTGCTCCAGG TTCTACCAGGGAATCTCCCTCTGGTACTGCTGCACCA |
| LCW0403_024_GFP-N_A03.ab1 | GSTSSTAESPGPGSTS STAESPGPGTSSTAE SPGP | GGTTCCACCAGCTCTACTGCTGAATCTCCTGGCCCAG GTTCTACCAGCTCTACTGCTGAATCTCCGGGTCCAGG TTCCACCAGCTCTACCGCTGAATCTCCGGGTCCA |
| LCW0403_025_GFP-N_B03.ab1 | GSTSSTAESPGPGSTS STAESPGPGTSPSGES STAP | GGTTCCACTAGCTCTACCGCAGAATCTCCTGGTCCAG GTTCTACTAGCTCTACTGCTGAATCTCCGGGTCCAGG TACCTCCCCTAGCGGCGAATCTTCTACCGCTCCA |
| LCW0403_028_GFP-N_D03.ab1 | GSSPSASTGTGPGSST PSGATGSPGSSTPSGA TGSP | GGTTCTTAGCCCTTCTGCTTCCACCGGTACCGGCCCAG GTAGCTCTACTCCGTCTGGTGCAACTGGCTCTCCAGG TAGCTCTACTCCGTCTGGTGCAACCGGCTCCCCA |
| LCW0403_029_GFP-N_E03.ab1 | GTSPSGESSTAPGTST PESGSASPGSTSSTAE SPGP | GGTACTTCCCCTAGCGGTGAATCTTCTACTGCTCCAG GTACCTCTACTCCGGAAAGCGGCTCCGCATCTCCAG GTTCTACTAGCTCTACTGCTGAATCTCCTGGTCCA |
| LCW0403_030_GFP-N_F03.ab1 | GSTSSTAESPGPGSTS STAESPGPGTSTPESG SASP | GGTTCTACTAGCTCTACCGCTGAATCTCCGGGTCCAG GTTCTACCAGCTCTACTGCAGAATCTCCTGGCCCAGG TACTTCTACTCCGGAAAGCGGTTCCGCTTCTCCA |

| File name | Amino acid sequence | Nucleotide sequence |
|---|---|---|
| LCW0403_031_GFP-N_G03.ab1 | GTSPSGESSTAPGSTS STAESPGPGTSTPESG SASP | GGTACTTCTCCTAGCGGTGAATCTTCTACCGCTCCAG GTTCTACCAGCTCTACTGCTGAATCTCCTGGCCCAGG TACTTCTACCCCGGAAAGCGGCTCCGCTTCTCCA |
| LCW0403_033_GFP-N_H03.ab1 | GSTSESPSGTAPGSTS STAESPGPGSTSSTAE SPGP | GGTTCTACTAGCGAATCCCCTTCTGGTACTGCACCAG GTTCTACCAGCTCTACTGCTGAATCTCCGGGCCCAGG TTCCACCAGCTCTACCGCAGAATCTCCTGGTCCA |
| LCW0403_035_GFP-N_A04.ab1 | GSTSSTAESPGPGSTS ESPSGTAPGSTSSTAE SPGP | GGTTCCACCAGCTCTACCGCTGAATCTCCGGGCCCA GGTTCTACCAGCGAATCCCCTTCTGGCACTGCACCA GGTTCTACTAGCTCTACCGCAGAATCTCCGGGCCCA |
| LCW0403_036_GFP-N_ B04.ab1 | GSTSSTAESPGPGTSP SGESSTAPGTSTPESG SASP | GGTTCTACCAGCTCTACTGCTGAATCTCCGGGTCCAG GTACTTCCCCGAGCGGTGAATCTTCTACTGCACCAG GTACTTCTACTCCGGAAAGCGGTTCCGCTTCTCCA |
| LCW0403_039_GFP-N_C04.ab1 | GSTSESPSGTAPGSTS ESPSGTAPGTSPSGES STAP | GGTTCTACCAGCGAATCTCCTTCTGGCACCGCTCCAG GTTCTACTAGCGAATCCCCGTCTGGTACCGCACCAG GTACTTCTCCTAGCGGCGAATCTTCTACCGCACCA |
| LCW0403_041_GFP-N_D04.ab1 | GSTSESPSGTAPGSTS ESPSGTAPGTSTPESG SASP | GGTTCTACCAGCGAATCCCCTTCTGGTACTGCTCCAG GTTCTACCAGCGAATCCCCTTCTGGCACCGCACCAG GTACTTCTACCCCTGAAAGCGGCTCCGCTTCTCCA |
| LCW0403_044_GFP-N_E04.ab1 | GTSTPESGSASPGSTS STAESPGPGSTSSTAE SPGP | GGTACCTCTACTCCTGAAAGCGGTTCTGCATCTCCAG GTTCCACTAGCTCTACCGCAGAATCTCCGGGCCCAG GTTCTACTAGCTCTACTGCTGAATCTCCTGGCCCA |
| LCW0403_046_GFP-N_F04.ab1 | GSTSESPSGTAPGSTS | GGTTCTACCAGCGAATCCCCTTCTGGCACTGCACCA |
| | ESPSGTAPGTSPSGES STAP | GGTTCTACTAGCGAATCCCCTTCTGGTACCGCACCAG GTACTTCTCCGAGCGGCGAATCTTCTACTGCTCCA |
| LCW0403_047_GFP-N_G04ab1 | GSTSSTAESPGPGSTS STAESPGPGSTSESPS GTAP | GGTTCTACTAGCTCTACCGCTGAATCTCCTGGCCCAG GTTCCACTAGCTCTACCGCAGAATCTCCGGGCCCAG GTTCTACTAGCGAATCCCCTTCTGGTACCGCTCCA |

| File name | Amino acid sequence | Nucleotide sequence |
|---|---|---|
| LCW0403_049_GFP-N_H04.ab1 | GSTSSTAESPGPGSTS STAESPGPGTSTPESG SASP | GGTTCCACCAGCTCTACTGCAGAATCTCCTGGCCCA GGTTCTACTAGCTCTACCGCAGAATCTCCTGGTCCAG GTACCTCTACTCCTGAAAGCGGTTCCGCATCTCCA |
| LCW0403_051_GFP-N_A05.ab1 | GSTSSTAESPGPGSTS STAESPGPGTSESPS GTAP | GGTTCTACTAGCTCTACTGCTGAATCTCCGGGCCCAG GTTCTACTAGCTCTACCGCTGAATCTCCGGGTCCAGG TTCTACTAGCGAATCTCCTTCTGGTACCGCTCCA |
| LCW0403_053_GFP-N_B05.ab1 | GTSPSGESSTAPGSTS ESPSGTAPGSTSSTAE SPGP | GGTACCTCCCCGAGCGGTGAATCTTCTACTGCACCA GGTTCTACTAGCGAATCCCCTTCTGGTACTGCTCCAG GTTCCACCAGCTCTACTGCAGAATCTCCGGGTCCA |
| LCW0403_054_GFP-N_C05.ab1 | GSTSESPSGTAPGTSP SGESSTAPGSTSSTAE SPGP | GGTTCTACTAGCGAATCCCCGTCTGGTACTGCTCCAG GTACTTCCCCTAGCGGTGAATCTTCTACTGCTCCAGG TTCTACCAGCTCTACCGCAGAATCTCCGGGTCCA |
| LCW0403_057_GFP-N_D05.ab1 | GSTSSTAESPGPGSTS ESPSGTAPGTSPSGES STAP | GGTTCTACCAGCTCTACCGCTGAATCTCCTGGCCCAG GTTCTACTAGCGAATCTCCGTCTGGCACCGCACCAG GTACTTCCCCTAGCGGTGAATCTTCTACTGCACCA |
| LCW0403_058_GFP-N_E05.ab1 | GSTSESPSGTAPGSTS ESPSGTAPGTSTPESG SASP | GGTTCTACTAGCGAATCTCCTTCTGGCACTGCACCAG GTTCTACCAGCGAATCTCCGTCTGGCACTGCACCAG GTACCTCTACCCCTGAAAGCGGTTCCGCTTCTCCA |
| LCW0403_060_GFP-N_F05.ab1 | GTSTPESGSASPGSTS ESPSGTAPGSTSSTAE SPGP | GGTACCTCTACTCCGGAAAGCGGTTCCGCATCTCCA GGTTCTACCAGCGAATCCCCGTCTGGCACCGCACCA GGTTCTACTAGCTCTACTGCTGAATCTCCGGGCCCA |
| LCW0403_063_GFP-N_G05.ab1 | GSTSSTAESPGPGTSP SGESSTAPGTSPSGES STAP | GGTTCTACTAGCTCTACTGCAGAATCTCCGGGCCCA GGTACCTCTCCTAGCGGTGAATCTTCTACCGCTCCAG GTACTTCTCCGAGCGGTGAATCTTCTACCGCTCCA |
| LCW0403_064_GFP-N_H05.ab1 | GTSPSGESSTAPGTSP SGESSTAPGTSPSGES STAP | GGTACCTCCCCTAGCGGCGAATCTTCTACTGCTCCAG GTACCTCTCCTAGCGGCGAATCTTCTACCGCTCCAGG TACCTCCCCTAGCGGTGAATCTTCTACCGCACCA |
| LCW0403_065_GFP-N_A06.ab1 | GSTSSTAESPGPGTST PESGSASPGSTSESPS GTAP | GGTTCCACTAGCTCTACTGCTGAATCTCCTGGCCCAG GTACTTCTACTCCGGAAAGCGGTTCCGCTTCTCCAGG TTCTACTAGCGAATCTCCGTCTGGCACCGCACCA |

EP 3 431 098 A1

129

| File name | Amino acid sequence | Nucleotide sequence |
|---|---|---|
| LCW0403_066_GFP-N_B06.ab1 | GSTSESPSGTAPGTSP SGESSTAPGTSPSGES STAP | GGTTCTACTAGCGAATCTCCGTCTGGCACTGCTCCAG GTACTTCTCCTAGCGGTGAATCTTCTACCGCTCCAGG TACTTCCCCTAGCGGCGAATCTTCTACCGCTCCA |
| LCW0403_067_GFP-N_C06.ab1 | GSTSESPSGTAPGTST PESGSASPGSTSSTAE SPGP | GGTTCTACTAGCGAATCTCCTTCTGGTACCGCTCCAG GTACTTCTACCCCTGAAAGCGGCTCCGCTTCTCCAGG TTCCACTAGCTCTACCGCTGAATCTCCGGGTCCA |
| LCW0403_068_GFP-N_D06.ab1 | GSTSSTAESPGPGSTS STAESPGPGSTSESPS GTAP | GGTTCCACTAGCTCTACTGCTGAATCTCCTGGCCCAG GTTCTACCAGCTCTACCGCTGAATCTCCTGGCCCAGG TTCTACCAGCGAATCTCCGTCTGGCACCGCACCA |
| LCW0403_069_GFP-N_E06.ab1 | GSTSESPSGTAPGTST PESGSASPGTSTPESG SASP | GGTTCTACTAGCGAATCCCCGTCTGGTACCGCACCA GGTACTTCTACCCCGGAAAGCGGCTCTGCTTCTCCAG GTACTTCTACCCCGGAAAGCGGCTCCGCATCTCCA |
| LCW0403_070_GFP-N_F06.ab1 | GSTSESPSGTAPGTST PESGSASPGTSTPESG SASP | GGTTCTACTAGCGAATCCCCGTCTGGTACTGCTCCAG GTACTTCTACTCCTGAAAGCGGTTCCGCTTCTCCAGG TACCTCTACTCCGGAAAGCGGTTCTGCATCTCCA |

**Example 4: Construction of XTEN_AG36 segments**

A codon library encoding sequences of 36 amino acid length was constructed. The sequences were designated XTEN _AG36. Its segments have the amino acid sequence [X]$_3$ where X is a 12mer peptide with the sequence: GTPGSGTASSSP, GSSTPSGATGSP, GSSPSASTGTGP, or GASPGTSSTGSP. The insert was obtained by annealing the following pairs of phosphorylated synthetic oligonucleotide pairs:

AG1for: AGGTACYCCKGGYAGCGGTACYGCWTCTTCYTCTCC

AG1rev: ACCTGGAGARGAAGAWGCRGTACCGCTRCCMGGRGT

AG2for: AGGTAGCTCTACYCCKTCTGGTGCWACYGGYTCYCC

AG2rev: ACCTGGRGARCCRGTWGCACCAGAMGGRGTAGAGCT

AG3for: AGGTTCTAGCCCKTCTGCWTCYACYGGTACYGGYCC

AG3rev: ACCTGGRCCRGTACCRGTRGAWGCAGAMGGGCTAGA

AG4for: AGGTGCWTCYCCKGGYACYAGCTCTACYGGTTCTCC

AG4rev: ACCTGGAGAACCRGTAGAGCTRGTRCCMGGRGAWGC

We also annealed the phosphorylated oligonucleotide 3KpnIstopperFor: AGGTTCGTCTTCACTCGAGGGTAC and the non-phosphorylated oligonucleotide pr_3KpnIstopperRev: CCTCGAGTGAAGACGA. The annealed oligonucleotide pairs were ligated, which resulted in a mixture of products with varying length that represents the varying number of 12mer repeats ligated to one BbsI/KpnI segment. The products corresponding to the length of 36 amino acids were isolated from the mixture by preparative agarose gel electrophoresis and ligated into the BsaI/KpnI digested stuffer vector pCW0359. Most of the clones in the resulting library designated LCW0404 showed green fluorescence after induction which shows that the sequence AG36 had been ligated in frame with the GFP gene and most sequences _AG36 show good expression.

We screened 96 isolates from library LCW0404 for high level of fluorescence by stamping them onto agar plate containing IPTG. The same isolates were evaluated by PCR and 48 isolates were identified that contained segments with 36 amino acids as well as strong fluorescence. These isolates were sequenced and 44 clones were identified that contained correct XTEN_AG36 segments. The file names of the nucleotide and amino acid constructs for these segments are listed in Table 11.

Table 11: DNA and Amino Acid Sequences for 36-mer motifs

| File name | Amino acid sequence | Nucteotide sequence |
|---|---|---|
| LCW0404_001_GFP-N_A07.ab1 | GASPGTSSTGSPGTPG SGTASSSPGSSTPSGA TGSP | GGTGCATCCCCGGGCACTAGCTCTACCGGTTCTCCAGGTA CTCCTGGTAGCGGTACTGCTTCTTCTTCTCCAGGTAGCTCT ACTCCTTCTGGTGCTACTGGTTCTCCA |
| LCW0404_003_GFP-N_B07.ab1 | GSSTPSGATGSPGSSP SASTGTGPGSSTPSGA TGSP | GGTAGCTCTACCCCTTCTGGTGCTACCGGCTCTCCAGGTT CTAGCCCGTCTGCTTCTACCGGTACCGGTCCAGGTAGCTC TACCCCTTCTGGTGCTACTGGTTCTCCA |
| LCW0404_006_GFP-N_C07.ab1 | GASPGTSSTGSPGSSP SASTGTGPGSSTPSGA TGSP | GGTGCATCTCCGGGTACTAGCTCTACCGGTTCTCCAGGTT CTAGCCCTTCTGCTTCCACTGGTACCGGCCCAGGTAGCTC TACCCCGTCTGGTGCTACTGGTTCCCCA |
| LCW0404_007_GFP-N_D07.ab1 | GTPGSGTASSSPGSST PSGATGSPGASPGTSS TGSP | GGTACTCCGGGCAGCGGTACTGCTTCTTCCTCTCCAGGTA GCTCTACCCCTTCTGGTGCAACTGGTTCCCCAGGTGCATC CCCTGGTACTAGCTCTACCGGTTCTCCA |
| LCW0404_009_GFP-N_E07.ab1 | GTPGSGTASSSPGASP GTSSTGSPGSRPSAST GTGP | GGTACCCCTGGCAGCGGTACTGCTTCTTCTTCTCCAGGTG CTTCCCCTGGTACCAGCTCTACCGGTTCTCCAGGTTCTAG ACCTTCTGCATCCACCGGTACTGGTCCA |
| LCW0404_011_GFP-N_F07.ab1 | GASPGTSSTGSPGSST PSGATGSPGASPGTSS TGSP | GGTGCATCTCCTGGTACCAGCTCTACCGGTTCTCCAGGTA GCTCTACTCCTTCTGGTGCTACTGGCTCTCCAGGTGCTTCC CCGGGTACCAGCTCTACCGGTTCTCCA |
| LCW0404_012_GFP-N_G07.ab1 | GTPGSGTASSSPGSST PSGATGSPGSSTPSGA TGSP | GGTACCCCGGGCAGCGGTACCGCATCTTCCTCTCCAGGTA GCTCTACCCCGTCTGGTGCTACCGGTTCCCCAGGTAGCTC TACCCCGTCTGGTGCAACCGGCTCCCCA |
| LCW0404_014_GFP-N_H07.ab1 | GASPGTSSTGSPGASP GTSSTGSPGASPGTSS TGSP | GGTGCATCTCCGGGCACTAGCTCTACTGGTTCTCCAGGTG CATCCCCTGGCACTAGCTCTACTGGTTCTCCAGGTGCTTC TCCTGGTACCAGCTCTACTGGTTCTCCA |
| LCW0404_015_GFP-N_A08.ab1 | GSSTPSGATGSPGSSP SASTGTGPGASPGTSS TGSP | GGTAGCTCTACTCCGTCTGGTGCAACCGGCTCCCCAGGTT CTAGCCCGTCTGCTTCCACTGGTACTGGCCCAGGTGCTTC CCCGGGCACCAGCTCTACTGGTTCTCCA |
| LCW0404_016_GFP-N_B08.ab1 | GSSTPSGATGSPGSST PSGATGSPGTPGSGT ASSSP | GGTAGCTCTACTCCTTCTGGTGCTACCGGTTCCCCAGGTA GCTCTACTCCTTCTGGTGCTACTGGTTCCCCAGGTACTCC GGGCAGCGGTACTGCTTCTTCCTCTCCA |

132

(continued)

| File name | Amino acid sequence | Nucteotide sequence |
|---|---|---|
| LCW0404_017_GFP-N_C08.ab1 | GSSTPSGATGSPGSST PSGATGSPGASPGTSS TGSP | GGTAGCTCTACTCCGTCTGGTGCAACCGGTTCCCCAGGTA GCTCTACTCCTTCTGGTGCTACTGGCTCCCCAGGTGCATC CCCTGGCACCAGCTCTACCGGTTCTCCA |
| LCW0404_018_GFP-N_D08.ab1 | GTPGSGTASSSPGSSP SASTGTGPGSSTPSGA TGSP | GGTACTCCTGGTAGCGGTACCGCATCTTCCTCTCCAGGTT CTAGCCCTTCTGCATCTACCGGTACCGGTCCAGGTAGCTC TACTCCTTCTGGTGCTACTGGCTCTCCA |
| LCW0404_023_GFP-N_F08.ab1 | GASPGTSSTGSPGSSP SASTGTGPGTPGSGT ASSSP | GGTGCTTCCCCGGGCACTAGCTCTACCGGTTCTCCAGGTT CTAGCCCTTCTGCATCTACTGGTACTGGCCCAGGTACTCC GGGCAGCGGTACTGCTTCTTCCTCTCCA |
| LCW0404_025_GFP-N_G08.ab1 | GSSTPSGATGSPGSST PSGATGSPGASPGTSS TGSP | GGTAGCTCTACTCCGTCTGGTGCTACCGGCTCTCCAGGTA GCTCTACCCCTTCTGGTGCAACCGGCTCCCCAGGTGCTTC TCCGGGTACCAGCTCTACTGGTTCTCCA |
| LCW0404_029_GFP-N_A09.ab1 | GTPGSGTASSSPGSST PSGATGSPGSSPSAST GTGP | GGTACCCCTGGCAGCGGTACCGCTTCTTCCTCTCCAGGTA GCTCTACCCCGTCTGGTGCTACTGGCTCTCCAGGTTCTAG CCCGTCTGCATCTACCGGTACCGGCCCA |
| LCW0404 _030_GFP-N_B09.ab1 | GSSTPSGATGSPGTPG SGTASSSPGTPGSGTA SSSP | GGTAGCTCTACTCCTTCTGGTGCAACCGGCTCCCCAGGTA CCCCGGGCAGCGGTACCGCATCTTCCTCTCCAGGTACTCC GGGTAGCGGTACTGCTTCTTCTTCTCCA |
| LCW0404_031_GFP-N_C09.ab1 | GTPGSGTASSSPGSST PSGATGSPGASPGTSS TGSP | GGTACCCCGGGTAGCGGTACTGCTTCTTCCTCTCCAGGTA GCTCTACCCCTTCTGGTGCAACCGGCTCTCCAGGTGCTTC TCCGGGCACCAGCTCTACCGGTTCTCCA |
| LCW0404_034_GFP-N_ D09.ab1 | GSSTPSGATGSPGSST PSGATGSPGASPGTSS TGSP | GGTAGCTCTACCCCGTCTGGTGCTACCGGCTCTCCAGGTA GCTCTACCCCGTCTGGTGCAACCGGCTCCCCAGGTGCATC CCCGGGTACTAGCTCTACCGGTTCTCCA |
| LCW0404_035_GFP-N_E09.ab1 | GASPGTSSTGSPGTPG SGTASSSPGSSTPSGA TGSP | GGTGCTTCTCCGGGCACCAGCTCTACTGGTTCTCCAGGTA CCCCGGGCAGCGGTACCGCATCTTCTTCTCCAGGTAGCTC TACTCCTTCTGGTGCAACTGGTTCTCCA |
| LCW0404_036_GFP-N_F09.ab1 | GSSPSASTGTGPGSST PSGATGSPGTPGSGT ASSSP | GGTTCTAGCCCGTCTGCTTCCACCGGTACTGGCCCAGGTA GCTCTACCCCGTCTGGTGCAACTGGTTCCCCAGGTACCCC TGGTAGCGGTACCGCTTCTTCTTCTCCA |

| File name | Amino acid sequence | Nucteotide sequence |
|---|---|---|
| LCW0404_037_GFP-N_G09.ab1 | GASPGTSSTGSPGSSP SASTGTGPGSSTPSGA TGSP | GGTGCTTCTCCGGGCACCAGCTCTACTGGTTCTCCAGGTT CTAGCCCTTCTGCATCCACCGGTACCGGTCCAGGTAGCTC TACCCCTTCTGGTGCAACCGGCTCTCCA |
| LCW0404_040_GFP-N_H09.ab1 | GASPGTSSTGSPGSST PSGATGSPGSSTPSGA TGSP | GGTGCATCCCCGGGCACCAGCTCTACCGGTTCTCCAGGTA GCTCTACCCCGTCTGGTGCTACCGGCTCTCCAGGTAGCTC TACCCCGTCTGGTGCTACTGGCTCTCCA |
| LCW0404_041_GFP-N_A10.ab1 | GTPGSGTASSSPGSST PSGATGSPGTPGSGT ASSSP | GGTACCCCTGGTAGCGGTACTGCTTCTTCCTCTCCAGGTA GCTCTACTCCGTCTGGTGCTACCGGTTCTCCAGGTACCCC GGGTAGCGGTACCGCATCTTCTTCTCCA |
| LCW0404_043_GFP-N_C10.ab1 | GSSPSASTGTGPGSST PSGATGSPGSSTPSGA TGSP | GGTTCTAGCCCTTCTGCTTCCACCGGTACTGGCCCAGGTA GCTCTACCCCTTCTGGTGCTACCGGCTCCCCAGGTAGCTC TACTCCTTCTGGTGCAACTGGCTCTCCA |
| LCW0404_045_GFP-N_D10.ab1 | GASPGTSSTGSPGSSP SASTGTGPGSSPSAST GTGP | GGTGCTTCTCCTGGCACCAGCTCTACTGGTTCTCCAGGTT CTAGCCCTTCTGCTTCTACCGGTACTGGTCCAGGTTCTAG CCCTTCTGCATCCACTGGTACTGGTCCA |
| LCW0404_047_GFP-N F10.ab1 | GTPGSGTASSSPGASP GTSSTGSPGASPGTSS | GGTACTCCTGGCAGCGGTACCGCTTCTTCTTCTCCAGGTG CTTCTCCTGGTACTAGCTCTACTGGTTCTCCAGGTGCTTCT |
|  | TGSP | CCGGGCACTAGCTCTACTGGTTCTCCA |
| LCW0404_048_GFP-N_G10.ab1 | GSSTPSGATGSPGASP GTSSTGSPGSSTPSGA TGSP | GGTAGCTCTACCCCGTCTGGTGCTACCGGTTCCCCAGGTG CTTCTCCTGGTACTAGCTCTACCGGTTCTCCAGGTAGCTC TACCCCGTCTGGTGCTACTGGCTCTCCA |
| LCW0404_049_GFP-N_H10.ab1 | GSSTPSGATGSPGTPG SGTASSSPGSSTPSGA TGSP | GGTAGCTCTACCCCGTCTGGTGCTACTGGTTCTCCAGGTA CTCCGGGCAGCGGTACTGCTTCTTCCTCTCCAGGTAGCTC TACCCCTTCTGGTGCTACTGGCTCTCCA |
| LCW0404_050_GFP-N_A11.ab1 | GASPGTSSTGSPGSSP SASTGTGPGSSTPSGA TGSP | GGTGCATCTCCTGGTACCAGCTCTACTGGTTCTCCAGGTT CTAGCCCTTCTGCTTCTACCGGTACCGGTCCAGGTAGCTC TACTCCTTCTGGTGCTACCGGTTCTCCA |

| File name | Amino acid sequence | Nucteotide sequence |
|---|---|---|
| LCW0404_051_GFP-N_B11.ab1 | GSSTPSGATGSPGSSTPSGATGSPGSSTPSGATGSP | GGTAGCTCTACCCCGTCTGGTGCTACTGGCTCTCCAGGTAGCTCTACTCCTTCTGGTGCTACTGGTTCCCCAGGTAGCTCTACCCCGTCTGGTGCAACTGGCTCTCCA |
| LCW0404_052_GFP-N_C11.ab1 | GASPGTSSTGSPGTPGSGTASSSPGASPGTSSTGSP | GGTGCATCCCCGGGTACCAGCTCTACCGGTTCTCCAGGTACTCCTGGCAGCGGTACTGCATCTTCCTCTCCAGGTGCTTCTCCGGGCACCAGCTCTACTGGTTCTCCA |
| LCW0404_053_GFP-N_D11.ab1 | GSSTPSGATGSPGSSPSASTGTGPGASPGTSSTGSP | GGTAGCTCTACTCCTTCTGGTGCAACTGGTTCTCCAGGTTCTAGCCCGTCTGCATCCACTGGTACCGGTCCAGGTGCTTCCCCTGGCACCAGCTCTACCGGTTCTCCA |
| LCW0404_057_ GFP-N_E11.ab1 | GASPGTSSTGSPGSSTPSGATGSPGSSPSASTGTGP | GGTGCATCTCCTGGTACTAGCTCTACTGGTTCTCCAGGTAGCTCTACTCCGTCTGGTGCAACCGGCTCTCCAGGTTCTAGCCCTTCTGCATCTACCGGTACTGGTCCA |
| LCW0404_060_GFP-N_F11.ab1 | GTPGSGTASSSPGSSTPSGATGSPGASPGTSSTGSP | GGTACTCCTGGCAGCGGTACCGCATCTTCCTCTCCAGGTAGCTCTACTCCGTCTGGTGCAACTGGTTCCCCAGGTGCTTCTCCGGGTACCAGCTCTACCGGTTCTCCA |
| LCW0404_062_GFP-N_G11.ab1 | GSSTPSGATGSPGTPGSGTASSSPGSSTPSGATGSP | GGTAGCTCTACCCCGTCTGGTGCAACCGGCTCCCCAGGTACTCCTGGTAGCGGTACCGCTTCTTCTTCTCCAGGTAGCTCTACTCCGTCTGGTGCTACCGGCTCCCCA |
| LCW0404_066_GFP-N_H11.ab1 | GSSPSASTGTGPGSSPSASTGTGPGASPGTSSTGSP | GGTTCTAGCCCTTCTGCATCCACCGGTACCGGCCCAGGTTCTAGCCCGTCTGCTTCTACCGGTACTGGTCCAGGTGCTTCTCCGGGTACTAGCTCTACTGGTTCTCCA |
| LCW0404_067_GFP-N_A12.ab1 | GTPGSGTASSSPGSSTPSGATGSPGSNPSASTGTGP | GGTACCCCGGGTAGCGGTACCGCTTCTTCTTCTCCAGGTAGCTCTACTCCGTCTGGTGCTACCGGCTCTCCAGGTTCTAACCCCTTCTGCATCCACCGGTACCGGCCCA |
| LCW0404_068_GFP-N_B12.ab1 | GSSPSASTGTGPGSSTPSGATGSPGASPGTSSTGSP | GGTTCTAGCCCTTCTGCATCTACTGGTACTGGCCCAGGTAGCTCTACTCCTTCTGGTGCTACCGGCTCTCCAGGTGCTTCTCCGGGTACTAGCTCTACCGGTTCTCCA |
| LCW0404_069_GFP-N_C12.ab1 | GSSTPSGATGSPGASPGTSSTGSPGTPGSGTASSSP | GGTAGCTCTACCCCTTCTGGTGCAACCGGCTCTCCAGGTGCATCCCCGGGTACCAGCTCTACCGGTTCTCCAGGTACTCCGGGTAGCGGTACCGCTTCTTCCTCTCCA |

| File name | Amino acid sequence | Nucteotide sequence |
|---|---|---|
| LCW0404_070_GFP-N_D12.ab1 | GSSTPSGATGSPGSST PSGATGSPGSSTPSGA TGSP | GGTAGCTCTACTCCGTCTGGTGCAACCGGTTCCCCAGGTA GCTCTACCCCTTCTGGTGCAACCGGCTCCCCAGGTAGCTC TACCCCTTCTGGTGCAACTGGCTCTCCA |
| LCW0404_073_GFP-N_E12.ab1 | GASPGTSSTGSPGTPG SGTASSSPGSSTPSGA TGSP | GGTGCTTCTCCTGGCACTAGCTCTACCGGTTCTCCAGGTA CCCCTGGTAGCGGTACCGCATCTTCCTCTCCAGGTAGCTC TACTCCTTCTGGTGCTACTGGTTCCCCA |
| LCW0404_075_GFP-N_ F12.ab1 | GSSTPSGATGSPGSSP SASTGTGPGSSPSAST GTGP | GGTAGCTCTACCCCGTCTGGTGCTACTGGCTCCCCAGGTT CTAGCCCTTCTGCATCCACCGGTACCGGTCCAGGTTCTAG CCCGTCTGCATCTACTGGTACTGGTCCA |
| LCW0404_080_GFP-N_G12.ab1 | GASPGTSSTGSPGSSP SASTGTGPGSSPSAST GTGP | GGTGCTTCCCCGGGCACCAGCTCTACTGGTTCTCCAGGTT CTAGCCCGTCTGCTTCTACTGGTACTGGTCCAGGTTCTAG CCCTTCTGCTTCCACTGGTACTGGTCCA |
| LCW0404_081_GFP-N_H12.ab1 | GASPGTSSTGSPGSSP SASTGTGPGTPGSGT ASSSP | GGTGCTTCCCCGGGTACCAGCTCTACCGGTTCTCCAGGTT CTAGCCCTTCTGCTTCTACCGGTACCGGTCCAGGTACCCC TGGCAGCGGTACCGCATCTTCCTCTCCA |

**Example 5: Construction of XTEN_AE864**

XTEN _AE864 was constructed from serial dimerization of XTEN_AE36 to AE72, 144, 288, 576 and 864. A collection _AE72 segments was constructed from 37 different segments of XTEN_AE36. Cultures of E. coli harboring all 37 different 36-amino acid segments were mixed and plasmids were isolated. This plasmid pool was digested with BsaINcoI to generate the small fragment as the insert. The same plasmid pool was digested with BbsI/NcoI to generate the large fragment as the vector. The insert and vector fragments were ligated resulting in a doubling of the length and the ligation mixture was transformed into BL21Gold(DE3) cells to obtain colonies of XTEN_AE72.

This library of XTEN_AE72 segments was designated LCW0406. All clones from LCW0406 were combined and dimerized again using the same process as described above yielding library LCW0410 of XTEN_AE144. All clones from LCW0410 were combined and dimerized again using the same process as described above yielding library LCW0414 of XTEN_AE288. Two isolates LCW0414.001 and LCW0414.002 were randomly picked from the library and sequenced to verify the identities. All clones from LCW0414 were combined and dimerized again using the same process as described above yielding library LCW0418 of XTEN_AE576. We screened 96 isolates from library LCW0418 for high level of GFP fluorescence. 8 isolates with right sizes of inserts by PCR and strong fluorescence were sequenced and 2 isolates (LCW0418.018 and LCW0418.052) were chosen for future use based on sequencing and expression data.

The specific clone pCW0432 of XTEN AE864 was constructed by combining LCW0418.018 of XTEN_AE576 and LCW0414.002 of XTEN_AE288 using the same dimerization process as described above.

**Example 6: Construction of xTEN_AM144**

A collection _AM144 segments was constructed starting from 37 different segments of XTEN_AE36, 44 segments of XTEN_AF36, and 44 segments of XTEN_AG36.

Cultures of *E. coli* harboring all 125 different 36-amino acid segments were mixed and plasmids were isolated. This plasmid pool was digested with BsaI/NcoI to generate the small fragment as the insert. The same plasmid pool was digested with BbsI/NcoI to generate the large fragment as the vector. The insert and vector fragments were ligated resulting in a doubling of the length and the ligation mixture was transformed into BL21Gold(DE3) cells to obtain colonies of XTEN_AM72.

This library of XTEN_AM72 segments was designated LCW0461. All clones from LCW0461 were combined and dimerized again using the same process as described above yielding library LCW0462. 1512 Isolates from library LCW0462 were screened for protein expression. Individual colonies were transferred into 96 well plates and cultured overnight as starter cultures. These starter cultures were diluted into fresh autoinduction medium and cultured for 20-30h. Expression was measured using a fluorescence plate reader with excitation at 395 nm and emission at 510 nm. 192 isolates showed high level expression and were submitted to DNA sequencing. Most clones in library LCW0462 showed good expression and similar physicochemical properties suggesting that most combinations of XTEN_AM36 segments yield useful XTEN sequences. 30 isolates from LCW0462 were chosen as a preferred collection _AM144 segments for the construction of multifunctional proteins that contain multiple XTEN segments. The file names of the nucleotide and amino acid constructs for these segments are listed in Table 12.

**Table 12: DNA and amino acid sequences for AM144 segments**

| Clone | DNA Sequence | Protein Sequence |
|---|---|---|
| LCW462_r1 | GGTACCCCGGGCAGCGGTACCGCATCTTCCTCTCCAGGTAGCTCTACCCCGTCTGGTGCTACCGGTTCCCCAGGTAGCTCTACCCCGTCTGGTGCAACCGGCTCCCCAGGTAGCCCGGCTGGCTCTCCTACCTCTACTGAGGAAGGTACTTCTGAAAGCGCTACTCCTGAGTCTGGTCCAGGTACCTCTACTGAACCGTCCGAAGGTAGCGCTCCAGGTTCTAGCCCTTCTGCATCCACCGGTACCGGCCCAGGTTCTAGCCCGTCTGCTTCTACCGGTACTGGTCCAGGTGCTTCTCCGGGTACTAGCTCTACTGGTTCTCCAGGTACCTCTACCGAACCGTCCGAGGGTAGCGCACCAGGTACCTCTACTGAACCGTCTGAGGGTAGCGCTCCAGGTAGCGAACCGGCAACCTCCGGTTCTGAAACTCCA | GTPGSGTASSSPGSSTPSGATGSPGSSTPSGATGSPGSPAGSPTSTEEGTSESATPESGPGTSTEPSEGSAPGSSPSASTGTGPGSSPSASTGTGPGASPGTSSTGSPGTSTEPSEGSAPGTSTEPSEGSAPGSEPATSGSETP |

(continued)

| Clone | DNA Sequence | Protein Sequence |
|---|---|---|
| LCW462_r5 | GGTTCTACCAGCGAATCCCCTTCTGGCACTGCACCAGGTT CTACTAGCGAATCCCCTTCTGGTACCGCACCAGGTACTTC TCCGAGCGGCGAATCTTCTACTGCTCCAGGTACCTCTACT GAACCTTCCGAAGGCAGCGCTCCAGGTACCTCTACCGAA CCGTCCGAGGGCAGCGCACCAGGTACTTCTGAAAGCGCA ACCCCTGAATCCGGTCCAGGTGCATCTCCTGGTACCAGCT CTACCGGTTCTCCAGGTAGCTCTACTCCTTCTGGTGCTAC TGGCTCTCCAGGTGCTTCCCCGGGTACCAGCTCTACCGGT TCTCCAGGTTCTACTAGCGAATCTCCTTCTGGCACTGCAC CAGGTTCTACCAGCGAATCTCCGTCTGGCACTGCACCAGG TACCTCTACCCCTGAAAGCGGTTCCGCTTCTCCA | GSTSESPSGTAPGSTSES PSGTAPGTSPSGESSTAP GTSTEPSEGSAPGTSTEP SEGSAPGTSESATPESG PGASPGTSSTGSPGSSTP SGATGSPGASPGTSSTG SPGSTSESPSGTAPGSTS ESPSGTAPGTSTPESGS ASP |
| LCW462_r9 | GGTACTTCTACCGAACCTTCCGAGGGCAGCGCACCAGGT ACTTCTGAAAGCGCTACCCCTGAGTCCGGCCCAGGTACTT CTGAAAGCGCTACTCCTGAATCCGGTCCAGGTACCTCTAC TGAACCTTCTGAGGGCAGCGCTCCAGGTACTTCTGAAAG CGCTACCCCGGAGTCCGGTCCAGGTACTTCTACTGAACCG TCCGAAGGTAGCGCACCAGGTACTTCTACTGAACCTTCCG AAGGTAGCGCTCCAGGTAGCGAACCTGCTACTTCTGGTTC TGAAACCCCAGGTAGCCCGGCTGGCTCTCCGACCTCCACC GAGGAAGGTGCTTCTCCTGGCACCAGCTCTACTGGTTCTC CAGGTTCTAGCCCTTCTGCTTCTACCGGTACTGGTCCAGG TTCTAGCCCTTCTGCATCCACTGGTACTGGTCCA | GTSTEPSEGSAPGTSES ATPESGPGTSESATPES GPGTSTEPSEGSAPGTS ESATPESGPGTSTEPSEG SAPGTSTEPSEGSAPGS EPATSGSETPGSPAGSP TSTEEGASPGTSSTGSP GSSPSASTGTGPGSSPS ASTGTGP |
| LCW462_r10 | GGTAGCGAACCGGCAACCTCTGGCTCTGAAACCCCAGGT ACCTCTGAAAGCGCTACTCCGGAATCTGGTCCAGGTACTT CTGAAAGCGCTACTCCGGAATCCGGTCCAGGTTCTACCA GCGAATCTCCTTCTGGCACCGCTCCAGGTTCTACTAGCGA ATCCCCGTCTGGTACCGCACCAGGTACTTCTCCTAGCGGC GAATCTTCTACCGCACCAGGTGCATCTCCGGGTACTAGCT CTACCGGTTCTCCAGGTTCTAGCCCTTCTGCTTCCACTGGT ACCGGCCCAGGTAGCTCTACCCCGTCTGGTGCTACTGGTT CCCCAGGTAGCTCTACTCCGTCTGGTGCAACCGGTTCCCC AGGTAGCTCTACTCCTTCTGGTGCTACTGGCTCCCCAGGT GCATCCCCTGGCACCAGCTCTACCGGTTCTCCA | GSEPATSGSETPGTSES ATPESGPGTSESATPES GPGSTSESPSGTAPGSTS ESPSGTAPGTSPSGESST APGASPGTSSTGSPGSS PSASTGTGPGSSTPSGA TGSPGSSTPSGATGSPG SSTPSGATGSPGASPGT SSTGSP |
| LCW462_r15 | GGTGCTTCTCCGGGCACCAGCTCTACTGGTTCTCCAGGTT CTAGCCCTTCTGCATCCACCGGTACCGGTCCAGGTAGCTC TACCCCTTCTGGTGCAACCGGCTCTCCAGGTACTTCTGAA AGCGCTACCCCGGAATCTGGCCCAGGTAGCGAACCGGCT ACTTCTGGTTCTGAAACCCCAGGTAGCGAACCGGCTACCT CCGGTTCTGAAACTCCAGGTACTTCTGAAAGCGCTACTCC GGAGTCCGGTCCAGGTACCTCTACCGAACCGTCCGAAGG CAGCGCTCCAGGTACTTCTACTGAACCTTCTGAGGGTAGC GCTCCAGGTACCTCTACCGAACCGTCCGAGGGTAGCGCA | GASPGTSSTGSPGSSPS ASTGTGPGSSTPSGATG SPGTSESATPESGPGSEP ATSGSETPGSEPATSGS ETPGTSESATPESGPGTS TEPSEGSAPGTSTEPSEG SAPGTSTEPSEGSAPGT STEPSEGSAPGSEPATS GSETP |
| | CCAGGTACCTCTACTGAACCGTCTGAGGGTAGCGCTCCA GGTAGCGAACCGGCAACCTCCGGTTCTGAAACTCCA | |

(continued)

| Clone | DNA Sequence | Protein Sequence |
|---|---|---|
| LCW462_r16 | GGTACCTCTACCGAACCTTCCGAAGGTAGCGCTCCAGGT AGCCCGGCAGGTTCTCCTACTTCCACTGAGGAAGGTACTT CTACCGAACCTTCTGAGGGTAGCGCACCAGGTACCTCTG AAAGCGCAACTCCTGAGTCTGGCCCAGGTAGCGAACCTG CTACCTCCGGCTCTGAGACTCCAGGTACCTCTGAAAGCGC AACCCCGGAATCTGGTCCAGGTAGCCCGGCTGGCTCTCCT ACCTCTACTGAGGAAGGTACTTCTGAAAGCGCTACTCCTG AGTCTGGTCCAGGTACCTCTACTGAACCGTCCGAAGGTA GCGCTCCAGGTAGCGAACCTGCTACTTCTGGTTCTGAAAC TCCAGGTACTTCTACCGAACCGTCCGAGGGTAGCGCTCCA GGTAGCGAACCTGCTACTTCTGGTTCTGAAACTCCA | GTSTEPSEGSAPGSPAG SPTSTEEGTSTEPSEGSA PGTSESATPESGPGSEP ATSGSETPGTSESATPES GPGSPAGSPTSTEEGTS ESATPESGPGTSTEPSEG SAPGSEPATSGSETPGT STEPSEGSAPGSEPATS GSETP |
| LCW462_r20 | GGTACTTCTACCGAACCGTCCGAAGGCAGCGCTCCAGGT ACCTCTACTGAACCTTCCGAGGGCAGCGCTCCAGGTACCT CTACCGAACCTTCTGAAGGTAGCGCACCAGGTACTTCTAC CGAACCGTCCGAAGGCAGCGCTCCAGGTACCTCTACTGA ACCTTCCGAGGGCAGCGCTCCAGGTACCTCTACCGAACCT TCTGAAGGTAGCGCACCAGGTACTTCTACCGAACCTTCCG AGGGCAGCGCACCAGGTACTTCTGAAAGCGCTACCCCTG AGTCCGGCCCAGGTACTTCTGAAAGCGCTACTCCTGAATC CGGTCCAGGTACTTCTACTGAACCTTCCGAAGGTAGCGCT CCAGGTAGCGAACCTGCTACTTCTGGTTCTGAAACCCCAG GTAGCCCGGCTGGCTCTCCGACCTCCACCGAGGAA | GTSTEPSEGSAPGTSTEP SEGSAPGTSTEPSEGSA PGTSTEPSEGSAPGTSTE PSEGSAPGTSTEPSEGS APGTSTEPSEGSAPGTS ESATPESGPGTSESATPE SGPGTSTEPSEGSAPGS EPATSGSETPGSPAGSP TSTEE |
| LCW462_r23 | GGTACTTCTACCGAACCGTCCGAGGGCAGCGCTCCAGGT ACTTCTACTGAACCTTCTGAAGGCAGCGCTCCAGGTACTT CTACTGAACCTTCCGAAGGTAGCGCACCAGGTTCTACCA GCGAATCCCCTTCTGGTACTGCTCCAGGTTCTACCAGCGA ATCCCCTTCTGGCACCGCACCAGGTACTTCTACCCCTGAA AGCGGCTCCGCTTCTCCAGGTAGCGAACCTGCAACCTCTG GCTCTGAAACCCCAGGTACCTCTGAAAGCGCTACTCCTGA ATCTGGCCCAGGTACTTCTACTGAACCGTCCGAGGGCAG CGCACCAGGTACTTCTACTGAACCGTCTGAAGGTAGCGC ACCAGGTACTTCTGAAAGCGCAACCCCGGAATCCGGCCC AGGTACCTCTGAAAGCGCAACCCCGGAGTCCGGCCCA | GTSTEPSEGSAPGTSTEP SEGSAPGTSTEPSEGSA PGSTSESPSGTAPGSTSE SPSGTAPGTSTPESGSAS PGSEPATSGSETPGTSES ATPESGPGTSTEPSEGS APGTSTEPSEGSAPGTS ESATPESGPGTSESATPE SGP |
| LCW462_r24 | GGTAGCTCTACCCCTTCTGGTGCTACCGGCTCTCCAGGTT CTAGCCCGTCTGCTTCTACCGGTACCGGTCCAGGTAGCTC TACCCCTTCTGGTGCTACTGGTTCTCCAGGTAGCCCTGCT GGCTCTCCGACTTCTACTGAGGAAGGTAGCCCGGCTGGTT CTCCGACTTCTACTGAGGAAGGTACTTCTACCGAACCTTC CGAAGGTAGCGCTCCAGGTGCTTCCCCGGGCACTAGCTCT ACCGGTTCTCCAGGTTCTAGCCCTTCTGCATCTACTGGTA CTGGCCCAGGTACTCCGGGCAGCGGTACTGCTTCTTCCTC TCCAGGTTCTACTAGCTCTACTGCTGAATCTCCTGGCCCA GGTACTTCTCCTAGCGGTGAATCTTCTACCGCTCCAGGTA CCTCTACTCCGGAAAGCGGTTCTGCATCTCCA | GSSTPSGATGSPGSSPS ASTGTGPGSSTPSGATG SPGSPAGSPTSTEEGSPA GSPTSTEEGTSTEPSEGS APGASPGTSSTGSPGSS PSASTGTGPGTPGSGTA SSSPGSTSSTAESPGPGT SPSGESSTAPGTSTPESG SASP |
| LCW462_r27 | GGTACCTCTACTGAACCTTCTGAGGGCAGCGCTCCAGGTA CTTCTGAAAGCGCTACCCCGGAGTCCGGTCCAGGTACTTC TACTGAACCGTCCGAAGGTAGCGCACCAGGTACTTCTACT GAACCGTCTGAAGGTAGCGCACCAGGTACTTCTGAAAGC GCAACCCCGGAATCCGGCCCAGGTACCTCTGAAAGCGCA ACCCCGGAGTCCGGCCCAGGTACTCCTGGCAGCGGTACC GCTTCTTCTTCTCCAGGTGCTTCTCCTGGTACTAGCTCTAC TGGTTCTCCAGGTGCTTCTCCGGGCACTAGCTCTACTGGT TCTCCAGGTAGCCCTGCTGGCTCTCCGACTTCTACTGAGG AAGGTAGCCCGGCTGGTTCTCCGACTTCTACTGAGGAAG GTACTTCTACCGAACCTTCCGAAGGTAGCGCTCCA | GTSTEPSEGSAPGTSES ATPESGPGTSTEPSEGS APGTSTEPSEGSAPGTS ESATPESGPGTSESATPE SGPGTPGSGTASSSPGA SPGTSSTGSPGASPGTSS TGSPGSPAGSPTSTEEG SPAGSPTSTEEGTSTEPS EGSAP |

(continued)

| Clone | DNA Sequence | Protein Sequence |
|---|---|---|
| LCW462_r28 | GGTAGCCCAGCAGGCTCTCCGACTTCCACTGAGGAAGGT ACTTCTACTGAACCTTCCGAAGGCAGCGCACCAGGTACCT CTACTGAACCTTCTGAGGGCAGCGCTCCAGGTACCTCTAC | GSPAGSPTSTEEGTSTEP SEGSAPGTSTEPSEGSA PGTSTEPSEGSAPGTSES |
|  | CGAACCGTCTGAAGGTAGCGCACCAGGTACCTCTGAAAG CGCAACTCCTGAGTCCGGTCCAGGTACTTCTGAAAGCGC AACCCCGGAGTCTGGCCCAGGTACCCCGGGTAGCGGTAC TGCTTCTTCCTCTCCAGGTAGCTCTACCCCTTCTGGTGCAA CCGGCTCTCCAGGTGCTTCTCCGGGCACCAGCTCTACCGG TTCTCCAGGTACCTCTACTGAACCTTCTGAGGGCAGCGCT CCAGGTACTTCTGAAAGCGCTACCCCGGAGTCCGGTCCA GGTACTTCTACTGAACCGTCCGAAGGTAGCGCACCA | ATPESGPGTSESATPES GPGTPGSGTASSSPGSS TPSGATGSPGASPGTSS TGSPGTSTEPSEGSAPG TSESATPESGPGTSTEPS EGSAP |
| LCW462_r38 | GGTAGCGAACCGGCAACCTCCGGCTCTGAAACTCCAGGT ACTTCTGAAAGCGCTACTCCGGAATCCGGCCCAGGTAGC GAACCGGCTACTTCCGGCTCTGAAACCCCAGGTAGCTCTA CCCCGTCTGGTGCAACCGGCTCCCCAGGTACTCCTGGTAG CGGTACCGGCTTCTTCTTCTCCAGGTAGCTCTACTCCGTCTG GTGCTACCGGCTCCCCAGGTGCATCTCCTGGTACCAGCTC TACCGGTTCTCCAGGTAGCTCTACTCCTTCTGGTGCTACT GGCTCTCCAGGTGCTTCCCCGGGTACCAGCTCTACCGGTT CTCCAGGTAGCGAACCTGCTACTTCTGGTTCTGAAACTCC AGGTACTTCTACCGAACCGTCCGAGGGTAGCGCTCCAGG TAGCGAACCTGCTACTTCTGGTTCTGAAACTCCA | GSEPATSGSETPGTSES ATPESGPGSEPATSGSE TPGSSTPSGATGSPGTP GSGTASSSPGSSTPSGA TGSPGASPGTSSTGSPG SSTPSGATGSPGASPGT SSTGSPGSEPATSGSETP GTSTEPSEGSAPGSEPA TSGSETP |
| LCW462_r39 | GGTACCTCTACTGAACCTTCCGAAGGCAGCGCTCCAGGT ACCTCTACCGAACCGTCCGAGGGCAGCGCACCAGGTACT TCTGAAAGCGCAACCCCTGAATCCGGTCCAGGTAGCCCT GCTGGCTCTCCGACTTCTACTGAGGAAGGTAGCCCGGCTG GTTCTCCGACTTCTACTGAGGAAGGTACTTCTACCGAACC TTCCGAAGGTAGCGCTCCAGGTAGCCCGGCTGGTTCTCCG ACTTCCACCGAGGAAGGTACCTCTACTGAACCTTCTGAGG GTAGCGCTCCAGGTACCTCTACTGAACCTTCCGAAGGCA GCGCTCCAGGTGCTTCCCCGGGCACCAGCTCTACTGGTTC TCCAGGTTCTAGCCCGTCTGCTTCTACTGGTACTGGTCCA GGTTCTAGCCCTTCTGCTTCCACTGGTACTGGTCCA | GTSTEPSEGSAPGTSTEP SEGSAPGTSESATPESG PGSPAGSPTSTEEGSPA GSPTSTEEGTSTEPSEGS APGSPAGSPTSTEEGTS TEPSEGSAPGTSTEPSEG SAPGASPGTSSTGSPGS SPSASTGTGPGSSPSAST GTGP |
| LCW462_r41 | GGTAGCTCTACCCCGTCTGGTGCTACCGGTTCCCCAGGTG CTTCTCCTGGTACTAGCTCTACCGGTTCTCCAGGTAGCTC TACCCCGTCTGGTGCTACTGGCTCTCCAGGTAGCCCTGCT GGCTCTCCAACCTCCACCGAAGAAGGTACCTCTGAAAGC GCAACCCCTGAATCCGGCCCAGGTAGCGAACCGGCAACC TCCGGTTCTGAAACCCCAGGTGCATCTCCTGGTACTAGCT CTACTGGTTCTCCAGGTAGCTCTACTCCGTCTGGTGCAAC CGGCTCTCCAGGTTCTAGCCCTTCTGCATCTACCGGTACT GGTCCAGGTTCTACCAGCGAATCCCCTTCTGGTACTGCTC CAGGTTCTACCAGCGAATCCCCTTCTGGCACCGCACCAGG TACTTCTACCCCTGAAAGCGGCTCCGCTTCTCCA | GSSTPSGATGSPGASPG TSSTGSPGSSTPSGATGS PGSPAGSPTSTEEGTSES ATPESGPGSEPATSGSE TPGASPGTSSTGSPGSST PSGATGSPGSSPSASTG TGPGSTSESPSGTAPGS TSESPSGTAPGTSTPESG SASP |

(continued)

| Clone | DNA Sequence | Protein Sequence |
|---|---|---|
| LCW462_r42 | GGTTCTACCAGCGAATCTCCTTCTGGCACCGCTCCAGGTT CTACTAGCGAATCCCCGTCTGGTACCGCACCAGGTACTTC TCCTAGCGGCGAATCTTCTACCGCACCAGGTACCTCTGAA AGCGCTACTCCGGAGTCTGGCCCAGGTACCTCTACTGAAC CGTCTGAGGGTAGCGCTCCAGGTACTTCTACTGAACCGTC CGAAGGTAGCGCACCAGGTACCTCTACTGAACCTTCTGA GGGCAGCGCTCCAGGTACTTCTGAAAGCGCTACCCCGGA GTCCGGTCCAGGTACTTCTACTGAACCGTCCGAAGGTAGC GCACCAGGTAGCTCTACCCCGTCTGGTGCTACCGGTTCCC CAGGTGCTTCTCCTGGTACTAGCTCTACCGGTTCTCCAGG TAGCTCTACCCCGTCTGGTGCTACTGGCTCTCCA | GSTSESPSGTAPGSTSES PSGTAPGTSPSGESSTAP GTSESATPESGPGTSTEP SEGSAPGTSTEPSEGSA PGTSTEPSEGSAPGTSES ATPESGPGTSTEPSEGS APGSSTPSGATGSPGAS PGTSSTGSPGSSTPSGAT GSP |
| LCW462_r43 | GGTTCTACTAGCTCTACTGCAGAATCTCCGGGCCCAGGTA CCTCTCCTAGCGGTGAATCTTCTACCGCTCCAGGTACTTC TCCGAGCGGTGAATCTTCTACCGCTCCAGGTTCTACTAGC TCTACCGCTGAATCTCCGGGTCCAGGTTCTACCAGCTCTA CTGCAGAATCTCCTGGCCCAGGTACTTCTACTCCGGAAAG CGGTTCCGCTTCTCCAGGTACTTCTCCTAGCGGTGAATCT TCTACCGCTCCAGGTTCTACCAGCTCTACTGCTGAATCTC CTGGCCCAGGTACTTCTACCCCGGAAAGCGGCTCCGCTTC | GSTSSTAESPGPGTSPSG ESSTAPGTSPSGESSTAP GTSSTAESPGPGTSSST AESPGPGTSTPESGSASP GTSPSGESSTAPGTSSST AESPGPGTSTPESGSASP GSTSSTAESPGPGTSES PSGTAPGTSPSGESSTAP |
| | TCCAGGTTCTACCAGCTCTACCGCTGAATCTCCTGGCCCA GGTTCTACTAGCGAATCTCCGTCTGGCACCGCACCAGGTA CTTCCCCTAGCGGTGAATCTTCTACTGCACCA | |
| LCW462_r45 | GGTACCTCTACTCCGGAAAGCGGTTCCGCATCTCCAGGTT CTACCAGCGAATCCCCGTCTGGCACCGCACCAGGTTCTAC TAGCTCTACTGCTGAATCTCCGGGCCCAGGTACCTCTACT GAACCTTCCGAAGGCAGCGCTCCAGGTACCTCTACCGAA CCGTCCGAGGGCAGCGCACCAGGTACTTCTGAAAGCGCA ACCCCTGAATCCGGTCCAGGTACCTCTGAAAGCGCTACTC CGGAGTCTGGCCCAGGTACCTCTACTGAACCGTCTGAGG GTAGCGCTCCAGGTACTTCTACTGAACCGTCCGAAGGTA GCGCACCAGGTACTTCTGAAAGCGCTACTCCGGAGTCCG GTCCAGGTACCTCTACCGAACCGTCCGAAGGCAGCGCTC CAGGTACTTCTACTGAACCTTCTGAGGGTAGCGCTCCC | GTSTPESGSASPGSTSES PSGTAPGSTSSTAESPGP GTSTEPSEGSAPGTSTEP SEGSAPGTSESATPESG PGTSESATPESGPGTSTE PSEGSAPGTSTEPSEGS APGTSESATPESGPGTS TEPSEGSAPGTSTEPSEG SAP |
| LCW462_ r47 | GGTACCTCTACCGAACCGTCCGAGGGTAGCGCACCAGGT ACCTCTACTGAACCGTCTGAGGGTAGCGCTCCAGGTAGC GAACCGGCAACCTCCGGTTCTGAAACTCCAGGTACTTCTA CTGAACCGTCTGAAGGTAGCGCACCAGGTACTTCTGAAA GCGCAACCCCGGAATCCGGCCCAGGTACCTCTGAAAGCG CAACCCCGGAGTCCGGCCCAGGTGCATCTCCGGGTACTA GCTCTACCGGTTCTCCAGGTTCTAGCCCTTCTGCTTCCACT GGTACCGGCCCAGGTAGCTCTACCCCGTCTGGTGCTACTG GTTCCCCAGGTAGCTCTACTCCGTCTGGTGCAACCGGTTC CCCAGGTAGCTCTACTCCTTCTGGTGCTACTGGCTCCCCA GGTGCATCCCCTGGCACCAGCTCTACCGGTTCTCCA | GTSTEPSEGSAPGTSTEP SEGSAPGSEPATSGSET PGTSTEPSEGSAPGTSES ATPESGPGTSESATPES GPGASPGTSSTGSPGSS PSASTGTPGSSTPSGA TGSPGSSTPSGATGSPG SSTPSGATGSPGASPGT SSTGSP |

(continued)

| Clone | DNA Sequence | Protein Sequence |
|---|---|---|
| LCW462_r54 | GGTAGCGAACCGGCAACCTCTGGCTCTGAAACTCCAGGT AGCGAACCTGCAACCTCCGGCTCTGAAACCCCAGGTACT TCTACTGAACCTTCTGAGGGCAGCGCACCAGGTAGCGAA CCTGCAACCTCTGGCTCTGAAACCCCAGGTACCTCTGAAA GCGCTACTCCTGAATCTGGCCCAGGTACTTCTACTGAACC GTCCGAGGGCAGCGCACCAGGTAGCTCTACTCCGTCTGG TGCTACCGGCTCTCCAGGTAGCTCTACCCCTTCTGGTGCA ACCGGCTCCCCAGGTGCTTCTCCGGGTACCAGCTCTACTG GTTCTCCAGGTAGCTCTACCCCGTCTGGTGCTACCGGTTC CCCAGGTGCTTCTCCTGGTACTAGCTCTACCGGTTCTCCA GGTAGCTCTACCCCGTCTGGTGCTACTGGCTCTCCA | GSEPATSGSETPGSEPA TSGSETPGTSTEPSEGSA PGSEPATSGSETPGTSES ATPESGPGTSTEPSEGS APGSSTPSGATGSPGSS TPSGATGSPGASPGTSS TGSPGSSTPSGATGSPG ASPGTSSTGSPGSSTPSG ATGSP |
| LCW462_r55 | GGTACTTCTACCGAACCGTCCGAGGGCAGCGCTCCAGGT ACTTCTACTGAACCTTCTGAAGGCAGCGCTCCAGGTACTT CTACTGAACCTTCCGAAGGTAGCGCACCAGGTACTTCTGA AAGCGCTACTCCGGAGTCCGGTCCAGGTACCTCTACCGA ACCGTCCGAAGGCAGCGCTCCAGGTACTTCTACTGAACCT TCTGAGGGTAGCGCTCCAGGTTCTACTAGCGAATCTCCGT CTGGCACTGCTCCAGGTACTTCTCCTAGCGGTGAATCTTC TACCGCTCCAGGTACTTCCCCTAGCGGCGAATCTTCTACC GCTCCAGGTAGCCCGGCTGGCTCTCCTACCTCTACTGAGG AAGGTACTTCTGAAAGCGCTACTCCTGAGTCTGGTCCAGG TACCTCTACTGAACCGTCCGAAGGTAGCGCTCCA | GTSTEPSEGSAPGTSTEP SEGSAPGTSTEPSEGSA PGTSESATPESGPGTSTE PSEGSAPGTSTEPSEGS APGSTSESPSGTAPGTSP SGESSTAPGTSPSGESST APGSPAGSPTSTEEGTS ESATPESGPGTSTEPSEG SAP |
| LCW462_r57 | GGTACTTCTACTGAACCTTCCGAAGGTAGCGCTCCAGGTA GCGAACCTGCTACTTCTGGTTCTGAAACCCCAGGTAGCCC GGCTGGCTCTCCGACCTCCACCGAGGAAGGTAGCCCGGC AGGCTCTCCGACCTCTACTGAGGAAGGTACTTCTGAAAG CGCAACCCCGGAGTCCGGCCCAGGTACCTCTACCGAACC GTCTGAGGGCAGCGCACCAGGTACCTCTACTGAACCTTCC GAAGGCAGCGCTCCAGGTACCTCTACCGAACCGTCCGAG GGCAGCGCACCAGGTACTTCTGAAAGCGCAACCCCTGAA TCCGGTCCAGGTAGCTCTACTCCGTCTGGTGCAACCGGCT CCCCAGGTTCTAGCCCGTCTGCTTCCACTGGTACTGGCCC AGGTGCTTCCCCGGGCACCAGCTCTACTGGTTCTCCA | GTSTEPSEGSAPGSEPA TSGSETPGSPAGSPTSTE EGSPAGSPTSTEEGTSES ATPESGPGTSTEPSEGS APGTSTEPSEGSAPGTS TEPSEGSAPGTSESATPE SGPGSSTPSGATGSPGS SPSASTGTGPGASPGTS STGSP |
| LCW462_r61 | GGTAGCGAACCGGCTACTTCCGGCTCTGAGACTCCAGGT AGCCCTGCTGGCTCTCCGACCTCTACCGAAGAAGGTACCT | GSEPATSGSETPGSPAG SPTSTEEGTSESATPESG |
| | CTGAAAGCGCTACCCCTGAGTCTGGCCCAGGTACCTCTAC TGAACCTTCCGAAGGCAGCGCTCCAGGTACCTCTACCGA ACCGTCCGAGGGCAGCGCACCAGGTACTTCTGAAAGCGC AACCCCTGAATCCGGTCCAGGTACCTCTACTCCGGAAAG CGGTTCCGCATCTCCAGGTTCTACCAGCGAATCCCCGTCT GGCACCGCACCAGGTTCTACTAGCTCTACTGCTGAATCTC CGGGCCCAGGTACTTCTGAAAGCGCTACTCCGGAGTCCG GTCCAGGTACCTCTACCGAACCGTCCGAAGGCAGCGCTC CAGGTACTTCTACTGAACCTTCTGAGGGTAGCGCTCCA | PGTSTEPSEGSAPGTSTE PSEGSAPGTSESATPES GPGTSTPESGSASPGSTS ESPSGTAPGSTSSTAESP GPGTSESATPESGPGTS TEPSEGSAPGTSTEPSEG SAP |

(continued)

| Clone | DNA Sequence | Protein Sequence |
|---|---|---|
| LCW462_r64 | GGTACTTCTACCGAACCGTCCGAGGGCAGCGCTCCAGGT ACTTCTACTGAACCTTCTGAAGGCAGCGCTCCAGGTACTT CTACTGAACCTTCCGAAGGTAGCGCACCAGGTACCTCTAC CGAACCGTCTGAAGGTAGCGCACCAGGTACCTCTGAAAG CGCAACTCCTGAGTCCGTCCAGGTACTTCTGAAAGCGC AACCCCGGAGTCTGGCCCAGGTACTCCTGGCAGCGGTAC CGCATCTTCCTCTCCAGGTAGCTCTACTCCGTCTGGTGCA ACTGGTTCCCCAGGTGCTTCTCCGGGTACCAGCTCTACCG GTTCTCCAGGTTCCACCAGCTCTACTGCTGAATCTCCTGG TCCAGGTACCTCTCCTAGCGGTGAATCTTCTACTGCTCCA GGTACTTCTACTCCTGAAAGCGGCTCTGCTTCTCCA | GTSTEPSEGSAPGTSTEP SEGSAPGTSTEPSEGSA PGTSTEPSEGSAPGTSES ATPESGPGTSESATPES GPGTPGSGTASSSPGSS TPSGATGSPGASPGTSS TGSPGSTSSTAESPGPG TSPSGESSTAPGTSTPES GSASP |
| LCW462_r67 | GGTAGCCCGGCAGGCTCTCCGACCTCTACTGAGGAAGGT ACTTCTGAAAGCGCAACCCCGGAGTCCGGCCCAGGTACC TCTACCGAACCGTCTGAGGGCAGCGCACCAGGTACTTCT GAAAGCGCAACCCCTGAATCCGGTCCAGGTAGCGAACCG GCTACTTCTGGCTCTGAGACTCCAGGTACTTCTACCGAAC CGTCCGAAGGTAGCGCACCAGGTAGCCCGGCTGGTTCTC CGACTTCCACCGAGGAAGGTACCTCTACTGAACCTTCTGA GGGTAGCGCTCCAGGTACCTCTACTGAACCTTCCGAAGG CAGCGCTCCAGGTACTTCTACCGAACCGTCCGAGGGCAG CGCTCCAGGTACTTCTACTGAACCTTCTGAAGGCAGCGCT CCAGGTACTTCTACTGAACCTTCCGAAGGTAGCGCACCA | GSPAGSPTSTEEGTSES ATPESGPGTSTEPSEGS APGTSESATPESGPGSE PATSGSETPGTSTEPSEG SAPGSPAGSPTSTEEGT STEPSEGSAPGTSTEPSE GSAPGTSTEPSEGSAPG TSTEPSEGSAPGTSTEPS EGSAP |
| LCW462_ r69 | GGTACTTCTCCGAGCGGTGAATCTTCTACCGCACCAGGTT CTACTAGCTCTACCGCTGAATCTCCGGGCCCAGGTACTTC TCCGAGCGGTGAATCTTCTACTGCTCCAGGTACCTCTGAA AGCGCTACTCCGGAGTCTGGCCCAGGTACCTCTACTGAAC CGTCTGAGGGTAGCGCTCCAGGTACTTCTACTGAACCGTC CGAAGGTAGCGCACCAGGTTCTAGCCCTTCTGCATCTACT GGTACTGGCCCAGGTAGCTCTACTCCTTCTGGTGCTACCG GCTCTCCAGGTGCTTCTCCGGGTACTAGCTCTACCGGTTC TCCAGGTACTTCTACTCCGGAAAGCGGTTCCGCATCTCCA GGTACTTCTCCTAGCGGTGAATCTTCTACTGCTCCAGGTA CCTCTCCTAGCGGCGAATCTTCTACTGCTCCA | GTSPSGESSTAPGSTSST AESPGPGTSPSGESSTAP GTSESATPESGPGTSTEP SEGSAPGTSTEPSEGSA PGSSPSASTGTGPGSSTP SGATGSPGASPGTSSTG SPGTSTPESGSASPGTSP SGESSTAPGTSPSGESST AP |
| LCW462_r70 | GGTACCTCTGAAAGCGCTACTCCGGAGTCTGGCCCAGGT ACCTCTACTGAACCGTCTGAGGGTAGCGCTCCAGGTACTT CTACTGAACCGTCCGAAGGTAGCGCACCAGGTAGCCCTG CTGGCTCTCCGACTTCTACTGAGGAAGGTAGCCCGGCTGG TTCTCCGACTTCTACTGAGGAAGGTACTTCTACCGAACCT TCCGAAGGTAGCGCTCCAGGTTCTAGCCCTTCTGCTTCCA CCGGTACTGGCCCAGGTAGCTCTACCCCTTCTGGTGCTAC CGGCTCCCCAGGTAGCTCTACTCCTTCTGGTGCAACTGGC TCTCCAGGTAGCGAACCGGCAACTTCCGGCTCTGAAACC CCAGGTACTTCTGAAAGCGCTACTCCTGAGTCTGGCCCAG GTAGCGAACCTGCTACCTCTGGCTCTGAAACCCCA | GTSESATPESGPGTSTEP SEGSAPGTSTEPSEGSA PGSPAGSPTSTEEGSPA GSPTSTEEGTSTEPSEGS APGSSPSASTGTGPGSS TPSGATGSPGSSTPSGA TGSPGSEPATSGSETPG TSESATPESGPGSEPATS GSETP |
| LCW462_r72 | GGTACTTCTACCGAACCGTCCGAAGGCAGCGCTCCAGGT ACCTCTACTGAACCTTCCGAGGGCAGCGCTCCAGGTACCT CTACCGAACCTTCTGAAGGTAGCGCACCAGGTAGCTCTA CCCCGTCTGGTGCTACCGGTTCCCCAGGTGCTTCTCCTGG TACTAGCTCTACCGGTTCTCCAGGTAGCTCTACCCCGTCT GGTGCTACTGGCTCTCCAGGTACTTCTGAAAGCGCAACCC CTGAATCCGGTCCAGGTAGCGAACCGGCTACTTCTGGCTC | GTSTEPSEGSAPGTSTEP SEGSAPGTSTEPSEGSA PGSSTPSGATGSPGASP GTSSTGSPGSSTPSGAT GSPGTSESATPESGPGS EPATSGSETPGTSTEPSE GSAPGSTSESPSGTAPG |

143

(continued)

| Clone | DNA Sequence | Protein Sequence |
|---|---|---|
| | TGAGACTCCAGGTACTTCTACCGAACCGTCCGAAGGTAG CGCACCAGGTTCTACTAGCGAATCTCCTTCTGGCACTGCA CCAGGTTCTACCAGCGAATCTCCGTCTGGCACTGCACCAG GTACCTCTACCCCTGAAAGCGGTTCCGCTTCTCCA | STSESPSGTAPGTSTPES GSASP |
| LCW462_r73 | GGTACCTCTACTCCTGAAAGCGGTTCTGCATCTCCAGGTT CCACTAGCTCTACCGCAGAATCTCCGGGCCCAGGTTCTAC TAGCTCTACTGCTGAATCTCCTGGCCCAGGTTCTAGCCCT TCTGCATCTACTGGTACTGGCCCAGGTAGCTCTACTCCTT CTGGTGCTACCGGCTCTCCAGGTGCTTCTCCGGGTACTAG CTCTACCGGTTCTCCAGGTAGCGAACCGGCAACCTCCGGC TCTGAAACCCCAGGTACCTCTGAAAGCGCTACTCCTGAAT CCGGCCCAGGTAGCCCGGCAGGTTCTCCGACTTCCACTGA GGAAGGTTCTACTAGCGAATCTCCTTCTGGCACTGCACCA GGTTCTACCAGCGAATCTCCGTCTGGCACTGCACCAGGTA CCTCTACCCCTGAAAGCGGTTCCGCTTCTCCC | GTSTPESGSASPGSTSST AESPGPGSTSSTAESPGP GSSPSASTGTGPGSSTPS GATGSPGASPGTSSTGS PGSEPATSGSETPGTSES ATPESGPGSPAGSPTST EEGSTSESPSGTAPGSTS ESPSGTAPGTSTPESGS ASP |
| LCW462_r78 | GGTAGCCCGGCTGGCTCTCCTACCTCTACTGAGGAAGGTA CTTCTGAAAGCGCTACTCCTGAGTCTGGTCCAGGTACCTC TACTGAACCGTCCGAAGGTAGCGCTCCAGGTTCTACCAG CGAATCTCCTTCTGGCACCGCTCCAGGTTCTACTAGCGAA TCCCCGTCTGGTACCGCACCAGGTACTTCTCCTAGCGGCG AATCTTCTACCGCACCAGGTACCTCTACCGAACCTTCCGA AGGTAGCGCTCCAGGTAGCCCGGCAGGTTCTCCTACTTCC ACTGAGGAAGGTACTTCTACCGAACCTTCTGAGGGTAGC GCACCAGGTAGCGAACCTGCAACCTCTGGCTCTGAAACC CCAGGTACCTCTGAAAGCGCTACTCCTGAATCTGGCCCAG GTACTTCTACTGAACCGTCCGAGGGCAGCGCACCA | GSPAGSPTSTEEGTSES ATPESGPGTSTEPSEGS APGSTSESPSGTAPGSTS ESPSGTAPGTSPSGESST APGTSTEPSEGSAPGSP AGSPTSTEEGTSTEPSE GSAPGSEPATSGSETPG TSESATPESGPGTSTEPS EGSAP |
| LCW462_r79 | GGTACCTCTACCGAACCTTCCGAAGGTAGCGCTCCAGGT AGCCCGGCAGGTTCTCCTACTTCCACTGAGGAAGGTACTT CTACCGAACCTTCTGAGGGTAGCGCACCAGGTACCTCCCC TAGCGGCGAATCTTCTACTGCTCCAGGTACCTCTCCTAGC GGCGAATCTTCTACCGCTCCAGGTACCTCCCCTAGCGGTG AATCTTCTACCGCACCAGGTTCTACCAGCGAATCCCCTTC TGGTACTGCTCCAGGTTCTACCAGCGAATCCCCTTCTGGC ACCGCACCAGGTACTTCTACCCCTGAAAGCGGCTCCGCTT CTCCAGGTAGCGAACCTGCAACCTCTGGCTCTGAAACCCC AGGTACCTCTGAAAGCGCTACTCCTGAATCTGGCCCAGGT ACTTCTACTGAACCGTCCGAGGGCAGCGCACCA | GTSTEPSEGSAPGSPAG SPTSTEEGTSTEPSEGSA PGTSPSGESSTAPGTSPS GESSTAPGTSPSGESST APGSTSESPSGTAPGSTS ESPSGTAPGTSTPESGS ASPGSEPATSGSETPGT SESATPESGPGTSTEPSE GSAP |
| LCW462_r87 | GGTAGCGAACCGGCAACCTCTGGCTCTGAAACCCCAGGT ACCTCTGAAAGCGCTACTCCGGAATCTGGTCCAGGTACTT CTGAAAGCGCTACTCCGGAATCCGGTCCAGGTACTTCTCC GAGCGGTGAATCTTCTACCGCACCAGGTTCTACTAGCTCT ACCGCTGAATCTCCGGGCCCAGGTACTTCTCCGAGCGGTG AATCTTCTACTGCTCCAGGTTCTACTAGCGAATCCCCGTC TGGTACTGCTCCAGGTACTTCCCCTAGCGGTGAATCTTCT ACTGCTCCAGGTTCTACCAGCTCTACCGCAGAATCTCCGG GTCCAGGTAGCTCTACTCCGTCTGGTGCAACCGGTTCCCC AGGTAGCTCTACCCCTTCTGGTGCAACCGGCTCCCCAGGT AGCTCTACCCCTTCTGGTGCAAACTGGCTCTCC | GSEPATSGSETPGTSES ATPESGPGTSESATPES GPGTSPSGESSTAPGSTS STAESPGPGTSPSGESST APGSTSESPSGTAPGTSP SGESSTAPGSTSSTAESP GPGSSTPSGATGSPGSS TPSGATGSPGSSTPSGA NWLS |

(continued)

| Clone | DNA Sequence | Protein Sequence |
|---|---|---|
| LCW462_r88 | GGTAGCCCTGCTGGCTCTCCGACTTCTACTGAGGAAGGTA GCCCGGCTGGTTCTCCGACTTCTACTGAGGAAGGTACTTC TACCGAACCTTCCGAAGGTAGCGCTCCAGGTACCTCTACT GAACCTTCCGAAGGCAGCGCTCCAGGTACCTCTACCGAA CCGTCCGAGGGCAGCGCACCAGGTACTTCTGAAAGCGCA ACCCCTGAATCCGGTCCAGGTGCATCTCCTGGTACCAGCT CTACCGGTTCTCCAGGTAGCTCTACTCCTTCTGGTGCTAC TGGCTCTCCAGGTGCTTCCCCGGGTACCAGCTCTACCGGT TCTCCAGGTAGCTCTACCCCGTCTGGTGCTACTGGTTCTC CAGGTACTCCGGGCAGCGGTACTGCTTCTTCCTCTCCAGG TAGCTCTACCCCTTCTGGTGCTACTGGCTCTCCA | GSPAGSPTSTEEGSPAG SPTSTEEGTSTEPSEGSA PGTSTEPSEGSAPGTSTE PSEGSAPGTSESATPES GPGASPGTSSTGSPGSS TPSGATGSPGASPGTSS TGSPGSSTPSGATGSPG TPGSGTASSSPGSSTPSG ATGSP |
| LCW462_r89 | GGTAGCTCTACCCCGTCTGGTGCTACTGGTTCTCCAGGTA | GSSTPSGATGSPGTPGS |
| | CTCCGGGCAGCGGTACTGCTTCTTCCTCTCCAGGTAGCTC TACCCCTTCTGGTGCTACTGGCTCTCCAGGTAGCCCGGCT GGCTCTCCTACCTCTACTGAGGAAGGTACTTCTGAAAGCG CTACTCCTGAGTCTGGTCCAGGTACCTCTACTGAACCGTC CGAAGGTAGCGCTCCAGGTACCTCTGAAAGCGCAACTCC TGAGTCTGGCCCAGGTAGCGAACCTGCTACCTCCGGCTCT GAGACTCCAGGTACCTCTGAAAGCGCAACCCCGGAATCT GGTCCAGGTACTTCTACTGAACCGTCTGAAGGTAGCGCA CCAGGTACTTCTGAAAGCGCAACCCCGGAATCCGGCCCA GGTACCTCTGAAAGCGCAACCCCGGAGTCCGGCCCA | GTASSSPGSSTPSGATG SPGSPAGSPTSTEEGTSE SATPESGPGTSTEPSEGS APGTSESATPESGPGSE PATSGSETPGTSESATPE SGPGTSTEPSEGSAPGT SESATPESGPGTSESATP ESGP |

## Example 7: Construction of XTEN_AM288

The entire library LCW0462 was dimerized as described in Example 6 resulting in a library of XTEN_AM288 clones designated LCW0463. 1512 isolates from library LCW0463 were screened using the protocol described in Example 6. 176 highly expressing clones were sequenced and 40 preferred XTEN_AM288 segments were chosen for the construction of multifunctional proteins that contain multiple XTEN segments with 288 amino acid residues.

## Example 8: Construction of XTEN_AM432

We generated a library of XTEN_AM432 segments by recombining segments from library LCW0462 of XTEN_AM144 segments and segments from library LCW0463 of XTEN_AM288 segments. This new library of XTEN_AM432 segment was designated LCW0464. Plasmid was isolated from cultures of E. coli harboring LCW0462 and LCW0463, respectively. 1512 isolates from library LCW0464 were screened using the protocol described in Example 6. 176 highly expressing clones were sequenced and 39 preferred XTEN_AM432 segment were chosen for the construction of longer XTENs and for the construction of multifunctional proteins that contain multiple XTEN segments with 432 amino acid residues.

In parallel we constructed library LMS0100 of XTEN_AM432 segments using preferred segments of XTEN AM144 and XTEN AM288. Screening of this library yielded 4 isolates that were selected for further construction

## Example 9: Construction of XTEN_AM875

The stuffer vector pCW0359 was digested with BsaI and KpnI to remove the stuffer segment and the resulting vector fragment was isolated by agarose gel purification.

We annealed the phosphorylated oligonucleotide BsaI-AscI-KpnIforP: AGGTGCAAGCGCAAGCGGCGCGCCAAGCACGGGAGGTTCGTCTTCACTCGAGGGTAC and the non-phosphorylated oligonucleotide BsaI-AscI-KpnIrev: CCTCGAGTGAAGACGAACCTCCCGTGCTTGGCGCGCCGCTTGCGCTTGC for introducing the sequencing island A (SI-A) which encodes amino acids GASASGAPSTG and has the restriction enzyme AscI recognition nucleotide sequence GGCGCGCC inside. The annealed oligonucleotide pairs were ligated with BsaI and KpnI digested stuffer vector pCW0359 prepared above to yield pCW0466 containing SI-A. We then generated a library of XTEN_AM443 segments by recombining 43 preferred XTEN_AM432 segments from Example 8 and SI-A segments from pCW0466 at C-terminus using the same dimerization process described in Example 5. This new library of XTEN_AM443 segments was designated LCW0479.

We generated a library of XTEN_AM875 segments by recombining segments from library LCW0479 of XTEN_AM443 segments and 43 preferred XTEN_AM432 segments from Example 8 using the same dimerization process described in Example 5. This new library of XTEN_AM875 segment was designated LCW0481.

### Example 10: Construction of XTEN_AM1318

We annealed the phosphorylated oligonucleotide BsaI-FseI-KpnIforP: AGGTCCAGAACCAACGGGGCCGGCCCCAAGCGGAGGTTCGTCTTCACTCGAGGGTAC and the non-phosphorylated oligonucleotide BsaI-FseI-KpnIrev: CCTCGAGTGAAGACGAACCTCCGCTTGGGGCCGGCCCCGTTGGTTCTGG for introducing the sequencing island B (SI-B) which encodes amino acids GPEPTGPAPSG and has the restriction enzyme FseI recognition nucleotide sequence GGCCGGCC inside. The annealed oligonucleotide pairs were ligated with BsaI and KpnI digested stuffer vector pCW0359 as used in Example 9 to yield pCW0467 containing SI-B. We then generated a library of XTEN_AM443 segments by recombining 43 preferred XTEN_AM432 segments from Example 8 and SI-B segments from pCW0467 at C-terminus using the same dimerization process described in Example 5. This new library of XTEN_AM443 segments was designated LCW0480.

We generated a library of XTEN _AM1318 segments by recombining segments from library LCW0480 of XTEN_AM443 segments and segments from library LCW0481 of XTEN_AM875 segments using the same dimerization process as in Example 5. This new library of XTEN_AM1318 segment was designated LCW0487.

### Example 11: Construction of XTEN_AD864

Using the several consecutive rounds of dimerization, we assembled a collection of XTEN_AD864 sequences starting from segments of XTEN_AD36 listed in Example 1. These sequences were assembled as described in Example 5. Several isolates from XTEN_AD864 were evaluated and found to show good expression and excellent solubility under physiological conditions. One intermediate construct of XTEN_AD576 was sequenced. This clone was evaluated in a PK experiment in cynomolgus monkeys and a half-life of about 20h was measured.

### Example 12: Construction of XTEN_AF864

Using the several consecutive rounds of dimerization, we assembled a collection of XTEN_AF864 sequences starting from segments of XTEN_AF36 listed in Example 3. These sequences were assembled as described in Example 5. Several isolates from XTEN_AF864 were evaluated and found to show good expression and excellent solubility under physiological conditions. One intermediate construct of XTEN_AF540 was sequenced. This clone was evaluated in a PK experiment in cynomolgus monkeys and a half-life of about 20h was measured. A full length clone of XTEN_AF864 had excellent solubility and showed half-life exceeding 60h in cynomolgus monkeys. A second set of XTEN_AF sequences was assembled including a sequencing island as described in Example 9.

### Example 13: Construction of XTEN_AG864

Using the several consecutive rounds of dimerization, we assembled a collection of XTEN_AG864 sequences starting from segments of XTEN_AG36 listed in Example 4. These sequences were assembled as described in Example 5. Several isolates from XTEN_AG864 were evaluated and found to show good expression and excellent solubility under physiological conditions. A full-length clone of XTEN_AG864 had excellent solubility and showed half-life exceeding 60h in cynomolgus monkeys.

### Example 14: Methods of producing and evaluating GLP2-XTEN containing GLP-2 and AE_XTEN

A general schema for producing and evaluating GLP2-XTEN compositions is presented in FIG. 6, and forms the basis for the general description of this Example. The GLP-2 peptides and sequence variants may be prepared recombinantly. Exemplary recombinant methods used to prepare GLP-2 peptides include the following, among others, as will be apparent to one skilled in the art. Typically, a GLP-2 peptide or sequence variant as defined and/or described herein is prepared by constructing the nucleic acid encoding the desired peptide, cloning the nucleic acid into an expression vector in frame with nucleic acid encoding one or more XTEN, transforming a host cell (e.g., bacteria such as *Escherichia coli*, yeast such as *Saccharomyces cerevisiae* , or mammalian cell such as Chinese hamster ovary cell or baby hamster kidney cell), and expressing the nucleic acid to produce the desired GLP2-XTEN. Methods for producing and expressing recombinant polypeptides in vitro and in prokaryotic and eukaryotic host cells are known to those of ordinary skill in the art. See, for example, U.S. Pat. No. 4,868,122, and Sambrook et al., Molecular Cloning-A Laboratory Manual (Third Edition), Cold Spring Harbor Laboratory Press (2001).

Using the disclosed methods and those known to one of ordinary skill in the art, together with guidance provided in the illustrative examples, a skilled artesian can create and evaluate GLP2-XTEN fusion proteins comprising XTENs, GLP-2 and variants of GLP-2 disclosed herein or otherwise known in the art. The Example is, therefore, to be construed as merely illustrative, and not limitative of the methods in any way whatsoever; numerous variations will be apparent to the ordinarily skilled artisan. In this Example, a GLP2-XTEN comprising a GLP-2 linked to an XTEN of the AE family of motifs is created.

The general scheme for producing polynucleotides encoding XTEN is presented in FIGS. 4 and 5. FIG. 5 is a schematic flowchart of representative steps in the assembly of a XTEN polynucleotide construct in one of the embodiments of the invention. Individual oligonucleotides **501** are annealed into sequence motifs **502** such as a 12 amino acid motif ("12-mer"), which is ligated to additional sequence motifs from a library that can multimerize to create a pool that encompasses the desired length of the XTEN **504,** as well as ligated to a smaller concentration of an oligo containing BbsI, and KpnI restriction sites **503**. The motif libraries can be limited to specific sequence XTEN families; e.g., AD, AE, AF, AG, AM, or AQ sequences of Table 3. As illustrated in FIG. 5, the XTEN length in this case is 864 amino acid residues, but shorter or longer lengths can be achieved by this process. For example, multimerization can be performed by ligation, overlap extension, PCR assembly or similar cloning techniques known in the art. The resulting pool of ligation products is gel-purified and the band with the desired length of XTEN is cut, resulting in an isolated XTEN gene with a stopper sequence **505**. The XTEN gene can be cloned into a stuffer vector. In this case, the vector encodes an optional CBD sequence **506** and a GFP gene **508**. Digestion is than performed with BbsI/HindIII to remove **507** and **508** and place the stop codon. The resulting product is then cloned into a BsaI/HindIII digested vector containing a gene encoding the GLP-2, resulting in the gene **500** encoding a GLP2-XTEN fusion protein. As would be apparent to one of ordinary skill in the art, the methods can be applied to create constructs in alternative configurations and with varying XTEN lengths.

DNA sequences encoding GLP-2 can be conveniently obtained by standard procedures known in the art from a cDNA library prepared from an appropriate cellular source, from a genomic library, or may be created synthetically (e.g., automated nucleic acid synthesis), particularly where sequence variants (e.g., GLP-2-2G) are to be incorporated, using DNA sequences obtained from publicly available databases, patents, or literature references. In the present example, the GLP-2-2G sequence is utilized. A gene or polynucleotide encoding the GLP-2 portion of the protein or its complement can be then be cloned into a construct, such as those described herein, which can be a plasmid or other vector under control of appropriate transcription and translation sequences for high level protein expression in a biological system. A second gene or polynucleotide coding for the XTEN portion or its complement can be genetically fused to the nucleotides encoding the terminus of the GLP-2 gene by cloning it into the construct adjacent and in frame with the gene coding for the GLP-2, through a ligation or multimerization step. In this manner, a chimeric DNA molecule coding for (or complementary to) the GLP2-XTEN fusion protein is generated within the construct. Optionally, a gene encoding for a second XTEN is inserted and ligated in-frame internally to the nucleotides encoding the GLP-2-encoding region. Optionally, this chimeric DNA molecule is transferred or cloned into another construct that is a more appropriate expression vector; e.g., a vector appropriate for a prokaryotic host cell such as *E. coli,* a eukaryotic host cell such as yeast, or a mammalian host cell such as CHO, BHK and the like. At this point, a host cell capable of expressing the chimeric DNA molecule is transformed with the chimeric DNA molecule. The vectors containing the DNA segments of interest can be transferred into an appropriate host cell by well-known methods, depending on the type of cellular host, as described supra.

Host cells containing the GLP2-XTEN expression vector are cultured in conventional nutrient media modified as appropriate for activating the promoter. The culture conditions, such as temperature, pH and the like, are those previously used with the host cell selected for expression, and will be apparent to the ordinarily skilled artisan. After expression of the fusion protein, culture broth is harvested and separated from the cell mass and the resulting crude extract retained for purification of the fusion protein.

Gene expression is measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or in situ hybridization, using an appropriately labeled probe, based on the sequences provided herein. Alternatively, gene expression is measured by immunological of fluorescent methods, such as immunohistochemical staining of cells to quantitate directly the expression of gene product. Antibodies useful for immunohistochemical staining and/or assay of sample fluids may be either monoclonal or polyclonal, and may be prepared in any mammal. Conveniently, the antibodies may be prepared against the GLP-2 sequence polypeptide using a synthetic peptide based on the sequences provided herein or against exogenous sequence fused to GLP-2 and encoding a specific antibody epitope. Examples of selectable markers are well known to one of skill in the art and include reporters such as enhanced green fluorescent protein (EGFP), beta-galactosidase ($\beta$-gal) or chloramphenicol acetyltransferase (CAT).

The GLP2-XTEN polypeptide product is purified via methods known in the art. Procedures such as gel filtration, affinity purification, salt fractionation, ion exchange chromatography, size exclusion chromatography, hydroxyapatite adsorption chromatography, hydrophobic interaction chromatography or gel electrophoresis are all techniques that may be used in the purification. Specific methods of purification are described in Robert K. Scopes, Protein Purification: Principles and Practice, Charles R. Castor, ed., Springer-Verlag 1994, and Sambrook, *et al., supra.* Multi-step purification separations are also described in Baron, et al., Crit. Rev. Biotechnol. 10:179-90 (1990) and Below, et al., J. Chromatogr. A. 679:67-83 (1994).

As illustrated in FIG. 6, the isolated GLP2-XTEN fusion proteins would then be characterized for their chemical and activity properties. Isolated fusion protein is characterized, e.g., for sequence, purity, apparent molecular weight, solubility and stability using standard methods known in the art. The fusion protein meeting expected standards would then be evaluated for activity, which can be measured *in vitro* or *in vivo* by measuring one of the GLP-2-associated parameters described herein, using one or more assays disclosed herein, or using the assays of the Examples or the assays of Table 32.

In addition, the GLP2-XTEN fusion protein is administered to one or more animal species to determine standard pharmacokinetic parameters and pharmacodynamic properties, as described in Examples 18-21.

By the iterative process of producing, expressing, and recovering GLP2-XTEN constructs, followed by their characterization using methods disclosed herein or others known in the art, the GLP2-XTEN compositions comprising GLP-2 and an XTEN can be produced and evaluated by one of ordinary skill in the art to confirm the expected properties such as enhanced solubility, enhanced stability, improved pharmacokinetics and reduced immunogenicity, leading to an overall enhanced therapeutic activity compared to the corresponding unfused GLP-2. For those fusion proteins not possessing the desired properties, a different sequence can be constructed, expressed, isolated and evaluated by these methods in order to obtain a composition with such properties.

**Example 15: Construction of GLP2-XTEN genes and vectors**

Oligonucleotides were designed and constructed such that the entire GLP-2 gene could be assembled through the tiling together of these oligonucleotides via designed complementary over hang regions under conditions of a 48°C annealing temperature. The complementary regions were held constant, but the other regions of the oligonucleotides were varied such that a codon library was created with ~50% of the codons in the gene varied instead of the single native gene sequence. A PCR was performed to create a combined gene library which, as is typical, contained a variety of combinations of the oligonucleotides and presented as a smear on an agarose gel. A polishing PCR was performed to amplify those assemblies that had the correct termini using a set of amplification primers complimentary to the 5' and 3' ends of the gene. The product of this PCR was then gel purified, taking only bands at the ~100 bp length of the expected GLP-2 final gene product. This gel-purified product was digested with BsaI and NdeI and ligated into a similarly digested construct containing DNA encoding a CBD leader sequence and the AE864 XTEN, to produce a GLP2-XTEN_AE864 gene, and transformed in BL21 gold competent cells. Colonies from this transformation were picked into 500 $\mu$l cultures of SB in 96 deep well plates and grown to saturation overnight. These cultures were stored at 4°C after 20 $\mu$l of these cultures was used to inoculate 500 $\mu$l of auto-induction media and these cultures were grown at 26°C for >24 hours. Following the growth the GFP fluorescence of 100 $\mu$l of these auto-induction media cultures was measured using a fluorescence plate reader. The GFP fluorescence is proportional to the number of molecules of GLP2-XTEN_AE464 made and is therefore a read-out of total expression. The highest expressing clones were identified, and a new 1 ml overnight was started in SB from the original saturated overnight culture of that clone. Mini-preps were performed with these new cultures and the derived plasmids were sequenced to determine the exact nucleotide composition. An *E. coli* isolate was designated strain AC453 and was identified as a strain that produced the desired GLP-2_2G-XTEN_AE864 fusion protein. The DNA and amino acid sequences of the pre-cleavage expressed product (with a CBD leader and TEV cleavage sequence) and the amino acid sequence of the final product GLP-2-2G-XTEN AE864 (after TEV cleavage) are provided in Table 13.

**Table 13: GLP2-XTEN DNA and amino acid sequences**

| Clone Name | DNA Sequence | Amino Acid Sequence |
|---|---|---|
| **CBD-TEV-GLP-2-2G-AE864 (pCW812/ AC453)** | ATGGCAAATACACCGGTATCAGGCAATTTGAAGGTTGAAT TCTACAACAGCAATCCTTCAGATACTACTAACTCAATCAA TCCTCAGTTCAAGGTTACTAATACCGGAAGCAGTGCAATT GATTTGTCCAAACTCACATTGAGATATTATTATACAGTAGA CGGACAGAAAGATCAGACCTTCTGGGCTGACCATGCTGCA ATAATCGGCAGTAACGGCAGCTACAACGGAATTACTTCAA ATGTAAAAGGAACATTTGTAAAAATGAGTTCCTCAACAAA TAACGCAGACACCTACCTTGAAATCAGCTTTACAGGCGGA ACTCTTGAACCGGGTGCACATGTTCAGATACAAGGTAGAT TTGCAAAGAATGACTGGAGTAACTATACACAGTCAAATGA CTACTCATTCAAGTCTGCTTCACAGTTTGTTGAATGGGATC AGGTAACAGCATACTTGAACGGTGTTCTTGTATGGGGTAA AGAACCCGGTGGCAGTGTAGTAGGTTCAGGTTCAGGATCC GAAAATCTGTATTTTCAACATGGTGACGGCTCTTTTAGCGA TGAAATGAATACTATACTGGACAACCTTGCGGCACGCGAC | MANTPVSGNLKVEF YNSNPSDTTNSINPQ FKVTNTGSSAIDLSK LTLRYYYTVDGQKD QTFWADHAAIIGSN GSYNGITSNVKGTF VKMSSSTNNADTYL EISFTGGTLEPGAHV QIQGRFAKNDWSNY TQSNDYSFKSASQF VEWDQVTAYLNGV LVWGKEPGGSVVGS GSGSENLYFQHGDG |

(continued)

| Clone Name | DNA Sequence | Amino Acid Sequence |
|---|---|---|
| | TTCATTAACTGGCTGATCCAGACAAAAATCACCGATGGAG<br>GTAGCCCGGCTGGCTCTCCTACCTCTACTGAGGAAGGTAC<br>TTCTGAAAGCGCTACTCCTGAGTCTGGTCCAGGTACCTCTA<br>CTGAACCGTCCGAAGGTAGCGCTCCAGGTAGCCCAGCAGG<br>CTCTCCGACTTCCACTGAGGAAGGTACTTCTACTGAACCTT<br>CCGAAGGCAGCGCACCAGGTACCTCTACTGAACCTTCTGA<br>GGGCAGCGCTCCAGGTACTTCTGAAAGCGCTACCCCGGAA<br>TCTGGCCCAGGTAGCGAACCGGCTACTTCTGGTTCTGAAA<br>CCCCAGGTAGCGAACCGGCTACCTCCGGTTCTGAAACTCC<br>AGGTAGCCCGGCAGGCTCTCCGACCTCTACTGAGGAAGGT<br>ACTTCTGAAAGCGCAACCCCGGAGTCCGGCCCAGGTACCT<br>CTACCGAACCGTCTGAGGGCAGCGCACCAGGTACTTCTAC<br>CGAACCGTCCGAGGGTAGCGCACCAGGTAGCCCAGCAGG<br>TTCTCCTACCTCCACCGAGGAAGGTACTTCTACCGAACCGT<br>CCGAGGGTAGCGCACCAGGTACCTCTACTGAACCTTCTGA<br>GGGCAGCGCTCCAGGTACTTCTGAAAGCGCTACCCCGGAG<br>TCCGGTCCAGGTACTTCTACTGAACCGTCCGAAGGTAGCG<br>CACCAGGTACTTCTGAAAGCGCAACCCCTGAATCCGGTCC<br>AGGTAGCGAACCGGCTACTTCTGGCTCTGAGACTCCAGGT<br>ACTTCTACCGAACCGTCCGAAGGTAGCGCACCAGGTACTT<br>CTACTGAACCGTCTGAAGGTAGCGCACCAGGTACTTCTGA<br>AAGCGCAACCCCGGAATCCGGCCCAGGTACCTCTGAAAGC<br>GCAACCCCGGAGTCCGGCCCAGGTAGCCCTGCTGGCTCTC<br>CAACCTCCACCGAAGAAGGTACCTCTGAAAGCGCAACCCC<br>TGAATCCGGCCCAGGTAGCGAACCGGCAACCTCCGGTTCT<br>GAAACCCCAGGTACCTCTGAAAGCGCTACTCCGGAGTCTG<br>GCCCAGGTACCTCTACTGAACCGTCTGAGGGTAGCGCTCC<br>AGGTACTTCTACTGAACCGTCCGAAGGTAGCGCACCAGGT<br>ACTTCTACCGAACCGTCCGAAGGCAGCGCTCCAGGTACCT<br>CTACTGAACCTTCCGAGGGCAGCGCTCCAGGTACCTCTAC<br>CGAACCTTCTGAAGGTAGCGCACCAGGTACTTCTACCGAA<br>CCGTCCGAGGGTAGCGCACCAGGTAGCCCAGCAGGTTCTC<br>CTACCTCCACCGAGGAAGGTACTTCTACCGAACCGTCCGA<br>GGGTAGCGCACCAGGTACCTCTGAAAGCGCAACTCCTGAG<br>TCTGGCCCAGGTAGCGAACCTGCTACCTCCGGCTCTGAGA<br>CTCCAGGTACCTCTGAAAGCGCAACCCCGGAATCTGGTCC<br>AGGTAGCGAACCTGCAACCTCTGGCTCTGAAACCCCAGGT<br>ACCTCTGAAAGCGCTACTCCTGAATCTGGCCCAGGTACTT<br>CTACTGAACCGTCCGAGGGCAGCGCACCAGGTACTTCTGA<br>AAGCGCTACTCCTGAGTCCGGCCCAGGTAGCCCGGCTGGC<br>TCTCCGACTTCCACCGAGGAAGGTAGCCCGGCTGGCTCTC<br>CAACTTCTACTGAAGAAGGTAGCCCGGCAGGCTCTCCGAC<br>CTCTACTGAGGAAGGTACTTCTGAAAGCGCAACCCCGGAG<br>TCCGGCCCAGGTACCTCTACCGAACCGTCTGAGGGCAGCG<br>CACCAGGTACCTCTGAAAGCGCAACTCCTGAGTCTGGCCC<br>AGGTAGCGAACCTGCTACCTCCGGCTCTGAGACTCCAGGT<br>ACCTCTGAAAGCGCAACCCCGGAATCTGGTCCAGGTAGCG<br>AACCTGCAACCTCTGGCTCTGAAACCCCAGGTACCTCTGA<br>AAGCGCTACTCCTGAATCTGGCCCAGGTACTTCTACTGAA<br>CCGTCCGAGGGCAGCGCACCAGGTAGCCCTGCTGGCTCTC<br>CAACCTCCACCGAAGAAGGTACCTCTGAAAGCGCAACCCC<br>TGAATCCGGCCCAGGTAGCGAACCGGCAACCTCCGGTTCT<br>GAAACCCCAGGTACTTCTGAAAGCGCTACTCCTGAGTCCG<br>GCCCAGGTAGCCCGGCTGGCTCTCCGACTTCCACCGAGGA<br>AGGTAGCCCGGCTGGCTCTCCAACTTCTACTGAAGAAGGT<br>ACTTCTACCGAACCTTCCGAGGGCAGCGCACCAGGTACTT<br>CTGAAAGCGCTACCCCTGAGTCCGGCCCAGGTACTTCTGA<br>AAGCGCTACTCCTGAATCCGGTCCAGGTACTTCTGAAAGC<br>GCTACCCCGGAATCTGGCCCAGGTAGCGAACCGGCTACTT | SFSDEMNTILDNLA<br>ARDFINWLIQTKITD<br>GGSPAGSPTSTEEGT<br>SESATPESGPGTSTE<br>PSEGSAPGSPAGSPT<br>STEEGTSTEPSEGSA<br>PGTSTEPSEGSAPGT<br>SESATPESGPGSEPA<br>TSGSETPGSEPATSG<br>SETPGSPAGSPTSTE<br>EGTSESATPESGPGT<br>STEPSEGSAPGTSTE<br>PSEGSAPGSPAGSPT<br>STEEGTSTEPSEGSA<br>PGTSTEPSEGSAPGT<br>SESATPESGPGTSTE<br>PSEGSAPGTSESATP<br>ESGPGSEPATSGSET<br>PGTSTEPSEGSAPGT<br>STEPSEGSAPGTSES<br>ATPESGPGTSESATP<br>ESGPGSPAGSPTSTE<br>EGTSESATPESGPGS<br>EPATSGSETPGTSES<br>ATPESGPGTSTEPSE<br>GSAPGTSTEPSEGSA<br>PGTSTEPSEGSAPGT<br>STEPSEGSAPGTSTE<br>PSEGSAPGTSTEPSE<br>GSAPGSPAGSPTSTE<br>EGTSTEPSEGSAPGT<br>SESATPESGPGSEPA<br>TSGSETPGTSESATP<br>ESGPGSEPATSGSET<br>PGTSESATPESGPGT<br>STEPSEGSAPGTSES<br>ATPESGPGSPAGSPT<br>STEEGSPAGSPTSTE<br>EGSPAGSPTSTEEGT<br>SESATPESGPGTSTE<br>PSEGSAPGTSESATP<br>ESGPGSEPATSGSET<br>PGTSESATPESGPGS<br>EPATSGSETPGTSES<br>ATPESGPGTSTEPSE<br>GSAPGSPAGSPTSTE<br>EGTSESATPESGPGS<br>EPATSGSETPGTSES<br>ATPESGPGSPAGSPT<br>STEEGSPAGSPTSTE<br>EGTSTEPSEGSAPGT<br>SESATPESGPGTSES<br>ATPESGPGTSESATP<br>ESGPGSEPATSGSET |

(continued)

| Clone Name | DNA Sequence | Amino Acid Sequence |
|---|---|---|
| | CTGGTTCTGAAACCCCAGGTAGCGAACCGGCTACCTCCGG TTCTGAAACTCCAGGTAGCCCAGCAGGCTCTCCGACTTCC ACTGAGGAAGGTACTTCTACTGAACCTTCCGAAGGCAGCG CACCAGGTACCTCTACTGAACCTTCTGAGGGCAGCGCTCC AGGTAGCGAACCTGCAACCTCTGGCTCTGAAACCCCAGGT ACCTCTGAAAGCGCTACTCCTGAATCTGGCCCAGGTACTT CTACTGAACCGTCCGAGGGCAGCGCACCAGGT | PGSEPATSGSETPGS PAGSPTSTEEGTSTE PSEGSAPGTSTEPSE GSAPGSEPATSGSET PGTSESATPESGPGT STEPSEGSAPG |

(continued)

| Clone Name | DNA Sequence | Amino Acid Sequence |
|---|---|---|
| GLP-2-2G-AE864 | CATGGTGACGGCTCTTTTAGCGATGAAATGAATACTATAC TGGACAACCTTGCGGCACGCGACTTCATTAACTGGCTGAT CCAGACAAAAATCACCGATGGAGGTAGCCCGGCTGGCTCT CCTACCTCTACTGAGGAAGGTACTTCTGAAAGCGCTACTC CTGAGTCTGGTCCAGGTACCTCTACTGAACCGTCCGAAGG TAGCGCTCCAGGTAGCCCAGCAGGCTCTCCGACTTCCACT GAGGAAGGTACTTCTACTGAACCTTCCGAAGGCAGCGCAC CAGGTACCTCTACTGAACCTTCTGAGGGCAGCGCTCCAGG TACTTCTGAAAGCGCTACCCCGGAATCTGGCCCAGGTAGC GAACCGGCTACTTCTGGTTCTGAAACCCCAGGTAGCGAAC CGGCTACCTCCGGTTCTGAAACTCCAGGTAGCCCGGCAGG CTCTCCGACCTCTACTGAGGAAGGTACTTCTGAAAGCGCA ACCCCGGAGTCCGGCCCAGGTACCTCTACCGAACCGTCTG AGGGCAGCGCACCAGGTACTTCTACCGAACCGTCCGAGGG TAGCGCACCAGGTAGCCCAGCAGGTTCTCCTACCTCCACC GAGGAAGGTACTTCTACCGAACCGTCCGAGGGTAGCGCAC CAGGTACCTCTACTGAACCTTCTGAGGGCAGCGCTCCAGG TACTTCTGAAAGCGCTACCCCGGAGTCCGGTCCAGGTACT TCTACTGAACCGTCCGAAGGTAGCGCACCAGGTACTTCTG AAAGCGCAACCCCTGAATCCGGTCCAGGTAGCGAACCGGC TACTTCTGGCTCTGAGACTCCAGGTACTTCTACCGAACCGT CCGAAGGTAGCGCACCAGGTACTTCTACTGAACCGTCTGA AGGTAGCGCACCAGGTACTTCTGAAAGCGCAACCCCGGAA TCCGGCCCAGGTACCTCTGAAAGCGCAACCCCGGAGTCCG GCCCAGGTAGCCCTGCTGGCTCTCCAACCTCCACCGAAGA AGGTACCTCTGAAAGCGCAACCCCTGAATCCGGCCCAGGT AGCGAACCGGCAACCTCCGGTTCTGAAACCCCAGGTACCT CTGAAAGCGCTACTCCGGAGTCTGGCCCAGGTACCTCTAC TGAACCGTCTGAGGGTAGCGCTCCAGGTACTTCTACTGAA CCGTCCGAAGGTAGCGCACCAGGTACTTCTACCGAACCGT CCGAAGGCAGCGCTCCAGGTACCTCTACTGAACCTTCCGA GGGCAGCGCTCCAGGTACCTCTACCGAACCTTCTGAAGGT AGCGCACCAGGTACTTCTACCGAACCGTCCGAGGGTAGCG CACCAGGTAGCCCAGCAGGTTCTCCTACCTCCACCGAGGA AGGTACTTCTACCGAACCGTCCGAGGGTAGCGCACCAGGT ACCTCTGAAAGCGCAACTCCTGAGTCTGGCCCAGGTAGCG AACCTGCTACCTCCGGCTCTGAGACTCCAGGTACCTCTGA AAGCGCAACCCCGGAATCTGGTCCAGGTAGCGAACCTGCA ACCTCTGGCTCTGAAACCCCAGGTACCTCTGAAAGCGCTA CTCCTGAATCTGGCCCAGGTACTTCTACTGAACCGTCCGA GGGCAGCGCACCAGGTACTTCTGAAAGCGCTACTCCTGAG TCCGGCCCAGGTAGCCCGGCTGGCTCTCCGACTTCCACCG AGGAAGGTAGCCCGGCTGGCTCTCCAACTTCTACTGAAGA AGGTAGCCCGGCAGGCTCTCCGACCTCTACTGAGGAAGGT ACTTCTGAAAGCGCAACCCCGGAGTCCGGCCCAGGTACCT CTACCGAACCGTCTGAGGGCAGCGCACCAGGTACCTCTGA AAGCGCAACTCCTGAGTCTGGCCCAGGTAGCGAACCTGCT ACCTCCGGCTCTGAGACTCCAGGTACCTCTGAAAGCGCAA CCCCGGAATCTGGTCCAGGTAGCGAACCTGCAACCTCTGG CTCTGAAACCCCAGGTACCTCTGAAAGCGCTACTCCTGAA TCTGGCCCAGGTACTTCTACTGAACCGTCCGAGGGCAGCG CACCAGGTAGCCCTGCTGGCTCTCCAACCTCCACCGAAGA | HGDGSFSDEMNTIL DNLAARDFINWLIQ TKITDGGSPAGSPTS TEEGTSESATPESGP GTSTEPSEGSAPGSP AGSPTSTEEGTSTEP SEGSAPGTSTEPSEG SAPGTSESATPESGP GSEPATSGSETPGSE PATSGSETPGSPAGS PTSTEEGTSESATPE SGPGTSTEPSEGSAP GTSTEPSEGSAPGSP AGSPTSTEEGTSTEP SEGSAPGTSTEPSEG SAPGTSESATPESGP GTSTEPSEGSAPGTS ESATPESGPGSEPAT SGSETPGTSTEPSEG SAPGTSTEPSEGSAP GTSESATPESGPGTS ESATPESGPGSPAGS PTSTEEGTSESATPE SGPGSEPATSGSETP GTSESATPESGPGTS TEPSEGSAPGTSTEP SEGSAPGTSTEPSEG SAPGTSTEPSEGSAP GTSTEPSEGSAPGTS TEPSEGSAPGSPAGS PTSTEEGTSTEPSEG SAPGTSESATPESGP GSEPATSGSETPGTS ESATPESGPGSEPAT SGSETPGTSESATPE SGPGTSTEPSEGSAP GTSESATPESGPGSP AGSPTSTEEGSPAGS PTSTEEGSPAGSPTS TEEGTSESATPESGP GTSTEPSEGSAPGTS ESATPESGPGSEPAT SGSETPGTSESATPE SGPGSEPATSGSETP GTSESATPESGPGTS TEPSEGSAPGSPAGS PTSTEEGTSESATPE |

(continued)

| Clone Name | DNA Sequence | Amino Acid Sequence |
|---|---|---|
| | AGGTACCTCTGAAAGCGCAACCCCTGAATCCGGCCCAGGT AGCGAACCGGCAACCTCCGGTTCTGAAACCCCAGGTACTT CTGAAAGCGCTACTCCTGAGTCCGGCCCAGGTAGCCCGGC TGGCTCTCCGACTTCCACCGAGGAAGGTAGCCCGGCTGGC TCTCCAACTTCTACTGAAGAAGGTACTTCTACCGAACCTTC CGAGGGCAGCGCACCAGGTACTTCTGAAAGCGCTACCCCT GAGTCCGGCCCAGGTACTTCTGAAAGCGCTACTCCTGAAT CCGGTCCAGGTACTTCTGAAAGCGCTACCCCGGAATCTGG CCCAGGTAGCGAACCGGCTACTTCTGGTTCTGAAACCCCA GGTAGCGAACCGGCTACCTCCGGTTCTGAAACTCCAGGTA GCCCAGCAGGCTCTCCGACTTCCACTGAGGAAGGTACTTC TACTGAACCTTCCGAAGGCAGCGCACCAGGTACCTCTACT GAACCTTCTGAGGGCAGCGCTCCAGGTAGCGAACCTGCAA CCTCTGGCTCTGAAACCCCAGGTACCTCTGAAAGCGCTAC TCCTGAATCTGGCCCAGGTACTTCTACTGAACCGTCCGAG GGCAGCGCACCAGGT | SGPGSEPATSGSETP GTSESATPESGPGSP AGSPTSTEEGSPAGS PTSTEEGTSTEPSEG SAPGTSESATPESGP GTSESATPESGPGTS ESATPESGPGSEPAT SGSETPGSEPATSGS ETPGSPAGSPTSTEE GTSTEPSEGSAPGTS TEPSEGSAPGSEPAT SGSETPGTSESATPE SGPGTSTEPSEGSAP G |

## Example 16: Expression and purification of fusion proteins comprising GLP-2-2G fused to XTEN_AE864.

The host strain for expression, AmE025, was derived from *E.coli* W3110, a strain with a K-12 background, having a deletion of the *fhuA* gene and with the lambda DE3 prophage integrated onto the chromosome. The host cell contained the plasmid pCW1010 (AC616), encoding an amino acid sequence that is identical to that encoded by pCW812 (AC453). The final construct comprised the gene encoding the cellulosome anchoring protein cohesion region cellulose binding domain (CBD) from *Clostridium thermocellum* (accession #ABN54273), a tobacco etch virus (TEV) protease recognition site (ENLYFQ), the GLP2-2G sequence, and an AE864 amino acid XTEN sequence under control of a T7 promoter. The protein was expressed in a 5L glass jacketed fermentation vessel with a B. Braun Biostat B controller. Briefly, a starter culture of host strain AmE025 was used to inoculate 2L of fermentation batch media. After 6 hours of culture at 37°C, a 50% glucose feed was initiated. After 20 hours of culture, the temperature was reduced to 26°C and 1M IPTG was added to induce expression. After a total fermentation run time of 45 hours, the culture was harvested by centrifugation yielding cell pellets ~1 kg in wet weight. The pellets were stored frozen at -80°C until purification was initiated.

## Lysis, heat flocculation and clarification

The resulting cell paste was resuspended at ambient temperature in 20 mM Tris-HCl pH 7.5, 50 mM NaCl, at a ratio of ~4 ml per 1 g of cell paste. The cells were lysed by 2 passes through an APV 2000 homogenizer at an operating pressure of 800-900 bar. After lysis, the homogenate was heated to ~85°C in a heat exchanger and held for 20 minutes to coagulate host cell protein, then rapidly cooled to ~10°C. The cooled homogenate was clarified by centrifugation at 4,000 rpm for 60 min using a Sorvall H6000A rotor in a Sorvall RC-3C centrifuge. The supernatant was decanted, passed through a 60SP03A Zeta Plus EXT depth filter (3M), followed by passage through a 0.2 μm LifeASSURE PDA sterile capsule and stored at 4°C overnight.

## Initial Anion Exchange capture with Toyopearl SuperQ-650M resin

GLP2-2G-XTEN was isolated out of the clarified lysate using 3 columns steps at ambient temperature. GLP2-2G-XTEN was captured using Toyopearl SuperQ-650M (Tosoh) anion exchange resin, which selects for the negatively charged XTEN polypeptide tail and removes the bulk of host cell protein. An appropriately scaled SuperQ-650M column was equilibrated with 5 column volumes of 20 mM Tris-HCl pH 7.5, 50 mM NaCl and the lysate was loaded onto the column at a linear flow rate of 120 cm/hr. The column was then washed with 3 column volumes of 20 mM Tris-HCl pH 7.5, 50 mM NaCl and 3 column volumes of 20 mM Tris-HCl pH 7.5, 150 mM NaCl, until the UV absorbance returned to baseline. GLP2-2G-XTEN protein was eluted with a 7 column volume linear gradient from 150 mM NaCl to 300 mM NaCl in 20 mM NaCl Tris-HCl, pH 7.5. Fractions were collected throughout and analyzed by SDS-PAGE for pooling and storage at 2-8°C. Product purity was determined to be ~80% after the Super Q capture step.

Intermediate Anion Exchange capture with GE MacroCap Q resin

The resulting SuperQ pool was diluted ~4-fold with 20 mM Tris-HCl pH 7.5 to reduce the conductivity to < 10 mS/cm. An appropriately scaled MacroCap Q anion exchange column (GE Life Sciences) selects for the full-length intact XTEN polypeptide tail and removes the bulk of endotoxin and any residual host cell protein and DNA. The column was equilibrated with 5 column volumes of 20 mM Tris-HCl pH 7.5, 50 mM NaCl. The diluted SuperQ pool was loaded at a linear flow rate of 120 cm/hr. The column was then washed with 3 column volumes of 20 mM Tris-HCl pH 7.5, 50 mM NaCl, and then 3 column volumes of 20mM Tris-HCl pH 7.5, 150 mM NaCl, until the UV absorbance returned to baseline. GLP2-2G-XTEN protein was eluted with a 12 column volume linear gradient from 150 mM NaCl to 300 mM NaCl in 20 mM Tris-HCl pH 7.5. Fractions were collected throughout and analyzed by SDS-PAGE for pooling and storage at 2-8°C. Product purity was determined to be >95% after the MacroCap Q intermediate step.

Hydrophobic Interaction Chromatography (HIC) using Toyopearl Phenyl-650M resin

An appropriate amount of solid NaCl salt was dissolved in the MacroCap Q pool to adjust load to 4 M NaCl, and then was sterile filtered through a 0.2 μm filter. An appropriately scaled Toyopearl Phenyl-650M (Tosoh) column selects for the hydrophobic residues of the GLP2 payload and removes residual XTEN fragments and endotoxin. The column was equilibrated with 5 column volumes of 20 mM Tris-HCl pH 7.5, 4 M NaCl. The MacroCap Q pool was loaded at a linear flow rate of 60 cm/hr. The column was then washed with 3 column volumes of 20 mM Tris-HCl pH 7.5, 4 M NaCl. GLP2-2G-XTEN protein was eluted with a step-down gradient to 1.2 M NaCl in 20 mM Tris-HCl pH 7.5. The elution peak was fractionated and analyzed by SDS-PAGE to confirm successful capture and elution of GLP2-2G-XTEN. Product purity was determined to be >95% after the final polishing step. The resulting pool was concentrated to ~11 mg/ml and buffer exchanged into 20 mM Tris-HCl pH 7.5, 135 mM NaCl formulation buffer using a 30 KDa MWCO Pellicon XL 50 Ultrafiltration Cassette (Millipore). The purified lot of GLP2-2G-XTEN was designated AP690 and stored at -80°C until further use.

SDS-PAGE Analysis

SDS-PAGE analysis was conducted with 2 μg, 5 μg and 10 μg of AP690 loaded onto a NuPAGE 4-12% Bis Tris Gel (Invitrogen) and then run for 35 minutes at a constant 200V. The results (FIG. 11A) showed that the AP690 protein was free from host cell impurities and that it migrated near the 160 kDa marker, the expected result for a payload-XTEN fusion protein of this molecular weight and composition.

Endotoxin Content

Endotoxin levels of lot AP690 was assessed using an EndoSafe PTS test cartridge (Carles River) and determined to be 3.5 EU/mg of protein, making the AP690 lot appropriate for injection into test animals for pharmacokinetic or pharmacodynamic studies.

Analytical size exclusion HPLC

Gel filtration analysis was performed using a Phenomenex BioSep-SEC-s4000 (7.8mm x 600mm) column. 20 μg or AP690 GLP2-2G-XTEN fusion protein were analyzed at a flowrate of 0.5 ml/min with 50 mM Phosphate pH 6.5, 300 mM NaCl mobile phase. Elution was monitored using OD215nm. Column calibration was performed using a size exclusion check standard from Phenomenex, with the following markers: thyroglobulin (670 kDa), IgG (156 kDa), BSA (66 kDa) and ovalbumin (17 kDa). The result (FIG. 11B) indicated an apparent molecular weight of 1002 kDa for the fusion protein of 83.1 kDa actual weight, for an apparent molecular weight factor of 12.5.

Intact mass determination by ESI-MS

200 μg of AP690 GLP2-2G-XTEN protein was desalted by solid phase extraction using an Extract-Clean C18 column (Discovery Sciences). The desalted protein solution in 0.1% formic acid, 50% acetonitrile was infused at 4 μl/min into a QSTAR XL mass spectrometer (AB Sciex). Multicharge TOF spectrum was acquired in 800-1400 amu range. A zero-charge spectrum was obtained by Bayesian reconstruction in 10-100 kDa range (FIG. 12). The experimental mass of the full length intact GLP2-2G-XTEN was determined to be 83,142 Da, with an additional minor peak of 83,003 Da detected, representing the des-His GLP2-2G-XTEN at <5% of total protein.

**Example 17: Characterization of GLP2-XTEN *in vitro* receptor binding by calcium flux potency assay**

A receptor binding assay was performed using a GPCRProfiler assay (Millipore) to assess GLP2-2G-XTEN preparations (including AP690). The assay employed a transfected GLP2R cell line (Millipore, Cat# HTS164C) consisting of a Chem-11 human cell stably transfected with the GLP2 G-protein coupled receptor and a G alpha protein that stimulates calcium flux upon agonism of the GLP2 receptor. Assays were performed by addition of serial dilutions of GLP2-2G-XTEN, synthetic GLP2-2G peptide (without XTEN) and synthetic native GLP2 peptide, and the calcium flux was monitored in real-time by a FLIPR TETRA instrument (Molecular Devices) using the no wash calcium assay kit (Molecular devices). The results, presented in FIG. 13, were used to derive EC50 values of 370 nM for GLP2-2G-XTEN and 7 nM for GLP2-2G peptide. The results indicate that the GLP2-2G-XTEN was able to bind and activate the GLP-2 receptor, with about 2% of the potency compared to GLP2-2G.

**Example 18: Pharmacokinetic evaluation of GLP2-XTEN in mice**

The fusion protein GLP2-2G-XTEN_AE864 was evaluated for its pharmacokinetic properties in C57B1/6 mice following subcutaneous (SC) administration. Female C57B1/6 mice were injected SC with 2 mg/kg (25 nmol/kg) of the GLP2-2G-XTEN (lot AP498A) at 0.25mg/mL (8 mL/kg). Three mice were sacrificed at each of the following time points: Predose, 0.08, 4, 8, 24, 48, 72, 96 and 120 hours post-dose. Blood samples were collected from the mice and placed into prechilled heparinized tubes at each interval and were separated by centrifugation to recover the plasma. The samples were analyzed for fusion protein concentration, performed by both anti-XTEN/anti- XTEN sandwich ELISA (AS 1405) and anti-GLP2/anti-XTEN sandwich ELISA (AS 1717), and the results were analyzed using WinNonLin to obtain the PK parameters. Terminal half-life was fit from 24 to 120 hours. The results are presented in Table 14 and FIG. 14, with both assays showing essentially equivalent results, with a terminal half-life of 31.6-33.9 h determined.

**Table 14: GLP2-2G-XTEN-864 Pharmacokinetics**

| Group | $C_{max}$ (ng/ml) | AUClast [hr*ng/ml] | T½ (hr) | Vd (ml) |
|---|---|---|---|---|
| **XTEN-XTEN ELISA** | 13,600 | 773,000 | 31.6 | 2.7 |
| **GLP2-XTEN ELISA** | 11,200 | 720,000 | 33.9 | 3.4 |

**Example 19: Pharmacokinetic evaluation of GLP2-XTEN in rats**

The fusion protein GLP2-2G-XTEN _AE864 was evaluated for its pharmacokinetic properties in Wistar rats following SC administration of two different dosage levels. Prior to the experiment, catheters were surgically implanted into the jugular vein of female Wistar rats. The catheterized animals were randomized into two groups containing three rats each. The fusion protein GLP2-2G-XTEN (lot AP510) was administered to each rat via SC injection as follows: 1) Low Dose 2 mg/kg (25 nmol/kg); or 2) High Dose 16 mg/kg (200 nmol/kg). Blood samples (~0.2mL) were collected through the jugular vein catheter from each rat into prechilled heparinized tubes at pre-dose, 0.08, 4, 8, 24, 48, 72, 96, 120 and 168 hours after test compound administration (10 time points). Blood was processed into plasma by centrifugation, split into two aliquots for analysis by ELISA. The samples were analyzed for fusion protein concentration, performed by both anti-XTEN/anti-XTEN sandwich ELISA (AS 1602) and anti-GLP2/anti-XTEN sandwich ELISA (AS1705) and the results were analyzed using WinNonLin to obtain the PK parameters. Terminal half life was fit from 48 to 168 hours. The results are presented in Table 15 and FIG. 15, with both assays showing essentially equivalent results and with a terminal half-life of 37.5-49.7 h determined, greatly exceeding the reported terminal half-life for GLP-2 and for GLP2-2G. In addition, the pharmacokinetic profile of GLP2-2G-XTEN after single subcutaneous administration to rats at 25 nmol/kg and 200 nmol/kg was dose proportional with the $C_{max}$ and AUC increasing in an approximately linear manner.

**Table 15: GLP2-2G-XTEN-864 Pharmacokinetics**

| | T½ (hr) | $C_{max}$ (ng/ml) | AUCInf (hr*ng/mL) | Vz (mL) | CI (mL/hr) |
|---|---|---|---|---|---|
| **ANTI-XTEN ELISA** | | | | | |
| High Dose (16mg/kg) | 42.0 | 37900 | 3000000 | 65.0 | 1.07 |
| Low Dose (2 mg/kg) | 42.6 | 6270 | 530000 | 43.4 | 0.71 |

(continued)

| ANTI-GLP2-XTEN ELISA | | | | | |
|---|---|---|---|---|---|
| High Dose (16mg/kg) | 49.7 | 40300 | 3660000 | 70.2 | 0.972 |
| Low Dose (2 mg/kg) | 37.5 | 6900 | 530000 | 43.4 | 0.797 |

**Example 20: Pharmacokinetic evaluation of GLP2-XTEN in cynomolgus monkeys**

The fusion protein GLP2-2G-XTEN_AE864 was evaluated for its pharmacokinetic properties in male cynomolgus monkeys following either subcutaneous or intravenous administration of the fusion protein at a single dosage level. Three male cynomolgus monkeys were injected IV and 3 male cynomolgus monkeys were injected SC with 2 mg/kg (25 nmol/kg) GLP2-2G-XTEN at time 0. Blood samples were collected from each monkey into prechilled heparinized tubes at pre-dose and at approximately 0.083 h (5 min), 1, 2, 4, 8, 24, 48, 72, 96, 120, 168, 216, 264, and 336 hours after administration of the fusion protein for the first phase of the study. Animals were allowed to "wash-out" for a 6 week period (4 weeks post-last collection time point of Phase 1), the groups were crossed over (SC to IV and IV to SC), and dosed again with the same dose of GLP2-2G-XTEN fusion protein. Blood samples were collected at pre-dose and at approximately 0.083 h (5 min), 1, 2, 4, 8, 24, 48, 72, 96, 120, 168, 216, 264, 336, 384, 432, and 504 hours post-dose in the second phase of the study. All blood samples were processed into plasma by centrifugation and split into two aliquots for analysis by ELISA. The samples were analyzed for fusion protein concentration, performed by anti-GLP2/anti-XTEN ELISA (AS 1705) and the results were analyzed using WinNonLin to obtain the PK parameters. The results are presented in Table 16 and FIG. 16, with a terminal half-life for the GLP2-2G-XTEN_AE864 fusion protein of 110 h for IV and 120 h for SC administration determined. The bioavailability was 96% demonstrating that GLP2-2G-XTEN is rapidly and near completely absorbed after subcutaneous administration.

**Table 16: GLP2-2G-XTEN-864 Pharmacokinetics**

| GROUP | T½ (hr) | $C_{max}$ (ng/ml) | AUCInf (hr*ng/mL) | Vd (mL/kg) | CI (mL/hr) |
|---|---|---|---|---|---|
| IV | 110.0 | 62000 | 3,700,000 | 90 | 1.9 |
| SC | 120.0 | 20000 | 3,400,000 | 110 | 2.0 |

The cumulative results of the PK analyses were used to perform allometric scaling of GLP2-2G _AE864 terminal half-life, clearance and volume of distribution using data from three species (mouse, rat and monkey). Pharmacokinetic values for a 70 kg human were predicted by extrapolating the log linear relationship between body weight and each pharmacokinetic parameter, as shown in FIG. 17. The data for terminal half life, volume of distribution and clearance are presented in Table 17. The predicted terminal half-life in humans of 240 h, greatly exceeds the reported 3.2 h terminal half-life of teduglutide in humans (Marier, J-F, et al. Pharmacokinetics, Safety, and Tolerability of Teduglutide, a Glucagon-Like Peptide-2 (GLP-2) Analog, Following Multiple Ascending Subcutaneous Administrations in Healthy Subjects. J Clin Pharmacol (2008) 48:1289-1299). The terminal half-life in humans can also be estimated using the predicted values for clearance (CI) and volume of distribution (Vd) as $0.693 \times$ Vd/CI. Applying this formula yields a predicted terminal half-life of 230 h in humans, which agrees well with the extrapolation from the animal T½ data, and which greatly exceeds the reported terminal half-life for native GLP-2 and for GLP2-2G.

**Table 17: Allometric scaling of GLP2-2G-XTEN-864 pharmacokinetics**

| Species | Mass (kg) | T1/2 (hr) | Vd (mL/kg) | CI (ml/hr) |
|---|---|---|---|---|
| Mouse | 0.025 | 33.9 | 140 | 0.07 |
| Rat | 0.206 | 43.6 | 210 | 0.80 |
| Cyno | 2.9 | 125 | 98 | 1.6 |
| Human | 70 | 240* | 91* | 17* |
| *predicted value | | | | |

**Example 21: Pharmacodynamic evaluation of GLP2-XTEN in animal models**

The in vivo pharmacologic activity of the GLP2-2G-XTEN_AE864 fusion protein was assessed using preclinical models of intestinotrophic growth in normal rats and efficacy in mouse DSS-colitis and rat Crohn's Disease.

In vivo evaluation of GLP2-2G-XTEN-AE864 in normal rats

To determine the intestinotrophic properties of GLP2-XTEN, small intestine growth in rats was measured as a primary pharmacodynamic endpoint. GLP2-2G-XTEN-AE864 fusion protein , GLP2-2G peptide, or vehicle was administered via subcutaneous injection into male Sprague-Dawley rats weighing 200-220 grams (10-12 rats per group). GLP2-2G peptide was dosed using the previously published regimen of 12.5 nmol/kg (0.05 mg/kg) twice daily for 12 days. GLP2-2G-XTEN was dosed at 25 nmol/kg once daily for 12 days. After sacrifice, a midline incision was made, the small intestines were removed, stretched to their maximum length and the length recorded. The fecal material was flushed from the lumen and the small intestinal wet weight recorded. The small intestine length and weight data were analyzed with an ANOVA model with a Tukey/Kramer post-hoc test for pairwise comparisons, with significance at p = 0.05.
Results: Treatment with GLP2-2G peptide for 12 days (12.5 nmol/kg/dose using the standard twice daily dosing regimen) resulted in a significant increase in small intestine weight of 24% (FIG. ???A). There were no significant effects on small intestine length. Administration of equal moles GLP2-2G-XTEN over the 12 day study (25 nmol/kg/dose, once daily) resulted in a similar significant increase in small intestine weight of 31%. In contrast to the results seen with GLP2-2G peptide, the small intestine of GLP2-2G-XTEN treated rats showed a significant increase in length of 9% (10 cm), and was visibly thicker than the tissues from vehicle-treated control animals. (FIG. 18).
Conclusions: The results of the study show that GLP2-2G-XTEN induced small intestine growth that was as good or better than GLP2-2G peptide, using equal nmol/kg dosing.

In vivo evaluation of GLP2-2G-XTEN-AE864 in murine acute DSS-induced colitis model

To determine the efficacy of GLP2-XTEN, the GLP2-2G-XTEN-AE864 fusion protein was evaluated in a mouse model of intestinal inflammatory colitis. Intestinal colitis was induced in female C57B1/6 mice (9-10 weeks of age) by feeding mice with 4.5% dextran sodium sulfate (DSS) dissolved in drinking water for 10 days, until ~20% body weight loss is observed. A naive, non-treated control group (group 1) was given normal drinking water for the duration of the experiment. The DSS treated groups (groups 2-7) were treated SC with vehicle (group2), GLP2-2G peptide (no XTEN) (group 3) or GLP2-2G-XTEN (lot AP5100 (groups 4-7). The treatment doses and regimens are outlined in Table 18, below; the GLP-2G peptide was administered BID days 1-10 while the fusion protein was administered QD in the morning with vehicle control administered in the evening days 1-10. Measured parameters included body weights (recorded daily) and the following terminal endpoints, determine at day 10 of the experiment: colon weight and length, small intestine weight and length, and stomach weight. Tissues were fixed in formalin and then transferred to ethanol for staining and histopathology. The anatomical data was analyzed with an ANOVA model with a Tukey/Kramer post-hoc test for pairwise comparisons, with significance at p = 0.05.

**Table 18: Treatment groups**

| GROUP | N | Treatment | Dose | Route | Regimen |
|-------|-----|-----------------------|---------------------------|-------|------------------------------|
| 1 | 10 | Normal water + Vehicle | NA | SC | BID (10-12h) |
| 2 | 10 | DSS + Vehicle | | SC | BID (10-12h) |
| 3 | 10 | DSS + GLP2-2G peptide | 0.05 mg/kg (12.5 nmol/kg) | SC | BID (10-12h) |
| 4 | 10 | DSS + GLP2-2G-XTEN | 6 mg/kg (75 nmol/kg) | SC | Fusion protein AM Vehicle PM |
| 5 | 10 | DSS + GLP2-2G-XTEN | 2 mg/kg (25 nmol/kg) | SC | Fusion protein AM Vehicle PM |
| 6 | 10 | DSS + GLP2-2G-XTEN | 0.2 mg/kg (2.5 nmol/kg) | SC | Fusion protein AM Vehicle PM |
| 7 | 10 | DSS + GLP2-2G-XTEN | 0.02 mg/kg (0.25 nmol/kg) | SC | Fusion protein AM Vehicle PM |

Results: Treatment effects on body weight colon length and weight, small intestine weight and length and stomach weight were assessed on the day of sacrifice. Although DSS-treated mice showed the expected significant decrease in body weight as compared to the control mice (see FIG. 19), neither the mice treated with GLP2-2G peptide nor any of the groups of mice treated with any dose of GLP2-2G-XTEN mice showed a reduced loss of body weight loss over the course of the experiment. With respect to treatment effects on colon, small intestine and stomach, the parameter with a statistically significant change was an increase in small intestine weight in the GLP2-2G-XTEN high dose group (6 mg/kg), compared to the control groups 1 and 2 and the GLP2-2G-XTEN medium dose group (2 mg/kg), compared to group 1 (data not shown). The GLP2-2G peptide did not induce significant growth in the assayed tissues in the current study. Histopathology examination was performed on group 2 (DSS/vehicle treated) and group4 (DSS/GLP2-2G-XTEN 6 mg/kg qd treated). Results of the examination indicated that small intestine samples from the vehicle treated mice show mild-moderate and marked degrees of mucosal atrophy (see FIG. 20A, B). The mucosa were sparsely lined by stunted villi (diminished height) and decreased mucosal thickness. In contrast, small intestine samples from mice treated with GLP2-2G-XTEN at 6 mg/kg qd showed normal mucosal architecture with elongated villi densely populated with columnar epithelial and goblet cells (see FIG. 20C, D). The results support the conclusion that, under the conditions of the experiment, treatment with the GLP2-2G-XTEN fusion protein protected the intestines from the inflammatory effects of DSS, with maintenance of normal villi and mucosal architecture.

Efficacy of GLP2-2G-XTEN vs. GLP2-2G peptide in rat Crohn's Disease indomethacin induced inflammation model

To determine the efficacy of GLP2-XTEN using single dose or qd dosing, the GLP2-2G-XTEN-AE864 fusion protein was evaluated in a rat model of Crohn's Disease of indomethacin-induced intestinal inflammation in three separate studies.

Study 1: Intestinal inflammation was induced in eighty male Wistar rats (Harlan Sprague Dawley) using indomethacin administered on Days 0 and 1 of the experiment. The rats were divided into seven treatment groups for treatment according to Table 19.

**Table 19: Treatment groups**

| GROUP | Treatment | Dose | Route | Regimen | + Indomethacin |
|-------|-----------|------|-------|---------|----------------|
| 1 | Vehicle | 10 ml/kg | SC | BID | No |
| 2 | Vehicle | 10 ml/kg | SC | BID | Yes |
| 3 | GLP2-2G | 0.05 mg/kg (12.5 nmol/kg) | SC | BID | Yes |
| 4 | GLP2-2G | 0.5 mg/kg (125 nmol/kg) | SC | BID | Yes |
| 5 | GLP2-2G-XTEN | 2 mg/kg (25 nmol/kg) | SC | QD | Yes |
| 6 | GLP2-2G-XTEN | 6 mg/kg (75 nmol/kg) | SC | QD | Yes |
| 7 | Prednisolone | 10 mg/kg | PO | QD | Yes |

All treatments were administered per the schedule starting on Day -3 of the experiment. Body weights were determined daily. Groups 3 and 5 were dosed equimolar/day. On Day 2 (24 hours post-2nd indomethacin dose), the animals were prepped for sacrifice and analysis. Thirty minutes prior to sacrifice, the rats were injected intravenously with 1 ml 1% Evans Blue dye, in order to visualize ulcers and extent of inflammation by histopathology analysis. The rats were anesthetized (SOP 1810), blood samples were removed to determine the concentration of GLP-2-2G-XTEN using the anti-XTEN/anti-GLP2 ELISA method. The rats were euthanized then necropsied and scored by gross examination of the intestines for the presence of adhesions; i.e., none = 0, mild = 1, moderate = 2, or severe = 3. The small intestines were removed and the length of each was recorded. In each small intestine, a longitudinal incision was made and the interior was examined. The degree and length of the ulcerated area was recorded as a score; i.e., none = 0, few = 1, multiple = 2, or continuous = 3. For TNF$\alpha$ determination, intestinal samples were thawed and homogenized in a total of 20 ml with DPBS. The supernatants were equilibrated to room temperature and assayed for TNF$\alpha$ by ELISA (R&D Systems, Cat. RTA00, lot 281687, exp. 07SEP11). The samples for Group 1 were assayed undiluted. The samples for Groups 2-7 were diluted 1:4. For histopathology, the small intestines were gently washed with saline to remove the fecal material and were blotted to remove excess fluid. Each small intestine was weighed then processed for histopathology examination to quantitate the degree of inflammation; i.e., .0% = 0, 1-33% = 1, 34-66% = 2, 67-100% = 3.

Results: The values and scores for the body weight and various small intestine parameters are presented graphically in FIG. 21. The changes in parameters and scores for Group 2 control animals versus Group 1 healthy controls indicates that the model is representative of the disease process. Results of body weights (FIG. 21A) indicate that the GLP2-2G did not have a significant increase in body weight compared to disease control (Group 2), while the GLP-2-2G-XTEN groups demonstrated a significant increase. Results from the small intestine length (FIG. 21B) showed a significant increase for both the GLP-2-2G peptide and GLP-2-2G-XTEN fusion protein treatments, with the latter resulting in length equivalent to the non-diseased control (Group 1). Results from the small intestine weight (FIG. 21C) showed a significant increase for the 0.5 mg/kg GLP-2-2G peptide and both GLP-2-2G-XTEN fusion protein groups, compared to diseased control Group 2. Based on gross pathology scoring of the small intestine, both the GLP-2-2G peptide and GLP2-2G-XTEN fusion protein treatments resulted in significant decreases in ulceration (FIG. 21D), with the 6 mg/kg fusion protein resulting in a score that was not significantly different from the non-diseased control (Group 1). Based on scoring of adhesions and transulceration (FIG. 21E), both the GLP-2-2G peptide and GLP-2-2G-XTEN fusion protein treatments showed significant decreases compared to diseased control (Group 2), with the 2 and 6 mg/kg fusion protein resulting in scores that were not significantly different from the non-diseased control (Group 1). Based on scoring of small intestine inflammation (FIG. 21F), neither the GLP-2-2G peptide nor the GLP-2-2G-XTEN fusion protein treatments showed a significant effect on inflammation. Based on TNFα assays (FIG. 21G), both the GLP-2-2G peptide and GLP-2-2G-XTEN fusion protein treatments showed significantly decreased cytokine levels compared to the diseased control Group 2.

Conclusions: The results of the study show that GLP2-2G-XTEN provided efficacy that was as good or better than GLP2-2G peptide, using equal nmol/kg dosing, in improving indomethacin-induced small intestine damage.

Study 2: Intestinal inflammation was induced in eighty male Wistar rats (Harlan Sprague Dawley) using indomethacin administered on Days 0 and 1 of the experiment. The rats were divided into eight treatment groups for treatment according to Table 20.

**Table 20: Treatment groups**

| GROUP | Treatment | Dose | Route | Regimen | + Indomethacin |
|-------|-----------|------|-------|---------|----------------|
| 1 | Vehicle | 10 ml/kg | SC | BID | No |
| 2 | Vehicle | 10 ml/kg | SC | BID | Yes |
| 3 | GLP2-2G | 0.05 mg/kg (12.5 nmol/kg) | SC | BID | Yes |
| 4 | GLP2-2G-XTEN | 2 mg/kg (25 nmol/kg) | SC | Once daily (QD) | Yes |
| 5 | GLP2-2G-XTEN | 0.22 mg/kg (2.5 nmol/kg) | SC | Once day -3 only | Yes |
| 6 | GLP2-2G-XTEN | 0.66 mg/kg (7.5 nmol/kg) | SC | Once day -3 only | Yes |
| 7 | GLP2-2G-XTEN | 2 mg/kg (25 nmol/kg) | SC | Once day -3 only | Yes |
| 8 | GLP2-2G-XTEN | 6 mg/kg (75 nmol/kg) | SC | Once day -3 only | Yes |

All treatments were administered per the schedule starting on Day -3 of the experiment. Body weights were determined daily. On Day 2 (24 hours post-2nd indomethacin dose), the animals were prepped for sacrifice and analysis. Thirty minutes prior to sacrifice, the rats were injected intravenously with 1 ml 1% Evans Blue dye, in order to visualize ulcers and extent of inflammation by histopathology analysis. The rats were anesthetized and blood samples were removed to determine the concentration of GLP-2-2G-XTEN using the anti-XTEN/anti-GLP2 ELISA method. The rats were euthanized then necropsied and scored by gross examination of the intestines for the presence of adhesions; i.e., none = 0, mild = 1, moderate = 2, or severe = 3. The small intestines were removed and the length of each was recorded. In each small intestine, a longitudinal incision was made and the interior was examined. The degree and length of the ulcerated area was recorded as a score; i.e., none = 0, few = 1, multiple = 2, or continuous = 3. The fecal material was washed away with saline and blotted to remove excess fluid and each small intestine was weighed then processed for histopathology examination to quantitate the degree of inflammation; i.e., .0% = 0, 1-33% = 1, 34-66% = 2, 67-100% = 3.

Results: The scores for the various parameters are presented graphically in FIG. 22. In the vehicle negative control group, the gross pathologic changes due to indomethacin treatment were most severe in the ileum and jejunum, with a total disease score of 8.5-9 by assessment of this group. Of the various GLP-2-2G peptide and GLP-2-2G-XTEN treatment groups, the GLP-2-2G peptide delivered bid, the GLP-2-2G-XTEN delivered qd, and the single doses of GLP-2-2G-XTEN at 6 or 2 mg/kg resulted in significantly improved scores compared to the indomethacin-treated vehicle control group. In the transulceration scores, the same treatment groups as per the total disease score reached statistical significance (FIG. 22A, with star indicating statistically significant difference compared to vehicle group). In the adhesions score analysis, the indomethacin-treated vehicle control group approached the maximum score of 3 (FIG. 22B). Once-daily treatment with the GLP-2-2G-XTEN provided nearly complete protection from adhesions, and the single high-dose 6 mg/kg GLP-2-2G-XTEN group reached statistically significant difference compared to vehicle control (star in figure indicating statistically significant difference), as did the daily bid dosed GLP-2-2G peptide group. In the small intestine length analysis (with the non-indomethacin treated group normalized to 100%), the once-daily treatment with the GLP-2-2G-XTEN group and the daily bid dosed GLP-2-2G peptide group reached statistically significant difference compared to indomethacin-treated vehicle control group. The histopathology assessment finding were essentially similar to the gross pathology findings. The histopathologic changes in the vehicle control group due to indomethacin treatment were most severe in the ileum and jejunum. The vehicle control group showed severe mucosal atrophy, ulceration and infiltration (FIG. 23A). The protective effects of the daily bid GLP-2-2G peptide and once-daily GLP-2-2G-XTEN treatments were most pronounced in the ileum, but were also seen in the jejunum. Group 3 had one rat with essentially normal tissue (FIG. 23B) while two rats each showed ulceration and infiltration but no atrophy and two rats had histopathologic changes similar to the vehicle control disease group 2. Group 4 (FIG. 23D) showed protective effects with two rats with essentially normal tissue, one rat showing no atrophy or ulceration but with slight infiltration, one rat with no atrophy but slight ulceration and infiltration, and one rat had histopathologic changes similar to the vehicle control disease group 2. Group 7 showed protective effects with one rat with essentially normal tissue, two rats with no ulceration or infiltration but showing muscular atrophy, and two rats had histopathologic changes similar to the vehicle control disease group 2. Group 8 (FIG. 23C) showed protective effects with one rat with no ulceration or infiltration, one rat with reduced ulceration and infiltration, and three rats had histopathologic changes similar to the vehicle control disease group 2. The ELISA results indicate that the GLP-2-2G-XTEN fusion protein was detectable at Day 2 in all animals of Group 4 and Group 8, and three rats in Group 7.

The results support the conclusion that, under the conditions of the experiment, treatment with the GLP2-2G-XTEN fusion protein provided significant protection to the intestines from the inflammatory effects of indomethacin, with daily dosing at 2 mg/kg showing the greatest efficacy and single doses of 6 mg/kg or 2 mg/kg showing significant efficacy in some parameters.

Study 3: A third indomethacin-induced inflammation study was performed to verify previous results and test additional dose regimens. Intestinal inflammation was induced in male Wistar rats (Harlan Sprague Dawley) using indomethacin administered on Days 0 and 1 of the experiment according to Table 21.

**Table 21: Treatment groups**

| GROUP | Treatment | Dose | Route | Regimen | Total Dose |
|---|---|---|---|---|---|
| 1 | Vehicle | 10 ml/kg | SC | QD | ND |
| 2 | GLP2-2G | 0.05 mg/kg (12.5 nmol/kg) | SC | BID | 125 nmol/kg |
| 3 | GLP2-2G-XTEN | 2 mg/kg (25 nmol/kg) | SC | Once daily (QD) | 125 nmol/kg |
| 4 | GLP2-2G-XTEN | 2 mg/kg (25 nmol/kg) | SC | Day -3, -1, 1 (Q2D) | 75 nmol/kg |
| 5 | GLP2-2G-XTEN | 6 mg/kg (75 nmol/kg) | SC | Once day -3 only | 75 nmol/kg |

All treatments were administered per the schedule starting on Day -3 of the experiment. Body weights were determined daily. On Day 2 (24 hours post-2nd indomethacin dose), the animals were prepped for sacrifice and analysis. The small intestines were removed and the length of each was recorded. Quantitative histopathology was performed on a subset of samples. Rat small intestine samples consisted of a 3 cm section of proximal jejunum and a 3 cm section of mid-jejunum collected 15 cm and 30 cm from the pylorus, respectively. Samples were fixed in 10% neutral buffered formalin. Samples were trimmed into multiple sections without bias toward lesion presence or absence. These sections were placed in cassettes, embedded in paraffin, microtomed at approximately 4 microns thickness, and stained with hematoxylin and eosin (H&E). The slides were evaluated microscopically by a board certified veterinary pathologist and scored for villous height as well as infiltration/inflammation, mucosal atrophy, villi/crypt appearance, abscesses/ulceration. A 1 to 4 severity grading scale was used, where 1 = minimal, 2 = mild, 3 = moderate, 4 = marked/severe, reflecting the combination of the cellular reactions seen histopathologically. Small intestine length was analyzed with an ANOVA model with a Tukey/Kramer post-hoc test for pairwise comparisons, with significance at $p = 0.05$. Non-parametric histology score variables were compared with the vehicle control using a Mann Whitney U test with a Bonferroni correction for the p-value to create an overall alpha of 0.05.

Results: As seen in the initial studies, there was an increase in small intestine length in the GLP2-2G-XTEN-treated diseased rats as compared to vehicle-treated diseased rats (FIG. 24A). This increase correlated with a significant increase in villi height (FIG. 24B). Both high (total dose of 125 nmol/kg) and low (total dose of 75 nmol/kg) dose GLP2-2G-XTEN-treated groups showed a significant increase in villi height; the increase in villi height seen in peptide treated rats was not significant. There was also a significant decrease in mucosal atrophy as both high and low dose GLP2-2G-XTEN-treated rats showed a significantly lower mucosal atrophy score than vehicle-treated diseased rats (FIG. 24C). Although there was a trend showing a reduction in mucosal ulceration and mixed cell infiltrate following GLP2-2G-XTEN and GLP2-2G peptide treatment, these results were not significant for any of the three treatment groups.

Conclusions: Histopathological results support the conclusion that GLP2-2G-XTEN provided efficacy that was as good or better than GLP2-2G peptide in improving indomethacin-induced small intestine damage. Furthermore, GLP2-2G-XTEN dosed once at 75 nmol/kg or three times at 25 nmol/kg is as effective as GLP2-2G peptide dosed ten times at 12.5 nmol/kg.

**Example 22: Human Clinical Trial Designs for Evaluating GLP2-XTEN comprising GLP-2**

As demonstrated in Examples 18-20, fusion of XTEN to the C-terminus of GLP-2-2glycine results in improved half-life compared to that known for the native form of the GLP-2 or the GLP-2-2G peptide, which, it is believed, would enable a reduced dosing frequency yet still result in clinical efficacy when using such GLP2-XTEN-containing fusion protein compositions. Clinical trials in humans comparing a GLP2-XTEN fusion protein to GLP-2 (or GLP-2-2G peptide) formulations are performed to establish the efficacy and advantages, compared to current or experimental modalities, of the GLP2-XTEN binding fusion protein compositions. Such studies comprise three phases. First, a Phase I safety and pharmacokinetics study in adult patients is conducted to determine the maximum tolerated dose and pharmacokinetics and pharmacodynamics in humans (e.g., normal healthy volunteer subjects), as well as to define potential toxicities and adverse events to be tracked in future studies. A Phase I study is conducted in which single rising doses of a GLP2-XTEN composition, such as are disclosed herein, are administered by the desired route (e.g., by subcutaneous, intramuscular, or intravenous routes) and biochemical, PK, and clinical parameters are measured at defined intervals, as well as adverse events. A Phase Ib study will multiple doses would follow, also measuring the biochemical, PK, and clinical parameters at defined intervals. This would permit the determination of the minimum effective dose and the maximum tolerated dose and establishes the threshold and maximum concentrations in dosage and circulating drug that constitute the therapeutic window for the active component. From this information, the dose and dose schedule that permits less frequent administration of the GLP2-XTEN compositions (compared to GLP-2 not linked to XTEN), yet retains the pharmacologic response, is obtained. Thereafter, Phase II and III clinical trials are conducted in patients with the GLP-2 associated condition, verifying the effectiveness and safety of the GLP2-XTEN compositions under the dose conditions. Clinical trials could be conducted in patients suffering from any disease in which native GLP-2 or the standard of care for the given condition may be expected to provide clinical benefit. For example, such indications include gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, mucosal damage of the small intestine, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy-associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, ischemia, and stroke. Trials monitor patients before, during and after treatment for changes in physiologic and clinical parameters associated with the respective indications; e.g., weight gain, inflammation, cytokine levels, pain, bowel function, appetite, febrile episodes, wound healing, glucose levels; enhancing or accelerating hunger satiety; parameters that are tracked relative to the placebo or positive control groups. Efficacy outcomes are determined using standard statistical methods. Toxicity and adverse event markers are also followed in the study to verify that the compound is safe when used in the manner described.

**Example 23: GLP2-XTEN with cleavage sequences**

<u>C-terminal XTEN releasable by FXIa</u>

An GLP2-XTEN fusion protein consisting of an XTEN protein fused to the C-terminus of GLP-2 can be created with a XTEN release site cleavage sequence placed in between the GLP-2 and XTEN components, as depicted in FIG. 7. Exemplary sequences are provided in Table 34. In this case, the release site cleavage sequence can be incorporated into the GLP2-XTEN that contains an amino acid sequence that is recognized and cleaved by the FXIa protease (EC 3.4.21.27, Uniprot P03951). Specifically the amino acid sequence KLTRAET is cut after the arginine of the sequence by FXIa protease. FXI is the pro-coagulant protease located immediately before FVIII in the intrinsic or contact activated coagulation pathway. Active FXIa is produced from FXI by proteolytic cleavage of the zymogen by FXIIa. Production of FXIa is tightly controlled and only occurs when coagulation is necessary for proper hemostasis. Therefore, by incorporation of the KLTRAET cleavage sequence, the XTEN domain is removed from GLP-2 concurrent with activation of the intrinsic coagulation pathway in proximity to the GLP2-XTEN.

<u>C-terminal XTEN releasable by Elastase-2</u>

An GLP2-XTEN fusion protein consisting of an XTEN protein fused to the C-terminus of GLP-2 can be created with a XTEN release site cleavage sequence placed in between the GLP-2 and XTEN components, as depicted in FIG. 7. Exemplary sequences are provided in Table 34. In this case, the release site contains an amino acid sequence that is recognized and cleaved by the elastase-2 protease (EC 3.4.21.37, Uniprot P08246). Specifically the sequence LGPVSGVP [Rawlings N.D., et al. (2008) Nucleic Acids Res., 36: D320], is cut after position 4 in the sequence. Elastase is constitutively expressed by neutrophils and is present at all times in the circulation, but particularly during acute inflammation. Therefore as the long lived GLP2-XTEN circulates, a fraction of it is cleaved, particularly locally during inflammatory responses (e.g., inflammation of the bowel), creating a pool of shorter-lived GLP-2 at the site of inflammation, e.g., in Crohn's Disease, where the GLP-2 is most needed.

**C-terminal XTEN releasable bv MMP-12**

An GLP2-XTEN fusion protein consisting of an XTEN protein fused to the C-terminus of GLP-2 can be created with a XTEN release site cleavage sequence placed in between the GLP-2 and XTEN components, as depicted in FIG. 7. Exemplary sequences are provided in Table 34. In this case, the release site contains an amino acid sequence that is recognized and cleaved by the MMP-12 protease (EC 3.4.24.65, Uniprot P39900). Specifically the sequence GPAGLGGA [Rawlings N.D., et al. (2008) Nucleic Acids Res., 36: D320], is cut after position 4 of the sequence. MMP-12 is constitutively expressed in whole blood. Therefore as the GLP2-XTEN circulates, a fraction of it is cleaved, creating a pool of shorter-lived GLP-2 to be used. In a desirable feature of the inventive composition, this creates a circulating pro-drug depot that constantly releases a prophylactic amount of GLP-2, with higher amounts released during an inflammatory response, e.g., in Crohn's Disease, where the GLP-2 is most needed.

**C-terminal XTEN releasable by MMP-13**

An GLP2-XTEN fusion protein consisting of an XTEN protein fused to the C-terminus of GLP-2 can be created with a XTEN release site cleavage sequence placed in between the GLP-2 and XTEN components, as depicted in FIG. 7. Exemplary sequences are provided in Table 34. In this case, the release site contains an amino acid sequence that is recognized and cleaved by the MMP-13 protease (EC 3.4.24.-, Uniprot P45452). Specifically the sequence GPAGLRGA [Rawlings N.D., et al. (2008) Nucleic Acids Res., 36: D320], is cut after position 4. MMP-13 is constitutively expressed in whole blood. Therefore as the long lived GLP2-XTEN circulates, a fraction of it is cleaved, creating a pool of shorter-lived GLP-2 to be used. In a desirable feature of the inventive composition, this creates a circulating pro-drug depot that constantly releases a prophylactic amount of GLP-2, with higher amounts released during an inflammatory response, e.g., in Crohn's Disease, where the GLP-2 is most needed.

**C-terminal XTEN releasable by MMP-17**

A GLP2-XTEN fusion protein consisting of an XTEN protein fused to the C-terminus of GLP-2 can be created with a XTEN release site cleavage sequence placed in between the GLP-2 and XTEN components, as depicted in FIG. 7. Exemplary sequences are provided in Table 34. In this case, the release site contains an amino acid sequence that is recognized and cleaved by the MMP-20 protease (EC.3.4.24.-, Uniprot Q9ULZ9). Specifically the sequence APLGLRLR [Rawlings N.D., et al. (2008) Nucleic Acids Res., 36: D320], is cut after position 4 in the sequence. MMP-17 is constitutively expressed in whole blood. Therefore as the GLP2-XTEN circulates, a fraction of it is cleaved, creating a pool of shorter-lived GLP-2 to be used. In a desirable feature of the inventive composition, this creates a circulating pro-drug depot that constantly releases a prophylactic amount of GLP-2, with higher amounts released during an inflammatory response, e.g., in Crohn's Disease, where the GLP-2 is most needed.

**C-terminal XTEN releasable by MMP-20**

A GLP2-XTEN fusion protein consisting of an XTEN protein fused to the C-terminus of GLP-2 can be created with a XTEN release site cleavage sequence placed in between the GLP-2 and XTEN components, as depicted in FIG. 7. Exemplary sequences are provided in Table 34. In this case, the release site contains an amino acid sequence that is recognized and cleaved by the MMP-20 protease (EC.3.4.24.-, Uniprot O60882). Specifically the sequence PALPLVAQ [Rawlings N.D., et al. (2008) Nucleic Acids Res., 36: D320], is cut after position 4 (depicted by the arrow). MMP-20 is constitutively expressed in whole blood. Therefore as the GLP2-XTEN circulates, a fraction of it is cleaved, creating a pool of shorter-lived GLP-2 to be used. In a desirable feature of the inventive composition, this creates a circulating pro-drug depot that constantly releases a prophylactic amount of GLP-2, with higher amounts released during an inflammatory response, e.g., in Crohn's Disease, where the GLP-2 is most needed.

**Optimization of the release rate of C-terminal XTEN**

Variants of the foregoing constructs of the Examples can be created in which the release rate of C-terminal XTEN is altered. As the rate of XTEN release by an XTEN release protease is dependent on the sequence of the XTEN release site, by varying the amino acid sequence in the XTEN release site one can control the rate of XTEN release. The sequence specificity of many proteases is well known in the art, and is documented in several data bases. In this case, the amino acid specificity of proteases is mapped using combinatorial libraries of substrates [Harris, JL, et al. (2000) Proc Natl Acad Sci U S A, 97: 7754] or by following the cleavage of substrate mixtures as illustrated in [Schellenberger, V, et al. (1993) Biochemistry, 32: 4344]. An alternative is the identification of optimal protease cleavage sequences by phage display [Matthews, D., et al. (1993) Science, 260: 1113]. Constructs are made with variant sequences and assayed for XTEN release using standard assays for detection of the XTEN.

**Example 24: Biodistribution of large XTEN molecules**

To verify that constructs with long XTEN fusions can penetrate into tissue, the biodistribution of three fluorescently tagged constructs were tested in mice, aHer2-XTEN-864-Alexa 680, aHer2-XTEN-576-Alexa 680, and aHer2-XTEN-288-Alexa 680, using fluorescence imaging. The aHer2 payload is a scFv fragment specific for binding the Her2 antigen, which is not found on normal tissues (and hence should not affect biodistribution in normal animals). This study also included fluorescently tagged Herceptin-Alexa 680 as a control antibody. The mice were given a single intravenous injection of each agent. After 72 hours, all groups were euthanized and liver, lung, heart, spleen and kidneys were ex vivo imaged using fluorescence imaging. The data are shown Table 22.

Conclusions: All constructs showed significant penetration into all tissues assayed. The lower overall fluorescence signals of the XTEN_576 and XTEN_288 groups are attributed to the increased clearance of the shorter XTEN constructs over the 72 hour distribution period. Similar proportions for lung fluorescence relative to total signal were observed for all groups, including the antibody control, supporting that XTEN fusion protein constructs are bioavailable in tissue under these conditions.

**Table 22: Fluorescence Signals by Organ**

| Test Material | Dose (nmol/ mouse) | Total Fluorescence Efficiency (group mean) (x1e-6) | | | | |
|---|---|---|---|---|---|---|
| | | Heart | Lungs | Spleen | Liver | Kidney |
| scFv-XTEN-864-Alexa 680 | 6.7 | 28 | 130 | 16 | 180 | 120 |
| scFv-XTEN-576-Alexa 680 | 6.7 | 6.8 | 24 | 3.4 | 48 | 31 |
| scFv-XTEN-288-Alexa 680 | 6.7 | 1.9 | 5.6 | 2.1 | 20 | 34 |
| mAb-Alexa680 Control | 3.3 | 32 | 150 | 25 | 370 | 110 |

**Example 25: Analytical size exclusion chromatography of XTEN fusion proteins with diverse payloads**

Size exclusion chromatography analyses were performed on fusion proteins containing various therapeutic proteins and unstructured recombinant proteins of increasing length. An exemplary assay used a TSKGel-G4000 SWXL (7.8mm x 30cm) column in which 40 $\mu$g of purified glucagon fusion protein at a concentration of 1 mg/ml was separated at a flow rate of 0.6 ml/min in 20 mM phosphate pH 6.8, 114 mM NaCl. Chromatogram profiles were monitored using OD214nm and OD280nm. Column calibration for all assays were performed using a size exclusion calibration standard from BioRad; the markers include thyroglobulin (670 kDa), bovine gamma-globulin (158 kDa), chicken ovalbumin (44 kDa), equine myoglobuin (17 kDa) and vitamin B12 (1.35 kDa). Representative chromatographic profiles of Glucagon-Y288, Glucagon-Y144, Glucagon-Y72, Glucagon-Y36 are shown as an overlay in FIG. 25. The data show that the apparent molecular weight of each compound is proportional to the length of the attached XTEN sequence. However, the data also show that the apparent molecular weight of each construct is significantly larger than that expected for a globular protein (as shown by comparison to the standard proteins run in the same assay). Based on the SEC analyses for all constructs evaluated, the apparent molecular weights, the apparent molecular weight factor (expressed as the ratio of apparent molecular weight to the calculated molecular weight) and the hydrodynamic radius ($R_H$ in nm) are shown in Table 23. The results indicate that incorporation of different XTENs of 576 amino acids or greater confers an apparent molecular weight for the fusion protein of approximately 339 kDa to 760, and that XTEN of 864 amino acids or greater confers an apparent molecular weight greater than at least approximately 800 kDA. The results of proportional increases in apparent molecular weight to actual molecular weight were consistent for fusion proteins created with XTEN from several different motif families; i.e., AD, AE, AF, AG, and AM, with increases of at least four-fold and ratios as high as about 17-fold. Additionally, the incorporation of XTEN fusion partners with 576 amino acids or more into fusion proteins with the various payloads (and 288 residues in the case of glucagon fused to Y288) resulted with a hydrodynamic radius of 7 nm or greater; well beyond the glomerular pore size of approximately 3-5 nm. Accordingly, it is expected that fusion proteins comprising growth and XTEN have reduced renal clearance, contributing to increased terminal half-life and improving the therapeutic or biologic effect relative to a corresponding un-fused biologic payload protein.

**Table 23: SEC analysis of various polypeptides**

| Construct Name | XTEN or fusion partner | Therapeutic Protein | Actual MW (kDa) | Apparent MW (kDa) | Apparent Molecular Weight Factor | R_H (nm) |
|---|---|---|---|---|---|---|
| AC14 | Y288 | Glucagon | 28.7 | 370 | 12.9 | 7.0 |
| AC28 | Y144 | Glucagon | 16.1 | 117 | 7.3 | 5.0 |
| AC34 | Y72 | Glucagon | 9.9 | 58.6 | 5.9 | 3.8 |
| AC33 | Y36 | Glucagon | 6.8 | 29.4 | 4.3 | 2.6 |
| AC89 | AF120 | Glucagon | 14.1 | 76.4 | 5.4 | 4.3 |
| AC88 | AF108 | Glucagon | 13.1 | 61.2 | 4.7 | 3.9 |
| AC73 | AF144 | Glucagon | 16.3 | 95.2 | 5.8 | 4.7 |
| AC53 | AG576 | GFP | 74.9 | 339 | 4.5 | 7.0 |
| AC39 | AD576 | GFP | 76.4 | 546 | 7.1 | 7.7 |
| AC41 | AE576 | GFP | 80.4 | 760 | 9.5 | 8.3 |
| AC52 | AF576 | GFP | 78.3 | 526 | 6.7 | 7.6 |
| AC398 | AE288 | FVII | 76.3 | 650 | 8.5 | 8.2 |
| AC404 | AE864 | FVII | 129 | 1900 | 14.7 | 10.1 |
| AC85 | AE864 | Exendin-4 | 83.6 | 938 | 11.2 | 8.9 |
| AC114 | AM875 | Exendin-4 | 82.4 | 1344 | 16.3 | 9.4 |
| AC143 | AM875 | hGH | 100.6 | 846 | 8.4 | 8.7 |
| AC227 | AM875 | IL-1ra | 95.4 | 1103 | 11.6 | 9.2 |
| AC228 | AM1318 | IL-Ira | 134.8 | 2286 | 17.0 | 10.5 |

**Example 26: Pharmacokinetics of extended polypeptides fused to GFP in cynomolgus monkeys**

The pharmacokinetics of GFP-L288, GFP-L576, GFP-XTEN AF576, GFP-XTEN_Y576 and XTEN_AD836-GFP were tested in cynomolgus monkeys to determine the effect of composition and length of the unstructured polypeptides on PK parameters. Blood samples were analyzed at various times after injection and the concentration of GFP in plasma was measured by ELISA using a polyclonal antibody against GFP for capture and a biotinylated preparation of the same polyclonal antibody for detection. Results are summarized in FIG. 26. They show a surprising increase of half-life with increasing length of the XTEN sequence. For example, a half-life of 10 h was determined for GFP-XTEN_L288 (with 288 amino acid residues in the XTEN). Doubling the length of the unstructured polypeptide fusion partner to 576 amino acids increased the half-life to 20-22 h for multiple fusion protein constructs; i.e., GFP-XTEN_L576, GFP-XTEN_AF576, GFP-XTEN_Y576. A further increase of the unstructured polypeptide fusion partner length to 836 residues resulted in a half-life of 72-75 h for XTEN_AD836-GFP. Thus, increasing the polymer length by 288 residues from 288 to 576 residues increased in vivo half-life by about 10 h. However, increasing the polypeptide length by 260 residues from 576 residues to 836 residues increased half-life by more than 50 h. These results show that there is a surprising threshold of unstructured polypeptide length that results in a greater than proportional gain in *in vivo* half-life. Thus, fusion proteins comprising extended, unstructured polypeptides are expected to have the property of enhanced pharmacokinetics compared to polypeptides of shorter lengths.

**Example 27: Serum stability of XTEN**

A fusion protein containing XTEN_AE864 fused to the N-terminus of GFP was incubated in monkey plasma and rat kidney lysate for up to 7 days at 37°C. Samples were withdrawn at time 0, Day 1 and Day 7 and analyzed by SDS PAGE followed by detection using Western analysis and detection with antibodies against GFP as shown in FIG. 27. The sequence of XTEN_AE864 showed negligible signs of degradation over 7 days in plasma. However, XTEN _AE864 was rapidly degraded in rat kidney lysate over 3 days. The in vivo stability of the fusion protein was tested in plasma samples wherein the GFP AE864 was immunoprecipitated and analyzed by SDS PAGE as described above. Samples that were withdrawn up to 7 days after injection showed very few signs of degradation. The results demonstrate the resistance of GLP2-XTEN to degradation due to serum proteases; a factor in the enhancement of pharmacokinetic properties of the GLP2-XTEN fusion proteins.

**Example 28: Increasing solubility and stability of a peptide payload by linking to XTEN**

In order to evaluate the ability of XTEN to enhance the physicochemical properties of solubility and stability, fusion proteins of glucagon plus shorter-length XTEN were prepared and evaluated. The test articles were prepared in Tris-buffered saline at neutral pH and characterization of the Gcg-XTEN solution was by reverse-phase HPLC and size exclusion chromatography to affirm that the protein was homogeneous and non-aggregated in solution. The data are presented in Table 24. For comparative purposes, the solubility limit of unmodified glucagon in the same buffer was measured at 60 $\mu$M (0.2 mg/mL), and the result demonstrate that for all lengths of XTEN added, a substantial increase in solubility was attained. Importantly, in most cases the glucagon-XTEN fusion proteins were prepared to achieve target concentrations and were not evaluated to determine the maximum solubility limits for the given construct. However, in the case of glucagon linked to the AF-144 XTEN, the limit of solubility was determined, with the result that a 60-fold increase in solubility was achieved, compared to glucagon not linked to XTEN. In addition, the glucagon-AF144 GLP2-XTEN was evaluated for stability, and was found to be stable in liquid formulation for at least 6 months under refrigerated conditions and for approximately one month at 37°C (data not shown).

The data support the conclusion that the linking of short-length XTEN polypeptides to a biologically active protein such as glucagon can markedly enhance the solubility properties of the protein by the resulting fusion protein, as well as confer stability at the higher protein concentrations.

**Table 24: Solubility of Glucagon-XTEN constructs**

| Test Article | Solubility |
|---|---|
| Glucagon | 60 $\mu$M |
| Glucagon-Y36 | >370 $\mu$M |
| Glucagon-Y72 | >293 $\mu$M |
| Glucagon-AF108 | >145 $\mu$M |
| Glucagon-AF120 | >160 $\mu$M |
| Glucagon-Y144 | >497 $\mu$M |
| Glucagon-AE144 | >467 $\mu$M |
| Glucagon-AF144 | >3600 $\mu$M |
| Glucagon-Y288 | >163 $\mu$M |

**Example 29: Analysis of sequences for secondary structure by prediction algorithms**

Amino acid sequences can be assessed for secondary structure via certain computer programs or algorithms, such as the well-known Chou-Fasman algorithm (Chou, P. Y., et al. (1974) Biochemistry, 13: 222-45) and the Garnier-Osguthorpe-Robson, or "GOR" method (Garnier J, Gibrat JF, Robson B. (1996). GOR method for predicting protein secondary structure from amino acid sequence. Methods Enzymol 266:540-553). For a given sequence, the algorithms can predict whether there exists some or no secondary structure at all, expressed as total and/or percentage of residues of the sequence that form, for example, alpha-helices or beta-sheets or the percentage of residues of the sequence predicted to result in random coil formation.

Several representative sequences from XTEN "families" have been assessed using two algorithm tools for the Chou-Fasman and GOR methods to assess the degree of secondary structure in these sequences. The Chou-Fasman tool was provided by William R. Pearson and the University of Virginia, at the "Biosupport" internet site, URL located on the World Wide Web at .fasta.bioch.virginia.edu/fasta_www2/fasta_www.cgi?rm=misc1 as it existed on June 19, 2009. The GOR tool was provided by Pole Informatique Lyonnais at the Network Protein Sequence Analysis internet site, URL located on the World Wide Web at .npsa-pbil.ibcp.fr/cgi-bin/secpred_gor4.pl as it existed on June 19, 2008.

As a first step in the analyses, a single XTEN sequence was analyzed by the two algorithms. The AE864 composition is a XTEN with 864 amino acid residues created from multiple copies of four 12 amino acid sequence motifs consisting of the amino acids G, S, T, E, P, and A. The sequence motifs are characterized by the fact that there is limited repetitiveness within the motifs and within the overall sequence in that the sequence of any two consecutive amino acids is not repeated more than twice in any one 12 amino acid motif, and that no three contiguous amino acids of full-length the XTEN are identical. Successively longer portions of the AF 864 sequence from the N-terminus were analyzed by the Chou-Fasman and GOR algorithms (the latter requires a minimum length of 17 amino acids). The sequences were analyzed by entering the FASTA format sequences into the prediction tools and running the analysis. The results from the analyses are presented in Table 25.

The results indicate that, by the Chou-Fasman calculations, short XTEN of the AE and AG families, up to at least 288 amino acid residues, have no alpha-helices or beta sheets, but amounts of predicted percentage of random coil by the GOR algorithm vary from 78-99%. With increasing XTEN lengths of 504 residues to greater than 1300, the XTEN analyzed by the Chou-Fasman algorithm had predicted percentages of alpha-helices or beta sheets of 0 to about 2%, while the calculated percentages of random coil increased to from 94-99%. Those XTEN with alpha-helices or beta sheets were those sequences with one or more instances of three contiguous serine residues, which resulted in predicted beta-sheet formation. However, even these sequences still had approximately 99% random coil formation.

The data provided herein suggests that 1) XTEN created from multiple sequence motifs of G, S, T, E, P, and A that have limited repetitiveness as to contiguous amino acids are predicted to have very low amounts of alpha-helices and beta-sheets; 2) that increasing the length of the XTEN does not appreciably increase the probability of alpha-helix or beta-sheet formation; and 3) that progressively increasing the length of the XTEN sequence by addition of non-repetitive 12-mers consisting of the amino acids G, S, T, E, P, and A results in increased percentage of random coil formation. Results further indicate that XTEN sequences defined herein (including e.g., XTEN created from sequence motifs of G, S, T, E, P, and A) have limited repetitiveness (including those with no more than two identical contiguous amino acids in any one motif) are expected to have very limited secondary structure. Any order or combination of sequence motifs from Table 3 can be used to create an XTEN polypeptide that will result in an XTEN sequence that is substantially devoid of secondary structure, though three contiguous serines are not preferred. The unfavorable property of three contiguous series however, can be ameliorated by increasing the length of the XTEN. Such sequences are expected to have the characteristics described in the GLP2-XTEN embodiments of the invention disclosed herein.

**Table 25: CHOU-FASMAN and GOR prediction calculations of polypeptide sequences**

| SEQ NAME | Sequence | No. Residues | Chou-Fasman Calculation | GOR Calculation |
|---|---|---|---|---|
| AE36: LCW0402 _002 | GSPAGSPTSTEEGTSESATPESGPGTST EPSEGSAP | 36 | Residue totals: H: 0 E: 0<br>percent: H: 0.0 E: 0.0 | 94.44% |
| AE36: LCW0402 _003 | GTSTEPSEGSAPGTSTEPSEGSAPGTST EPSEGSAP | 36 | Residue totals: H: 0 E: 0<br>percent: H: 0.0 E: 0.0 | 94.44% |
| AG36: LCW0404 _001 | GASPGTSSTGSPGTPGSGTASSSPGSST PSGATGSP | 36 | Residue totals: H: 0 E: 0<br>percent: H: 0.0 E: 0.0 | 77.78% |
| AG36: LCW0404 _003 | GSSTPSGATGSPGSSPSASTGTGPGSST | 36 | Residue totals: H: 0 E: 0 | 83.33 % |
| | PSGATGSP | | percent: H: 0.0 E: 0.0 | |
| AE42_1 | TEPSEGSAPGSPAGSPTSTEEGTSESAT PESGPGSEPATSGS | 42 | Residue totals: H: 0 E: 0<br>percent: H: 0.0 E: 0.0 | 90.48% |

(continued)

| SEQ NAME | Sequence | No. Residues | Chou-Fasman Calculation | GOR Calculation |
|---|---|---|---|---|
| AE42_1 | TEPSEGSAPGSPAGSPTSTEEGTSESAT PESGPGSEPATSGS | 42 | Residue totals: H: 0 E: 0<br>percent: H: 0.0 E: 0.0 | 90.48% |
| AG42_1 | GAPSPSASTGTGPGTPGSGTASSSPGS STPSGATGSPGPSGP | 42 | Residue totals: H: 0 E: 0<br>percent: H: 0.0 E: 0.0 | 88.10% |
| AG42_2 | GPGTPGSGTASSSPGSSTPSGATGSPG SSPSASTGTGPGASP | 42 | Residue totals: H: 0 E: 0<br>percent: H: 0.0 E: 0.0 | 88.10% |
| AE144 | GSEPATSGSETPGTSESATPESGPGSEP ATSGSETPGSPAGSPTSTEEGTSTEPSE GSAPGSEPATSGSETPGSEPATSGSETP GSEPATSGSETPGTSTEPSEGSAPGTSE SATPESGPGSEPATSGSETPGTSTEPSE GSAP | 144 | Residue totals: H: 0 E: 0<br>percent: H: 0.0 E: 0.0 | 98.61 % |
| AG144_1 | PGSSPSASTGTGPGSSPSASTGTGPGTP GSGTASSSPGSSTPSGATGSPGSSPSAS TGTGPGASPGTSSTGSPGTPGSGTASS SPGSSTPSGATGSPGTPGSGTASSSPG ASPGTSSTGSPGASPGTSSTGSPGTPGS GTASSS | 144 | Residue totals: H: 0 E: 0<br>percent: H: 0.0 E: 0.0 | 91.67% |
| AE288 | GTSESATPESGPGSEPATSGSETPGTSE SATPESGPGSEPATSGSETPGTSESATP ESGPGTSTEPSEGSAPGSPAGSPTSTEE GTSESATPESGPGSEPATSGSETPGTSE SATPESGPGSPAGSPTSTEEGSPAGSPT STEEGTSTEPSEGSAPGTSESATPESGP GTSESATPESGPGTSESATPESGPGSEP ATSGSETPGSEPATSGSETPGSPAGSPT STEEGTSTEPSEGSAPGTSTEPSEGSAP GSEPATSGSETPGTSESATPESGPGTST EPSEGSAP | 288 | Residue totals: H: 0 E: 0<br>percent: H: 0.0 E: 0.0 | 99.31% |
| AG288_2 | GSSPSASTGTGPGSSPSASTGTGPGTP GSGTASSSPGSSTPSGATGSPGSSPSAS TGTGPGASPGTSSTGSPGTPGSGTASS SPGSSTPSGATGSPGTPGSGTASSSPG ASPGTSSTGSPGASPGTSSTGSPGTPGS GTASSSPGSSTPSGATGSPGASPGTSST GSPGTPGSGTASSSPGSSTPSGATGSP GSSPSASTGTGPGSSPSASTGTGPGSST PSGATGSPGSSTPSGATGSPGASPGTS STGSPGASPGTSSTGSPGASPGTSSTGS PGTPGSGTASSSP | 288 | Residue totals: H: 0 E: 0<br>percent: H: 0.0 E: 0.0 | 92.71 |

(continued)

| SEQ NAME | Sequence | No. Residues | Chou-Fasman Calculation | GOR Calculation |
|---|---|---|---|---|
| AF504 | GASPGTSSTGSPGSSPSASTGTGPGSSP SASTGTGPGTPGSGTASSSPGSSTPSG ATGSPGSNPSASTGTGPGASPGTSSTG SPGTPGSGTASSSPGSSTPSGATGSPGT PGSGTASSSPGASPGTSSTGSPGASPG TSSTGSPGTPGSGTASSSPGSSTPSGAT GSPGASPGTSSTGSPGTPGSGTASSSP GSSTPSGATGSPGSNPSASTGTGPGSS PSASTGTGPGSSTPSGATGSPGSSTPSG ATGSPGASPGTSSTGSPGASPGTSSTG SPGASPGTSSTGSPGTPGSGTASSSPG ASPGTSSTGSPGASPGTSSTGSPGASP | 504 | Residue totals: H: 0 E: 0<br>percent: H: 0.0 E: 0.0 | 94.44% |
| | GTSSTGSPGSSPSASTGTGPGTPGSGT ASSSPGASPGTSSTGSPGASPGTSSTGS PGASPGTSSTGSPGSSTPSGATGSPGSS TPSGATGSPGASPGTSSTGSPGTPGSG TASSSPGSSTPSGATGSPGSSTPSGATG SPGSSTPSGATGSPGSSPSASTGTGPG ASPGTSSTGSP | | | |
| AD 576 | GSSESGSSEGGPGSGGEPSESGSSGSSE SGSSEGGPGSSESGSSEGGPGSSESGSS EGGPGSSESGSSEGGPGSSESGSSEGG PGESPGGSSGSESGSEGSSGPGESSGSS ESGSSEGGPGSSESGSSEGGPGSSESGS SEGGPGSGGEPSESGSSGESPGGSSGS ESGESPGGSSGSESGSGGEPSESGSSGS SESGSSEGGPGSGGEPSESGSSGSGGE PSESGSSGSEGSSGPGESSGESPGGSSG SESGSGGEPSESGSSGSGGEPSESGSSG SGGEPSESGSSGSSESGSSEGGPGESPG GSSGSESGESPGGSSGSESGESPGGSS GSESGESPGGSSGSESGESPGGSSGSES GSSESGSSEGGPGSGGEPSESGSSGSE GSSGPGESSGSSESGSSEGGPGSGGEP SESGSSGSSESGSSEGGPGSGGEPSESG SSGESPGGSSGSESGESPGGSSGSESGS SESGSSEGGPGSGGEPSESGSSGSSESG SSEGGPGSGGEPSESGSSGSGGEPSES GSSGESPGGSSGSESGSEGSSGPGESS GSSESGSSEGGPGSEGSSGPGESS | 576 | Residue totals: H: 7 E: 0<br>percent: H: 1.2 E: 0.0 | 99.65% |

(continued)

| SEQ NAME | Sequence | No. Residues | Chou-Fasman Calculation | GOR Calculation |
|---|---|---|---|---|
| AE576 | GSPAGSPTSTEEGTSESATPESGPGTST EPSEGSAPGSPAGSPTSTEEGTSTEPSE GSAPGTSTEPSEGSAPGTSESATPESGP GSEPATSGSETPGSEPATSGSETPGSPA GSPTSTEEGTSESATPESGPGTSTEPSE GSAPGTSTEPSEGSAPGSPAGSPTSTEE GTSTEPSEGSAPGTSTEPSEGSAPGTSE SATPESGPGTSTEPSEGSAPGTSESATP ESGPGSEPATSGSETPGTSTEPSEGSAP GTSTEPSEGSAPGTSESATPESGPGTSE SATPESGPGSPAGSPTSTEEGTSESATP ESGPGSEPATSGSETPGTSESATPESGP GTSTEPSEGSAPGTSTEPSEGSAPGTST EPSEGSAPGTSTEPSEGSAPGTSTEPSE GSAPGTSTEPSEGSAPGSPAGSPTSTEE GTSTEPSEGSAPGTSESATPESGPGSEP ATSGSETPGTSESATPESGPGSEPATS GSETPGTSESATPESGPGTSTEPSEGSA PGTSESATPESGPGSPAGSPTSTEEGSP AGSPTSTEEGSPAGSPTSTEEGTSESAT PESGPGTSTEPSEGSAP | 576 | Residue totals: H: 2 E: 0 percent: H: 0.4 E: 0.0 | 99.65% |
| AG576 | PGTPGSGTASSSPGSSTPSGATGSPGSS PSASTGTGPGSSPSASTGTGPGSSTPSG ATGSPGSSTPSGATGSPGASPGTSSTG SPGASPGTSSTGSPGASPGTSSTGSPGT PGSGTASSSPGASPGTSSTGSPGASPG TSSTGSPGASPGTSSTGSPGSSPSASTG TGPGTPGSGTASSSPGASPGTSSTGSP GASPGTSSTGSPGASPGTSSTGSPGSST PSGATGSPGSSTPSGATGSPGASPGTS | 576 | Residue totals: H: 0 E: 3 percent: H: 0.4 E: 0.5 | 99.31% |
|  | STGSPGTPGSGTASSSPGSSTPSGATGS PGSSTPSGATGSPGSSTPSGATGSPGSS PSASTGTGPGASPGTSSTGSPGASPGT SSTGSPGTPGSGTASSSPGASPGTSSTG SPGASPGTSSTGSPGASPGTSSTGSPG ASPGTSSTGSPGTPGSGTASSSPGSSTP SGATGSPGTPGSGTASSSPGSSTPSGA TGSPGTPGSGTASSSPGSSTPSGATGSP GSSTPSGATGSPGSSPSASTGTGPGSSP SASTGTGPGASPGTSSTGSPGTPGSGT ASSSPGSSTPSGATGSPGSSPSASTGTG PGSSPSASTGTGPGASPGTSSTGS |  |  |  |

(continued)

| SEQ NAME | Sequence | No. Residues | Chou-Fasman Calculation | GOR Calculation |
|---|---|---|---|---|
| AF540 | GSTSSTAESPGPGSTSSTAESPGPGSTS ESPSGTAPGSTSSTAESPGPGSTSSTAE SPGPGTSTPESGSASPGSTSESPSGTAP GTSPSGESSTAPGSTSESPSGTAPGSTS ESPSGTAPGTSPSGESSTAPGSTSESPS GTAPGSTSESPSGTAPGTSPSGESSTAP GSTSESPSGTAPGSTSESPSGTAPGSTS ESPSGTAPGTSTPESGSASPGSTSESPS GTAPGTSTPESGSASPGSTSSTAESPGP GSTSSTAESPGPGTSTPESGSASPGTST PESGSASPGSTSESPSGTAPGTSTPESG SASPGTSTPESGSASPGSTSESPSGTAP GSTSESPSGTAPGSTSESPSGTAPGSTS STAESPGPGTSTPESGSASPGTSTPESG SASPGSTSESPSGTAPGSTSESPSGTAP GTSTPESGSASPGSTSESPSGTAPGSTS ESPSGTAPGTSTPESGSASPGTSPSGES STAPGSTSSTAESPGPGTSPSGESSTAP GSTSSTAESPGPGTSTPESGSASPGSTS ESPSGTAP | 540 | Residue totals: H: 2 E: 0 percent: H: 0.4 E: 0.0 | 99.65 |
| AD836 | GSSESGSSEGGPGSSESGSSEGGPGESP GGSSGSESGSGGEPSESGSSGESPGGS SGSESGESPGGSSGSESGSSESGSSEGG PGSSESGSSEGGPGSSESGSSEGGPGES PGGSSGSESGESPGGSSGSESGESPGG SSGSESGSSESGSSEGGPGSSESGSSEG GPGSSESGSSEGGPGSSESGSSEGGPG SSESGSSEGGPGSSESGSSEGGPGSGG EPSESGSSGESPGGSSGSESGESPGGSS GSESGSGGEPSESGSSGSEGSSGPGESS GSSESGSSEGGPGSGGEPSESGSSGSE GSSGPGESSGSSESGSSEGGPGSGGEP SESGSSGESPGGSSGSESGSGGEPSESG SSGSGGEPSESGSSGSSESGSSEGGPGS GGEPSESGSSGSGGEPSESGSSGSEGSS GPGESSGESPGGSSGSESGSEGSSGPG ESSGSEGSSGPGESSGSGGEPSESGSSG SSESGSSEGGPGSSESGSSEGGPGESPG GSSGSESGSGGEPSESGSSGSEGSSGP GESSGESPGGSSGSESGSEGSSGPGSSE SGSSEGGPGSGGEPSESGSSGSEGSSG PGESSGSEGSSGPGESSGSEGSSGPGES SGSGGEPSESGSSGSGGEPSESGSSGES PGGSSGSESGESPGGSSGSESGSGGEP SESGSSGSEGSSGPGESSGESPGGSSGS ESGSSESGSSEGGPGSSESGSSEGGPGS | 836 | Residue totals: H: 0 E: 0 percent: H: 0.0 E: 0.0 | 98.44% |
| | SESGSSEGGPGSGGEPSESGSSGSSESG SSEGGPGESPGGSSGSESGSGGEPSES GSSGSSESGSSEGGPGESPGGSSGSES GSGGEPSESGSSGESPGGSSGSESGSG GEPSESGSS | | | |

(continued)

| SEQ NAME | Sequence | No. Residues | Chou-Fasman Calculation | GOR Calculation |
|---|---|---|---|---|
| AE864 | GSPAGSPTSTEEGTSESATPESGPGTST EPSEGSAPGSPAGSPTSTEEGTSTEPSE GSAPGTSTEPSEGSAPGTSESATPESGP GSEPATSGSETPGSEPATSGSETPGSPA GSPTSTEEGTSESATPESGPGTSTEPSE GSAPGTSTEPSEGSAPGSPAGSPTSTEE GTSTEPSEGSAPGTSTEPSEGSAPGTSE SATPESGPGTSTEPSEGSAPGTSESATP ESGPGSEPATSGSETPGTSTEPSEGSAP GTSTEPSEGSAPGTSESATPESGPGTSE SATPESGPGSPAGSPTSTEEGTSESATP ESGPGSEPATSGSETPGTSESATPESGP GTSTEPSEGSAPGTSTEPSEGSAPGTST EPSEGSAPGTSTEPSEGSAPGTSTEPSE GSAPGTSTEPSEGSAPGSPAGSPTSTEE GTSTEPSEGSAPGTSESATPESGPGSEP ATSGSETPGTSESATPESGPGSEPATS GSETPGTSESATPESGPGTSTEPSEGSA PGTSESATPESGPGSPAGSPTSTEEGSP AGSPTSTEEGSPAGSPTSTEEGTSESAT PESGPGTSTEPSEGSAPGTSESATPESG PGSEPATSGSETPGTSESATPESGPGSE PATSGSETPGTSESATPESGPGTSTEPS EGSAPGSPAGSPTSTEEGTSESATPES GPGSEPATSGSETPGTSESATPESGPGS PAGSPTSTEEGSPAGSPTSTEEGTSTEP SEGSAPGTSESATPESGPGTSESATPES GPGTSESATPESGPGSEPATSGSETPGS EPATSGSETPGSPAGSPTSTEEGTSTEP SEGSAPGTSTEPSEGSAPGSEPATSGSE TPGTSESATPESGPGTSTEPSEGSAP | 864 | Residue totals: H: 2 E: 3<br>percent: H: 0.2 E: 0.4 | 99.77% |
| AF864 | GSTSESPSGTAPGTSPSGESSTAPGSTS ESPSGTAPGSTSESPSGTAPGTSTPESG SASPGTSTPESGSASPGSTSESPSGTAP GSTSESPSGTAPGTSPSGESSTAPGSTS ESPSGTAPGTSPSGESSTAPGTSPSGES STAPGSTSSTAESPGPGTSPSGESSTAP GTSPSGESSTAPGSTSSTAESPGPGTST PESGSASPGTSTPESGSASPGSTSESPS GTAPGSTSESPSGTAPGTSTPESGSASP GSTSSTAESPGPGTSTPESGSASPGSTS ESPSGTAPGTSPSGESSTAPGSTSSTAE SPGPGTSPSGESSTAPGTSTPESGSASP GSTSSTAESPGPGSTSSTAESPGPGSTS STAESPGPGSTSSTAESPGPGTSPSGES STAPGSTSESPSGTAPGSTSESPSGTAP GTSTPESGPXXXGASASGAPSTXXXX SESPSGTAPGSTSESPSGTAPGSTSESP SGTAPGSTSESPSGTAPGSTSESPSGTA PGTSESPSGTAPGTSTPESGSASPGTS PSGESSTAPGTSPSGESSTAPGSTSSTA ESPGPGTSPSGESSTAPGTSTPESGSAS PGSTSESPSGTAPGSTSESPSGTAPGTS | 875 | Residue totals: H: 2 E: 0<br>percent: H: 0.2 E: 0.0 | 95.20% |

(continued)

| SEQ NAME | Sequence | No. Residues | Chou-Fasman Calculation | GOR Calculation |
|---|---|---|---|---|
| | PSGESSTAPGSTSESPSGTAPGTSTPES GSASPGTSTPESGSASPGSTSESPSGTA PGTSTPESGSASPGSTSSTAESPGPGST SESPSGTAPGSTSESPSGTAPGTSPSGE SSTAPGSTSSTAESPGPGTSPSGESSTA PGTSTPESGSASPGTSPSGESSTAPGTS PSGESSTAPGTSPSGESSTAPGSTSSTA ESPGPGSTSSTAESPGPGTSPSGESSTA PGSSPSASTGTGPGSSTPSGATGSPGSS TPSGATGSP | | | |
| AG864 | GASPGTSSTGSPGSSPSASTGTGPGSSP SASTGTGPGTPGSGTASSSPGSSTPSG ATGSPGSSPSASTGTGPGASPGTSSTG SPGTPGSGTASSSPGSSTPSGATGSPGT PGSGTASSSPGASPGTSSTGSPGASPG TSSTGSPGTPGSGTASSSPGSSTPSGAT GSPGASPGTSSTGSPGTPGSGTASSSP GSSTPSGATGSPGSSPSASTGTGPGSSP SASTGTGPGSSTPSGATGSPGSSTPSG ATGSPGASPGTSSTGSPGASPGTSSTG SPGASPGTSSTGSPGTPGSGTASSSPG ASPGTSSTGSPGASPGTSSTGSPGASP GTSSTGSPGSSPSASTGTGPGTPGSGT ASSSPGASPGTSSTGSPGASPGTSSTGS PGASPGTSSTGSPGSSTPSGATGSPGSS TPSGATGSPGASPGTSSTGSPGTPGSG TASSSPGSSTPSGATGSPGSSTPSGATG SPGSSTPSGATGSPGSSPSASTGTGPG ASPGTSSTGSPGASPGTSSTGSPGTPGS GTASSSPGASPGTSSTGSPGASPGTSST GSPGASPGTSSTGSPGASPGTSSTGSP GTPGSGTASSSPGSSTPSGATGSPGTP GSGTASSSPGSSTPSGATGSPGTPGSG TASSSPGSSTPSGATGSPGSSTPSGATG SPGSSPSASTGTGPGSSPSASTGTGPG ASPGTSSTGSPGTPGSGTASSSPGSSTP SGATGSPGSSPSASTGTGPGSSPSAST GTGPGASPGTSSTGSPGASPGTSSTGS PGSSTPSGATGSPGSSPSASTGTGPGA SPGTSSTGSPGSSPSASTGTGPGTPGSG TASSSPGSSTPSGATGSPGSSTPSGATG SPGASPGTSSTGSP | 864 | Residue totals: H: 0 E: 0<br>percent: H: 0.0 E: 0.0 | 94.91% |

(continued)

| SEQ NAME | Sequence | No. Residues | Chou-Fasman Calculation | GOR Calculation |
|---|---|---|---|---|
| AM875 | GTSTEPSEGSAPGSEPATSGSETPGSPA GSPTSTEEGSTSSTAESPGPGTSTPESG SASPGSTSESPSGTAPGSTSESPSGTAP GTSTPESGSASPGTSTPESGSASPGSEP ATSGSETPGTSESATPESGPGSPAGSPT STEEGTSTEPSEGSAPGTSESATPESGP GTSTEPSEGSAPGTSTEPSEGSAPGSPA GSPTSTEEGTSTEPSEGSAPGTSTEPSE GSAPGTSESATPESGPGTSESATPESGP GTSTEPSEGSAPGTSTEPSEGSAPGTSE SATPESGPGTSTEPSEGSAPGSEPATSG SETPGSPAGSPTSTEEGSSTPSGATGSP GTPGSGTASSSPGSSTPSGATGSPGTS TEPSEGSAPGTSTEPSEGSAPGSEPATS GSETPGSPAGSPTSTEEGSPAGSPTSTE EGTSTEPSEGSAPGASASGAPSTGGTS | 875 | Residue totals: H: 7 E: 3 percent: H: 0.8 E: 0.3 | 98.63% |
| | ESATPESGPGSPAGSPTSTEEGSPAGSP TSTEEGSTSSTAESPGPGSTSESPSGTA PGTSPSGESSTAPGTPGSGTASSSPGSS TPSGATGSPGSSPSASTGTGPGSEPAT SGSETPGTSESATPESGPGSEPATSGSE TPGSTSSTAESPGPGSTSSTAESPGPGT SPSGESSTAPGSEPATSGSETPGSEPAT SGSETPGTSTEPSEGSAPGSTSSTAESP GPGTSTPESGSASPGSTSESPSGTAPGT STEPSEGSAPGTSTEPSEGSAPGTSTEP SEGSAPGSSTPSGATGSPGSSPSASTGT GPGASPGTSSTGSPGSEPATSGSETPG TSESATPESGPGSPAGSPTSTEEGSSTP SGATGSPGSSPSASTGTGPGASPGTSS TGSPGTSESATPESGPGTSTEPSEGSAP GTSTEPSEGSAP | | | |

(continued)

| SEQ NAME | Sequence | No. Residues | Chou-Fasman Calculation | GOR Calculation |
|---|---|---|---|---|
| AM1318 | GTSTEPSEGSAPGSEPATSGSETPGSPA GSPTSTEEGSTSSTAESPGPGTSTPESG SASPGSTSESPSGTAPGSTSESPSGTAP GTSTPESGSASPGTSTPESGSASPGSEP ATSGSETPGTSESATPESGPGSPAGSPT STEEGTSTEPSEGSAPGTSESATPESGP GTSTEPSEGSAPGTSTEPSEGSAPGSPA GSPTSTEEGTSTEPSEGSAPGTSTEPSE GSAPGTSESATPESGPGTSESATPESGP GTSTEPSEGSAPGTSTEPSEGSAPGTSE SATPESGPGTSTEPSEGSAPGSEPATSG SETPGSPAGSPTSTEEGSSTPSGATGSP GTPGSGTASSSPGSSTPSGATGSPGTS TEPSEGSAPGTSTEPSEGSAPGSEPATS GSETPGSPAGSPTSTEEGSPAGSPTSTE EGTSTEPSEGSAPGPEPTGPAPSGGSEP ATSGSETPGTSESATPESGPGSPAGSPT STEEGTSESATPESGPGSPAGSPTSTEE GSPAGSPTSTEEGTSESATPESGPGSPA GSPTSTEEGSPAGSPTSTEEGSTSSTAE SPGPGTSESPSGTAPGTSPSGESSTAP GSTSESPSGTAPGSTSESPSGTAPGTSP SGESSTAPGTSTEPSEGSAPGTSESATP ESGPGTSESATPESGPGSEPATSGSETP GTSESATPESGPGTSESATPESGPGTST EPSEGSAPGTSESATPESGPGTSTEPSE GSAPGTSPSGESSTAPGTSPSGESSTAP GTSPSGESSTAPGTSTEPSEGSAPGSPA GSPTSTEEGTSTEPSEGSAPGSSPSAST GTGPGSSTPSGATGSPGSSTPSGATGS PGSSTPSGATGSPGSSTPSGATGSPGA SPGTSSTGSPGASASGAPSTGGTSPSG ESSTAPGSTSSTAESPGPGTSPSGESST APGTSESATPESGPGTSTEPSEGSAPG TSTEPSEGSAPGSSPSASTGTGPGSSTP SGATGSPGASPGTSSTGSPGTSTPESG SASPGTSPSGESSTAPGTSPSGESSTAP GTSESATPESGPGSEPATSGSETPGTST EPSEGSAPGSTSESPSGTAPGSTSESPS GTAPGTSTPESGSASPGSPAGSPTSTEE GTSESATPESGPGTSTEPSEGSAPGSPA GSPTSTEEGTSESATPESGPGSEPATSG | 1318 | Residue totals: H: 7 E: 0 percent: H: 0.7 E: 0.0 | 99.17% |
| | SETPGSSTPSGATGSPGASPGTSSTGSP GSSTPSGATGSPGSTSESPSGTAPGTSP SGESSTAPGSTSSTAESPGPGSSTPSGA TGSPGASPGTSSTGSPGTPGSGTASSSP GSPAGSPTSTEEGSPAGSPTSTEEGTST EPSEGSAP | | | |

(continued)

| SEQ NAME | Sequence | No. Residues | Chou-Fasman Calculation | GOR Calculation |
|---|---|---|---|---|
| AM923 | MAEPAGSPTSTEEGASPGTSSTGSPGS STPSGATGSPGSSTPSGATGSPGTSTEP SEGSAPGSEPATSGSETPGSPAGSPTST EEGSTSSTAESPGPGTSTPESGSASPGS TSESPSGTAPGSTSESPSGTAPGTSTPE SGSASPGTSTPESGSASPGSEPATSGSE TPGTSESATPESGPGSPAGSPTSTEEGT STEPSEGSAPGTSESATPESGPGTSTEP SEGSAPGTSTEPSEGSAPGSPAGSPTST EEGTSTEPSEGSAPGTSTEPSEGSAPGT SESATPESGPGTSESATPESGPGTSTEP SEGSAPGTSTEPSEGSAPGTSESATPES GPGTSTEPSEGSAPGSEPATSGSETPGS PAGSPTSTEEGSSTPSGATGSPGTPGS GTASSSPGSSTPSGATGSPGTSTEPSEG SAPGTSTEPSEGSAPGSEPATSGSETPG SPAGSPTSTEEGSPAGSPTSTEEGTSTE PSEGSAPGASASGAPSTGGTSESATPE SGPGSPAGSPTSTEEGSPAGSPTSTEEG STSSTAESPGPGTSESPSGTAPGTSPS GESSTAPGTPGSGTASSSPGSSTPSGA TGSPGSSPSASTGTGPGSEPATSGSETP GTSESATPESGPGSEPATSGSETPGSTS STAESPGPGTSSTAESPGPGTSPSGES STAPGSEPATSGSETPGSEPATSGSETP GTSTEPSEGSAPGSTSSTAESPGPGTST PESGSASPGSTSESPSGTAPGTSTEPSE GSAPGTSTEPSEGSAPGTSTEPSEGSAP GSSTPSGATGSPGSSPSASTGTGPGAS PGTSSTGSPGSEPATSGSETPGTSESAT PESGPGSPAGSPTSTEEGSSTPSGATGS PGSSPSASTGTGPGASPGTSSTGSPGTS ESATPESGPGTSTEPSEGSAPGTSTEPS EGSAP | 924 | Residue totals: H: 4 E: 3<br>percent: H: 0.4 E: 0.3 | 98.70% |
| AE912 | MAEPAGSPTSTEEGTPGSGTASSSPGS STPSGATGSPGASPGTSSTGSPGSPAG SPTSTEEGTSESATPESGPGTSTEPSEG SAPGSPAGSPTSTEEGTSTEPSEGSAPG TSTEPSEGSAPGTSESATPESGPGSEPA TSGSETPGSEPATSGSETPGSPAGSPTS TEEGTSESATPESGPGTSTEPSEGSAPG TSTEPSEGSAPGSPAGSPTSTEEGTSTE PSEGSAPGTSTEPSEGSAPGTSESATPE SGPGTSTEPSEGSAPGTSESATPESGPG SEPATSGSETPGTSTEPSEGSAPGTSTE PSEGSAPGTSESATPESGPGTSESATPE SGPGSPAGSPTSTEEGTSESATPESGPG SEPATSGSETPGTSESATPESGPGTSTE PSEGSAPGTSTEPSEGSAPGTSTEPSEG SAPGTSTEPSEGSAPGTSTEPSEGSAPG TSTEPSEGSAPGSPAGSPTSTEEGTSTE PSEGSAPGTSESATPESGPGSEPATSGS | 913 | Residue totals: H: 8 E: 3<br>percent: H: 0.9 E: 0.3 | 99.45% |

(continued)

| SEQ NAME | Sequence | No. Residues | Chou-Fasman Calculation | GOR Calculation |
|---|---|---|---|---|
|  | ETPGTSESATPESGPGSEPATSGSETPG TSESATPESGPGTSTEPSEGSAPGTSES ATPESGPGSPAGSPTSTEEGSPAGSPTS TEEGSPAGSPTSTEEGTSESATPESGPG TSTEPSEGSAPGTSESATPESGPGSEPA TSGSETPGTSESATPESGPGSEPATSGS ETPGTSESATPESGPGTSTEPSEGSAPG SPAGSPTSTEEGTSESATPESGPGSEPA TSGSETPGTSESATPESGPGSPAGSPTS TEEGSPAGSPTSTEEGTSTEPSEGSAPG TSESATPESGPGTSESATPESGPGTSES ATPESGPGSEPATSGSETPGSEPATSGS ETPGSPAGSPTSTEEGTSTEPSEGSAPG TSTEPSEGSAPGSEPATSGSETPGTSES ATPESGPGTSTEPSEGSAP |  |  |  |
| BC 864 | GTSTEPSEPGSAGTSTEPSEPGSAGSEP ATSGTEPSGSGASEPTSTEPGSEPATS GTEPSGSEPATSGTEPSGSEPATSGTEP SGSGASEPTSTEPGTSTEPSEPGSAGSE PATSGTEPSGTSTEPSEPGSAGSEPATS GTEPSGSEPATSGTEPSGTSTEPSEPGS AGTSTEPSEPGSAGSEPATSGTEPSGS EPATSGTEPSGTSEPSTSEPGAGSGAS EPTSTEPGTSEPSTSEPGAGSEPATSGT EPSGSEPATSGTEPSGTSTEPSEPGSAG TSTEPSEPGSAGSGASEPTSTEPGSEPA TSGTEPSGSEPATSGTEPSGSEPATSGT EPSGSEPATSGTEPSGTSTEPSEPGSAG SEPATSGTEPSGSGASEPTSTEPGTSTE PSEPGSAGSEPATSGTEPSGSGASEPTS TEPGTSTEPSEPGSAGSGASEPTSTEPG SEPATSGTEPSGSGASEPTSTEPGSEPA TSGTEPSGSGASEPTSTEPGTSTEPSEP GSAGSEPATSGTEPSGSGASEPTSTEP GTSTEPSEPGSAGSEPATSGTEPSGTST EPSEPGSAGSEPATSGTEPSGTSTEPSE PGSAGTSTEPSEPGSAGTSTEPSEPGSA GTSTEPSEPGSAGTSTEPSEPGSAGTST EPSEPGSAGTSEPSTSEPGAGSGASEPT STEPGTSTEPSEPGSAGTSTEPSEPGSA GTSTEPSEPGSAGSEPATSGTEPSGSG ASEPTSTEPGSEPATSGTEPSGSEPATS GTEPSGSEPATSGTEPSGSEPATSGTEP SGTSEPSTSEPGAGSEPATSGTEPSGSG ASEPTSTEPGTSTEPSEPGSAGSEPATS GTEPSGSGASEPTSTEPGTSTEPSEPGS A | | Residue totals: H: 0 E: 0<br>percent: H: 0 E: 0 | 99.77% |
| * H: alpha-helix E: beta-sheet | | | | |

### Example 30: Analysis of polypeptide sequences for repetitiveness

In this Example, different polypeptides, including several XTEN sequences, were assessed for repetitiveness in the amino acid sequence. Polypeptide amino acid sequences can be assessed for repetitiveness by quantifying the number of times a shorter subsequence appears within the overall polypeptide. For example, a polypeptide of 200 amino acid residues length has a total of 165 overlapping 36-amino acid "blocks" (or "36-mers") and 198 3-mer "subsequences", but the number of unique 3-mer subsequences will depend on the amount of repetitiveness within the sequence. For the analyses, different polypeptide sequences were assessed for repetitiveness by determining the subsequence score obtained by application of the following equation:

$$\text{Subsequence score} = \frac{\sum_{i=1}^{m} Count_i}{m} \qquad \text{I}$$

wherein: m = (amino acid length of polypeptide) - (amino acid length of subsequence) + 1; and $Count_i$ = cumulative number of occurrences of each unique subsequence within sequence$_i$

In the analyses of the present Example, the subsequence score for the polypeptides of Table 26 were determined using the foregoing equation in a computer program using the algorithm depicted in FIG. 1, wherein the subsequence length was set at 3 amino acids. The resulting subsequence score is a reflection of the degree of repetitiveness within the polypeptide.

The results, shown in Table 26, indicate that the unstructured polypeptides consisting of 2 or 3 amino acid types have high subsequence scores, while those of consisting of the 12 amino acid motifs of the six amino acids G, S, T, E, P, and A with a low degree of internal repetitiveness, have subsequence scores of less than 10, and in some cases, less than 5. For example, the L288 sequence has two amino acid types and has short, highly repetitive sequences, resulting in a subsequence score of 50.0. The polypeptide J288 has three amino acid types but also has short, repetitive sequences, resulting in a subsequence score of 33.3. Y576 also has three amino acid types, but is not made of internal repeats, reflected in the subsequence score of 15.7 over the first 200 amino acids. W576 consists of four types of amino acids, but has a higher degree of internal repetitiveness, e.g., "GGSG", resulting in a subsequence score of 23.4. The AD576 consists of four types of 12 amino acid motifs, each consisting of four types of amino acids. Because of the low degree of internal repetitiveness of the individual motifs, the overall subsequence score over the first 200 amino acids is 13.6. In contrast, XTEN's consisting of four motifs contains six types of amino acids, each with a low degree of internal repetitiveness have lower subsequence scores; i.e., AE864 (6.1), AF864 (7.5), and AM875 (4.5), while XTEN consisting of four motifs containing five types of amino acids were intermediate; i.e., AE864, with a score of 7.2.

Conclusions: The results indicate that the combination of 12 amino acid subsequence motifs, each consisting of four to six amino acid types that are non-repetitive, into a longer XTEN polypeptide results in an overall sequence that is substantially non-repetitive, as indicated by overall average subsequence scores less than 10 and, in many cases, less than 5. This is despite the fact that each subsequence motif may be used multiple times across the sequence. In contrast, polymers created from smaller numbers of amino acid types resulted in higher average subsequence scores, with polypeptides consisting of two amino acid type having higher scores that those consisting of three amino acid types.

**Table 26: Average subsequence score calculations of polypeptide sequences**

| Seq Name | SEQ ID NO: | Amino Acid Sequence | Score |
|---|---|---|---|
| J288 | 783 | GSGGEGGSGGEGGSGGEGGSGGEGGSGGEGGSGGEGGSGGEGGSGGEG GSGGEGGSGGEGGSGGEGGSGGEGGSGGEGGSGGEGGSGGEGGSGGEG GSGGEGGSGGEGGSGGEGGSGGEGGSGGEGGSGGEGGSGGEGGSGGEG GSGGEGGSGGEGGSGGEGGSGGEGGSGGEGGSGGEGGSGGEGGSGGEG GSGGEGGSGGEGGSGGEGGSGGEGGSGGEGGSGGEGGSGGEGGSGGEG GSGGEGGSGGEGGSGGEGGSGGEGGSGGEGGSGGEGGSGGEGGSGGEG | 33.3 |
| K288 | 784 | GEGEGGGEGGEGEGGGEGGEGEGGGEGGEGEGGGEGGEGEGGGEGGE GEGGGEGGEGEGGGEGGEGEGGGEGGEGEGGGEGGEGEGGGEGGEGE GGGEGGEGEGGGEGGEGEGGGEGGEGEGGGEGGEGEGGGEGGEGEGG GEGGEGEGGGEGGEGEGGGEGGEGEGGGEGGEGEGGGEGGEGEGGGE GGEGEGGGEGGEGEGGGEGGEGEGGGEGGEGEGGGEGGEGEGGGEGG EGEGGGEGGEGEGGGEGGEGEGGGEGGEGEGGGEGGEGEGGGEGGEG EGGGEG | 46.9 |

(continued)

| Seq Name | SEQ ID NO: | Amino Acid Sequence | Score |
|---|---|---|---|
| L288 | 785 | SSESSESSSSESSSESSSESSSESSSESSESSSSESSSESSESSSSESSSESSESSS SESSSESSSSESSSESSSESSSESSSESSESSSSESSSESSESSSSESSSESSE SSSSESSSESSESSSSESSSESSESSSSESSSESSESSSSESSSESSESSSSESSSE SSESSSSESSSESSESSSSESSSESSESSSSESSSESSESSSSESSSESSSSES SSESSESSSSESSSESSSSESSSESSESSSSESSSESSESSSSESSSESSESSS SES | 50.0 |
| Y288 | 786 | GEGSGEGSEGEGSEGSGEGEGSEGSGEGEGGSEGSEGEGGSEGSEGEGGS EGSEGEGSGEGSEGEGGSEGSEGEGSGEGSEGEGSEGGSEGEGGSEGSEG EGSGEGSEGEGGEGGSEGEGSEGSGEGEGSEGSEGEGSEGSGEGEGSGE GSEGEGSEGSGEGEGSEGSGEGEGGSEGSEGEGSEGSGEGEGGEGSGEGE GSGEGSEGEGGGEGSEGEGSEGGGEGEGSEGGSEGEGGSEGGEGEGSEG SGEGEGSEGGSEGEGSEGGSEGEGSEGSGEGEGSEGSGE | 26.8 |
| Q576 | 787 | GGKPGEGGKPEGGGGKPGGKPEGEGEGKPGGKPEGGGKPGGGEGGKPE GGKPEGEGKPGGGEGKPGGKPEGGGGKPEGEGKPGGGGGKPGGKPEGE GKPGGGEGGKPEGKPGEGGEGKPGGKPEGGGEGKPGGGKPGEGGKPGE GKPGGGEGGKPEGGKPEGEGKPGGGEGKPGGKPGEGGKPEGGGEGKPG GKPGEGGEGKPGGGKPEGEGKPGGGKPGGGEGGKPEGEGKPGGKPEGG GEGKPGGKPEGGGKPEGGGEGKPGGGKPGEGGKPGEGEGKPGGKPEGE GKPGGEGGGKPEGKPGGGEGGKPEGGKPGEGGKPEGGKPGEGGEGKPG GGKPGEGGKPEGGGKPEGEGKPGGGGKPGEGGKPEGGKPEGGGEGKPG GGKPEGEGKPGGGEGKPGGKPEGGGKPGEGGKPEGGKPGGEGGGKPE GEGKPGGKPGEGGGGKPGGKPEGEGKPGEGGEGKPGGKPEGGGEGKPG GKPEGGGEGKPGGGKPGEGGKPEGGKPGEGGKPGEGGKPEGEGKPGG GEGKPGGKPGEGGKPEGGGEGKPGGKPGGEGGGKPEGGKPGEGGKPEG | 18.5 |
| U576 | 788 | GEGKPGGKPGSGGGKPGEGGKPGSGEGKPGGKPGSGGSGKPGGKPGEG GKPEGGSGGKPGGGGKPGGKPGGEGSGKPGGKPEGGGKPEGGSGGKPG GKPEGGSGGKPGGKPGSGEGGKPGGGKPGGEGKPGSGKPGGEGSGKPG GKPEGGSGGKPGGKPEGGSGGKPGGSGKPGGKPGEGGKPEGGSGGKPG GSGKPGGKPEGGSGKPGGKPGEGGKPGSGEGGKPGGGKPGGEGKPGS GKPGGEGSGKPGGKPGSGGEGKPGGKPEGGSGGKPGGGKPGGEGKPGS GGKPGEGGKPGSGGGKPGGKPGGEGEGKPGGKPGEGGKPGGEGSGKPG GGGKPGGKPGGEGGKPEGSGKPGGGSGKPGGKPEGGGGKPEGSGKPGG GGKPEGSGKPGGGKPEGGSGGKPGGSGKPGGKPGEGGGKPEGSGKPGG GSGKPGGKPEGGGKPEGGSGGKPGGKPEGGSGGKPGGKPGGEGSGKPG GKPGSGEGGKPGGKPGEGSGGKPGGKPEGGSGGKPGGSGKPGGKPEGG GSGKPGGKPGEGGKPGGEGSGKPGGSGKPG | 18.1 |
| W576 | 789 | GGSGKPGKPGGSGSGKPGSGKPGGGSGKPGSGKPGGGSGKPGSGKPGG GSGKPGSGKPGGGGKPGSGSGKPGGGKPGGSGGKPGGGSGKPGKPGSG GSGGKPGKPGSGGSGGKPGKPGSGGSGKPGSGKPGGGSGKPGSGKPGSG GSGGKPGKPGSGGSGKPGSGKPGSGSGKPGSGKPGGGSGKPGSGKPGSGG SGKPGKPGSGGGKPGSGSGKPGGGKPGSGSGKPGGGKPGGSGGKPGGS GGKPGKPGSGGGSGKPGKPGSGGGSGKPGKPGGSGSGKPGSGKPGGGS | 23.4 |
| | | GKPGSGKPGSGGSGKPGKPGSGGSGGKPGKPGSGGGKPGSGSGKPGGG KPGSGSGKPGGGKPGSGSGKPGGSGKPGSGKPGGGSG GKPGKPGSGGSGKPGSGKPGSGGSGKPGKPGGSGSGKPGSGKPGGGSGK PGSGKPGGGSGKPGSGKPGGGGKPGSGSGKPGGSGG KPGKPGSGGSGGKPGKPGSGGSGKPGSGKPGGGSGGKPGKPGSGG | |

(continued)

| Seq Name | SEQ ID NO: | Amino Acid Sequence | Score |
|---|---|---|---|
| Y576 | 790 | GEGSGEGSEGEGSEGSGEGEGSEGSGEGEGGSEGSEGEGSEGSGEGEGGE GSGEGEGSGEGSEGEGGGEGSEGEGSGEGGEGEGSEGGSEGEGGSEGGE GEGSEGSGEGEGSEGGSEGEGSEGGSEGEGSEGSGEGEGSEGSGEGEGSE GSGEGEGSEGSGEGEGSEGGSEGEGGSEGSEGEGSGEGSEGEGGSEGSEG EGGGEGSEGEGSGEGSEGEGGSEGSEGEGGSEGSEGEGGEGSGEGEGSE GSGEGEGSGEGSEGEGSEGSGEGEGSEGSGEGEGGSEGSEGEGSGEGSEG EGSEGSGEGEGGSEGSEGEGGSEGSEGEGGSEGSEGEGGSEGSEGEGGEG SGEGEGSEGSGEGEGSGEGSEGEGSEGSGEGEGSEGSGEGEGGSEGSEGE GSEGSGEGEGGEGSGEGEGSGEGSEGEGGGEGSEGEGSEGSGEGEGSEGS GEGEGSEGGSEGEGGSEGSEGEGSEGGSEGEGSEGGSEGEGSEGSGEGEG SEGSGEGEGSEGSGEGEGSEGSGEGEGSGEGSEGEGGSEGGEGEGSEGGSEGEGSEGGSEGEGGEGSG EGEGGGEGSEGEGSEGSGEGEGSGEGSE | 15.7 |
| AE288 | 288 | GTSESATPESGPGSEPATSGSETPGTSESATPESGPGSEPATSGSETPGTSES ATPESGPGTSTEPSEGSAPGSPAGSPTSTEEGTSESATPESGPGSEPATSGS ETPGTSESATPESGPGSPAGSPTSTEEGSPAGSPTSTEEGTSTEPSEGSAPG TSESATPESGPGTSESATPESGPGTSESATPESGPGSEPATSGSETPGSEPAT SGSETPGSPAGSPTSTEEGTSTEPSEGSAPGTSTEPSEGSAPGSEPATSGSET PGTSESATPESGPGTSTEPSEGSAP | 6.0 |
| AG288_1 | 288 | PGASPGTSSTGSPGASPGTSSTGSPGTPGSGTASSSPGSSTPSGATGSPGTP GSGTASSSPGSSTPSGATGSPGTPGSGTASSSPGSSTPSGATGSPGSSTPSG ATGSPGSSPSASTGTGPGSSPSASTGTGPGASPGTSSTGSPGTPGSGTASSS PGSSTPSGATGSPGSSPSASTGTGPGSSPSASTGTGPGASPGTSSTGSPGAS PGTSSTGSPGSSTPSGATGSPGSSPSASTGTGPGASPGTSSTGSPGSSPSAST GTGPGTPGSGTASSSPGSSTPSGATGS | 6.9 |
| AD576 | 791 | GSSESGSSEGGPGSGGEPSESGSSGSSESGSSEGGPGSSESGSSEGGPGSSE SGSSEGGPGSSESGSSEGGPGSSESGSSEGGPGESPGGSSGSESGSEGSSGP GESSGSSESGSSEGGPGSSESGSSEGGPGSSESGSSEGGPGSGGEPSESGSS GESPGGSSGSESGESPGGSSGSESGSGGEPSESGSSGSSESGSSEGGPGSGG EPSESGSSGSGGEPSESGSSGSEGSSGPGESSGESPGGSSGSESGSGGEPSE SGSSGSGGEPSESGSSGSGGEPSESGSSGSSESGSSEGGPGESPGGSSGSES GESPGGSSGSESGESPGGSSGSESGESPGGSSGSESGESPGGSSGSESGSSE SGSSEGGPGSGGEPSESGSSGSEGSSGPGESSGSSESGSSEGGPGSGGEPSE SGSSGSSESGSSEGGPGSGGEPSESGSSGESPGGSSGSESGESPGGSSGSES GSSESGSSEGGPGSGGEPSESGSSGSSESGSSEGGPGSGGEPSESGSSGSGG EPSESGSSGESPGGSSGSESGSEGSSGPGESSGSSESGSSEGGPGSEGSSGP GESS | 13.6 |
| AE576 | 792 | AGSPAGSPTSTEEGTSESATPESGPGTSTEPSEGSAPGSPAGSPTSTEEGTS TEPSEGSAPGTSTEPSEGSAPGTSESATPESGPGSEPATSGSETPGSEPATS GSETPGSPAGSPTSTEEGTSESATPESGPGTSTEPSEGSAPGTSTEPSEGSAP GSPAGSPTSTEEGTSTEPSEGSAPGTSTEPSEGSAPGTSESATPESGPGTST EPSEGSAPGTSESATPESGPGSEPATSGSETPGTSTEPSEGSAPGTSTEPSEG SAPGTSESATPESGPGTSESATPESGPGSPAGSPTSTEEGTSESATPESGPG SEPATSGSETPGTSESATPESGPGTSTEPSEGSAPGTSTEPSEGSAPGTSTEP SEGSAPGTSTEPSEGSAPGTSTEPSEGSAPGSPAGSPTST EEGTSTEPSEGSAPGTSESATPESGPGSEPATSGSETPGTSESATPESGPGS EPATSGSETPGTSESATPESGPGTSTEPSEGSAPGTSESATPESGPGSPAGS PTSTEEGSPAGSPTSTEEGSPAGSPTSTEEGTSESATPESGPGTSTEPSEGSA P | 6.1 |

(continued)

| Seq Name | SEQ ID NO: | Amino Acid Sequence | Score |
|---|---|---|---|
| AF540 | 793 | GSTSSTAESPGPGSTSSTAESPGPGSTSESPSGTAPGSTSSTAESPGPGSTSS TAESPGPGTSTPESGSASPGSTSESPSGTAPGTSPSGESSTAPGSTSESPSGT APGSTSESPSGTAPGTSPSGESSTAPGSTSESPSGTAPGSTSESPSGTAPGTS PSGESSTAPGSTSESPSGTAPGSTSESPSGTAPGSTSESPSGTAPGTSTPESG SASPGSTSESPSGTAPGTSTPESGSASPGSTSSTAESPGPGSTSSTAESPGPG | 8.8 |
|  |  | TSTPESGSASPGTSTPESGSASPGSTSESPSGTAPGTSTPESGSASPGTSTPE SGSASPGSTSESPSGTAPGSTSESPSGTAPGSTSESPSGTAPGSTSSTAESPG PGTSTPESGSASPGTSTPESGSASPGSTSESPSGTAPGSTSESPSGTAPGTST PESGSASPGSTSESPSGTAPGSTSESPSGTAPGTSTPESGSASPGTSPSGESS TAPGSTSSTAESPGPGTSPSGESSTAPGSTSSTAESPGPGTSTPESGSASPGS TSESPSGTAP |  |
| AF504 | 794 | GASPGTSSTGSPGSSPSASTGTGPGSSPSASTGTGPGTPGSGTASSSPGSST PSGATGSPGSNPSASTGTGPGASPGTSSTGSPGTPGSGTASSSPGSSTPSGA TGSPGTPGSGTASSSPGASPGTSSTGSPGASPGTSSTGSPGTPGSGTASSSP GSSTPSGATGSPGASPGTSSTGSPGTPGSGTASSSPGSSTPSGATGSPGSNP SASTGTGPGSSPSASTGTGPGSSTPSGATGSPGSSTPSGATGSPGASPGTSS TGSPGASPGTSSTGSPGASPGTSSTGSPGTPGSGTASSSPGASPGTSSTGSP GASPGTSSTGSPGASPGTSSTGSPGSSPSASTGTGPGTPGSGTASSSPGASP GTSSTGSPGASPGTSSTGSPGASPGTSSTGSPGSSTPSGATGSPGSSTPSGA TGSPGASPGTSSTGSPGTPGSGTASSSPGSSTPSGATGSPGSSTPSGATGSP GSSTPSGATGSPGSSPSASTGTGPGASPGTSSTGSP | 7.0 |
| AE864 | 795 | GSPAGSPTSTEEGTSESATPESGPGTSTEPSEGSAPGSPAGSPTSTEEGTST EPSEGSAPGTSTEPSEGSAPGTSESATPESGPGSEPATSGSETPGSEPATSG SETPGSPAGSPTSTEEGTSESATPESGPGTSTEPSEGSAPGTSTEPSEGSAPG SPAGSPTSTEEGTSTEPSEGSAPGTSTEPSEGSAPGTSESATPESGPGTSTEP SEGSAPGTSESATPESGPGSEPATSGSETPGTSTEPSEGSAPGTSTEPSEGS APGTSESATPESGPGTSESATPESGPGSPAGSPTSTEEGTSESATPESGPGS EPATSGSETPGTSESATPESGPGTSTEPSEGSAPGTSTEPSEGSAPGTSTEPS EGSAPGTSTEPSEGSAPGTSTEPSEGSAPGTSTEPSEGSAPGSPAGSPTSTE EGTSTEPSEGSAPGTSESATPESGPGSEPATSGSETPGTSESATPESGPGSEP ATSGSETPGTSESATPESGPGTSTEPSEGSAPGTSESATPESGPGSPAGSPT STEEGSPAGSPTSTEEGSPAGSPTSTEEGTSESATPESGPGTSTEPSEGSAP GTSESATPESGPGSEPATSGSETPGTSESATPESGPGSEPATSGSETPGTSES ATPESGPGTSTEPSEGSAPGSPAGSPTSTEEGTSESATPESGPGSEPATSGS ETPGTSESATPESGPGSPAGSPTSTEEGSPAGSPTSTEEGTSTEPSEGSAPG TSESATPESGPGTSESATPESGPGTSESATPESGPGSEPATSGSETPGSEPAT SGSETPGSPAGSPTSTEEGTSTEPSEGSAP | 6.1 |

(continued)

| Seq Name | SEQ ID NO: | Amino Acid Sequence | Score |
|---|---|---|---|
| AF864 | 796 | GSTSESPSGTAPGTSPSGESSTAPGSTSESPSGTAPGSTSESPSGTAPGTSTP ESGSASPGTSTPESGSASPGTSESPSGTAPGSTSESPSGTAPGTSPSGESST APGSTSESPSGTAPGTSPSGESSTAPGTSPSGESSTAPGSTSSTAESPGPGTS PSGESSTAPGTSPSGESSTAPGSTSSTAESPGPGTSTPESGSASPGTSTPESG SASPGSTSESPSGTAPGTSESPSGTAPGTSTPESGSASPGSTSSTAESPGPG TSTPESGSASPGSTSESPSGTAPGTSPSGESSTAPGSTSSTAESPGPGTSPSG ESSTAPGTSTPESGSASPGSTSSTAESPGPGSTSSTAESPGPGSTSSTAESPG PGSTSSTAESPGPGTSPSGESSTAPGSTSESPSGTAPGSTSESPSGTAPGTST PESGPXXXGASASGAPSTXXXXSESPSGTAPGSTSESPSGTAPGSTSESPS GTAPGSTSESPSGTAPGSTSESPSGTAPGSTSESPSGTAPGTSTPESGSASP GTSPSGESSTAPGTSPSGESSTAPGSTSSTAESPGPGTSPSGESSTAPGTSTP ESGSASPGTSESPSGTAPGTSESPSGTAPGTSPSGESSTAPGSTSESPSGT APGTSTPESGSASPGTSTPESGSASPGTSESPSGTAPGTSTPESGSASPGST SSTAESPGPGTSESPSGTAPGTSESPSGTAPGTSPSGESSTAPGSTSSTAE SPGPGTSPSGESSTAPGTSTPESGSASPGTSPSGESSTAPGTSPSGESSTAPG TSPSGESSTAPGSTSSTAESPGPGTSSTAESPGPGTSPSGESSTAPGSSPSA STGTGPGSSTPSGATGSPGSSTPSGATGSP | 7.5 |
| AG864 | 864 | GASPGTSSTGSPGSSPSASTGTGPGSSPSASTGTGPGTPGSGTASSSPGSST PSGATGSPGSSPSASTGTGPGASPGTSSTGSPGTPGSGTASSSPGSSTPSGA TGSPGTPGSGTASSSPGASPGTSSTGSPGASPGTSSTGSPGTPGSGTASSSP GSSTPSGATGSPGASPGTSSTGSPGTPGSGTASSSPGSSTPSGATGSPGSSP SASTGTGPGSSPSASTGTGPGSSTPSGATGSPGSSTPSGATGSPGASPGTSS TGSPGASPGTSSTGSPGASPGTSSTGSPGTPGSGTASSSPGASPGTSSTGSP GASPGTSSTGSPGASPGTSSTGSPGSSPSASTGTGPGTPGSGTASSSPGASP GTSSTGSPGASPGTSSTGSPGASPGTSSTGSPGSSTPSGATGSPGSSTPSGA TGSPGASPGTSSTGSPGTPGSGTASSSPGSSTPSGATGSPGSSTPSGATGSP GSSTPSGATGSPGSSPSASTGTGPGASPGTSSTGSPGASPGTSSTGSPGTPG | 7.2 |
| | | SGTASSSPGASPGTSSTGSPGASPGTSSTGSPGASPGTSSTGSPGASPGTSS TGSPGTPGSGTASSSPGSSTPSGATGSPGTPGSGTASSSPGSSTPSGATGSP GTPGSGTASSSPGSSTPSGATGSPGSSTPSGATGSPGSSPSASTGTGPGSSP SASTGTGPGASPGTSSTGSPGTPGSGTASSSPGSSTPSGATGSPGSSPSAST GTGPGSSPSASTGTGPGASPGTSSTGSPGASPGTSSTGSPGSSTPSGATGSP GSSPSASTGTGPGASPGTSSTGSPGSSPSASTGTGPGTPGSGTASSSPGSST PSGATGSPGSSTPSGATGSPGASPGTSSTGSP | |
| AG868 | 797 | GGSPGASPGTSSTGSPGSSPSASTGTGPGSSPSASTGTGPGTPGSGTASSSP GSSTPSGATGSPGSNPSASTGTGPGASPGTSSTGSPGTPGSGTASSSPGSST PSGATGSPGTPGSGTASSSPGASPGTSSTGSPGASPGTSSTGSPGTPGSGTA SSSPGSSTPSGATGSPGASPGTSSTGSPGTPGSGTASSSPGSSTPSGATGSP GSNPSASTGTGPGSSPSASTGTGPGSSTPSGATGSPGSSTPSGATGSPGASP GTSSTGSPGASPGTSSTGSPGASPGTSSTGSPGTPGSGTASSSPGASPGTSS TGSPGASPGTSSTGSPGASPGTSSTGSPGSSPSASTGTGPGTPGSGTASSSP GASPGTSSTGSPGASPGTSSTGSPGASPGTSSTGSPGSSTPSGATGSPGSST PSGATGSPGASPGTSSTGSPGTPGSGTASSSPGSSTPSGATGSPGSSTPSGA TGSPGSSTPSGATGSPGSSPSASTGTGPGASPGTSSTGSPGASPGTSSTGSP GTPGSGTASSSPGASPGTSSTGSPGASPGTSSTGSPGASPGTSSTGSPGASP GTSSTGSPGTPGSGTASSSPGSSTPSGATGSPGTPGSGTASSSPGSSTPSGA TGSPGTPGSGTASSSPGSSTPSGATGSPGSSTPSGATGSPGSSPSASTGTGP GSSPSASTGTGPGASPGTSSTGSPGTPGSGTASSSPGSSTPSGATGSPGSSP SASTGTGPGSSPSASTGTGPGASPGTSSTGSPGASPGTSSTGSPGSSTPSGA TGSPGSSPSASTGTGPGASPGTSSTGSPGSSPSASTGTGPGTPGSGTASSSP GSSTPSGATGSPGSSTPSGATGSPGASPGTSSTGSP | 7.5 |

(continued)

| Seq Name | SEQ ID NO: | Amino Acid Sequence | Score |
|---|---|---|---|
| AM875 | 798 | GTSTEPSEGSAPGSEPATSGSETPGSPAGSPTSTEEGSTSSTAESPGPGTSTPESGSASPGSTSESPSGTAPGSTSESPSGTAPGTSTPESGSASPGTSTPESGSASPGSEPATSGSETPGTSESATPESGPGSPAGSPTSTEEGTSTEPSEGSAPGTSESATPESGPGTSTEPSEGSAPGTSTEPSEGSAPGSPAGSPTSTEEGTSTEPSEGSAPGTSTEPSEGSAPGTSESATPESGPGTSESATPESGPGTSTEPSEGSAPGTSTEPSEGSAPGTSESATPESGPGTSTEPSEGSAPGSEPATSGSETPGSPAGSPTSTEEGSSTPSGATGSPGTPGSGTASSSPGSSTPSGATGSPGTSTEPSEGSAPGTSTEPSEGSAPGSEPATSGSETPGSPAGSPTSTEEGSPAGSPTSTEEGTSTEPSEGSAPGASASGAPSTGGTSESATPESGPGSPAGSPTSTEEGSPAGSPTSTEEGSTSSTAESPGPGTSSESPSGTAPGTSPSGESSTAPGTPGSGTASSSPGSSTPSGATGSPGSSPSASTGTGPGSEPATSGSETPGTSESATPESGPGSEPATSGSETPGTSSTAESPGPGTSSTAESPGPGTSPSGESSTAPGSEPATSGSETPGSEPATSGSETPGTSTEPSEGSAPGSTSSTAESPGPGTSTPESGSASPGSTSESPSGTAPGTSTEPSEGSAPGTSTEPSEGSAPGTSTEPSEGSAPGSSTPSGATGSPGSSPSASTGTGPGASPGTSSTGSPGSEPATSGSETPGTSESATPESGPGSPAGSPTSTEEGSSTPSGATGSPGSSPSASTGTGPGASPGTSSTGSPGTSESATPESGPGTSTEPSEGSAPGTSTEPSEGSAP | 4.5 |
| AE912 | 913 | MAEPAGSPTSTEEGTPGSGTASSSPGSSTPSGATGSPGASPGTSSTGSPGSPAGSPTSTEEGTSESATPESGPGTSTEPSEGSAPGSPAGSPTSTEEGTSTEPSEGSAPGTSTEPSEGSAPGTSESATPESGPGSEPATSGSETPGSEPATSGSETPGSPAGSPTSTEEGTSESATPESGPGTSTEPSEGSAPGTSTEPSEGSAPGSPAGSPTSTEEGTSTEPSEGSAPGTSTEPSEGSAPGTSESATPESGPGTSTEPSEGSAPGTSESATPESGPGSEPATSGSETPGTSTEPSEGSAPGTSTEPSEGSAPGTSESATPESGPGTSESATPESGPGSPAGSPTSTEEGTSESATPESGPGSEPATSGSETPGTSESATPESGPGTSTEPSEGSAPGTSTEPSEGSAPGTSTEPSEGSAPGTSTEPSEGSAPGTSTEPSEGSAPGSPAGSPTSTEEGTSTEPSEGSAPGTSESATPESGPGSEPATSGSETPGTSESATPESGPGSEPATSGSETPGTSESATPESGPGTSTEPSEGSAPGTSESATPESGPGSPAGSPTSTEEGSPAGSPTSTEEGSPAGSPTSTEEGTSESATPESGPGTSTEPSEGSAPGTSESATPESGPGSEPATSGSETPGTSESATPESGPGSEPATSGSETPGTSESATPESGPGTSTEPSEGSAPGSPAGSPTSTEEGTSESATPESGPGSEPATSGSETPGTSESATPESGPGSPAGSPTSTEEGSPAGSPTSTEEGTSTEPSEGSAPGTSESATPESGPGTSESATPESGPGTSESATPESGPGSEPATSGSETPGSEPATSGSETPGSPAGSPTSTEEGTSTEPSEGSAPGTSTEPSEGSAPGSEPATSGSETPGTSESATPESGPGTSTEPSEGSAP | 4.5 |
| AM923 | 924 | MAEPAGSPTSTEEGASPGTSSTGSPGSSTPSGATGSPGSSTPSGATGSPGTSTEPSEGSAPGSEPATSGSETPGSPAGSPTSTEEGTSSTAESPGPGTSTPESGSASPGSTSESPSGTAPGSTSESPSGTAPGTSTPESGSASPGTSTPESGSASPGSEPATSGSETPGTSESATPESGPGSPAGSPTSTEEGTSTEPSEGSAPGTSESATPESGPGTSTEPSEGSAPGTSTEPSEGSAPGSPAGSPTSTEEGTSTEPSEGSAPGTSTEPSEGSAPGTSESATPESGPGTSESATPESGPGTSTEPSEGSAPGTSTEPSEGSAPGTSESATPESGPGTSTEPSEGSAPGSEPATSGSETPGSPAGSPTSTEEGSSTPSGATGSPGTPGSGTASSSPGSSTPSGATGSPGTSTEPSEGSAPGTSTEPSEGSAPGSEPATSGSETPGSPAGSPTSTEEGSPAGSPTSTEEGTSTEPSEGSAPGASASGAPSTGGTSESATPESGPGSPAGSPTSTEEGSPAGSPTSTEEGSTSSTAESPGPGTSSESPSGTAPGTSPSGESSTAPGTPGSGTASSSPGSSTPSGATGSPGSSPSASTGTGPGSEPATSGSETPGTSESATPESGPGSEPATSGSETPGTSSTAESPGPGTSSTAESPGPGTSPSGESSTAPGSEPATSGSETPGSEPATSGSETPGTSTEPSEGSAPGTSSTAESPGPGTSTPESGSASPGSTSESPSGTAPGTSTEPSEGSAPGTSTEPSEGSAPGTSTEPSEGSAPGSSTPSGATGSPGSSPSASTGTGPGASPGTSSTGSPGSEPATSGSETPGTSESATPESGPGSPAGSPTSTEEGSSTPSGATGSPGSSPSASTGTGPGASPGTSSTGSPGTSESATPESGPGTSTEPSEGSAPGTSTEPSEGSAP | 4.5 |

(continued)

| Seq Name | SEQ ID NO: | Amino Acid Sequence | Score |
|---|---|---|---|
| AM1296 | 799 | GTSTEPSEGSAPGSEPATSGSETPGSPAGSPTSTEEGSTSSTAESPGPGTSTP ESGSASPGSTSESPSGTAPGSTSESPSGTAPGTSTPESGSASPGTSTPESGSA SPGSEPATSGSETPGTSESATPESGPGSPAGSPTSTEEGTSTEPSEGSAPGTS ESATPESGPGTSTEPSEGSAPGTSTEPSEGSAPGSPAGSPTSTEEGTSTEPSE GSAPGTSTEPSEGSAPGTSESATPESGPGTSESATPESGPGTSTEPSEGSAP GTSTEPSEGSAPGTSESATPESGPGTSTEPSEGSAPGSEPATSGSETPGSPA GSPTSTEEGSSTPSGATGSPGTPGSGTASSSPGSSTPSGATGSPGTSTEPSE GSAPGTSTEPSEGSAPGSEPATSGSETPGSPAGSPTSTEEGSPAGSPTSTEE GTSTEPSEGSAPGPEPTGPAPSGGSEPATSGSETPGTSESATPESGPGSPAG SPTSTEEGTSESATPESGPGSPAGSPTSTEEGSPAGSPTSTEEGTSESATPES GPGSPAGSPTSTEEGSPAGSPTSTEEGTSSTAESPGPGSTSESPSGTAPGTS PSGESSTAPGSTSESPSGTAPGSTSESPSGTAPGTSPSGESSTAPGTSTEPSE GSAPGTSESATPESGPGTSESATPESGPGSEPATSGSETPGTSESATPESGP GTSESATPESGPGTSTEPSEGSAPGTSESATPESGPGTSTEPSEGSAPGTSPS GESSTAPGTSPSGESSTAPGTSPSGESSTAPGTSTEPSEGSAPGSPAGSPTST EEGTSTEPSEGSAPGSSPSASTGTGPGSSTPSGATGSPGSSTPSGATGSPGS STPSGATGSPGSSTPSGATGSPGASPGTSSTGSPGASASGAPSTGGTSPSGE SSTAPGSTSSTAESPGPGTSPSGESSTAPGTSESATPESGPGTSTEPSEGSAP GTSTEPSEGSAPGSSPSASTGTGPGSSTPSGATGSPGASPGTSSTGSPGTST PESGSASPGTSPSGESSTAPGTSPSGESSTAPGTSESATPESGPGSEPATSGS ETPGTSTEPSEGSAPGSTSESPSGTAPGSTSESPSGTAPGTSTPESGSASPGS PAGSPTSTEEGTSESATPESGPGTSTEPSEGSAPGSPAGSPTSTEEGTSESA TPESGPGSEPATSGSETPGSSTPSGATGSPGASPGTSSTGSPGSSTPSGATG SPGSTSESPSGTAPGTSPSGESSTAPGSTSSTAESPGPGSSTPSGATGSPGAS PGTSSTGSPGTPGSGTASSSPGSPAGSPTSTEEGSPAGSPTSTEEGTSTEPSE GSAP | 4.5 |

## Example 31: Calculation of TEPITOPE scores

TEPITOPE scores of 9mer peptide sequence can be calculated by adding pocket potentials as described by Sturniolo [Sturniolo, T., et al. (1999) Nat Biotechnol, 17: 555]. In the present Example, separate Tepitope scores were calculated for individual HLA alleles. Table 27 shows as an example the pocket potentials for HLA*0101B, which occurs in high frequency in the Caucasian population. To calculate the TEPITOPE score of a peptide with sequence P1-P2-P3-P4-P5-P6-P7-P8-P9, the corresponding individual pocket potentials in Table 27 were added. The HLA*0101B score of a 9mer peptide with the sequence FDKLPRTSG is the sum of 0, -1.3, 0, 0.9, 0, -1.8, 0.09, 0, 0.

To evaluate the TEPITOPE scores for long peptides one can repeat the process for all 9mer subsequences of the sequences. This process can be repeated for the proteins encoded by other HLA alleles. Tables 28-31 give pocket potentials for the protein products of HLA alleles that occur with high frequency in the Caucasian population.

TEPITOPE scores calculated by this method range from approximately -10 to +10. However, 9mer peptides that lack a hydrophobic amino acid (FKLMVWY) in P1 position have calculated TEPITOPE scores in the range of -1009 to -989. This value is biologically meaningless and reflects the fact that a hydrophobic amino acid serves as an anchor residue for HLA binding and peptides lacking a hydrophobic residue in P1 are considered non binders to HLA. Because most XTEN sequences lack hydrophobic residues, all combinations of 9mer subsequences will have TEPITOPEs in the range in the range of-1009 to -989. This method confirms that XTEN polypeptides may have few or no predicted T-cell epitopes.

### Table 27: Pocket potential for HLA*0101B allele.

| Amino Acid | P1 | P2 | P3 | P4 | P5 | P6 | P7 | P8 | P9 |
|---|---|---|---|---|---|---|---|---|---|
| A | -999 | 0 | 0 | 0 | - | 0 | 0 | - | 0 |
| C | -999 | 0 | 0 | 0 | - | 0 | 0 | - | 0 |
| D | -999 | -1.3 | -1.3 | -2.4 | - | -2.7 | -2 | - | -1.9 |

(continued)

| Amino Acid | P1 | P2 | P3 | P4 | P5 | P6 | P7 | P8 | P9 |
|---|---|---|---|---|---|---|---|---|---|
| E | -999 | 0.1 | -1.2 | -0.4 | - | -2.4 | -0.6 | - | -1.9 |
| F | 0 | 0.8 | 0.8 | 0.08 | - | -2.1 | 0.3 | - | -0.4 |
| G | -999 | 0.5 | 0.2 | -0.7 | - | -0.3 | -1.1 | - | -0.8 |
| H | -999 | 0.8 | 0.2 | -0.7 | - | -2.2 | 0.1 | - | -1.1 |
| I | -1 | 1.1 | 1.5 | 0.5 | - | -1.9 | 0.6 | - | 0.7 |
| K | -999 | 1.1 | 0 | -2.1 | - | -2 | -0.2 | - | -1.7 |
| L | -1 | 1 | 1 | 0.9 | - | -2 | 0.3 | - | 0.5 |
| M | -1 | 1.1 | 1.4 | 0.8 | - | -1.8 | 0.09 | - | 0.08 |
| N | -999 | 0.8 | 0.5 | 0.04 | - | -1.1 | 0.1 | - | -1.2 |
| P | -999 | -0.5 | 0.3 | -1.9 | - | -0.2 | 0.07 | - | -1.1 |
| Q | -999 | 1.2 | 0 | 0.1 | - | -1.8 | 0.2 | - | -1.6 |
| R | -999 | 2.2 | 0.7 | -2.1 | - | -1.8 | 0.09 | - | -1 |
| S | -999 | -0.3 | 0.2 | -0.7 | - | -0.6 | -0.2 | - | -0.3 |
| T | -999 | 0 | 0 | -1 | - | -1.2 | 0.09 | - | -0.2 |
| V | -1 | 2.1 | 0.5 | -0.1 | - | -1.1 | 0.7 | - | 0.3 |
| W | 0 | -0.1 | 0 | -1.8 | - | -2.4 | -0.1 | - | -1.4 |
| Y | 0 | 0.9 | 0.8 | -1.1 | - | -2 | 0.5 | - | -0.9 |

**Table 28: Pocket potential for HLA*0301B allele.**

| Amino acid | P1 | P2 | P3 | P4 | P5 | P6 | P7 | P8 | P9 |
|---|---|---|---|---|---|---|---|---|---|
| A | -999 | 0 | 0 | 0 | - | 0 | 0 | - | 0 |
| C | -999 | 0 | 0 | 0 | - | 0 | 0 | - | 0 |
| D | -999 | -1.3 | -1.3 | 2.3 | - | -2.4 | -0.6 | - | -0.6 |
| E | -999 | 0.1 | -1.2 | -1 | - | -1.4 | -0.2 | - | -0.3 |
| F | -1 | 0.8 | 0.8 | -1 | - | -1.4 | 0.5 | - | 0.9 |
| G | -999 | 0.5 | 0.2 | 0.5 | - | -0.7 | 0.1 | - | 0.4 |
| H | -999 | 0.8 | 0.2 | 0 | - | -0.1 | -0.8 | - | -0.5 |
| I | 0 | 1.1 | 1.5 | 0.5 | - | 0.7 | 0.4 | - | 0.6 |
| K | -999 | 1.1 | 0 | -1 | - | 1.3 | -0.9 | - | -0.2 |
| L | 0 | 1 | 1 | 0 | - | 0.2 | 0.2 | - | -0 |
| M | 0 | 1.1 | 1.4 | 0 | - | -0.9 | 1.1 | - | 1.1 |
| N | -999 | 0.8 | 0.5 | 0.2 | - | -0.6 | -0.1 | - | -0.6 |
| P | -999 | -0.5 | 0.3 | -1 | - | 0.5 | 0.7 | - | -0.3 |
| Q | -999 | 1.2 | 0 | 0 | - | -0.3 | -0.1 | - | -0.2 |
| R | -999 | 2.2 | 0.7 | -1 | - | 1 | -0.9 | - | 0.5 |
| S | -999 | -0.3 | 0.2 | 0.7 | - | -0.1 | 0.07 | - | 1.1 |
| T | -999 | 0 | 0 | -1 | - | 0.8 | -0.1 | - | -0.5 |

(continued)

| Amino acid | P1 | P2 | P3 | P4 | P5 | P6 | P7 | P8 | P9 |
|---|---|---|---|---|---|---|---|---|---|
| V | 0 | 2.1 | 0.5 | 0 | - | 1.2 | 0.2 | - | 0.3 |
| W | -1 | -0.1 | 0 | -1 | - | -1.4 | -0.6 | - | -1 |
| Y | -1 | 0.9 | 0.8 | -1 | - | -1.4 | -0.1 | - | 0.3 |

**Table 29: Pocket potential for HLA*0401B allele.**

| Amino acid | P1 | P2 | P3 | P4 | P5 | P6 | P7 | P8 | P9 |
|---|---|---|---|---|---|---|---|---|---|
| A | -999 | 0 | 0 | 0 | - | 0 | 0 | - | 0 |
| C | -999 | 0 | 0 | 0 | - | 0 | 0 | - | 0 |
| D | -999 | -1.3 | -1.3 | 1.4 | - | -1.1 | -0.3 | - | -1.7 |
| E | -999 | 0.1 | -1.2 | 1.5 | - | -2.4 | 0.2 | - | -1.7 |
| F | 0 | 0.8 | 0.8 | -0.9 | - | -1.1 | -1 | - | -1 |
| G | -999 | 0.5 | 0.2 | -1.6 | - | -1.5 | -1.3 | - | -1 |
| H | -999 | 0.8 | 0.2 | 1.1 | - | -1.4 | 0 | - | 0.08 |
| I | -1 | 1.1 | 1.5 | 0.8 | - | -0.1 | 0.08 | - | -0.3 |
| K | -999 | 1.1 | 0 | -1.7 | - | -2.4 | -0.3 | - | -0.3 |
| L | -1 | 1 | 1 | 0.8 | - | -1.1 | 0.7 | - | -1 |
| M | -1 | 1.1 | 1.4 | 0.9 | - | -1.1 | 0.8 | - | -0.4 |
| N | -999 | 0.8 | 0.5 | 0.9 | - | 1.3 | 0.6 | - | -1.4 |
| P | -999 | -0.5 | 0.3 | -1.6 | - | 0 | -0.7 | - | -1.3 |
| Q | -999 | 1.2 | 0 | 0.8 | - | -1.5 | 0 | - | 0.5 |
| R | -999 | 2.2 | 0.7 | -1.9 | - | -2.4 | -1.2 | - | -1 |
| S | -999 | -0.3 | 0.2 | 0.8 | - | 1 | -0.2 | - | 0.7 |
| T | -999 | 0 | 0 | 0.7 | - | 1.9 | -0.1 | - | -1.2 |
| V | -1 | 2.1 | 0.5 | -0.9 | - | 0.9 | 0.08 | - | -0.7 |
| W | 0 | -0.1 | 0 | -1.2 | - | -1 | -1.4 | - | -1 |
| Y | 0 | 0.9 | 0.8 | -1.6 | - | -1.5 | -1.2 | - | -1 |

**Table 30: Pocket potential for HLA*0701B allele.**

| Amino acid | P1 | P2 | P3 | P4 | P5 | P6 | P7 | P8 | P9 |
|---|---|---|---|---|---|---|---|---|---|
| A | -999 | 0 | 0 | 0 | - | 0 | 0 | - | 0 |
| C | -999 | 0 | 0 | 0 | - | 0 | 0 | - | 0 |
| D | -999 | -1.3 | -1.3 | -1.6 | - | -2.5 | -1.3 | - | -1.2 |
| E | -999 | 0.1 | -1.2 | -1.4 | - | -2.5 | 0.9 | - | -0.3 |
| F | 0 | 0.8 | 0.8 | 0.2 | - | -0.8 | 2.1 | - | 2.1 |
| G | -999 | 0.5 | 0.2 | -1.1 | - | -0.6 | 0 | - | -0.6 |
| H | -999 | 0.8 | 0.2 | 0.1 | - | -0.8 | 0.9 | - | -0.2 |
| I | -1 | 1.1 | 1.5 | 1.1 | - | -0.5 | 2.4 | - | 3.4 |

(continued)

| Amino acid | P1 | P2 | P3 | P4 | P5 | P6 | P7 | P8 | P9 |
|------------|------|------|-----|------|----|------|------|----|------|
| K | -999 | 1.1 | 0 | -1.3 | - | -1.1 | 0.5 | - | -1.1 |
| L | -1 | 1 | 1 | -0.8 | - | -0.9 | 2.2 | - | 3.4 |
| M | -1 | 1.1 | 1.4 | -0.4 | - | -0.8 | 1.8 | - | 2 |
| N | -999 | 0.8 | 0.5 | -1.1 | - | -0.6 | 1.4 | - | -0.5 |
| P | -999 | -0.5 | 0.3 | -1.2 | - | -0.5 | -0.2 | - | -0.6 |
| Q | -999 | 1.2 | 0 | -1.5 | - | -1.1 | 1.1 | - | -0.9 |
| R | -999 | 2.2 | 0.7 | -1.1 | - | -1.1 | 0.7 | - | -0.8 |
| S | -999 | -0.3 | 0.2 | 1.5 | - | 0.6 | 0.4 | - | -0.3 |
| T | -999 | 0 | 0 | 1.4 | - | -0.1 | 0.9 | - | 0.4 |
| V | -1 | 2.1 | 0.5 | 0.9 | - | 0.1 | 1.6 | - | 2 |
| W | 0 | -0.1 | 0 | -1.1 | - | -0.9 | 1.4 | - | 0.8 |
| Y | 0 | 0.9 | 0.8 | -0.9 | - | -1 | 1.7 | - | 1.1 |

**Table 31: Pocket potential for HLA*1501B allele.**

| Amino acid | P1 | P2 | P3 | P4 | P5 | P6 | P7 | P8 | P9 |
|------------|------|------|------|------|----|------|------|----|------|
| A | -999 | 0 | 0 | 0 | - | 0 | 0 | - | 0 |
| C | -999 | 0 | 0 | 0 | - | 0 | 0 | - | 0 |
| D | -999 | -1.3 | -1.3 | -0.4 | - | -0.4 | -0.7 | - | -1.9 |
| E | -999 | 0.1 | -1.2 | -0.6 | - | -1 | -0.7 | - | -1.9 |
| F | -1 | 0.8 | 0.8 | 2.4 | - | -0.3 | 1.4 | - | -0.4 |
| G | -999 | 0.5 | 0.2 | 0 | - | 0.5 | 0 | - | -0.8 |
| H | -999 | 0.8 | 0.2 | 1.1 | - | -0.5 | 0.6 | - | -1.1 |
| I | 0 | 1.1 | 1.5 | 0.6 | - | 0.05 | 1.5 | - | 0.7 |
| K | -999 | 1.1 | 0 | -0.7 | - | -0.3 | -0.3 | - | -1.7 |
| L | 0 | 1 | 1 | 0.5 | - | 0.2 | 1.9 | - | 0.5 |
| M | 0 | 1.1 | 1.4 | 1 | - | 0.1 | 1.7 | - | 0.08 |
| N | -999 | 0.8 | 0.5 | -0.2 | - | 0.7 | 0.7 | - | -1.2 |
| P | -999 | -0.5 | 0.3 | -0.3 | - | -0.2 | 0.3 | - | -1.1 |
| Q | -999 | 1.2 | 0 | -0.8 | - | -0.8 | -0.3 | - | -1.6 |
| R | -999 | 2.2 | 0.7 | 0.2 | - | 1 | -0.5 | - | -1 |
| S | -999 | -0.3 | 0.2 | -0.3 | - | 0.6 | 0.3 | - | -0.3 |
| T | -999 | 0 | 0 | -0.3 | - | -0 | 0.2 | - | -0.2 |
| V | 0 | 2.1 | 0.5 | 0.2 | - | -0.3 | 0.3 | - | 0.3 |
| W | -1 | -0.1 | 0 | 0.4 | - | -0.4 | 0.6 | - | -1.4 |
| Y | -1 | 0.9 | 0.8 | 2.5 | - | 0.4 | 0.7 | - | -0.9 |

**Table 32: Exemplary Biological Activity, Exemplary Assays and Indications**

| Biologically Active Protein | Biological Activity | Exemplary Activity Assays | Indication: |
|---|---|---|---|
| Glucagon-Like-Peptide 2 (GLP2; $Gly^2$ GLP-2) | Stimulates proliferation and inhibits apoptosis of intestinal epithelial cells; reduces epithelial permeability; decreases gastric acid secretion and gastrointestinal motility; promotes wound healing. | Intestinal epithelial cell proliferation can be measured using methods known in the art, including the cell proliferation assays described in Dig. Dis. Sci. 47(5): 1135-1140 (2002). Protection of intestinal epithelium can be evaluated using methods known in the art, including the in vitro intestinal injury model described in J. Surg. Res 107(1): 44-9 | Gastrointestinal conditions including, but not limited to: gastrointestinal epithelial injury; recovery from bowel resection; enteritis; colitis; gastritis; chemotherapy-induced mucositis; short bowel syndrome; intestinal atrophy; inflammatory bowel disease; Crohn's disease; Ulcerative colitis; acid reflux; peptic ulcers; diabetes-associated bowel growth; intestinal |

| Biologically Active Protein | Biological Activity | Exemplary Activity Assays | Indication: |
|---|---|---|---|
|  |  | (2002). GLP-2 can be assayed by radioimmunoassay described in Regu. Physiol. 278(4): R1057-R1063 (2000). Contractility of intestinal tissue by GLP-2 can be measured as described in US Pat. No. 7,498,141; Measurement of cAMP levels in isolated rat small intestinal mucosal cells expressing GLP-2 receptors or in COS cells transfected with the GLP-2 receptor, or AP-1 luciferase reporter gene activity in BHK fibroblast cells endogenously expressing the GLP-2 receptor as described in US Pat App. No. 20110171164; EC50 determinations by Flipper assay measuring calcium flux by fluorescence triggered by binding of GLP-2 to an engineered cell line with a stable GLP2-R and G α q/11 expression. | ischemia syndromes; maintenance of gut integrity after major burn trauma; regulation of intestinal permeability and nutrient absorption. Hyperglycemia; Diabetes; Diabetes Insipidus; Diabetes mellitus; Type 1 diabetes; Type 2 diabetes; Insulin resistance; Insulin deficiency; Hyperlipidemia; Hyperketonemia; Non-insulin dependent Diabetes Mellitus (NIDDM); Insulin-dependent Diabetes Mellitus (IDDM); Conditions associated with Diabetes including, but not limited to Obesity, Heart Disease, Hyperglycemia, Infections, Retinopathy, And/Or Ulcers; Metabolic Disorders; Immune Disorders; Obesity; Vascular Disorders; Suppression of Body Weight; Suppression of Appetite; Syndrome X. |

**Table 33: Exemplary GLP2-XTEN comprising GLP-2 and terminal XTEN**

| GLP2-XTEN Name* | Amino Acid Sequence |
|---|---|
| GLP-2-AE144 | HADGSFSDEMNTILDNLAARDFINWLIQTKITDGGSEPATSGSETPGTSESATPESGPGSEPATSGSETPGSPAGSPTSTEEGTSTEPSEGSAPGSEPATSGSETPGSEPATSGSETPGSEPATSGSETPGTSTEPSEGSAPGTSESATPESGPGSEPATSGSETPGTSTEPSEGSAP |
| GLP-2 variant 2-AE144 | HGDGSFSDEMNTILDNLAARDFINWLIQTKITDGGSEPATSGSETPGTSESATPESGPGSEPATSGSETPGSPAGSPTSTEEGTSTEPSEGSAPGSEPATSGSETPGSEPATSGSETPGSEPATSGSETPGTSTEPSEGSAPGTSESATPESGPGSEPATSGSETPGTSTEPSEGSAP |

(continued)

| GLP2-XTEN Name* | Amino Acid Sequence |
|---|---|
| GLP-2-AE288 | HADGSFSDEMNTILDNLAARDFINWLIQTKITDGGTSESATPESGPGSEPATSGSETPGTSESAT PESGPGSEPATSGSETPGTSESATPESGPGTSTEPSEGSAPGSPAGSPTSTEEGTSESATPESGPG SEPATSGSETPGTSESATPESGPGSPAGSPTSTEEGSPAGSPTSTEEGTSTEPSEGSAPGTSESATP ESGPGTSESATPESGPGTSESATPESGPGSEPATSGSETPGSEPATSGSETPGSPAGSPTSTEEGT STEPSEGSAPGTSTEPSEGSAPGSEPATSGSETPGTSESATPESGPGTSTEPSEGSAP |
| GLP-2 variant 2-AE288 | HGDGSFSDEMNTILDNLAARDFINWLIQTKITDGGTSESATPESGPGSEPATSGSETPGTSESAT PESGPGSEPATSGSETPGTSESATPESGPGTSTEPSEGSAPGSPAGSPTSTEEGTSESATPESGPG SEPATSGSETPGTSESATPESGPGSPAGSPTSTEEGSPAGSPTSTEEGTSTEPSEGSAPGTSESATP |
| | ESGPGTSESATPESGPGTSESATPESGPGSEPATSGSETPGSEPATSGSETPGSPAGSPTSTEEGT STEPSEGSAPGTSTEPSEGSAPGSEPATSGSETPGTSESATPESGPGTSTEPSEGSAP |
| GLP-2-AF144 | HADGSFSDEMNTILDNLAARDFINWLIQTKITDGGTSTPESGSASPGTSPSGESSTAPGTSPSGE SSTAPGSTSSTAESPGPGTSSESPSGTAPGSTSSTAESPGPGTSPSGESSTAPGTSTPESGSASPGS TSSTAESPGPGTSPSGESSTAPGTSPSGESSTAPGTSPSGESSTAP |
| GLP-2 variant 2-AF144 | HGDGSFSDEMNTILDNLAARDFINWLIQTKITDGGTSTPESGSASPGTSPSGESSTAPGTSPSGE SSTAPGSTSSTAESPGPGTSSESPSGTAPGSTSSTAESPGPGTSPSGESSTAPGTSTPESGSASPGS TSSTAESPGPGTSPSGESSTAPGTSPSGESSTAPGTSPSGESSTAP |
| GLP-2-AD576 | HADGSFSDEMNTILDNLAARDFINWLIQTKITDGGSSESGSSEGGPGSGGEPSESGSSGSSESGS SEGGPGSSESGSSEGGPGSSESGSSEGGPGSSESGSSEGGPGSSESGSSEGGPGESPGGSSGSESG SEGSSGPGESSGSSESGSSEGGPGSSESGSSEGGPGSSESGSSEGGPGSGGEPSESGSSGSGGEPSESGSSG SGSESGESPGGSSGSESGSGGEPSESGSSGSSESGSSEGGPGSGGEPSESGSSGSGGEPSESGSSG SEGSSGPGESSGESPGGSSGSESGSGGEPSESGSSGSGGEPSESGSSGSGGEPSESGSSGSSESGS SEGGPGESPGGSSGSESGESPGGSSGSESGESPGGSSGSESGESPGGSSGSESGESPGGSSGSESG SSESGSSEGGPGSGGEPSESGSSGSEGSSGPGESSGSSESGSSEGGPGSGGEPSESGSSGSSESGS SEGGPGSGGEPSESGSSGSGGEPSESGSSGESPGGSSGSESGSEGSSGPGESSGSSESGS SEGGPGSEGSSGPGESS |
| GLP-2 variant 2-AD576 | HGDGSFSDEMNTILDNLAARDFINWLIQTKITDGGSSESGSSEGGPGSGGEPSESGSSGSSESGS SEGGPGSSESGSSEGGPGSSESGSSEGGPGSSESGSSEGGPGSSESGSSEGGPGESPGGSSGSESG SEGSSGPGESSGSSESGSSEGGPGSSESGSSEGGPGSSESGSSEGGPGSGGEPSESGSSGESPGGS SGSESGESPGGSSGSESGSGGEPSESGSSGSSESGSSEGGPGSGGEPSESGSSGSGGEPSESGSSG SEGSSGPGESSGESPGGSSGSESGSGGEPSESGSSGSGGEPSESGSSGSGGEPSESGSSGSSESGS SEGGPGESPGGSSGSESGESPGGSSGSESGESPGGSSGSESGESPGGSSGSESGESPGGSSGSESG SSESGSSEGGPGSGGEPSESGSSGSEGSSGPGESSGSSESGSSEGGPGSGGEPSESGSSGSSESGS SEGGPGSGGEPSESGSSGSGGEPSESGSSGESPGGSSGSESGSEGSSGPGESSGSSESGS SEGGPGSEGSSGPGESS |
| GLP-2-AE576 | HADGSFSDEMNTILDNLAARDFINWLIQTKITDGGSPAGSPTSTEEGTSESATPESGPGTSTEPS EGSAPGSPAGSPTSTEEGTSTEPSEGSAPGTSTEPSEGSAPGTSESATPESGPGSEPATSGSETPG SEPATSGSETPGSPAGSPTSTEEGTSESATPESGPGTSTEPSEGSAPGTSTEPSEGSAPGSPAGSP TSTEEGTSTEPSEGSAPGTSTEPSEGSAPGTSESATPESGPGTSTEPSEGSAPGTSESATPESGPG SEPATSGSETPGTSTEPSEGSAPGTSTEPSEGSAPGTSESATPESGPGTSESATPESGPGSPAGSP TSTEEGTSESATPESGPGSEPATSGSETPGTSESATPESGPGTSTEPSEGSAPGTSTEPSEGSAPG TSTEPSEGSAPGTSTEPSEGSAPGTSTEPSEGSAPGSPAGSPTSTEEGTSTEPSE GSAPGTSESATPESGPGSEPATSGSETPGTSESATPESGPGSEPATSGSETPGTSESATPESGPGT STEPSEGSAPGTSESATPESGPGSPAGSPTSTEEGSPAGSPTSTEEGSPAGSPTSTEEGTSESATPE SGPGTSTEPSEGSAP |

(continued)

| GLP2-XTEN Name* | Amino Acid Sequence |
|---|---|
| GLP-2 variant 2-AE576 | HGDGSFSDEMNTILDNLAARDFINWLIQTKITDGGSPAGSPTSTEEGTSESATPESGPGTSTEPS EGSAPGSPAGSPTSTEEGTSTEPSEGSAPGTSTEPSEGSAPGTSESATPESGPGSEPATSGSETPG SEPATSGSETPGSPAGSPTSTEEGTSESATPESGPGTSTEPSEGSAPGTSTEPSEGSAPGSPAGSP TSTEEGTSTEPSEGSAPGTSTEPSEGSAPGTSESATPESGPGTSTEPSEGSAPGTSESATPESGPG SEPATSGSETPGTSTEPSEGSAPGTSTEPSEGSAPGTSESATPESGPGTSESATPESGPGSPAGSP TSTEEGTSESATPESGPGSEPATSGSETPGTSESATPESGPGTSTEPSEGSAPGTSTEPSEGSAPG TSTEPSEGSAPGTSTEPSEGSAPGTSTEPSEGSAPGSPAGSPTSTEEGTSTEPSE GSAPGTSESATPESGPGSEPATSGSETPGTSESATPESGPGSEPATSGSETPGTSESATPESGPGT STEPSEGSAPGTSESATPESGPGSPAGSPTSTEEGSPAGSPTSTEEGSPAGSPTSTEEGTSESATPE SGPGTSTEPSEGSAP |
| GLP-2-AF576 | HADGSFSDEMNTILDNLAARDFINWLIQTKITDGGSTSSTAESPGPGSTSSTAESPGPGSTSESP SGTAPGSTSSTAESPGPGSTSSTAESPGPGTSTPESGSASPGSTSESPSGTAPGSTSPSGESSTAPGS TSESPSGTAPGSTSESPSGTAPGTSPSGESSTAPGSTSESPSGTAPGSTSESPSGTAPGTSPSGESS TAPGSTSESPSGTAPGSTSESPSGTAPGSTSESPSGTAPGTSTPESGSASPGSTSESPSGTAPGTST PESGSASPGSTSSTAESPGPGSTSSTAESPGPGTSTPESGSASPGTSTPESGSASPGSTSESPSGTA PGTSTPESGSASPGTSTPESGSASPGSTSESPSGTAPGSTSESPSGTAPGSTSESPSGTAPGSTSST AESPGPGTSTPESGSASPGTSTPESGSASPGSTSESPSGTAPGSTSESPSGTAPGTSTPESGSASPG STSESPSGTAPGSTSESPSGTAPGTSTPESGSASPGTSPSGESSTAPGSTSSTAESPGPGTSPSGES STAPGSTSSTAESPGPGTSTPESGSASPGSTSESPSGTAPGSTSSTAESPGPGTSTPESGSASPGTS |
| | TPESGSASP |
| GLP-2 variant 2-AF576 | HGDGSFSDEMNTILDNLAARDFINWLIQTKITDGGSTSSTAESPGPGSTSSTAESPGPGSTSESP SGTAPGSTSSTAESPGPGSTSSTAESPGPGTSTPESGSASPGSTSESPSGTAPGTSPSGESSTAPGS TSESPSGTAPGSTSESPSGTAPGTSPSGESSTAPGSTSESPSGTAPGSTSESPSGTAPGTSPSGESS TAPGSTSESPSGTAPGSTSESPSGTAPGSTSESPSGTAPGTSTPESGSASPGSTSESPSGTAPGTST PESGSASPGSTSSTAESPGPGSTSSTAESPGPGTSTPESGSASPGTSTPESGSASPGSTSESPSGTA PGTSTPESGSASPGTSTPESGSASPGSTSESPSGTAPGSTSESPSGTAPGSTSESPSGTAPGSTSST AESPGPGTSTPESGSASPGTSTPESGSASPGSTSESPSGTAPGSTSESPSGTAPGTSTPESGSASPG STSESPSGTAPGSTSESPSGTAPGTSTPESGSASPGTSPSGESSTAPGSTSSTAESPGPGTSPSGES STAPGSTSSTAESPGPGTSTPESGSASPGSTSESPSGTAPGSTSSTAESPGPGTSTPESGSASPGTS TPESGSASP |
| GLP-2 variant 2-AE624 | HGDGSFSDEMNTILDNLAARDFINWLIQTKITDGMAEPAGSPTSTEEGTPGSGTASSSPGSSTP SGATGSPGASPGTSSTGSPGSPAGSPTSTEEGTSESATPESGPGTSTEPSEGSAPGSPAGSPTSTE EGTSTEPSEGSAPGTSTEPSEGSAPGTSESATPESGPGSEPATSGSETPGSEPATSGSETPGSPAG SPTSTEEGTSESATPESGPGTSTEPSEGSAPGTSTEPSEGSAPGSPAGSPTSTEEGTSTEPSEGSAP GTSTEPSEGSAPGTSESATPESGPGTSTEPSEGSAPGTSESATPESGPGSEPATSGSETPGTSTEPS EGSAPGTSTEPSEGSAPGTSESATPESGPGTSESATPESGPGSPAGSPTSTEEGTSESATPESGPG SEPATSGSETPGTSESATPESGPGTSTEPSEGSAPGTSTEPSEGSAPGTSTEPSEGSAPGTSTEPSE GSAPGTSTEPSEGSAPGTSTEPSEGSAPGSPAGSPTSTEEGTSTEPSEGSAPGTSESATPESGPGS EPATSGSETPGTSESATPESGPGSEPATSGSETPGTSESATPESGPGTSTEPSEGSAPGTSESATP ESGPGSPAGSPTSTEEGSPAGSPTSTEEGSPAGSPTSTEEGTSESATPESGPGTSTEPSEGSAP |

(continued)

| GLP2-XTEN Name* | Amino Acid Sequence |
|---|---|
| GLP-2-AD836 | HADGSFSDEMNTILDNLAARDFINWLIQTKITDGGSSESGSSEGGPGSSESGSSEGGPGESPGG SSGSESGSGGEPSESGSSGESPGGSSGSESGESPGGSSGSESGSSESGSSEGGPGSSESGSSEGGP GSSESGSSEGGPGESPGGSSGSESGESPGGSSGSESGESPGGSSGSESGSSESGSSEGGPGSSESG SSEGGPGSSESGSSEGGPGSSESGSSEGGPGSSESGSSEGGPGSSESGSSEGGPGSGGEPSESGSS GESPGGSSGSESGESPGGSSGSESGSGGEPSESGSSGSEGSSGPGESSGSSESGSSEGGPGSGGEP SESGSSGSEGSSGPGESSGSSESGSSEGGPGSGGEPSESGSSGESPGGSSGSESGSGGEPSESGSS GSGGEPSESGSSGSSESGSSEGGPGSGGEPSESGSSGSGGEPSESGSSGSEGSSGPGESSGESPGG SSGSESGSEGSSGPGESSGSEGSSGPGESSGSGGEPSESGSSGSSESGSSEGGPGSSESGSSEGGP GESPGGSSGSESGSGGEPSESGSSGSEGSSGPGESSGESPGGSSGSESGSEGSSGPGSSESGSSEG GPGSGGEPSESGSSGSEGSSGPGESSGSEGSSGPGESSGSEGSSGPGESSGSGGEPSESGSSGSG GEPSESGSSGESPGGSSGSESGESPGGSSGSESGSGGEPSESGSSGSEGSSGPGESSGESPGGSSG SESGSSESGSSEGGPGSSESGSSEGGPGSSESGSSEGGPGSGGEPSESGSSGSSESGSSEGGPGES PGGSSGSESGSGGEPSESGSSGSSESGSSEGGPGESPGGSSGSESGSGGEPSESGSSGESPGGSSG SESGSGGEPSESGSS |
| GLP-2 variant 2-AD836 | HGDGSFSDEMNTILDNLAARDFINWLIQTKITDGGSSESGSSEGGPGSSESGSSEGGPGESPGG SSGSESGSGGEPSESGSSGESPGGSSGSESGESPGGSSGSESGSSESGSSEGGPGSSESGSSEGGP GSSESGSSEGGPGESPGGSSGSESGESPGGSSGSESGESPGGSSGSESGSSESGSSEGGPGSSESG SSEGGPGSSESGSSEGGPGSSESGSSEGGPGSSESGSSEGGPGSSESGSSEGGPGSGGEPSESGSS GESPGGSSGSESGESPGGSSGSESGSGGEPSESGSSGSEGSSGPGESSGSSESGSSEGGPGSGGEP SESGSSGSEGSSGPGESSGSSESGSSEGGPGSGGEPSESGSSGESPGGSSGSESGSGGEPSESGSS GSGGEPSESGSSGSSESGSSEGGPGSGGEPSESGSSGSGGEPSESGSSGSEGSSGPGESSGESPGG SSGSESGSEGSSGPGESSGSEGSSGPGESSGSGGEPSESGSSGSSESGSSEGGPGSSESGSSEGGP GESPGGSSGSESGSGGEPSESGSSGSEGSSGPGESSGESPGGSSGSESGSEGSSGPGSSESGSSEG GPGSGGEPSESGSSGSEGSSGPGESSGSEGSSGPGESSGSEGSSGPGESSGSGGEPSESGSSGSG GEPSESGSSGESPGGSSGSESGESPGGSSGSESGSGGEPSESGSSGSEGSSGPGESSGESPGGSSG SESGSSESGSSEGGPGSSESGSSEGGPGSSESGSSEGGPGSGGEPSESGSSGSSESGSSEGGPGES PGGSSGSESGSGGEPSESGSSGSSESGSSEGGPGESPGGSSGSESGSGGEPSESGSSGESPGGSSG SESGSGGEPSESGSS |
| GLP-2-AE864 | HADGSFSDEMNTILDNLAARDFINWLIQTKITDGGSPAGSPTSTEEGTSESATPESGPGTSTEPS EGSAPGSPAGSPTSTEEGTSTEPSEGSAPGTSTEPSEGSAPGTSESATPESGPGSEPATSGSETPG SEPATSGSETPGSPAGSPTSTEEGTSESATPESGPGTSTEPSEGSAPGTSTEPSEGSAPGSPAGSP TSTEEGTSTEPSEGSAPGTSTEPSEGSAPGTSESATPESGPGTSTEPSEGSAPGTSESATPESGPG SEPATSGSETPGTSTEPSEGSAPGTSTEPSEGSAPGTSESATPESGPGTSESATPESGPGSPAGSP TSTEEGTSESATPESGPGSEPATSGSETPGTSESATPESGPGTSTEPSEGSAPGTSTEPSEGSAPG TSTEPSEGSAPGTSTEPSEGSAPGTSTEPSEGSAPGTSTEPSEGSAPGSPAGSPTSTEEGTSTEPSE GSAPGTSESATPESGPGSEPATSGSETPGTSESATPESGPGSEPATSGSETPGTSESATPESGPGT STEPSEGSAPGTSESATPESGPGSPAGSPTSTEEGSPAGSPTSTEEGSPAGSPTSTEEGTSESATPE |
| | SGPGTSTEPSEGSAPGTSESATPESGPGSEPATSGSETPGTSESATPESGPGSEPATSGSETPGTS ESATPESGPGTSTEPSEGSAPGSPAGSPTSTEEGTSESATPESGPGSEPATSGSETPGTSESATPES GPGSPAGSPTSTEEGSPAGSPTSTEEGTSTEPSEGSAPGTSESATPESGPGTSESATPESGPGTSE SATPESGPGSEPATSGSETPGSEPATSGSETPGSPAGSPTSTEEGTSTEPSEGSAPGTSTEPSEGS APGSEPATSGSETPGTSESATPESGPGTSTEPSEGSAPG |

(continued)

| GLP2-XTEN Name* | Amino Acid Sequence |
|---|---|
| GLP-2 variant 2-AE864 | HGDGSFSDEMNTILDNLAARDFINWLIQTKITDGGSPAGSPTSTEEGTSESATPESGPGTSTEPS EGSAPGSPAGSPTSTEEGTSTEPSEGSAPGTSTEPSEGSAPGTSESATPESGPGSEPATSGSETPG SEPATSGSETPGSPAGSPTSTEEGTSESATPESGPGTSTEPSEGSAPGTSTEPSEGSAPGSPAGSP TSTEEGTSTEPSEGSAPGTSTEPSEGSAPGTSESATPESGPGTSTEPSEGSAPGTSESATPESGPG SEPATSGSETPGTSTEPSEGSAPGTSTEPSEGSAPGTSESATPESGPGTSESATPESGPGSPAGSP TSTEEGTSESATPESGPGSEPATSGSETPGTSESATPESGPGTSTEPSEGSAPGTSTEPSEGSAPG TSTEPSEGSAPGTSTEPSEGSAPGTSTEPSEGSAPGTSTEPSEGSAPGSPAGSPTSTEEGTSTEPSE GSAPGTSESATPESGPGSEPATSGSETPGTSESATPESGPGSEPATSGSETPGTSESATPESGPGT STEPSEGSAPGTSESATPESGPGSPAGSPTSTEEGSPAGSPTSTEEGSPAGSPTSTEEGTSESATPE SGPGTSTEPSEGSAPGTSESATPESGPGSEPATSGSETPGTSESATPESGPGSEPATSGSETPGTS ESATPESGPGTSTEPSEGSAPGSPAGSPTSTEEGTSESATPESGPGSEPATSGSETPGTSESATPES GPGSPAGSPTSTEEGSPAGSPTSTEEGTSTEPSEGSAPGTSESATPESGPGTSESATPESGPGTSE SATPESGPGSEPATSGSETPGSEPATSGSETPGSPAGSPTSTEEGTSTEPSEGSAPGTSTEPSEGS APGSEPATSGSETPGTSESATPESGPGTSTEPSEGSAPG |
| GLP-2 variant 1-AE864 | HADGSFSDEMNTILDNLATRDFINWLIQTKITDGGSPAGSPTSTEEGTSESATPESGPGTSTEPS EGSAPGSPAGSPTSTEEGTSTEPSEGSAPGTSTEPSEGSAPGTSESATPESGPGSEPATSGSETPG SEPATSGSETPGSPAGSPTSTEEGTSESATPESGPGTSTEPSEGSAPGTSTEPSEGSAPGSPAGSP TSTEEGTSTEPSEGSAPGTSTEPSEGSAPGTSESATPESGPGTSTEPSEGSAPGTSESATPESGPG SEPATSGSETPGTSTEPSEGSAPGTSTEPSEGSAPGTSESATPESGPGTSESATPESGPGSPAGSP TSTEEGTSESATPESGPGSEPATSGSETPGTSESATPESGPGTSTEPSEGSAPGTSTEPSEGSAPG TSTEPSEGSAPGTSTEPSEGSAPGTSTEPSEGSAPGTSTEPSEGSAPGSPAGSPTSTEEGTSTEPSE GSAPGTSESATPESGPGSEPATSGSETPGTSESATPESGPGSEPATSGSETPGTSESATPESGPGT STEPSEGSAPGTSESATPESGPGSPAGSPTSTEEGSPAGSPTSTEEGSPAGSPTSTEEGTSESATPE SGPGTSTEPSEGSAPGTSESATPESGPGSEPATSGSETPGTSESATPESGPGSEPATSGSETPGTS ESATPESGPGTSTEPSEGSAPGSPAGSPTSTEEGTSESATPESGPGSEPATSGSETPGTSESATPES GPGSPAGSPTSTEEGSPAGSPTSTEEGTSTEPSEGSAPGTSESATPESGPGTSESATPESGPGTSE SATPESGPGSEPATSGSETPGSEPATSGSETPGSPAGSPTSTEEGTSTEPSEGSAPGTSTEPSEGS APGSEPATSGSETPGTSESATPESGPGTSTEPSEGSAPG |
| GLP-2 variant 3-AE864 | HVDGSFSDEMNTILDNLAARDFINWLIQTKITDGGSPAGSPTSTEEGTSESATPESGPGTSTEPS EGSAPGSPAGSPTSTEEGTSTEPSEGSAPGTSTEPSEGSAPGTSESATPESGPGSEPATSGSETPG SEPATSGSETPGSPAGSPTSTEEGTSESATPESGPGTSTEPSEGSAPGTSTEPSEGSAPGSPAGSP TSTEEGTSTEPSEGSAPGTSTEPSEGSAPGTSESATPESGPGTSTEPSEGSAPGTSESATPESGPG SEPATSGSETPGTSTEPSEGSAPGTSTEPSEGSAPGTSESATPESGPGTSESATPESGPGSPAGSP TSTEEGTSESATPESGPGSEPATSGSETPGTSESATPESGPGSEPATSGSETPGTSESATPESGPGT STEPSEGSAPGTSESATPESGPGSPAGSPTSTEEGSPAGSPTSTEEGSPAGSPTSTEEGTSESATPE SGPGTSTEPSEGSAPGTSESATPESGPGSEPATSGSETPGTSESATPESGPGSEPATSGSETPGTS ESATPESGPGTSTEPSEGSAPGSPAGSPTSTEEGTSESATPESGPGSEPATSGSETPGTSESATPES GPGSPAGSPTSTEEGSPAGSPTSTEEGTSTEPSEGSAPGTSESATPESGPGTSESATPESGPGTSE SATPESGPGSEPATSGSETPGSEPATSGSETPGSPAGSPTSTEEGTSTEPSEGSAPGTSTEPSEGS APGSEPATSGSETPGTSESATPESGPGTSTEPSEGSAPG |
| GLP-2-AF864 | HADGSFSDEMNTILDNLAARDFINWLIQTKITDGGSTSESPSGTAPGTSPSGESSTAPGSTSESP SGTAPGSTSESPSGTAPGTSTPESGSASPGTSTPESGSASPGTSESPSGTAPGSTSESPSGTAPGT SPSGESSTAPGSTSESPSGTAPGTSPSGESSTAPGTSPSGESSTAPGSTSSTAESPGPGTSPSGESS TAPGTSPSGESSTAPGSTSSTAESPGPGTSTPESGSASPGTSTPESGSASPGTSESPSGTAPGSTS ESPSGTAPGTSTPESGSASPGTSSTAESPGPGTSTPESGSASPGTSESPSGTAPGTSPSGESSTA PGTSSTAESPGPGTSPSGESSTAPGTSTPESGSASPGTSSTAESPGPGTSSTAESPGPGTSSST AESPGPGTSSTAESPGPGTSPSGESSTAPGTSESPSGTAPGTSESPSGTAPGTSTPESGPXXX GASASGAPSTXXXXSESPSGTAPGSTSESPSGTAPGSTSESPSGTAPGSTSESPSGTAPGSTSESP SGTAPGSTSESPSGTAPGTSTPESGSASPGTSPSGESSTAPGTSPSGESSTAPGSTSSTAESPGPGT SPSGESSTAPGTSTPESGSASPGTSESPSGTAPGSTSESPSGTAPGTSPSGESSTAPGSTSESPSG TAPGTSTPESGSASPGTSTPESGSASPGTSESPSGTAPGTSTPESGSASPGSTSSTAESPGPGSTS |

(continued)

| GLP2-XTEN Name* | Amino Acid Sequence |
|---|---|
| | ESPSGTAPGSTSESPSGTAPGTSPSGESSTAPGSTSSTAESPGPGTSPSGESSTAPGTSTPESGSAS PGTSPSGESSTAPGTSPSGESSTAPGTSPSGESSTAPGSTSSTAESPGPGTSSTAESPGPGTSPSG ESSTAPGSSPSASTGTGPGSSTPSGATGSPGSSTPSGATGSP |
| GLP-2 variant 2-AF864 | HGDGSFSDEMNTILDNLAARDFINWLIQTKITDGGSTSESPSGTAPGTSPSGESSTAPGSTSESP SGTAPGSTSESPSGTAPGTSTPESGSASPGTSTPESGSASPGSTSESPSGTAPGSTSESPSGTAPGT SPSGESSTAPGSTSESPSGTAPGTSPSGESSTAPGTSPSGESSTAPGSTSSTAESPGPGTSPSGESS TAPGTSPSGESSTAPGSTSSTAESPGPGTSTPESGSASPGTSTPESGSASPGSTSESPSGTAPGSTS ESPSGTAPGTSTPESGSASPGSTSSTAESPGPGTSTPESGSASPGSTSESPSGTAPGTSPSGESSTA PGTSSTAESPGPGTSPSGESSTAPGTSTPESGSASPGSTSSTAESPGPGTSSTAESPGPGTSSST AESPGPGTSSTAESPGPGTSPSGESSTAPGTSESPSGTAPGSTSESPSGTAPGTSTPESGPXXX GASASGAPSTXXXXSESPSGTAPGSTSESPSGTAPGSTSESPSGTAPGSTSESPSGTAPGSTSESP SGTAPGSTSESPSGTAPGTSTPESGSASPGTSPSGESSTAPGTSPSGESSTAPGSTSSTAESPGPGT SPSGESSTAPGTSTPESGSASPGSTSESPSGTAPGSTSESPSGTAPGTSPSGESSTAPGSTSESPSG TAPGTSTPESGSASPGTSTPESGSASPGSTSESPSGTAPGTSTPESGSASPGSTSSTAESPGPGSTS ESPSGTAPGSTSESPSGTAPGTSPSGESSTAPGSTSSTAESPGPGTSPSGESSTAPGTSTPESGSAS PGTSPSGESSTAPGTSPSGESSTAPGTSPSGESSTAPGSTSSTAESPGPGTSSTAESPGPGTSPSG ESSTAPGSSPSASTGTGPGSSTPSGATGSPGSSTPSGATGSP |
| GLP-2-AG864 | HADGSFSDEMNTILDNLAARDFINWLIQTKITDGGASPGTSSTGSPGSSPSASTGTGPGSSPSAS TGTGPGTPGSGTASSSPGSSTPSGATGSPGSNPSASTGTGPGASPGTSSTGSPGTPGSGTASSSP GSSTPSGATGSPGTPGSGTASSSPGASPGTSSTGSPGASPGTSSTGSPGTPGSGTASSSPGSSTPS GATGSPGASPGTSSTGSPGTPGSGTASSSPGSSTPSGATGSPGSNPSASTGTGPGSSPSASTGTG PGSSTPSGATGSPGSSTPSGATGSPGASPGTSSTGSPGASPGTSSTGSPGASPGTSSTGSPGTPGS GTASSSPGASPGTSSTGSPGASPGTSSTGSPGASPGTSSTGSPGSSPSASTGTGPGTPGSGTASSS PGASPGTSSTGSPGASPGTSSTGSPGASPGTSSTGSPGSSTPSGATGSPGSSTPSGATGSPGASPG TSSTGSPGTPGSGTASSSPGSSTPSGATGSPGSSTPSGATGSPGSSTPSGATGSPGSSPSASTGTG PGASPGTSSTGSPGASPGTSSTGSPGTPGSGTASSSPGASPGTSSTGSPGASPGTSSTGSPGASPG TSSTGSPGASPGTSSTGSPGTPGSGTASSSPGSSTPSGATGSPGTPGSGTASSSPGSSTPSGATGS PGTPGSGTASSSPGSSTPSGATGSPGSSTPSGATGSPGSSPSASTGTGPGSSPSASTGTGPGASPG TSSTGSPGTPGSGTASSSPGSSTPSGATGSPGSSPSASTGTGPGSSPSASTGTGPGASPGTSSTGS PGASPGTSSTGSPGSSTPSGATGSPGSSPSASTGTGPGASPGTSSTGSPGSSPSASTGTGPGTPGS GTASSSPGSSTPSGATGSPGSSTPSGATGSPGASPGTSSTGSP |
| GLP-2 variant 2-AG864 | HGDGSFSDEMNTILDNLAARDFINWLIQTKITDGGASPGTSSTGSPGSSPSASTGTGPGSSPSAS TGTGPGTPGSGTASSSPGSSTPSGATGSPGSNPSASTGTGPGASPGTSSTGSPGTPGSGTASSSP GSSTPSGATGSPGTPGSGTASSSPGASPGTSSTGSPGASPGTSSTGSPGTPGSGTASSSPGSSTPS GATGSPGASPGTSSTGSPGTPGSGTASSSPGSSTPSGATGSPGSNPSASTGTGPGSSPSASTGTG PGSSTPSGATGSPGSSTPSGATGSPGASPGTSSTGSPGASPGTSSTGSPGASPGTSSTGSPGTPGS GTASSSPGASPGTSSTGSPGASPGTSSTGSPGASPGTSSTGSPGSSTPSGATGSPGSSTPSGATGSPGASPG TSSTGSPGTPGSGTASSSPGSSTPSGATGSPGSSTPSGATGSPGSSTPSGATGSPGSSPSASTGTG PGASPGTSSTGSPGASPGTSSTGSPGTPGSGTASSSPGASPGTSSTGSPGASPGTSSTGSPGASPG TSSTGSPGASPGTSSTGSPGTPGSGTASSSPGSSTPSGATGSPGTPGSGTASSSPGSSTPSGATGS PGTPGSGTASSSPGSSTPSGATGSPGSSTPSGATGSPGSSPSASTGTGPGSSPSASTGTGPGASPG TSSTGSPGTPGSGTASSSPGSSTPSGATGSPGSSPSASTGTGPGSSPSASTGTGPGASPGTSSTGS PGASPGTSSTGSPGSSTPSGATGSPGSSPSASTGTGPGASPGTSSTGSPGSSPSASTGTGPGTPGS GTASSSPGSSTPSGATGSPGSSTPSGATGSPGASPGTSSTGSP |

(continued)

| GLP2-XTEN Name* | Amino Acid Sequence |
|---|---|
| GLP-2 variant 2-AM875 | HGDGSFSDEMNTILDNLAARDFINWLIQTKITDGGTSTEPSEGSAPGSEPATSGSETPGSPAGSPTSTEEGSTSSTAESPGPGTSTPESGSASPGSTSESPSGTAPGSTSESPSGTAPGTSTPESGSASPGTSTPESGSASPGSEPATSGSETPGTSESATPESGPGSPAGSPTSTEEGTSTEPSEGSAPGTSESATPESGPGTSTEPSEGSAPGTSTEPSEGSAPGSPAGSPTSTEEGTSTEPSEGSAPGTSTEPSEGSAPGTSESATPESGPGTSESATPESGPGTSTEPSEGSAPGTSTEPSEGSAPGTSESATPESGPGTSTEPSEGSAPGSEPATSGSETPGSPAGSPTSTEEGSSTPSGATGSPGTPGSGTASSSPGSSTPSGATGSPGTSTEPSEGSAPGTSTEPSEGSAPGSEPATSGSETPGSPAGSPTSTEEGSPAGSPTSTEEGTSTEPSEGSAPGASASGAPSTGGTSESATPESGPGSPAGSPTSTEEGSPAGSPTSTEEGTSSTAESPGPGTSESPSGTAPGTSPSGESSTAPGTPGSGTASSSPGSSTPSGATGSPGSSPSASTGTGPGSEPATSGSETPGTSESATPESGPGSEPATSGSETPGTSSTAESPGPGTSSTAESPGPGTSPSGESSTAPGSEPATSGSETPGSEPATSGSETPGTSTEPSEGSAPGTSSTAESPGPGTSTPESGSASPGSTSESPSGTAPGTSTEPSEGSAPGTSTEPSEGSAPGTSTEPSEGSAPGSSTPSGATGSPGSSPSASTGTGPGASPGTSSTGSPGSEPATSGSETPGTSESATPESGPGSPAGSPTSTEEGSSTPSGATGSPGSSPSASTGTGPG |
| | ASPGTSSTGSPGTSESATPESGPGTSTEPSEGSAPGTSTEPSEGSAP |
| GLP-2 bovine-AE864 | HADGSFSDEMNTVLDSLATRDFINWLLQTKITDGGSPAGSPTSTEEGTSESATPESGPGTSTEPSEGSAPGSPAGSPTSTEEGTSTEPSEGSAPGTSTEPSEGSAPGTSESATPESGPGSEPATSGSETPGSEPATSGSETPGSPAGSPTSTEEGTSESATPESGPGTSTEPSEGSAPGTSTEPSEGSAPGSPAGSPTSTEEGTSTEPSEGSAPGTSTEPSEGSAPGTSESATPESGPGTSTEPSEGSAPGTSESATPESGPGSEPATSGSETPGTSTEPSEGSAPGTSTEPSEGSAPGTSESATPESGPGTSESATPESGPGSPAGSPTSTEEGTSESATPESGPGSEPATSGSETPGTSESATPESGPGTSTEPSEGSAPGTSTEPSEGSAPGTSTEPSEGSAPGTSTEPSEGSAPGTSTEPSEGSAPGTSTEPSEGSAPGSPAGSPTSTEEGTSTEPSEGSAPGTSESATPESGPGSEPATSGSETPGTSESATPESGPGSEPATSGSETPGTSESATPESGPGTSTEPSEGSAPGTSESATPESGPGSPAGSPTSTEEGSPAGSPTSTEEGSPAGSPTSTEEGTSESATPESGPGTSTEPSEGSAPGTSESATPESGPGSEPATSGSETPGTSESATPESGPGSEPATSGSETPGTSESATPESGPGTSTEPSEGSAPGSPAGSPTSTEEGTSESATPESGPGSEPATSGSETPGTSESATPESGPGSPAGSPTSTEEGSPAGSPTSTEEGTSTEPSEGSAPGTSESATPESGPGTSESATPESGPGTSESATPESGPGSEPATSGSETPGSEPATSGSETPGSPAGSPTSTEEGTSTEPSEGSAPGTSTEPSEGSAPGSEPATSGSETPGTSESATPESGPGTSTEPSEGSAPG |
| GLP-2 pig-AG864 | HADGSFSDEMNTVLDNLATRDFINWLLHTKITDSLGGASPGTSSTGSPGSSPSASTGTGPGSSPSASTGTGPGTPGSGTASSSPGSSTPSGATGSPGSNPSASTGTGPGASPGTSSTGSPGTPGSGTASSSPGSSTPSGATGSPGTPGSGTASSSPGASPGTSSTGSPGASPGTSSTGSPGTPGSGTASSSPGSSTPSGATGSPGSNPSASTGTGPGSSPSASTGTGPGSSTPSGATGSPGSSTPSGATGSPGASPGTSSTGSPGASPGTSSTGSPGASPGTSSTGSPGTPGSGTASSSPGASPGTSSTGSPGASPGTSSTGSPGASPGTSSTGSPGSSPSASTGTGPGTPGSGTASSSPGASPGTSSTGSPGASPGTSSTGSPGASPGTSSTGSPGSSTPSGATGSPGSSTPSGATGSPGASPGTSSTGSPGTPGSGTASSSPGSSTPSGATGSPGSSTPSGATGSPGSSTPSGATGSPGSSPSASTGTGPGASPGTSSTGSPGASPGTSSTGSPGTPGSGTASSSPGASPGTSSTGSPGASPGTSSTGSPGASPGTSSTGSPGTPGSGTASSSPGSSTPSGATGSPGTPGSGTASSSPGSSTPSGATGSPGTPGSGTASSSPGSSTPSGATGSPGSSPSASTGTGPGSSPSASTGTGPGASPGTSSTGSPGASPGTSSTGSPGSSTPSGATGSPGSSPSASTGTGPGASPGTSSTGSPGSSPSASTGTGPGTPGSGTASSSPGSSTPSGATGSPGSSTPSGATGSPGASPGTSSTGSP |
| GLP-2 rat-AE576 | HADGSFSDEMNTILDNLATRDFINWLIQTKITDGGSPAGSPTSTEEGTSESATPESGPGTSTEPSEGSAPGSPAGSPTSTEEGTSTEPSEGSAPGTSTEPSEGSAPGTSESATPESGPGSEPATSGSETPGSEPATSGSETPGSPAGSPTSTEEGTSESATPESGPGTSTEPSEGSAPGTSTEPSEGSAPGSPAGSPTSTEEGTSTEPSEGSAPGTSTEPSEGSAPGTSESATPESGPGTSTEPSEGSAPGTSESATPESGPGSEPATSGSETPGTSTEPSEGSAPGTSTEPSEGSAPGTSESATPESGPGTSESATPESGPGSPAGSPTSTEEGTSESATPESGPGSEPATSGSETPGTSESATPESGPGTSTEPSEGSAPGTSTEPSEGSAPGTSTEPSEGSAPGTSTEPSEGSAPGTSTEPSEGSAPGTSTEPSEGSAPGSPAGSPTSTEEGTSTEPSEGSAPGTSESATPESGPGSEPATSGSETPGTSESATPESGPGSEPATSGSETPGTSESATPESGPGTSTEPSEGSAPGTSESATPESGPGSPAGSPTSTEEGSPAGSPTSTEEGSPAGSPTSTEEGTSESATPESGPGTSTEPSEGSAP |

(continued)

| GLP2-XTEN Name* | Amino Acid Sequence |
|---|---|
| GLP-2 variant 5-AF864 | HKDGSFSDEMNTILDNLAARDFINWLIQTKITDGGSTSESPSGTAPGTSPSGESSTAPGSTSESP SGTAPGSTSESPSGTAPGTSTPESGSASPGTSTPESGSASPGSTSESPSGTAPGSTSESPSGTAPGT SPSGESSTAPGSTSESPSGTAPGTSPSGESSTAPGTSPSGESSTAPGSTSSTAESPGPGTSPSGESS TAPGTSPSGESSTAPGSTSSTAESPGPGTSTPESGSASPGTSTPESGSASPGSTSESPSGTAPGSTS ESPSGTAPGTSTPESGSASPGSTSSTAESPGPGTSTPESGSASPGSTSESPSGTAPGTSPSGESSTA PGSTSSTAESPGPGTSPSGESSTAPGTSTPESGSASPGSTSSTAESPGPGSTSSTAESPGPGTSSST AESPGPGSTSSTAESPGPGTSPSGESSTAPGTSESPSGTAPGTSESPSGTAPGTSTPESGPXXX GASASGAPSTXXXXSESPSGTAPGSTSESPSGTAPGSTSESPSGTAPGSTSESPSGTAPGSTSESP SGTAPGSTSESPSGTAPGTSTPESGSASPGTSPSGESSTAPGTSPSGESSTAPGSTSSTAESPGPGT SPSGESSTAPGTSTPESGSASPGSTSESPSGTAPGSTSESPSGTAPGTSPSGESSTAPGSTSESPSG TAPGTSTPESGSASPGTSTPESGSASPGSTSESPSGTAPGTSTPESGSASPGSTSSTAESPGPGSTS ESPSGTAPGSTSESPSGTAPGTSPSGESSTAPGSTSSTAESPGPGTSPSGESSTAPGTSTPESGSAS PGTSPSGESSTAPGTSPSGESSTAPGTSPSGESSTAPGSTSSTAESPGPGSTSSTAESPGPGTSPSG ESSTAPGSSPSASTGTGPGSSTPSGATGSPGSSTPSGATGSP |
| GLP-2 variant 6-AE864 | HRDGSFSDEMNTILDNLAARDFINWLIQTKITDGGSPAGSPTSTEEGTSESATPESGPGTSTEPS EGSAPGSPAGSPTSTEEGTSTEPSEGSAPGTSTEPSEGSAPGTSESATPESGPGSEPATSGSETPG SEPATSGSETPGSPAGSPTSTEEGTSESATPESGPGTSTEPSEGSAPGTSTEPSEGSAPGSPAGSP TSTEEGTSTEPSEGSAPGTSTEPSEGSAPGTSESATPESGPGTSTEPSEGSAPGTSESATPESGPG |
|  | SEPATSGSETPGTSTEPSEGSAPGTSTEPSEGSAPGTSESATPESGPGTSESATPESGPGSPAGSP TSTEEGTSESATPESGPGSEPATSGSETPGTSESATPESGPGTSTEPSEGSAPGTSTEPSEGSAPG TSTEPSEGSAPGTSTEPSEGSAPGTSTEPSEGSAPGTSTEPSEGSAPGSPAGSPTSTEEGTSTEPSE GSAPGTSESATPESGPGSEPATSGSETPGTSESATPESGPGSEPATSGSETPGTSESATPESGPGT STEPSEGSAPGTSESATPESGPGSPAGSPTSTEEGSPAGSPTSTEEGSPAGSPTSTEEGTSESATPE SGPGTSTEPSEGSAPGTSESATPESGPGSEPATSGSETPGTSESATPESGPGSEPATSGSETPGTS ESATPESGPGTSTEPSEGSAPGSPAGSPTSTEEGTSESATPESGPGSEPATSGSETPGTSESATPES GPGSPAGSPTSTEEGSPAGSPTSTEEGTSTEPSEGSAPGTSESATPESGPGTSESATPESGPGTSE SATPESGPGSEPATSGSETPGSEPATSGSETPGSPAGSPTSTEEGTSTEPSEGSAPGTSTEPSEGS APGSEPATSGSETPGTSESATPESGPGTSTEPSEGSAPG |
| GLP-2 variant 2-AE1236 | HGDGSFSDEMNTILDNLAARDFINWLIQTKITDGGSPAGSPTSTEEGTSTEPSEGSAPGSPAGSP TSTEEGTSESATPESGPGSEPATSGSETPGTSTEPSEGSAPGTSTEPSEGSAPGTSESATPESGPG TSTEPSEGSAPGTSTEPSEGSAPGSEPATSGSETPGSPAGSPTSTEEGTSESATPESGPGSPAGSP TSTEEGTSTEPSEGSAPGSPAGSPTSTEEGTSTEPSEGSAPGSPAGSPTSTEEGTSTEPSEGSAPG SPAGSPTSTEEGSPAGSPTSTEEGTSTEPSEGSAPGSEPATSGSETPGSPAGSPTSTEEGTSESATP ESGPGSEPATSGSETPGSEPATSGSETPGTSESATPESGPGSPAGSPTSTEEGTSESATPESGPGS EPATSGSETPGSEPATSGSETPGTSESATPESGPGTSESATPESGPGTSTEPSEGSAPGTSTEPSE GSAPGTSESATPESGPGSPAGSPTSTEEGTSTEPSEGSAPGSEPATSGSETPGSPAGSPTSTEEGT STEPSEGSAPGTSTEPSEGSAPGSPAGSPTSTEEGSPAGSPTSTEEGSPAGSPTSTEEGTSESATPE SGPGSEPATSGSETPGTSESATPESGPGTSESATPESGPGTSTEPSEGSAPGTSESATPESGPGSP AGSPTSTEEGTSESATPESGPGTSESATPESGPGTSTEPSEGSAPGSEPATSGSETPGTSTEPSEG SAPGSPAGSPTSTEEGTSESATPESGPGTSTEPSEGSAPGTSTEPSEGSAPGSEPATSGSETPGSE PATSGSETPGTSESATPESGPGSEPATSGSETPGSPAGSPTSTEEGTSESATPESGPGSEPATSGS ETPGTSTEPSEGSAPGTSESATPESGPGTSESATPESGPGTSTEPSEGSAPGSEPATSGSETPGTS TEPSEGSAPGSPAGSPTSTEEGSPAGSPTSTEEGTSESATPESGPGSEPATSGSETPGSPAGSPTS TEEGSPAGSPTSTEEGTSESATPESGPGSEPATSGSETPGTSTEPSEGSAPGTSTEPSEGSAPGSE P |

(continued)

| GLP2-XTEN Name* | Amino Acid Sequence |
|---|---|
| GLP-2 variant 2-AE1332 | HGDGSFSDEMNTILDNLAARDFINWLIQTKITDGGSPAGSPTSTEEGTSTEPSEGSAPGTSESAT PESGPGTSESATPESGPGSEPATSGSETPGTSTEPSEGSAPGTSESATPESGPGSPAGSPTSTEEG TSTEPSEGSAPGTSESATPESGPGSPAGSPTSTEEGTSTEPSEGSAPGTSESATPESGPGTSTEPSE GSAPGSEPATSGSETPGSEPATSGSETPGSEPATSGSETPGTSESATPESGPGSPAGSPTSTEEGT SESATPESGPGSEPATSGSETPGTSESATPESGPGTSESATPESGPGSPAGSPTSTEEGTSTEPSEG SAPGTSESATPESGPGTSTEPSEGSAPGTSTEPSEGSAPGTSTEPSEGSAPGSEPATSGSETPGTS TEPSEGSAPGTSESATPESGPGTSESATPESGPGTSTEPSEGSAPGTSESATPESGPGTSESATPES GPGSEPATSGSETPGTSTEPSEGSAPGTSESATPESGPGSPAGSPTSTEEGSPAGSPTSTEEGTST EPSEGSAPGSEPATSGSETPGSEPATSGSETPGTSTEPSEGSAPGSEPATSGSETPGSPAGSPTST EEGTSESATPESGPGSEPATSGSETPGSEPATSGSETPGTSESATPESGPGTSESATPESGPGTST EPSEGSAPGSPAGSPTSTEEGTSESATPESGPGTSTEPSEGSAPGSEPATSGSETPGSPAGSPTST EEGTSTEPSEGSAPGTSTEPSEGSAPGSEPATSGSETPGTSESATPESGPGTSTEPSEGSAPGSPA GSPTSTEEGTSESATPESGPGTSESATPESGPGTSTEPSEGSAPGSEPATSGSETPGSEPATSGSE TPGTSTEPSEGSAPGTSESATPESGPGSPAGSPTSTEEGTSTEPSEGSAPGTSTEPSEGSAPGSPA GSPTSTEEGSPAGSPTSTEEGTSTEPSEGSAPGSEPATSGSETPGSPAGSPTSTEEGTSESATPES GPGSEPATSGSETPGSEPATSGSETPGTSESATPESGPGTSESATPESGPGTSTEPSEGSAPGTST |
| GLP-2 variant 2-AE612A | HGDGSFSDEMNTILDNLAARDFINWLIQTKITDGGSETPGTSTEPSEGSAPGTSTEPSEGSAPGT SESATPESGPGTSESATPESGPGSPAGSPTSTEEGTSESATPESGPGSEPATSGSETPGTSESATPE SGPGTSTEPSEGSAPGTSTEPSEGSAPGTSTEPSEGSAPGTSTEPSEGSAPGTSTEPSEGSAPGTS TEPSEGSAPGSPAGSPTSTEEGTSTEPSEGSAPGTSESATPESGPGSEPATSGSETPGTSESATPES GPGSEPATSGSETPGTSESATPESGPGTSTEPSEGSAPGTSESATPESGPGSPAGSPTSTEEGSPA GSPTSTEEGSPAGSPTSTEEGTSESATPESGPGTSTEPSEGSAPGTSESATPESGPGSEPATSGSE TPGTSESATPESGPGSEPATSGSETPGTSESATPESGPGTSTEPSEGSAPGSPAGSPTSTEEGTSES ATPESGPGSEPATSGSETPGTSESATPESGPGSPAGSPTSTEEGSPAGSPTSTEEGTSTEPSEGSA PGTSESATPESGPGTSESATPESGPGTSESATPESGPGSEPATSGSETPGSEPATSGSETPGSPAG SPTSTEEGTSTEPSEGSAPGTSTEPSEGSAPGSEPATSGSETPGTSESAT |
| GLP-2 variant 2-AE720A | HGDGSFSDEMNTILDNLAARDFINWLIQTKITDGTSGSETPGSEPATSGSETPGSPAGSPTSTEE GTSESATPESGPGTSTEPSEGSAPGTSTEPSEGSAPGSPAGSPTSTEEGTSTEPSEGSAPGTSTEPS EGSAPGTSESATPESGPGTSTEPSEGSAPGTSESATPESGPGSEPATSGSETPGTSTEPSEGSAPG TSTEPSEGSAPGTSESATPESGPGTSESATPESGPGSPAGSPTSTEEGTSESATPESGPGSEPATS GSETPGTSESATPESGPGTSTEPSEGSAPGTSTEPSEGSAPGTSTEPSEGSAPGTSTEPSEGSAPG |
|  | TSTEPSEGSAPGTSTEPSEGSAPGSPAGSPTSTEEGTSTEPSEGSAPGTSESATPESGPGSEPATS GSETPGTSESATPESGPGSEPATSGSETPGTSESATPESGPGTSTEPSEGSAPGTSESATPESGPG SPAGSPTSTEEGSPAGSPTSTEEGSPAGSPTSTEEGTSESATPESGPGTSTEPSEGSAPGTSESATP ESGPGSEPATSGSETPGTSESATPESGPGSEPATSGSETPGTSESATPESGPGTSTEPSEGSAPGS PAGSPTSTEEGTSESATPESGPGSEPATSGSETPGTSESATPESGPGSPAGSPTSTEEGSPAGSPT STEEGTSTEPSEGSAPGTSESATPESGPGTSESATPESGPGTSESATPESGPGSEPATSGSETPGS EPATSGSETPGSPAGSPTSTEEGTSTE |
| GLP-2 variant 2-AG612A | HGDGSFSDEMNTILDNLAARDFINWLIQTKITDGSTGSPGTPGSGTASSSPGSSTPSGATGSPG ASPGTSSTGSPGTPGSGTASSSPGSSTPSGATGSPGSSPSASTGTGPGSSPSASTGTGPGSSTPSG ATGSPGSSTPSGATGSPGASPGTSSTGSPGASPGTSSTGSPGASPGTSSTGSPGSSPSASTGTGPGTPGSGTASSSPGASPGTS STGSPGASPGTSSTGSPGASPGTSSTGSPGSSTPSGATGSPGSSTPSGATGSPGASPGTSSTGSPG TPGSGTASSSPGSSTPSGATGSPGSSTPSGATGSPGSSTPSGATGSPGSSPSASTGTGPGASPGTS STGSPGASPGTSSTGSPGTPGSGTASSSPGASPGTSSTGSPGASPGTSSTGSPGASPGTSSTGSPG ASPGTSSTGSPGTPGSGTASSSPGSSTPSGATGSPGTPGSGTASSSPGSSTPSGATGSPGTPGSGT ASSSPGSSTPSGATGSPGSSTPSGATGSPGSSPSASTGTGPGSSPSASTGTGPGASPGTSSTGSPG TPGSGTASSSPGSSTPSGATGSPGSSPSASTGTGPGSSPSASTGTGPGASPGTS |

(continued)

| GLP2-XTEN Name* | Amino Acid Sequence |
|---|---|
| GLP-2 variant 2-AG792A | HGDGSFSDEMNTILDNLAARDFINWLIQTKITDGTSSTGSPGSSPSASTGTGPGSSPSASTGTGPGTPGSGTASSSPGSSTPSGATGSPGSSPSASTGTGPGASPGTSSTGSPGTPGSGTASSSPGSSTPSGATGSPGTPGSGTASSSPGASPGTSSTGSPGASPGTSSTGSPGTPGSGTASSSPGSSTPSGATGSPGASPGTSSTGSPGTPGSGTASSSPGSSTPSGATGSPGSSPSASTGTGPGSSPSASTGTGPGSSTPSGATGSPGSSTPSGATGSPGASPGTSSTGSPGASPGTSSTGSPGASPGTSSTGSPGTPGSGTASSSPGASPGTSSTGSPGASPGTSSTGSPGASPGTSSTGSPGSSPSASTGTGPGTPGSGTASSSPGASPGTSSTGSPGASPGTSSTGSPGSSTPSGATGSPGSSTPSGATGSPGASPGTSSTGSPGTPGSGTASSSPGSSTPSGATGSPGSSTPSGATGSPGSSTPSGATGSPGSSPSASTGTGPGASPGTSSTGSPGASPGTSSTGSPGTPGSGTASSSPGASPGTSSTGSPGASPGTSSTGSPGASPGTSSTGSPGASPGTSSTGSPGTPGSGTASSSPGSSTPSGATGSPGTPGSGTASSSPGSSTPSGATGSPGSSTPSGATGSPGSSPSASTGTGPGSSPSASTGTGPGASPGTSSTGSPGTPGSGTASSSPGSSTPSGATGSPGSSPSASTGTGPGSSPSASTGTGPGASPGTSSTGSPGASPGTSSTGSPGSSTPSGATGSPGSSPSASTGTGPGASPG |
| * Sequence name reflects N- to C-terminus configuration of the GLP-2 and XTEN (by family name and length) | |

**Table 34: Exemplary GLP2-XTEN comprising GLP-2, cleavage sequences and XTEN sequences**

| GLP2-XTEN Name* | Amino Acid Sequence |
|---|---|
| GLP2-Thrombin-AD836 | HADGSFSDEMNTILDNLAARDFINWLIQTKITDGLTPRSLLVGGGGSSESGSSEGGPGSSESGS SEGGPGESPGGSSGSESGSGGEPSESGSSGESPGGSSGSESGESPGGSSGSESGSSESGSSEGGPG SSESGSSEGGPGSSESGSSEGGPGESPGGSSGSESGESPGGSSGSESGESPGGSSGSESGSSESGS SEGGPGSSESGSSEGGPGSSESGSSEGGPGSSESGSSEGGPGSSESGSSEGGPGSSESGSSEGGPG SGGEPSESGSSGESPGGSSGSESGESPGGSSGSESGSGGEPSESGSSGSEGSSGPGESSGSSESGS SEGGPGSGGEPSESGSSGSEGSSGPGESSGSSESGSSEGGPGSGGEPSESGSSGESPGGSSGSESG SGGEPSESGSSGSGGEPSESGSSGSSESGSSEGGPGSGGEPSESGSSGSGGEPSESGSSGSEGSSG PGESSGESPGGSSGSESGSEGSSGPGESSGSEGSSGPGESSGSGGEPSESGSSGSSESGSSEGGPG SSESGSSEGGPGESPGGSSGSESGSGGEPSESGSSGSEGSSGPGESSGESPGGSSGSESGSEGSSG PGSSESGSSEGGPGSGGEPSESGSSGSEGSSGPGESSGSEGSSGPGESSGSEGSSGPGESSGSGGE PSESGSSGSGGEPSESGSSGESPGGSSGSESGESPGGSSGSESGSGGEPSESGSSGSEGSSGPGES SGESPGGSSGSESGSSESGSSEGGPGSSESGSSEGGPGSSESGSSEGGPGSGGEPSESGSSGSSES GSSEGGPGESPGGSSGSESGSGGEPSESGSSGSSESGSSEGGPGESPGGSSGSESGSGGEPSESGS SGESPGGSSGSESGSGGEPSESGSS |
| GLP2-FXIa-AE864 | HADGSFSDEMNTILDNLAARDFINWLIQTKITDGGGKLTRVVGGGGSPAGSPTSTEEGTSESA TPESGPGTSTEPSEGSAPGSPAGSPTSTEEGTSTEPSEGSAPGTSTEPSEGSAPGTSESATPESGP GSEPATSGSETPGSEPATSGSETPGSPAGSPTSTEEGTSESATPESGPGTSTEPSEGSAPGTSTEPS |
| | EGSAPGSPAGSPTSTEEGTSTEPSEGSAPGTSTEPSEGSAPGTSESATPESGPGTSTEPSEGSAPG TSESATPESGPGSEPATSGSETPGTSTEPSEGSAPGTSTEPSEGSAPGTSESATPESGPGTSESATP ESGPGSPAGSPTSTEEGTSESATPESGPGSEPATSGSETPGTSESATPESGPGTSTEPSEGSAPGT STEPSEGSAPGTSTEPSEGSAPGTSTEPSEGSAPGTSTEPSEGSAPGSPAGSPTS TEEGTSTEPSEGSAPGTSESATPESGPGSEPATSGSETPGTSESATPESGPGSEPATSGSETPGTS ESATPESGPGTSTEPSEGSAPGTSESATPESGPGSPAGSPTSTEEGSPAGSPTSTEEGSPAGSPTS TEEGTSESATPESGPGTSTEPSEGSAPGTSESATPESGPGSEPATSGSETPGTSESATPESGPGSE PATSGSETPGTSESATPESGPGTSTEPSEGSAPGSPAGSPTSTEEGTSESATPESGPGSEPATSGS ETPGTSESATPESGPGSPAGSPTSTEEGSPAGSPTSTEEGTSTEPSEGSAPGTSESATPESGPGTS ESATPESGPGTSESATPESGPGSEPATSGSETPGSEPATSGSETPGSPAGSPTSTEEGTSTEPSEGS APGTSTEPSEGSAPGSEPATSGSETPGTSESATPESGPGTSTEPSEGSAPG |

(continued)

| GLP2-XTEN Name* | Amino Acid Sequence |
|---|---|
| GLP2-Elastase-AF864 | HADGSFSDEMNTILDNLAARDFINWLIQTKITDGGGLGPVSGVPGGSTSESPSGTAPGTSPSGE SSTAPGSTSESPSGTAPGSTSESPSGTAPGTSTPESGSASPGTSTPESGSASPGSTSESPSGTAPGS TSESPSGTAPGTSPSGESSTAPGSTSESPSGTAPGTSPSGESSTAPGTSPSGESSTAPGSTSSTAES PGPGTSPSGESSTAPGTSPSGESSTAPGSTSSTAESPGPGTSTPESGSASPGTSTPESGSASPGSTS ESPSGTAPGSTSESPSGTAPGTSTPESGSASPGSTSSTAESPGPGTSTPESGSASPGSTSESPSGTA PGTSPSGESSTAPGSTSSTAESPGPGTSPSGESSTAPGTSTPESGSASPGSTSSTAESPGPGTSSST AESPGPGTSSTAESPGPGTSSTAESPGPGTSPSGESSTAPGSTSESPSGTAPGSTSESPSGTAPG TSTPESGPXXXGASASGAPSTXXXXSESPSGTAPGSTSESPSGTAPGSTSESPSGTAPGSTSESPS GTAPGSTSESPSGTAPGSTSESPSGTAPGTSTPESGSASPGTSPSGESSTAPGTSPSGESSTAPGST SSTAESPGPGTSPSGESSTAPGTSTPESGSASPGSTSESPSGTAPGSTSESPSGTAPGTSPSGESST APGSTSESPSGTAPGTSTPESGSASPGTSTPESGSASPGSTSESPSGTAPGTSTPESGSASPGSTSS TAESPGPGTSESPSGTAPGSTSESPSGTAPGTSPSGESSTAPGSTSSTAESPGPGTSPSGESSTAP GTSTPESGSASPGTSPSGESSTAPGTSPSGESSTAPGTSPSGESSTAPGSTSSTAESPGPGTSSTA ESPGPGTSPSGESSTAPGSSPSASTGTGPGSSTPSGATGSPGSSTPSGATGSP |
| GLP2-MMP-17-AG864 | HADGSFSDEMNTILDNLAARDFINWLIQTKITDGAPLGLRLRGGGGASPGTSSTGSPGSSPSAS TGTGPGSSPSASTGTGPGTPGSGTASSSPGSSTPSGATGSPGSNPSASTGTGPGASPGTSSTGSP GTPGSGTASSSPGSSTPSGATGSPGTPGSGTASSSPGASPGTSSTGSPGASPGTSSTGSPGTPGSG TASSSPGSSTPSGATGSPGASPGTSSTGSPGTPGSGTASSSPGSSTPSGATGSPGSNPSASTGTGP GSSPSASTGTGPGSSTPSGATGSPGSSTPSGATGSPGASPGTSSTGSPGASPGTSSTGSPGASPGT SSTGSPGTPGSGTASSSPGASPGTSSTGSPGASPGTSSTGSPGASPGTSSTGSPGSSPSASTGTGP GTPGSGTASSSPGASPGTSSTGSPGASPGTSSTGSPGASPGTSSTGSPGSSTPSGATGSPGSSTPS GATGSPGASPGTSSTGSPGTPGSGTASSSPGSSTPSGATGSPGSSTPSGATGSPGSSTPSGATGSP GSSPSASTGTGPGASPGTSSTGSPGASPGTSSTGSPGTPGSGTASSSPGASPGTSSTGSPGASPGT SSTGSPGASPGTSSTGSPGTPGSGTASSSPGSSTPSGATGSPGTPGSGTASSSP GSSTPSGATGSPGTPGSGTASSSPGSSTPSGATGSPGSSTPSGATGSPGSSPSASTGTGPGSSPSA STGTGPGASPGTSSTGSPGTPGSGTASSSPGSSTPSGATGSPGSSPSASTGTGPGSSPSASTGTGP GASPGTSSTGSPGASPGTSSTGSPGSSTPSGATGSPGSSPSASTGTGPGASPGTSSTGSPGSSPSA STGTGPGTPGSGTASSSPGSSTPSGATGSPGSSTPSGATGSPGASPGTSSTGSP |

| GLP2-XTEN Name* | Amino Acid Sequence |
|---|---|
| GLP-2 variant 2-Thrombin-AD836 | HGDGSFSDEMNTILDNLAARDFINWLIQTKITDGLTPRSLLVGGGGSSESGSSEGGPGSSESGS SEGGPGESPGGSSGSESGSGGEPSESGSSGESPGGSSGSESGESPGGSSGSESGSSESGSSEGGPG SSESGSSEGGPGSSESGSSEGGPGESPGGSSGSESGESPGGSSGSESGESPGGSSGSESGSSESGS SEGGPGSSESGSSEGGPGSSESGSSEGGPGSSESGSSEGGPGSSESGSSEGGPGSSESGSSEGGPG SGGEPSESGSSGESPGGSSGSESGESPGGSSGSESGSGGEPSESGSSGSEGSSGPGESSGSSESGS SEGGPGSGGEPSESGSSGSEGSSGPGESSGSSESGSSEGGPGSGGEPSESGSSGESPGGSSGSESG SGGEPSESGSSGSGGEPSESGSSGSSESGSSEGGPGSGGEPSESGSSGSGGEPSESGSSGSEGSSG PGESSGESPGGSSGSESGSEGSSGPGESSGSEGSSGPGESSGSGGEPSESGSSGSSESGSSEGGPG SSESGSSEGGPGESPGGSSGSESGSGGEPSESGSSGSEGSSGPGESSGESPGGSSGSESGSEGSSG PGSSESGSSEGGPGSGGEPSESGSSGSEGSSGPGESSGSEGSSGPGESSGSEGSSGPGESSGSGGE PSESGSSGSGGEPSESGSSGESPGGSSGSESGESPGGSSGSESGSGGEPSESGSSGSEGSSGPGES SGESPGGSSGSESGSSESGSSEGGPGSSESGSSEGGPGSSESGSSEGGPGSGGEPSESGSSGSSES GSSEGGPGESPGGSSGSESGSGGEPSESGSSGSSESGSSEGGPGESPGGSSGSESGSGGEPSESGS SGESPGGSSGSESGSGGEPSESGSS |
| GLP-2 variant 2-FXIa-AE864 | HGDGSFSDEMNTILDNLAARDFINWLIQTKITDGGGKLTRVVGGGGSPAGSPTSTEEGTSESA TPESGPGTSTEPSEGSAPGSPAGSPTSTEEGTSTEPSEGSAPGTSTEPSEGSAPGTSESATPESGP GSEPATSGSETPGSEPATSGSETPGSPAGSPTSTEEGTSESATPESGPGTSTEPSEGSAPGTSTEPS EGSAPGSPAGSPTSTEEGTSTEPSEGSAPGTSTEPSEGSAPGTSESATPESGPGTSTEPSEGSAPG TSESATPESGPGSEPATSGSETPGTSTEPSEGSAPGTSTEPSEGSAPGTSESATPESGPGTSESATP |
|  | ESGPGSPAGSPTSTEEGTSESATPESGPGSEPATSGSETPGTSESATPESGPGTSTEPSEGSAPGT STEPSEGSAPGTSTEPSEGSAPGTSTEPSEGSAPGTSTEPSEGSAPGTSTEPSEGSAPGSPAGSPTS TEEGTSTEPSEGSAPGTSESATPESGPGSEPATSGSETPGTSESATPESGPGSEPATSGSETPGTS ESATPESGPGTSTEPSEGSAPGTSESATPESGPGSPAGSPTSTEEGSPAGSPTSTEEGSPAGSPTS TEEGTSESATPESGPGTSTEPSEGSAPGTSESATPESGPGSEPATSGSETPGTSESATPESGPGSE PATSGSETPGTSESATPESGPGTSTEPSEGSAPGSPAGSPTSTEEGTSESATPESGPGSEPATSGS ETPGTSESATPESGPGSPAGSPTSTEEGSPAGSPTSTEEGTSTEPSEGSAPGTSESATPESGPGTS ESATPESGPGTSESATPESGPGSEPATSGSETPGSEPATSGSETPGSPAGSPTSTEEGTSTEPSEGS APGTSTEPSEGSAPGSEPATSGSETPGTSESATPESGPGTSTEPSEGSAPG |

EP 3 431 098 A1

| GLP2-XTEN Name* | Amino Acid Sequence |
|---|---|
| GLP-2 variant 2-Elastase-AF864 | HGDGSFSDEMNTILDNLAARDFINWLIQTKITDGGGLGPVSGVPGGSTSESPSGTAPGTSPSGE SSTAPGSTSESPSGTAPGSTSESPSGTAPGTSTPESGSASPGTSTPESGSASPGSTSESPSGTAPGS TSESPSGTAPGTSPSGESSTAPGSTSESPSGTAPGTSPSGESSTAPGTSPSGESSTAPGSTSSTAES PGPGTSPSGESSTAPGTSPSGESSTAPGSTSSTAESPGPGTSTPESGSASPGTSTPESGSASPGSTS ESPSGTAPGSTSESPSGTAPGTSTPESGSASPGSTSSTAESPGPGTSTPESGSASPGSTSESPSGTA PGTSPSGESSTAPGSTSSTAESPGPGTSPSGESSTAPGTSTPESGSASPGSTSSTAESPGPGTSSST AESPGPGTSSTAESPGPGTSSTAESPGPGTSPSGESSTAPGSTSESPSGTAPGSTSESPSGTAPG TSTPESGPXXXGASASGAPSTXXXXSESPSGTAPGSTSESPSGTAPGSTSESPSGTAPGSTSESPS GTAPGSTSESPSGTAPGSTSESPSGTAPGTSTPESGSASPGTSPSGESSTAPGTSPSGESSTAPGST SSTAESPGPGTSPSGESSTAPGTSTPESGSASPGSTSESPSGTAPGSTSESPSGTAPGTSPSGESST APGSTSESPSGTAPGTSTPESGSASPGTSTPESGSASPGSTSESPSGTAPGTSTPESGSASPGSTSS TAESPGPGTSESPSGTAPGSTSESPSGTAPGTSPSGESSTAPGSTSSTAESPGPGTSPSGESSTAP GTSTPESGSASPGTSPSGESSTAPGTSPSGESSTAPGTSPSGESSTAPGSTSSTAESPGPGTSSTA ESPGPGTSPSGESSTAPGSSPSASTGTGPGSSTPSGATGSPGSSTPSGATGSP |
| GLP-2 variant 2-MMP-17-AG864 | HGDGSFSDEMNTILDNLAARDFINWLIQTKITDGAPLGLRLRGGGGASPGTSSTGSPGSSPSAS TGTGPGSSPSASTGTGPGTPGSGTASSSPGSSTPSGATGSPGSNPSASTGTGPGASPGTSSTGSP GTPGSGTASSSPGSSTPSGATGSPGTPGSGTASSSPGASPGTSSTGSPGASPGTSSTGSPGTPGSG TASSSPGSSTPSGATGSPGASPGTSSTGSPGTPGSGTASSSPGSSTPSGATGSPGSNPSASTGTGP GSSPSASTGTGPGSSTPSGATGSPGSSTPSGATGSPGASPGTSSTGSPGASPGTSSTGSPGASPGT SSTGSPGTPGSGTASSSPGASPGTSSTGSPGASPGTSSTGSPGASPGTSSTGSPGSSPSASTGTGP GTPGSGTASSSPGASPGTSSTGSPGASPGTSSTGSPGASPGTSSTGSPGSSTPSGATGSPGSSTPS GATGSPGASPGTSSTGSPGTPGSGTASSSPGSSTPSGATGSPGSSTPSGATGSPGSSTPSGATGSP GSSPSASTGTGPGASPGTSSTGSPGASPGTSSTGSPGTPGSGTASSSPGASPGTSSTGSPGASPGT SSTGSPGASPGTSSTGSPGTPGSGTASSSPGSSTPSGATGSPGTPGSGTASSSP GSSTPSGATGSPGTPGSGTASSSPGSSTPSGATGSPGSSTPSGATGSPGSSPSASTGTGPGSSPSA STGTGPGASPGTSSTGSPGTPGSGTASSSPGSSTPSGATGSPGSSPSASTGTGPGSSPSASTGTGP GASPGTSSTGSPGASPGTSSTGSPGSSTPSGATGSPGSSPSASTGTGPGASPGTSSTGSPGSSPSA STGTGPGTPGSGTASSSPGSSTPSGATGSPGSSTPSGATGSPGASPGTSSTGSP |

EP 3 431 098 A1

201

| GLP2-XTEN Name* | Amino Acid Sequence |
|---|---|
| AE912-Thrombin-GLP2 | MAEPAGSPTSTEEGTPGSGTASSSPGSSTPSGATGSPGASPGTSSTGSPGSPAGSPTSTEEGTSES ATPESGPGTSTEPSEGSAPGSPAGSPTSTEEGTSTEPSEGSAPGTSTEPSEGSAPGTSESATPESG PGSEPATSGSETPGSEPATSGSETPGSPAGSPTSTEEGTSESATPESGPGTSTEPSEGSAPGTSTEP SEGSAPGSPAGSPTSTEEGTSTEPSEGSAPGTSTEPSEGSAPGTSESATPESGPGTSTEPSEGSAP GTSESATPESGPGSEPATSGSETPGTSTEPSEGSAPGTSTEPSEGSAPGTSESATPESGPGTSESA TPESGPGSPAGSPTSTEEGTSESATPESGPGSEPATSGSETPGTSESATPESGPGTSTEPSEGSAP GTSTEPSEGSAPGTSTEPSEGSAPGTSTEPSEGSAPGTSTEPSEGSAPGTSTEPSEGSAPGSPAGS PTSTEEGTSTEPSEGSAPGTSESATPESGPGSEPATSGSETPGTSESATPESGPGSEPATSGSETP GTSESATPESGPGTSTEPSEGSAPGTSESATPESGPGSPAGSPTSTEEGSPAGSPTSTEEGSPAGS PTSTEEGTSESATPESGPGTSTEPSEGSAPGTSESATPESGPGSEPATSGSETPGTSESATPESGP GSEPATSGSETPGTSESATPESGPGTSTEPSEGSAPGSPAGSPTSTEEGTSESATPESGPGSEPAT SGSETPGTSESATPESGPGSPAGSPTSTEEGSPAGSPTSTEEGTSTEPSEGSAPGTSESATPESGP GTSESATPESGPGTSESATPESGPGSEPATSGSETPGSEPATSGSETPGSPAGSPTSTEEGTSTEPS EGSAPGTSTEPSEGSAPGSEPATSGSETPGTSESATPESGPGTSTEPSEGSAPGLTPRSLLVGGG HADGSFSDEMNTILDNLAARDFINWLIQTKITD |
| AE912-FXIa-GLP-2 variant 2 | MAEPAGSPTSTEEGTPGSGTASSSPGSSTPSGATGSPGASPGTSSTGSPGSPAGSPTSTEEGTSES ATPESGPGTSTEPSEGSAPGSPAGSPTSTEEGTSTEPSEGSAPGTSTEPSEGSAPGTSESATPESG PGSEPATSGSETPGSEPATSGSETPGSPAGSPTSTEEGTSESATPESGPGTSTEPSEGSAPGTSTEP SEGSAPGSPAGSPTSTEEGTSTEPSEGSAPGTSTEPSEGSAPGTSESATPESGPGTSTEPSEGSAP GTSESATPESGPGSEPATSGSETPGTSTEPSEGSAPGTSTEPSEGSAPGTSESATPESGPGTSESA TPESGPGSPAGSPTSTEEGTSESATPESGPGSEPATSGSETPGTSESATPESGPGTSTEPSEGSAP |
|  | GTSTEPSEGSAPGTSTEPSEGSAPGTSTEPSEGSAPGTSTEPSEGSAPGTSTEPSEGSAPGSPAGS PTSTEEGTSTEPSEGSAPGTSESATPESGPGSEPATSGSETPGTSESATPESGPGSEPATSGSETP GTSESATPESGPGTSTEPSEGSAPGTSESATPESGPGSPAGSPTSTEEGSPAGSPTSTEEGSPAGS PTSTEEGTSESATPESGPGTSTEPSEGSAPGTSESATPESGPGSEPATSGSETPGTSESATPESGP GSEPATSGSETPGTSESATPESGPGTSTEPSEGSAPGSPAGSPTSTEEGTSESATPESGPGSEPAT SGSETPGTSESATPESGPGSPAGSPTSTEEGSPAGSPTSTEEGTSTEPSEGSAPGTSESATPESGP GTSESATPESGPGTSESATPESGPGSEPATSGSETPGSEPATSGSETPGSPAGSPTSTEEGTSTEPS EGSAPGTSTEPSEGSAPGSEPATSGSETPGTSESATPESGPGTSTEPSEGSAPGGGKLTRVVGGG HGDGSFSDEMNTILDNLAARDFINWLIQTKITD |

EP 3 431 098 A1

| GLP2-XTEN Name* | Amino Acid Sequence |
|---|---|
| AE912-Elastase-GLP-2 variant 2 | MAEPAGSPTSTEEGTPGSGTASSSPGSSTPSGATGSPGASPGTSSTGSPGSPAGSPTSTEEGTSES ATPESGPGTSTEPSEGSAPGSPAGSPTSTEEGTSTEPSEGSAPGTSTEPSEGSAPGTSESATPESG PGSEPATSGSETPGSEPATSGSETPGSPAGSPTSTEEGTSESATPESGPGTSTEPSEGSAPGTSTEP SEGSAPGSPAGSPTSTEEGTSTEPSEGSAPGTSTEPSEGSAPGTSESATPESGPGTSTEPSEGSAP GTSESATPESGPGSEPATSGSETPGTSTEPSEGSAPGTSTEPSEGSAPGTSESATPESGPGTSESA TPESGPGSPAGSPTSTEEGTSESATPESGPGSEPATSGSETPGTSESATPESGPGTSTEPSEGSAP GTSTEPSEGSAPGTSTEPSEGSAPGTSTEPSEGSAPGTSTEPSEGSAPGTSTEPSEGSAPGSPAGS PTSTEEGTSTEPSEGSAPGTSESATPESGPGSEPATSGSETPGTSESATPESGPGSEPATSGSETP GTSESATPESGPGTSTEPSEGSAPGTSESATPESGPGSPAGSPTSTEEGSPAGSPTSTEEGSPAGS PTSTEEGTSESATPESGPGTSTEPSEGSAPGTSESATPESGPGSEPATSGSETPGTSESATPESGP GSEPATSGSETPGTSESATPESGPGTSTEPSEGSAPGSPAGSPTSTEEGTSESATPESGPGSEPAT SGSETPGTSESATPESGPGSPAGSPTSTEEGSPAGSPTSTEEGTSTEPSEGSAPGTSESATPESGP GTSESATPESGPGTSESATPESGPGSEPATSGSETPGSEPATSGSETPGSPAGSPTSTEEGTSTEPS EGSAPGTSTEPSEGSAPGSEPATSGSETPGTSESATPESGPGTSTEPSEGSAPGGGLGPVSGVPG HGDGSFSDEMNTILDNLAARDFINWLIQTKITD |
| GLP-2 variant 2-MMP-17-AE864 | HGDGSFSDEMNTILDNLAARDFINWLIQTKITDGAPLGLRLRGGGGSPAGSPTSTEEGTSESAT PESGPGTSTEPSEGSAPGSPAGSPTSTEEGTSTEPSEGSAPGTSTEPSEGSAPGTSESATPESGPG SEPATSGSETPGSEPATSGSETPGSPAGSPTSTEEGTSESATPESGPGTSTEPSEGSAPGTSTEPSE GSAPGSPAGSPTSTEEGTSTEPSEGSAPGTSTEPSEGSAPGTSESATPESGPGTSTEPSEGSAPGT SESATPESGPGSEPATSGSETPGTSTEPSEGSAPGTSTEPSEGSAPGTSESATPESGPGTSESATPE SGPGSPAGSPTSTEEGTSESATPESGPGSEPATSGSETPGTSESATPESGPGTSTEPSEGSAPGTS TEPSEGSAPGTSTEPSEGSAPGTSTEPSEGSAPGTSTEPSEGSAPGTSTEPSEGSAPGSPAGSPTS TEEGTSTEPSEGSAPGTSESATPESGPGSEPATSGSETPGTSESATPESGPGSEPATSGSETPGTS ESATPESGPGTSTEPSEGSAPGTSESATPESGPGSPAGSPTSTEEGSPAGSPTSTEEGSPAGSPTS TEEGTSESATPESGPGTSTEPSEGSAPGTSESATPESGPGSEPATSGSETPGTSESATPESGPGSE PATSGSETPGTSESATPESGPGTSTEPSEGSAPGSPAGSPTSTEEGTSESATPESGPGSEPATSGS ETPGTSESATPESGPGSPAGSPTSTEEGSPAGSPTSTEEGTSTEPSEGSAPGTSESATPESGPGTS ESATPESGPGTSESATPESGPGSEPATSGSETPGSEPATSGSETPGSPAGSPTSTEEGTSTEPSEGS APGTSTEPSEGSAPGSEPATSGSETPGTSESATPESGPGTSTEPSEGSAPG |

* Sequence name reflects N- to C-terminus configuration of the GLP-2, XTEN (by family name and length) and cleavage sequence denoted by protease name active on the sequence.

203

EP 3 431 098 A1

The following numbered paragraphs set out preferred features and preferred combinations of features of the present invention

1. A recombinant fusion protein comprising a glucagon-like protein-2 (GLP-2) and an extended recombinant polypeptide (XTEN), wherein the XTEN is characterized in that:

(a) the XTEN comprises at least 36 amino acid residues;
(b) the sum of glycine (G), alanine (A), serine (S), threonine (T), glutamate (E) and proline (P) residues constitutes more than about 80% of the total amino acid residues of the XTEN;
(c) the XTEN is substantially non-repetitive such that (i) the XTEN contains no three contiguous amino acids that are identical unless the amino acids are serine; (ii) at least about 80% of the XTEN sequence consists of non-overlapping sequence motifs, each of the sequence motifs comprising about 9 to about 14 amino acid residues consisting of four to six amino acids selected from glycine (G), alanine (A), serine (S), threonine (T), glutamate (E) and proline (P), wherein any two contiguous amino acid residues do not occur more than twice in each of the non-overlapping sequence motifs; or (iii) the XTEN sequence has a subsequence score of less than 10;
(d) the XTEN has greater than 90% random coil formation as determined by GOR algorithm;
(e) the XTEN has less than 2% alpha helices and 2% beta-sheets as determined by Chou-Fasman algorithm; and
(f) the XTEN lacks a predicted T-cell epitope when analyzed by TEPITOPE algorithm, wherein the TEPITOPE threshold score for said prediction by said algorithm has a threshold of -9, wherein said fusion protein exhibits an apparent molecular weight factor of at least about 4 and exhibits an intestinotrophic effect when administered to a subject using a therapeutically effective amount.

2. The recombinant fusion protein of para 1, wherein the intestinotrophic effect is at least about 30%, or at least about 40%, or at least about 50%, or at least about 60%, or at least about 70%, or at least about 80%, or at least about 90%, or at least about 100% or at least about 120% or at least about 150% or at least about 200% of the intestinotrophic effect compared to the corresponding GLP-2 not linked to XTEN upon administration of said corresponding GLP-2 to a subject using comparable dose.

3. The recombinant fusion protein of para 1, wherein the subject is selected from the group consisting of mouse, rat, monkey, and human.

4. The recombinant fusion protein of any one of the preceding paras wherein said administration is subcutaneous, intramuscular, or intravenous.

5. The recombinant fusion protein of any one of the preceding paras, wherein the intestinotrophic effect is determined after administration of 1 dose, or 3 doses, or 6 doses, or 10 doses, or 12 or more doses of the fusion protein.

6. The recombinant fusion protein of any one of the preceding paras, wherein the intestinotrophic effect is selected from the group consisting of intestinal growth, increased hyperplasia of the villus epithelium, increased crypt cell proliferation, increased height of the crypt and villus axis, increased healing after intestinal anastomosis, increased small bowel weight, increased small bowel length, decreased small bowel epithelium apoptosis, and enhancement of intestinal function.

7. The recombinant fusion protein of para 6, wherein the administration results in an increase in small intestine weight of at least about 10%, or at least about 20%, or at least about 30%.

8. The recombinant fusion protein of para 6, wherein the administration results in an increase in small intestine length of at least about 5%, or at least about 6%, or at least about 7%, or at least about 8%, or at least about 9%, or at least about 10%, or at least about 20%, or at least about 30%.

9. The recombinant fusion protein of para any one of the preceding claims, wherein the GLP-2 sequence has at least 90%, or at least about 91%, or at least about 92%, or at least about 93%, or at least about 94%, or at least about 95%, or at least about 96%, or at least about 97%, or at least about 98%, or at least about 99%, or about 100% sequence identity to a sequence selected from the group consisting of the sequences in Table 1, when optimally aligned.

10. The recombinant fusion protein of any one of the preceding paras, wherein the GLP-2 comprises human GLP-2.

11. The recombinant fusion protein of any one of paras 9-11, wherein the GLP-2 is selected from the group consisting of bovine GLP-2, pig GLP-2, sheep GLP-2, chicken GLP-2, and canine GLP-2.

12. The recombinant fusion protein of any one of the preceding paras, wherein the GLP-2 has an amino acid substitution in place of Ala$^2$, and wherein the substitution is glycine.

13. The recombinant fusion protein of any one of paras 1-9, wherein the GLP-2 has the sequence HGDGSFSDEMNTILDNLAARDFINWLIQTKITD.

14. The recombinant fusion protein any one of the preceding paras, wherein the XTEN is linked to the C-terminus of the GLP-2.

15. The recombinant fusion protein of para 14, further comprising a spacer sequence of 1 to about 50 amino acid residues linking the GLP-2 and XTEN.

16. The recombinant fusion protein of para 15, wherein the spacer sequence comprises a glycine residue.

17. The recombinant fusion protein of any one of the preceding paras, wherein the XTEN is characterized in that:

   (a) the total XTEN amino acid residues are at least 36 to about 3000 amino acid residues; and
   (b) the sum of glycine (G), alanine (A), serine (S), threonine (T), glutamate (E) and proline (P) residues constitutes at least about 90% of the total amino acid residues of the XTEN.

18. The recombinant fusion protein of any one of the preceding paras, wherein the XTEN is characterized in that the sum of asparagine and glutamine residues is less than 10% of the total amino acid sequence of the XTEN.

19. The recombinant fusion protein of any one of the preceding paras, wherein the XTEN is characterized in that the sum of methionine and tryptophan residues is less than 2% of the total amino acid residues of the XTEN.

20. The recombinant fusion protein any one of the preceding paras, wherein the XTEN has at least 90%, or at least about 91%, or at least about 92%, or at least about 93%, or at least about 94%, or at least about 95%, or at least about 96%, or at least about 97%, or at least about 98%, or at least about 99%, or 100% sequence identity when compared to a sequence of comparable length selected from any one of Table 4, Table 8, Table 9, Table 10, Table 11, and Table 12, when optimally aligned.

21. The recombinant fusion protein any one of the preceding paras, wherein the XTEN has at least 90%, or at least about 91%, or at least about 92%, or at least about 93%, or at least about 94%, or at least about 95%, or at least about 96%, or at least about 97%, or at least about 98%, or at least about 99%, or 100% sequence identity when compared to an AE864 sequence from Table 4, when optimally aligned.

22. The recombinant fusion protein of any one of paras 1-9 or 13, wherein the fusion protein sequence has a sequence with at least 90%, or at least about 91%, or at least about 92%, or at least about 93%, or at least about 94%, or at least about 95%, or at least about 96%, or at least about 97%, or at least about 98%, or at least about 99%, or 100% sequence identity to the sequence set forth in FIG. 28.

23. The recombinant fusion protein of any one of the preceding paras, wherein the fusion protein exhibits a terminal half-life that is at least about 30 hours when administered to a subject.

24. The recombinant fusion protein of any one of the preceding paras, wherein the fusion protein binds to a GLP-2 receptor with an $EC_{50}$ of less than about 30 nM, or about 100 nM, or about 200 nM, or about 300 nM, or about 370 nM, or about 400 nM, or about 500 nM, or about 600 nM, or about 700 nM, or about 800 nM, or about 1000 nM, or about 1200 nM, or about 1400 nM when assayed using an *in vitro* GLP2R cell assay wherein the GLP2R cell is a human recombinant GLP-2 glucagon family receptor calcium-optimized cell.

25. The recombinant fusion protein of any one of the preceding paras, wherein the fusion protein retains at least about 1%, or about 2%, or about 3%, or about 4%, or about 5%, or about 10%, or about 20%, or about 30% of the potency of the corresponding GLP-2 not linked to XTEN when assayed using an *in vitro* GLP2R cell assay wherein the GLP2R cell is a human recombinant GLP-2 glucagon family receptor calcium-optimized cell.

26. The recombinant fusion protein of any one of the preceding paras, characterized in that

   (a) when an equivalent amount, in nmoles/kg, of the fusion protein and the corresponding GLP-2 that lacks the XTEN are each administered to comparable subjects, the fusion protein achieves a terminal half-life in the subject that is at least about 3-fold, or at least 4-fold, or at least 5-fold, or at least 10-fold, or at least 15-fold, or at least 20-fold longer compared to the corresponding GLP-2 that lacks the XTEN;
   (b) when a 2-fold, or 3-fold, or 4-fold, or 5-fold, or 6-fold smaller amount, in nmoles/kg, of the fusion protein than the corresponding GLP-2 that lacks the XTEN are each administered to comparable subjects with a gastrointestinal condition, the fusion protein achieves a comparable therapeutic effect in the subject as the corresponding GLP-2 that lacks the XTEN;
   (c) when the fusion protein is administered to a subject in consecutive dosesusing a dose interval that is at least about 2-fold, or at least 3-fold, or at least 4-fold, or at least 5-fold, or at least 10-fold, or at least 15-fold, or at least 20-fold longer as compared to a dose interval for the corresponding GLP-2 that lacks the XTEN and is administered to a comparable subject using an otherwise equivalent nmoles/kg amount, the fusion protein achieves a similar blood concentration in the subject as compared to the corresponding GLP-2 that lacks the XTEN; or
   (d) when the fusion protein is administered to a subject in consecutive doses using a dose interval that is at least about 3-fold, or at least 4-fold, or at least 5-fold, or at least 10-fold, or at least 15-fold, or at least 20-fold longer as compared to a dose interval for the corresponding GLP-2 that lacks the XTEN and is administered to a comparable subject using an otherwise equivalent nmoles/kg amount, the fusion protein achieves a comparable therapeutic effect in the subject as compared to the corresponding GLP-2 that lacks the XTEN.

27. The recombinant fusion protein of para 26, wherein the subject is selected from the group consisting of mouse, rat, monkey, and human.

28. The recombinant fusion protein of para 27, wherein the subject is rat.

29. The recombinant fusion protein of any one of paras 26-28, wherein the administration results in a greater pharmacologic effect compared to the effect observed with the corresponding GLP-2 not linked to XTEN.

30. The recombinant fusion protein of any one of paras 26-29, wherein administration of an effective amount the fusion protein results in a greater therapeutic effect in a subject with enteritis compared to administration of the corresponding GLP-2 not linked to XTEN to a comparable subject using a comparable nmoles/kg amount.

31. The recombinant fusion protein of any one of paras 26-30, wherein the subject is selected from the group consisting of mouse, rat, monkey, and human.

32. The recombinant fusion protein of para 31, wherein the subject is human and the enteritis is Crohn's disease.

33. The recombinant fusion protein of para 31, wherein the subject is rat subject and the enteritis is induced with indomethacin.

34. The recombinant fusion protein of any one of paras 29-33, wherein the greater therapeutic effect is selected from the group consisting of body weight gain, small intestine length, reduction in TNFα content of the small intestine tissue, reduced mucosal atrophy, reduced incidence of perforated ulcers, and height of villi.

35. The recombinant fusion protein of para 34, wherein the administration results in an increase in small intestine weight of at least about 10%, or at least about 20%, or at least about 30%, or at least about 40% greater compared to that of the corresponding GLP-2 not linked to XTEN.

36. The recombinant fusion protein of para 34, wherein the administration results in an increase in small intestine length of at least about 5%, or at least about 6%, or at least about 7%, or at least about 8%, or at least about 9%, or at least about 10%, or at least about 20%, or at least about 30%, or at least about 40% greater compared to that of the corresponding GLP-2 not linked to XTEN.

37. The recombinant fusion protein of para 34, wherein the administration results in an increase in body weight is at least about 5%, or at least about 6%, or at least about 7%, or at least about 8%, or at least about 9%, or at least about 10%, or at least about 20%, or at least about 30%, or at least about 40% greater compared to that of the corresponding GLP-2 not linked to XTEN.

38. The recombinant fusion protein of para 34, wherein the reduction in TNFα content is at least about 0.5 ng/g, or at least about 0.6 ng/g, or at least about 0.7 ng/g, or at least about 0.8 ng/g, or at least about 0.9 ng/g, or at least about 1.0 ng/g, or at least about 1.1 ng/g, or at least about 1.2 ng/g, or at least about 1.3 ng/g, or at least about 1.4 ng/g of small intestine tissue or greater compared to that of the corresponding GLP-2 not linked to XTEN.

39. The recombinant fusion protein of para 34, wherein the villi height is at least about 5%, or at least about 6%, or at least about 7%, or at least about 8%, or at least about 9%, or at least about 10%, or at least about 11%, or at least about 12% greater compared to that of the corresponding GLP-2 not linked to XTEN.

40. The recombinant fusion protein of any one of paras 29-39, wherein the fusion protein is administered as 1, or 2, or 3, or 4, or 5, or 6, or 10, or 12 or more consecutive doses.

41. The recombinant fusion protein of any one of paras 30-40, wherein the effective amount is at least about 5, or least about 10, or least about 25, or least about 100, or least about 200 nmoles/kg.

42. The recombinant fusion protein of any one of the preceding paras, wherein the GLP-2 is linked to the XTEN via a cleavage sequence that is cleavable by a mammalian protease selected from the group consisting of factor XIa, factor XIIa, kallikrein, factor VIIa, factor IXa, factor Xa, factor IIa (thrombin), Elastase-2, MMP-12, MMP13, MMP-17 and MMP-20, wherein cleavage at the cleavage sequence by the mammalian protease releases the GLP-2 sequence from the XTEN sequence, and wherein the released GLP-2 sequence exhibits an increase in receptor binding activity of at least about 30% compared to the uncleaved fusion protein.

43. A method of producing a fusion protein comprising GLP-2 fused to one or more extended recombinant polypeptides (XTEN), comprising:

(a) providing a host cell comprising a recombinant nucleic acid encoding the fusion protein of any one of claims 1 to 42;
(b) culturing the host cell under conditions permitting the expression of the fusion protein; and
(c) recovering the fusion protein.

44. The method of para 43, wherein:

(a) the host cell is a prokaryotic cell; or
(b) the fusion protein is recovered from the host cell cytoplasm in substantially soluble form.

45. The method of para 43, wherein the recombinant nucleic acid molecule has a sequence with at least 90%, or at least about 91%, or at least about 92%, or at least about 93%, or at least about 94%, or at least about 95%, or at least about 96%, or at least about 97%, or at least about 98%, or at least about 99%, or 100% sequence identity to a sequence selected from the group consisting of the DNA sequences set forth in Table 13, when optimally aligned, or the complement thereof.

46. An isolated nucleic acid comprising:

(a) a nucleic acid sequence that has at least 70%, or at least about 80%, or at least about 90%,or at least about 91%, or at least about 92%, or at least about 93%, or at least about 94%, or at least about 95%, or at least about 96%, or at least about 97%, or at least about 98%, or at least about 99%, or 100% sequence identity to a DNA sequence selected from Table 13, or the complement thereof; or
(b) a nucleotide sequence encoding the fusion protein of any one of paras 1 to 42, or the complement thereof.

47. An expression vector or isolated host cell comprising the nucleic acid of any one of 43-46.

48. A host cell comprising the expression vector of para 47.

49. A pharmaceutical composition comprising the fusion protein of any one of 1 to 42, and a pharmaceutically acceptable carrier.

50. The recombinant fusion protein of para 1 configured according to formula V:

$$(GLP\text{-}2)\text{-}(S)_x\text{-}(XTEN) \qquad (V)$$

wherein independently for each occurrence,

GLP-2 is a sequence having at least 90%, or at least about 91%, or at least about 92%, or at least about 93%, or at least about 94%, or at least about 95%, or at least about 96%, or at least about 97%, or at least about 98%, or at least about 99%, or about 100% sequence identity to a sequence selected from the group consisting of the sequences in Table 1, when optimally aligned;
S is a spacer sequence having between 1 to about 50 amino acid residues that can optionally include a cleavage sequence from Table 6 or amino acids compatible with restrictions sites; and
x is either 0 or 1.

51. The recombinant fusion protein of para 50, wherein the GLP-2 comprises human GLP-2.

52. The recombinant fusion protein of para 50, wherein the GLP-2 is selected from the group consisting of bovine GLP-2, pig GLP-2, sheep GLP-2, chicken GLP-2, and canine GLP-2.

53. The recombinant fusion protein of para 51 or para 52, wherein the GLP-2 has an amino acid substitution in place of $Ala^2$, and wherein the substitution is glycine.

54. The recombinant fusion protein of para 50, wherein the GLP-2 has the sequence HGDGSFSDEMNTILDNLAARDFINWLIQTKITD.

55. The recombinant fusion protein of any one of paras 50-54, comprising a spacer sequence wherein the spacer sequence comprises a glycine residue.

56. The recombinant fusion protein any one of paras 50-55, wherein the XTEN has at least 90%, or at least about 91%, or at least about 92%, or at least about 93%, or at least about 94%, or at least about 95%, or at least about 96%, or at least about 97%, or at least about 98%, or at least about 99%, or 100% sequence identity when compared to a sequence of comparable length selected from any one of Table 4, Table 8, Table 9, Table 10, Table 11, and Table 12, when optimally aligned.

57. The recombinant fusion protein any one of paras 50-55, wherein the XTEN has at least 90%, or at least about 91%, or at least about 92%, or at least about 93%, or at least about 94%, or at least about 95%, or at least about 96%, or at least about 97%, or at least about 98%, or at least about 99%, or 100% sequence identity when compared to a AE864 sequence from Table 4, when optimally aligned.

58. The pharmaceutical composition of para 49, wherein administration of a therapeutically effective amount of the pharmaceutical composition to a subject with a gastrointestinal condition results in maintaining blood concentrations of the fusion protein within a therapeutic window for the fusion protein at least three-fold longer compared to administration of the corresponding GLP-2 not linked to the XTEN and administered at a comparable amount to a subject.

59. The pharmaceutical composition of para 49, wherein administration of three or more doses of the pharmaceutical composition to a subject with a gastrointestinal condition using a therapeutically-effective dose regimen results in a gain in time of at least four-fold between at least two consecutive $C_{max}$ peaks and/or $C_{min}$ troughs for blood levels of the fusion protein compared to administration of the corresponding GLP-2 not linked to the XTEN and administered using a comparable dose regimen to a subj ect.

60. The pharmaceutical composition of para 59 or 60, wherein the gastrointestinal condition is selected from the group consisting of gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy- associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, and gastrointestinal ischemia.

61. The pharmaceutical composition of para 49, wherein after intravenous, subcutaneous, or intramuscular administration of the pharmaceutical composition comprising at least about 5, or least about 10, or least about 25, or least about 100, or least about 200 nmoles/kg of the fusion protein to a subject, the fusion protein blood levels are maintained above 1000 ng/ml for at least 72 hours.

62. The pharmaceutical composition of para 61, wherein the subject is selected from mouse, rat, monkey and human.

63. A recombinant fusion protein according to any one of paras 1 to 42 for use in the manufacture of a medicament for the treatment of a gastrointestinal condition.

64. The recombinant fusion protein of para 63 wherein the gastrointestinal condition is selected from the group consisting of gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy- associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, and gastrointestinal ischemia.

65. A recombinant fusion protein according to any one of paras 1 to 42 for use in a method of treating a gastrointestinal condition in a subject, comprising administering to the subject a therapeutically effective amount of the fusion protein.

66. The recombinant fusion protein for use according to para 65, wherein the gastrointestinal condition is selected from the group consisting of gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy- associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, and gastrointestinal ischemia.

67. The recombinant fusion protein for use according to para 65, wherein administration of two or more consecutive doses of the fusion protein administered using a therapeutically effective dose regimen to a subject results in a prolonged period between consecutive $C_{max}$ peaks and/or $C_{min}$ troughs for blood levels of the fusion protein compared to the corresponding GLP-2 that lacks the XTEN and administered using a therapeutically effective dose regimen established for the GLP-2.

68. The recombinant fusion protein for use according to para 65, wherein a smaller amount in nmoles/kg of the fusion protein is administered to a subject in comparison to the corresponding GLP-2 that lacks the XTEN administered to a subject under an otherwise equivalent dose regimen, and the fusion protein achieves a comparable therapeutic effect as the corresponding GLP-2 that lacks the XTEN.

69. The recombinant fusion protein for use according to para 68, wherein the therapeutic effect is selected from the group consisting of blood concentrations of GLP-2, increased mesenteric blood flow, decreased inflammation, increased weight gain, decreased diarrhea, decreased fecal wet weight, intestinal wound healing, increase in plasma citrulline concentrations, decreased CRP levels, decreased requirement for steroid therapy, enhancing or stimulating mucosal integrity, decreased sodium loss, minimizing, mitigating, or preventing bacterial translocation in the intestines, enhancing, stimulating or accelerating recovery of the intestines after surgery, preventing relapses of inflammatory bowel disease, and maintaining energy homeostasis.

70. A recombinant fusion protein for use in a pharmaceutical regimen for treatment of a gastrointestinal condition in a subject, said regimen comprising a pharmaceutical composition comprising the fusion protein of any one of paras 1 to 42.

71. The recombinant fusion protein of para 70, wherein the pharmaceutical regimen further comprises the step of determining the amount of pharmaceutical composition needed to achieve a therapeutic effect in the subject, wherein the therapeutic effect is selected from the group consisting of increased mesenteric blood flow, decreased inflammation, increased weight gain, decreased diarrhea, decreased fecal wet weight, intestinal wound healing, increase in plasma citrulline concentrations, decreased CRP levels, decreased requirement for steroid therapy, enhanced mucosal integrity, decreased sodium loss, preventing bacterial translocation in the intestines, accelerated recovery of the intestines after surgery, prevention of relapses of inflammatory bowel disease, and maintaining energy homeostasis.

72. The recombinant fusion protein of para 70, wherein the gastrointestinal condition is selected from the group consisting of gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy- associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, and gastrointestinal ischemia.

73. The recombinant fusion protein of para 70, wherein the pharmaceutical regimen for treating a subject with a gastrointestinal condition comprises administering the pharmaceutical composition in two or more successive doses to the subject at an effective amount, wherein the administration results in at least a 5%, or 10%, or 20%, or 30%, or 40%, or 50%, or 60%, or 70%, or 80%, or 90% greater improvement of at least one, two, or three parameters associated with the gastrointestinal condition compared to the GLP-2 not linked to XTEN and administered using a comparable nmol/kg amount.

74. The recombinant fusion protein of para 73, wherein the parameter improved is selected from increased blood concentrations of GLP-2, increased mesenteric blood flow, decreased inflammation, increased weight gain, decreased diarrhea, decreased fecal wet weight, intestinal wound healing, increase in plasma citrulline concentrations, decreased CRP levels, decreased requirement for steroid therapy, enhanced mucosal integrity, decreased sodium loss, preventing bacterial translocation in the intestines, accelerated recovery of the intestines after surgery, prevention of relapses of inflammatory bowel disease, and maintaining energy homeostasis.

75. The recombinant fusion protein of para 70, wherein the regimen comprises administering a therapeutically effective amount of the pharmaceutical composition of claim 49 once every 7, or 10, or 14, or 21, or 28 or more days.

76. The recombinant fusion protein of para 75, wherein the effective amount is at least about 5, or least about 10, or least about 25, or least about 100, or least about 200 nmoles/kg.

77. The recombinant fusion protein of any one of paras 73-76, wherein said administration is subcutaneous, intramuscular, or intravenous.

78. A method of treating a gastrointestinal condition in a subject, comprising administering to said subject a composition comprising an effective amount of the pharmaceutical composition of 49.

79. The method of para 78, wherein the effective amount is at least about 5, or least about 10, or least about 25, or least about 100, or least about 200 nmoles/kg.

80. The method of para 79, wherein the fusion protein exhibits a terminal half-life of greater than about 30 hours in said subject.

81. The method of any one of paras 78-80, wherein the gastrointestinal condition is selected from the group consisting of gastritis, digestion disorders, malabsorption syndrome, short-gut syndrome, short bowel syndrome, cul-de-sac syndrome, inflammatory bowel disease, celiac disease, tropical sprue, hypogammaglobulinemic sprue, Crohn's disease, ulcerative colitis, enteritis, chemotherapy-induced enteritis, irritable bowel syndrome, small intestine damage, small intestinal damage due to cancer-chemotherapy, gastrointestinal injury, diarrheal diseases, intestinal insufficiency, acid-induced intestinal injury, arginine deficiency, idiopathic hypospermia, obesity, catabolic illness, febrile neutropenia, diabetes, obesity, steatorrhea, autoimmune diseases, food allergies, hypoglycemia, gastrointestinal barrier disorders, sepsis, bacterial peritonitis, burn-induced intestinal damage, decreased gastrointestinal motility, intestinal failure, chemotherapy- associated bacteremia, bowel trauma, bowel ischemia, mesenteric ischemia, malnutrition, necrotizing enterocolitis, necrotizing pancreatitis, neonatal feeding intolerance, NSAID-induced gastrointestinal damage, nutritional insufficiency, total parenteral nutrition damage to gastrointestinal tract, neonatal nutritional insufficiency, radiation-induced enteritis, radiation-induced injury to the intestines, mucositis, pouchitis, and gastrointestinal ischemia.

82. The method of para 81, wherein the gastrointestinal condition is Crohn's disease.

83. The method of any one of paras 78-82, wherein the subject is selected from the group consisting of mouse, rat, monkey, and human.

84. The method of any one of paras 78-83, wherein said administration is subcutaneous, intramuscular, or intravenous.

85. The method of any one of paras 78-84, wherein said administration results in an intestinotrophic effect in said subject.

86. The method of para 85, wherein the intestinotrophic effect is at least about 30%, or at least about 40%, or at least about 50%, or at least about 60%, or at least about 70%, or at least about 80%, or at least about 90%, or at least about 100% or at least about 120% or at least about 150% or at least about 200% of the intestinotrophic effect compared to the corresponding GLP-2 not linked to XTEN and administered to a subject using a comparable dose.

87. The method of para 85 or 86, wherein the intestinotrophic effect is determined after administration of 1 dose, or 3 doses, or 6 doses, or 10 doses, or 12 or more doses of the fusion protein.

88. The method of any one of paras 85-87, wherein the intestinotrophic effect is selected from the group consisting of intestinal growth, increased hyperplasia of the villus epithelium, increased crypt cell proliferation, increased height of the crypt and villus axis, increased healing after intestinal anastomosis, increased small bowel weight, increased small bowel length, decreased small bowel epithelium apoptosis, and enhancement of intestinal function.

89. A kit, comprising packaging material and at least a first container comprising the pharmaceutical composition comprising a fusion protein of any one of paras 1 to 42, and a sheet of instructions for the reconstitution and/or administration of the pharmaceutical compositions to a subject.

**Claims**

1. A recombinant fusion protein comprising a glucagon-like protein-2 and an extended recombinant polypeptide, wherein the fusion protein consists of the sequence:

```
HGDGSFSDEMNTILDNLAARDFINWLIQTKITDGGSPAGSPTSTEEGTSES
ATPESGPGTSTEPSEGSAPGSPAGSPTSTEEGTSTEPSEGSAPGTSTEPSE
GSAPGTSESATPESGPGSEPATSGSETPGSEPATSGSETPGSPAGSPTSTE
EGTSESATPESGPGTSTEPSEGSAPGTSTEPSEGSAPGSPAGSPTSTEEGT
STEPSEGSAPGTSTEPSEGSAPGTSESATPESGPGTSTEPSEGSAPGTSES
ATPESGPGSEPATSGSETPGTSTEPSEGSAPGTSTEPSEGSAPGTSESATP
ESGPGTSESATPESGPGSPAGSPTSTEEGTSESATPESGPGSEPATSGSET
PGTSESATPESGPGTSTEPSEGSAPGTSTEPSEGSAPGTSTEPSEGSAPGT
STEPSEGSAPGTSTEPSEGSAPGTSTEPSEGSAPGSPAGSPTSTEEGTSTE
PSEGSAPGTSESATPESGPGSEPATSGSETPGTSESATPESGPGSEPATSG
SETPGTSESATPESGPGTSTEPSEGSAPGTSESATPESGPGSPAGSPTSTE
EGSPAGSPTSTEEGSPAGSPTSTEEGTSESATPESGPGTSTEPSEGSAPGT
SESATPESGPGSEPATSGSETPGTSESATPESGPGSEPATSGSETPGTSES
ATPESGPGTSTEPSEGSAPGSPAGSPTSTEEGTSESATPESGPGSEPATSG
SETPGTSESATPESGPGSPAGSPTSTEEGSPAGSPTSTEEGTSTEPSEGSA
PGTSESATPESGPGTSESATPESGPGTSESATPESGPGSEPATSGSETPGS
EPATSGSETPGSPAGSPTSTEEGTSTEPSEGSAPGTSTEPSEGSAPGSEPA
TSGSETPGTSESATPESGPGTSTEPSEGSAPG
```

2. The recombinant fusion protein of claim 1 for use in treating enteritis in a mammalian subject by achieving an intestinotrophic effect

3. The recombinant fusion protein for use of claim 2, wherein the subject is selected from the group consisting of mouse, rat, monkey, and human.

4. The recombinant fusion protein for use of claim 2 or claim 3, wherein administering said recombinant fusion protein to the subject is subcutaneous, intramuscular, or intravenous.

5. The recombinant fusion protein for use of any of claims 2 to 4, wherein the intestinotrophic effect is an increase in small intestine weight of at least about 10%, or at least about 20%, or at least about 30%.

6. The recombinant fusion protein for use of any of claims 2 to 4, wherein the administration results in an increase in small intestine length of at least about 5%, or at least about 6%, or at least about 7%, or at least about 8%, or at least about 9%, or at least about 10%, or at least about 20%, or at least about 30%.

7. The recombinant fusion protein for use of any of claims 2 to 64, wherein the fusion protein exhibits a terminal half-life that is at least about 30 hours when administered to a subject.

8. A pharmaceutical composition comprising the fusion protein of claim 1, and a pharmaceutically acceptable carrier.

**FIG. 1**

212

**XTEN Sequence**

| 1 | 2 | 3 | 4 | 5 | 6 | 7 | 8 | 9 | 10 | 11 |
|---|---|---|---|---|---|---|---|---|----|----|
| A | S | T | G | E | T | G | E | T | G  | E  |

**XTEN Length: N=11**

**Subsequence length: S=3**

|        |     | Seg 1 AST | Seg 2 STG | Seg 3 TGE | Seg 4 GET | Seg 5 ETG | Seg 6 TGE | Seg 7 GET | Seg 8 ETG | Seg 9 TGE |
|--------|-----|-----------|-----------|-----------|-----------|-----------|-----------|-----------|-----------|-----------|
| Seg 1  | AST | x         |           |           |           |           |           |           |           |           |
| Seg 2  | STG |           | x         |           |           |           |           |           |           |           |
| Seg 3  | TGE |           |           | x         |           |           | x         |           |           |           |
| Seg 4  | GET |           |           |           | x         |           |           | x         |           |           |
| Seg 5  | ETG |           |           |           |           | x         |           |           | x         |           |
| Seg 6  | TGE |           |           | x         |           |           | x         |           |           | x         |
| Seg 7  | GET |           |           |           | x         |           |           | x         |           |           |
| Seg 8  | ETG |           |           |           |           | x         |           |           | x         |           |
| Seg 9  | TGE |           |           |           |           |           | x         |           |           | x         |
|        |     | 1         | 1         | 2         | 2         | 2         | 3         | 2         | 2         | 2         |

**Subsequence Score = 1.89**

**FIG. 2**

**AG864**

```
GASPGTSSTGSPGSSPSASTGTGPGSSPSASTGTGPGTPGSGTASSSPGSSTPSGATGSPGSSPSASTGTG
PGASPGTSSTGSPGTPGSGTASSSPGSSTPSGATGSPGTPGSGTASSSPGASPGTSSTGSPGASPGTSSTG
SPGTPGSGTASSSPGSSTPSGATGSPGASPGTSSTGSPGTPGSGTASSSPGSSTPSGATGSPGSSPSASTG
TGPGSSPSASTGTGPGSSTPSGATGSPGSSTPSGATGSPGASPGTSSTGSPGASPGTSSTGSPGASPGTSS
TGSPGTPGSGTASSSPGASPGTSSTGSPGASPGTSSTGSPGASPGTSSTGSPGSSPSASTGTGPGTPGSGT
ASSSPGASPGTSSTGSPGASPGTSSTGSPGASPGTSSTGSPGSSTPSGATGSPGSSTPSGATGSPGASPGT
SSTGSPGTPGSGTASSSPGSSTPSGATGSPGSSTPSGATGSPGSSTPSGATGSPGSSPSASTGTGPGASPG
TSSTGSPGASPGTSSTGSPGTPGSGTASSSPGASPGTSSTGSPGASPGTSSTGSPGASPGTSSTGSPGASP
GTSSTGSPGTPGSGTASSSPGSSTPSGATGSPGTPGSGTASSSPGSSTPSGATGSPGTPGSGTASSSPGSS
TPSGATGSPGSSTPSGATGSPGSSPSASTGTGPGSSPSASTGTGPGASPGTSSTGSPGTPGSGTASSSPGS
STPSGATGSPGSSPSASTGTGPGSSPSASTGTGPGASPGTSSTGSPGASPGTSSTGSPGSSTPSGATGSPG
SSPSASTGTGPGASPGTSSTGSPGSSPSASTGTGPGTPGSGTASSSPGSSTPSGATGSPGSSTPSGATGSP
GASPGTSSTGSP
```

↓

**AG576**

```
PGTPGSGTASSSPGSSTPSGATGSPGSSPSASTGTGPGSSPSASTGTGPGSSTPSGATGSPGSSTPSGATG
SPGASPGTSSTGSPGASPGTSSTGSPGASPGTSSTGSPGTPGSGTASSSPGASPGTSSTGSPGASPGTSST
GSPGASPGTSSTGSPGSSPSASTGTGPGTPGSGTASSSPGASPGTSSTGSPGASPGTSSTGSPGASPGTSS
TGSPGSSTPSGATGSPGSSTPSGATGSPGASPGTSSTGSPGTPGSGTASSSPGSSTPSGATGSPGSSTPSG
ATGSPGSSTPSGATGSPGSSPSASTGTGPGASPGTSSTGSPGASPGTSSTGSPGTPGSGTASSSPGASPGT
SSTGSPGASPGTSSTGSPGASPGTSSTGSPGTPGSGTASSSPGSSTPSGATGSPGTPGS
GTASSSPGSSTPSGATGSPGTPGSGTASSSPGSSTPSGATGSPGSSPSASTGTGPGSSP
SASTGTGPGASPGTSSTGSPGTPGSGTASSSPGSSTPSGATGSPGSSPSASTGTGPGSSPSASTGTGPGAS
PGTSSTGS
```

**FIG. 3A**

## AG864

GASPGTSSTGSPGSSPSASTGTGPGSSPSASTGTGPGTPGSGTASSSPGSSTPSGATGSPGSSPSASTGTG
PGASPGTSSTGSPGTPGSGTASSSPGSSTPSGATGSPGTPGSGTASSSPGASPGTSSTGSPGASPGTSSTG
SPGTPGSGTASSSPGSSTPSGATGSPGASPGTSSTGSPGTPGSGTASSSPGSSTPSGATGSPGSSPSASTG
TGPGSSPSASTGTGPGSSTPSGATGSPGSSTPSGATGSPGASPGTSSTGSPGASPGTSSTGSPGASPGTSS
TGSPGTPGSGTASSSPGASPGTSSTGSPGASPGTSSTGSPGASPGTSSTGSPGSSPSASTGTGPGTPGSGT
ASSSPGASPGTSSTGSPGASPGTSSTGSPGASPGTSSTGSPGSSTPSGATGSPGSSTPSGATGSPGASPGT
SSTGSPGTPGSGTASSSPGSSTPSGATGSPGSSTPSGATGSPGSSTPSGATGSPGSSPSASTGTGPGASPG
TSSTGSPGASPGTSSTGSPGTPGSGTASSSPGASPGTSSTGSPGASPGTSSTGSPGASPGTSSTGSPGASP
GTSSTGSPGTPGSGTASSSPGSSTPSGATGSPGTPGSGTASSSPGSSTPSGATGSPGTPGSGTASSSPGSS
TPSGATGSPGSSTPSGATGSPGSSPSATASSSPGSSTPSGATGSSTGTGPGSSPSASTGTGPGASPGTSST
GSPGTPGSGTASSSPGSSTPSGATGSPGSSPSASTGTGPGSSPSASTGTGPGASPGTSSTGSPGASPGTSS
TGSPGSSTPSGATGSPGSSPSASTGTGPGASPGTSSTGSPGSSPSASTGTGPGTPGSGTASSSPGSSTPSG
ATGSPGSSTPSGATGSPGASPGTSSTGSP

## AG288

ASSSPGASPGTSSTGSPGASPGTSSTGSPGASPGTSSTGSPGASPGTSSTGSPGTPGSGTASSSPGSST
PSGATGSPGTPGSGTASSSPGSSTPSGATGSPGTPGSGTASSSPGSSTPSGATGSPGSSTPSGATGSPG
SSPSASTGTGPGSSPSASTGTGPGASPGTSSTGSPGTPGSGTASSSPGSSTPSGATGSPGSSPSASTGT
GPGSSPSASTGTGPGASPGTSSTGSPGASPGTSSTGSPGSSTPSGATGSPGSSPSASTGTGPGASPGTS
STGSPGSSPSASTGTGPGTPGSGTASSSPGSSTPSGATGS

## FIG. 3B

**AG864**

GASPGTSSTGS<u>PGSSPSASTGTGPGSSPSASTGTGPGTPGSGTASSSPGSSTPSGATGSPGSSPSASTGTG</u>
<u>PGASPGTSSTGSPGTPGSGTASSSPGSSTPSGATGSPGTPGSGTASSSPGASPGTSSTGSPGASPGTSSTG</u>
<u>SPGTPGSGTASSS</u>PGSSTPSGATGSPGASPGTSSTGSPGTPGSGTASSSPGSSTPSGATGSPGSSPSASTG
TGPGSSPSASTGTGPGSSTPSGATGSPGSSTPSGATGSPGASPGTSSTGSPGASPGTSSTGSPGASPGTSS
TGSPGTPGSGTASSSPGASPGTSSTGSPGASPGTSSTGSPGASPGTSSTGSPGSSPSASTGTGPGTPGSGT
ASSSPGASPGTSSTGSPGASPGTSSTGSPGASPGTSSTGSPGSSTPSGATGSPGSSTPSGATGSPGASPGT
SSTGSPGTPGSGTASSSPGSSTPSGATGSPGSSTPSGATGSPGSSTPSGATGSPGSSPSASTGTGPGASPG
TSSTGSPGASPGTSSTGSPGTPGSGTASSSPGASPGTSSTGSPGASPGTSSTGSPGASPGTSSTGSPGASP
GTSSTGSPGTPGSGTASSSPGSSTPSGATGSPGTPGSGTASSSPGSSTPSGATGSPGTPGSGTASSSPGSS
TPSGATGSPGSSTPSGATGSPGSSPSASTGTGPGSSPSASTGTGPGASPGTSSTGSPGTPGSGTASSSPGS
STPSGATGSPGSSPSASTGTGPGSSPSASTGTGPGASPGTSSTGSPGASPGTSSTGSPGSSTPSGATGSPG
SSPSASTGTGPGASPGTSSTGSPGSSPSASTGTGPGTPGSGTASSSPGSSTPSGATGSPGSSTPSGATGSP
GASPGTSSTGSP

↓

**AG144**

PGSSPSASTGTGPGSSPSASTGTGPGTPGSGTASSSPGSSTPSGATGSPGSSPSASTGTGPGASPGTSS
TGSPGTPGSGTASSSPGSSTPSGATGSPGTPGSGTASSSPGASPGTSSTGSPGASPGTSSTGSPGTPGS
GTASSS

**FIG. 3C**

**AE864**

GSPAGSPTSTEEGTSESATPESGPGTSTEPSEGSAPGSPAGSPTSTEEGTSTEPSEGSAPGTSTEPSEGSA
PGTSESATPESGPGSEPATSGSETPGSEPATSGSETPGSPAGSPTSTEEGTSESATPESGPGTSTEPSEGS
APGTSTEPSEGSAPGSPAGSPTSTEEGTSTEPSEGSAPGTSTEPSEGSAPGTSESATPESGPGTSTEPSEG
SAPGTSESATPESGPGSEPATSGSETPGTSTEPSEGSAPGTSTEPSEGSAPGTSESATPESGPGTSESATP
ESGPGSPAGSPTSTEEGTSESATPESGPGSEPATSGSETPGTSESATPESGPGTSTEPSEGSAPGTSTEPS
EGSAPGTSTEPSEGSAPGTSTEPSEGSAPGTSTEPSEGSAPGSPAGSPTSTEEGTSTEP
SEGSAPGTSESATPESGPGSEPATSGSETPGTSESATPESGPGSEPATSGSETPGTSESATPESGPGTSTE
PSEGSAPGTSESATPESGPGSPAGSPTSTEEGSPAGSPTSTEEGSPAGSPTSTEEGTSESATPESGPGTST
EPSEGSAPGTSESATPESGPGSEPATSGSETPGTSESATPESGPGSEPATSGSETPGTSESATPESGPGTS
TEPSEGSAPGSPAGSPTSTEEGTSESATPESGPGSEPATSGSETPGTSESATPESGPGSPAGSPTSTEEGS
PAGSPTSTEEGTSTEPSEGSAPGTSESATPESGPGTSESATPESGPGTSESATPESGPGSEPATSGSETPG
SEPATSGSETPGSPAGSPTSTEEGTSTEPSEGSAPGTSTEPSEGSAPGSEPATSGSETPGTSESATPESGP
GTSTEPSEGSAP

↓

**AE576**

GSPAGSPTSTEEGTSESATPESGPGTSTEPSEGSAPGSPAGSPTSTEEGTSTEPSEGSAPGTSTEPSEG
SAPGTSESATPESGPGSEPATSGSETPGSEPATSGSETPGSPAGSPTSTEEGTSESATPESGPGTSTEP
SEGSAPGTSTEPSEGSAPGSPAGSPTSTEEGTSTEPSEGSAPGTSTEPSEGSAPGTSESATPESGPGTS
TEPSEGSAPGTSESATPESGPGSEPATSGSETPGTSTEPSEGSAPGTSTEPSEGSAPGTSESATPESGP
GTSESATPESGPGSPAGSPTSTEEGTSESATPESGPGSEPATSGSETPGTSESATPESGPGTSTEPSEG
SAPGTSTEPSEGSAPGTSTEPSEGSAPGTSTEPSEGSAPGTSTEPSEGSAPGTSTEPSEGSAPGSPAGS
PTSTEEGTSTEPSEGSAPGTSESATPESGPGSEPATSGSETPGTSESATPESGPGSEPATSGSETPGTS
ESATPESGPGTSTEPSEGSAPGTSESATPESGPGSPAGSPTSTEEGSPAGSPTSTEEGSPAGSPTSTEE
GTSESATPESGPGTSTEPSEGSAP

**FIG. 3D**

**AE864**

GSPAGSPTSTEEGTSESATPESGPGTSTEPSEGSAPGSPAGSPTSTEEGTSTEPSEGSAPGTSTEPSEGSA
PGTSESATPESGPGSEPATSGSETPGSEPATSGSETPGSPAGSPTSTEEGTSESATPESGPGTSTEPSEGS
APGTSTEPSEGSAPGSPAGSPTSTEEGTSTEPSEGSAPGTSTEPSEGSAPGTSESATPESGPGTSTEPSEG
SAPGTSESATPESGPGSEPATSGSETPGTSTEPSEGSAPGTSTEPSEGSAPGTSESATPESGPGTSESATP
ESGPGSPAGSPTSTEEGTSESATPESGPGSEPATSGSETPGTSESATPESGPGTSTEPSEGSAPGTSTEPS
EGSAPGTSTEPSEGSAPGTSTEPSEGSAPGTSTEPSEGSAPGSPAGSPTSTEEGTSTEP
SEGSAPGTSESATPESGPGSEPATSGSETPGTSESATPESGPGSEPATSGSETPGTSESATPESGPGTSTE
PSEGSAPGTSESATPESGPGSPAGSPTSTEEGSPAGSPTSTEEGSPAGSPTSTEEGTSESATPESGPGTST
EPSEGSAPGTSESATPESGPGSEPATSGSETPGTSESATPESGPGSEPATSGSETPGTSESATPESGPGTS
TEPSEGSAPGSPAGSPTSTEEGTSESATPESGPGSEPATSGSETPGTSESATPESGPGSPAGSPTSTEEGS
PAGSPTSTEEGTSTEPSEGSAPGTSESATPESGPGTSESATPESGPGTSESATPESGPGSEPATSGSETPG
SEPATSGSETPGSPAGSPTSTEEGTSTEPSEGSAPGTSTEPSEGSAPGSEPATSGSETPGTSESATPESGP
GTSTEPSEGSAP

↓

**AE288**

GTSESATPESGPGSEPATSGSETPGTSESATPESGPGSEPATSGSETPGTSESATPESGPGTSTEPSEG
SAPGSPAGSPTSTEEGTSESATPESGPGSEPATSGSETPGTSESATPESGPGSPAGSPTSTEEGSPAGS
PTSTEEGTSTEPSEGSAPGTSESATPESGPGTSESATPESGPGTSESATPESGPGSEPATSGSETPGSE
PATSGSETPGSPAGSPTSTEEGTSTEPSEGSAPGTSTEPSEGSAPGSEPATSGSETPGTSESATPESGP
GTSTEPSEGSAP

**FIG. 3E**

**FIG. 4**

**FIG. 5**

FIG. 6

FIG. 7

FIG. 8

**FIG. 9**

| Seg1_A | Seg2_A | Seg3_A | Seg4_A | Seg5_A | Seg6_A |
|--------|--------|--------|--------|--------|--------|
| Seg1_B | Seg2_B | Seg3_B | Seg4_B | Seg5_B | Seg6_B |
| Seg1_C | Seg2_C | Seg3_C | Seg4_C | Seg5_C | Seg6_C |
| Seg1_D | Seg2_D | Seg3_D | Seg4_D | Seg5_D | Seg6_D |

**Fragment Collection**

**Combinatorial assembly**

| Seg1_B | Seg2_C | Seg3_A | Seg4_D | Seg5_D | Seg6_B |
|--------|--------|--------|--------|--------|--------|
| Seg1_C | Seg2_A | Seg3_C | Seg4_C | Seg5_D | Seg6_A |
| Seg1_A | Seg2_D | Seg3_B | Seg4_B | Seg5_B | Seg6_D |
| Seg1_C | Seg2_B | Seg3_D | Seg4_B | Seg5_A | Seg6_B |
| Seg1_D | Seg2_C | Seg3_B | Seg4_C | Seg5_C | Seg6_C |
| Seg1_B | Seg2_B | Seg3_D | Seg4_A | Seg5_B | Seg6_A |

**Library of XTEN Genes**

**FIG. 10**

A

B

Retention time (min)

FIG. 11

**FIG. 12**

FIG. 13

**FIG. 14**

FIG. 15

**FIG. 16**

FIG. 17

**FIG. 18**

FIG. 19

**FIG. 20**

FIG. 21

FIG. 21

**FIG. 21**

G

**FIG. 21**

FIG. 22

**FIG. 23**

FIG. 24

**FIG. 25**

**FIG. 26**

Time (days)

0    1    7

A

Monkey
plasma

B

Monkey
in vivo

C

Kidney
homogenate

FIG. 27

HGDGSFSDEMNTILDNLAARDFINWLIQTKITDGGSPAGSPTSTEEGTSES
ATPESGPGTSTEPSEGSAPGSPAGSPTSTEEGTSTEPSEGSAPGTSTEPSE
GSAPGTSESATPESGPGSEPATSGSETPGSEPATSGSETPGSPAGSPTSTE
EGTSESATPESGPGTSTEPSEGSAPGTSTEPSEGSAPGSPAGSPTSTEEGT
STEPSEGSAPGTSTEPSEGSAPGTSESATPESGPGTSTEPSEGSAPGTSES
ATPESGPGSEPATSGSETPGTSTEPSEGSAPGTSTEPSEGSAPGTSESATP
ESGPGTSESATPESGPGSPAGSPTSTEEGTSESATPESGPGSEPATSGSET
PGTSESATPESGPGTSTEPSEGSAPGTSTEPSEGSAPGTSTEPSEGSAPGT
STEPSEGSAPGTSTEPSEGSAPGTSTEPSEGSAPGSPAGSPTSTEEGTSTE
PSEGSAPGTSESATPESGPGSEPATSGSETPGTSESATPESGPGSEPATSG
SETPGTSESATPESGPGTSTEPSEGSAPGTSESATPESGPGSPAGSPTSTE
EGSPAGSPTSTEEGSPAGSPTSTEEGTSESATPESGPGTSTEPSEGSAPGT
SESATPESGPGSEPATSGSETPGTSESATPESGPGSEPATSGSETPGTSES
ATPESGPGTSTEPSEGSAPGSPAGSPTSTEEGTSESATPESGPGSEPATSG
SETPGTSESATPESGPGSPAGSPTSTEEGSPAGSPTSTEEGTSTEPSEGSA
PGTSESATPESGPGTSESATPESGPGTSESATPESGPGSEPATSGSETPGS
EPATSGSETPGSPAGSPTSTEEGTSTEPSEGSAPGTSTEPSEGSAPGSEPA
TSGSETPGTSESATPESGPGTSTEPSEGSAPG

# FIG. 28

Europäisches
Patentamt
European
Patent Office
Office européen
des brevets

# EUROPEAN SEARCH REPORT

Application Number

EP 18 17 6085

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2010/239554 A1 (SCHELLENBERGER VOLKER [US] ET AL) 23 September 2010 (2010-09-23) * the whole document * * paragraphs [[0068]] - [[0070]]; figures 13, 14, 24-26; examples 27, 28, 30, 58, 65 * * page 26, column 21, paragraphs [0192],[0215],[0287]; table 3 * * paragraphs [[0192]], [[207]]; claims; table 3 * * claims 1-4; example 74; tables 1,32 * * paragraphs [[0178]], [[0262]], [[0263]], [[0279]]; table 6 * | 1-8 | INV. A61K38/26 C07K14/605 |
| A,D | US 5 789 379 A (DRUCKER DANIEL J [CA] ET AL) 4 August 1998 (1998-08-04) * the whole document * * column 21; sequence 5 * | 1-8 | |
| A | WO 2011/084808 A2 (AMUNIX OPERATING INC [US]; SILVERMAN JOSHUA [US]; SCHELLENBERGER VOLKE) 14 July 2011 (2011-07-14) * the whole document * * page 280, paragraph [0051], - page 285; figures 21-25 * | 1-8 | TECHNICAL FIELDS SEARCHED (IPC)<br><br>A61K<br>C07K |
| A | US 2011/046060 A1 (SCHELLENBERGER VOLKER [US] ET AL) 24 February 2011 (2011-02-24) * the whole document * * pages 156-157, paragraphs [0037],[0038]; example 20; sequence 599 * | 1-8 | |

-/--

The present search report has been drawn up for all claims

| Place of search | Date of completion of the search | Examiner |
|---|---|---|
| Munich | 20 November 2018 | Madruga, Jaime |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 18 17 6085

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GEETHING NATHAN C ET AL: "Gcg-XTEN: An Improved Glucagon Capable of Preventing Hypoglycemia without Increasing Baseline Blood Glucose", PLOS ONE, PUBLIC LIBRARY OF SCIENCE, US, vol. 5, no. 4, 14 April 2010 (2010-04-14), pages 1-11, XP002694467, ISSN: 1932-6203, DOI: 10.1371/JOURNAL.PONE.0010175 [retrieved on 2010-04-14] * the whole document * | 1-8 | |
| A | SCHELLENBERGER V ET AL: "A recombinant polypeptide extends the in vivo half-life of peptides and proteins in a tunable manner", NATURE BIOTECHNOLOGY, NATURE PUBLISHING GROUP, US, vol. 27, no. 12, 15 November 2009 (2009-11-15), pages 1186-1190, XP002694466, ISSN: 1087-0156, DOI: 10.1038/NBT.1588 [retrieved on 2009-11-15] * the whole document * | 1-8 | |
| A | -& VOLKER SCHELLENBERGER ET AL: "Online Supplementary Material: A recombinant polypeptide extends the in vivo half-life of peptides and proteins in a tunable manner", NATURE BIOTECHNOLOGY, vol. 27, no. 12, 15 November 2009 (2009-11-15), pages 1186-1190, XP055190665, ISSN: 1087-0156, DOI: 10.1038/nbt.1588 * the whole document * | 1-8 | TECHNICAL FIELDS SEARCHED (IPC) |

-/--

The present search report has been drawn up for all claims

| Place of search | Date of completion of the search | Examiner |
|---|---|---|
| Munich | 20 November 2018 | Madruga, Jaime |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 5

EUROPEAN SEARCH REPORT

Application Number

EP 18 17 6085

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Marketwired .: "Versartis Data for Type 2 Diabetes Drug VRS-859 (Exenatide-XTEN) Featured at American Diabetes Association Annual Scientific Meeting / Currently Enrolling Type 2 Diabetes Mellitus Patients in Phase I Study ) Versartis Data for Type 2 Diabetes Drug VRS-859 (Exenatide-XTEN) Featured at American Diabet", , 26 June 2010 (2010-06-26), page 1, XP055190668, Orlando Retrieved from the Internet: URL:http://www.finanznachrichten.de/nachrichten-2010-06/17258806-versartis-data-for-type-2-diabetes-drug-vrs-859-exenatide-xten-featured-at-american-diabetes-association-annual-scientific-meeting-currently-enro-256.htm [retrieved on 2015-05-20] * the whole document * | 1-8 | |
| | ----- | | TECHNICAL FIELDS SEARCHED (IPC) |
| T | SUSAN E. ALTERS ET AL: "GLP2-2G-XTEN: A Pharmaceutical Protein with Improved Serum Half-Life and Efficacy in a Rat Crohn's Disease Model", PLOS ONE, vol. 7, no. 11, 26 November 2012 (2012-11-26), page e50630, XP055190652, DOI: 10.1371/journal.pone.0050630 * the whole document * ----- | | |
| | -/-- | | |

The present search report has been drawn up for all claims

| Place of search | Date of completion of the search | Examiner |
|---|---|---|
| Munich | 20 November 2018 | Madruga, Jaime |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 18 17 6085

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | V. N. PODUST ET AL: "Extension of in vivo half-life of biologically active peptides via chemical conjugation to XTEN protein polymer", PROTEIN ENGINEERING DESIGN AND SELECTION, vol. 26, no. 11, 16 October 2013 (2013-10-16), pages 743-753, XP055190651, ISSN: 1741-0126, DOI: 10.1093/protein/gzt048 * the whole document * | | |
| A | TAVAKKOLIZADEH A ET AL: "Glucagon-like Peptide 2: A New Treatment for Chemotherapy-Induced Enteritis", JOURNAL OF SURGICAL RESEARCH, ACADEMIC PRESS INC., SAN DIEGO, CA, US, vol. 91, 1 June 2000 (2000-06-01), pages 77-82, XP002173145, ISSN: 0022-4804, DOI: 10.1006/JSRE.2000.5917 * the whole document * | 1-8 | |
| A | TORRES ET AL: "Glucagon-Like Peptide-2 Improves Both Acute and Late Experimental Radiation Enteritis in the Rat", INTERNATIONAL JOURNAL OF RADIATION: ONCOLOGY BIOLOGY PHYSICS, PERGAMON PRESS, USA, vol. 69, no. 5, 22 November 2007 (2007-11-22), pages 1563-1571, XP022354206, ISSN: 0360-3016, DOI: 10.1016/J.IJROBP.2007.08.051 * the whole document * | 1-8 | |

-/--

The present search report has been drawn up for all claims

| Place of search | Date of completion of the search | Examiner |
|---|---|---|
| Munich | 20 November 2018 | Madruga, Jaime |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

TECHNICAL FIELDS SEARCHED (IPC)

EPO FORM 1503 03.82 (P04C01)

page 4 of 5

Europäisches
Patentamt

European
Patent Office

Office européen
des brevets

# EUROPEAN SEARCH REPORT

Application Number

EP 18 17 6085

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BOUSHEY R P ET AL: "GLUCAGON-LIKE PEPTIDE 2 DECREASES MORTALITY AND REDUCES THE SEVERITY OF INDOMETHACIN-INDUCED MURINE ENTERITIS", AMERICAN JOURNAL OF PHYSIOLOGY, AMERICAN PHYSIOLOGICAL SOCIETY, US, vol. 277, no. 5, 1 November 1999 (1999-11-01), pages E937-E947, XP008010199, ISSN: 0002-9513 * the whole document * ----- | 1-8 | |

TECHNICAL FIELDS
SEARCHED      (IPC)

The present search report has been drawn up for all claims

| Place of search | Date of completion of the search | Examiner |
|---|---|---|
| Munich | 20 November 2018 | Madruga, Jaime |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

...............................................................................................
& : member of the same patent family, corresponding
    document

EPO FORM 1503 03.82 (P04C01)

page 5 of 5

## ANNEX TO THE EUROPEAN SEARCH REPORT
## ON EUROPEAN PATENT APPLICATION NO.

EP 18 17 6085

This annex lists the patent family members relating to the patent documents cited in  the above-mentioned European search report.
The members are as contained in the European Patent Office EDP file on
The European Patent Office is in no way liable for these particulars which are merely given for the purpose of information.

20-11-2018

| Patent document cited in search report | Publication date | Patent family member(s) | Publication date |
|---|---|---|---|
| US 2010239554 A1 | 23-09-2010 | AU 2010210618 A1 | 21-07-2011 |
| | | BR PI1008061 A2 | 15-03-2016 |
| | | CA 2748314 A1 | 12-08-2010 |
| | | CN 102348715 A | 08-02-2012 |
| | | CN 108530543 A | 14-09-2018 |
| | | DK 2393828 T3 | 23-01-2017 |
| | | EA 201170993 A1 | 28-02-2012 |
| | | EP 2393828 A1 | 14-12-2011 |
| | | EP 3178835 A1 | 14-06-2017 |
| | | ES 2610356 T3 | 27-04-2017 |
| | | HR P20161807 T1 | 24-02-2017 |
| | | HU E031840 T2 | 28-08-2017 |
| | | JP 5843357 B2 | 13-01-2016 |
| | | JP 6012684 B2 | 25-10-2016 |
| | | JP 6265564 B2 | 24-01-2018 |
| | | JP 6348946 B2 | 27-06-2018 |
| | | JP 2012516854 A | 26-07-2012 |
| | | JP 2015034165 A | 19-02-2015 |
| | | JP 2016106135 A | 16-06-2016 |
| | | JP 2017031218 A | 09-02-2017 |
| | | JP 2018076378 A | 17-05-2018 |
| | | KR 20110127669 A | 25-11-2011 |
| | | LT 2393828 T | 25-01-2017 |
| | | NZ 593833 A | 25-10-2013 |
| | | NZ 606427 A | 31-10-2014 |
| | | NZ 628987 A | 27-11-2015 |
| | | PL 2393828 T3 | 30-06-2017 |
| | | PT 2393828 T | 18-01-2017 |
| | | US 2010239554 A1 | 23-09-2010 |
| | | US 2014301974 A1 | 09-10-2014 |
| | | US 2017037088 A1 | 09-02-2017 |
| | | US 2018244736 A1 | 30-08-2018 |
| | | WO 2010091122 A1 | 12-08-2010 |
| | | ZA 201104758 B | 27-05-2015 |
| US 5789379 A | 04-08-1998 | NONE | |
| WO 2011084808 A2 | 14-07-2011 | US 2011312881 A1 | 22-12-2011 |
| | | WO 2011084808 A2 | 14-07-2011 |
| US 2011046060 A1 | 24-02-2011 | AU 2010290077 A1 | 22-03-2012 |
| | | AU 2010290131 A1 | 22-03-2012 |
| | | BR 112012004094 A2 | 08-03-2016 |
| | | BR 112012004104 A2 | 15-03-2016 |
| | | CA 2771999 A1 | 10-03-2011 |
| | | CA 2772051 A1 | 10-03-2011 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 18 17 6085

This annex lists the patent family members relating to the patent documents cited in the above-mentioned European search report.
The members are as contained in the European Patent Office EDP file on
The European Patent Office is in no way liable for these particulars which are merely given for the purpose of information.

20-11-2018

| Patent document cited in search report | Publication date | | Patent family member(s) | Publication date |
|---|---|---|---|---|
| | | CN | 102741275 A | 17-10-2012 |
| | | CN | 102741422 A | 17-10-2012 |
| | | EP | 2470559 A1 | 04-07-2012 |
| | | EP | 2470670 A1 | 04-07-2012 |
| | | EP | 3222287 A1 | 27-09-2017 |
| | | JP | 5813641 B2 | 17-11-2015 |
| | | JP | 2013502458 A | 24-01-2013 |
| | | JP | 2013502459 A | 24-01-2013 |
| | | JP | 2015042679 A | 05-03-2015 |
| | | JP | 2015052015 A | 19-03-2015 |
| | | US | 2011046060 A1 | 24-02-2011 |
| | | US | 2011046061 A1 | 24-02-2011 |
| | | US | 2012263701 A1 | 18-10-2012 |
| | | US | 2012263703 A1 | 18-10-2012 |
| | | US | 2014186327 A1 | 03-07-2014 |
| | | US | 2014328819 A1 | 06-11-2014 |
| | | US | 2015344862 A1 | 03-12-2015 |
| | | US | 2016362672 A1 | 15-12-2016 |
| | | US | 2017247676 A1 | 31-08-2017 |
| | | WO | 2011028228 A1 | 10-03-2011 |
| | | WO | 2011028229 A1 | 10-03-2011 |

EPO FORM P0459

For more details about this annex : see Official Journal of the European Patent Office, No. 12/82

page 2 of 2

## REFERENCES CITED IN THE DESCRIPTION

**Patent documents cited in the description**

- US 61573748 A
- US 6406632 B
- US 7294513 B
- US 5789379 A
- US 5834428 A
- US 5990077 A
- US 5994500 A
- US 6184201 B
- US 7186683 B
- US 7563770 B
- US 20020025933 A
- US 20030162703 A
- US 5364934 A
- US 20030228309 A1
- WO 9852976 A
- WO 02079232 A
- WO 003317 A
- EP 36776 A
- EP 139383 A
- US 4943529 A
- EP 402226 A
- EP 183070 A
- EP 244234 A
- EP 394538 A
- US 4882279 A
- US 4599311 A
- US 4931373 A
- US 4870008 A
- US 5037743 A
- US 4845075 A
- EP 272277 A
- EP 238023 A
- EP 184438 A
- US 4713339 A, Levinson and Simonsen
- US 6316024 B
- US 6126966 A
- US 6056973 A
- US 6043094 A

- US 5874104 A
- US 5916588 A
- US 6759057 B
- US 6406713 B
- US 6352716 B
- US 6294191 B
- US 5965156 A
- US 5215680 A
- US 4684479 A
- US 6890918 B
- US 6838093 B
- US 6814979 B
- US 6713086 B
- US 6534090 B
- US 6514532 B
- US 6361796 B
- US 6352721 B
- US 6294201 B
- US 6284276 B
- US 6110498 A
- US 5573776 A
- US 42000984 B
- US 4088864 A
- US 4976696 A
- US 4933185 A
- US 5017378 A
- US 6309370 B
- US 6254573 B
- US 4435173 A
- US 4398908 A
- US 6572585 B
- US 5298022 A
- US 5176502 A
- US 5492534 A
- US 5318540 A
- US 4988337 A
- US 4868122 A

**Non-patent literature cited in the description**

- **REDSTONE, HA et al.** The Effect of Glucagon-Like Peptide-2 Receptor Agonists on Colonic Anastomotic Wound Healing. *Gastroenterol Res Pract.,* 2010

- **LOVSHIN, J. ; D.J. DRUCKER.** Synthesis, secretion and biological actions of the glucagon-like peptides. *Ped. Diabetes,* 2000, vol. 1 (1), 49-57
- **BREMHOLM, L. et al.** Glucagon-like peptide-2 increases mesenteric blood flow in humans. *Scan. J. Gastro.,* 2009, vol. 44 (3), 314-319

- **BUCHMAN, A. L. et al.** Teduglutide, a novel mucosally active analog of glucagon-like peptide-2 (GLP-2) for the treatment of moderate to severe Crohn's disease. *Inflammatory Bowel Diseases,* 2010, vol. 16, 962-973
- **MOOR, BA et al.** GLP-2 receptor agonism ameliorates inflammation and gastrointestinal stasis in murine post-operative ileus. *J Pharmacol Exp Ther.,* 2010, vol. 333 (2), 574-583
- **JEPPESEN PB et al.** Teduglutide (ALX-0600), a dipeptidyl peptidase IV resistant glucagon-like peptide 2 analogue, improves intestinal function in short bowel syndrome patients. *Gut.,* 2005, vol. 54 (9), 1224-1231
- **HARTMANN B et al.** Dipeptidyl peptidase IV inhibition enhances the intestinotrophic effect of glucagon-like peptide-2 in rats and mice. *Endocrinology,* 2000, vol. 141, 4013-4020
- **MARIER JF.** Population pharmacokinetics of teduglutide following repeated subcutaneous administrations in healthy participants and in patients with short bowel syndrome and Crohn's disease. *J Clin Pharmacol.,* 2010, vol. 50 (1), 36-49
- **JEPPESEN PB.** Randomized placebo-controlled trial of teduglutide in reducing parenteral nutrition and/or intravenous fluid requirements in patients with short bowel syndrome. *Gut,* 2011, vol. 60 (7), 902-914
- **LEVITT, M et al.** *J Mol Biol,* 1976, vol. 104, 59
- **HOPP, TP et al.** *Proc Natl Acad Sci U S A,* 1981, vol. 78, 3824
- **SAMBROOK, J. et al.** Molecular Cloning: A Laboratory Manual. Cold Spring Harbor Laboratory Press, 2001
- Current protocols in molecular biology. 1987
- Methods in Enzymology. Academic Press
- PCR 2: a practical approach. Oxford University Press, 1995
- Antibodies, a laboratory manua. Cold Spring Harbor Laboratory, 1988
- Goodman & Gilman's The Pharmacological Basis of Therapeutics. McGraw-Hill, 2005
- **FRESHNEY, R.I.** Culture of Animal Cells: A Manual of Basic Technique. John Wiley & Sons, 2000
- **ORSKOV et al.** *FEBS Lett.,* 1989, vol. 247, 193-196
- **HARTMANN et al.** *Peptides,* 2000, vol. 21, 73-80
- **ESTALL JL ; DRUCKER DJ.** Glucagon-like peptide-2. *Annual Rev Nutr,* 2006, vol. 26, 391-411
- **GUAN X et al.** GLP-2 receptor localizes to enteric neurons and endocrine cells expressing vasoactive peptides and mediates increased blood flow. *Gastroenterology,* 2006, vol. 130, 150-164
- **STEPHENS J et al.** Glucagon-like peptide-2 acutely increases proximal small intestinal blood flow in TPN-fed neonatal piglets. *Am J Physiol Regul Integr Comp Physiol,* 2006, vol. 290, R283-R289
- **NELSON DW et al.** Localization and activation of GLP-2 receptors on vagal afferents in the rat. *Endocrinology,* 2007, vol. 148, 1954-1962
- **BJERKNES M ; CHENG H.** Modulation of specific intestinal epithelial progenitors by enteric neurons. *Proc Natl Acad Sci USA,* 2001, vol. 98, 12497-12502
- **ORSKOV C et al.** GLP-2 stimulates colonic growth via KGF, released by subepithelial myofibroblasts with GLP-2 receptors. *Regul Pept,* 2005, vol. 124, 105-11
- **THULESEN J et al.** Potential targets for glucagon-like peptide 2 (GLP-2) in the rat: distribution and binding of i.v. injected (125)1-GLP-2. *Peptides,* 2000, vol. 21, 1511-1517
- **SIGALET DL et al.** Enteric neural pathways mediate the anti-inflammatory actions of glucagon-like peptide 2. *Am J Physiol Gastrointest Liver Physiol,* 2007, vol. 293, G211-G221
- **JEPPESEN et al.** *Gastroenterology,* 2001, vol. 120, 806-815
- **ARNAU et al.** *Prot Expr and Purif,* 2006, vol. 48, 1-13
- **CHOU, P. Y. et al.** *Biochemistry,* 1974, vol. 13, 222-45
- **GAMIER J ; GIBRAT JF ; ROBSON B.** GOR method for predicting protein secondary structure from amino acid sequence. *Methods Enzymol,* 1996, vol. 266, 540-553
- **JOHANSSON, J. et al.** *Vaccine,* 2007, vol. 25, 1676-82
- **YANKAI, Z. et al.** *Biochem Biophys Res Commun,* 2006, vol. 345, 1365-71
- **HSU, C. T. et al.** *Cancer Res,* 2000, vol. 60, 3701-5
- **BACHMANN MF et al.** *Eur J Immunol.,* 1995, vol. 25 (12), 3445-3451
- **STICKLER, M. et al.** *J Immunol Methods,* 2003, vol. 281, 95-108
- **STUMIOLO, T. et al.** *Nat Biotechnol,* 1999, vol. 17, 555-61
- **STURNIOLO, T. et al.** *Nature Biotechnology,* 1999, vol. 17, 555
- **CALICETI.** Pharmacokinetic and biodistribution properties of poly(ethylene glycol)-protein conjugates. *Adv Drug Deliv Rev,* 2003, vol. 55, 1261-1277
- **GEORGE et al.** *Protein Engineering,* 2003, vol. 15, 871-879
- **D SCHUPPAN ; T FREITAG.** Fistulising Crohn's disease: MMPs gone awry. *Gut,* 2004, vol. 53 (5), 622-624

- **JEPPESEN PB.** Impaired meal stimulated glucagon-like peptide 2 response in ileal resected short bowel patients with intestinal failure. *Gut.,* 1999, vol. 45 (4), 559-963
- **JEPPSSON et al.** *Proc. Natl. Acad. Sci. USA,* 1994, vol. 81, 5690-93
- **KIDD et al.** *Nature,* 1983, vol. 304, 230-34
- **BRAUN et al.** *Eur. J. Clin. Chem. Clin. Biochem.,* 1996, vol. 34, 761-64
- **ENGELS et al.** Agnew. Chem. Int. Ed. Engl. 1989, vol. 28, 716-734
- **GUSTAFSSON, C. et al.** *Trends Biotechnol,* 2004, vol. 22, 346-53
- **SMITH ; WATERMAN.** *Advances in Applied Mathematics,* 1981, vol. 2, 482-489
- **NEEDLEMAN ; WUNSCH.** *Journal of Molecular Biology.,* 1970, vol. 48, 443-453
- **SMITH et al.** *Gene,* 1988, vol. 57, 31-40
- **SUBRAMANI et al.** *Mol. Cell. Biol.,* 1981, vol. 1, 854-864
- **PALMITER et al.** *Science,* 1983, vol. 222, 809-814
- **BOSHART et al.** *Cell,* 1985, vol. 41, 521-530
- **KAUFMAN ; SHARP.** *Mol. Cell. Biol,* 1982, vol. 2, 1304-1319
- **CHANG et al.** *Nature,* 1978, vol. 275, 615
- **GOEDDEL et al.** *Nature,* 1979, vol. 281, 544
- **GOEDDEL.** *Nucleic Acids Res.,* 1980, vol. 8, 4057
- **DEBOER et al.** *Proc. Natl. Acad. Sci. USA,* 1983, vol. 80, 21-25
- **SUMMERS et al.** *Virology,* 1978, vol. 84, 390-402
- **ALBER ; KAWASAKI.** *J. Mol. Appl. Gen.,* 1982, vol. 1, 419-434
- **MCKNIGHT et al.** *The EMBO J.,* 1985, vol. 4, 2093-2099
- **DENOTO et al.** *Nucl. Acids Res.,* 1981, vol. 9, 3719-3730
- **GRAHAM et al.** *J. Gen. Virol.,* 1977, vol. 36, 59-72
- **URLAUB ; CHASIN.** *Proc. Natl. Acad. Sci. USA,* 1980, vol. 77, 4216-4220
- **BEACH ; NURSE.** *Nature,* 1981, vol. 290, 140
- **FLEER et al.** *Bio/Technology,* 1991, vol. 9, 968-975
- **LOUVENCOURT et al.** *J. Bacteriol.,* 1983, vol. 737
- **VAN DEN BERG et al.** *Bio/Technology,* 1990, vol. 8, 135
- **SREEKRISHNA et al.** *J. Basic Microbiol.,* 1988, vol. 28, 265-278
- **CASE et al.** *Proc. Natl. Acad. Sci. USA,* 1979, vol. 76, 5259-5263
- **GLEESON et al.** *J. Gen. Microbiol.,* 1986, vol. 132, 3459-3465
- **C. ANTHONY.** *The Biochemistry of Methylotrophs,* 1982, vol. 269
- **MALARDIER et al.** *Gene,* 1989, vol. 78, 147-156
- **KAUFMAN ; SHARP.** *J. Mol. Biol.,* 1982, vol. 159, 601-621
- **SOUTHERN ; BERG.** *J. Mol. Appl. Genet.,* 1982, vol. 1, 327-341
- **LOYTER et al.** *Proc. Natl. Acad. Sci. USA,* 1982, vol. 79, 422-426
- **WIGLER et al.** *Cell,* 1978, vol. 14, 725
- **CORSARO ; PEARSON.** *Somatic Cell Genetics,* 1981, vol. 7, 603
- **GRAHAM ; VAN DER EB.** *Virology,* 1973, vol. 52, 456
- **NEUMANN et al.** *EMBO J.,* 1982, vol. 1, 841-845
- **WIGLER et al.** *Cell,* 1978, vol. 14, 725-732
- **CORSARO ; PEARSON.** *Somatic Cell Genetics,* 1981, vol. 7, 603-616
- **GRAHAM ; VAN DER EB.** *Virology,* 1973, vol. 52d, 456-467
- **THILLY.** Mammalian Cell Technology. Butterworth Publishers
- **THOMAS.** *Proc. Natl. Acad. Sci. USA,* 1980, vol. 77, 5201-5205
- **ROBERT K. SCOPES.** Protein Purification: Principles and Practice. Springer-Verlag, 1994
- **BARON et al.** *Crit. Rev. Biotechnol.,* 1990, vol. 10, 179-90
- **BELOW et al.** *J. Chromatogr. A.,* 1994, vol. 679, 67-83
- Remington's Pharmaceutical Sciences. 1980
- **SAMBROOK et al.** Molecular Cloning-A Laboratory Manual. Cold Spring Harbor Laboratory Press, 2001
- **MARIER, J-F et al.** Pharmacokinetics, Safety, and Tolerability of Teduglutide, a Glucagon-Like Peptide-2 (GLP-2) Analog, Following Multiple Ascending Subcutaneous Administrations in Healthy Subjects. *J Clin Pharmacol,* 2008, vol. 48, 1289-1299
- **RAWLINGS N.D. et al.** *Nucleic Acids Res.,* 2008, vol. 36, D320
- **HARRIS, JL et al.** *Proc Natl Acad Sci U S A,* 2000, vol. 97, 7754
- **SCHELLENBERGER, V et al.** *Biochemistry,* 1993, vol. 32, 4344
- **MATTHEWS, D. et al.** *Science,* 1993, vol. 260, 1113
- **GARNIER J ; GIBRAT JF ; ROBSON B.** GOR method for predicting protein secondary structure from amino acid sequence. *Methods Enzymol,* 1996, vol. 266, 540-553
- **STURNIOLO, T. et al.** *Nat Biotechnol,* 1999, vol. 17, 555